# EUROPEAN PATENT SPECIFICATION

(11) **EP 2 662 445 B1**
(45) Date of publication and mention of the grant of the patent: **25.10.2017**
(21) Application number: 13179288.9
(22) Date of filing: 28.06.2007
(51) Int. Cl.: C12N 15/09, C12N 1/15, C12N 1/19, C12N 1/21, C12N 5/10, C12N 9/44

(54) **Method for designing mutated enzyme, method for preparing the same and mutated enzyme**
Verfahren zur Konstruktion eines mutierten Enzyms, Verfahren zur Herstellung davon und mutiertes Enzym
Procédé de conception d'enzyme mutée, son procédé de préparation et enzyme mutée

(30) Priority: 04.08.2006 JP 2006213490
(43) Date of publication of application: 13.11.2013
(62) Divisional of application: 07767790.4
(73) Proprietor: Amano Enzyme Inc., Nagoya-shi, Aichi 460-0003 (JP)
(72) Inventor: Iwamoto, Hiroyuki, Hiroshima 729-0292 (JP); Mikami, Bunzo, Kyoto 611-0011 (JP); Yamaguchi, Shotaro, Gifu 509-0109 (JP)
(74) Representative: Steinecke, Peter

(56) References cited:
- EP-A1- 0 605 040
- WO-A-01/51620
- WO-A-99/45124
- JP-A- 2006 174 841
- US-B1- 6 265 197
- DATABASE UniProt [Online] 1 January 1998 (1998-01-01), "SubName: Full=AmyX protein;", XP002540760, retrieved from EBI accession no. UNIPROT:O34587 Database accession no. O34587
- DATABASE Geneseq [Online] 11 March 1998 (1998-03-11), "Trimmed enzyme protein.", XP002717737, retrieved from EBI accession no. GSP:AAW37372 Database accession no. AAW37372
- DATABASE Geneseq [Online] 9 February 2006 (2006-02-09), "Bacterial pullulanase protein sequence.", XP002717738, retrieved from EBI accession no. GSP:AEE27547 Database accession no. AEE27547
- DATABASE UniProt [Online] 1 July 1989 (1989-07-01), "RecName: Full=Isoamylase; EC=3.2.1.68; Flags: Precursor;", XP002717739, retrieved from EBI accession no. UNIPROT:P10342 Database accession no. P10342
- DATABASE Geneseq [Online] 29 July 2004 (2004-07-29), "Klebsiella pneumoniae polypeptide seqid 12330.", XP002717740, retrieved from EBI accession no. GSP:ABO65813 Database accession no. ABO65813
- DATABASE UNIPROT [Online] 25 October 2004 'SubName: Full=Pullulanase; EC=3.2.1.41;' Retrieved from EBI, accession no. UNIPROT:Q63AL5 Database accession no. Q63AL5
- DATABASE UNIPROT [Online] 01 March 2005 'SubName: Full=Thermostable pullulanase; EC=3.2.1.41;' Retrieved from EBI, accession no. UNIPROT:Unreviewed Database accession no. Unreviewed
- DATABASE EMBL [Online] 02 September 1993 'Klebsiella pneumoniae DNA sequence.' Retrieved from EBI, accession no. EMBL:L19312 Database accession no. L19312
- DATABASE GENESEQ [Online] 25 March 2003 'Polypeptide with isoamylase activity from Pseudomonas amyloderamosa.' Retrieved from EBI, accession no. GSP:AAP90615 Database accession no. AAP90615

## Description

### TECHNICAL FIELD

The present invention relates to a mutated enzyme hydrolyzing an α-1, 6-glycosidic linkage.

### BACKGROUND ART

Pullulanase (EC 3.2.1.41) is an enzyme hydrolyzing an α-1,6 linkage of, for example, amylopectin in starch. Pullulanase is an enzyme having a high industrial applicability in the fields of sugar, for example, production of maltooligosaccharides such as glucose, maltose, maltotriose, maltotetraose, maltopentaose and maltohexaose (OLIGOSACCHARIDES, Gordon and Breach Science Publishers, p3), improvement of rice cooking (patent document 1), and the like.

Pullulanase derived from microorganism includes Bacillus sp. APC-9603 (patent document 2), and ones derived from Klebsiella pneumonia (AMANO ENZYME INC.), Bacillus deramificans, Bacillus acidpullulyticus, Bacillus stearothermophilus, Bacillus sectorramus, Bacillus circulans, Bacillus cereus, and Bacillus sectorramus.

Similar to the other enzymes, when pullulanase is used, concentration of substrate and enzyme, reaction temperatures, reaction time, and the like are adjusted depending upon the applications of use. However, with adjustment of such enzyme reaction conditions alone, it may not be possible to produce intended products or to obtain an expected yield. Thus, it has been necessary to modify the properties themselves of pullulanase.

In order to modify the properties of pullulanase, it is necessary that mutants of pullulanase should be produced, and the activity, substrate specificity, and the like, should be evaluated so as to search for an excellent mutant. However, such processes have required much labor. Patent document 3 discloses one example of a mutant of pullulanase.
[Patent document 1 JP H7-289186 A
[Patent document 2] JP H5-292962 A
[Patent document 3] JP 2002-505108 A
[Non-patent document 1] J Mol Biol. 2006 Jun 9; 359 (3): 690-707

In addition, WO 99/45124 A describes the recombinant expression of pullulanase obtained from Bacillus deramificans and modifications of the pullulanase at its amino terminus. JP 2006 174841 A discloses pullulan variants having different pH optima or better durability to oxidants. US 6 265 197 B1 relates to pullulanases variants with improved thermostability and WO 01/51620 A describes pullulanase variants, where certain amino acids of the pullulanase have been modified in order to provide an enzyme having altered properties.

### [DISCLOSURE OF THE INVENTION]

### [Problems to be Solved by the Invention]

One of objects of the present invention is to provide a novel enzyme hydrolyzing an α-1,6-glycosidic linkage whose action properties have been improved. With the change in action property, it is possible to reduce the amount of enzyme to be used, to shorten a reaction time, to increase applications of use, and the like.

### [Means to Solve the Problems]

In order to solve the above-mentioned problems, the present inventors have keenly investigated further. As a result, the present inventors have obtained an important finding regarding the recognition of a substrate in pullulanase derived from Bacillus subtilis strain 168 by making good use of an X-ray analysis technology for a crystalline structure. That is to say, regarding the pullulanase, the present inventors have succeeded in crystallizing it into a state containing a substrate analog (α-cyclodextrin), and in obtaining information about the three-dimensional structure thereof. Thereby, they have clarified a site to which a substrate analog is bound. Thus, amino acid that is thought to be involved in recognition of a substrate has been specified. Furthermore, as a result of comparison between the three-dimensional structure of the pullulanase and the three-dimensional structure of the same kinds of enzymes derived from the other microorganisms, high similarity is recognized as a whole. In particular, it has been determined that the similarity is extremely high in the site relating to the recognition of a substrate. Since such a high similarity is recognized, it is predicted that an amino acid corresponding to the amino acid specified in the above-mentioned pullulanase plays an important role in the recognition of a substrate in each enzyme.

By the way, as to pullulanase of Klebsiella pneumoniae that is one of the enzymes used in the investigation at this time, an active site is searched for by using G4 (maltotetraose) (see, non-patent document 1). The binding site of the substrate indicated therein is located in the vicinity of the binding site of a substrate analog predicted by the above-mentioned method (a method by comparing with pullulanase derived from Bacillus subtilis strain 168). This fact supports the involvement of the binding site of the substrate analog successfully found by the present inventors in the recognition of actual substrate.

The present invention relates to the embodiments characterized in the claims and is mainly based on the above-mentioned results and provides an enzyme mentioned below.
[1] A mutated enzyme, wherein the activity and/or substrate specificity in terms of its Km value and/or Kcat value for hydrolyzing an α-1,6-glycosidic linkage with respect to pullulan or amylopectin in 50 mM acetate buffer at pH 5.6 at 25 °C is improved as compared with the enzyme to be mutated, comprising an amino acid sequence in which one or two or more amino acids selected from the group shown below in an amino acid sequence of an enzyme (enzyme to be mutated) that hydrolyzes an α-1,6-glycosidic linkage, the group consisting of an amino acid in the substrate binding site of the enzyme and involved in binding of the substrate, which corresponds to an amino acid at the 292 position, an amino acid corresponding to an amino acid at the 371 position, an amino acid corresponding to an amino acid at the 406 position, an amino acid corresponding to an amino acid at the 407 position, an amino acid corresponding to an amino acid at the 437 position, an amino acid corresponding to an amino acid at the 465 position, an amino acid corresponding to an amino acid at the 475 position, an amino acid corresponding to an amino acid at the 476 position; an amino acid corresponding to an amino acid at the 525 position, an amino acid corresponding to an amino acid at the 526 position, an amino acid corresponding to an amino acid at the 580 position and an amino acid corresponding to an amino acid at the 582 position of the amino acid sequence set forth in SEQ ID NO: 2 is/are substituted with another amino acid or deleted, wherein the amino acid sequence of the enzyme to be mutated is the amino acid sequence set forth in SEQ ID NO: 13 and wherein the mutated enzyme differs from the parent enzyme in less than 10% with respect to the entire amino acid sequence.
[2] The mutated enzyme according to [1], wherein the substituted or deleted amino acid is one or two or more amino acids selected from the group consisting of an amino acid corresponding to an amino acid at the 292 position, an amino acid corresponding to an amino acid at the 371 position, an amino acid corresponding to an amino acid at the 407 position, an amino acid corresponding to an amino acid at the 475 position, an amino acid corresponding to an amino acid at the 476 position, and an amino acid corresponding to an amino acid at the 582 position of the amino acid sequence set forth in SEQ ID NO: 2.
[3] The mutated enzyme according to [1], wherein the substituted or deleted amino acid is an amino acid corresponding to an amino acid at the 476 position of the amino acid sequence set forth in SEQ ID NO: 2.
[4] The mutated enzyme according to any one of [1] to [3], wherein the enzyme to be mutated is a wild type enzyme.
[5] The mutated enzyme according to any one of [1] to [3], wherein the enzyme to be mutated is pullulanase or isoamylase derived from a microorganism.
[6] The mutated enzyme according to [5], wherein the microorganism is a microorganism of genus bacillus, a microorganism of genus Klebsiella, or a microorganism of genus pseudomonas.
[7] The mutated enzyme according to any of [1] to [6], wherein an action property with respect to pullulan or an action property with respect to amylopectin is improved as compared with the enzyme to be mutated.
[8] A gene encoding the mutated enzyme according to any of [1] to [7].
[9] A recombinant DNA including the gene according to [8].
[10] A microorganism carrying the recombinant DNA according to [9].

### [BRIEF DESCRIPTION OF THE DRAWINGS]

Fig. 1 is a view showing a three-dimensional structure of Bacillus subtilis pullulanase having α-cyclodextrin as a ligand, which is shown by the use of a ribbon model. CD: α-cyclodextrin.
Fig. 2 is a view shown by superimposing Bacillus subtilis pullulanase (BSP) having α-cyclodextrin as a ligand and α carbon of pullulanase (KPP) Klebsiella pneumonia onto each other. CD: α-cyclodextrin.
Fig. 3 is an enlarged view showing a substrate binding region of Fig. 2. CD: α-cyclodextrin, G4: maltotetraose. An amino acid of Bacillus subtilis pullulanase (upper stage) and an amino acid of pullulanase of Klebsiella pneumonia (lower stage) corresponding to the amino acid of the upper stage are shown.
Fig. 4 is a view shown by superimposing Bacillus subtilis pullulanase (BSP) having α-cyclodextrin as a ligand and α carbon of isoamylase of Pseudomonas amyloderamosa (PIA) onto each other. CD: α-cyclodextrin.
Fig. 5 is an enlarged view showing a substrate binding region of Fig. 4. CD: α-cyclodextrin, G4: maltotetraose. An amino acid of Bacillus subtilis pullulanase (upper stage) and an amino acid of isoamylase of Pseudomonas amyloderamosa (lower stage) corresponding to the amino acid of the upper stage are shown.

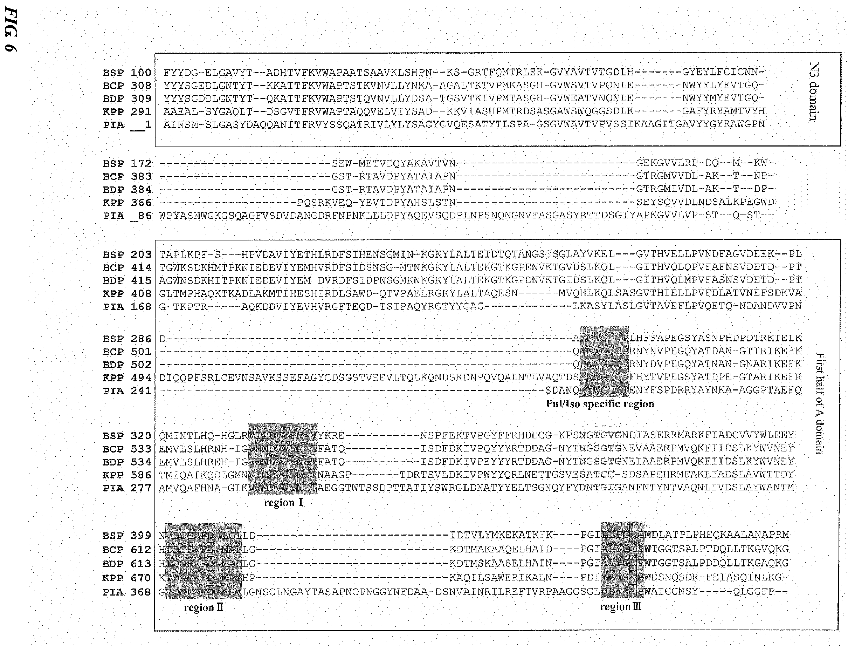

Fig. 6 shows a multiple alignment of amino acid sequences of five kinds of pullulanase and isoamylase. BSP: pullulanase of Bacillus subtilis, BCP: pullulanase of Bacillus sp. APC-9603, BDP: pullulanase of Bacillus deramificans, KPP: pullulanase of Klebsiella pneumoniae, PIA: isoamylase of Pseudomonas amyloderamosa (Amemura, A., Chakraborty, R., Fujita, M., Noumi, T. and Futai, M., Cloning and nucleotide sequence of the isoamylase gene from Pseudomonas amyloderamosa SB-15, JOURNAL J. Biol. Chem. 263 (19), 9271-9275 (1988)). *: position of an amino acid that has been deduced to be involved in binding to α-cyclodextrin, +: amino acid that has been deduced to be involved in binding to G4 altotetraose) among amino acids that have been deduced to be involved in binding to α-cyclodextrin in the above-mentioned report, -: position of the amino acid that has been deduced to be involved in binding to α-cyclodextrin in the above-mentioned report (excluding the position of amino acid that has been deduced to be involved in binding to α-cyclodextrin). Pul / Iso (pullulanase /isoamylase) specific region, region I, region II, region III and region IV are shown by a shaded area. Furthermore, an amino acid of the active site is surrounded by square.
Fig. 7 is the continuation of Fig. 6.
Fig. 8 is an enlarged view showing an α-cyclodextrin biding site of Ifg. 1.

### [BEST MODE FOR CARRYING OUT THE INVENTION ]

The present invention relates to the embodiments characterized in the claims.

### 1. Designing method of mutated enzyme

The present application discloses a designing method of a mutated enzyme based on an enzyme hydrolyzing an α-1, 6-glycosidic linkage. With the designing method, it is possible to obtain an enzyme that is different from the enzyme before mutation in terms of action properties. In other words, the designing method is used as a technique for changing the action properties of an enzyme. Specifically, for example, the designing method can be used for the purpose of improving the activity and/or substrate specificity of pullulanase with respect to pullulan, or the activity and/or substrate specificity of pullulanase with respect to amylopectin. It can be expected that the improvement of the activity enables obtaining of a sufficient effect with less amount. That is to say, reduction of the amount to be used can be expected. On the other hand, the improvement of the substrate specificity facilitates the use thereof and reduces the amount to be used. Furthermore, if different substrate specificities are provided, a novel application of use can be achieved.

Pullulanase that is one of the enzymes hydrolyzing an α-1, 6-glycosidic linkage can act on amylopectin in starch so as to form straight chain amylase. By the use of this characteristic, pullulanase has been widely used for processing starch, production of glucose, maltose, oligosaccharide, or the like, or brewing. Furthermore, pullulanase is an enzyme that is used for various industrial purposes of, for example, manufacturing a material of thermally stable microcapsule, a carrier of an immobilized enzyme, and the like. If the reactivity with respect to α-1,6 binding can be freely changed, for example, it is possible to increase the yield in products, to reduce the amount of enzyme to be used (an amount to be added). At the same time, this enzyme can be applied to new fields.

In the present specification, unless otherwise noted, the term "action property" is used as a term including properties (including activity and substrate specificity with respect to pullulan and activity and substrate specificity with respect to amylopectin) relating to the actions for hydrolyzing an α-1,6-glycosidic linkage. The evaluation of the "action property" can be carried out by using the Km value, Kcat value, and the like, obtained by the test system using pullulan, amylopectin, and starch as a substrate. Km value, Kcat value can be determined by the following method.
(1) Substrates (for example, pullulan or amylopectin) with various concentrations are dissolved in 50 mM acetate buffer (pH 5.6) and reacted at 25°C.
(2) The concentration of a reducing sugar contained in a regularly sampled reaction solution is determined by a Park-Johnson method, and the reaction rate is measured from the increasing rate of the reducing sugar.
(3) Km value and Kcat value are obtained by curve fitting into Michaelis-Menten equation by the non-linear minimum square method.

Note here that although depending upon the experiment conditions, by comparing the concentrations of the reducing sugar contained in the reaction solution at certain points, the action properties of two enzymes can be compared and evaluated.

The designing method of the mutated enzyme includes roughly two steps, that is, a step of specifying an amino acid to be mutated (step (1)) and a step of constructing an amino acid sequence of the mutated amino acid (step (2)). Hereinafter, the respective steps are described in detail. Note here that in this specification, an enzyme as a base in designing a mutated enzyme (an enzyme to which mutation is carried out) is referred to as "enzyme to be mutated."

### Step (1)

In step (1), in an amino acid sequence of an enzyme (enzyme to be mutated) hydrolyzing an α-1,6-glycosidic linkage, one or two or more of amino acid(s) to which mutation is carried out (hereinafter, which is also referred to as "amino acid to be mutated") is specified. The amino acid to be mutated is selected from the group shown below in an amino acid sequence of an enzyme that hydrolyzes an α-1,6-glycosidic linkage, that is, the group consisting of an amino acid corresponding to an amino acid at the 292 position, an amino acid corresponding to an amino acid at the 371 position, an amino acid corresponding to an amino acid at the 406 position, an amino acid corresponding to an amino acid at the 407 position, an amino acid corresponding to an amino acid at the 437 position, an amino acid corresponding to an amino acid at the 465 position, an amino acid corresponding to an amino acid at the 475 position, an amino acid corresponding to an amino acid at the 476 position; an amino acid corresponding to an amino acid at the 525 position, an amino acid corresponding to an amino acid at the 526 position, an amino acid corresponding to an amino acid at the 580 position and an amino acid corresponding to an amino acid at the 582 position of the amino acid sequence set forth in SEQ ID NO: 2. Note here that these amino acids to be mutated are amino acids that have been suggested to be involved in the recognition of the substrate as a result of analysis of the three-dimensional structure at the time of binding of a substrate analog (α-cyclodextrin, hereinafter, referred to as "CD") regarding pullulanase derived from Bacillus subtilis strain 168 (including the amino acid sequence set force in SEQ ID NO: 2), and from the comparison results between this three-dimensional structure and the three-dimensional structure of an enzyme derived from a microorganism. By mutating these amino acids, it is expected that the action property (in particular, substrate specificity) of the enzyme is changed.

Herein, the term "corresponding" to be used for amino acid residues in the specification means the equal contribution to exhibition of the function between proteins (enzymes) to be compared. In particular, it means that the contribution to the substrate specificity is equivalent. For example, when an amino acid sequence to be compared is arranged with respect to the reference amino acid sequence (that is to say, amino acid sequence set forth in SEQ ID NO: 2) so that suitable comparison can be carried out while considering the partial homology of the primary structure (that is to say, an amino acid sequence) (at this time, a gap may be introduced so as to optimize the alignment if necessary), an amino acid in a position corresponding to a certain amino acid in the reference to amino acid sequence can be defined as "corresponding amino acid." Instead of comparison between primary structures, or in addition thereto, by comparison between the stereostructures (three-dimensional structures), "corresponding amino acid" can be specified. By using the three-dimensional structure information, it is possible to comparison results with high reliability. In this case, atomic coordinates of the three-dimensional structures of a plurality of enzymes can be compared with each other so as to carry out alignment. The three-dimensional structure information on the enzyme to be mutated can be obtained from, for example, Protein Data Bank (http://www.pdbj.org/index_j.html).

An example of the method of determining the three-dimensional structure of protein by an X-ray analysis of crystalline structure is described below.
(1) Protein is crystallized. The crystallization is indispensable for determination of the three-dimensional structure. Besides, the crystallization is industrially useful as a purification of protein with high purity and a preservation method of protein with high density. In this case, protein to which a substrate or an analog compound thereof is bound as a ligand may be crystallized.
(2) The prepared crystal is irradiated with X ray and analysis data are collected. Note here that protein crystal may be damaged by X ray irradiation and may be deteriorated in its diffraction ability so often. In such a case, a low-temperature measurement method for rapidly cooling a crystal to about -173°C and collecting diffraction data in this state has been recently widespread. Note here that finally, in order to collect high resolution data used for determining a structure, synchrotron radiation light with high intensity is used.
(3) For carrying out analysis of a crystalline structure, phase information is necessary in addition to the diffraction data. When the crystalline structure of a related protein with respect to the intended protein is not known, it is impossible to determine the structure by a molecule substitution method. Problem as to the phase must be resolved by the heavy atom isomorphous replacement method. The heavy atom isomorphous replacement method is a method of introducing a metal atom having a larger atomic number such as mercury and platinum into a crystal and using the contribution of the metal atom to X-ray diffraction data of X-ray scattering power, thereby obtaining phase information. The determined phase can be improved by smoothing the electron density in the solvent region in the crystal. Since the water molecule in the solvent region is largely fluctuated, electrical density is hardly observed. Therefore, by approximating the electron density in this region to 0, it can approach to the real electron density. Consequently, the phase is improved. Furthermore, when a plurality of molecules are included in an asymmetrical unit, by averaging the electron densities of these molecules, the phase is further radically improved. A protein model is fitted to the view of the electron density calculated by using the thus improved phase. This process is carried out by using a program such as QUANTA (MSI, America) on a computer graphics. Thereafter, by using a program such as X-PLOR (MSI), refinement of the structure is carried out. Thus, the structure analysis is completed.

When the crystalline structure of a related protein with respect to the intended protein is known, the structure can be determined by a molecule substitution method by using an atomic coordinate of the known protein. The molecule substitution and structure refinement can be carried out by using a program such as CNS_SOLUE ver.11.

The present inventors have tried to crystallize the recombinant pullulanase purified from Bacillus subtilis strain 168 and to crystallize the pullulanase in a state in which it contains CD as a substrate analog and have succeeded in obtaining three-dimensional structure of both types of pullulanase. Note here that atomic coordinates of the three-dimensional structure of the pullulanase containing CD are shown in the last part of this specification. Furthermore, the amino acid sequence of pullulanase and the base sequence of a gene encoding thereof are shown in SEQ ID NO: 2 and SEQ ID NO: 1 in the sequence listing, respectively.

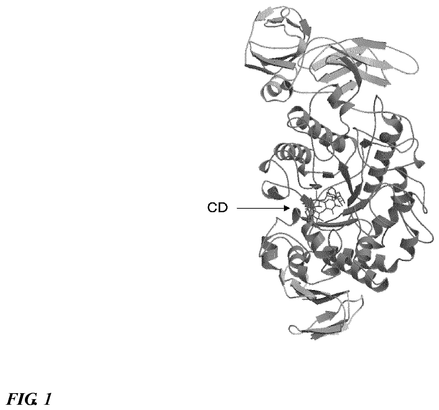

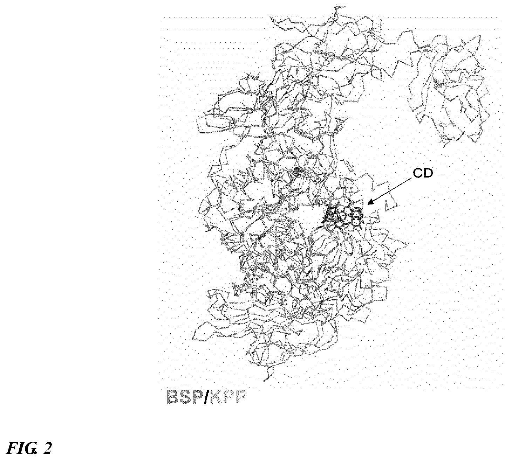

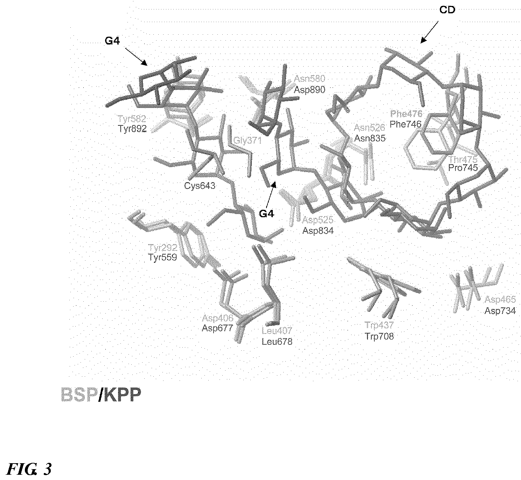

As shown in the below-mentioned Examples, it has been determined that the pullulanase molecule derived from Bacillus subtilis strain 168 has rhombic system P2(1)2(1)2(1) having 70.568 × 127.68 × 189.25 Ǻ (see, Figs. 1 to 3). Fig. 1 is a view showing a crystalline structure of pullulanase by a ribbon model. α-helix and β-sheet are shown in a helix shape and an arrow shape, respectively (Fig. 1), and a substrate analog (CD) is shown by an arrow CD (Figs. 1 to 3). Fig. 2 is a view shown by superimposing Bacillus subtilis pullulanase (BSP) having α-cyclodextrin as a ligand and α carbon of pullulanase (KPP) Klebsiella pneumonia onto each other. Fig. 3 is an enlarged view of a substrate binding region of Fig. 2.

The amino acid to be mutated is selected from the group consisting of an amino acid corresponding to an amino acid at the 292 position, an amino acid corresponding to an amino acid at the 371 position, an amino acid corresponding to an amino acid at the 407 position, an amino acid corresponding to an amino acid at the 475 position, an amino acid corresponding to an amino acid at the 476 position and an amino acid corresponding to an amino acid at the 582 position of the amino acid sequence set forth in SEQ ID NO: 2. Note here that amino acids to be mutated are an amino acid corresponding to an amino acid that has been determined to be directly involved in binding between pullulanase derived from Bacillus subtilis strain 168 and a substrate analog (CD).

By the way, it has clarified that an amino acid at the 476 position of the amino acid sequence set forth in SEQ ID NO: 2 is arranged so that it enters a ring structure of CD that is a substrate analog in the three-dimensional structure analysis about pullulanase derived from Bacillus subtilis strain 168, and this amino acid is thought to play an important role in the recognition of a substrate. Therefore, an amino acid corresponding to the amino acid is to be an amino acid to be mutated.

The enzyme to be mutated includes an enzyme (pullulanase of Klebsiella pneumoniae ATCC9621) consisting of an amino acid sequence set forth in SEQ ID NO: 13.

An enzyme consisting of an amino acid sequence having a high identity with the amino acid sequence set forth in SEQ ID NO: 2 is described as an example as an enzyme to be mutated.

Herein, the identity (%) between two amino acid sequences can be determined by the following procedure. Firstly, two sequences are aligned for optimum comparison of the two sequences (for example, a gap may be introduced in the first sequence so as to obtain an optimum alignment with the second sequence). When a molecule (amino acid residue) at the specific position in the first sequence and a molecule in the corresponding position in the second sequence are the same, the molecules in the positions are defined as being identical. The identity between two sequences is an action property of the number of identical positions shared by the sequences (i.e., identity (%) = number of identical positions / total number of positions × 100), preferably taking into account the number of gaps, and the length of each gap, which need to be introduced for optimal alignment of the two sequences.

The comparison and determination of identity between two sequences can be carried out by using a mathematical algorithm. A specific example of mathematical algorithm that can be used for comparing sequences include an algorithm described in Karlin and Altschul (1990) Proc. Natl. Acad. Sci. USA 87:2264-68 and modified by Karlin and Altschul (1993) Proc. Natl. Acad. Sci. USA 90:5873-77 but the algorithm is not limited to this. Such an algorithm is incorporated in NBLAST and XBLAST programs (version 2.0) of Altschul et al. (1990) J. Mol. Biol. 215: 403-10. BLAST polypeptide searches may be carried out by, for example, the NBLAST program, score = 50, wordlength = 3 to obtain amino acid sequence homologous to a certain amino acid sequence. To obtain gapped alignments for comparison purposes, Gapped BLAST as described in Altschul et al., (1997) Amino Acids Research 25(17): 3389-3402 can be utilized. When utilizing BLAST and Gapped BLAST programs, the default parameters of the respective programs (e.g., XBLAST and NBLAST) can be used. In detail, see http://www.ncbi.nlm.nih.gov. Another example of mathematical algorithm that can be used for comparing sequences includes an algorithm of Meyers and Miller (Comput. Appl. Biosci. 4: 11-17 (1988)) which has been incorporated into the ALIGN program that can be used for, for example, GENESTREAM network server (IGH Montpellier, France) or ISREC server. When the ALIGN program is used for comparison of the amino acid sequences, for example, a PAM120 weight residue table can be used with a gap length penalty of 12 and a gap penalty of 4.

The identity between two amino acid sequences can be determined using the GAP program in the GCG software package, using a Blossom 62 matrix or PAM250 matrix and a gap weight of 12, 10, 8, 6, or 4, and a gap length weight of 2, 3, or 4. Furthermore, the homology between two nucleic acid sequences can be determined using the GAP program in the GCG software package (available at http://www.gcg.com) with a gap weight of 50 and a gap length weight of 3.

The enzyme to be mutated is typically a wild type enzyme (naturally occurring enzyme). However, an enzyme to which some mutation or modification has already been given is not excluded. Thus, the present invention can be used for the purpose of further improving the property of an enzyme.

### Step (2)

In the step (2), an amino acid sequence, in which an amino acid specified in the step (1) has been substituted with another amino acid or the amino acid has been deleted, is constructed based on an amino acid sequence of the enzyme to be mutated. The kinds of substituted amino acids are not particularly limited and therefore may include conservative substitution of amino acid or non-conservative substitution of amino acid. Herein, a "conservative amino acid substitution" is one in which the amino acid residue is substituted with an amino acid residue having a side chain with similar feature. The amino acid residues are divided into some families including basic side chains (e. g. , lysine, arginine, histidine), acidic side chains (e.g., aspartic acid, glutamic acid), uncharged polar side chains (e. g., asparagine, glutamine, serine, threonine, tyrosine, cysteine), nonpolar side chains (e. g, glycine, alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan). Preferably, the conservative amino acid substitution is a substitution between preferably an amino acid residue of the same family.

### 2. Preparation method of mutated enzyme

The present application also discloses a preparation method of a mutated enzyme. The preparation method includes the following steps:
(1) preparing nucleic acid encoding an amino acid sequence constructed by the designing method as described above;
(2) expressing the nucleic acid; and
(3) collecting expression products.

In the step (1), necessary mutation (that is, substitution or deletion of amino acids in a certain position in protein as an expression product) is applied to a gene encoding the enzyme to be mutated based on an amino acid sequence constructed by the designing method, and thereby nucleic acid (gene) encoding a mutated enzyme is obtained. A large number of methods for the position specific substitution of base sequence have been known (see, for example, Molecular Cloning, Third Edition, Cold Spring Harbor Laboratory Press, New York). Among them, appropriate methods can be selected and used.

As the position specific mutation introduction method, a position specific amino acid saturated mutation method can be employed. The position specific amino acid saturated mutation method is a "Semi-rational, semi-random" technique in which a position relating to the intended function is deduced based on the three-dimensional structure of protein, and the amino acid saturated mutation is introduced (J. Mol. Biol. 331, 585-592 (2003)). For example, a position specific amino acid saturated mutation can be introduced by using a kit such as Quick change (Stratagene), Overlap extension PCR (Nucleic Acid Res. 16, 7351-7367 (1988)). As DNA polymerase used for PCR, Taq polymerase can be used. However, it is preferable that DNA polymerase with high purify, for example, KOD-PLUS- (TOYOBO), Pfu turbo (Stratagene) are used.

On the other hand, a gene encoding a mutated enzyme can be prepared by inserting random mutation into an enzyme gene, comparing the substrate specificities of expression product by mutants each other, and selecting a gene having preferable substrate specificity. When such a random mutation is introduced, firstly, for example, error-prone PCR is used and mutation is introduced into a targeted gene region randomly so as to construct a mutated enzyme gene library. Then, a clone is selected from the resultant library using the enzymatic activity or the substrate specificity as an index.

In the step (2), a gene prepared in the step (1) is expressed. Then, in the subsequent step (3), mutated enzymes as expression products are collected.

In general, from the step of expressing a gene to the step of collecting the expression products (mutated enzymes) are carried out by using an appropriate host-vector system. However, a cell-free synthesis system may be used. As to the detail of the preparation method of the mutated enzyme by using a host-vector system, the below-mentioned description may be employed (see, the column of 4. Nucleic acid encoding mutated enzyme).

Herein, the "cell-free synthesis system (cell-free transcription system, cell-free transcription / translation system)" denotes that living cells are not used but a ribosome derived from living cells (or cells obtained by genetically engineering technique) or by using a transcription / translation factor and the like, mRNA or protein encoded by nucleic acid (DNA or mRNA) as a template are synthesized from them in vitro. In general, in the cell-free synthesis system, a cell extract obtained by purifying a cell homogenized solution if necessary is used. In general, a cell extract includes ribosome necessary to synthesis of protein, various factors such an initiation factor, various enzymes such as tRNA. When synthesis of protein is carried out, various amino acids, energy sources such as ATP and GTP, creatine phosphate, and the like, are added to the cell extract solution. Needless to say, at the time of synthesis of protein, additionally prepared ribosome or various factors, and/or various enzymes may be replenished if necessary.

Development of a transcription / translation system in which each molecule (factor) necessary to synthesis of protein is reconstructed has been reported (Shimizu, Y. et al.: Nature Biotech., 19, 751-755, 2001). In this system, a gene of 31 kinds of factors consisting of three kinds of initiation factors constituting a protein synthesis system of bacteria, three kinds of elongation factors, four kinds of factors involved in termination, 20 kinds of aminoacyl tRNA synthases for binding each amino acid to tRNA, and methionyl tRNA formyl transferase is amplified from Escherichia coli genome. They are used so as to reconstruct a protein synthesis system in vitro. Such a re-constructed synthesis system may be used.

The term "cell-free transcription /translation system" can be used interchangeably with the term cell-free protein synthesis system, in vitro translation system or in vitro transcription / translation system. In the in vitro translation system, protein is synthesized by using RNA as a template. As the template RNA, total RNA, mRNA, in vitro transcription product, and the like, are used. On the other hand, in the in vitro transcription / translation system, DNA is used as a template. The template DNA should include a ribosome-binding region and preferably includes an appropriate terminator sequence. Note here that the in vitro transcription / translation system sets a condition in which factors necessary to reaction are added so that the transcription reaction and translation reaction proceed consecutively.

### 3. Mutated enzyme

According to the above-mentioned preparation method, it is possible to obtain a mutated enzyme in which the action property with respect to α-1, 6-glycosidic linkage has been changed. The present invention provides a mutated enzyme as characterized in the claims. In the mutated enzyme of the present invention, an action property with respect to pullulan or action property with respect to amylopectin are improved over the enzyme to be mutated.

The mutated enzyme of the present invention is an amino acid sequence wherein in the amino acid sequence of enzyme (enzyme to be mutated) hydrolyzing an α-1,6-glycosidic linkage, one or two or more amino acids selected from the group consisting of an amino acid corresponding to an amino acid at the 292 position, an amino acid corresponding to an amino acid at the 371 position, an amino acid corresponding to an amino acid at the 406 position, an amino acid corresponding to an amino acid at the 407 position, an amino acid corresponding to an amino acid at the 437 position, an amino acid corresponding to an amino acid at the 465 position, an amino acid corresponding to an amino acid at the 475 position, an amino acid corresponding to an amino acid at the 476 position; an amino acid corresponding to an amino acid at the 525 position, an amino acid corresponding to an amino acid at the 526 position, an amino acid corresponding to an amino acid at the 580 position and an amino acid corresponding to an amino acid at the 582 position of the amino acid sequence set forth in SEQ ID NO: 2 is/are substituted with another amino acid or deleted, wherein the amino acid sequence of the enzyme to be mutated is the amino acid sequence set forth in SEQ ID NO: 13 and wherein the mutated enzyme differs from the parent enzyme in less than 10% with respect to the entire amino acid sequence.

Preferably, the substituted or deleted amino acid is one or two or more amino acid selected from the group consisting of an amino acid corresponding to an amino acid at the 292 position, an amino acid corresponding to an amino acid at the 371 position, an amino acid corresponding to an amino acid at the 407 position, an amino acid corresponding to an amino acid at the 475 position, an amino acid corresponding to an amino acid at the 476 position, and an amino acid corresponding to an amino acid at the 582 position of the amino acid sequence set forth in SEQ ID NO: 2.

Further preferably, the substituted or deleted amino acid is an amino acid corresponding to an amino acid at the 476 position of the amino acid sequence set forth in SEQ ID NO: 2.

The enzyme to be mutated includes an enzyme (pullulanase of Klebsiella pneumoniae ATCC9621) consisting of an amino acid sequence set forth in SEQ ID NO: 13.

The mutated enzyme of the present invention is characterized by having an amino acid sequence in which a certain position of the amino acid sequence of the enzyme before mutation (enzyme to be mutated) has been mutated. In a position other than the position related to the mutation, a part of the amino acid may be mutated or modified. Thus, the present invention also provides protein having the same function as compared with the mutated enzyme having the amino acid sequence to which the above-mentioned mutation has been given but having a difference in a part of the amino acid sequence (hereinafter, also referred to as "homologous protein"). The term "having a difference in a part of the amino acid sequence" typically means that the amino acid sequence is mutated (changed) by the deletion and substitution of one to several amino acids constituting the amino acid sequence, or addition of one to several amino acids, or the combination thereof. The difference in the amino acid sequence herein is acceptable as long as the properties related to hydrolysis of an α-1,6-glycosidic linkage are not radically reduced (preferably, in the limit substantially held). As long as this condition is satisfied, the position of difference in the amino acid sequence is not particularly limited and the difference may occur in a plurality of positions. The plurality herein signifies a numerical value corresponding to less than 10%, preferably less than about 5%, and most preferably less than about 1% with respect to the entire amino acid. That is to say, homologous protein has, for example, 90% or more, preferably about 95% or more and most preferably about 99% or more of identity to the amino acid sequence of the mutated enzyme.

The mutated enzyme can be used for arbitrary applications that need hydrolyzing an α-1,6-glycosidic linkage. For example, the mutated enzyme can be used for production of maltooligosaccharide such as glucose, maltose, maltotriose, maltotetraose, maltopentaose, and maltohexaose, and for the improvement of rice cooking, and the like. The amount of the mutated enzyme to be used can be appropriately set so that the intended effect can be exhibited. For example, the enzyme reaction can be carried out under conditions so that the enzyme concentration in the reaction solution becomes about 10 nM to about 100 µM.

### 4. Nucleic acid encoding mutated enzyme

The present invention further provides a nucleic acid related to a mutated enzyme of the present invention. That is to say, the present invention provides a gene encoding a mutated enzyme, nucleic acid that can be used as a probe for identifying a nucleic acid encoding a mutated enzyme, and nucleic acid that can be used as a primer for amplifying or mutating a nucleic acid encoding the mutated enzyme.

A gene encoding a mutated enzyme is typically used for preparing a mutated enzyme. A genetically engineering preparation method using a gene encoding a mutated enzyme makes it possible to obtain a mutated enzyme in a more homogeneous state. Furthermore, the method is a suitable for preparing a large amount of mutated enzymes. Note here that the application of the gene encoding a mutated enzyme is not limited to preparation of a mutated enzyme. For example, the nucleic acid can be used as an research tool for the purpose of elucidating the mechanism of action of a mutated enzyme, or as a tool for designing or preparing a further mutant of an enzyme.

In the present invention, the "gene encoding a mutated enzyme" refers to nucleic acid capable of obtaining the mutated enzyme when it is expressed, and includes not only a nucleic acid having a base sequence corresponding to an amino acid sequence of the mutated enzyme but also a nucleic acid obtained by adding a sequence that does not encode the amino acid sequence to the nucleic acid. Furthermore, degeneracy of codon is also considered.

The nucleic acid of the present invention can be prepared in an isolated state by standard genetic engineering technique, molecular biological technique, biochemical technique, and the like, with reference to sequence information disclosed in this specification or attached sequence list.

A yet further aspect of the present invention relates to recombinant DNA including a gene of the present invention (a gene encoding a mutated enzyme). The recombinant DNA is provided in a form of, for example, a vector. The term "vector" in this specification refers to a nucleic acid molecule that can transport a nucleic acid inserted therein to the inside of a target such as a cell.

In accordance with the purpose of use (cloning, protein expression), and by considering the kinds of host cells, an appropriate vector is selected. Specific examples of a vector include a vector using Escherichia coli as a host (M13 phage or the modified body thereof, λ phage or the modified body thereof, pBR322 or the modified body thereof (pB325, pAT153, pUC8, etc.) and the like), a vector using yeast as a host (pYepSec1, pMFa, pYES2, etc.), a vector using insect cells as a host (pAc, pVL, etc.), a vector using mammalian cells as a host (pCDM8, pMT2PC, etc.).

The vector of the present invention is preferably an expression vector. The term "expression vector" is a vector capable of introducing the nucleic acid inserted therein into the target cells (host cells) and expressing in the cells. The expression vector usually includes a promoter sequence necessary for expression of the inserted nucleic acid and an enhancer sequence for promoting the expression, and the like. An expression vector including a selection marker can be used. When such an expression vector is used, by using the selection marker, the presence or absence of the introduction of an expression vector (and the degree thereof) can be confirmed.

Insertion of the nucleic acid of the present invention into a vector, insertion of the selection marker gene (if necessary), and insertion of a promoter (if necessary), and the like, can be carried out by a standard recombination DNA technology (see, for example, Molecular Cloning, Third Edition, 1.84, Cold Spring Harbor Laboratory Press, New York, a well-known method using restriction enzyme and DNA ligase).

As the host cell, from the viewpoint of ease in handling, it is preferable that a bacterial cell such as Escherichia coli is used. However, the host cell is not limited to the bacterial cell as long as it can reproduce the recombinant DNA and express a gene of the mutated enzyme. As a preferable example of the host includes T7 system promoter, Escherichia coli BL21 (DE3) pLysS can be used. In other case, Escherichia coli JM109 can be used.

A further aspect of the present invention relates to a microorganism (that is, transformant) carrying a recombinant DNA of the present invention. The microorganism of the present invention can be obtained by the transfection or transformation using the above-mentioned vector of the present invention. For example, a calcium chloride method (J. Mol. Biol., Vol. 53, page 159 (1970)), a Hanahan method (J. Mol. Biol., Vol. 166, page 557 (1983)), a SEM method (Gene, Vol. 96, page 23 (1990)), a method by Chung et al. (Proceeding of the national Academy of Sciences of the USA, Vol.86, page 2172 (1989)), a calcium phosphate coprecipitation method, electroporation (Potter, H. et al., Proc. Natl. Acad. Sci. U.S.A. 81, 7161-7165 (1984)), lipofection (Felgner, P.L. et al., Proc. Natl. Acad. Sci. U.S.A. 84,7413-7417 (1984)), and the like.

A microorganism of the present invention can be used for producing a mutated enzyme of the present invention. That is to say, a further aspect of the present invention provides a method for producing a mutated enzyme of the present invention by using the above-mentioned microorganism. The production method of the present invention includes at least a step of culturing the above-mentioned microorganism under the conditions in which the mutated enzyme of the present invention is produced. In general, in addition to this step, a step of collecting (separating and purifying) the produced protein is carried out.

The microorganism (transformant) in accordance with the present invention is cultured in a usual manner. As a carbon source to be used for a medium, a carbon compound that can be assimilated may be used. An example may include glucose, sucrose, lactose, maltose, syrup, pyruvic acid, and the like. Furthermore, as a nitrogen source, any available nitrogen compounds may be used. An example may include peptone, meat extract, yeast extract, casein hydrolysate, soybean cake alkali extract, and the like. Besides, salts of phosphate, carbonate, sulfate, magnesium, calcium, potassium, iron, manganese, zinc, and the like, certain amino acids, certain vitamins, can be used if necessary.

On the other hand, the culture temperature can be set 30 to 40°C (preferably about 37°C). Culture time can be set considering the growing property of transformant to be cultured or production property of mutated enzyme, and the like. The pH of the medium is adjusted in a range in which the transformant is grown and an enzyme is produced. Preferably, pH of the medium is adjusted to about 6.0 to 9.0 (preferably, around pH 7.0).

A culture solution containing a bacterial body producing a mutated enzyme can be used as an enzyme solution as it is or after concentration or removing of impurities are carried out. Generally, however, a mutated enzyme is once collected from a culture solution or a bacterial body. When the produced mutated enzyme is secreting type protein, the mutated enzyme can be collected from the culture solution, and in other case, the mutated enzyme can be collected from the bacterial body. When the mutated enzyme is collected from the culture solution, purified products of the mutated enzyme can be obtained by, for example, subjecting the culture supernatant to filtering or centrifugation so as to remove insoluble matters, followed by carrying out separation and purification by the combination of vacuum concentration, membrane concentration, salting out using ammonium sulfate and sodium sulfate, fractional precipitation by methanol, ethanol or acetone, dialysis, heat treatment, isoelectric point treatment, various chromatography such as gel filtration chromatography, adsorption chromatography, ion exchange chromatography and affinity chromatography (for example, gel filtration by Sephadex gel (Pharmacia Biotech) and the like, DEAE sepharose CL-6B (Pharmacia Biotech), octyl sepharose CL-6B (Pharmacia Biotech), CM sepharose CL-6B (Pharmacia Biotech)), and the like. On the other hand, when the mutated enzyme is collected from a bacterial body, purified products of the mutated enzyme can be obtained by subjecting a culture solution to filtration and centrifugation to collect a bacterial body, followed by destructing the bacterial body by mechanical method such as pressure treatment and ultrasonication, or by an enzymatic method using lysozyme, and then carrying out separation and purification as mentioned above.

Purified enzyme obtained as mentioned above can be provided in a state of powder by, for example, freeze drying, vacuum drying or spray drying. At this time, a purified enzyme may be solved in phosphate buffer, triethanolamine buffer, Tris hydrochloric acid buffer, GOOD buffer in advance. Preferably, phosphate buffer and triethanolamine buffer can be used. An example of the GOOD buffer may include PIPES, MES or MOPS.

Hereinafter, the present invention is described further specifically. However, the present invention is not limited to these Examples.

### [Example]

### 1. Preparation of Bacillus subtilis recombinant pullulanase

### (1) Cloning of Bacillus subtilis pullulanase gene

A gene *AmyX* (GenBank Accesion No. NC 000964) encoding pullulanase, which is found in the genome sequence of Bacillus subtilus, was amplified by PCR as follows. A chromosome DNA of Bacillus subtilis strain 168 isolated by the method by Sambrook et al. (Molecular Cloning: a laboratory manual, 2nd Edition, Cold Spring harbor Laboratory Press, 1989) was used as a template of PCR, and oligonucleotides of SEQ ID NO: 3 and SEQ ID NO: 4 were synthesized to form a primer. In the PCR reaction, 30 cycles of reactions of 94°C / 2 minutes, 94°C / 15 seconds - 60°C / 30 seconds and amplification of 68°C / 4 minutes were carried out by using KOD plus system (TOYOBO). The obtained PCR fragment was treated with restriction enzymes *NcoI* and *XhoI,* and then linked to plasmid pET21d (Novagen), which had been cleaved with the both restriction enzymes. Thus, an expression plasmid pEBSP was obtained. It was confirmed by determining the base sequence that the obtained PCR fragment encoded pullulanase correctly.

SEQ ID NO: 3
   5'-GGCCATGGTCAGCATCCGCCGCAGCTTCGA-3'
   (underlined part represents a restriction enzyme *NcoI* recognition site)
SEQ ID NO: 4
   5'-GGCTCGAGTCAAGCAAAACTCTTAAGATCT-3'
   (underlined part represents a restriction enzyme *XhoI* recognition site)

### (2) Expression of Bacillus subtilis recombinant pullulanase

The above-mentioned expression plasmid was introduced into Escherichia coli HMS174 (DE3) (Novagen) by transformation. The obtained transformant was inoculated on LB medium (500 ml) containing 100 µ/ml ampicillin and the medium was shaken at 37°C. At the time when the turbidity at 600 nm reached 0.6 to 0.8, isopropylthiogalactoside (IPTG) was added so that the final concentration became 0.5 mM and further cultured at 18°C for 60 hours. The bacterial bodies were collected from the culture solution by centrifugation and suspended in a buffer solution (20 mM tris-hydrochloric acid (pH 8.1) / 5 mM EDTA / 20 mM β-mercaptoethanol / 0.2 mM PMSF).

### (3) Purification of Bacillus subtilis recombinant pullulanase

The suspension obtained in (2) was subjected to ultrasonication (4°C, 30 minutes, 200 µA) and then subjected to centrifugation (14000 rpm, 4°C, 30 minutes) to obtain a crude solution. To this solution, 30% saturated ammonium sulfate was added. Then, impurities were removed by centrifugation. To this centrifuged supernatant, 60% saturated ammonium sulfate was added. Then, precipitates were collected by centrifugation. The precipitates were dissolved in the buffer solution of (2) containing 20% saturated ammonium sulfate, and subjected to Butyl-Toyopearl 650M column (TOSOH CORPORATION) that had been equilibrated by the buffer solution containing 20% saturated ammonium sulfate, and eluted at the concentration gradient of 20% to 0% of ammonium sulfate. The obtained pullulanase active fraction was dialyzed to buffer solution of (2), and subjected to HiLoad Q Sepharose Column (Amersham) that had been equilibrated by the same buffer solution, and eluted at the concentration gradient of 0 to 0.5 M of NaCl to obtain active fraction. This fraction was dialyzed to the buffer solution of (2), and subjected to Mono Q Column (Amersham) that had been equilibrated by the same buffer solution, and eluted at the concentration gradient of 0 to 0.5 M of NaCl to obtain purified Bacillus subtilis recombinant pullulanase. The analysis result of N-terminal amino acid sequence of this purified enzyme was MVSIRRSFEA and completely identity to gene sequence.

### 2. X-ray analysis of Bacillus subtilis pullulanase

### (1) Crystallization

Crystallization of Bacillus subtilis recombinant pullulanase was carried out by the following procedure. Firstly, screening was carried out by a sitting drop vapor diffusion method by using a 96-well plate (product by Emerald Biostructure and Hampton Research). After 24 hours at 20°C, small and thin crystal was observed in Wizard II No.8 well. Next, by changing the molecular weight and concentration of polyethylene glycol (PEG) or the concentration of buffer solution or salts, conditions were optimized. Finally, a crystal was obtained by a hanging drop vapor diffusion method by using a 24-well plate. The hanging drop (6 µl) consisting of 3µl of enzyme solution (10 mg/ml) and 3 µl of reservoir solution (10% PEG4000, 0.1 M acetate buffer, pH 5.2, 0.2 M Mg CH₃COO)₂) was used and incubated for one to two days. Thus, diamond shaped crystal was obtained. Prior to the X-ray analysis, treatment with a reservoir solution containing 30% glycerol was carried out and then cooled instantly by using liquid nitrogen (-173°C).

### (2) X-ray analysis

X-ray diffraction data were collected at a temperature of liquid nitrogen by using Synchrotron radiation BL-38B1 (SPring-8, Hyogo, Japan) and processed by using HKL2000 program. X-ray diffraction data of 2.1 Ǻ resolution were collected so as to determine the crystallographic parameter. Space group was P2₁2₁2₁ and lattice constant was a = 70.57 Ǻ, b = 127.68 Ǻ, and c = 189.25 Ǻ.

### (3) Determination of three-dimensional structure

A three-dimensional structure was determined at the resolution of 2.1 Ǻ by a molecule substitution method using an atomic coordinate (PDB accession code 2FGZ) of pullulanase of Klebsiella pneumonia. The substitution of molecules and the refinement of structure were carried out by using program CNS_SOLUE ver.11. Data related to statistics of data collection and refinement are shown in Table 1.

**[Table 1]**

| Crystal | Bacillus subtilis pullulanase |
|---|---|
| Data collection | Spring-8 BL38B1 |
| wavelength (Ǻ) | 0.8 |
| detector | Jupiter 210 CCD |
| space group | *P*2₁2₁2₁ |
| lattice constant (Ǻ) | A=70.57, b=127.68, c=189.25 |
| resolution (Ǻ) | 46.3 - 2.1(2.18 - 2.10) |
| measured reflection | 590,744(38,618) |
| unique reflection | 99,003 (9,775) |
| integrity (%) | 99.6(99.6) |
| *R*merge (%) | 7.8 (31.4) |
| determination of structure | molecule substitution method |
| Refinement | |
| Residues/Water/Ca2+/α-CD | 712 x 2, 673, 2, 0 |
| Resolution (Ǻ) | 15.0 - 2.1 (2.17 - 2.1) |
| used reflection | 98,636 (8,725) |
| r.m.s. bond (Ǻ), angle (°) | 0.005, 1.25 |
| R and Rfree | 0.201, 0.238 (0.224, 0.252) |

### 3. X-ray analysis of Bacillus subtilis pullulanase (in a state of containing α-cyclodextrin (CD))

Since α-cyclodextrin (CD) is cyclic oligosaccharide and is an analog having a structure of amylose helix consisting of six glucose residues, it has often been used for studying as an inhibitor of an amylolytic enzyme such as amylase and pullulanase. In order to determine the substrate binding site of Bacillus subtilis pullulanase, an analysis of the three-dimensional structure of enzyme containing CD as a ligand (hereinafter, referred to as "CD-containing Bacillus subtilis pullulanase") was carried out. The analysis of the three-dimensional structure was carried out basically by the same method as 2. except that purified Bacillus subtilis recombinant pullulanase was prepared in 1. and 20 mM CD was added in the hanging drop at the time of crystallization. Data related to statistics about data collection and data are shown in Table 2.

**[Table 2]**

| Crystal | Bacillus subtilis pullulanase containing α-cyclodextrin |
|---|---|
| Data collection | Spring-8 BL38B1 |
| wavelength (Ǻ) | 1.0 |
| detector | Jupiter 210 CCD |
| space group | *P*2₁2₁2₁ |
| lattice constant (Ǻ) | A=70.36, b=127.86, c=189.29 |
| resolution (Ǻ) | 40.0 - 2.2 (2.28 - 2.20) |
| measured reflection | 4.08,687 (35,910) |
| unique reflection | 87,392 (8,550) |
| integrity (%) | 99.8(99.3) |
| *R*merge (%) | 9.2 (42.4) |
| determination of structure | molecule substitution method |
| Refinement | |
| Residues/Water/Ca2+/α-CD | 712 x 2, 673, 2, 2 |
| Resolution (Ǻ) | 15.0 - 2.2 (2.28 -2.2) |
| used reflection | 87,004 (8,495) |
| r.m.s. bond (Ǻ), angle (°) | 0.005, 1.25 |
| R and Rfree | 0.197, 0.238 (0.249, 0.282) |

A model of the three-dimensional structure of the obtained CD-containing Bacillus subtilis pullulanase is shown in Fig. 1. Note here that data of atomic coordinate are shown in the last part of the specification.

### 4. Comparison analysis between three-dimensional structure of CD-containing Bacillus subtilis pullulanase and three-dimensional structure of Klebsiella pneumoniae pullulanase

The three-dimensional structure of CD-containing Bacillus subtilis pullulanase obtained in 3. and the three-dimensional structure of Klebsiella pneumoniae pullulanase (J. Mol. Biol., 359 (3): 690-707 (2006), RCSB Protein Data Bank code 2FHF) were superimposed onto each other by Least Square method by using program TURBO-FRODO, α carbons of amino acids of 499 residues were common within 2 Ǻ with the root means square deviation (r. m. s. d.) of 1.09 Ǻ. Fig. 2 shows the superimposed results. Fig. 3 is an enlarged view showing a CD binding site. Fig. 3 shows a CD binding site with respect to Bacillus subtilis pullulanase and the binding site of maltotetraose with respect to Klebsiella pneumoniae pullulanase (G4) (see, non-patent document 1). An amino acid residue (upper stage) in a position capable of forming CD and hydrogen binding in Bacillus subtilis pullulanase is shown together with the corresponding amino acid residue (lower stage) in Klebsiella pneumoniae pullulanase. Furthermore, amino acid residue (lower stage) that has been deduced to be involved in binding to G4 in Klebsiella pneumoniae pullulanase and the corresponding amino acid residue (upper stage) in Bacillus subtilis pullulanase are also shown together.

Although a domain (N1 domain) in the N terminal region observed in Klebsiella pneumoniae pullulanase was not observed in Bacillus subtilis pullulanase, both enzymes were very similar to each other in a catalytic region and a domain (A domain) as a nucleus including a substrate binding region. Furthermore, a CD binding site of Bacillus subtilis pullulanase is in the vicinity of G4 binding site of Klebsiella pneumonia.

As shown in Fig. 3, in Bacillus subtilis pullulanase, twelve in total of amino acid residues that are thought to be involved in binding to the substrate were successfully identified as an amino acid position in the binding site of CD: Y292 (tyrosine the 292 position), G371 (glycine at the 371 position), D406 (aspartic acid at the 406 position), L407 (leucine at the 407 position), W437 (tryptophane at the 437 position), D465 (aspartic acid at the 465 position), T475 (threonine at the 475 position), F476 (phenyl alanine at the 476 position), D525 (aspartic acid at the 525 position), N526 (asparagine at the 526 position), N580 (asparagine at the 580 position), and Y582 (tyrosine at the 582 position). Furthermore, in Klebsiella pneumoniae pullulanase, amino acid corresponding to these amino acid residues, that is, Y559 (tyrosine at the 559 position), C643 (cysteine at the 643 position), D677 (aspartic acid at the 677 position), L678 (leucine at the 678 position), W708 (tryptophane at the 708 position), D734 (aspartic acid at the 734 position), P745 (proline at the 745 position), F746 (phenyl alanine at the 746 position), D834 (aspartic acid at the 834 position), N835 (asparagine at the 835 position), D890 (aspartic acid at the 890 position), and Y892 (tyrosine at the 892 position) were successfully determined. Among the thus determined amino acids, D677, W708, D734, D834, N835 and D890 were amino acids deduced to be involved in binding to G4 in the above-mentioned report.

### 5. Comparison analysis between three-dimensional structure of CD-containing Bacillus subtilis pullulanase and three-dimensional structure of Pseudomonas amyloderamosa isoamylase

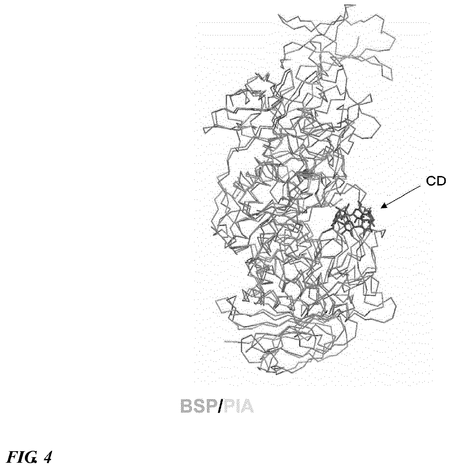

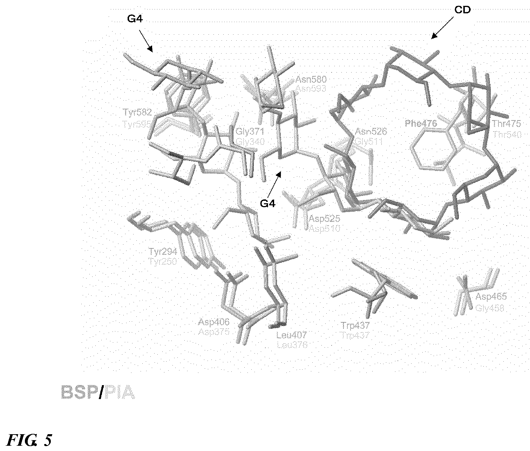

The three-dimensional structure of CD-containing Bacillus subtilis pullulanase obtained in 3. and the three-dimensional structure of Pseudomonas amyloderamosa isoamylase (J. Mol Biol., 281 (5): 885-97 (1998), RCSB Protein Data Bank code 1BF2) were superimposed onto each other by Least Square method by using program TURBO-FRODO, α carbons of amino acids of 421 residues were common within 2 Ǻ with the root means square deviation (r. m. s. d.) of 1.13 Ǻ. Fig. 4 shows the superimposed results. Fig. 5 is an enlarged view showing a CD binding site.

The both enzymes do not have a domain (N1 domain) in the N terminal region observed in Klebsiella pneumoniae pullulanase and is very similar in domain (A domain) in terms of, for example, a nucleus including a catalytic region or a substrate binding region. The amino acid residue (upper stage) in the position capable of forming hydrogen binding to CD in Bacillus subtilis pullulanase is shown together with the corresponding amino acid residue (lower stage) in Pseudomonas amyloderamosa isoamylase. Furthermore, amino acid residues corresponding to the amino acids deduced to be involved in binding to G4 in Klebsiella pneumoniae pullulanase (upper stage: Bacillus subtilis pullulanase, and lower stage: Pseudomonas amyloderamosa).

### 6. Formation of alignment of amino acid sequence based on three-dimensional structure

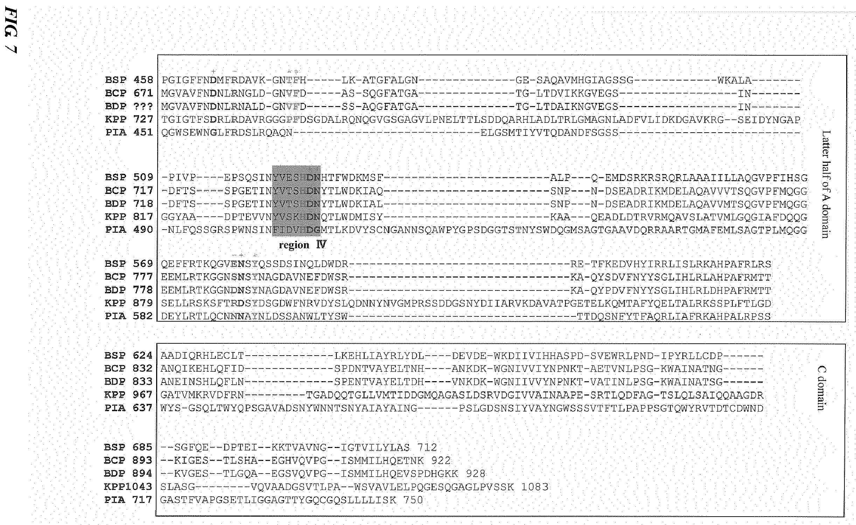

With the use of the result of comparison of the three-dimensional structures of CD-containing Bacillus subtilis pullulanase, Klebsiella pneumoniae pullulanase and Pseudomonas amyloderamosa isoamylase obtained in 4. and 5., alignments of amino acid sequences with respect to these three enzymes were formed based on the three-dimensional structures. Furthermore, alignments of amino acid sequences with respect to two kinds of pullulanases (pullulanase from Bacillus sp. APC-9603 and pullulanase from Bacillus deramificans) being derived from strains related to Bacillus subtilis and having a high homology in primary structure were formed by using ClustalW program. Alignments were formed with respect to five kinds of enzymes with these two alignments added (see Figs. 6 and 7). As shown in Figs. 6 and 7, these five kinds of enzymes have extremely high partial similarity.

### 7. Production of Bacillus subtilis recombinant pullulanase mutant

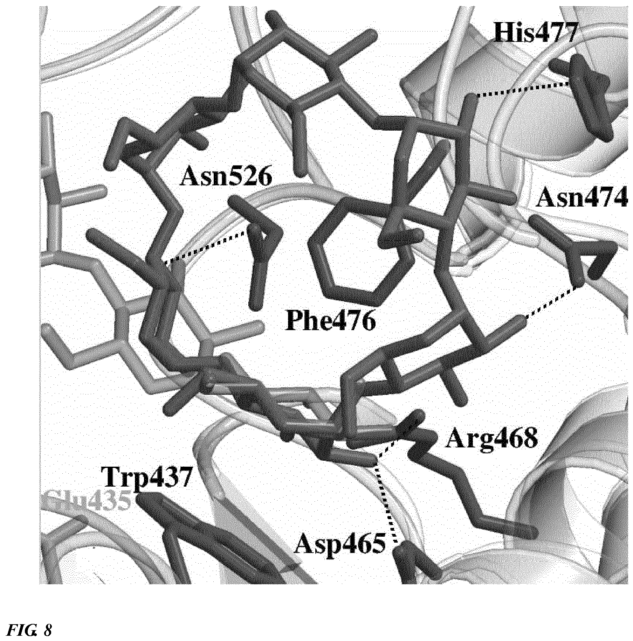

From the three-dimensional structure of Bacillus subtilis pullulanase to which CD obtained in 3. was bonded as a ligand, it is assumed that the side chain of Phe476 of Bacillus subtilis pullulanase is incorporated in the cyclic structure of the CD as a substrate analog (Fig. 8) and has something to do with the recognition of a substrate. Under this assumption, Phe476 was substituted with another amino acid by the following procedure and the effect thereof was verified.

Based on the sequence of gene *AmyX* encoding Bacillus subtilis pullulanase, a primer for substituting Phe476 with Gly or Ser was synthesized. In order to substitute Phe476 with Gly or Ser, forward primers and the corresponding reverse primers set forth in SEQ ID NOs: 5 and 6 were synthesized, respectively, and the concentration was adjusted to 100 ng/µl. By using an expression plasmid pEBSP of Bacillus subtilis recombinant pullulanase as a template, the following PCR reaction was carried out: 1.5 µl (30ng/µl) of pEBSP, 5 µl of 10 x PCR buffer solution (one attached to the below-mentioned DNA polymerase), 1.0 µl (2.5 mM each) of dNTP, 1.25 µl each of mutated primer sets (forward and reverse primers), 39 µl of sterile water and 1 µl (2.5 U) of Pfu Turbo Hotstart DNA polymerase (Stratagene) were prepared; 30 cycles of reactions at 95°C for 30 seconds (denaturation) - at 55°C for 60 seconds (annealing) - at 68°C for 12 minutes (elongation) were carried out; and finally amplification at 68°C for 5 minutes was carried out. The obtained PCR product was confirmed by 1% agarose gel electrophoresis, and then the rest of the PCR product was treated with restriction enzyme Dpn I to degrade a methylated template plasmid and transformed to Escherichia coli competent cell DH5α strain. Plasmid DNA was isolated from the obtained ampicillin resistance transformant, the base sequence was confirmed and the intended mutated gene was obtained. A plasmid having the intended mutated gene was introduced into an expression host Escherichia coli HMS174 (DE3) strain to express and purify the mutated pullulanase of Bacillus subtilis similar to 1. (2) and (3).

SEQ ID NO: 5
   5'-GTAAAAGGGAACACCGGTCACCTTAAGGCAATA-3'
SEQ ID NO: 6
   5'-GTAAAAGGGAACACCTCTCACCTTAAGGCAATA-3'

### 8. Substrate specificity of Bacillus subtilis recombinant pullulanase mutant

The kinetic parameters with respect to pullulan and amylopectin of Bacillus subtilis recombinant pullulanase mutant prepared in 4. were measure according to the method described in J Biochem (Tokyo), 116(6): 1264-8 (1994). Pullulan or amylopectin having various concentrations were dissolved in 50 mM acetate buffer (pH 5.6) and reacted at 25°C. The concentration of a reducing sugar contained in the regularly sampled reaction solution was determined by a Park-Johnson method, and the reaction rate was measured from the increasing rate of the reducing sugar. Km value and Kcat value were obtained by curve fitting into Michaelis-Menten equation by the non-linear minimum square method. Results are shown in Table 3.

**[Table 3]**

| | pullulan | | amylopectin | |
|---|---|---|---|---|
| | *K*m (mg/ml) | *k* cat (s⁻¹) | *K*m (mg/ml) | *k* cat (s⁻¹) |
| wild type pullulanase | 14.93 | 4317 | 199.20 | 4121 |
| mutant type (F476G) | 20.45 | 5362 | 53.19 | 1198 |
| mutant type (F476S) | 645.16 | 10365 | 156.25 | 2146 |

When the wild type and the mutant types are compared with each other, they are shown to be different in the Km value and Kcat value. For example, in the mutant type (F476G: mutated enzyme in which an amino acid residue at the 476 position is changed from phenyl alanine to glycine), the Km value when amylopectin is used as a substrate is considerably lower than that of the wild type, which shows that the affinity to amylopectin is radically improved. Furthermore, in the mutant type (F476S: mutated enzyme in which an amino acid residue at the 476 position is changed from phenyl alanine to serine), the Km value when amylopectin is used as a substrate is lower than that of the wild type while the Km value when pullulan is used as a substrate is radically higher than that of the wild type, which shows that the substrate specificity has radically changed. Thus, introduction of mutation enabled the action property with respect to pullulan and amylopectin to be changed.

### 9. Cloning of Klebsiella pneumoniae pullulanase gene

### (1) Cloning of Klebsiella pneumoniae pullulanase gene

Two primers (SEQ ID NOs: 7 and 8) were synthesized with reference to the base sequence of pullulanase of Klebsiella aerogence W70 strain (J. Bacteriol. 169 (5), 2301-2306 (1987) and J Bacteriol. 174(9): 3095.(1992), GenBank accession No. M16187). The PCR reaction was carried out under the following conditions by using chromosome DNA of Klebsiella pneumoniae ATCC9621 strain that had been isolated by the method by Sambrook et al. (Molecular Cloning: a laboratory manual, 2nd Edition, Cold Spring harbor Laboratory Press, 1989) as a template. The PCR reaction was carried out by using Accu TaqTM LA DNA polymerase system (Sigma), the reaction included one cycle of reaction at 98°C for 30 seconds - at 59°C for 20 seconds - at 68°C for 3 minutes; 29 cycles of reaction at 98°C for 15 seconds - at 59°C for 20 seconds - at 68°C for 3 minutes; and reaction at 68°C for 10 minutes of amplification was carried out. The obtained PCR fragment was subjected to TA cloning by using a pGEM-T vector (Promega). It was confirmed that the obtained PCR fragment encodes pullulanase correctly by determining the base sequence. The base sequence of the obtained PCR fragment and the amino acid sequence encoded thereby are shown in SEQ ID NO: 12 and SEQ ID NO: 13, respectively.

SEQ ID NO: 7
   5'-TTATTGCCGGAGAGTGGCGA-3'
SEQ ID NO: 8
   5'-CCAGACTGCTGACAAAGTGC-3'

### (2) Expression of Klebsiella pneumoniae pullulanase gene

In order to construct an expression vector, primers shown in SEQ ID NOs: 9 and 10 were synthesized, and PCR reaction was carried out by using a pullulanase gene of Klebsiella pneumoniae as a template. The obtained PCR product was treated with restriction enzymes *Nde* I and *Xba*I, and linked to a plasmid pCold-I (TAKARA) to obtain an expression plasmid pCold-KPP of Klebsiella pneumoniae pullulanase. The plasmid was introduced into Escherichia coli JM109 (TAKARA) by transformation. Culture of the obtained transformant and purification of the expressed recombinant pullulanase were carried out according to the method described in 1.

SEQ ID NO: 9
   5' - GGAATTCCATATGGATGTCGTCGTCCGCTTACCG -3' (34mer)
SEQ ID NO:10
   5' - GCTCTAGATTATTTACTGCTCACCGGCAG - 3' (29mer)

### 10. Production of Klebsiella pneumoniae pullulanase mutant

The results obtained by analysis by superimposing the three-dimensional structure of CD-containing Bacillus subtilis pullulanase obtained in 3. and the three-dimensional structure of Klebsiella pneumoniae pullulanase (RCSB Protein Data Bank code 2FHF) onto each other (Figs. 2 and 3) and the results of amino acid sequence alignments (Figs. 6 and 7), an amino acid residue of pullulanase of Klebsiella pneumonia corresponding to Phe 476 residue that was thought to be involved in recognition of a substrate in Bacillus subtilis pullulanase was thought to be a Phe746 residue. Then, the Phe746 residue of pullulanase of Klebsiella pneumonia was substituted with Ala (or other amino acid).

Forward primers and the corresponding reverse primers set forth in SEQ ID NO: 11 were synthesized respectively in order to replace Phe746 with Ala in Klebsiella pneumoniae, and the concentration was adjusted to 100 ng/µl. By using an expression plasmid pCold-KPP of Klebsiella pneumoniae pullulanase as a template, the following PCR reaction was carried out: 1.5 µl (30 ng/µl) of pEBSP, 5 µl of 10 x PCR buffer solution (one attached to the below-mentioned DNA polymerase), 1.0 µl (2.5 mM each) of dNTP, 1.25 µl each of mutated primer sets (forward and reverse primers), 39 µl of sterile water and 1 µl (2.5 U) of Pfu Turbo Hotstart DNA polymerase (Stratagene) were prepared; 30 cycles of reactions at 95°C for 30 seconds (denaturation) - at 55°C for 60 seconds (annealing) - at 68°C for 12 minutes (elongation) were carried out; and finally amplification at 68°C for 5 minutes was carried out. The obtained PCR product was confirmed by 1% agarose gel electrophoresis, and then the rest of the PCR product was treated with restriction enzyme *Dpn I* to degrade a methylated template plasmid and transformed to Escherichia coli competent cell DH5α strain. Plasmid DNA was isolated from the obtained ampicillin resistance transformant, the base sequence was confirmed and the intended mutated gene was obtained. A plasmid having the intended mutated gene was introduced into an expression host Escherichia coli HMS174 (DE3) strain to express and purify the mutated pullulanase of Klebsiella pneumoniae similar to 1. (2) and (3).

SEQ ID NO: 11
   5'- GCCGGCCGACTCCGGTGAC-3'

### 11. Substrate specificity of Klebsiella pneumoniae pullulanase mutant

The kinetic parameters with respect to pullulan and amylopectin of mutated pullulanase (F746A) and wild type pullulanase (WT) of Klebsiella pneumoniae prepared in 10. were measure according to the method described in J Biochem (Tokyo), 116(6): 1264-8 (1994). Pullulan or amylopectin having various concentrations were dissolved in 50 mM acetate buffer (pH 5.6) and reacted at 25°C. The concentration of a reducing sugar contained in the regularly sampled reaction solution was determined by a Park-Johnson method, and the reaction rate was measured from the increasing rate of the reducing sugar. Km value and Kcat value were obtained by curve fitting into Michaelis-Menten equation by the non-linear minimum square method. Results are shown in Table 4.

**[Table 4]**

| | pullulan | | amylopectin | |
|---|---|---|---|---|
| | *K*m (%) | *K*cat (s⁻¹) | *K*m (%) | Kcat (s⁻¹) |
| wild type pullulanase | 0.00091 | 52.0 | 0.0063 | 7.0 |
| mutant type (F746A) | 0.00045 | 18.2 | 0.0083 | 5.2 |

Km value when pullulan was used for a substrate was lowered in the case of mutated enzyme as compared with the case of the wild type enzyme. On the other hand, Km value when amylopectin was used for a substrate was increased. From the results, it can be evaluated that in the mutated enzyme, the affinity to pullulan is improved (the affinity to amylopectin is lowered). Furthermore, Kcat value is different between the wild type and the mutant type. Thus, introduction of mutation enabled the action property with respect to pullulan and amylopectin to be changed.

Note here that the enzyme in which the affinity to a certain substrate is improved can act on a smaller amount of substrate. Such a property is advantageous in that the presence of the small amount of substrates is required to be measured.

### [Industrial Applicability]

A designing method and a preparation method as described above are used for improving an enzyme hydrolyzing an α-1,6-glycosidic linkage. With a mutated enzyme of the present invention whose affinity and specificity to a substrate are improved, the increase in the yield of products and reduction of the amount of enzyme to be used (additive amount) can be achieved. On the other hand, it can be expected that the use of the designing method and the preparation method as described above provide a mutated enzyme of the present invention usable to a novel application that has not been assumed with the use of wild type enzymes. That is to say, the present invention is capable of contributing to expansion of the applications of use of enzymes hydrolyzing an α-1,6-glycosidic linkage.

The atomic coordinates of three-dimensional structure of CD-containing Bacillus subtilis (Bacillus subtilis strain 168) are shown below.
REMARK coordinates from restrained individual B-factor refinement
REMARK refinement resolution: 15.0 - 2.2 A
REMARK starting r= 0.1970 free_r= 0.2378
REMARK final r= 0.1966 free_r= 0.2377
REMARK B rmsd for bonded mainchain atoms= 1.246 target= 1.5
REMARK B rmsd for bonded sidechain atoms= 1.964 target= 2.0
REMARK B rmsd for angle mainchain atoms= 1.982 target= 2.0
REMARK B rmsd for angle sidechain atoms= 2.784 target= 2.5
REMARK rweight= 0.1000 (with wa= 2.63874)
REMARK target= mlf steps= 25
REMARK sg= P2(1)2(1)2(1) a= 70.327 b= 127.780 c= 189.274 alpha= 90 beta= 90 gamma= 90
REMARK parameter file 1 : CNS_TOPPAR:protein_rep.param
REMARK parameter file 2 : CNS_TOPPAR:carbohydrate.param
REMARK parameter file 3 : CNS_TOPPAR:ion.param
REMARK parameter file 4 : CNS_TOPPAR:water_rep.param
REMARK parameter file 5 : act.par
REMARK molecular structure file: generate.mtf
REMARK input coordinates: minimize2.pdb
REMARK reflection file= 050715bl38_bdpulcd.cv
REMARK ncs= none
REMARK B-correction resolution: 6.0 - 2.2
REMARK initial B-factor correction applied to fobs :
REMARK B11= -4.731 B22= -0.623 B33= 5.354
REMARK B12= 0.000 B13= 0.000 B23= 0.000
REMARK B-factor correction applied to coordinate array B: -0.013
REMARK bulk solvent: density level= 0.380879 e/A^3, B-factor= 43.8838 A^2
REMARK reflections with |Fobs|/sigma_F < 0.0 rejected
REMARK reflections with |Fobs| > 10000 * rms(Fobs) rejected
REMARK theoretical total number of refl. in resol. range: 87004 (100.0 %)
REMARK number of unobserved reflections (no entry or |F|=0): 142 (0.2%)
REMARK number of reflections rejected: 0 (0.0%)
REMARK total number of reflections used: 86862 (99.8 %)
REMARK number of reflections in working set: 78156 (89.8 %)
REMARK number of reflections in test set: 8706 (10.0 %)
CRYST1 70.327 127.780 189.274 90.00 90.00 90.00 P 21 21 21
REMARK FILENAME="/draco/usr1/people/sad/xplor/bspul/060312_pulcd/bindividual2"
REMARK DATE:12-Mar-06 02:57:02 created by user: sad
REMARK VERSION:1.1

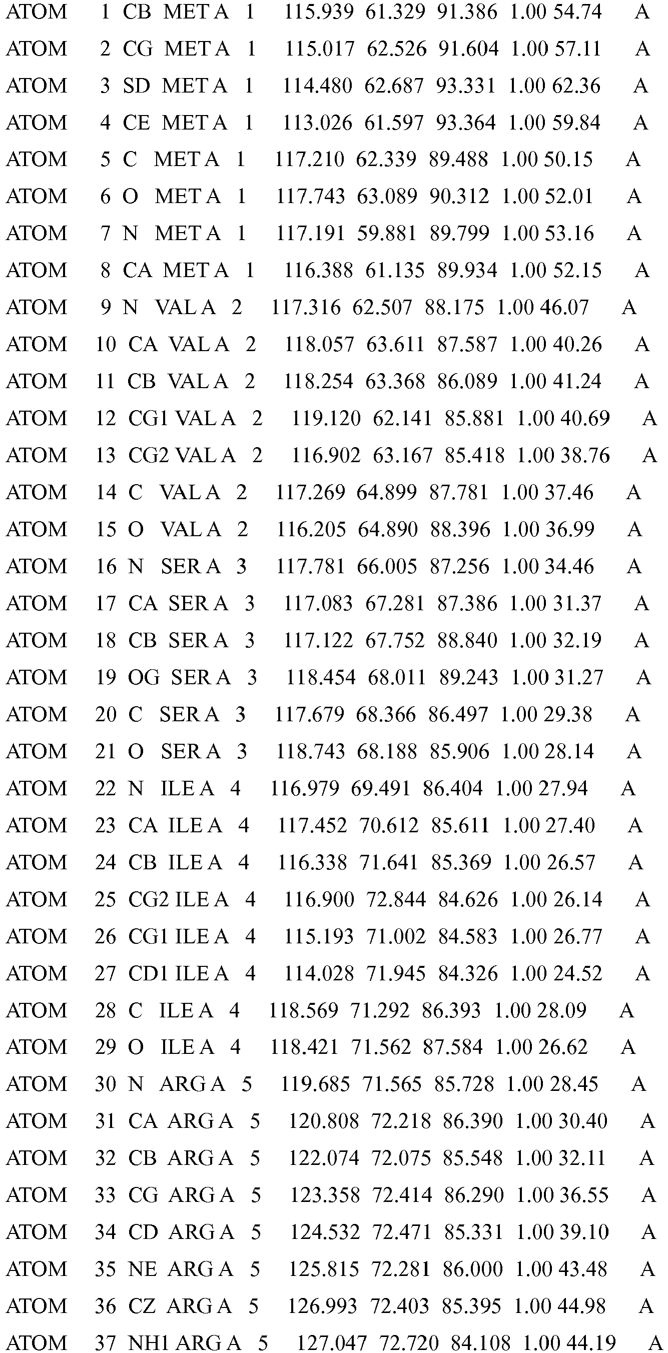

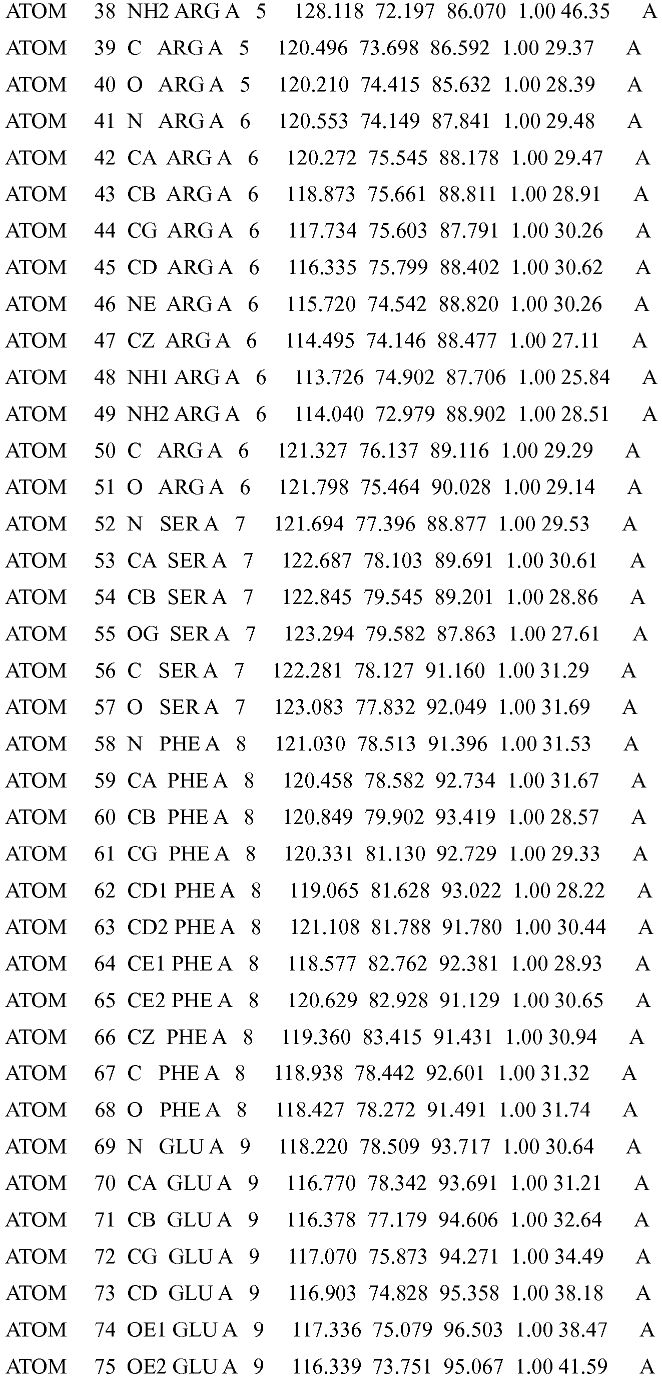

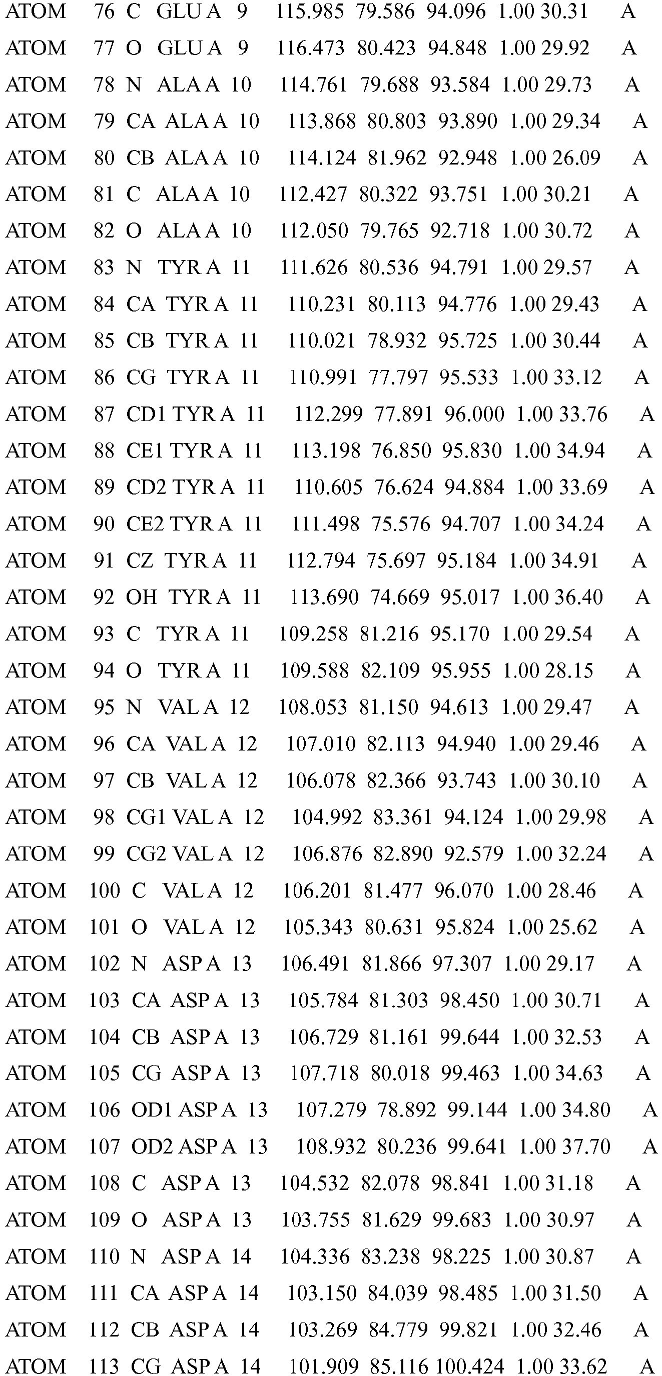

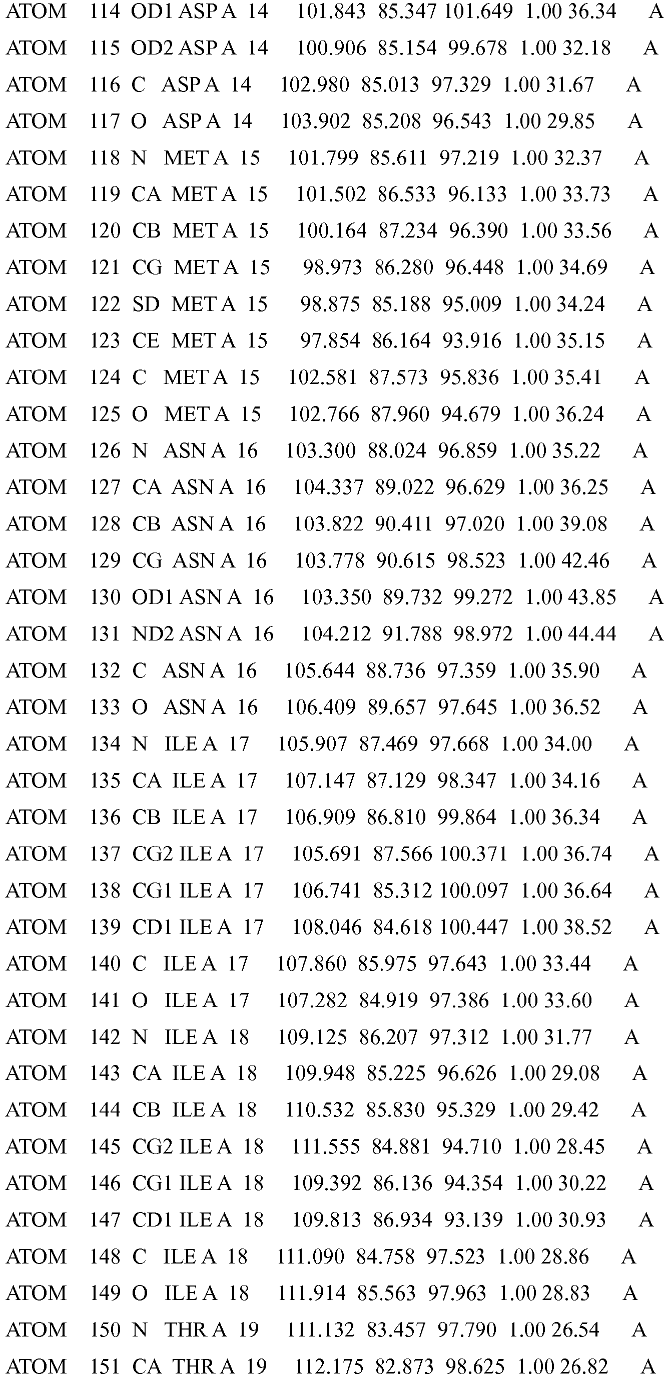

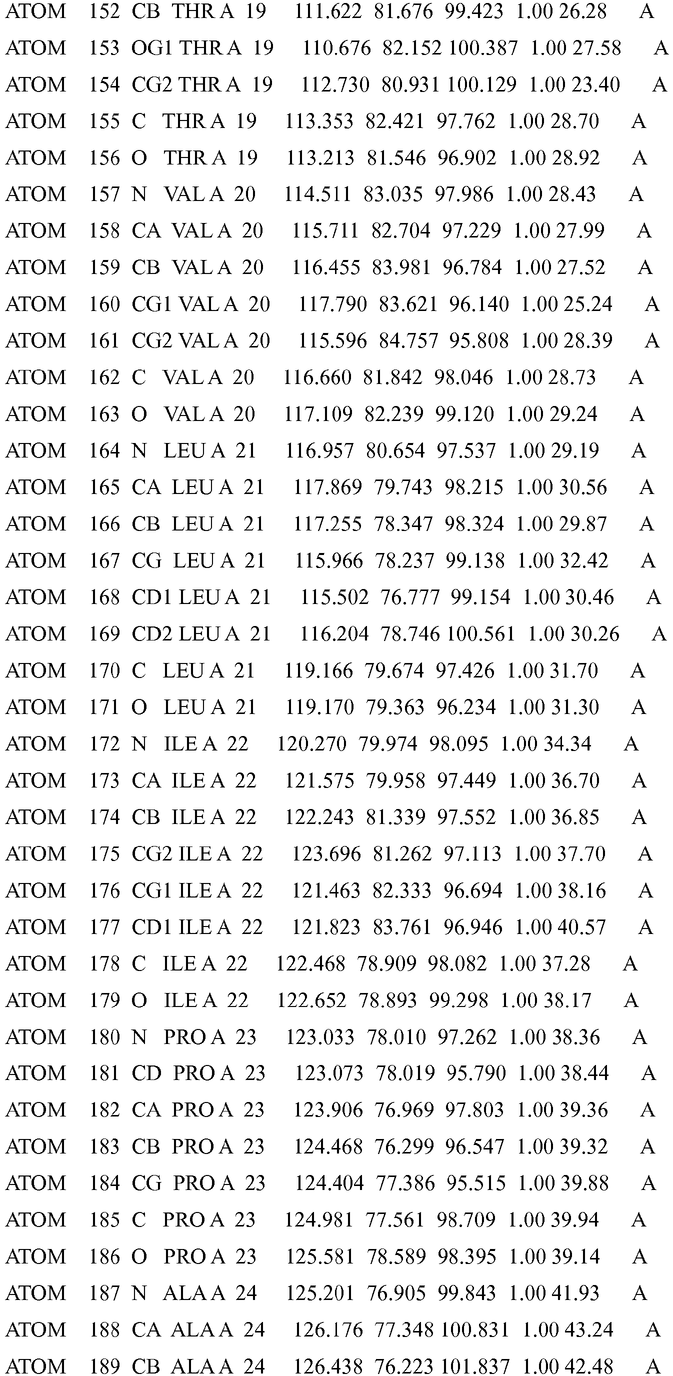

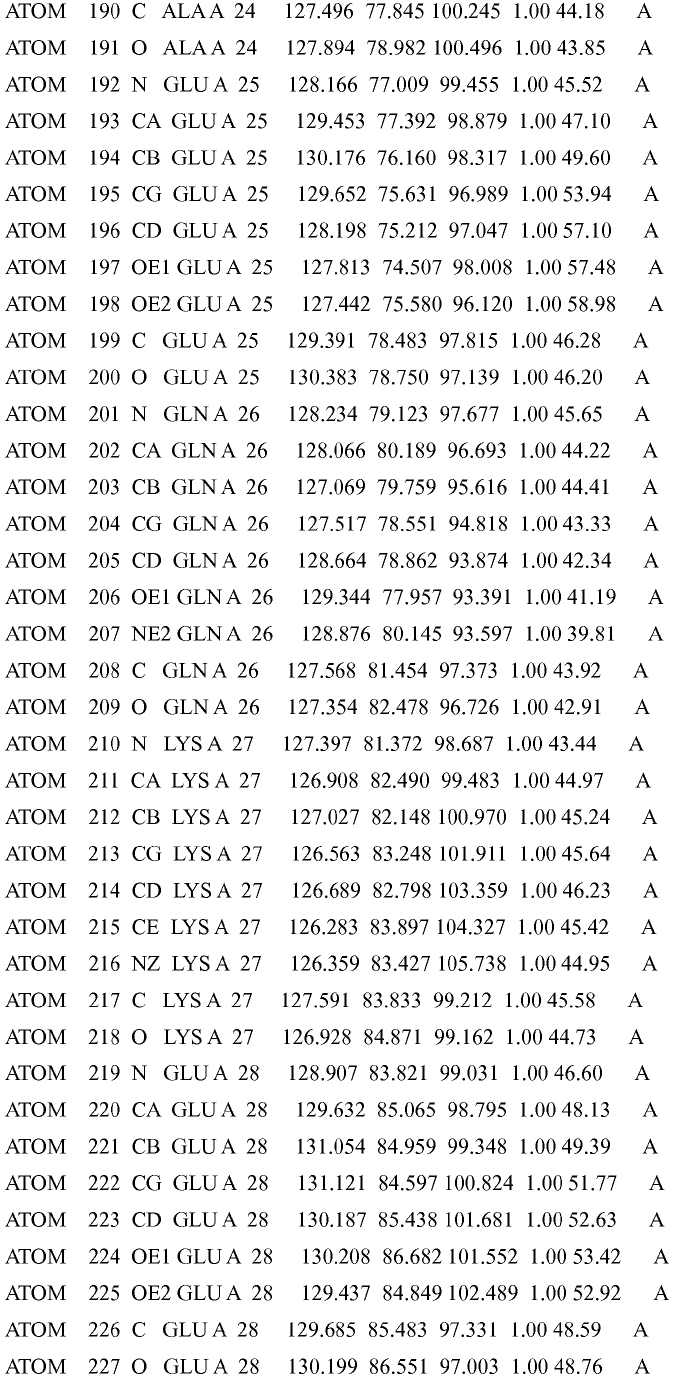

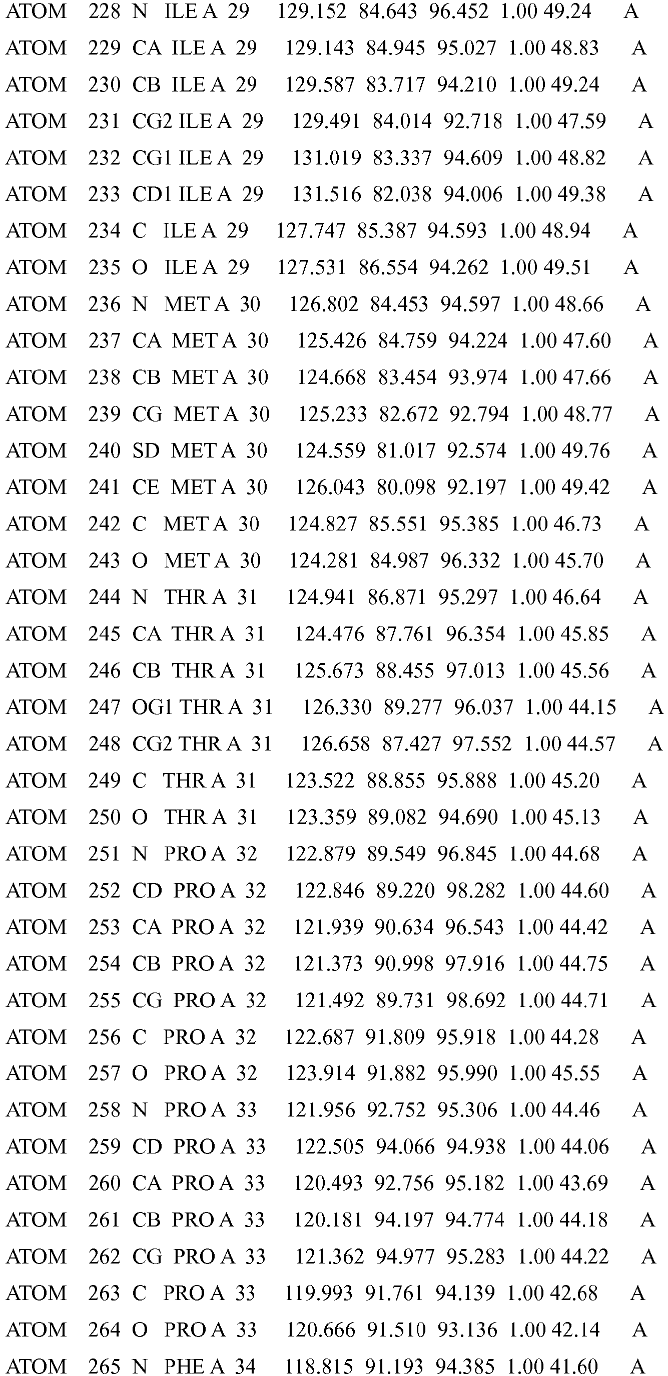

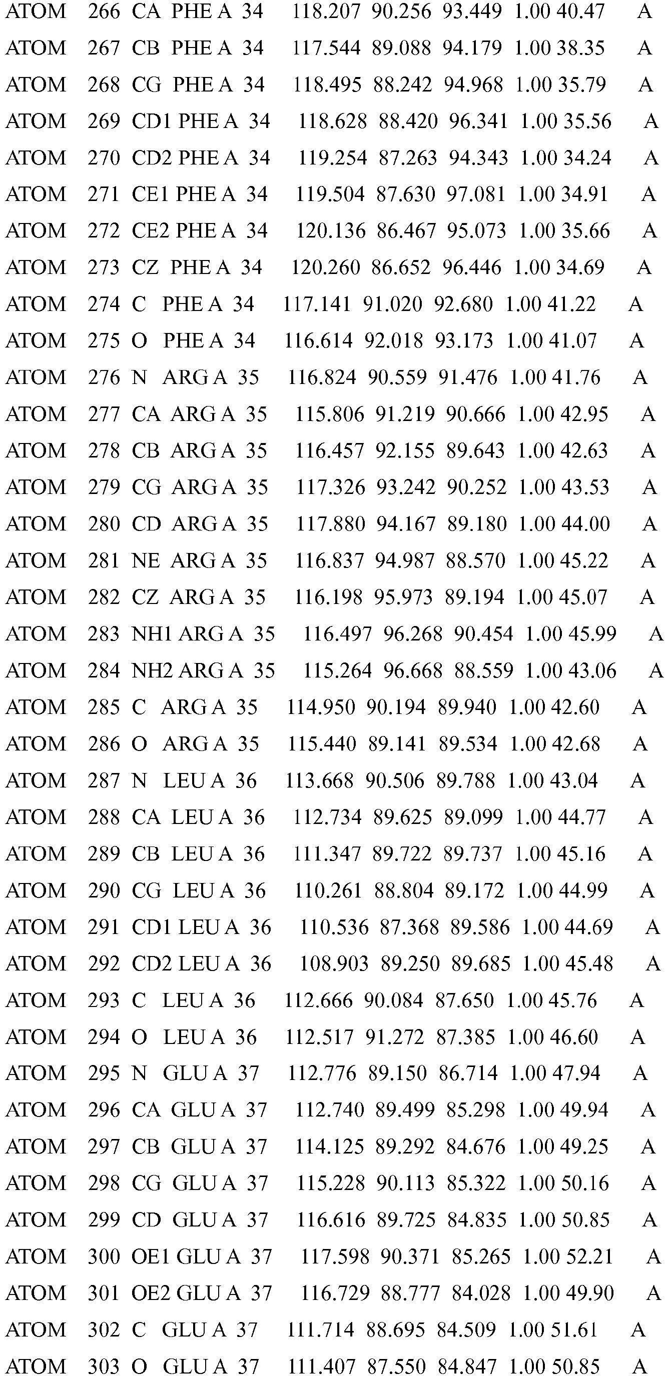

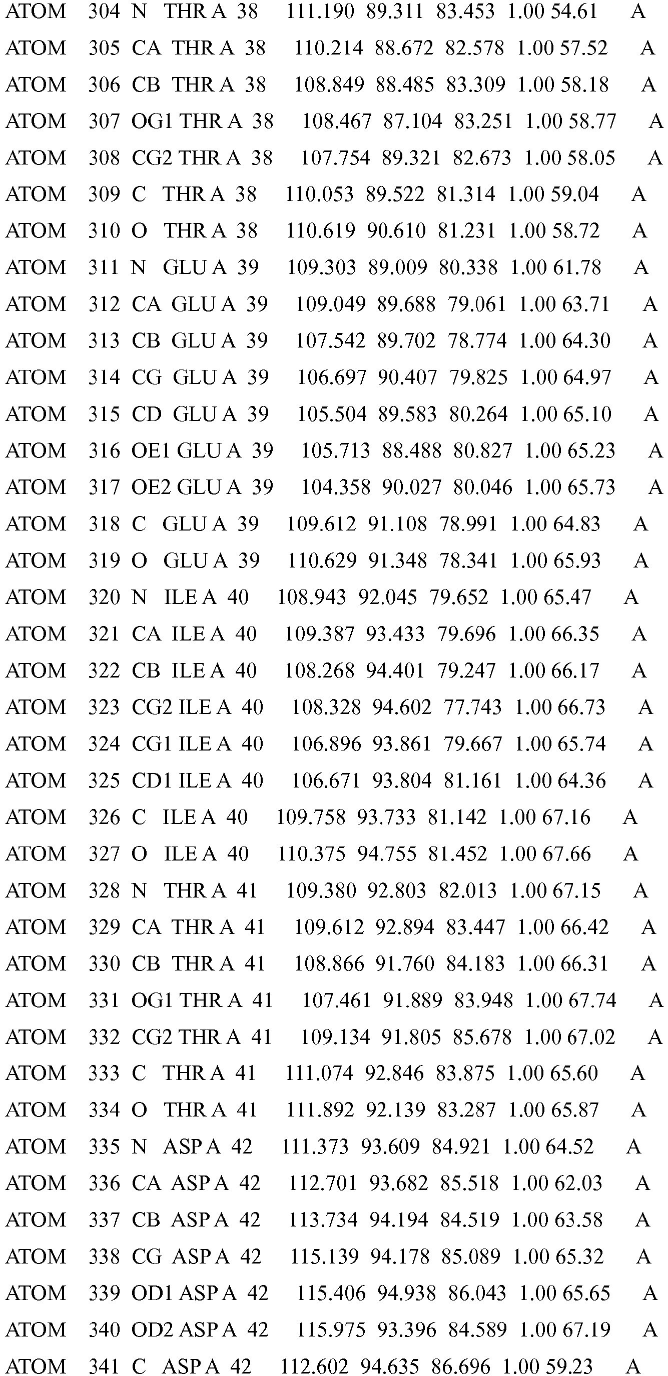

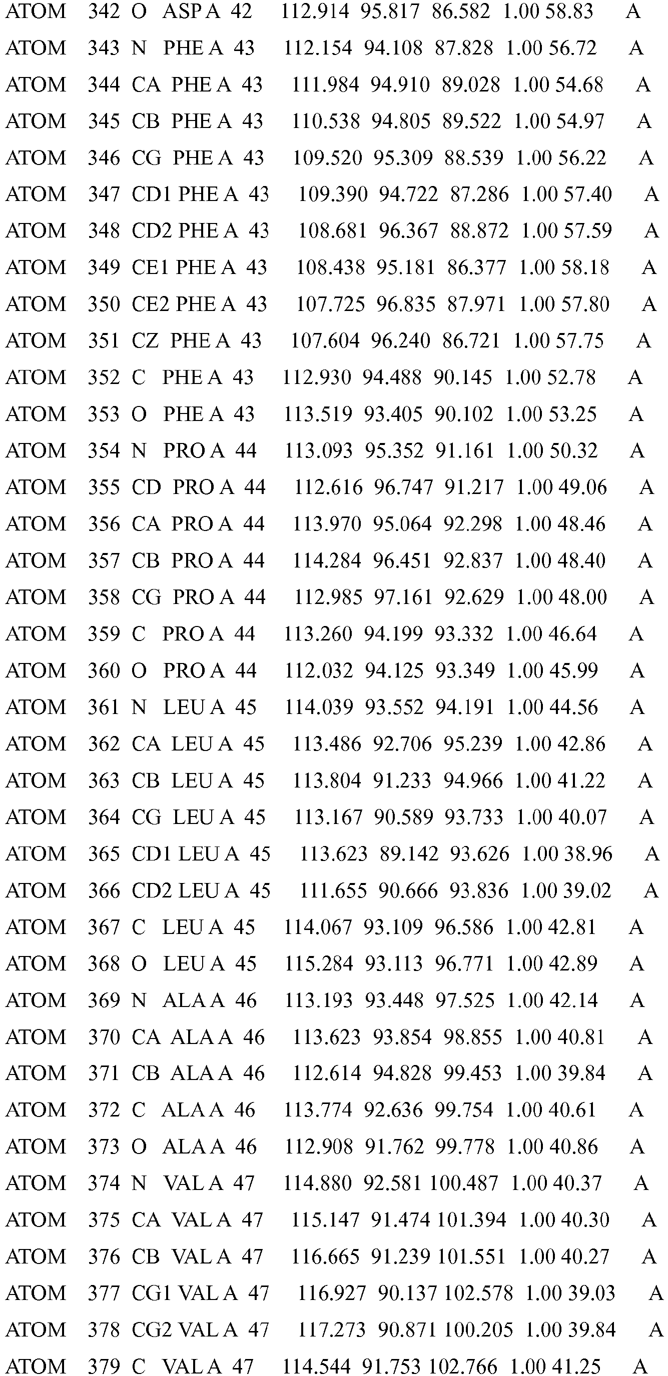

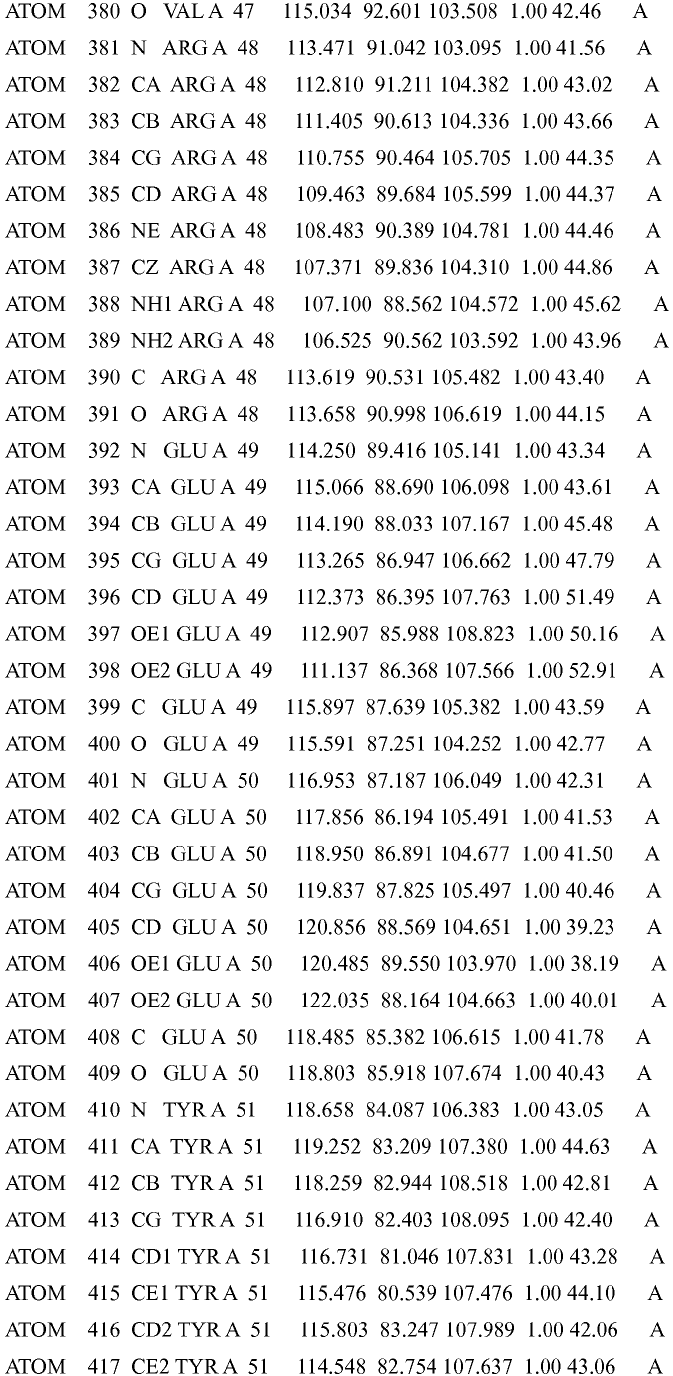

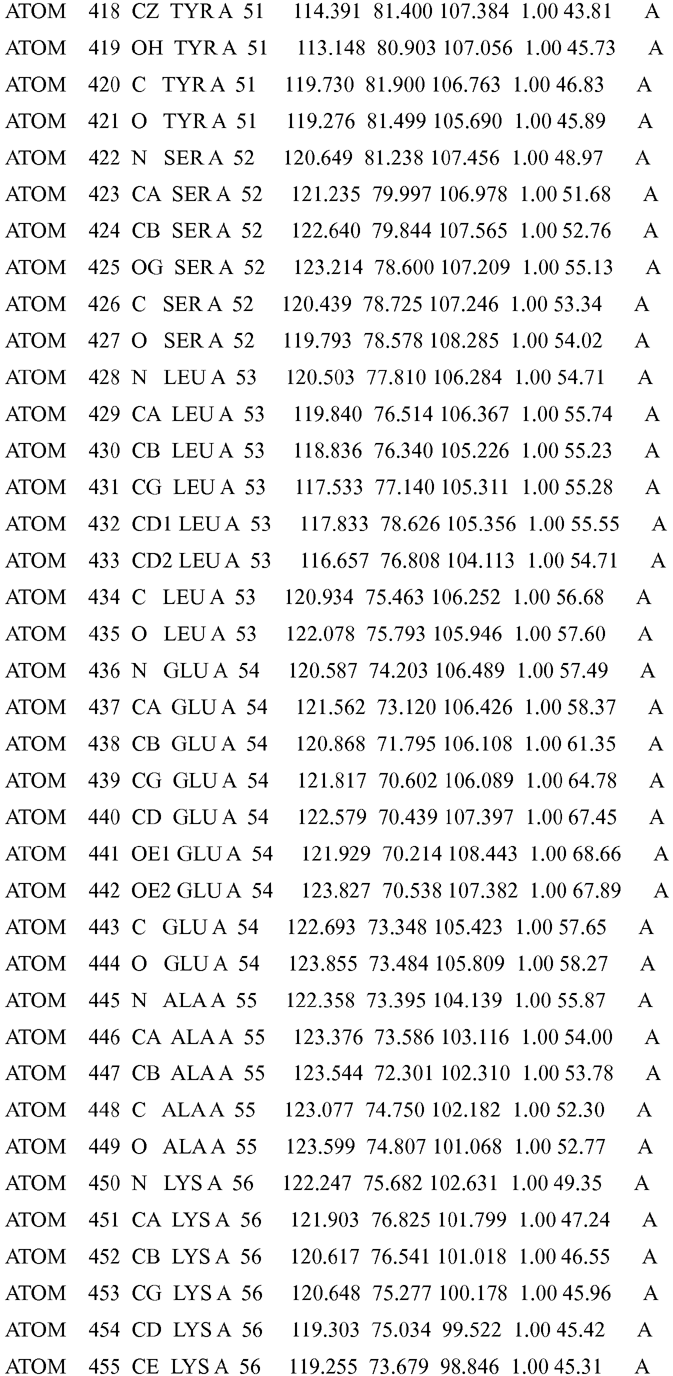

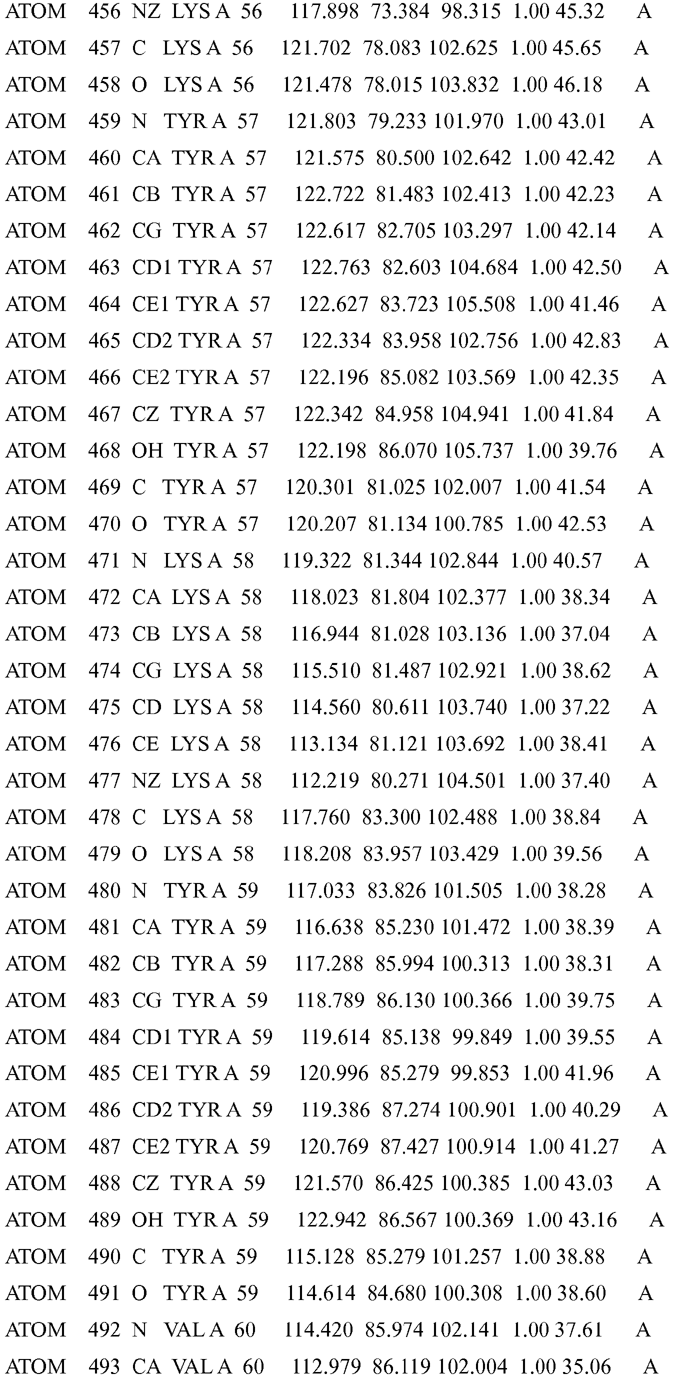

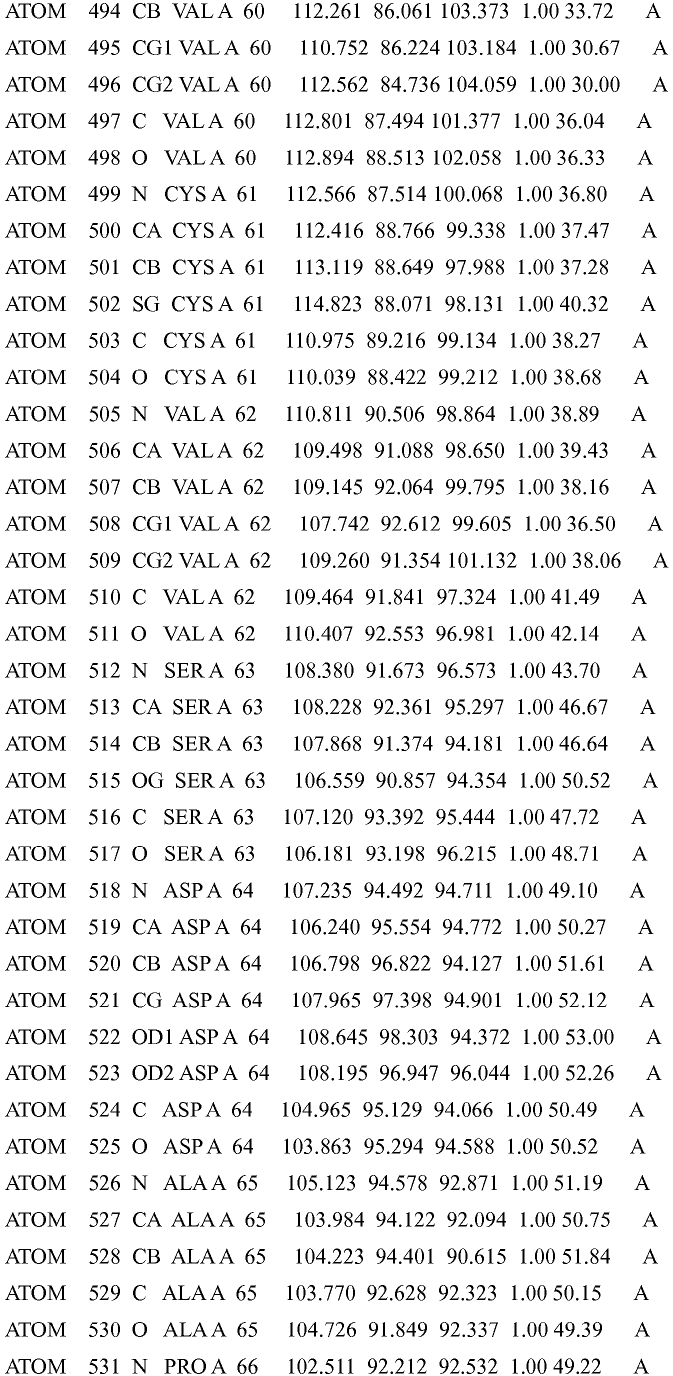

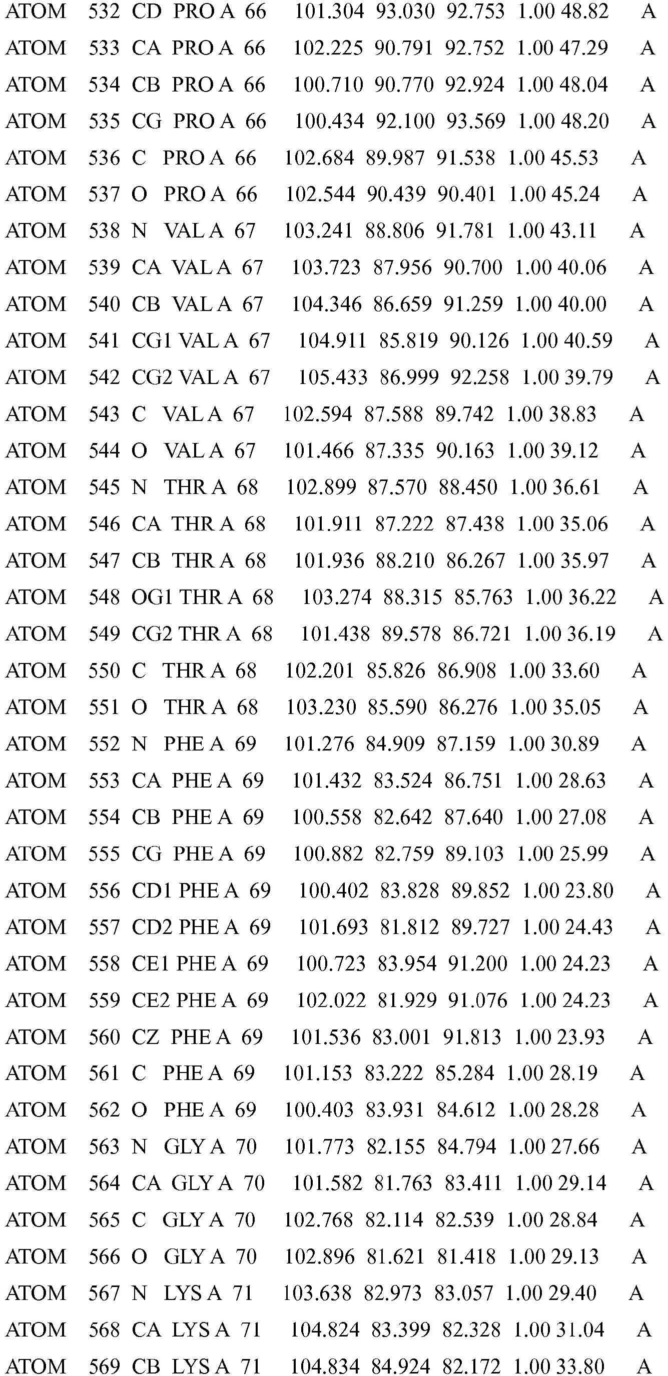

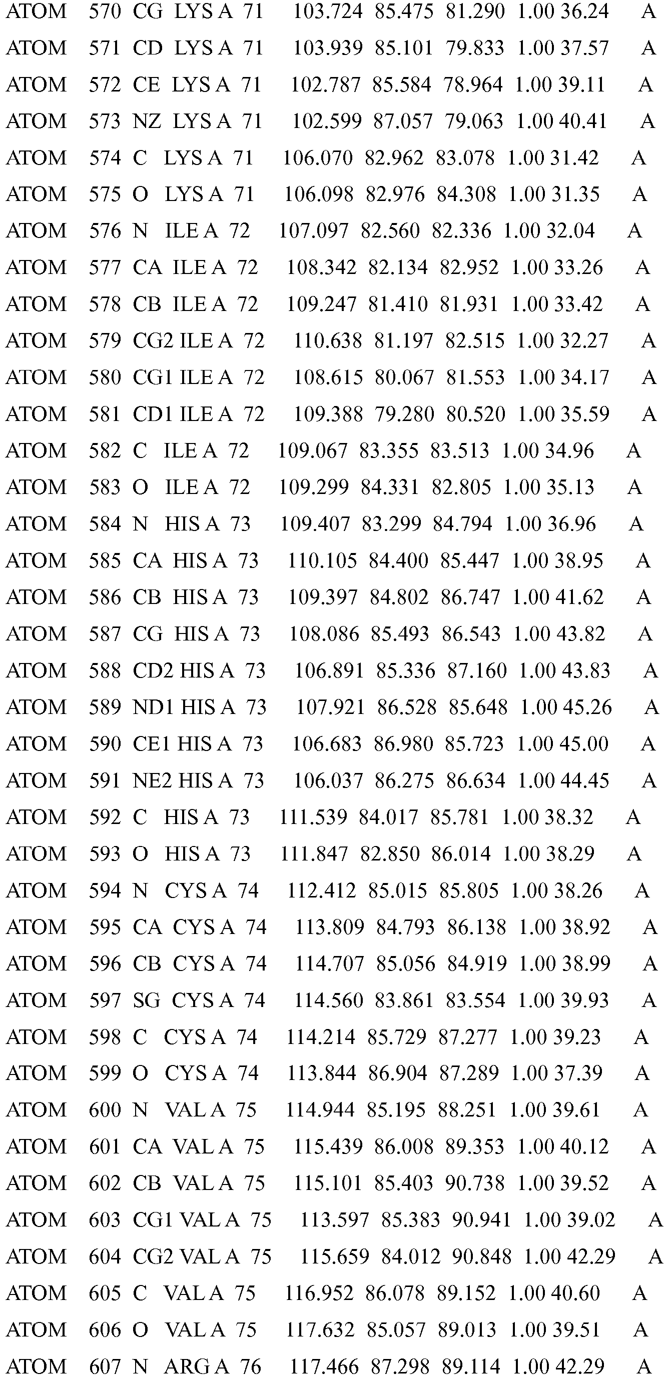

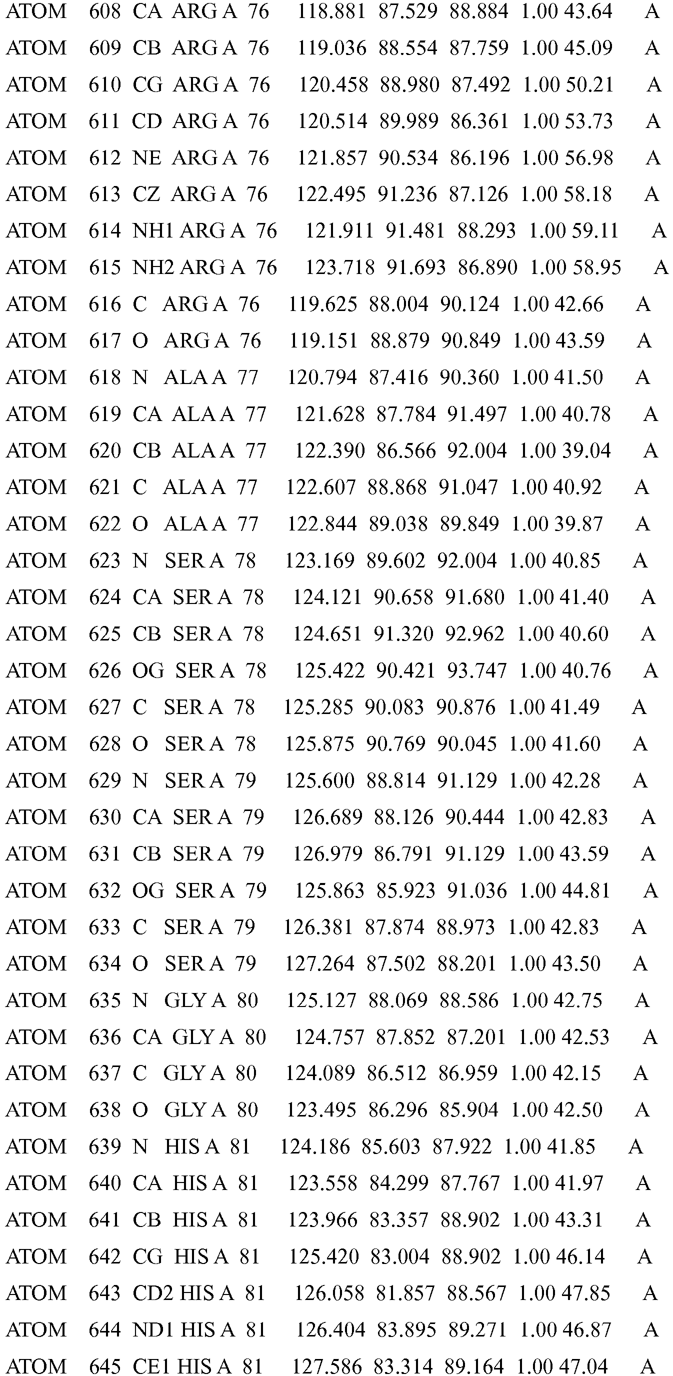

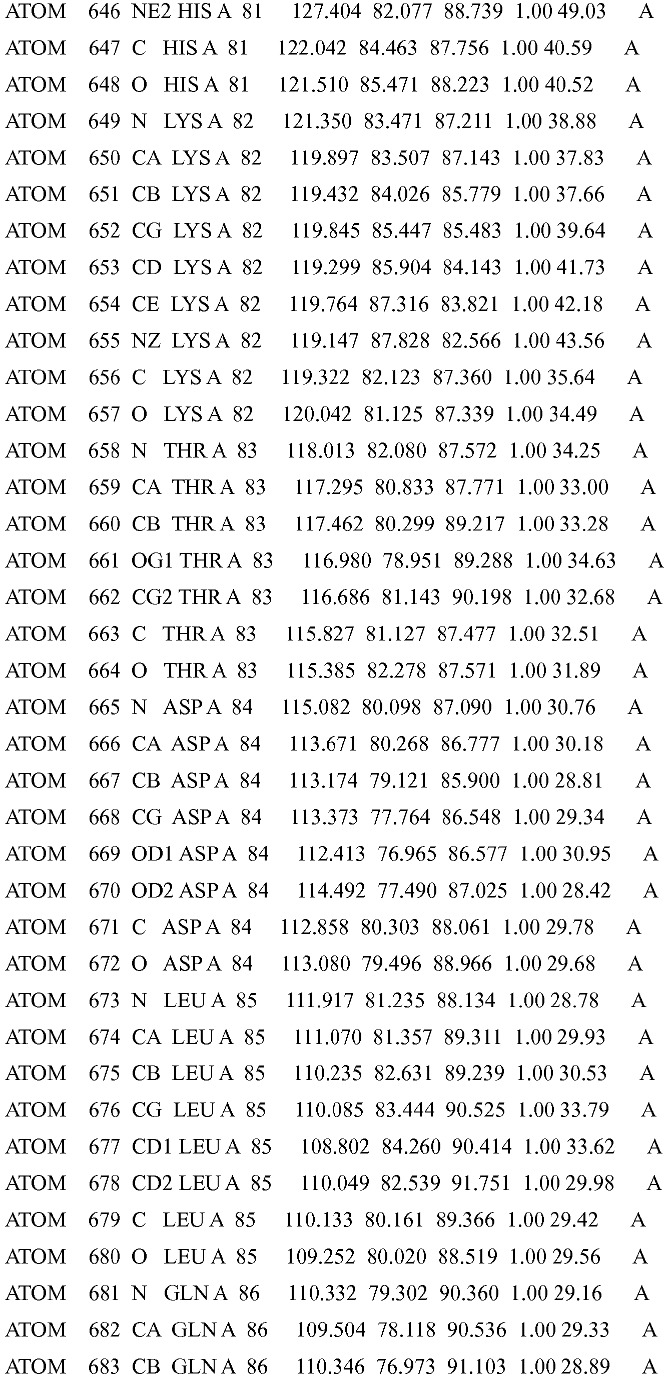

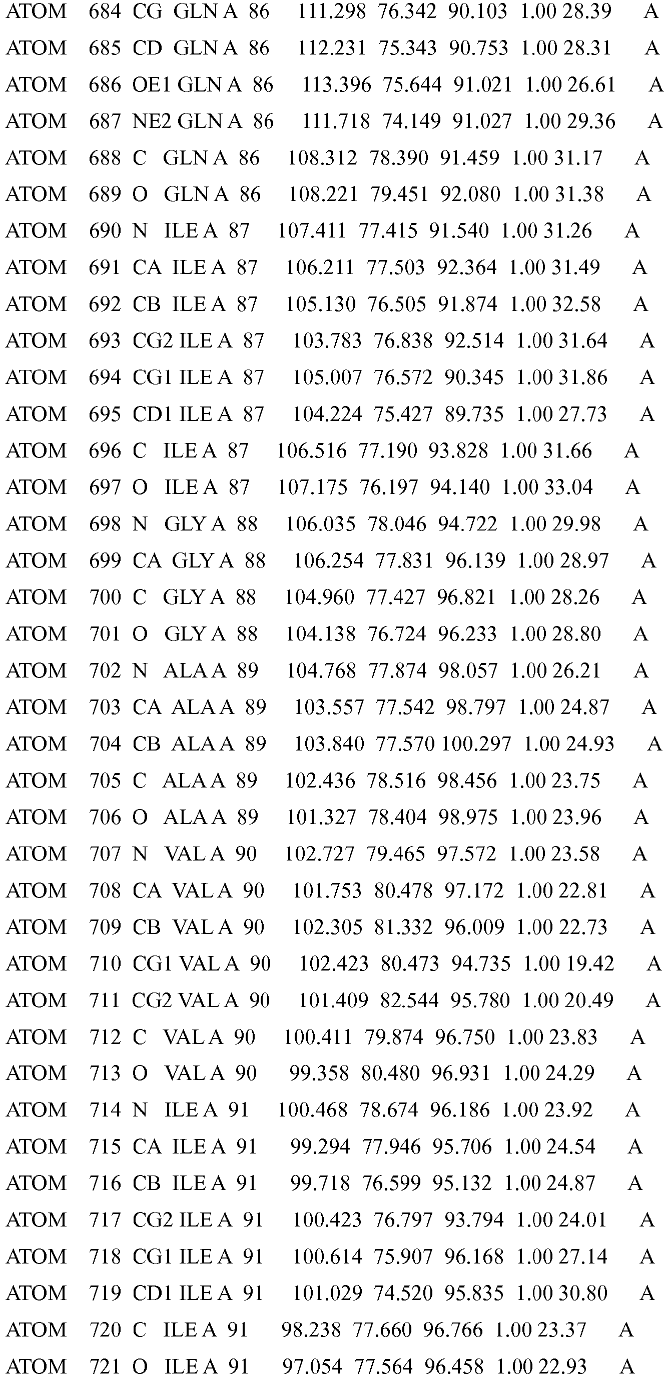

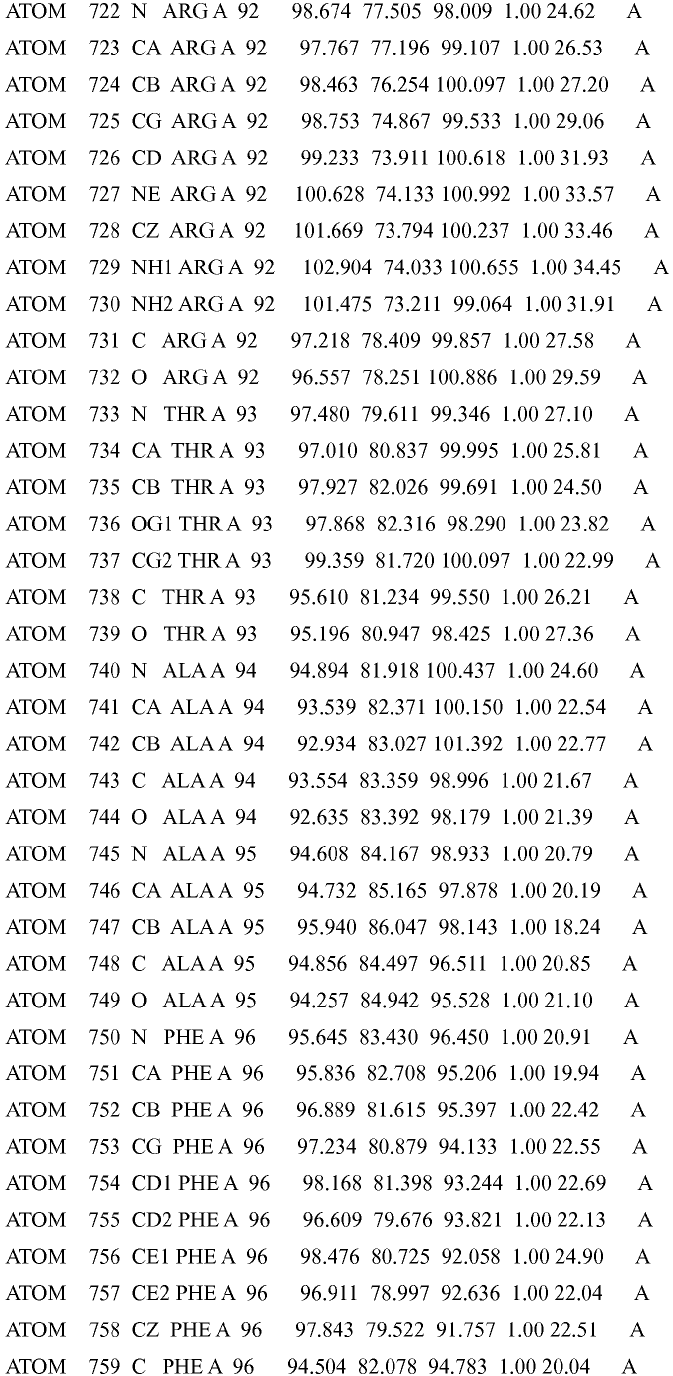

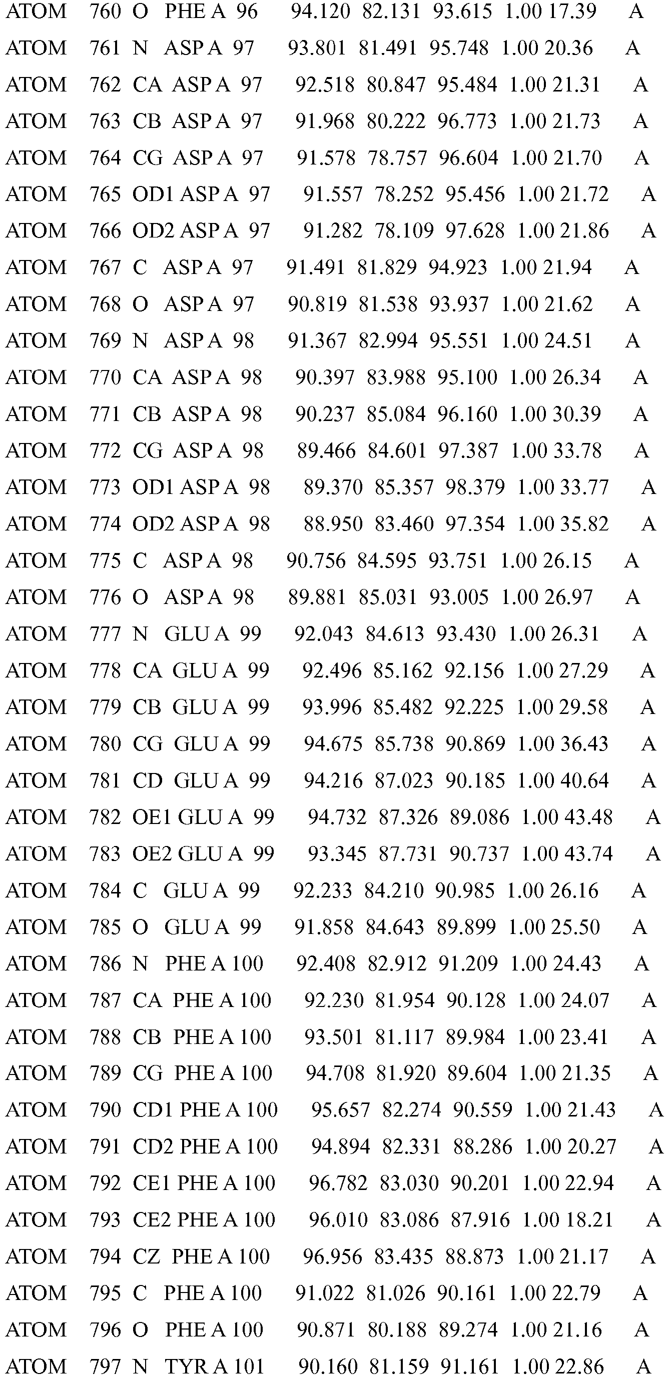

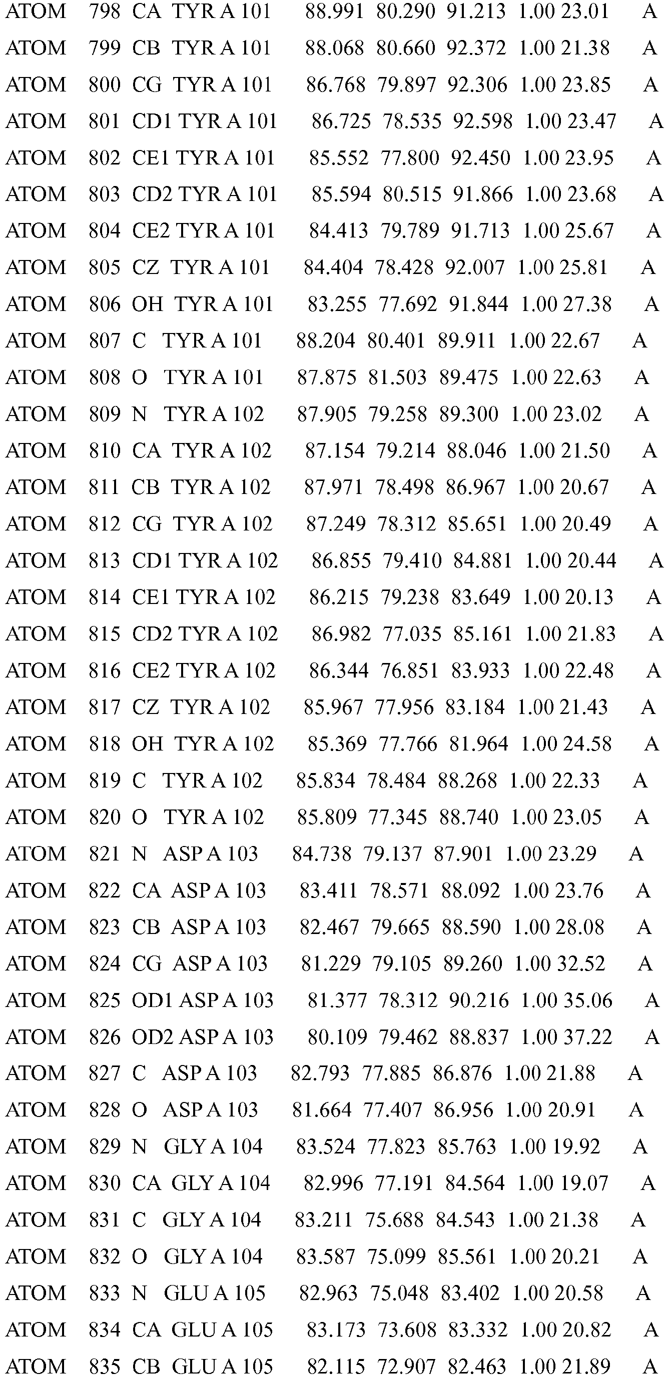

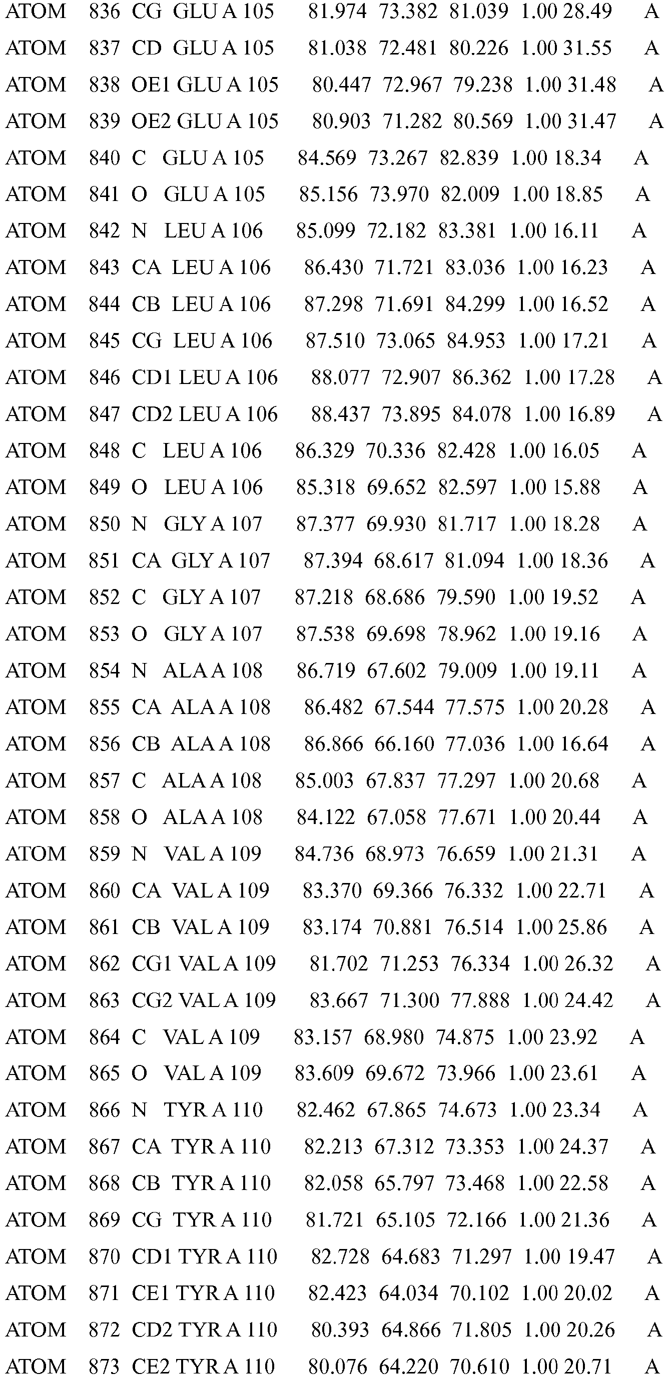

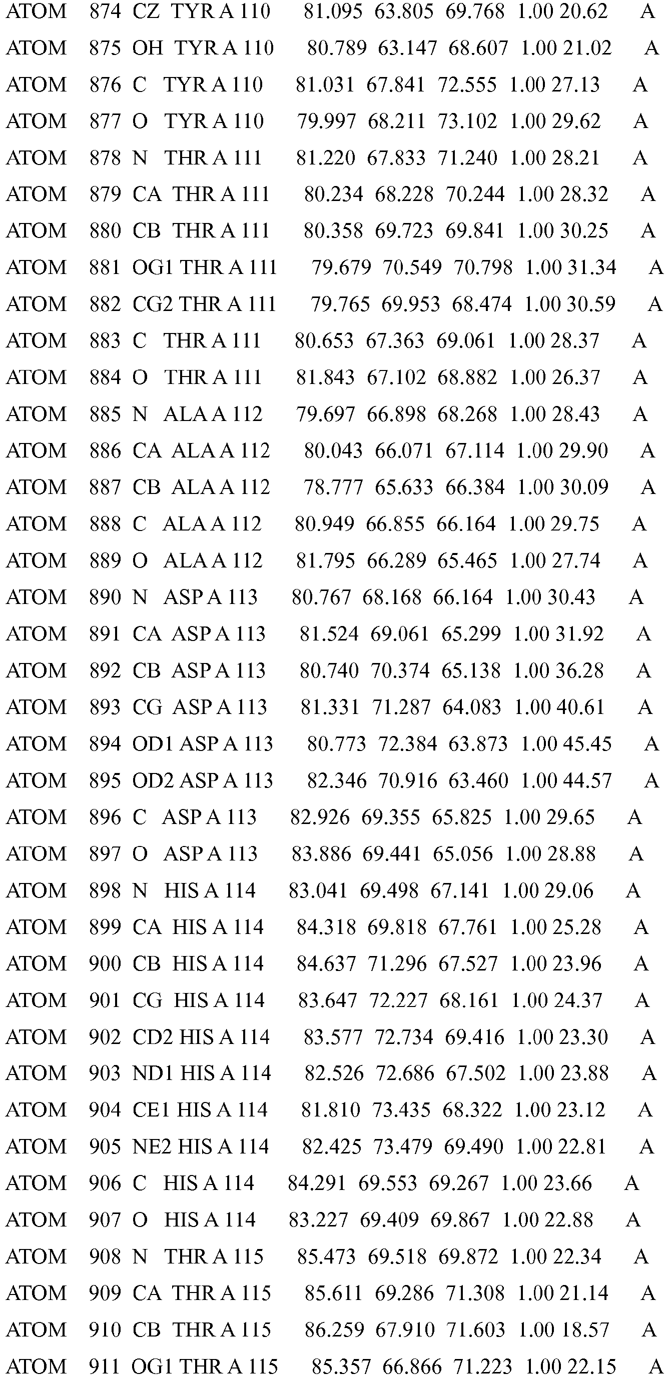

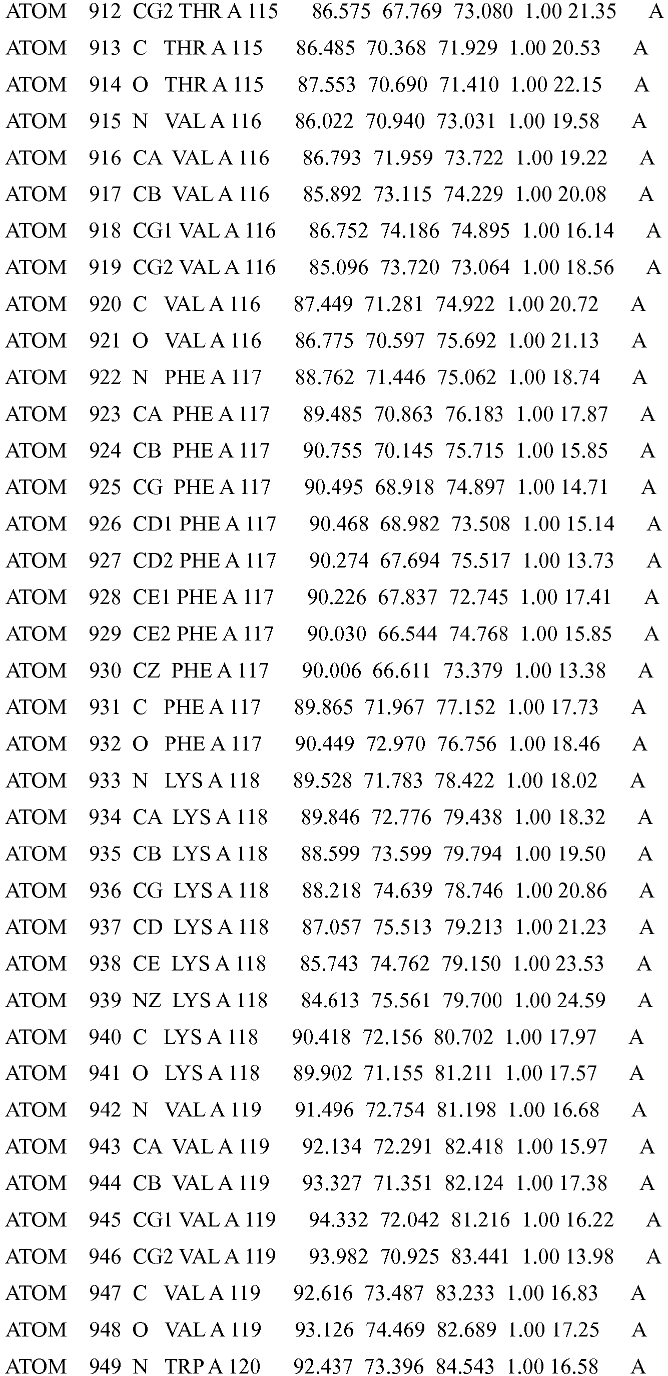

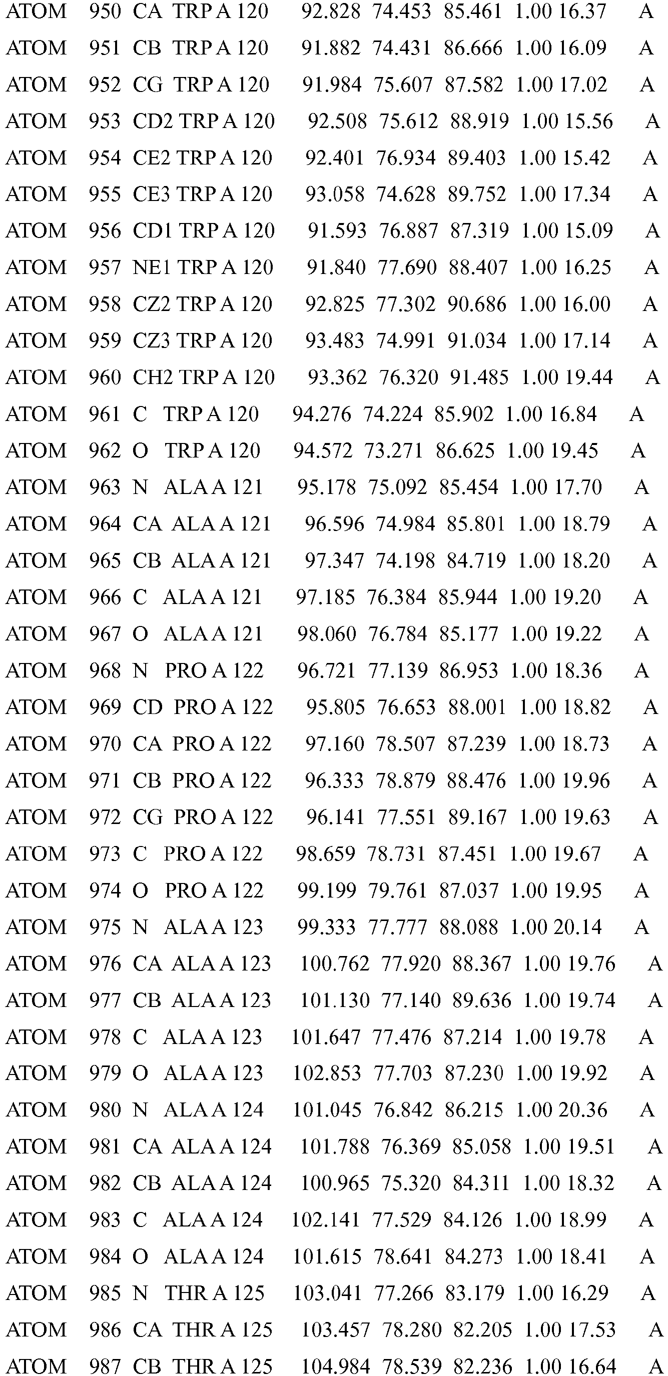

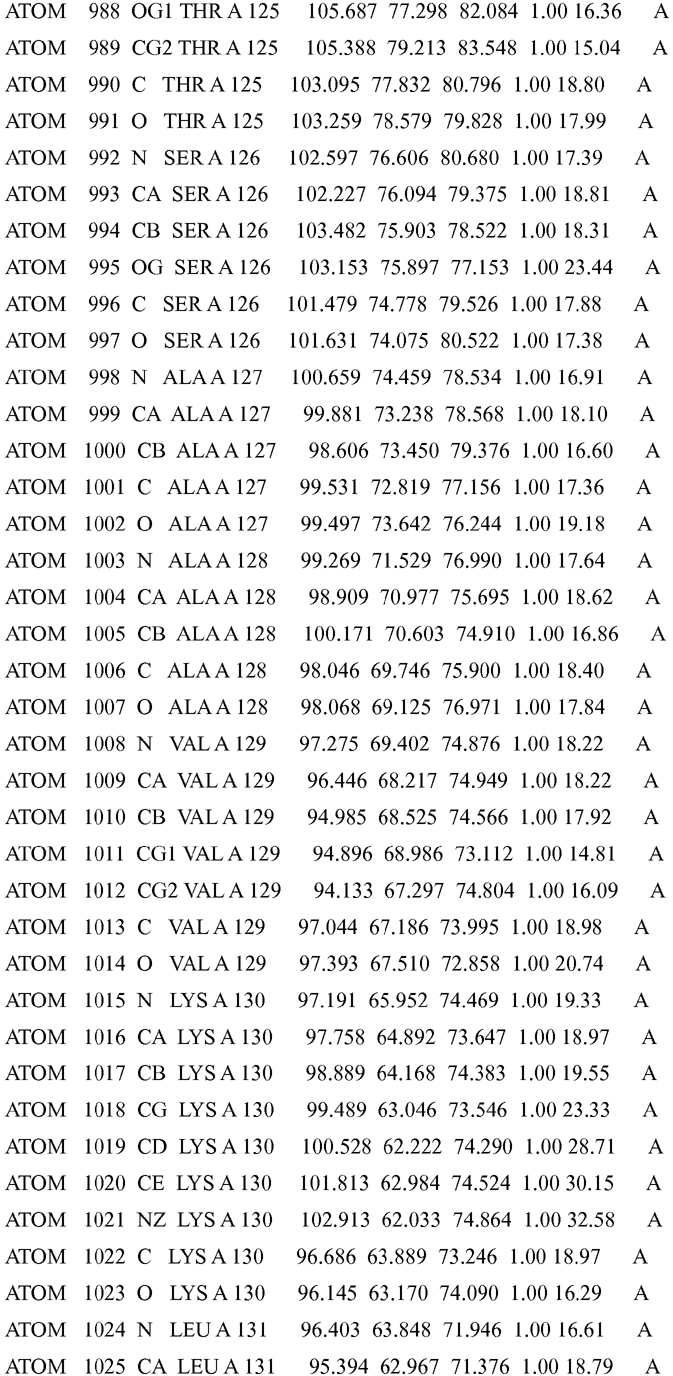

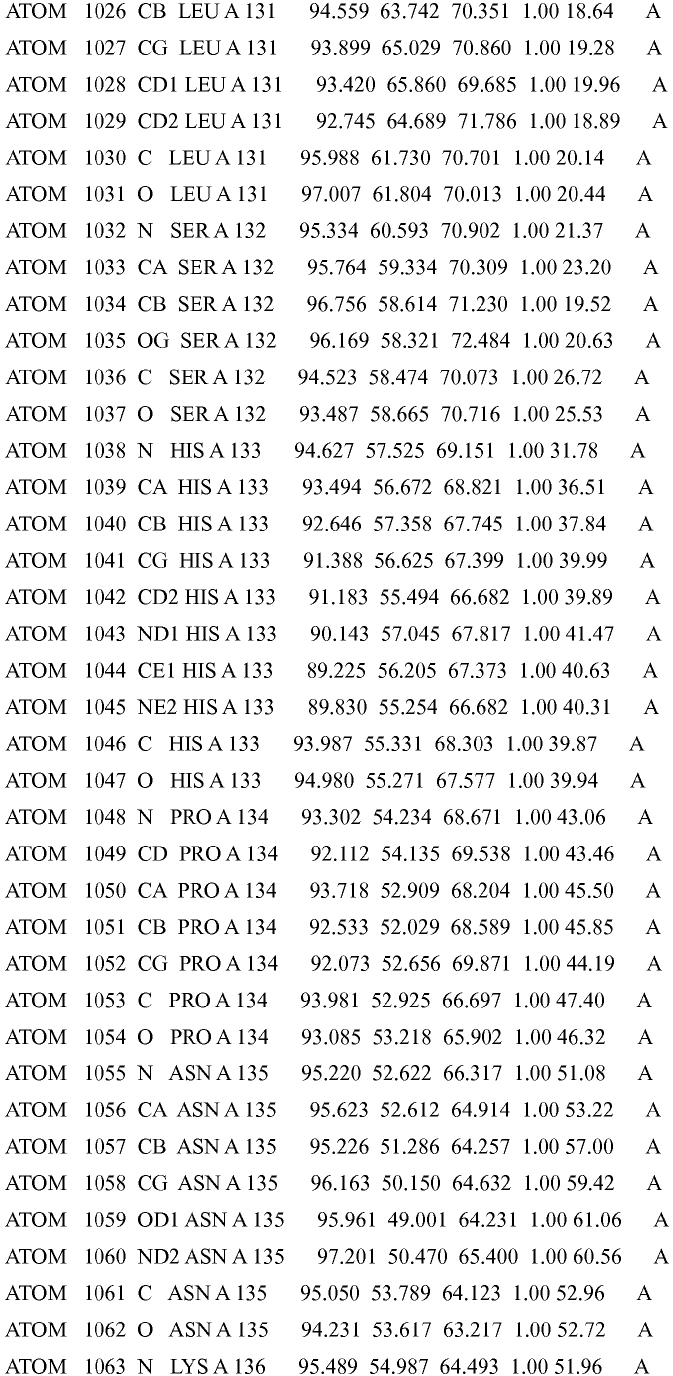

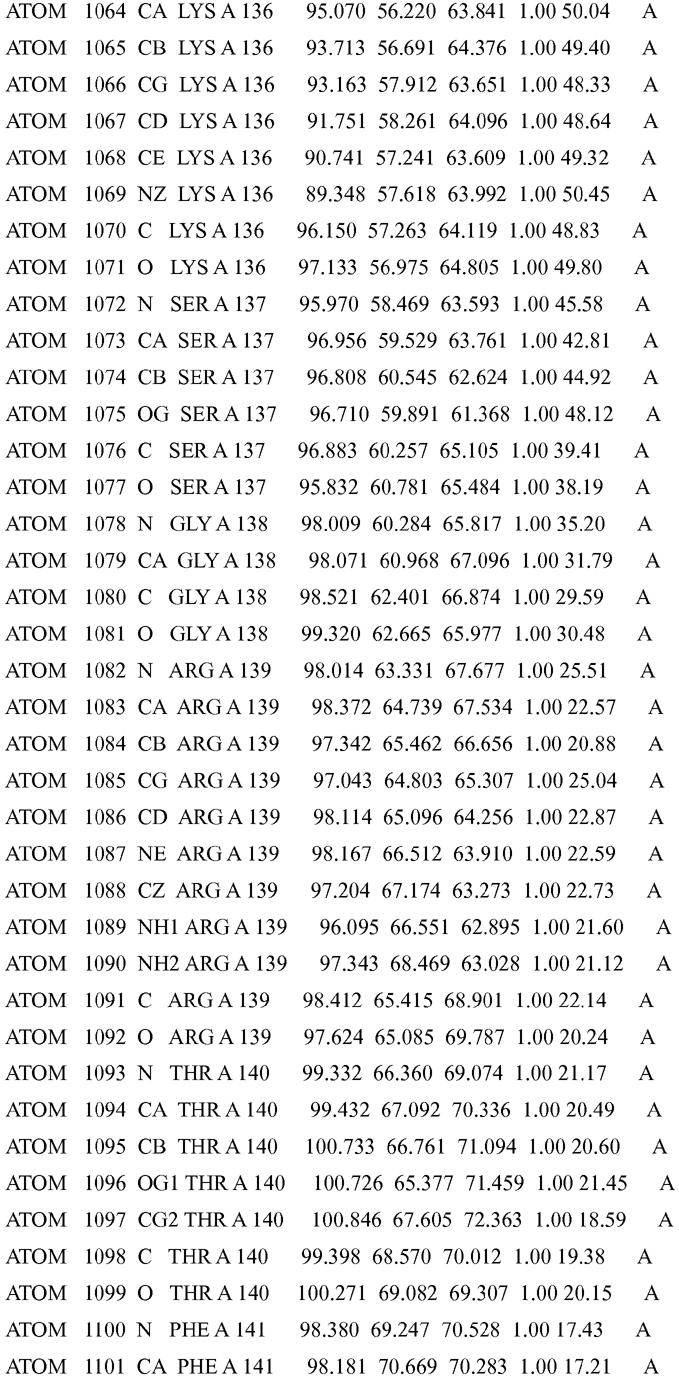

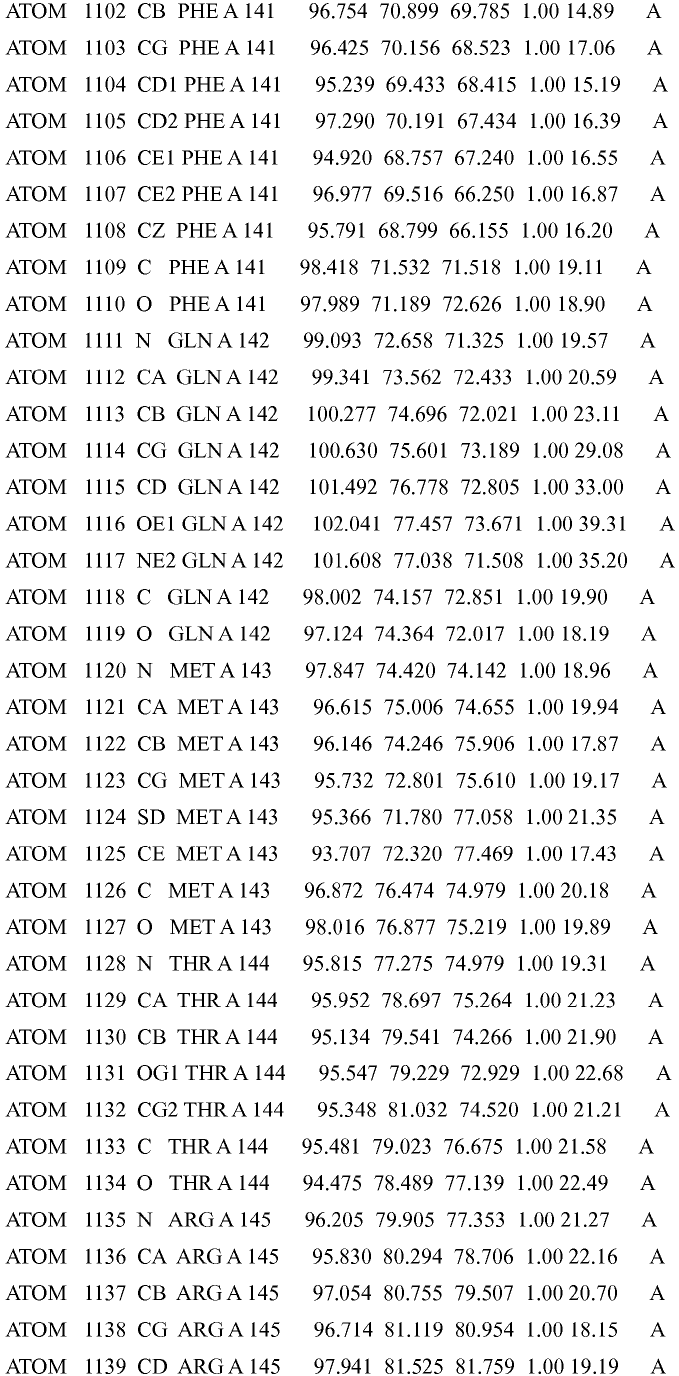

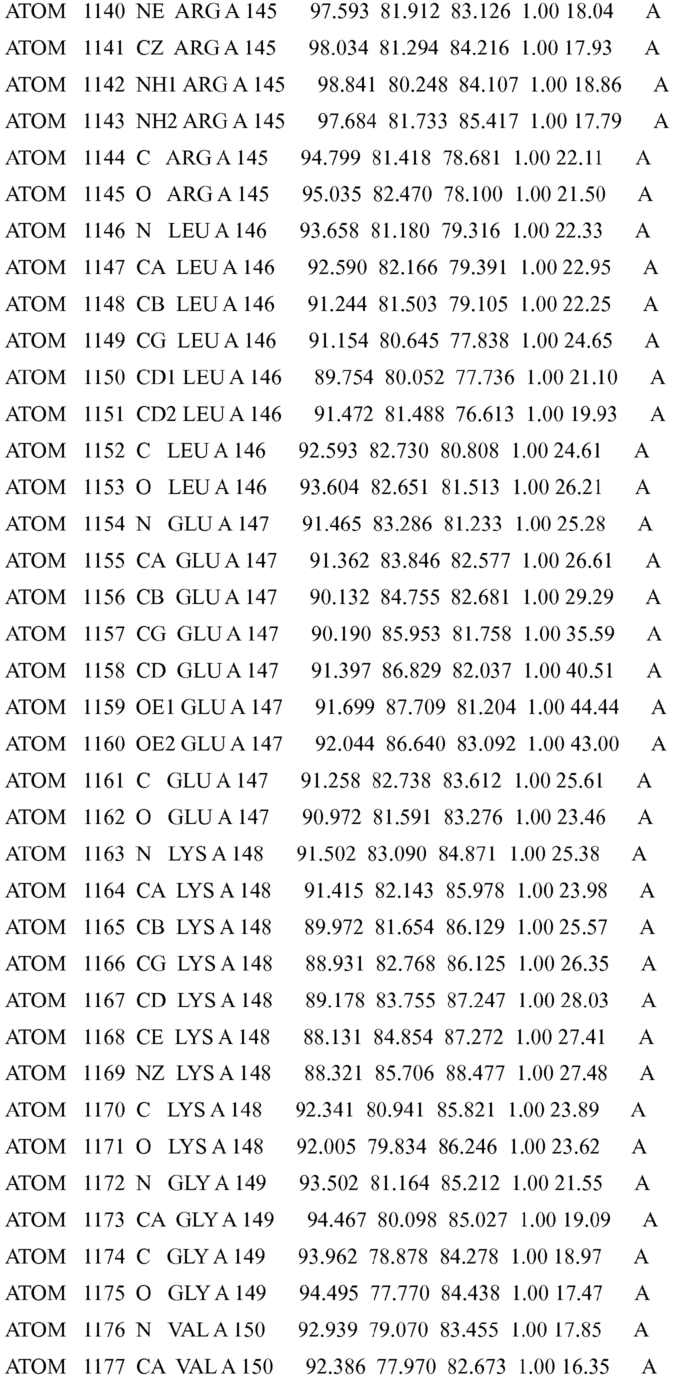

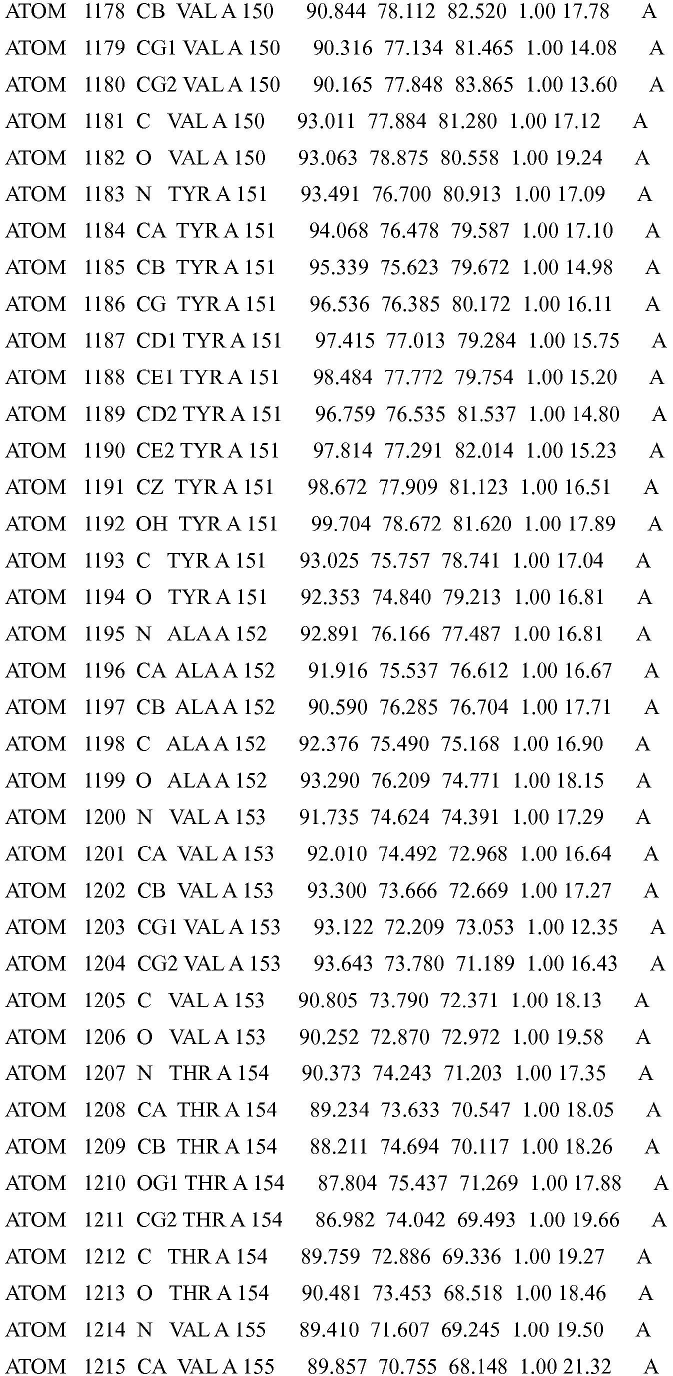

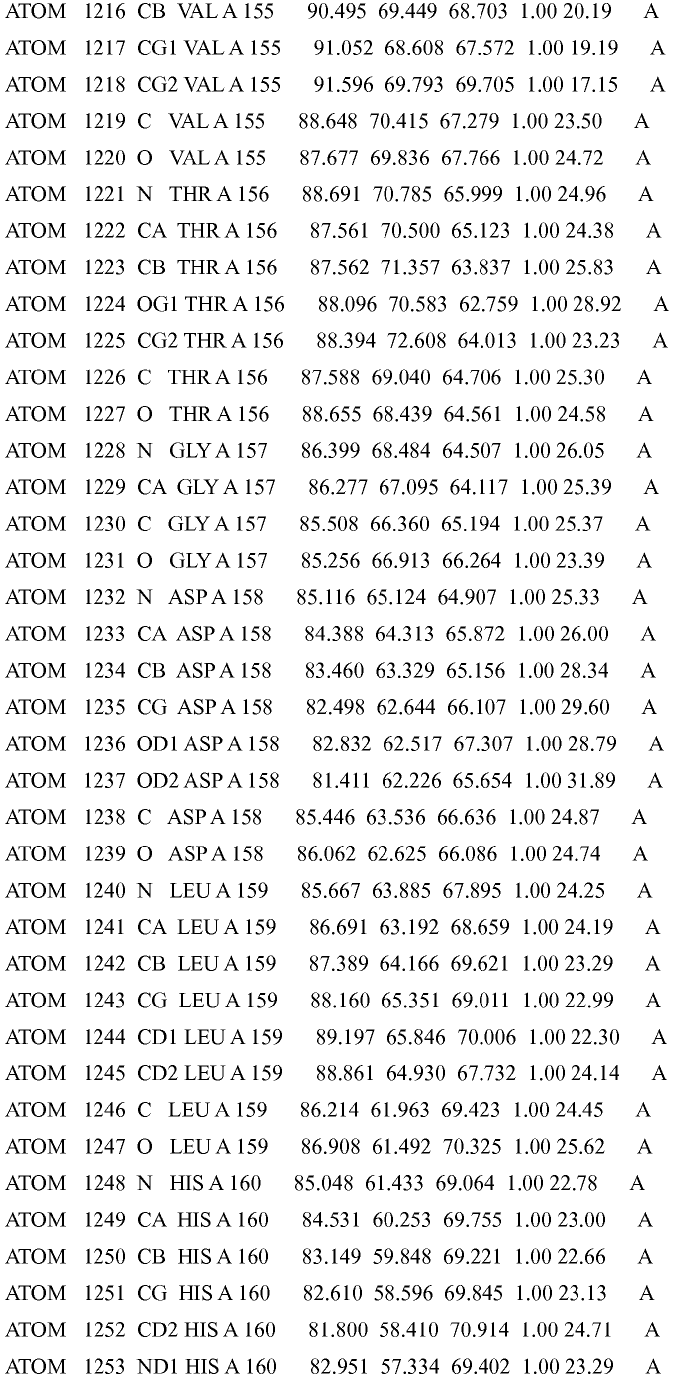

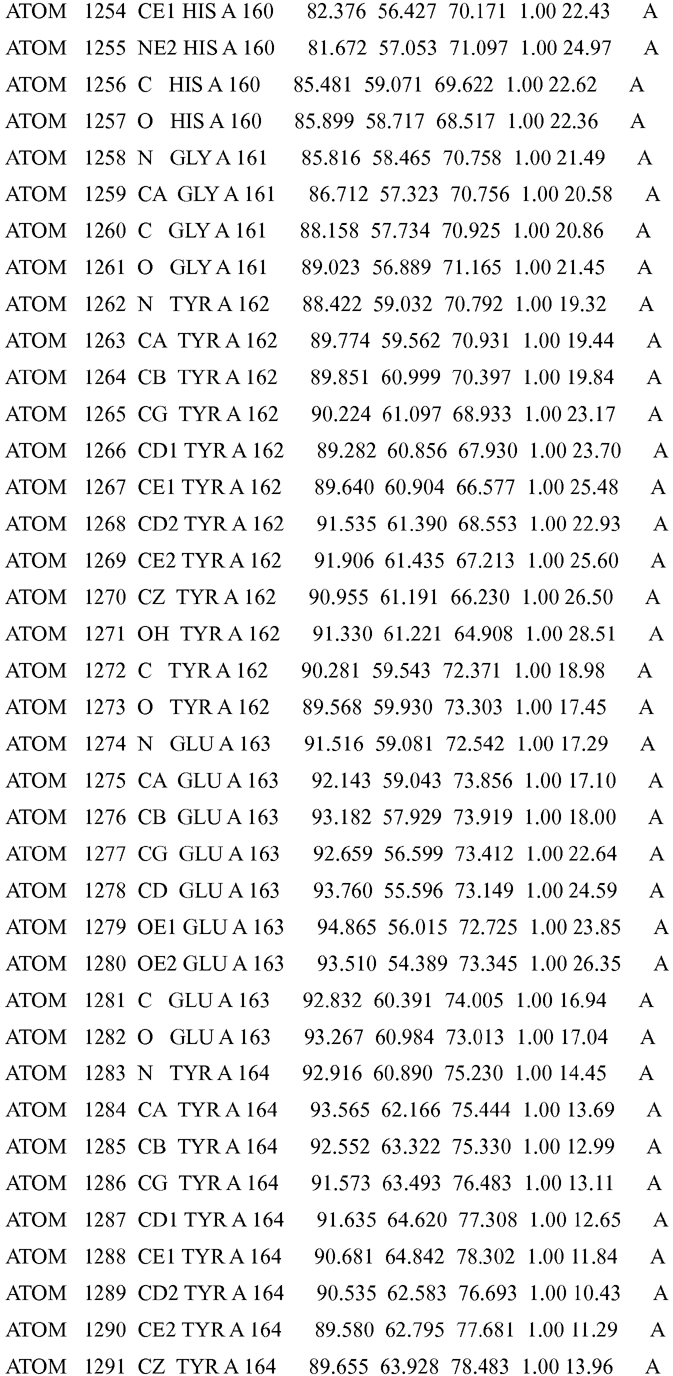

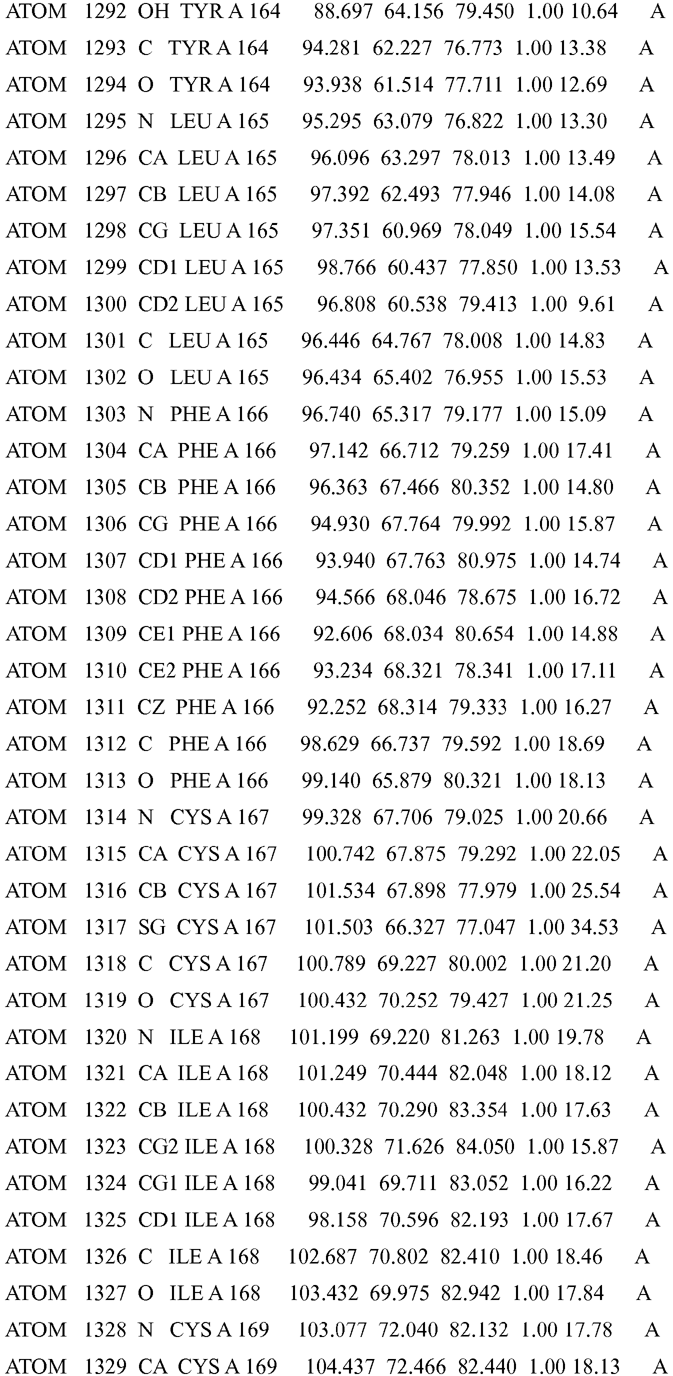

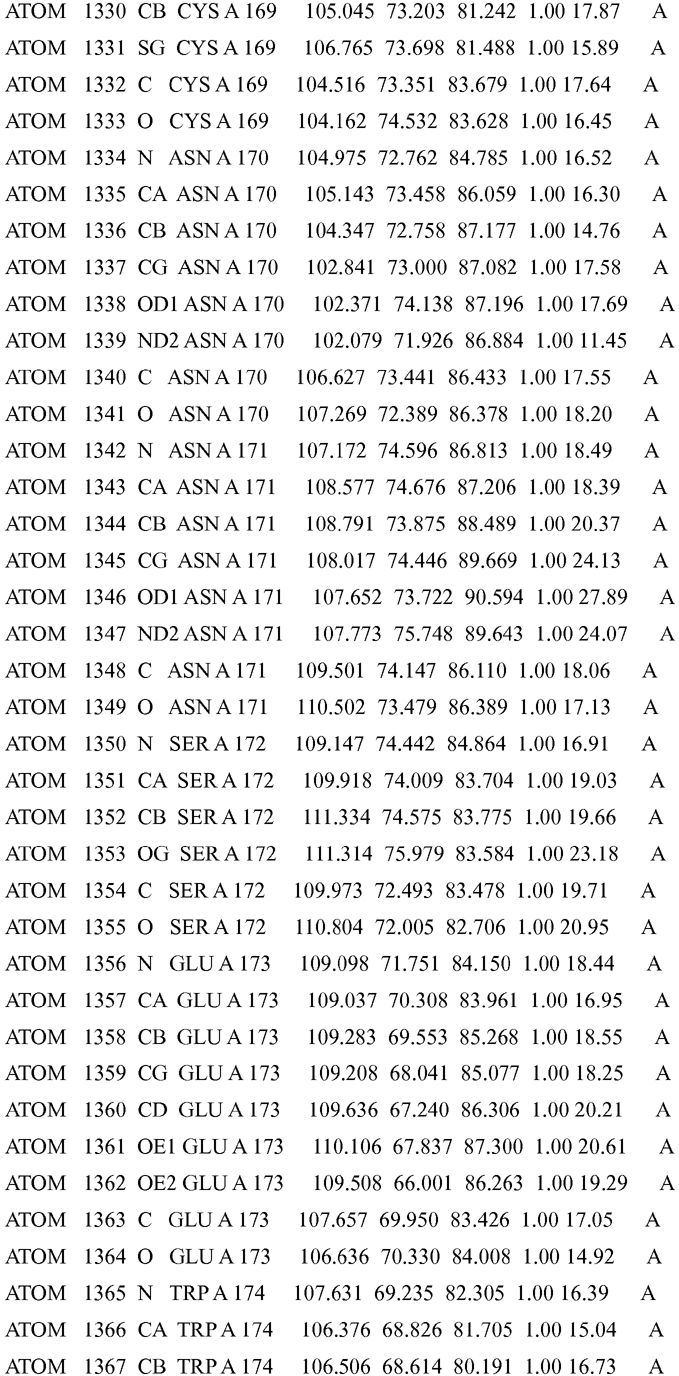

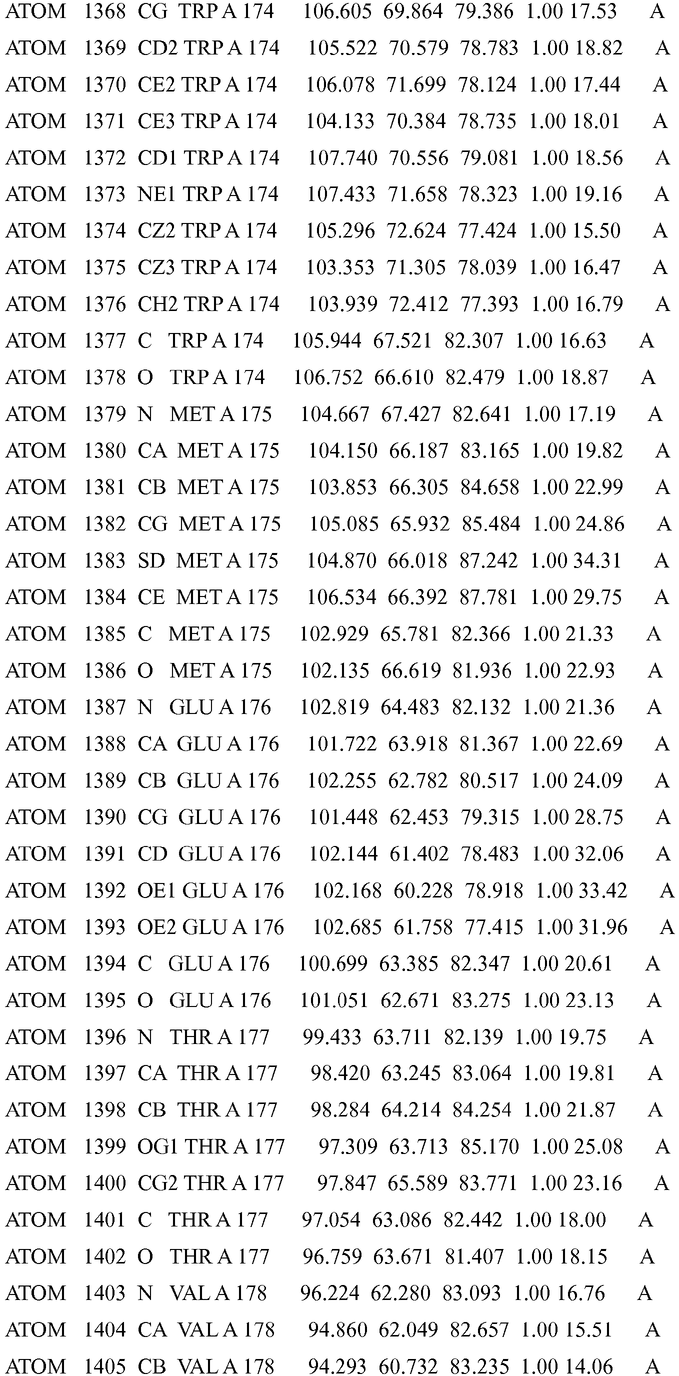

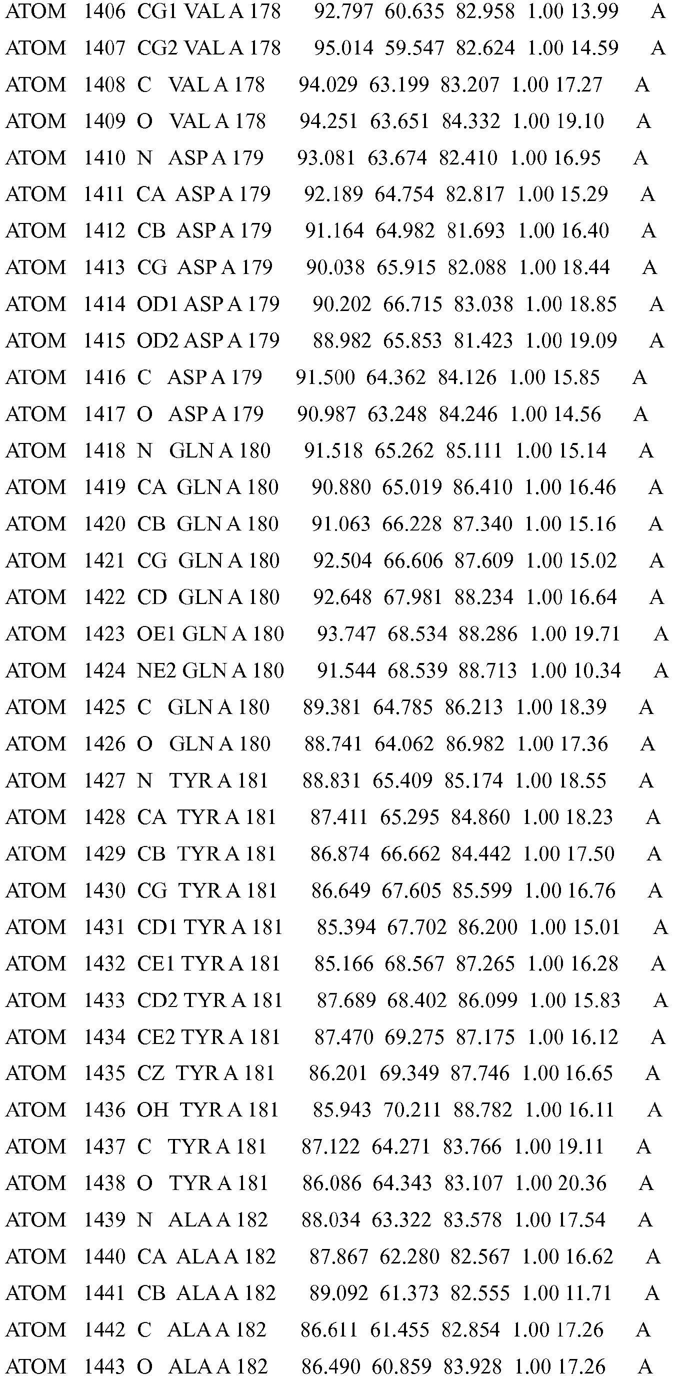

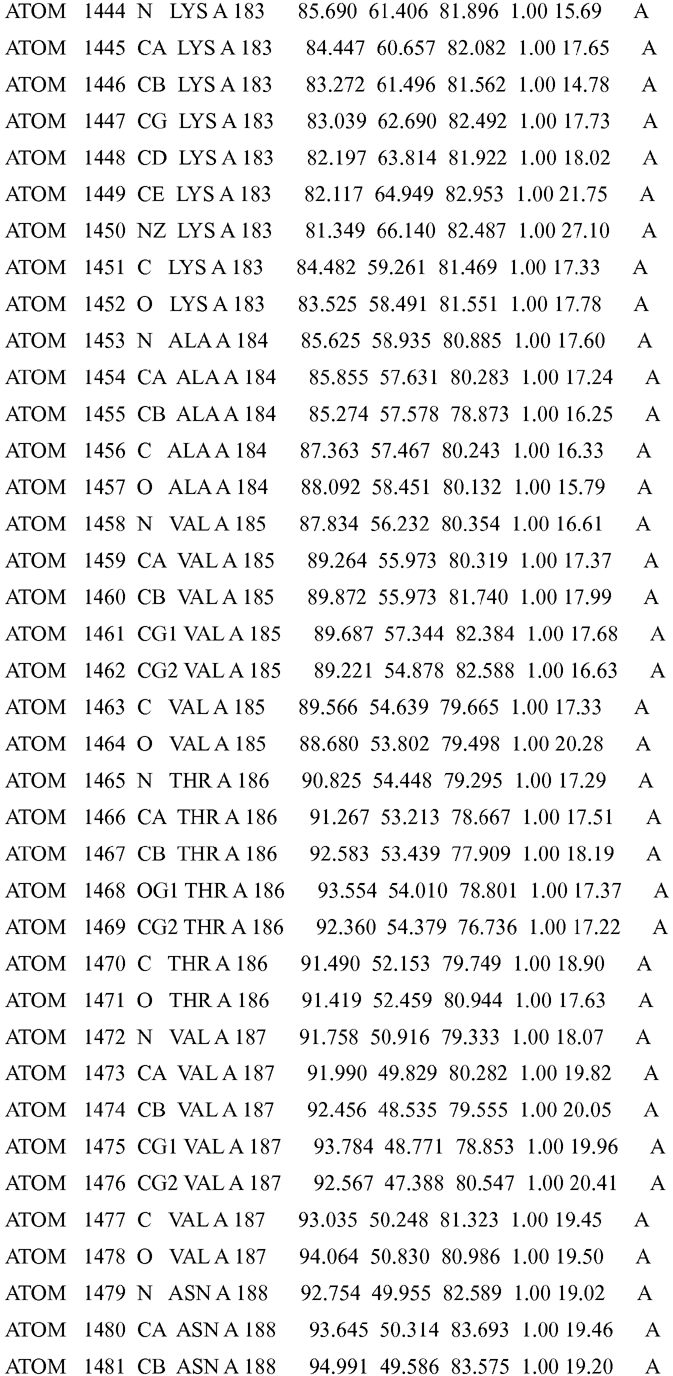

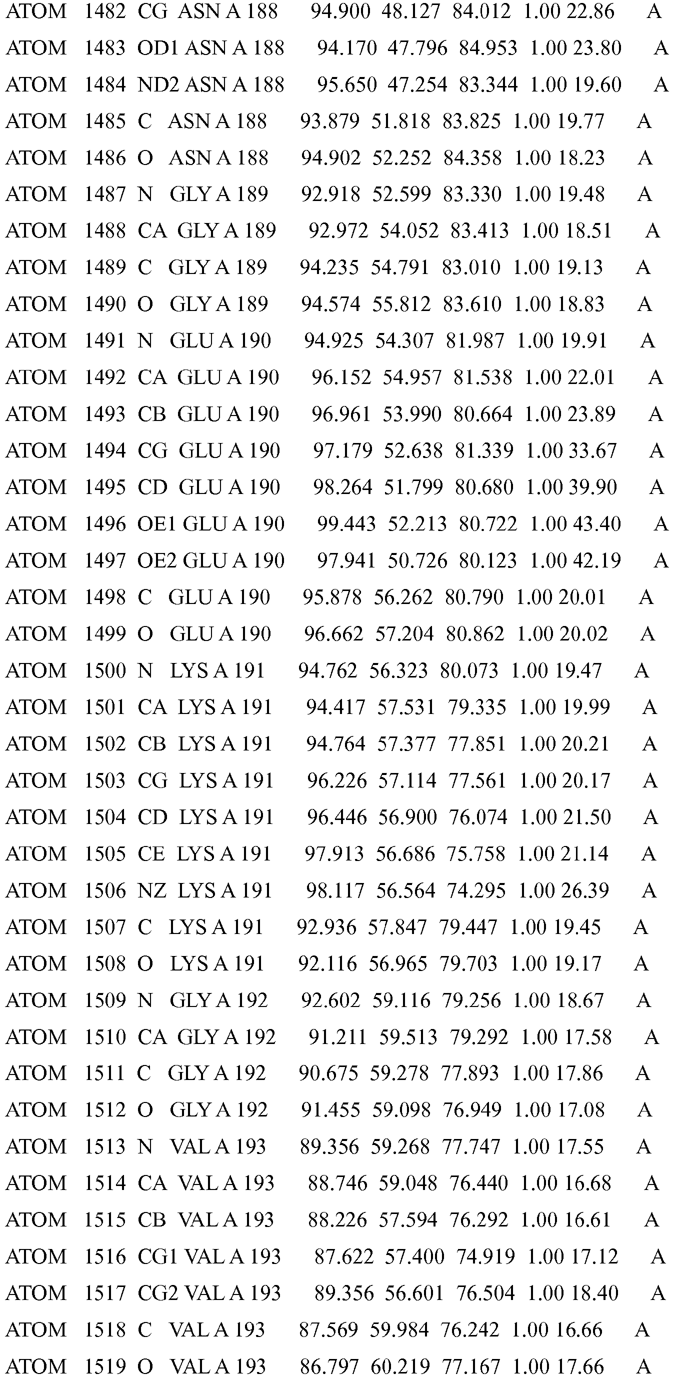

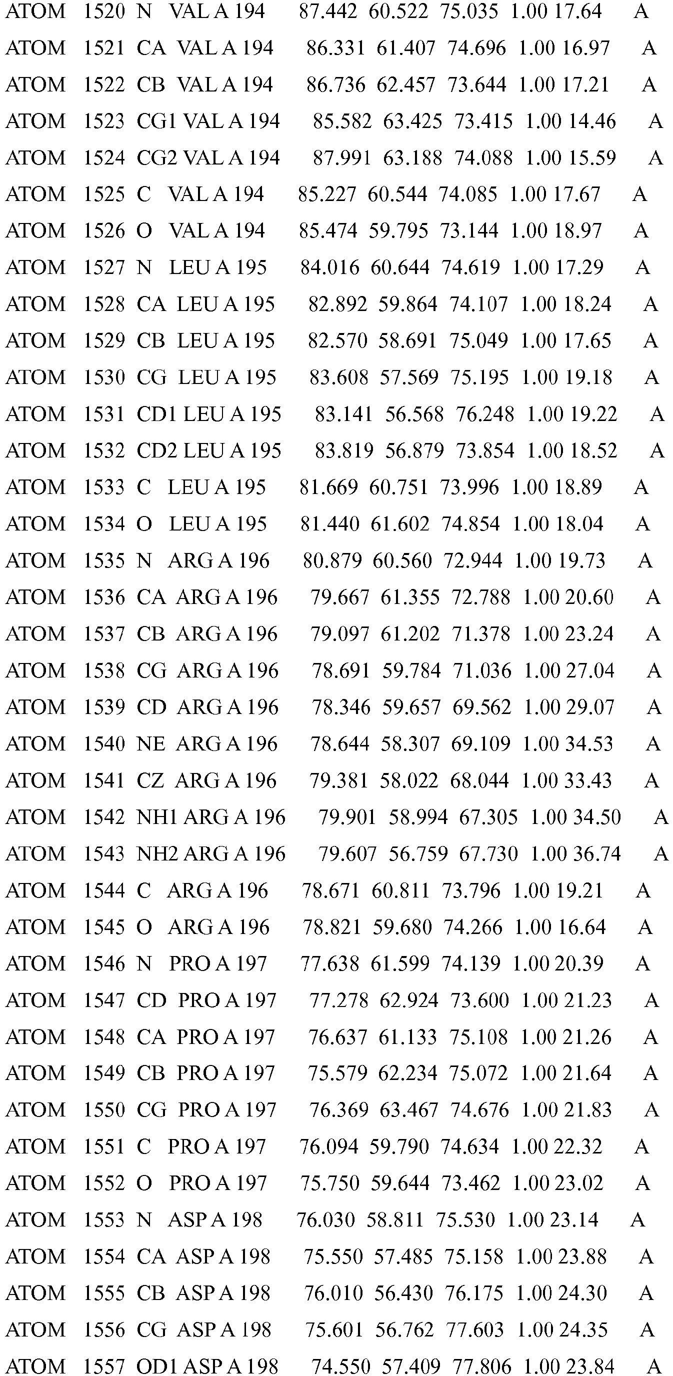

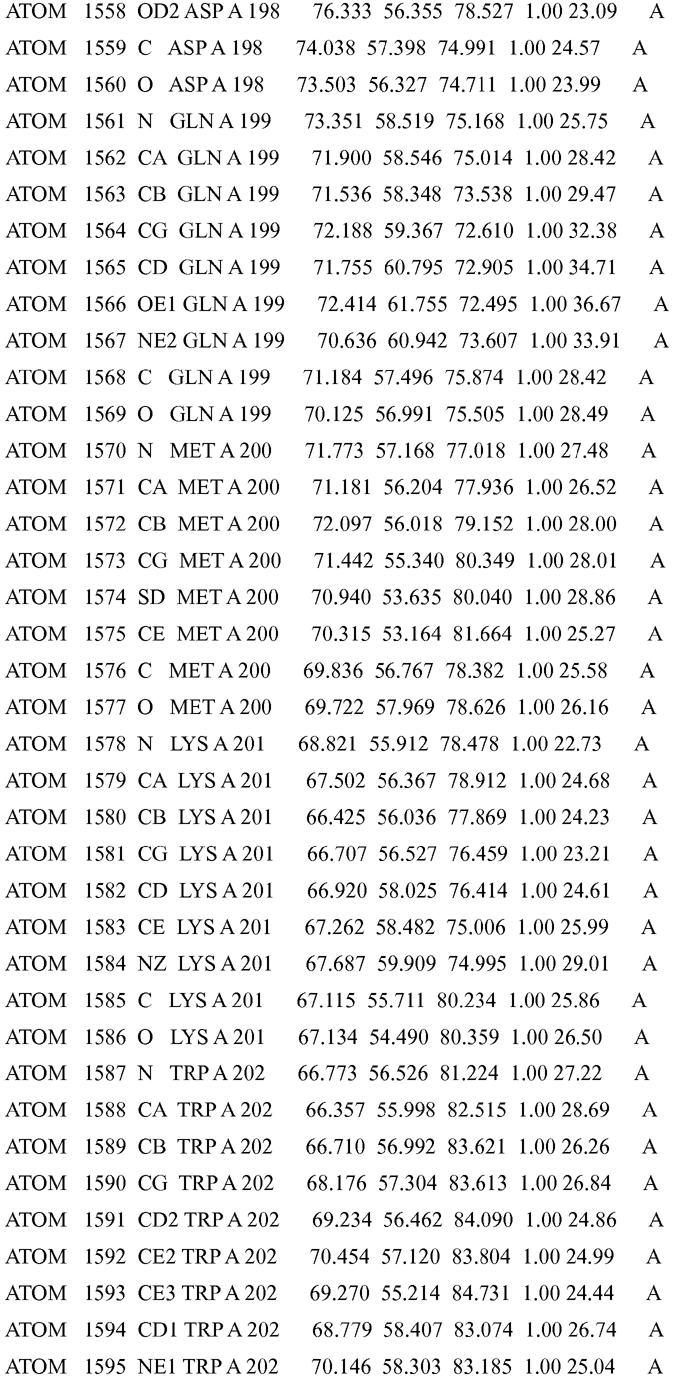

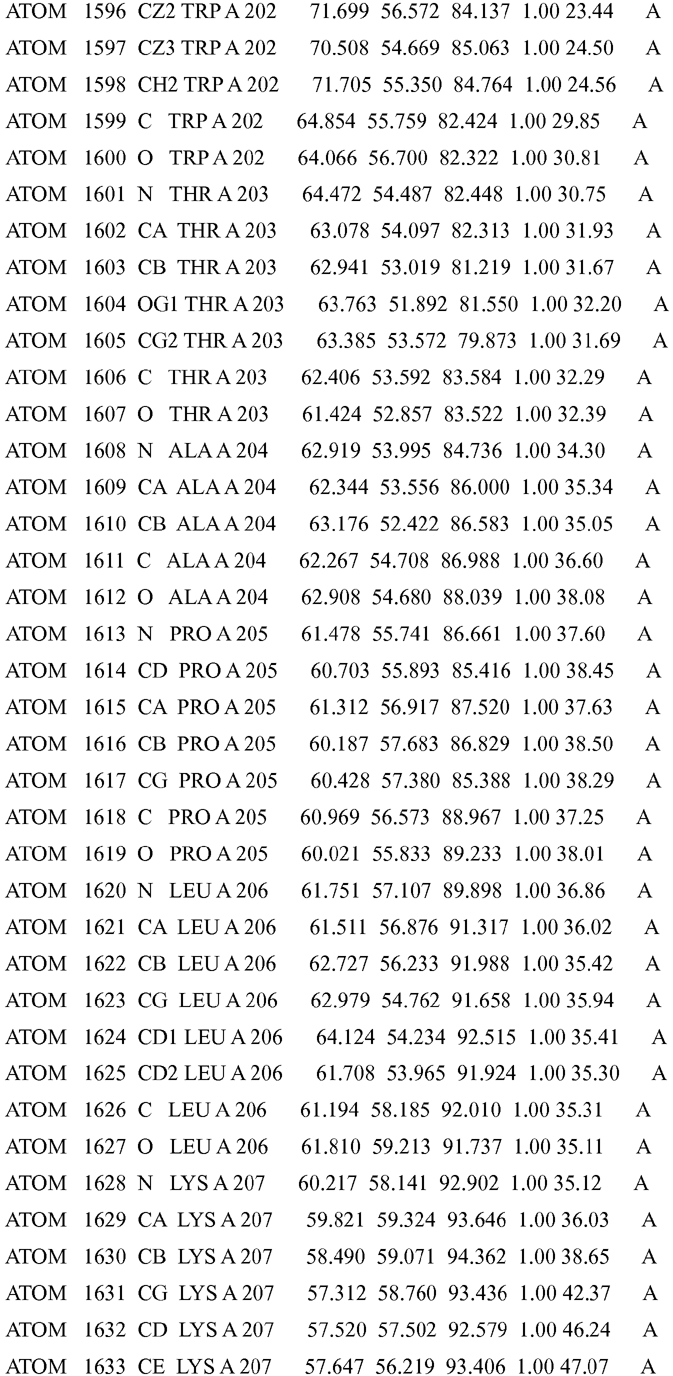

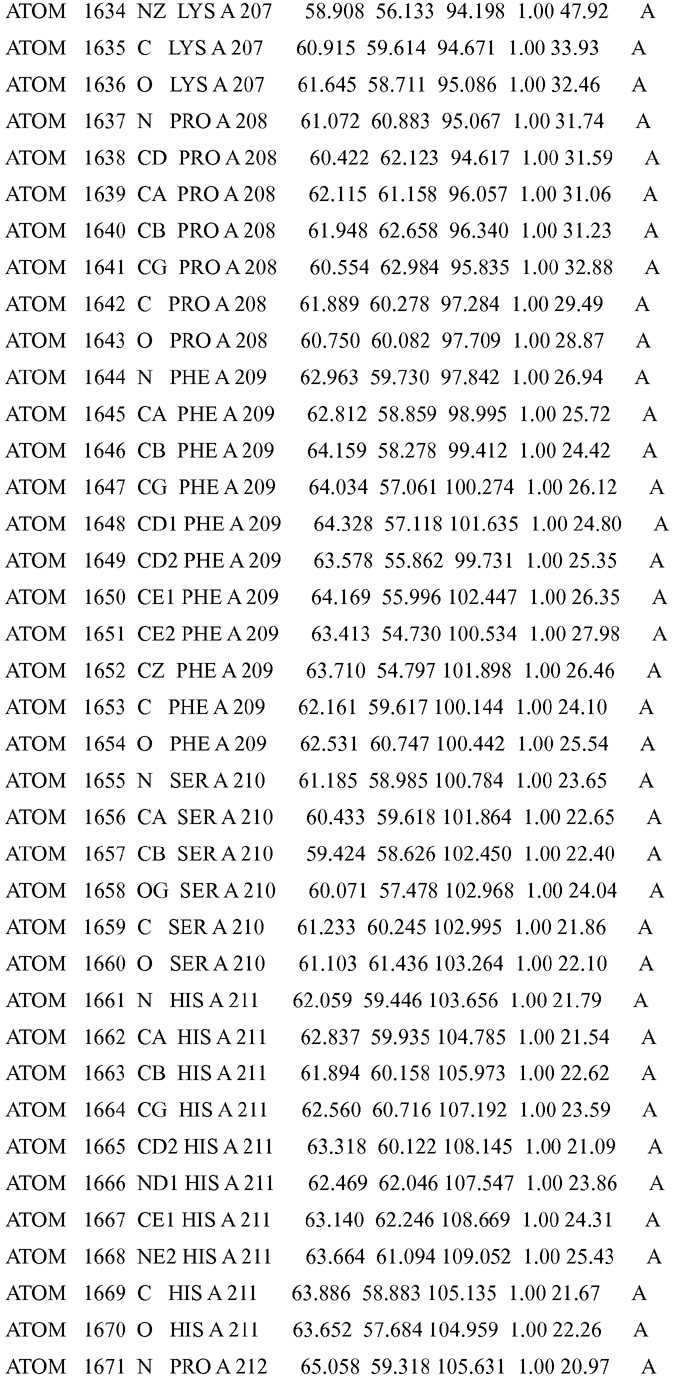

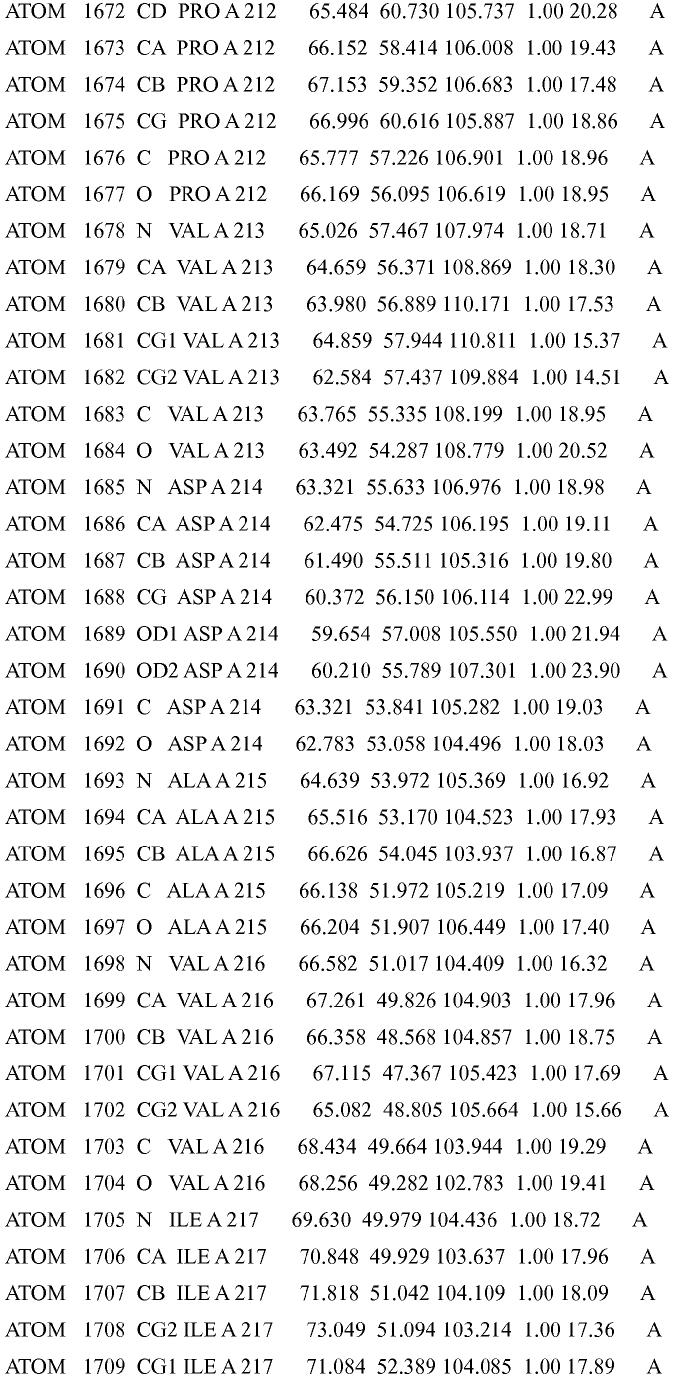

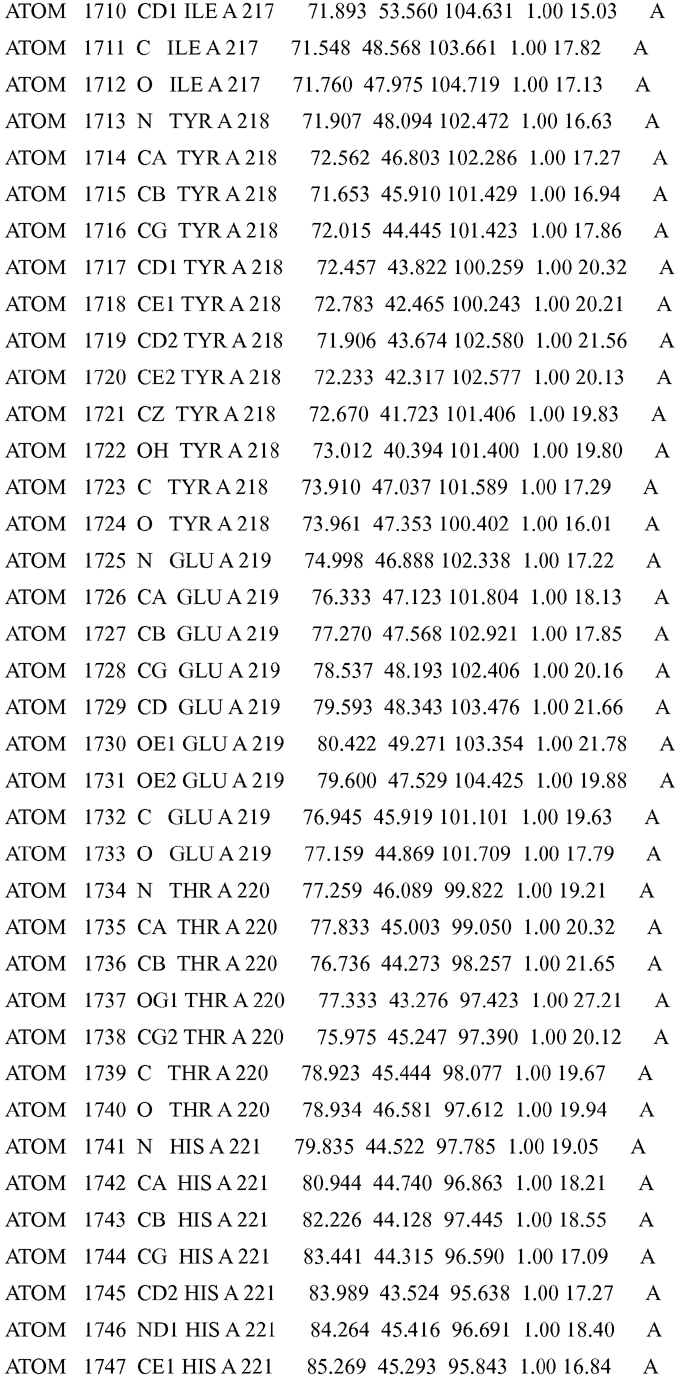

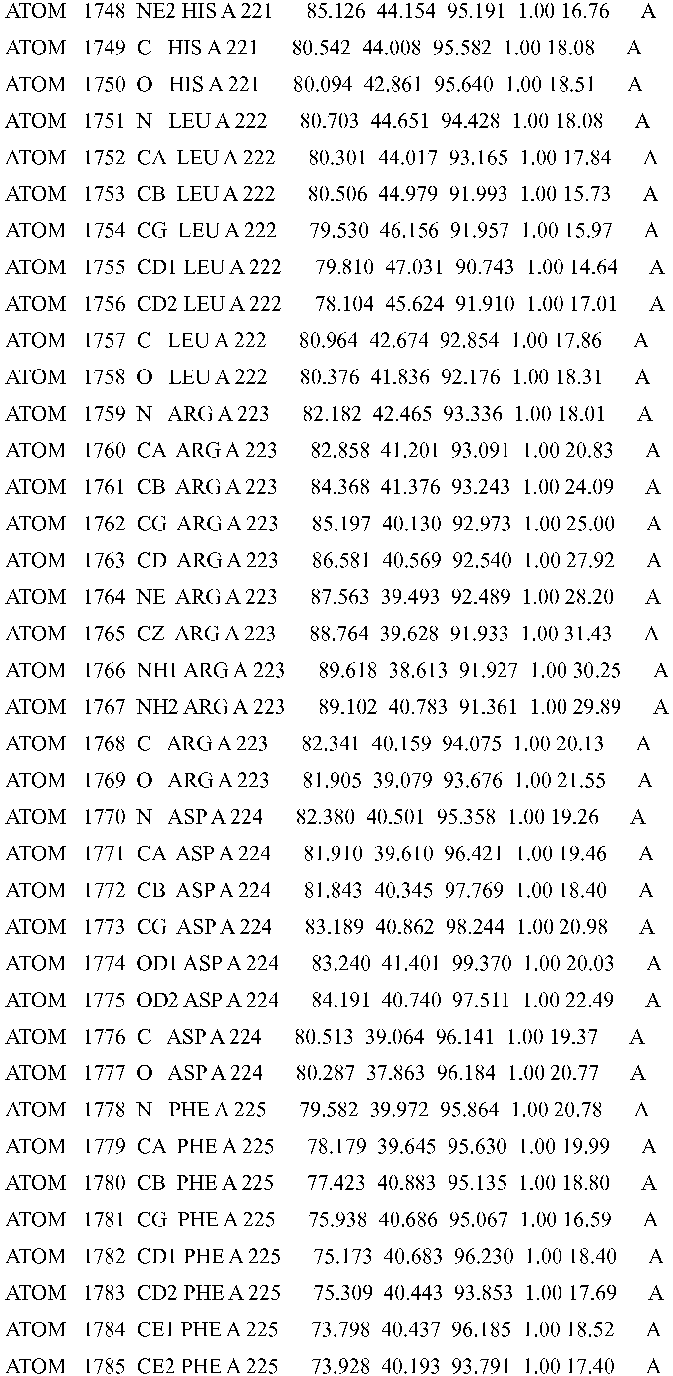

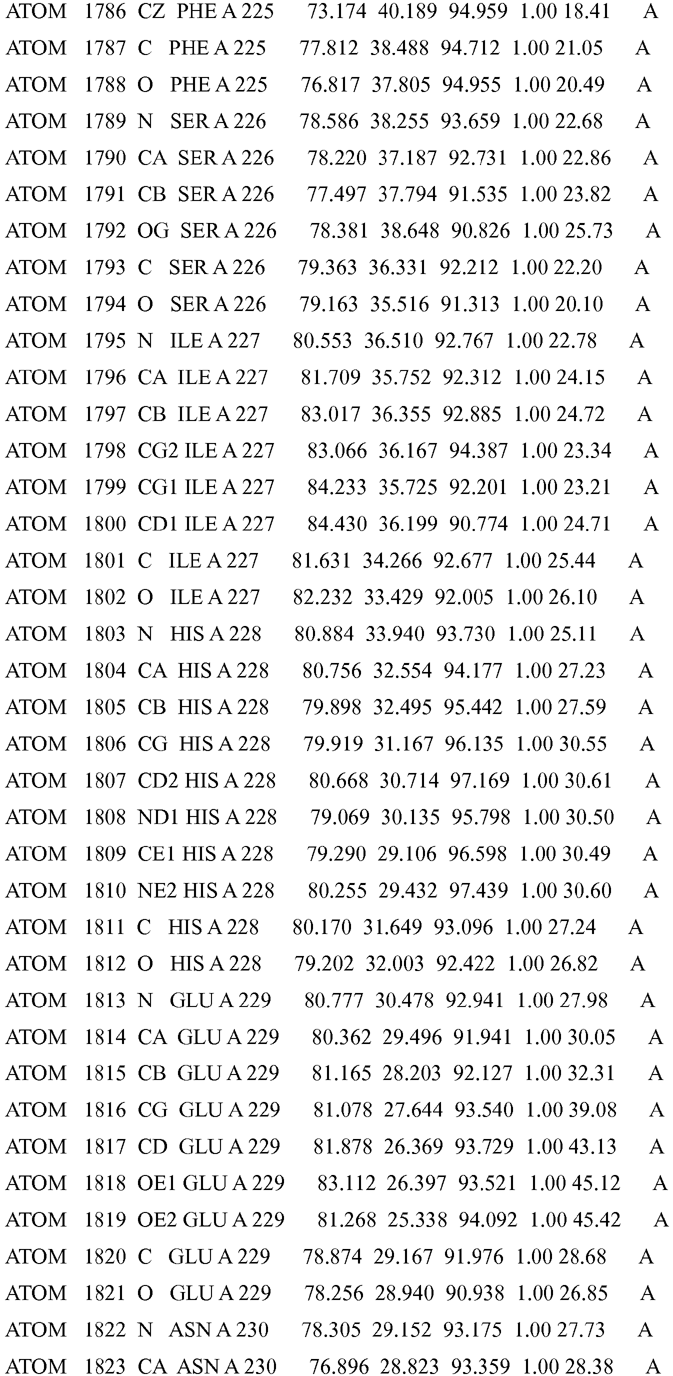

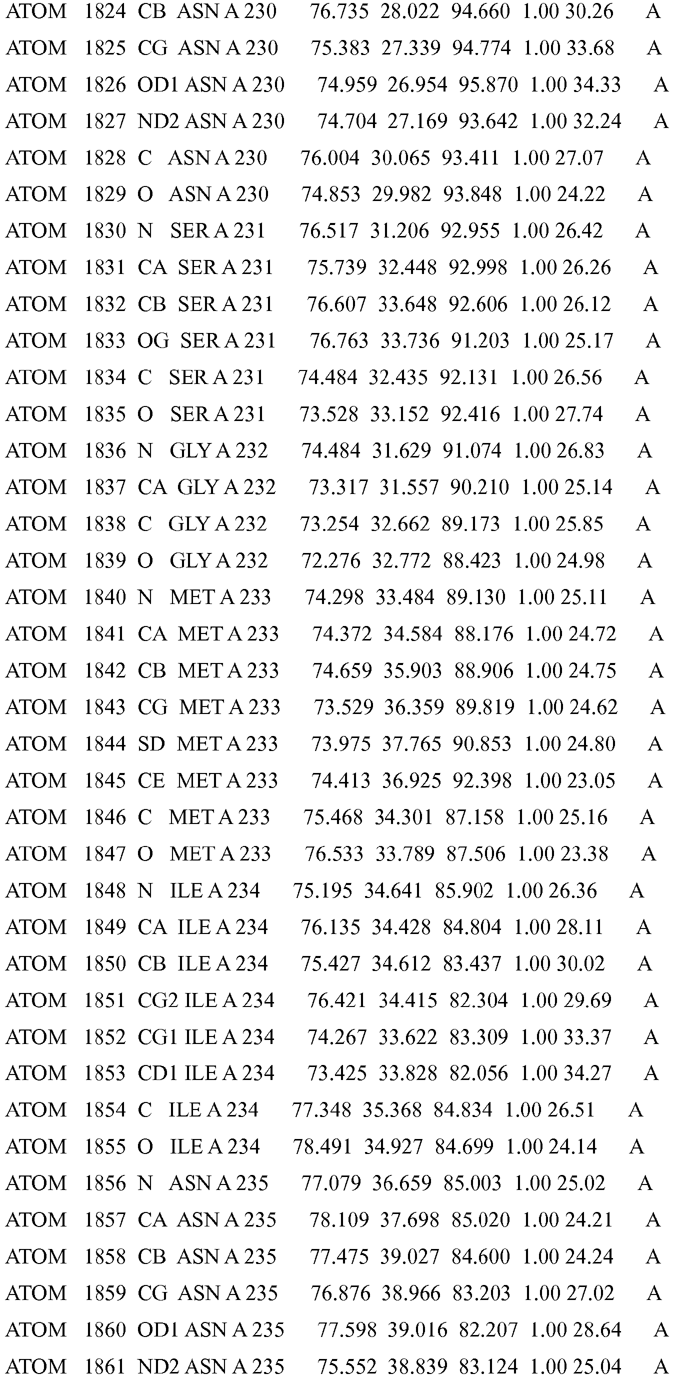

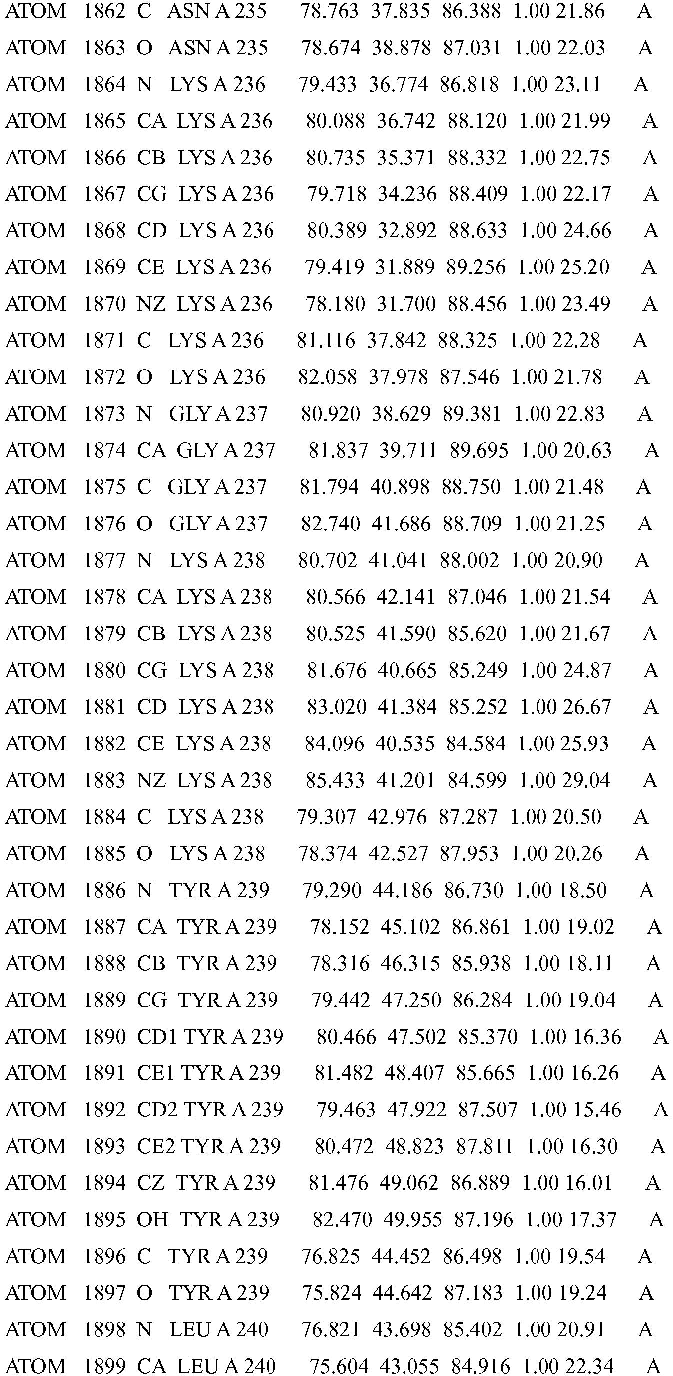

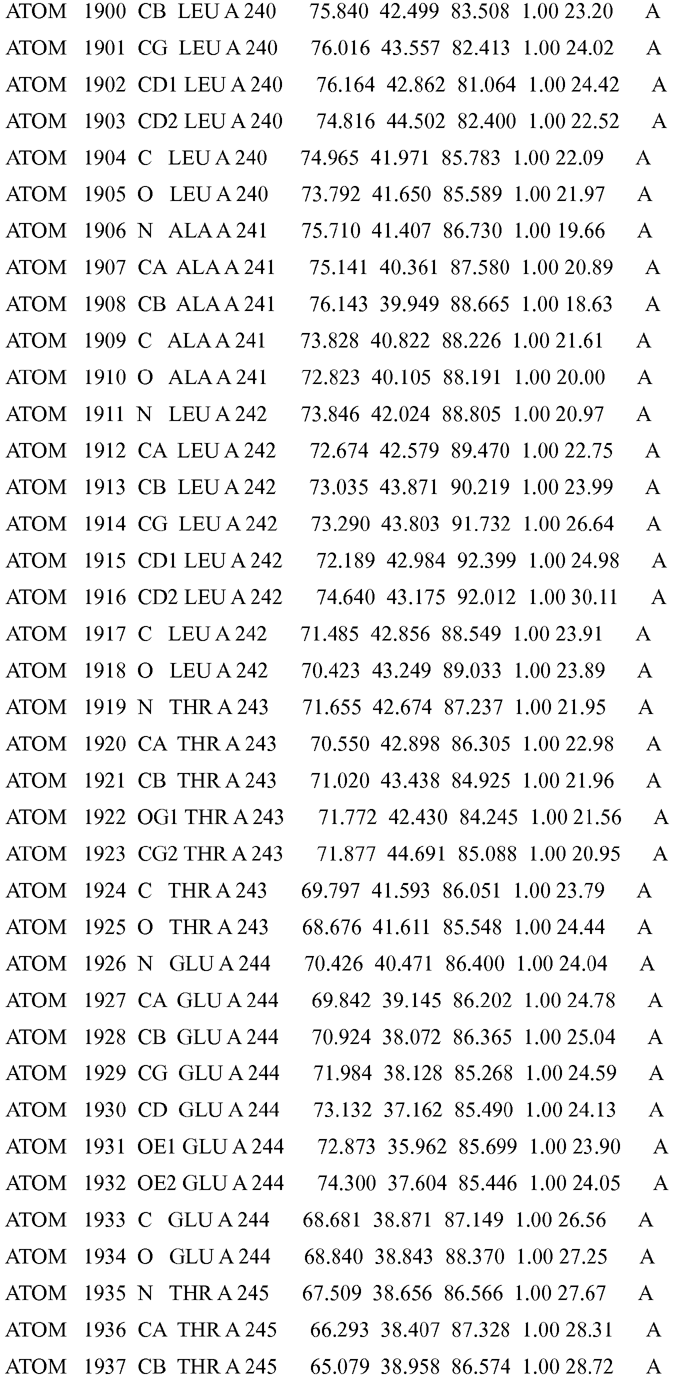

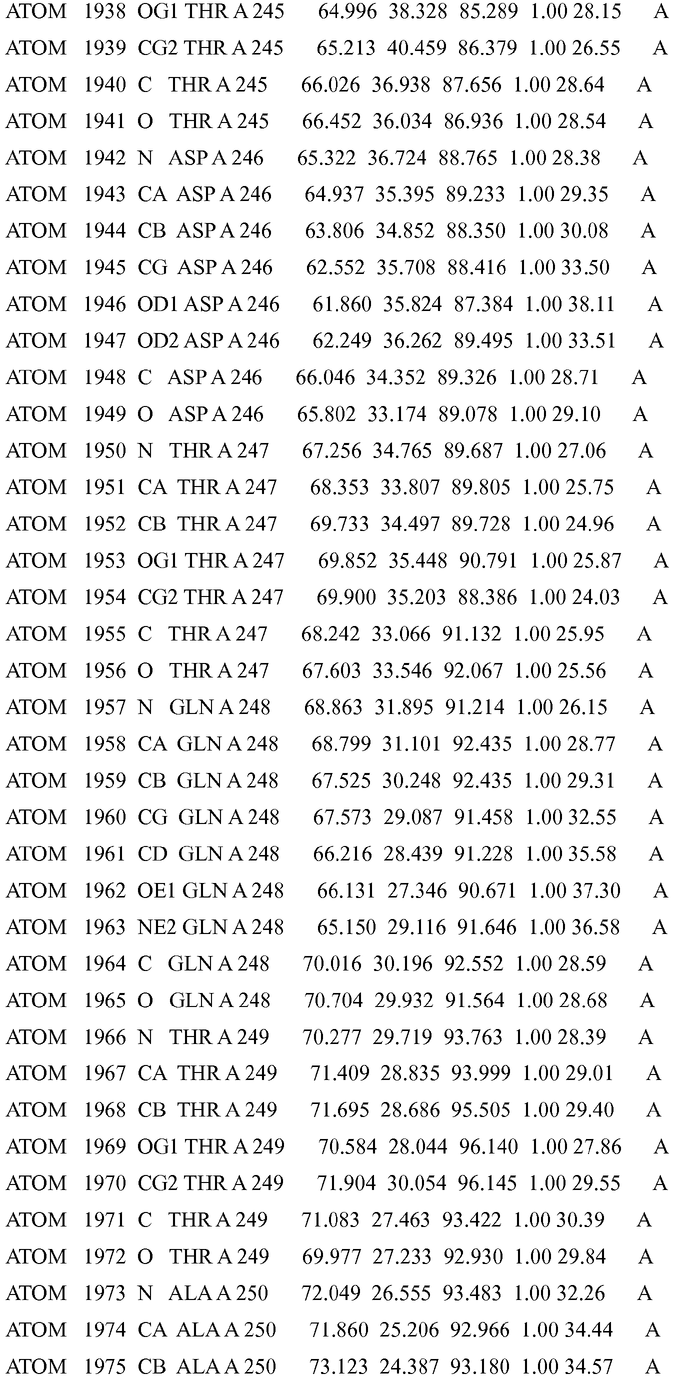

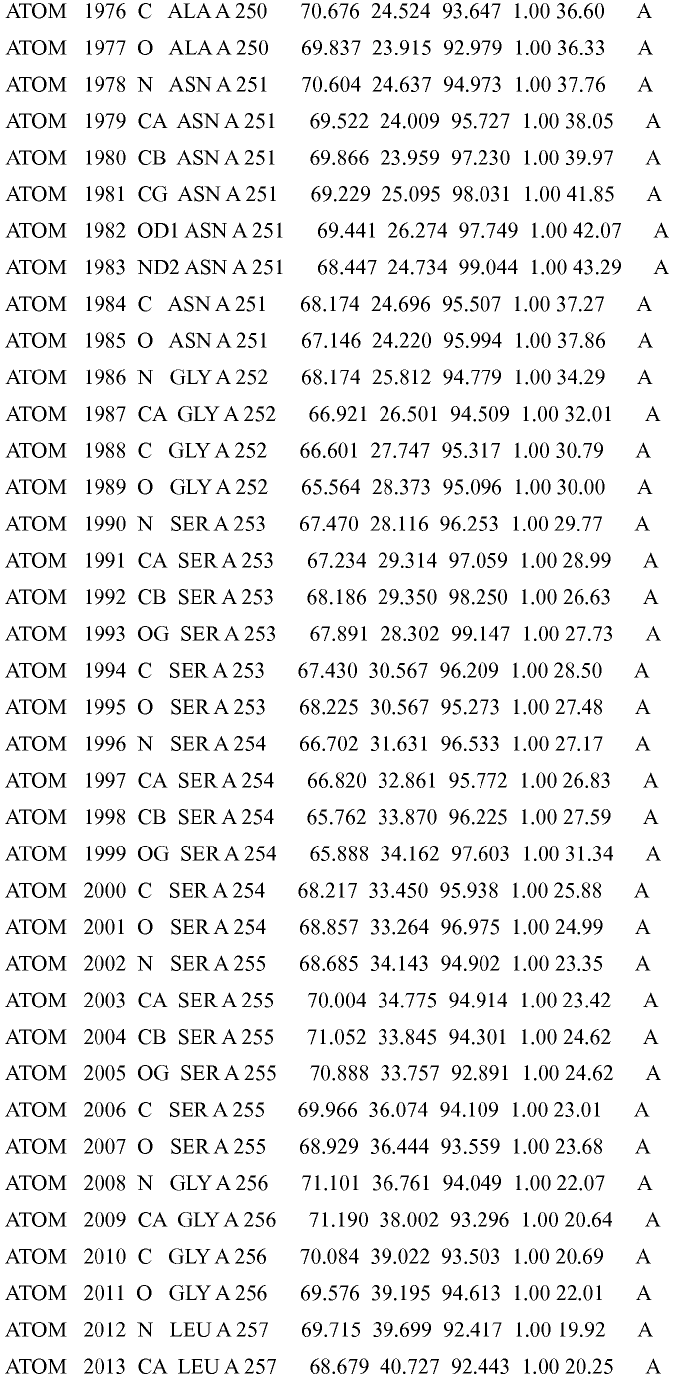

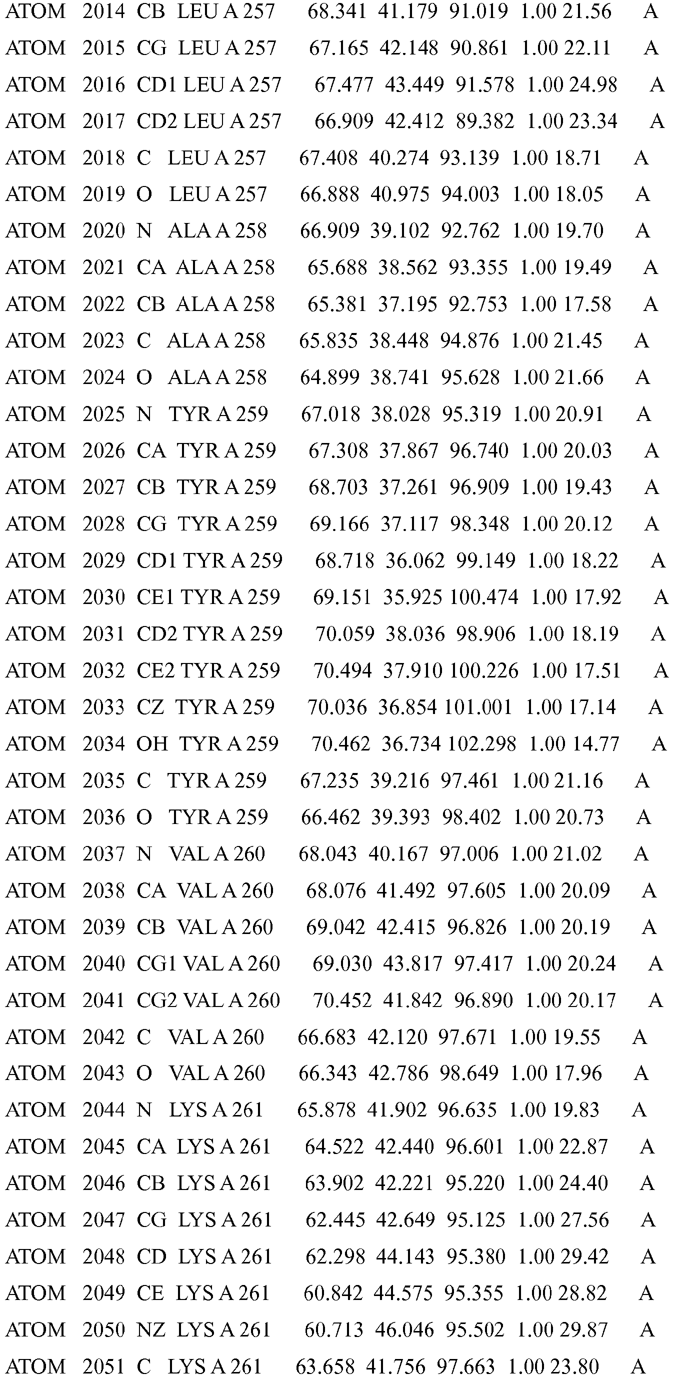

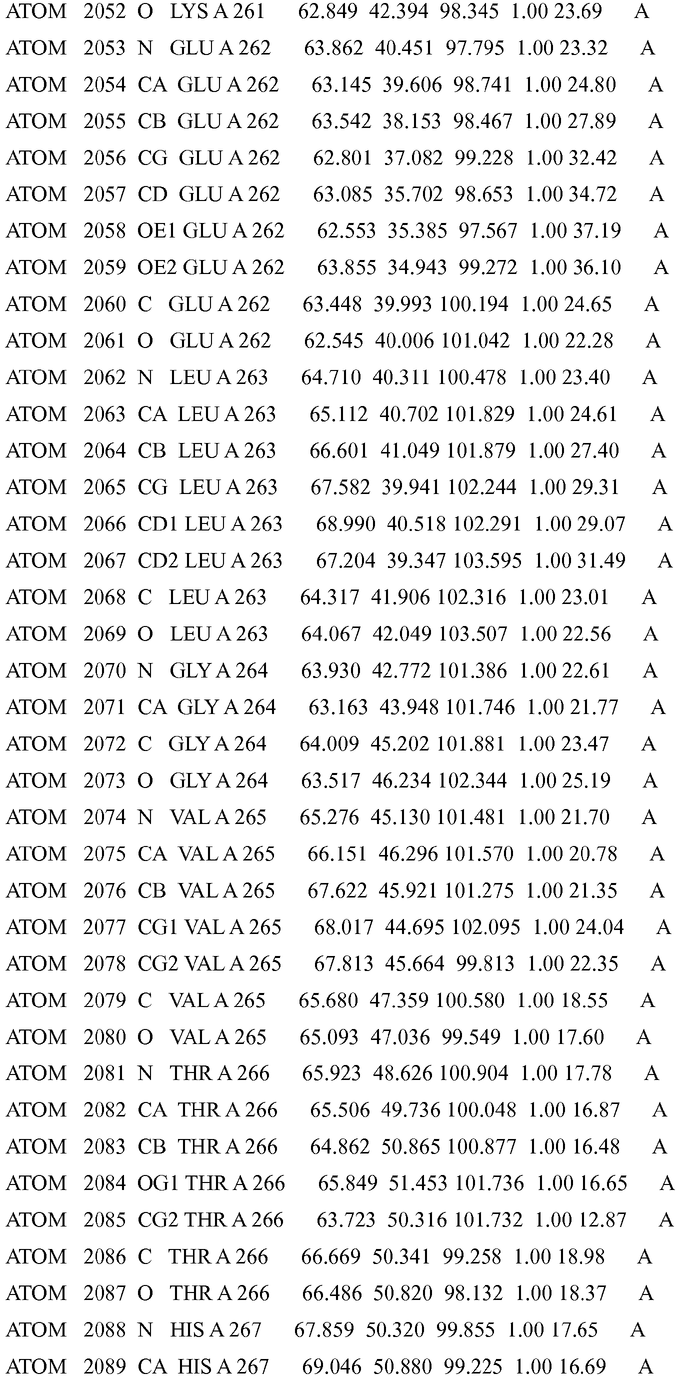

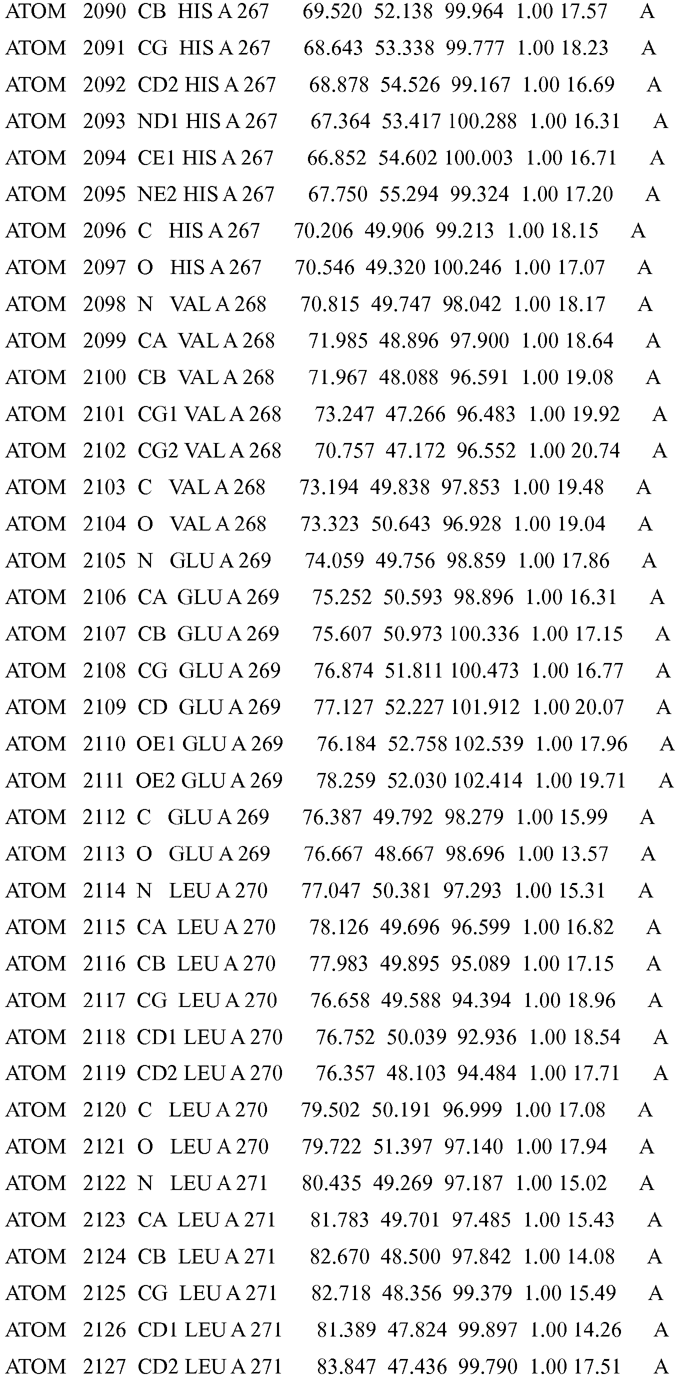

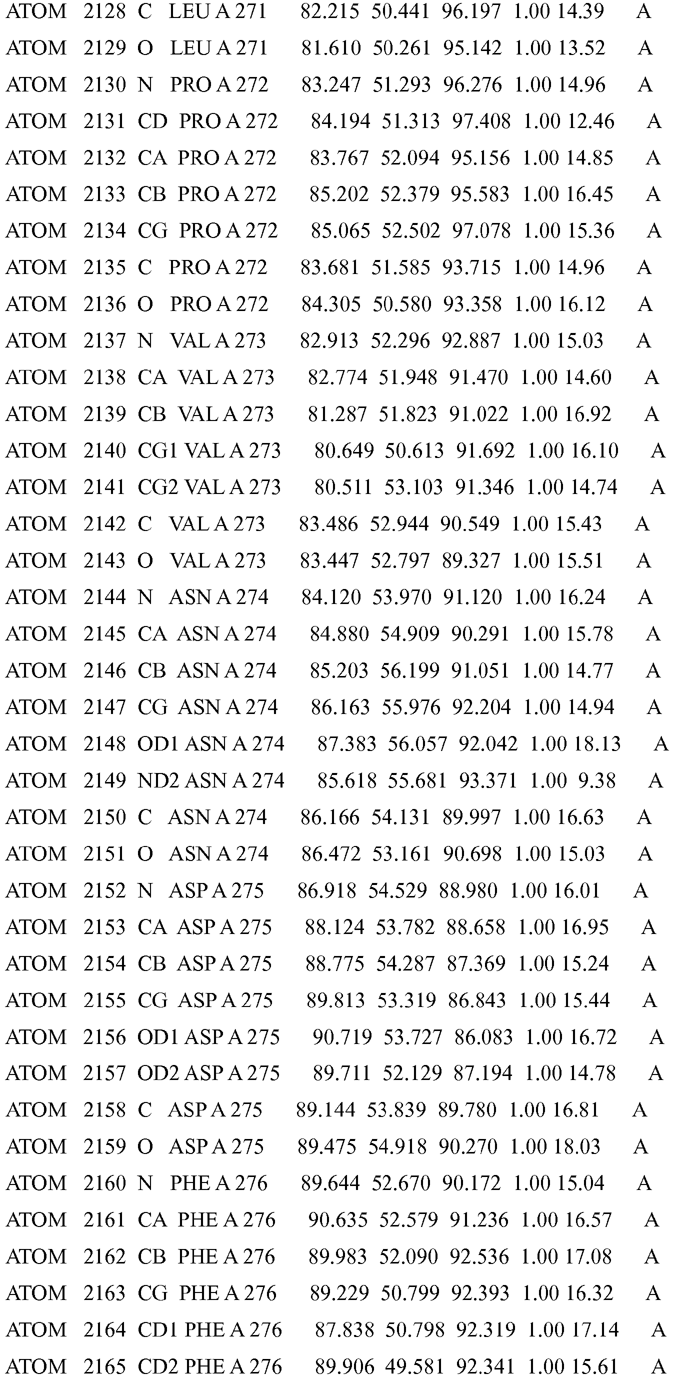

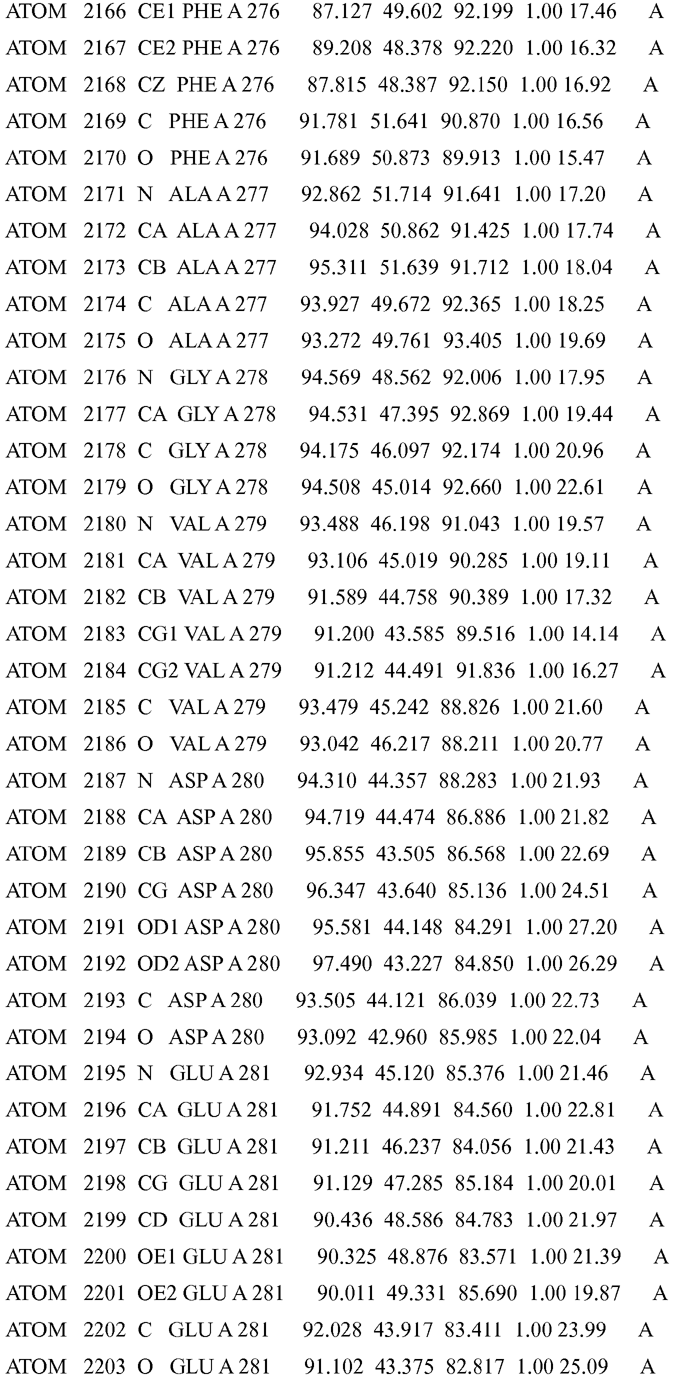

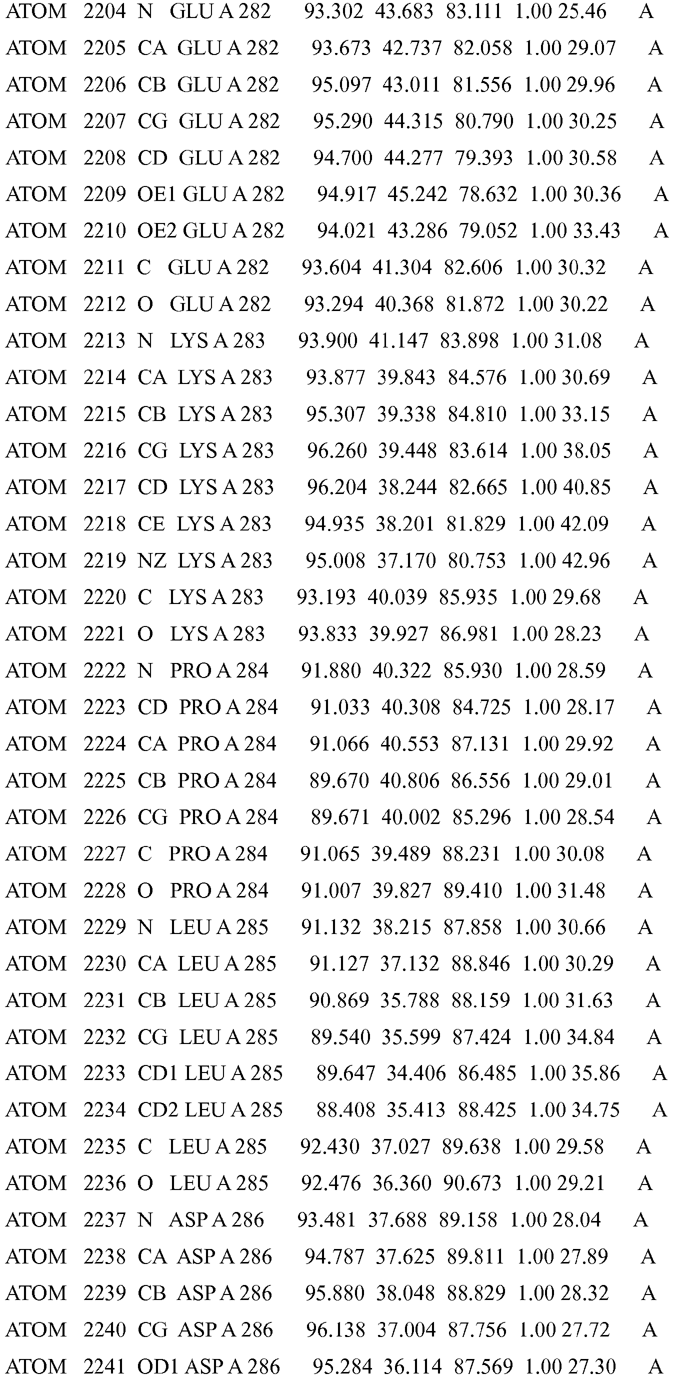

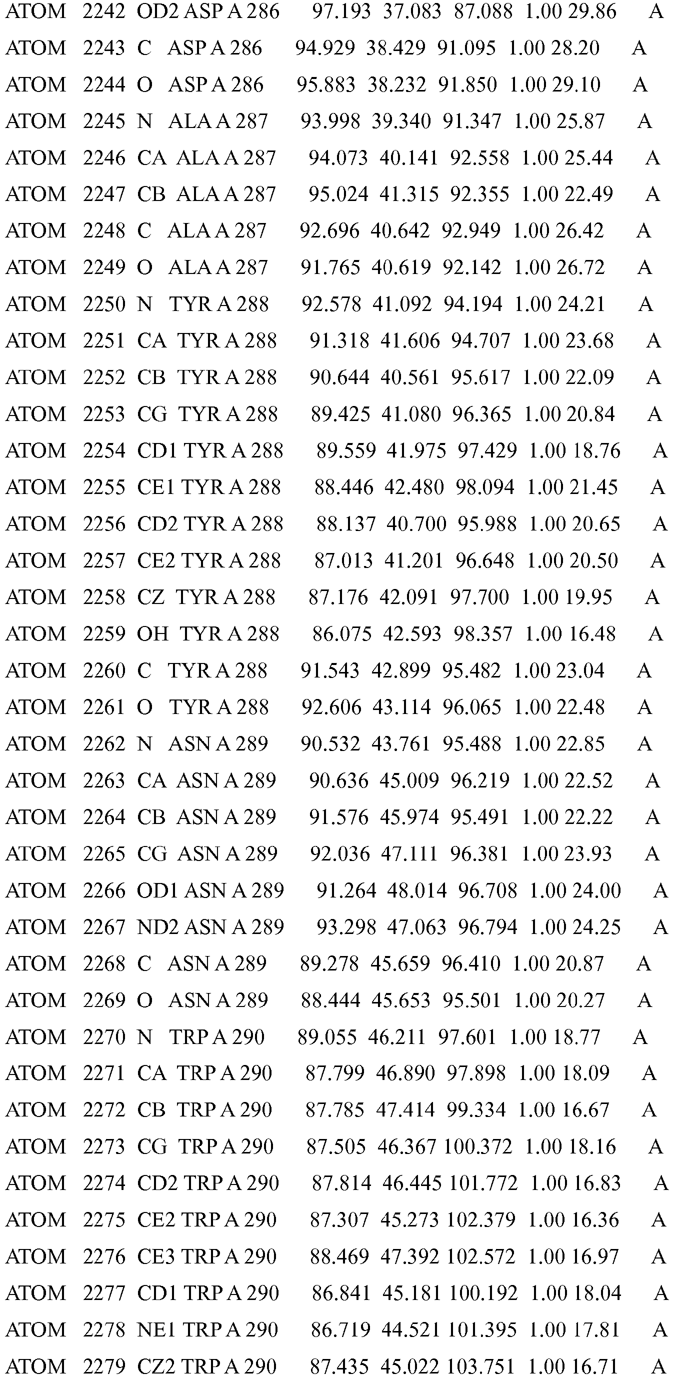

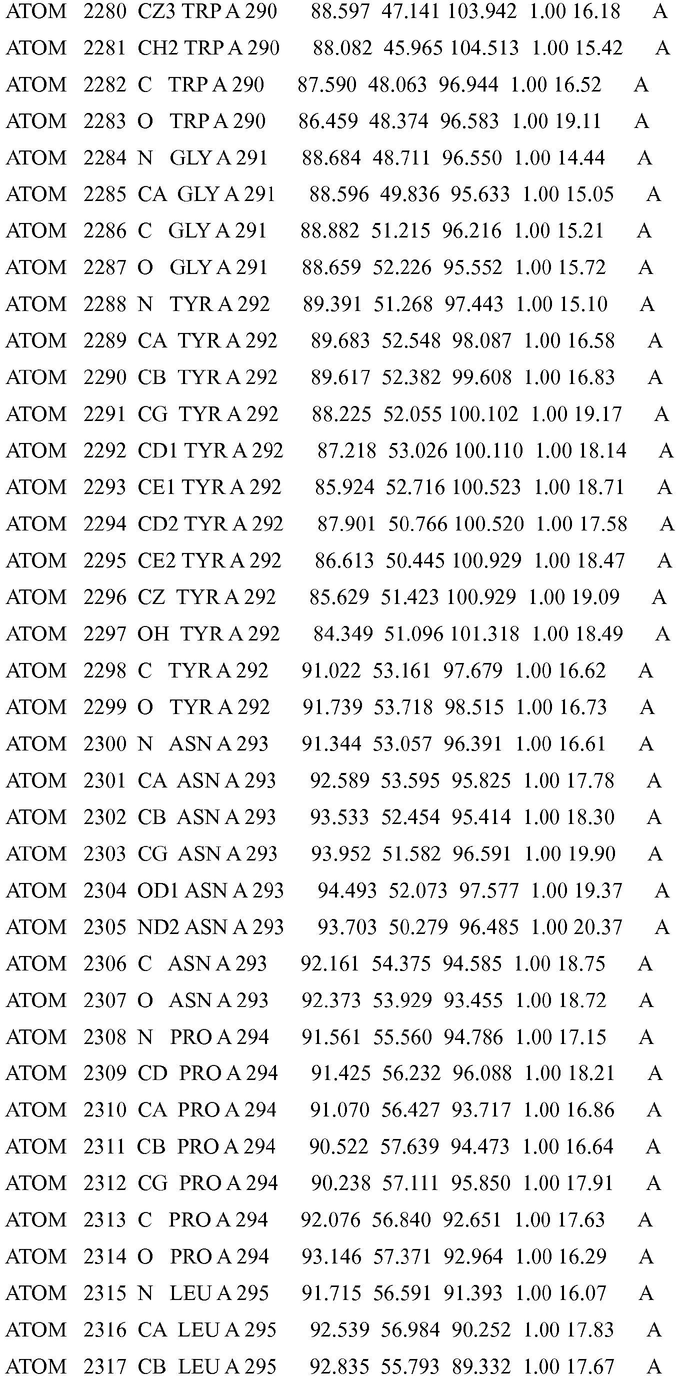

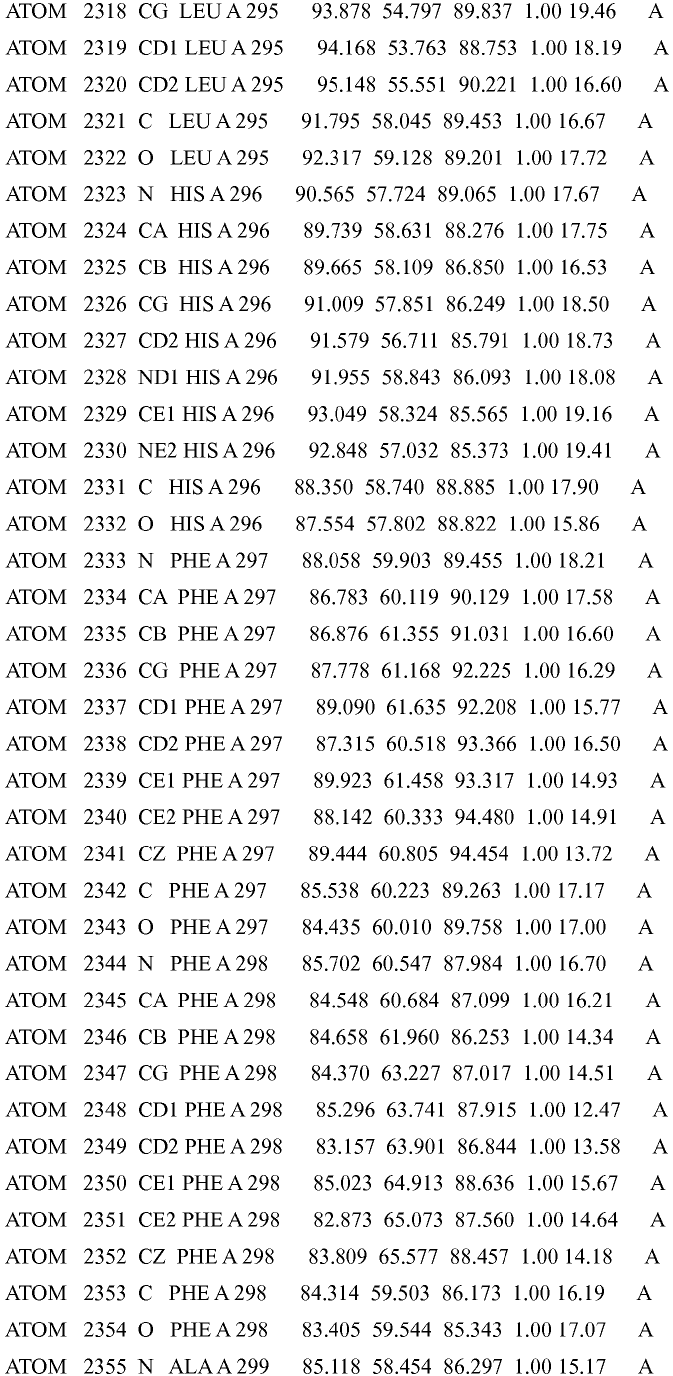

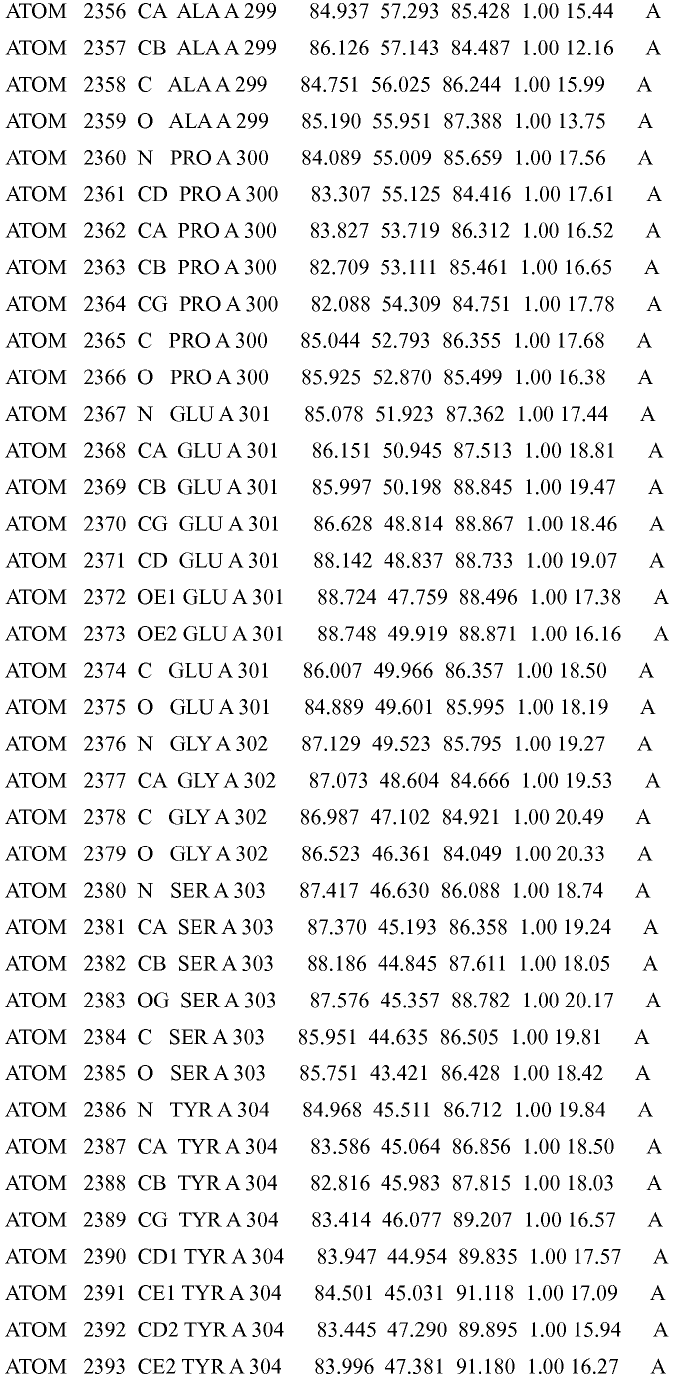

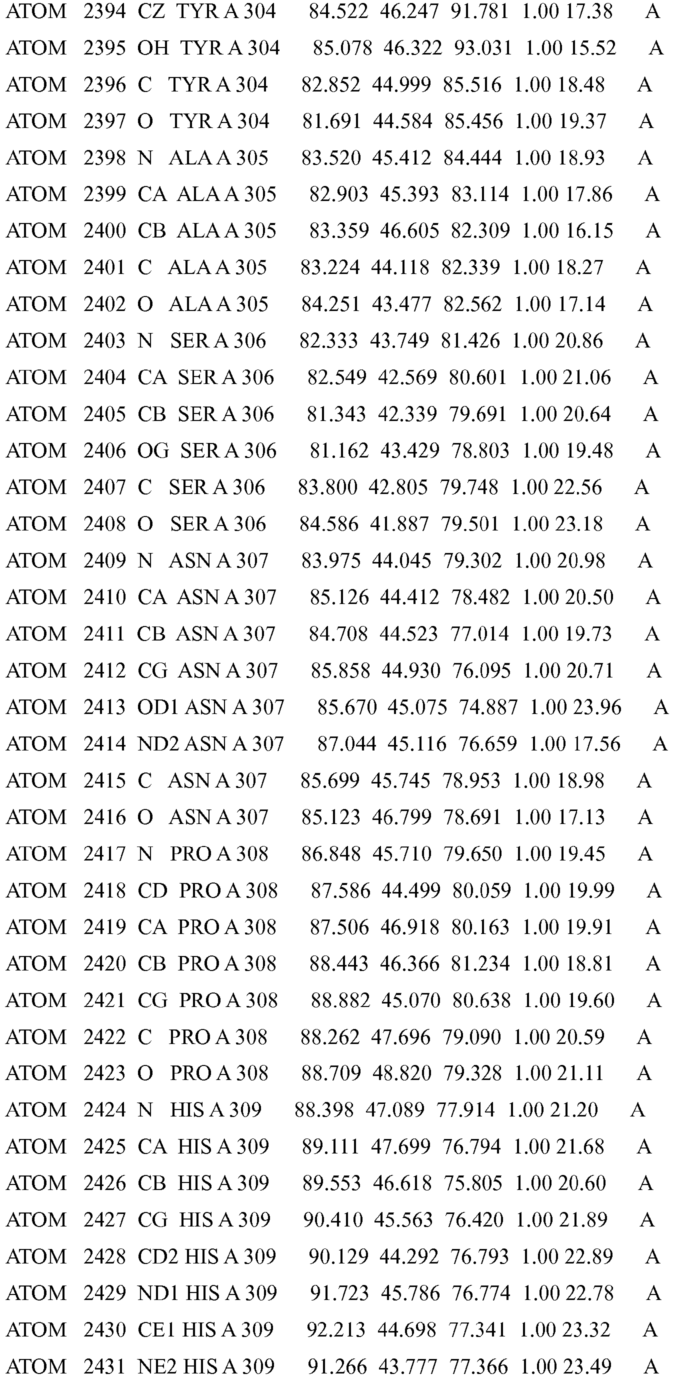

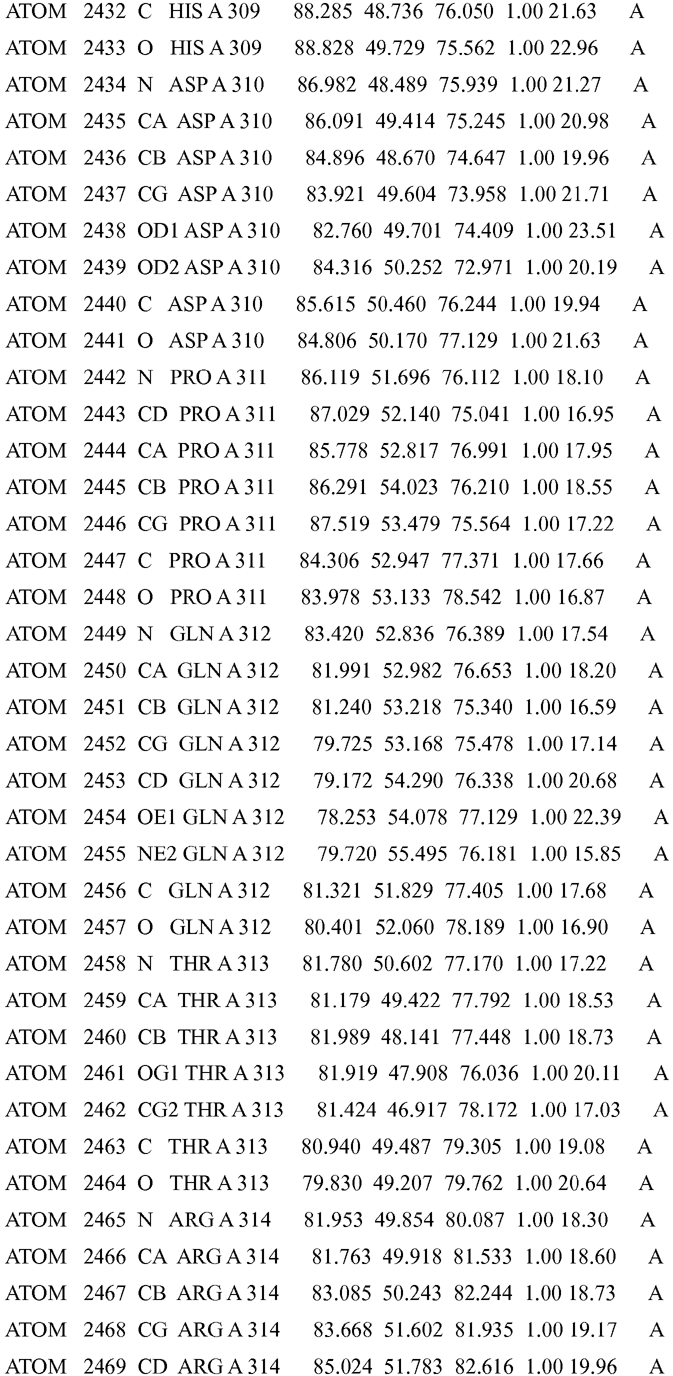

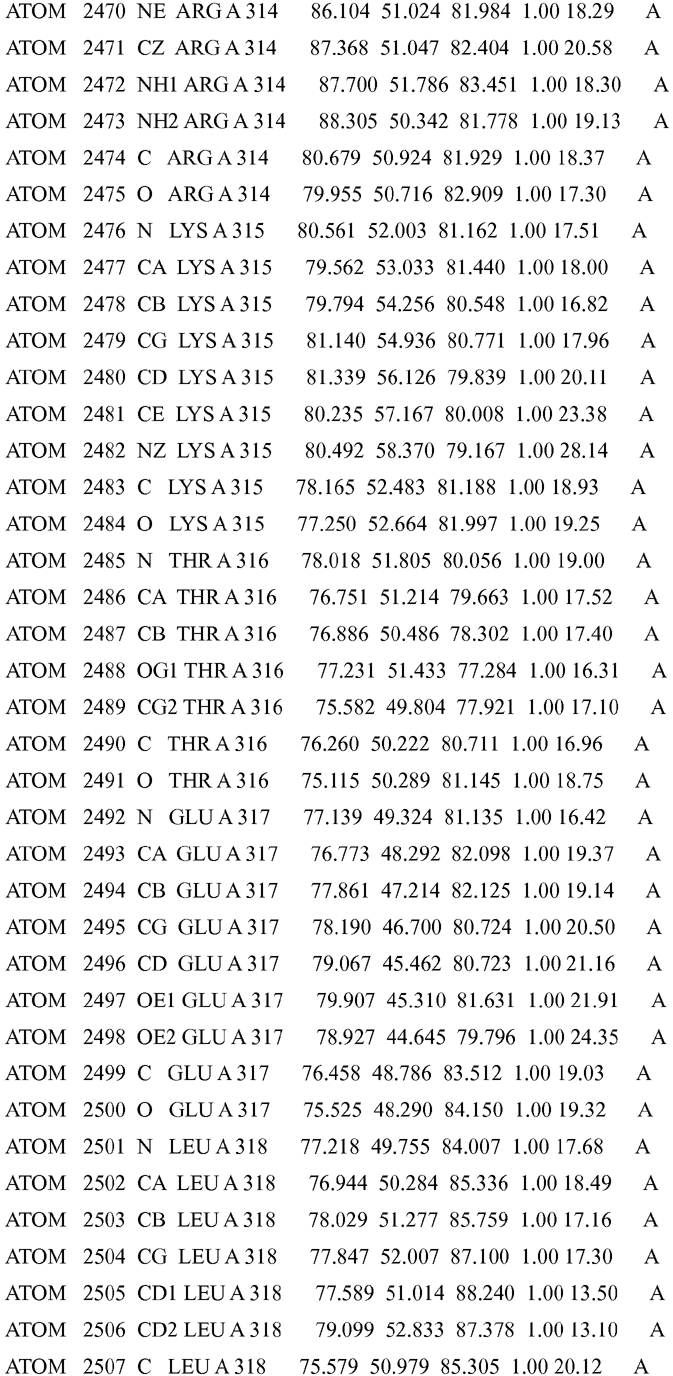

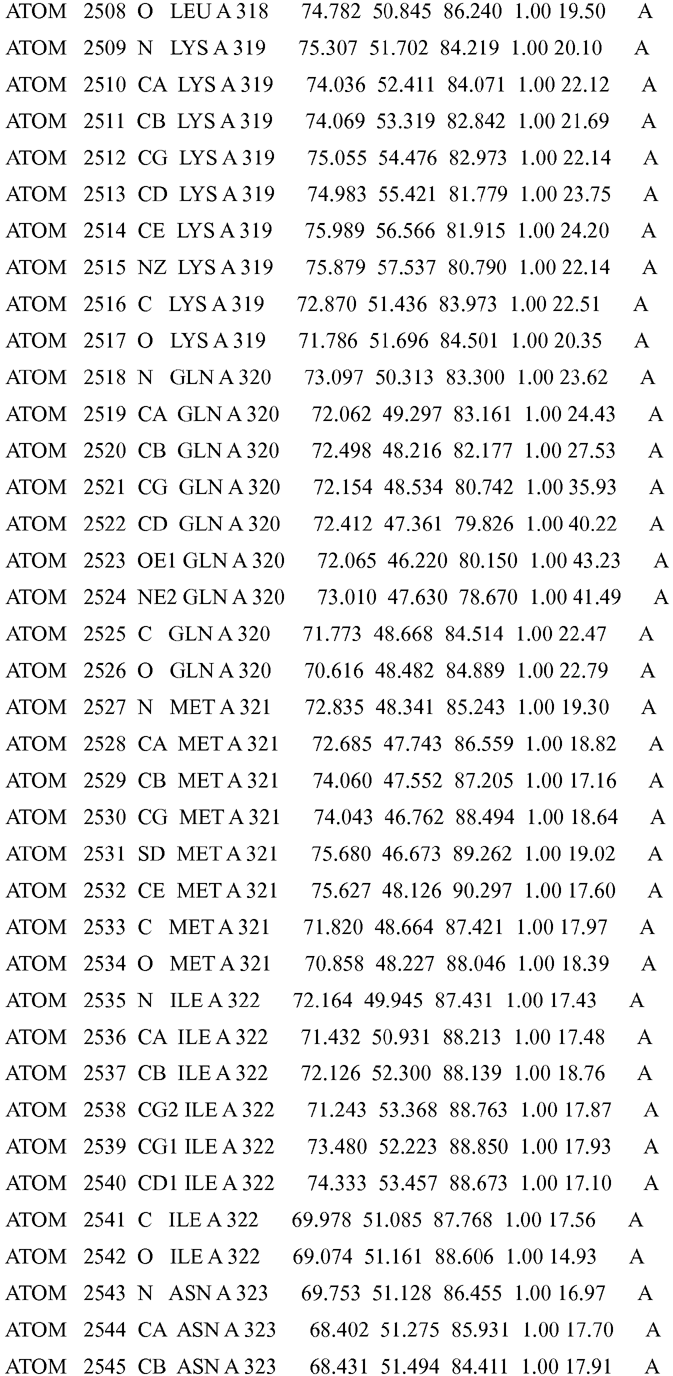

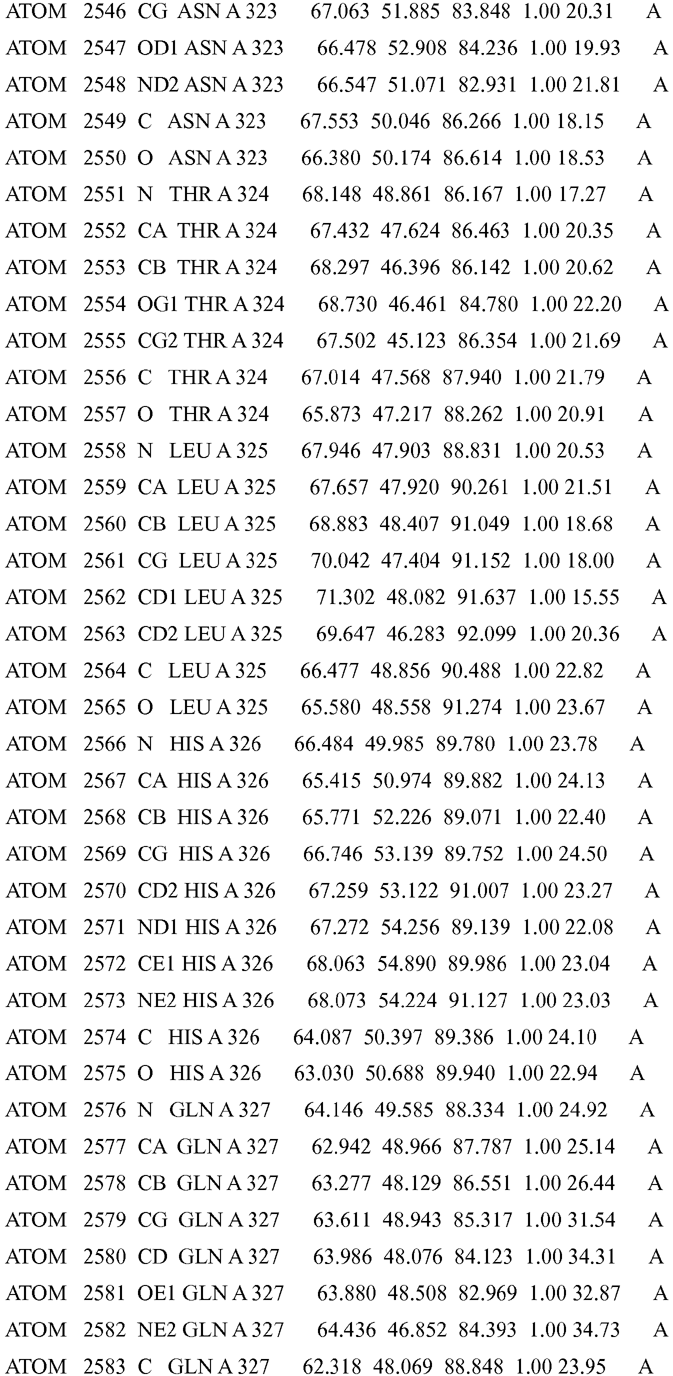

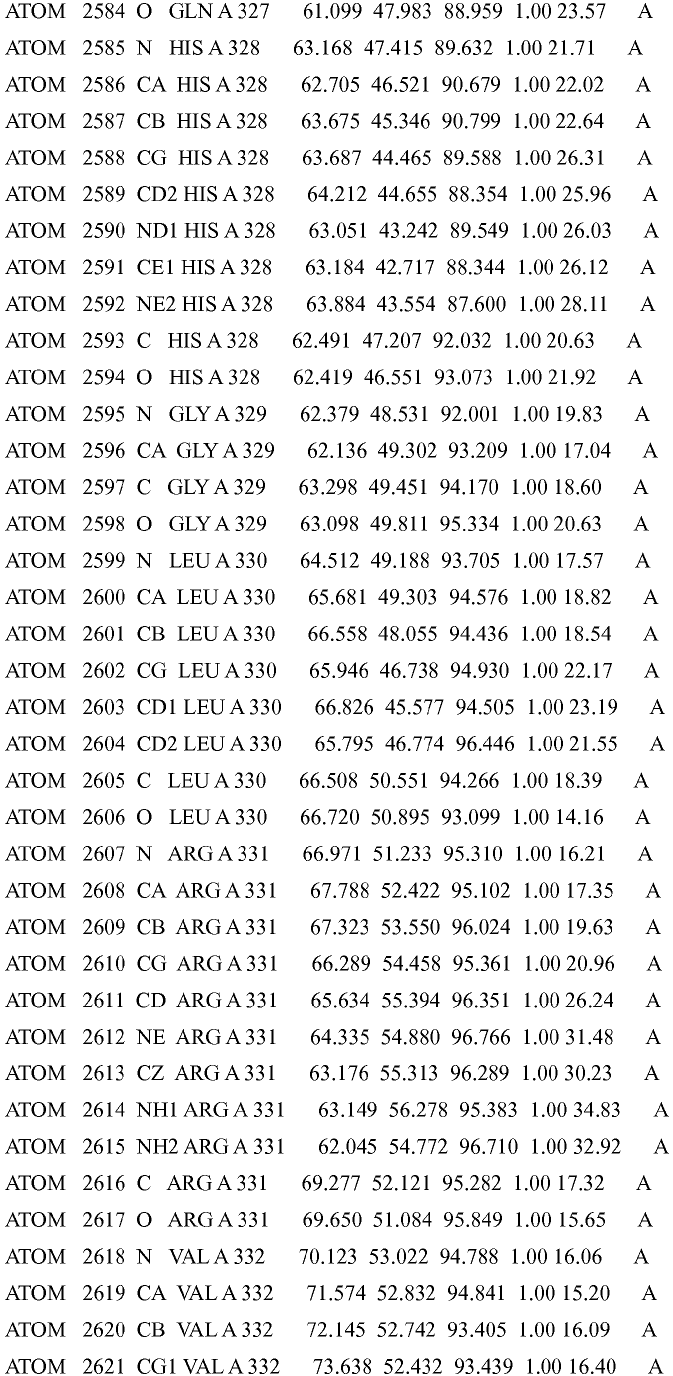

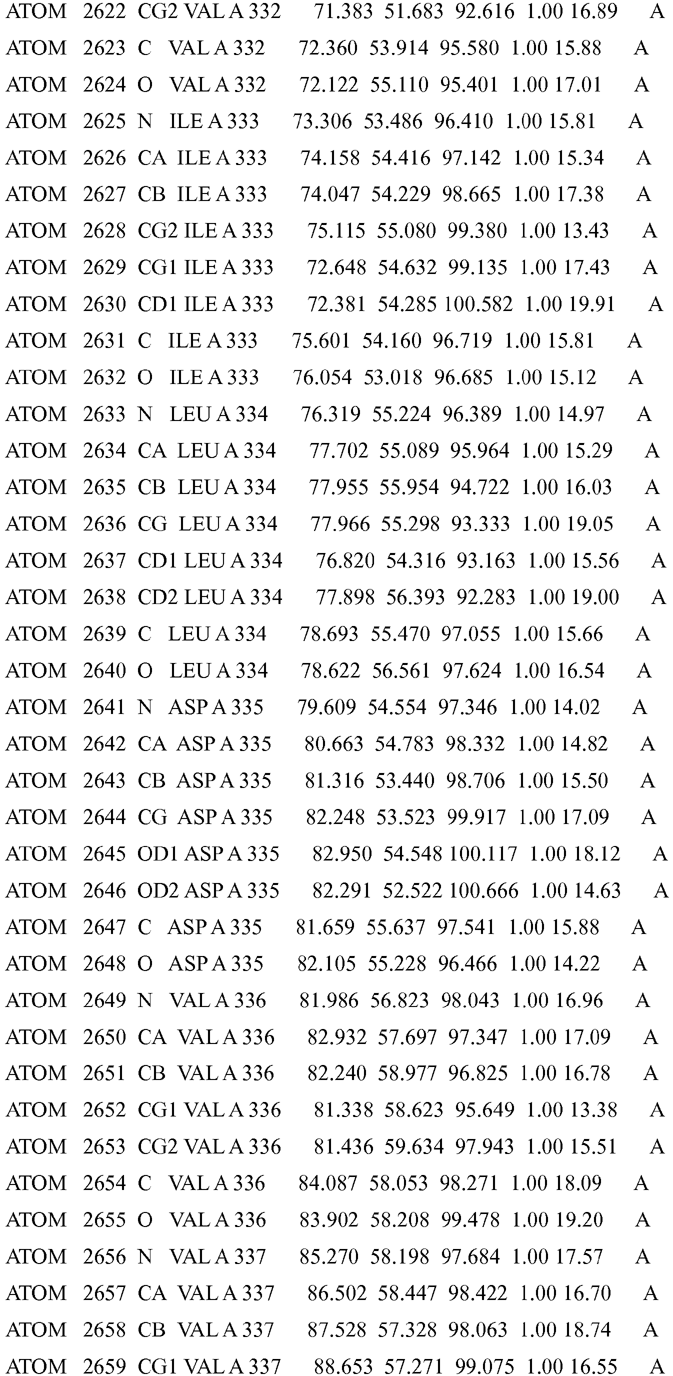

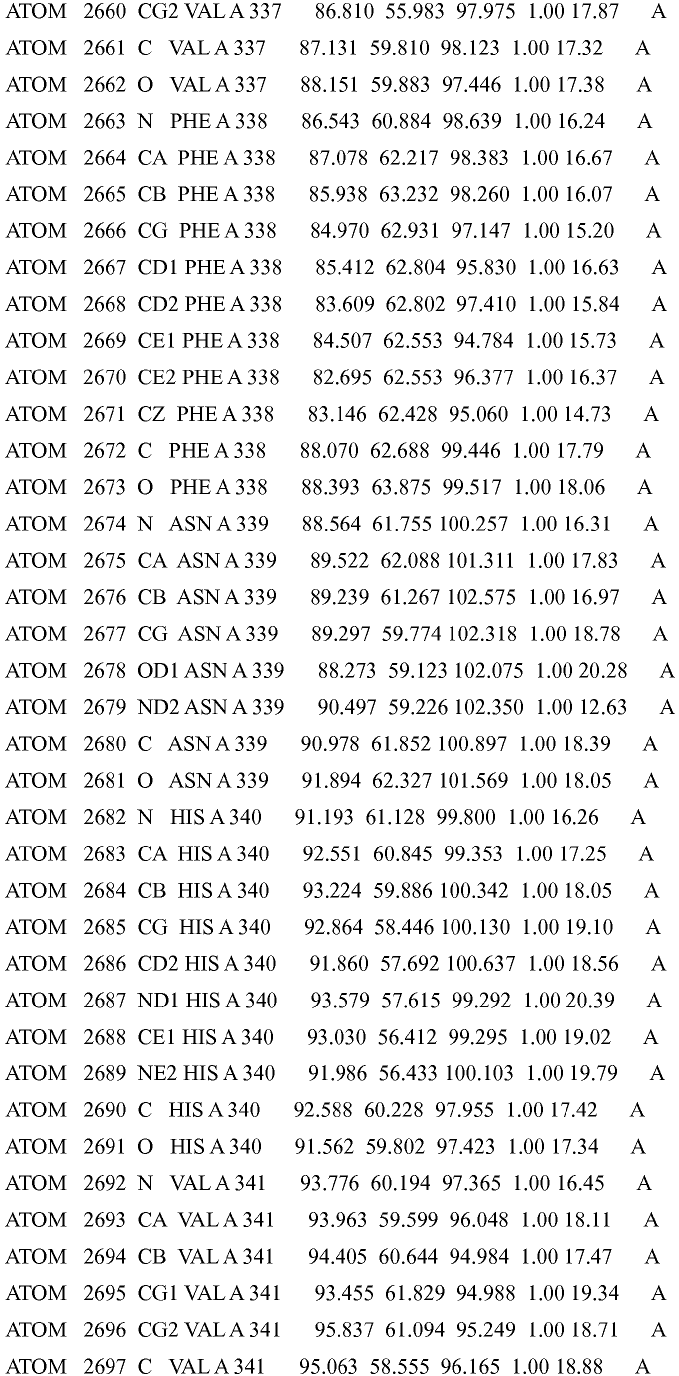

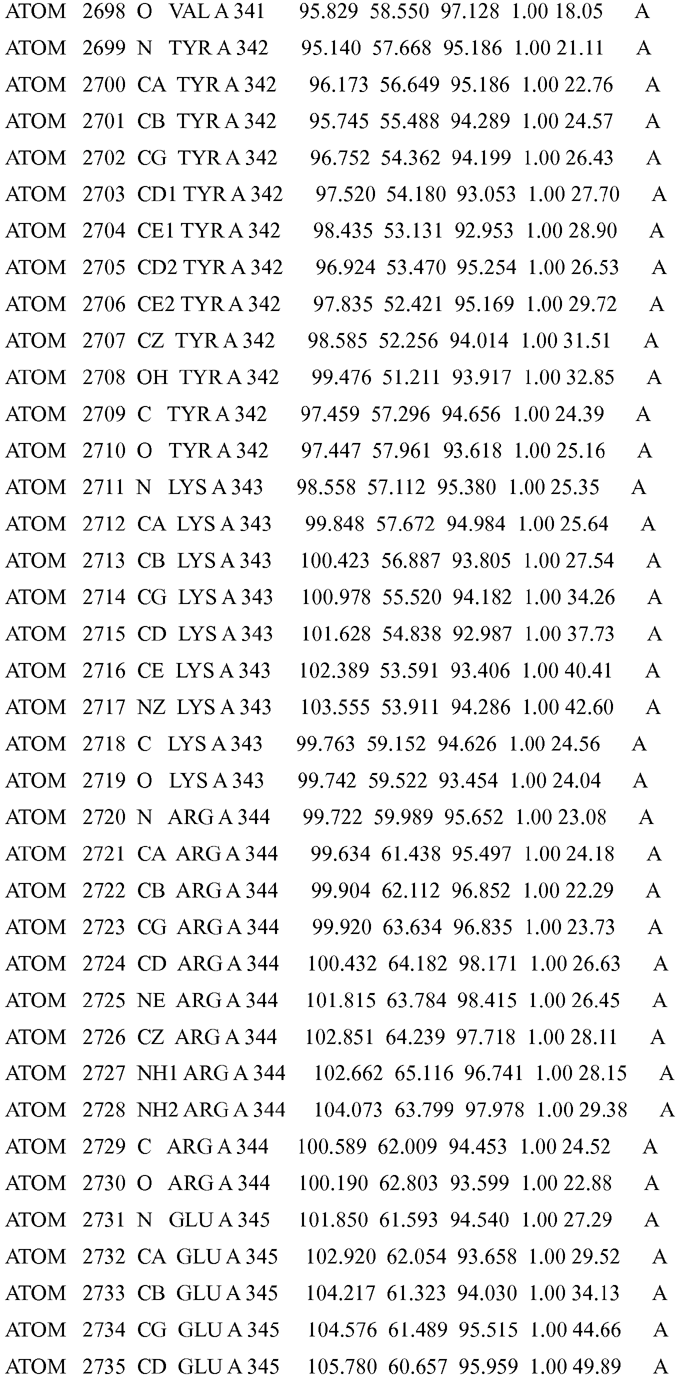

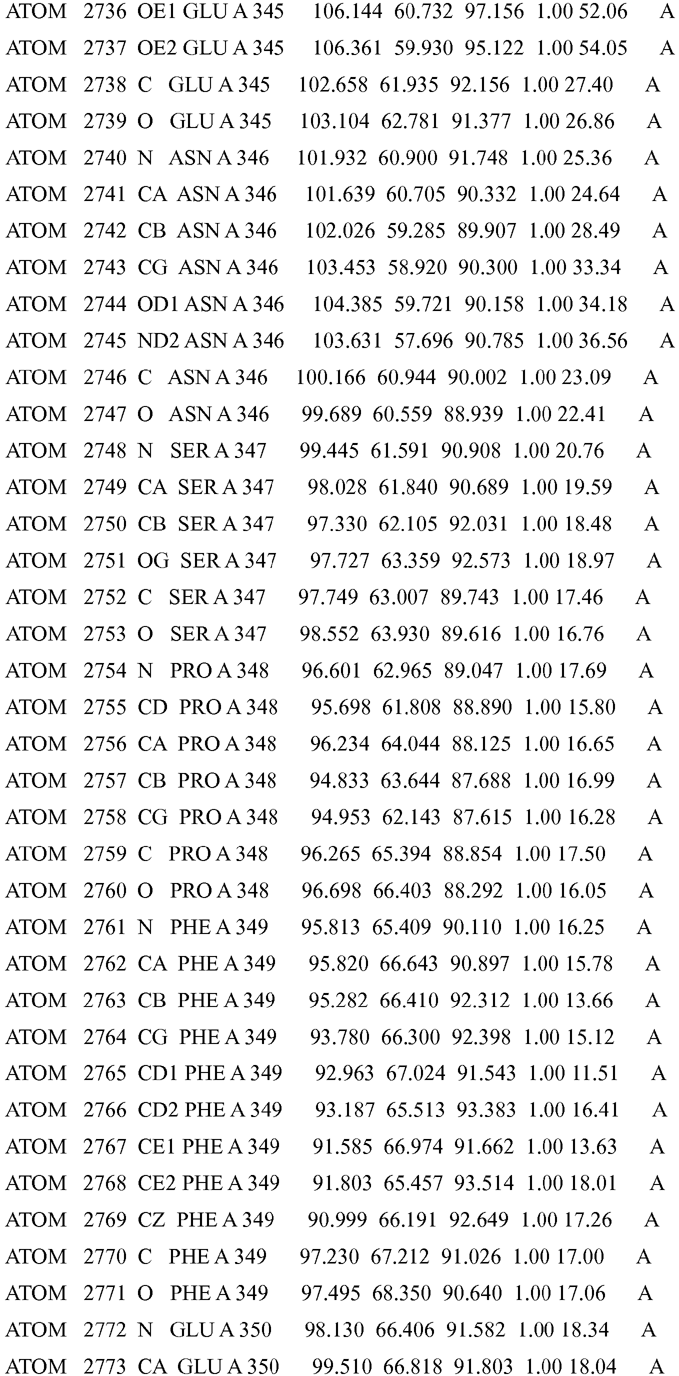

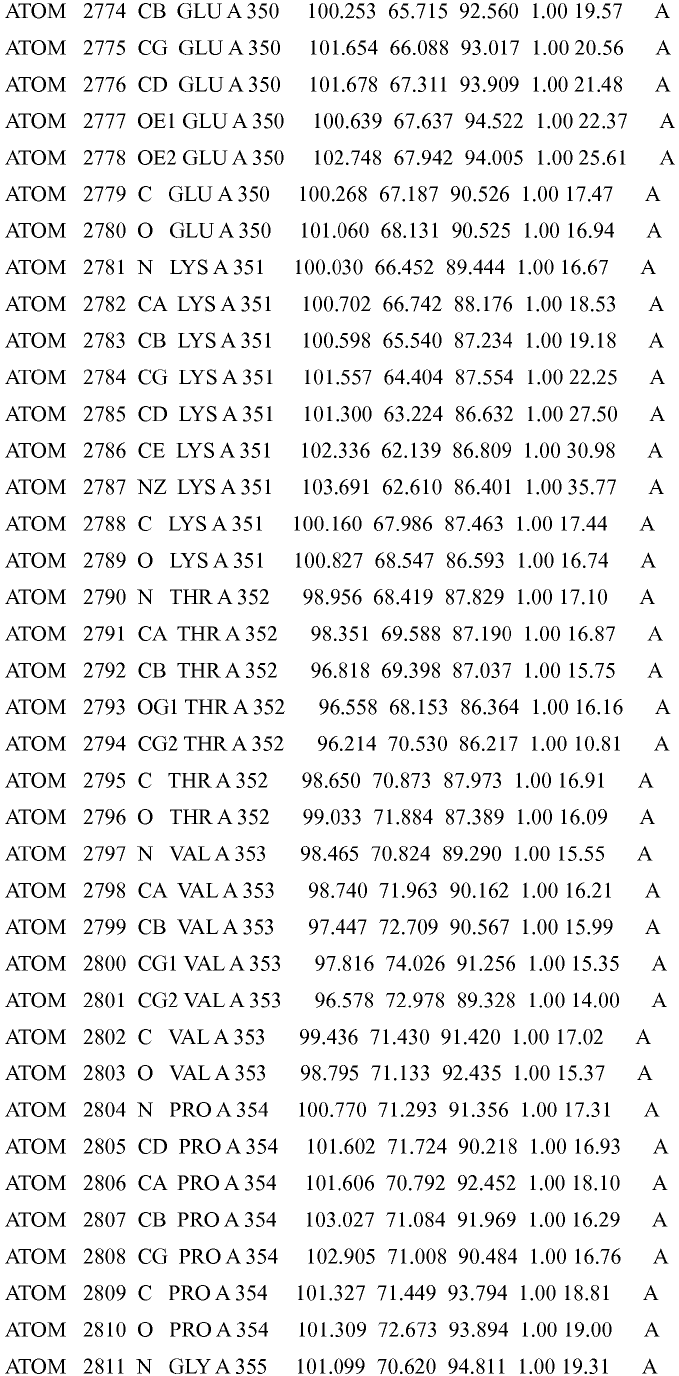

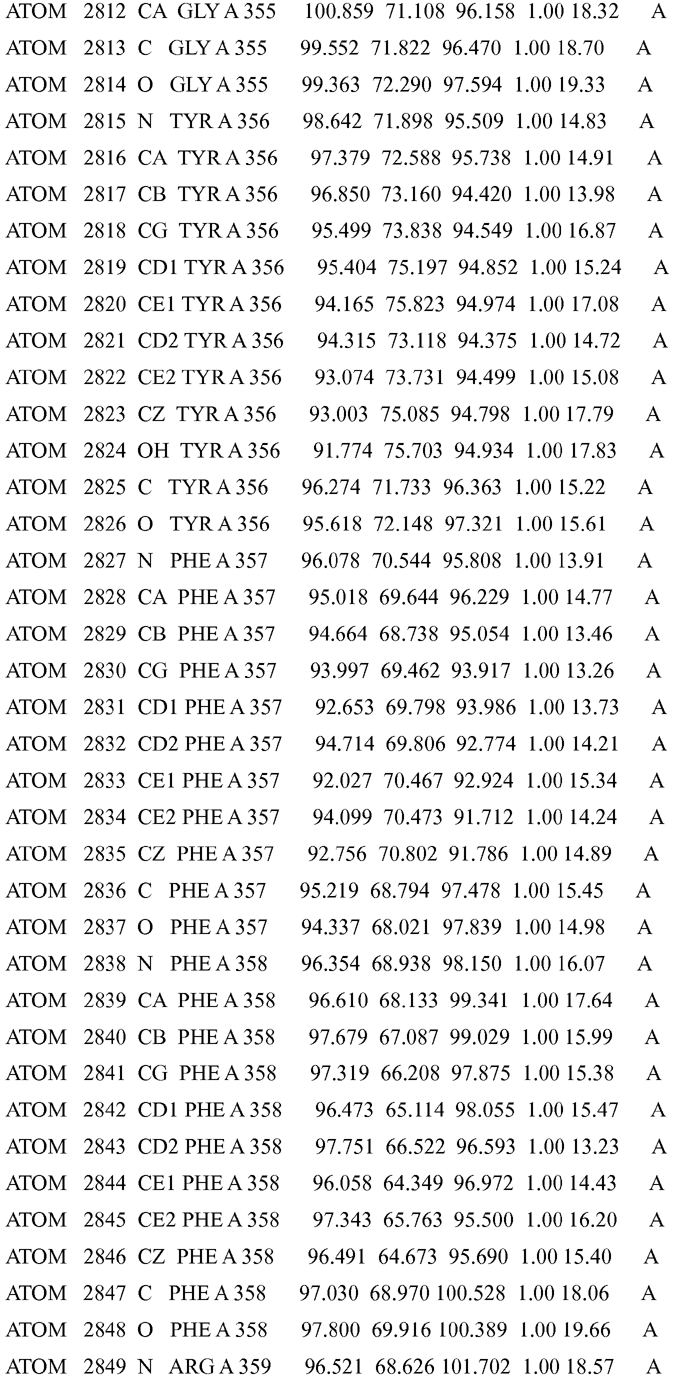

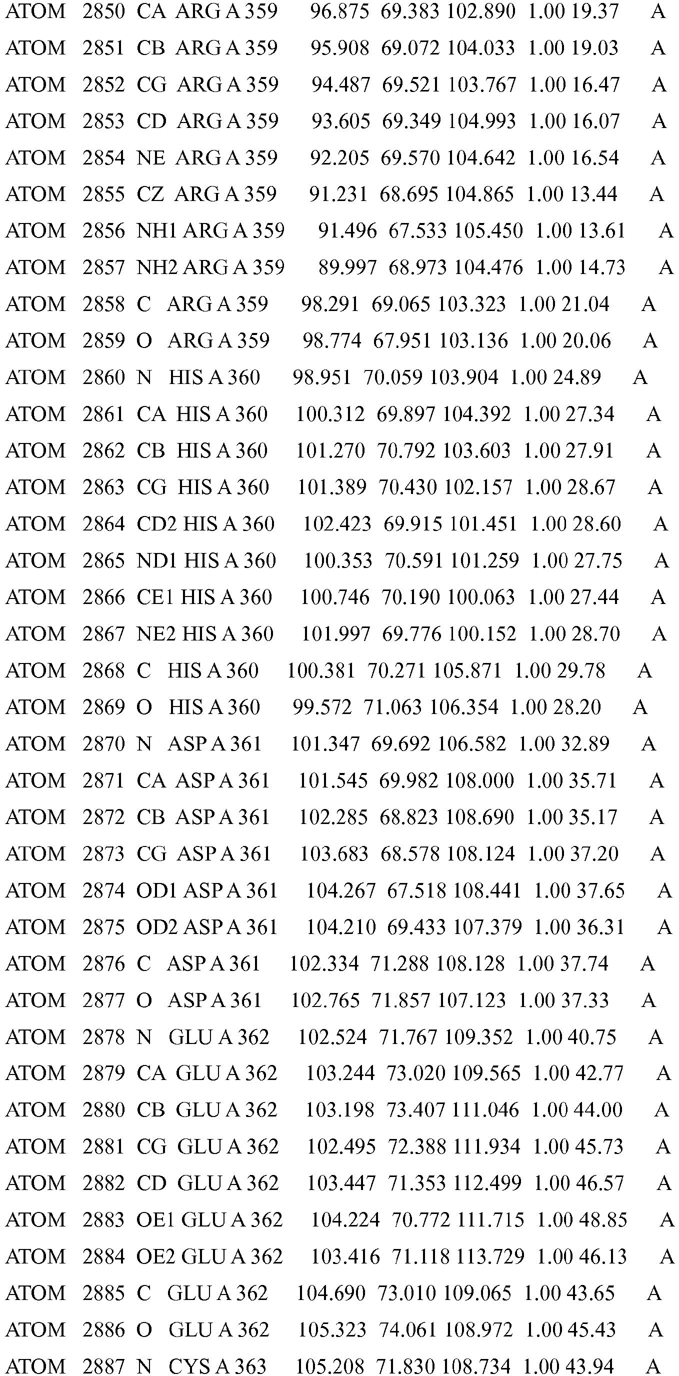

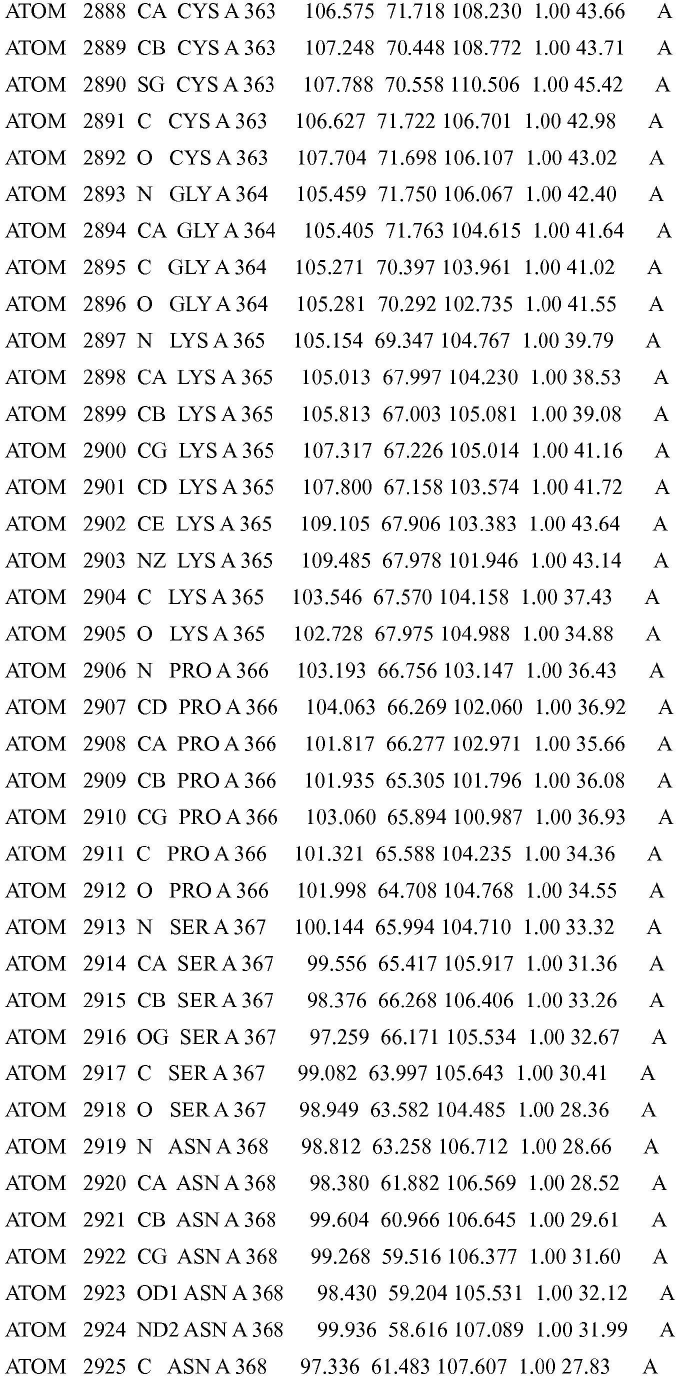

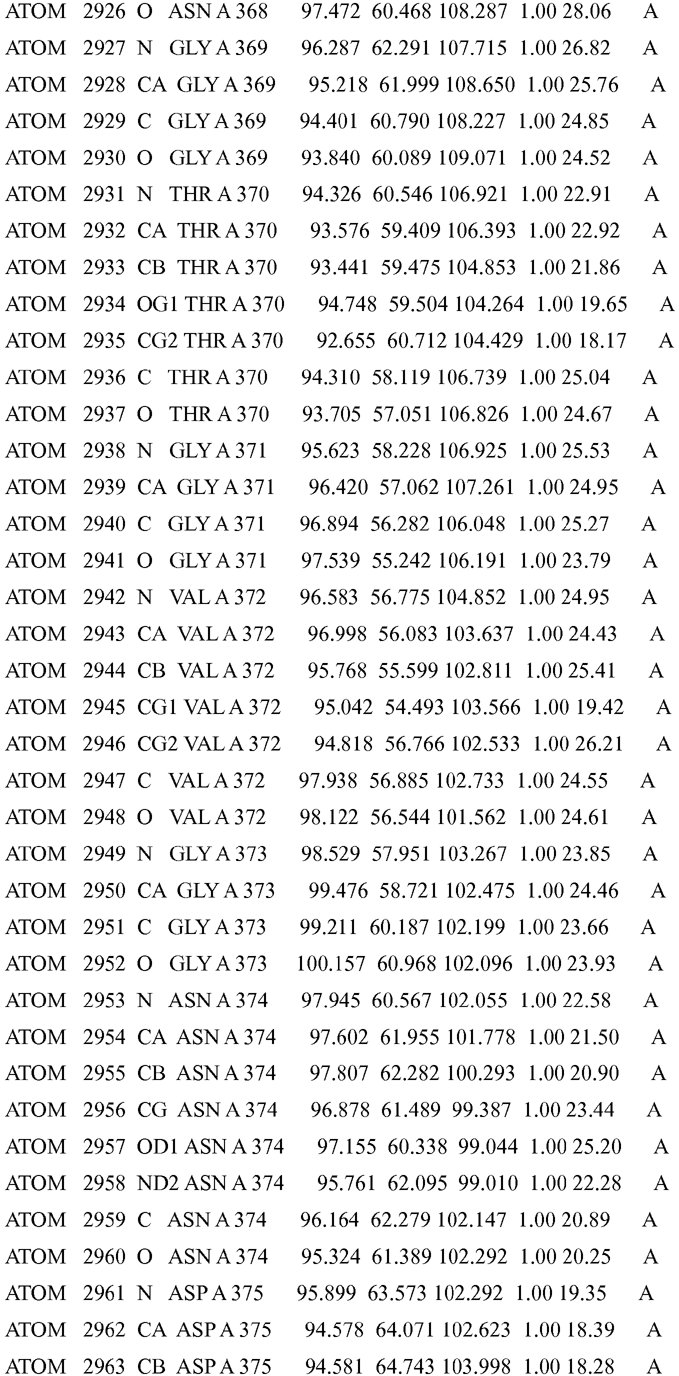

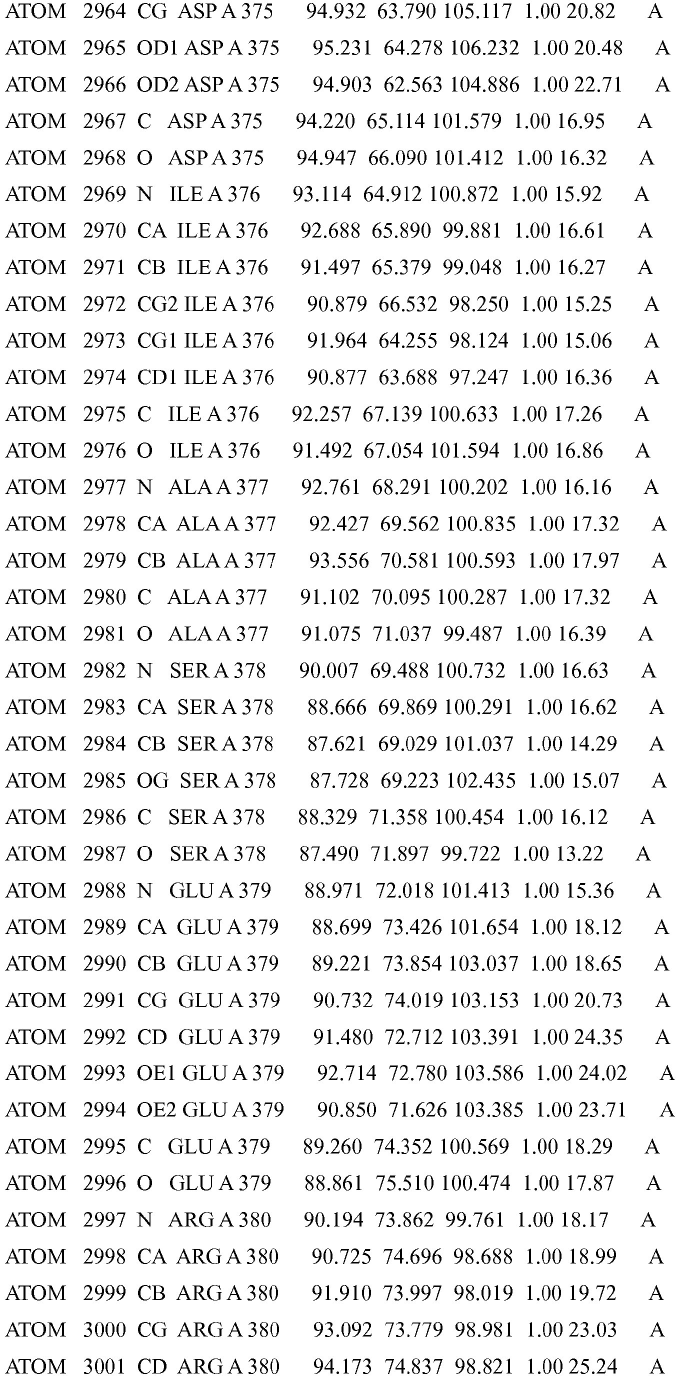

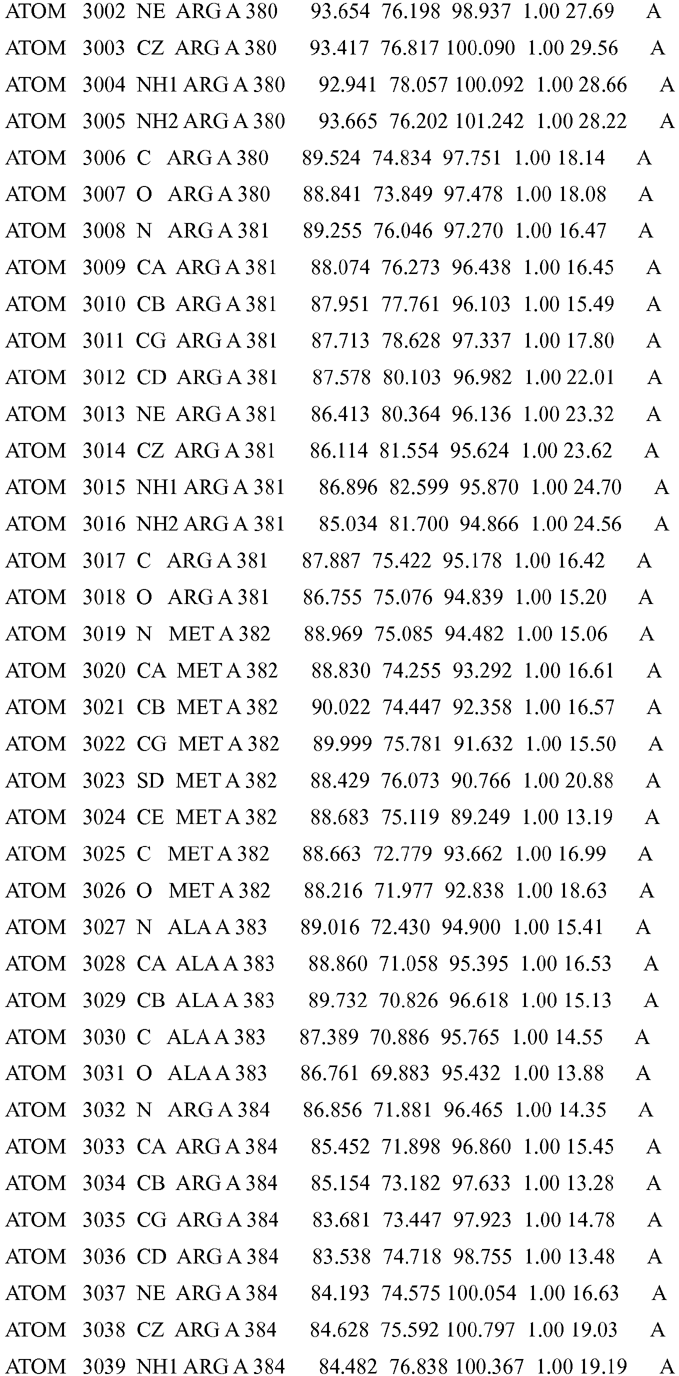

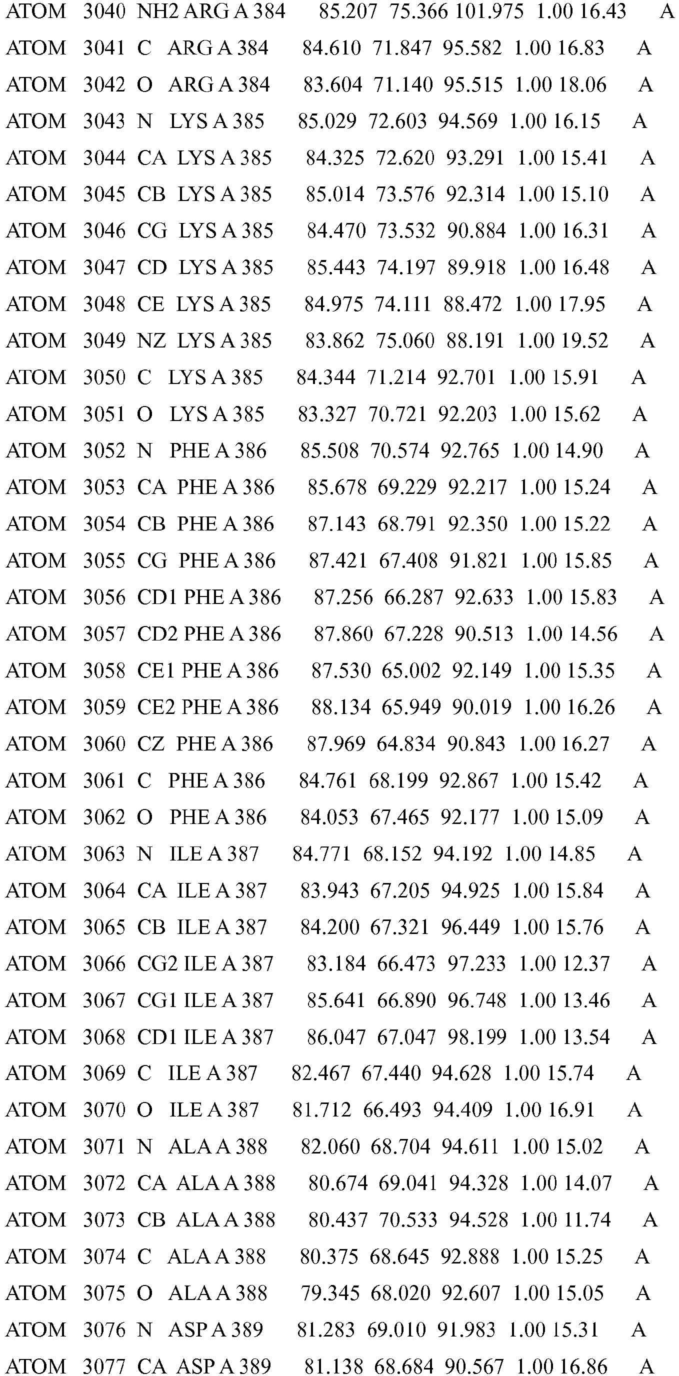

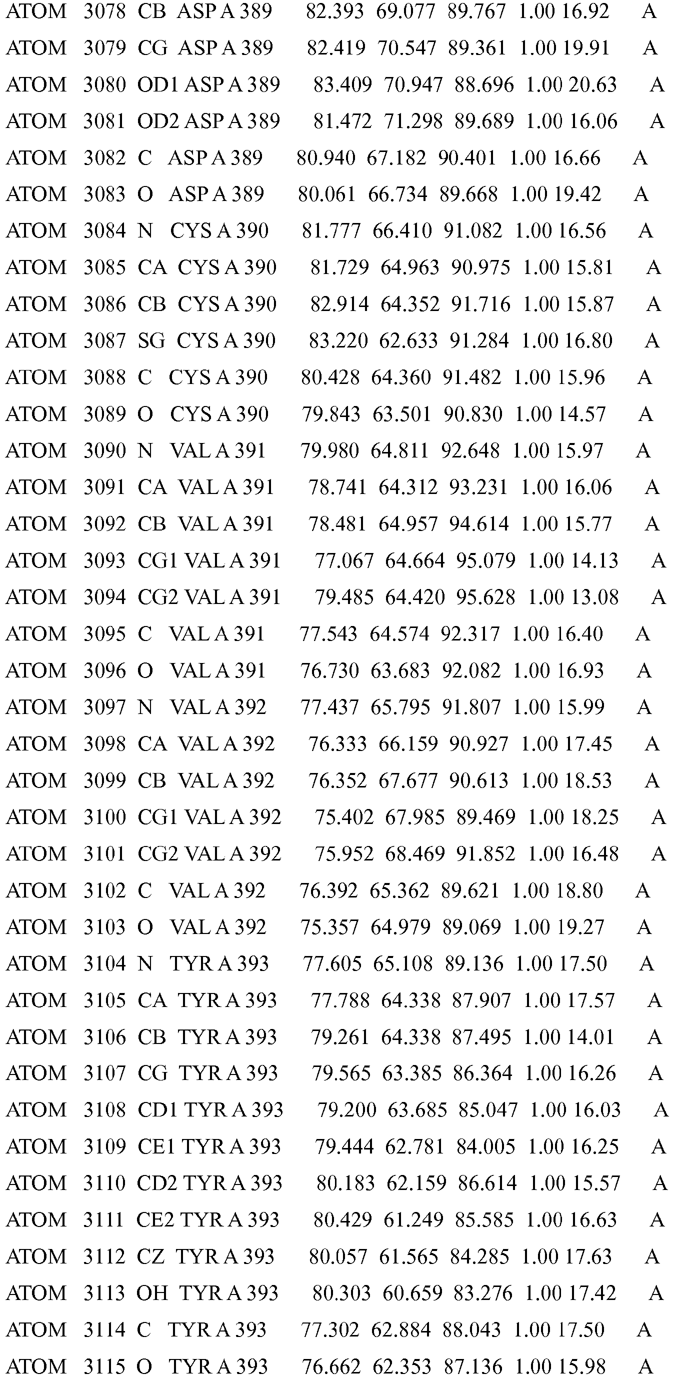

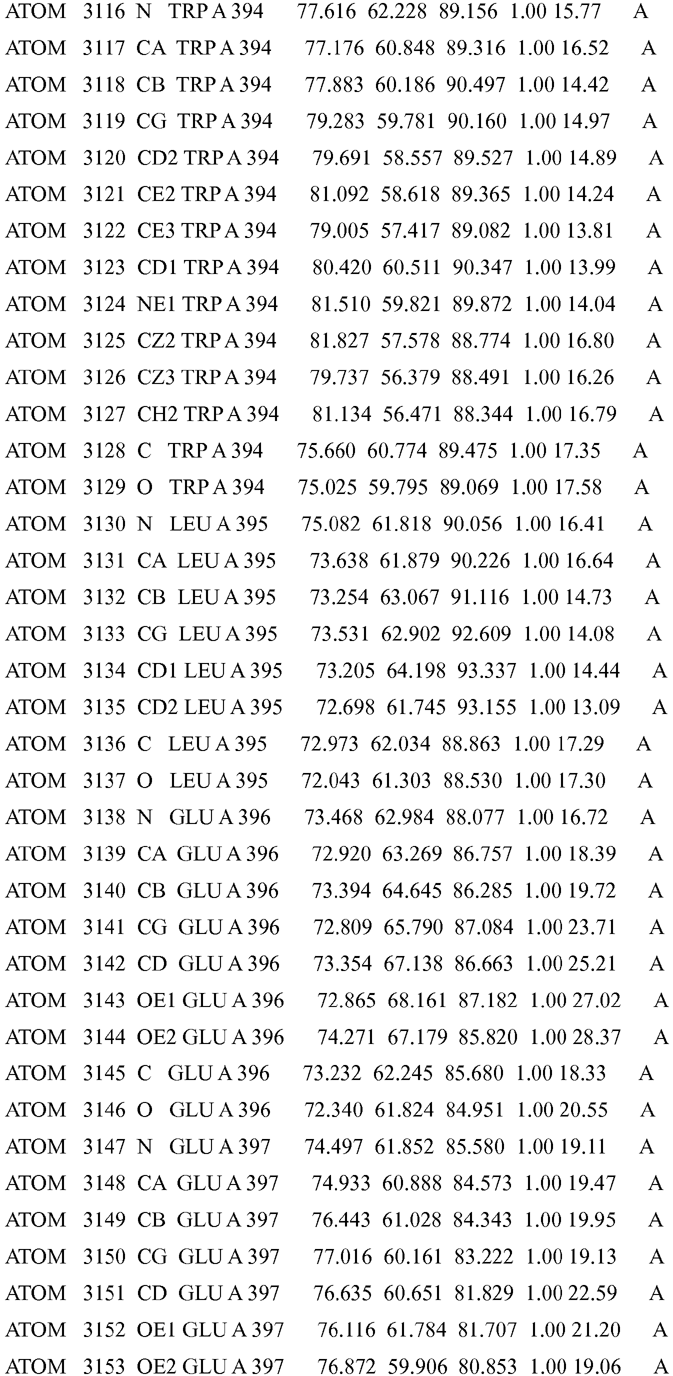

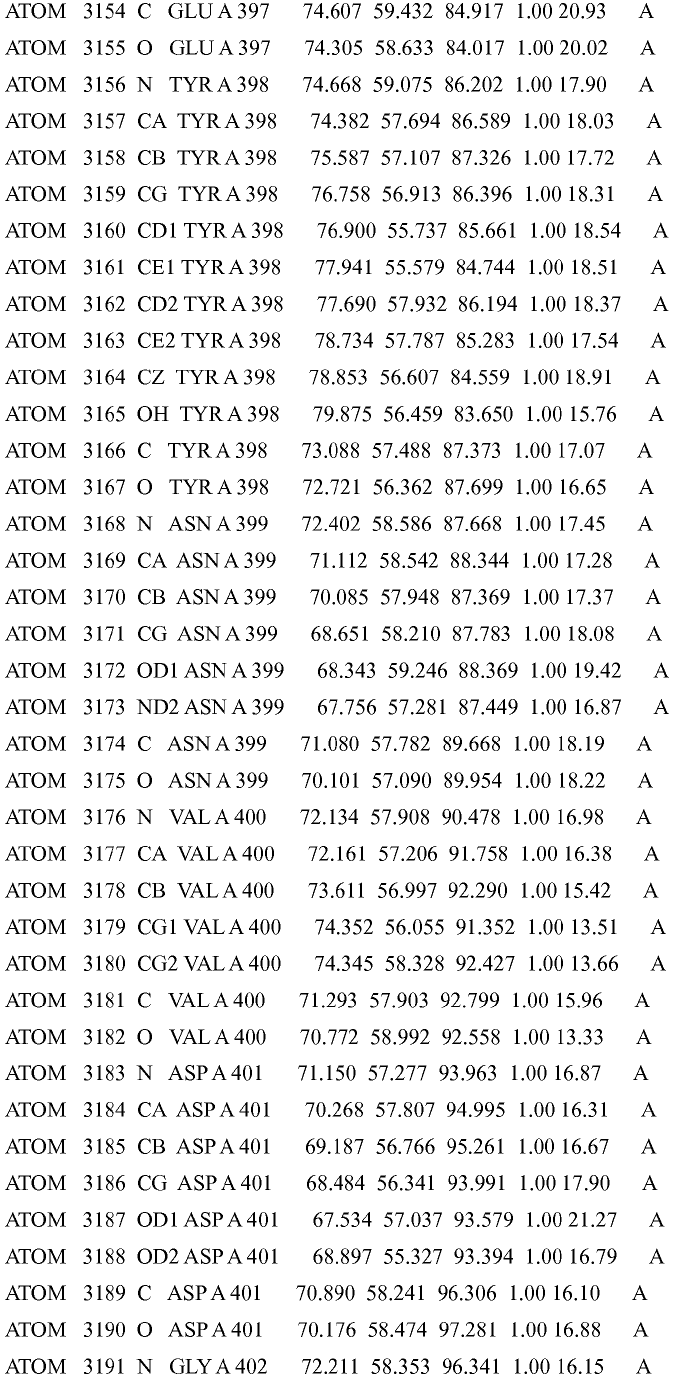

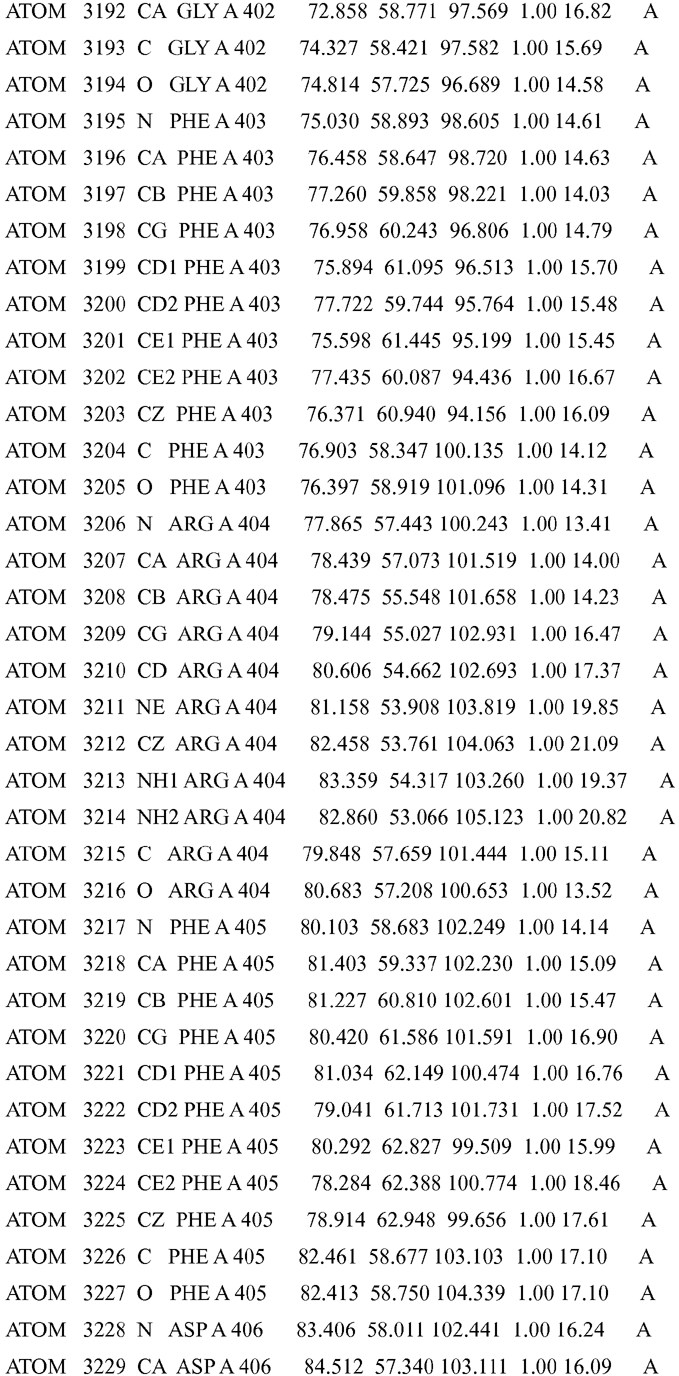

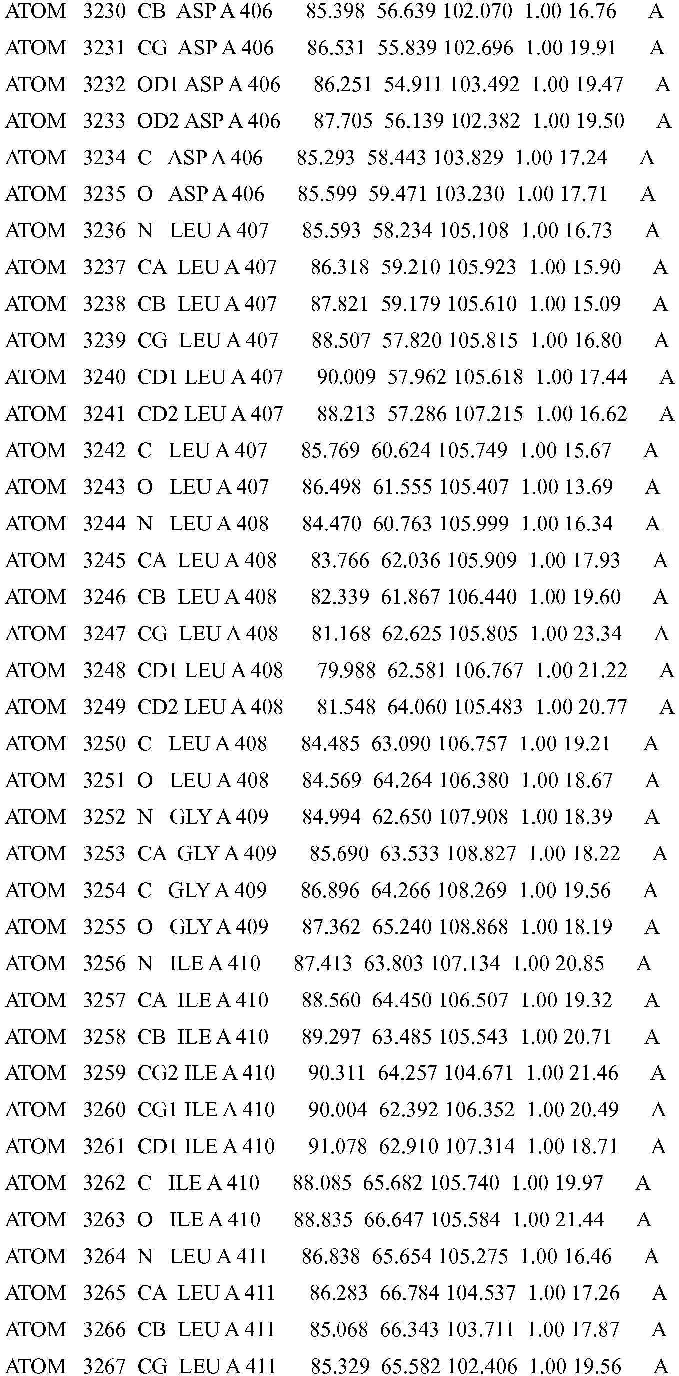

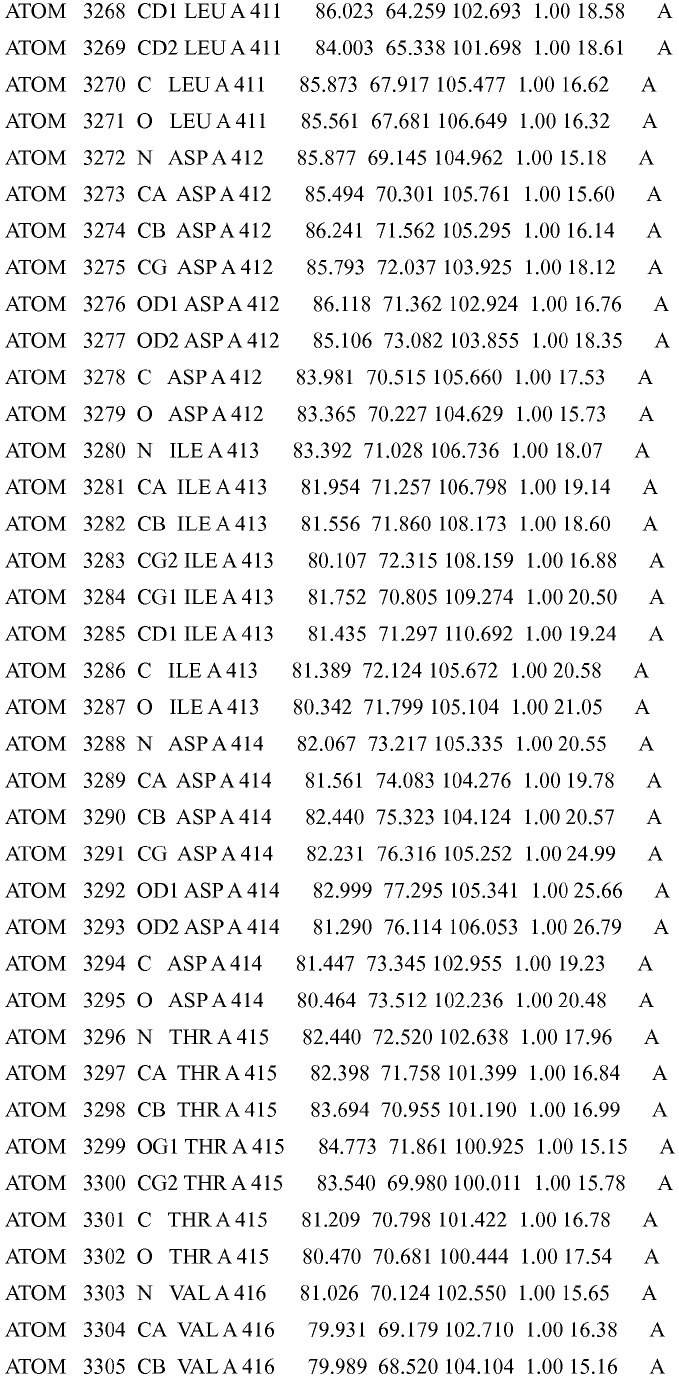

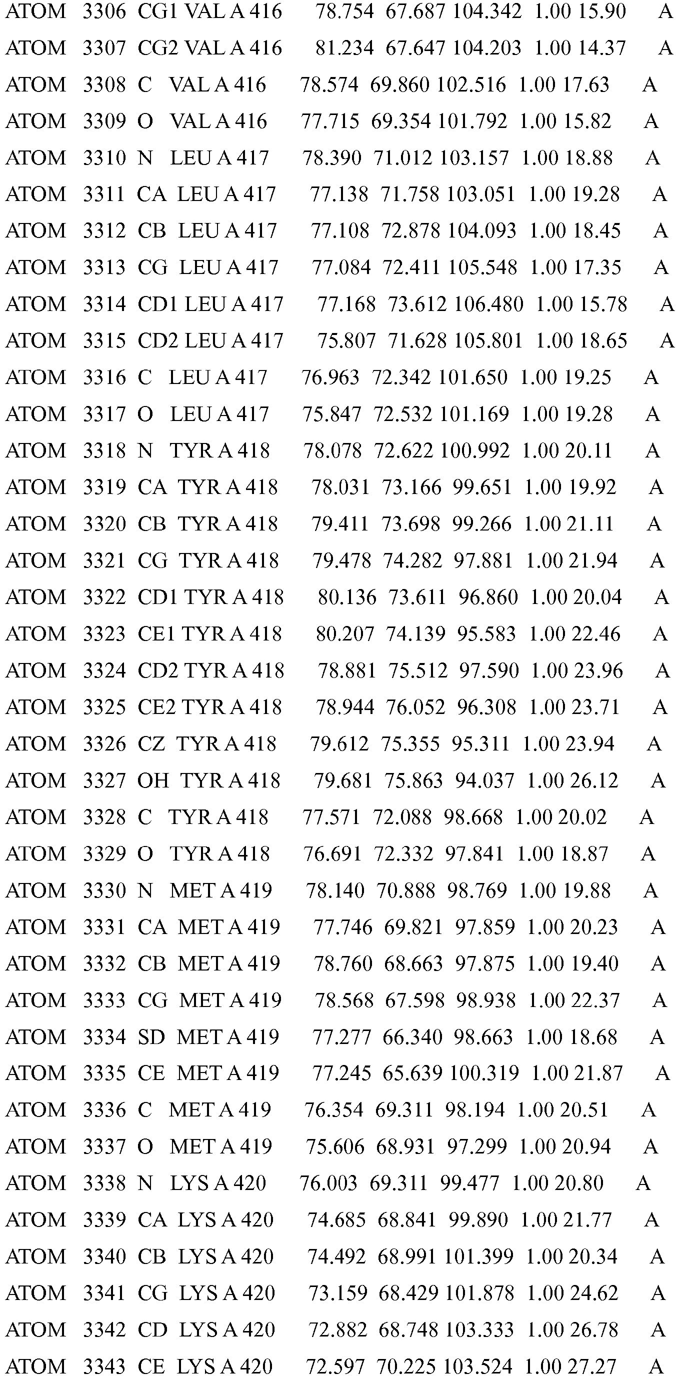

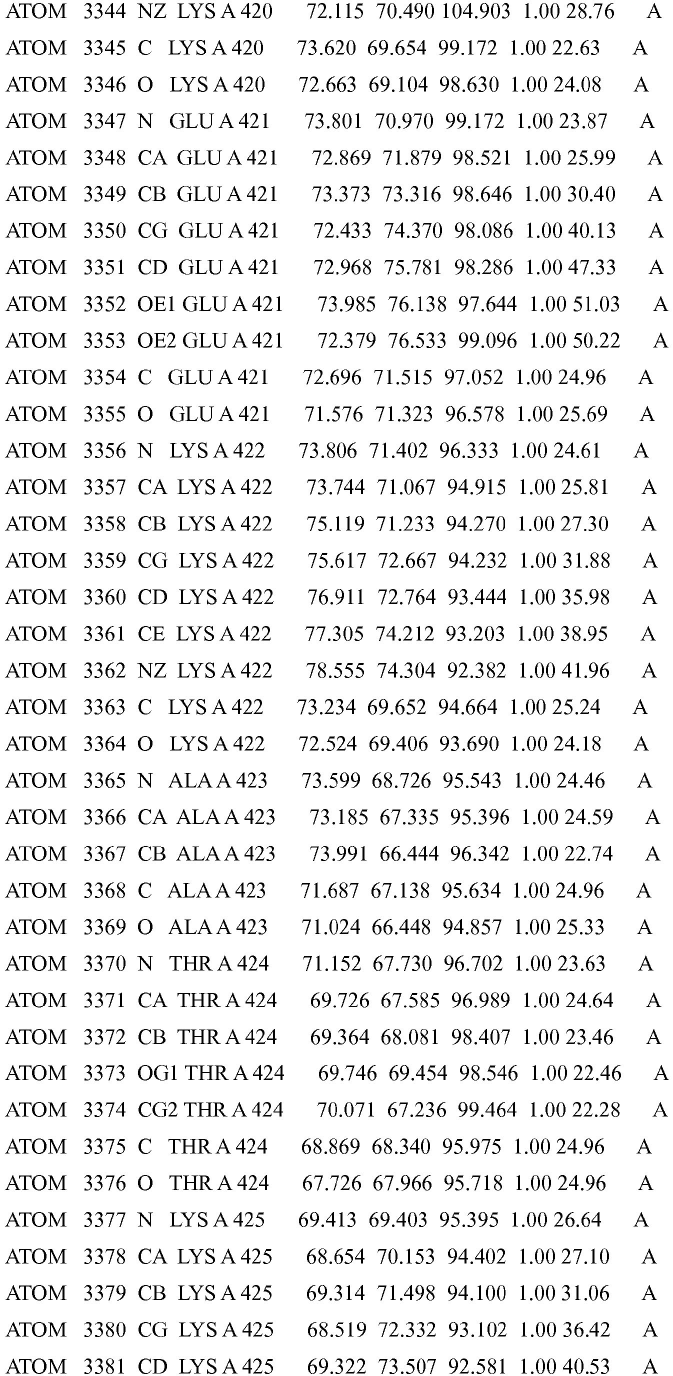

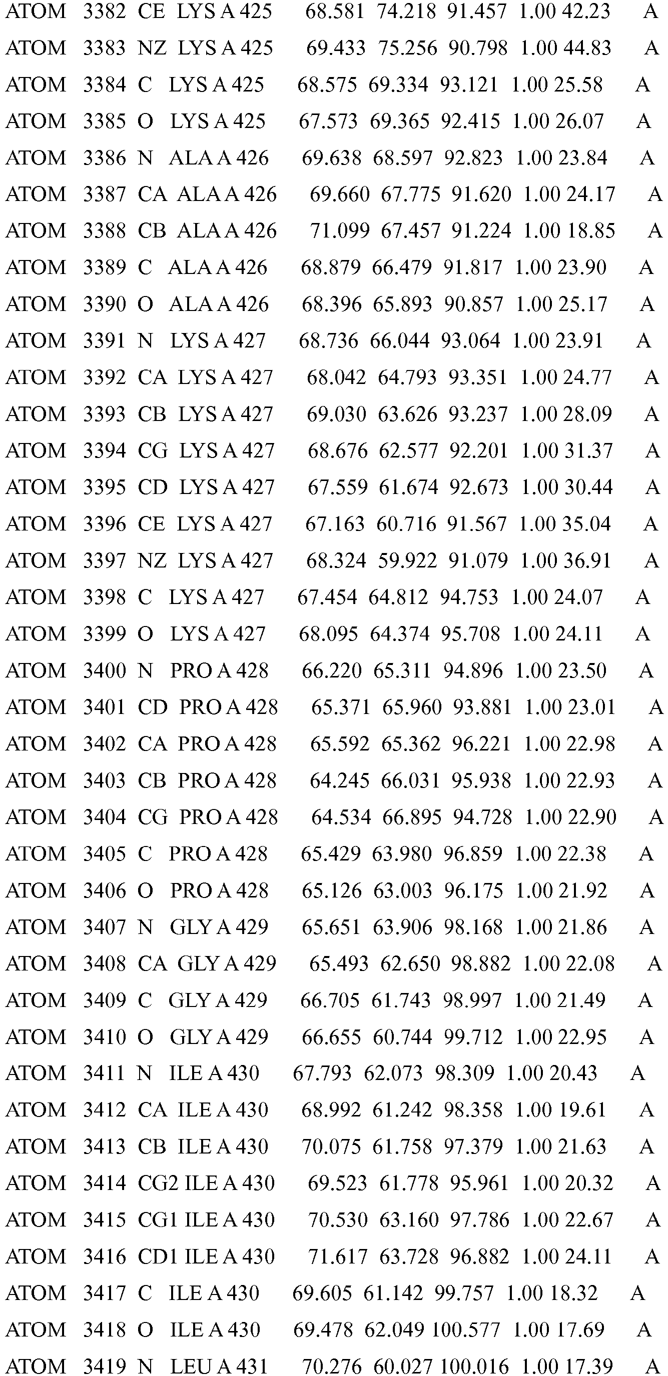

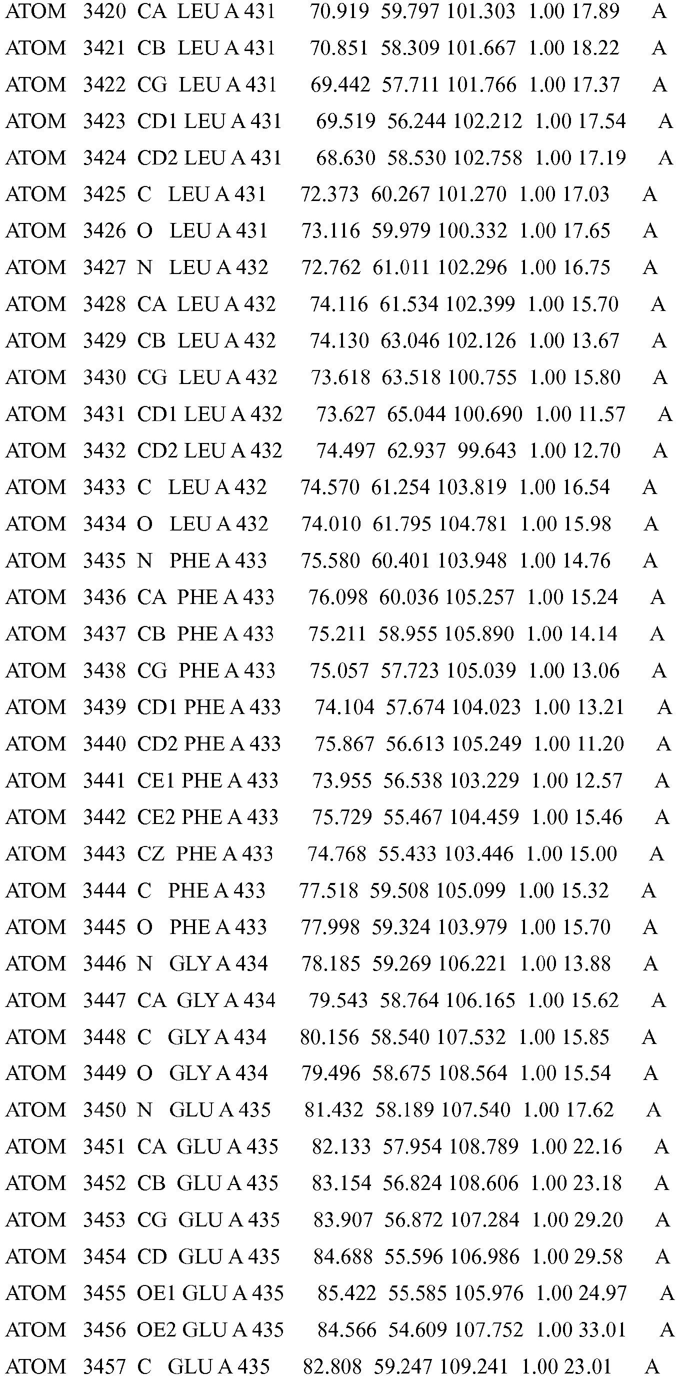

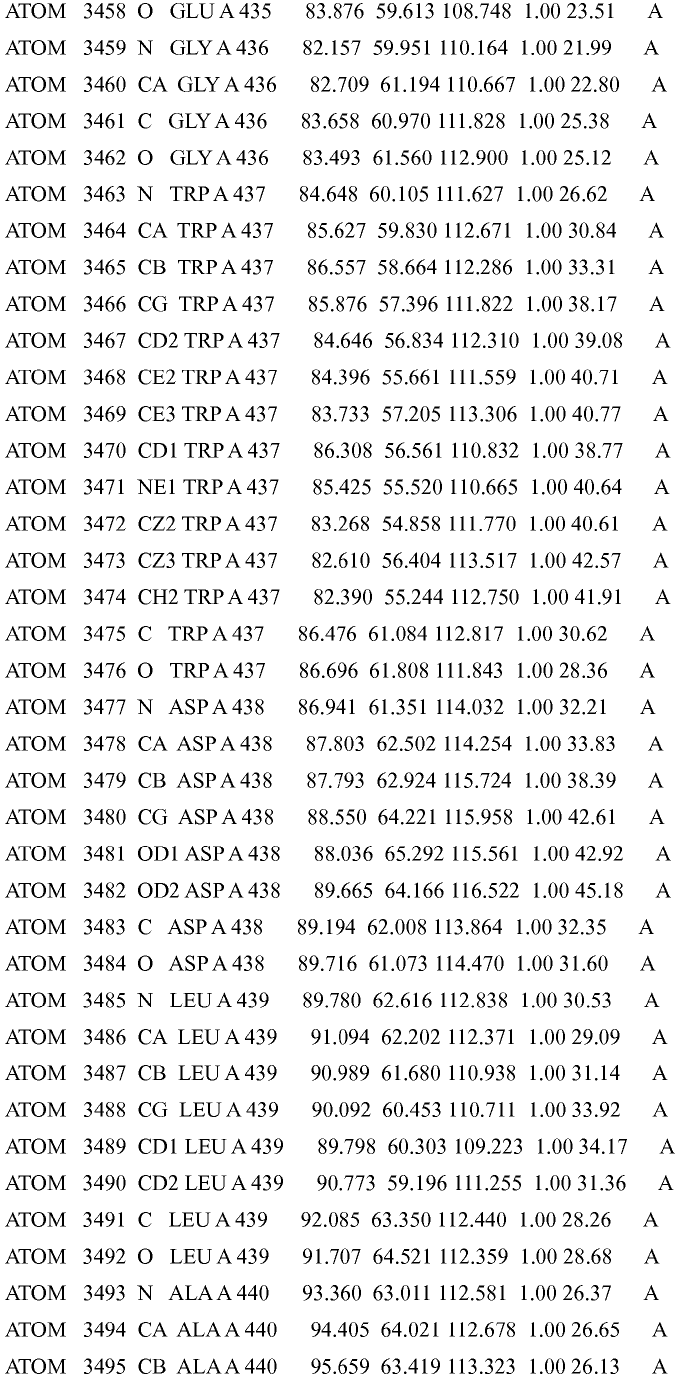

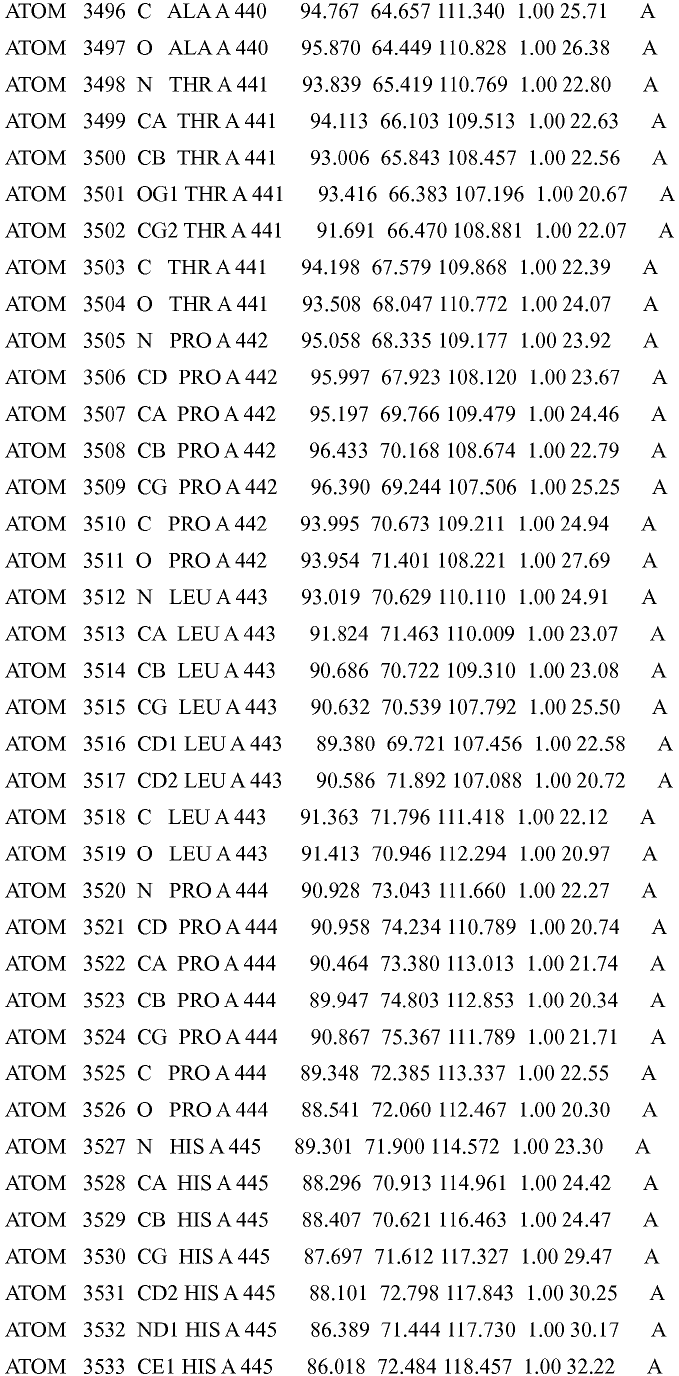

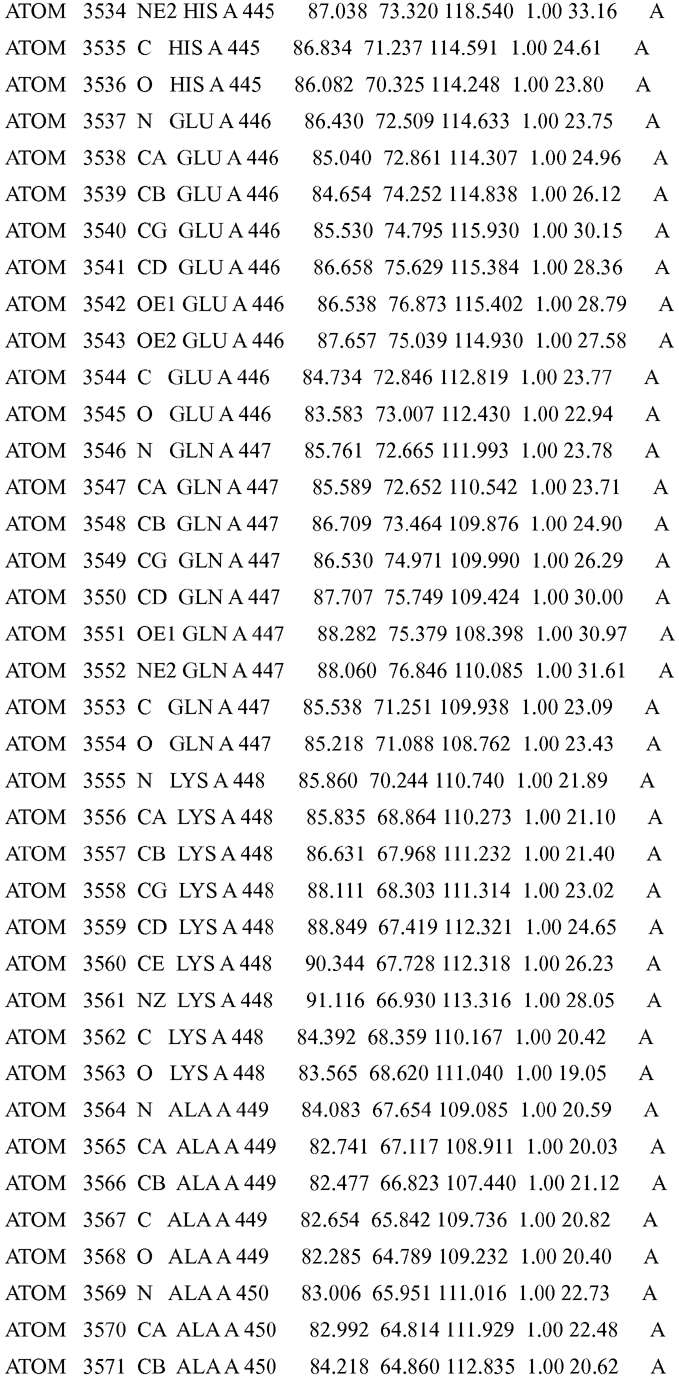

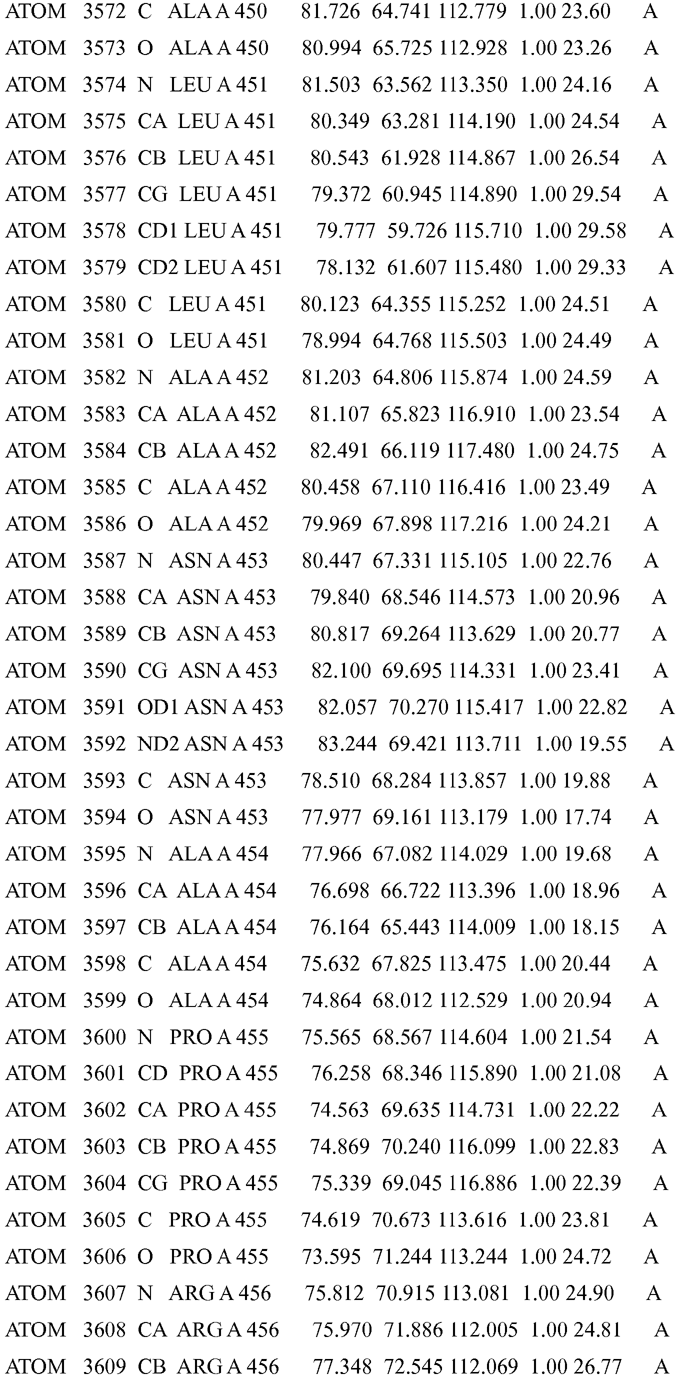

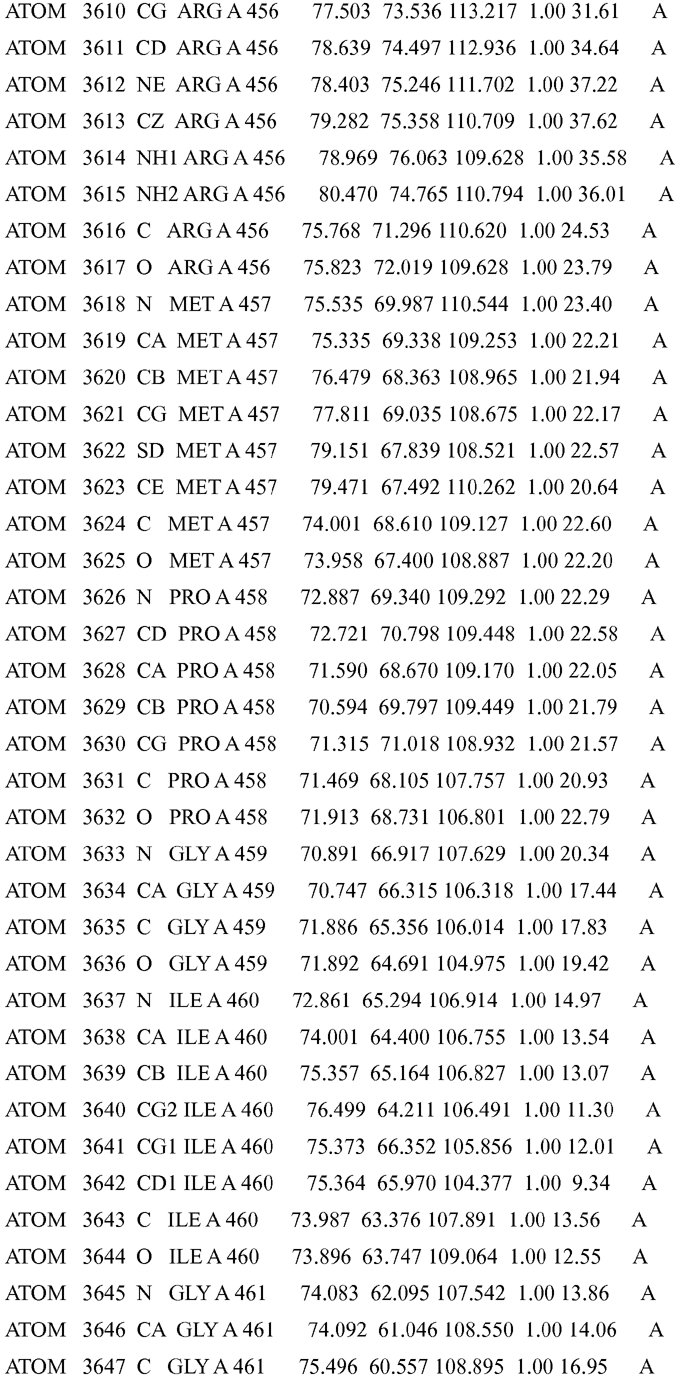

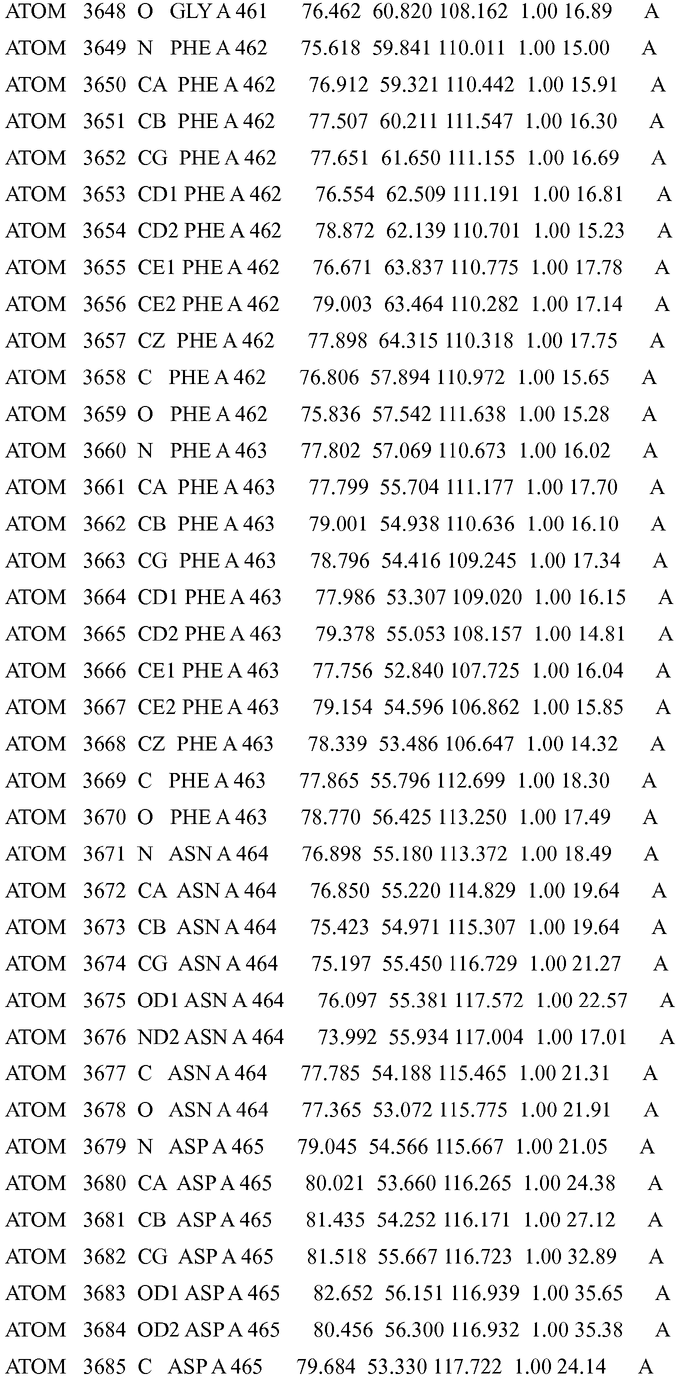

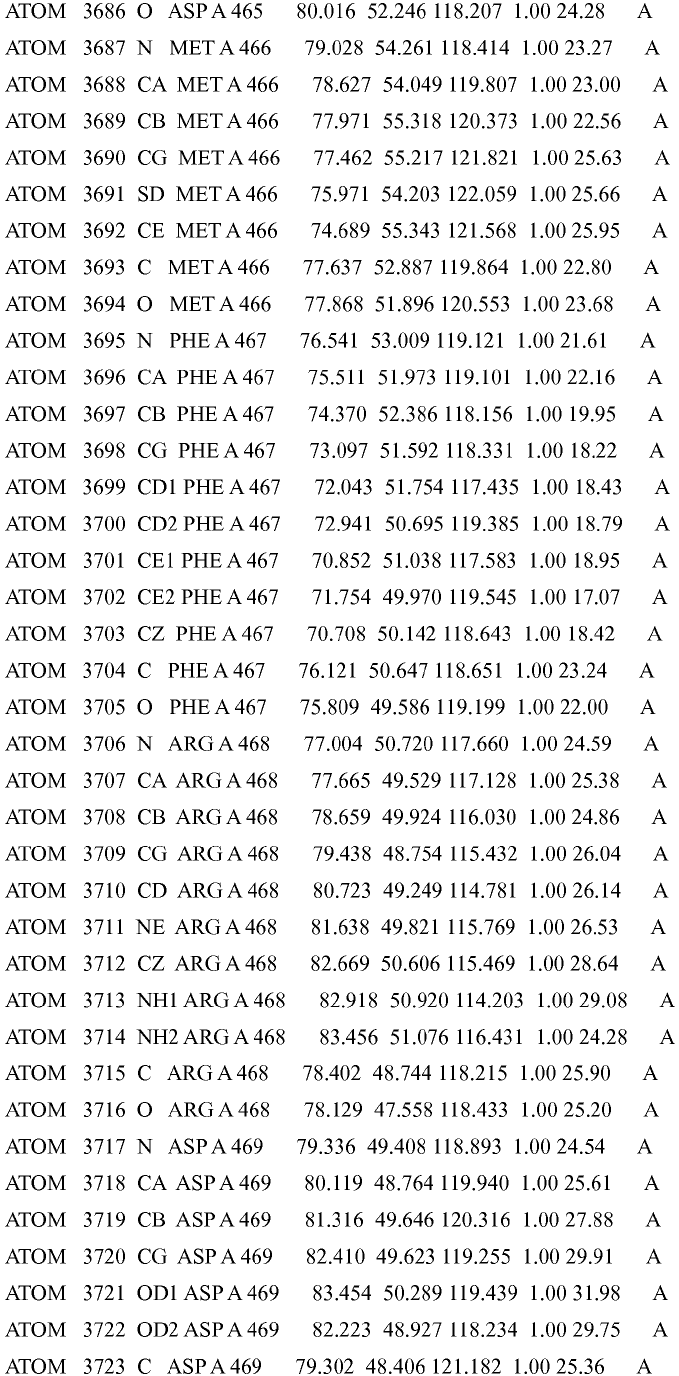

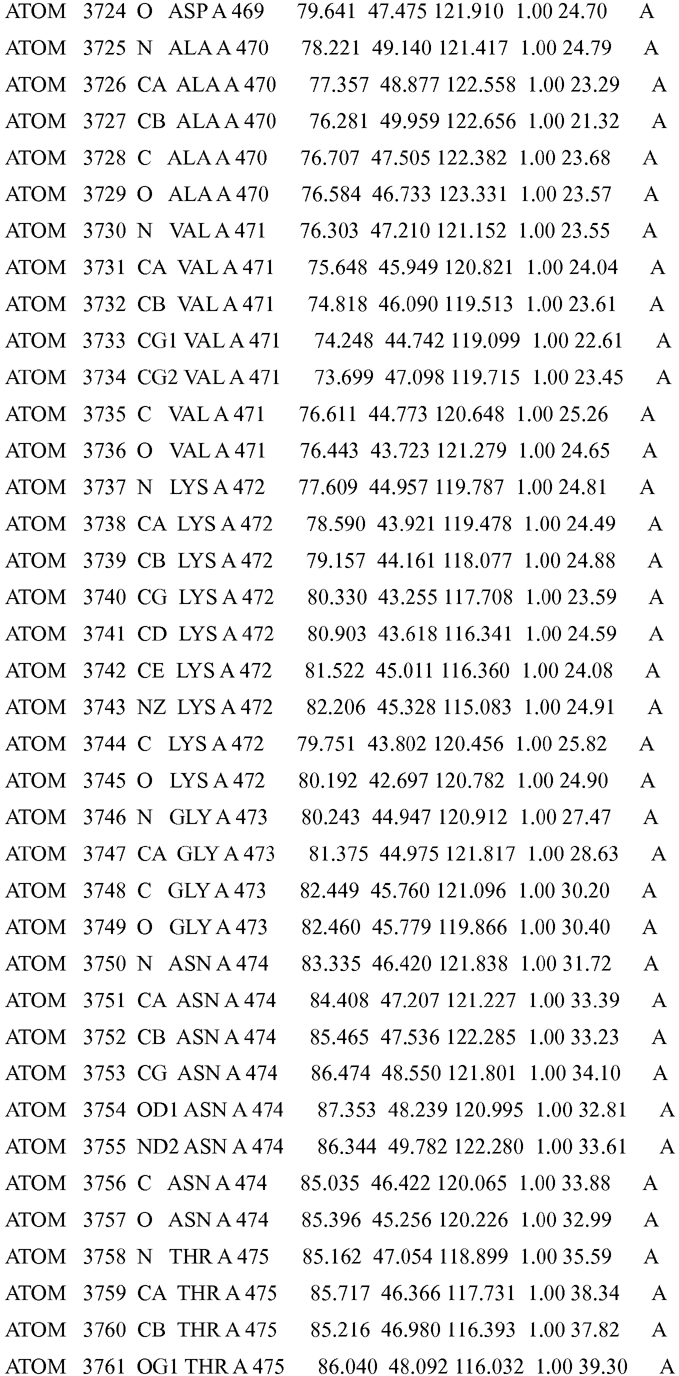

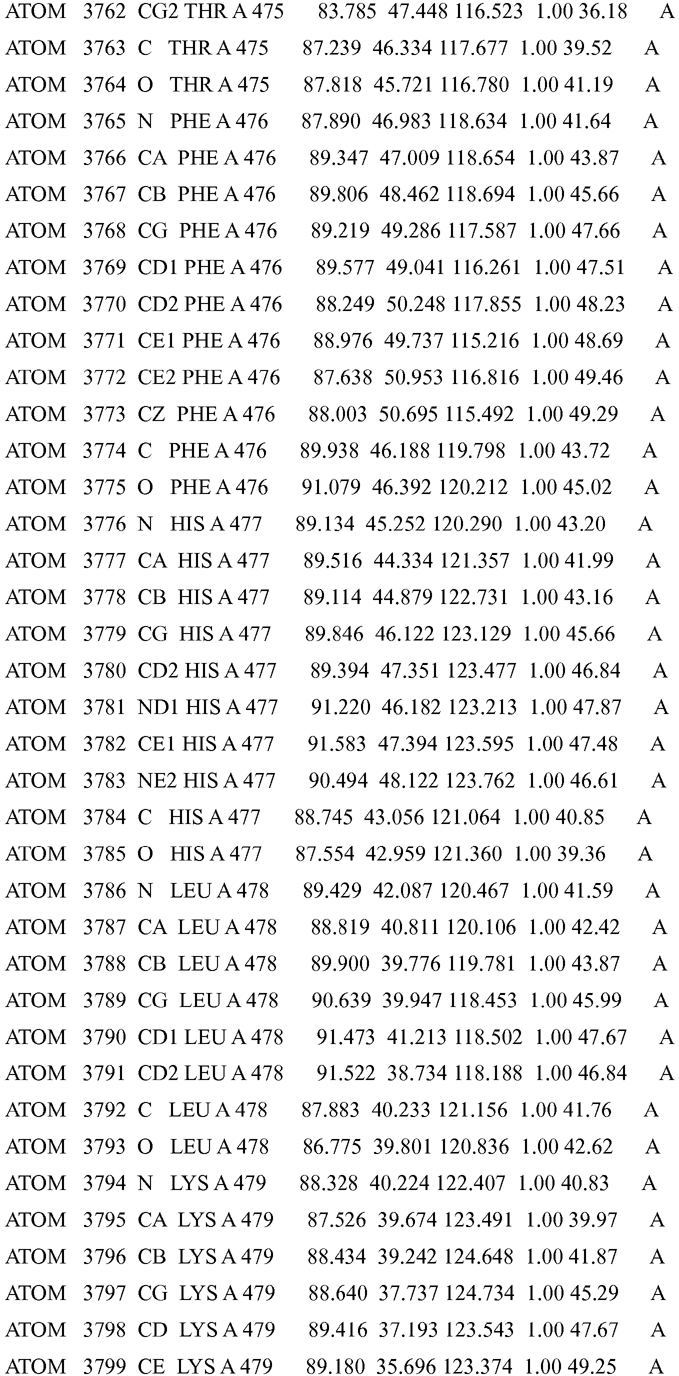

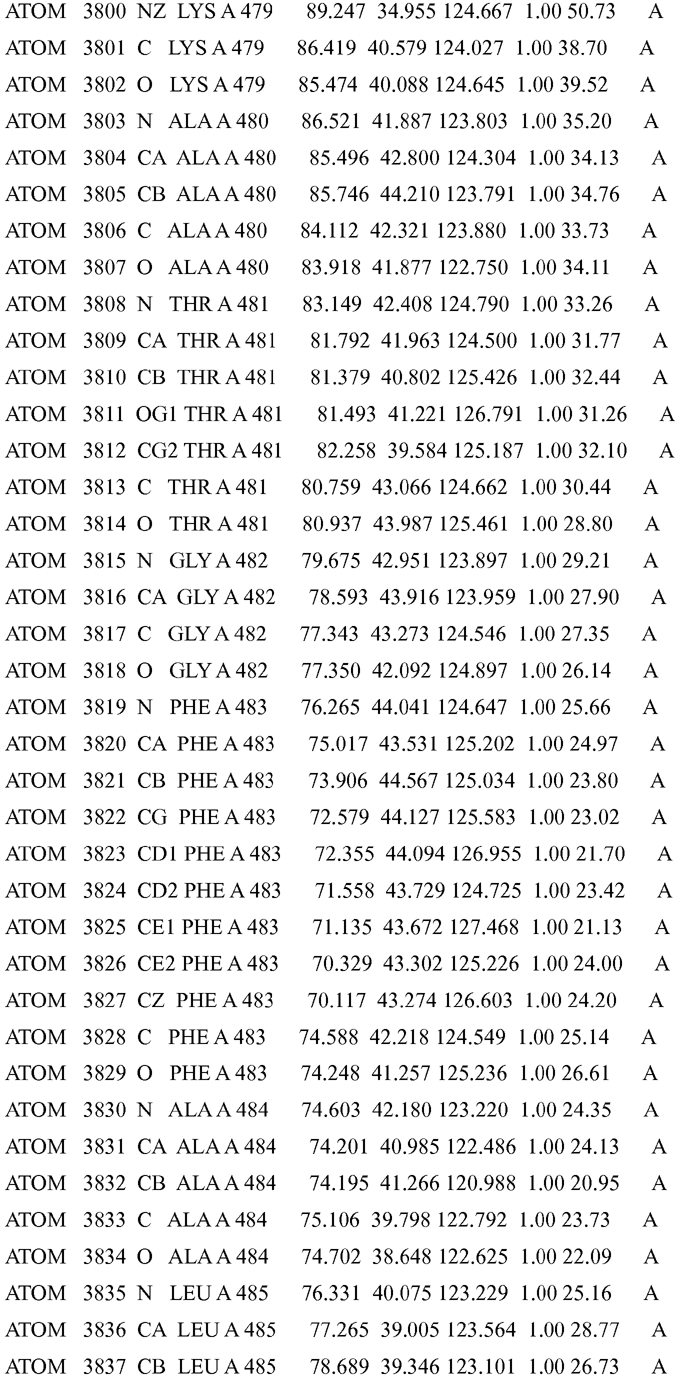

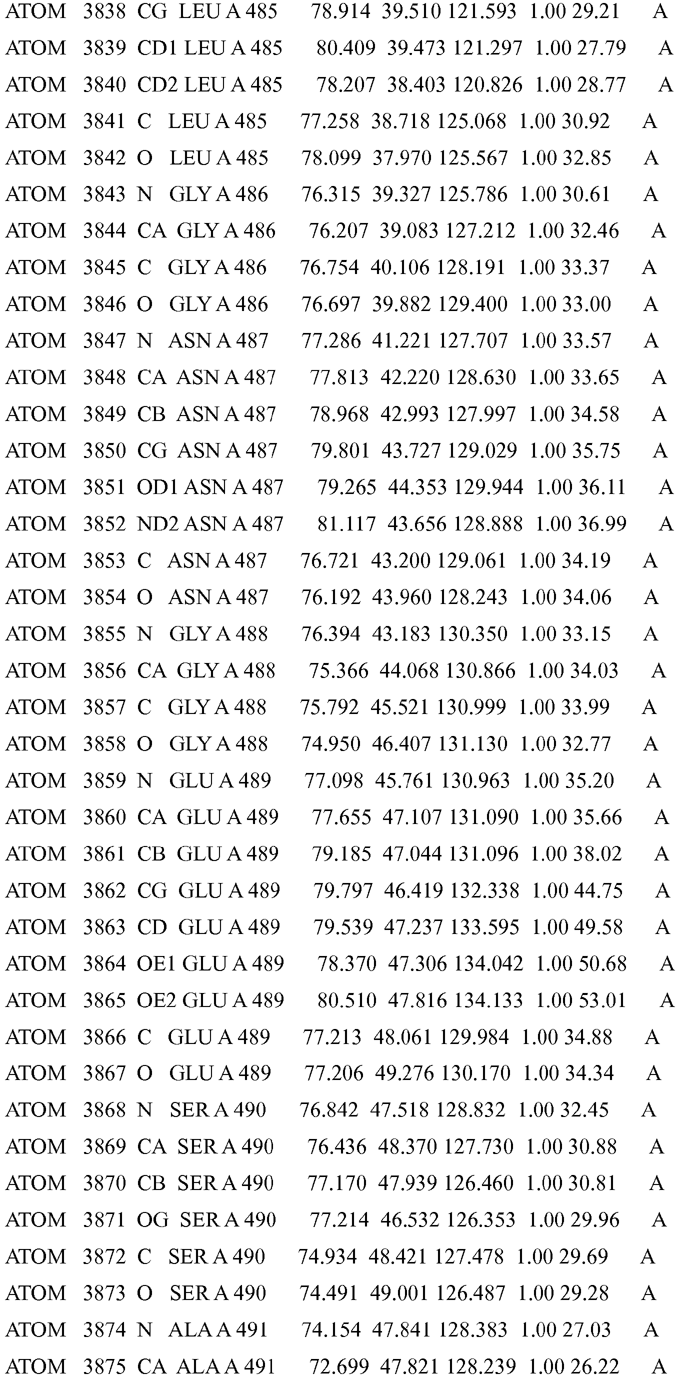

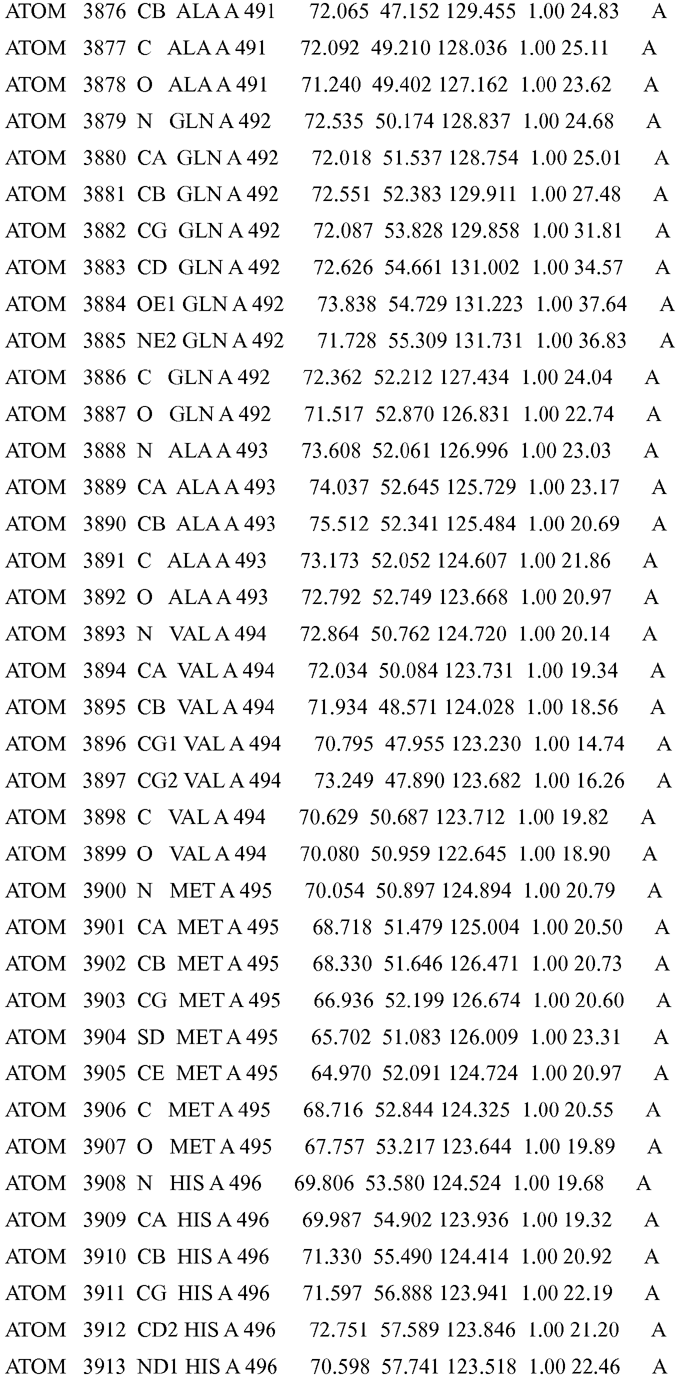

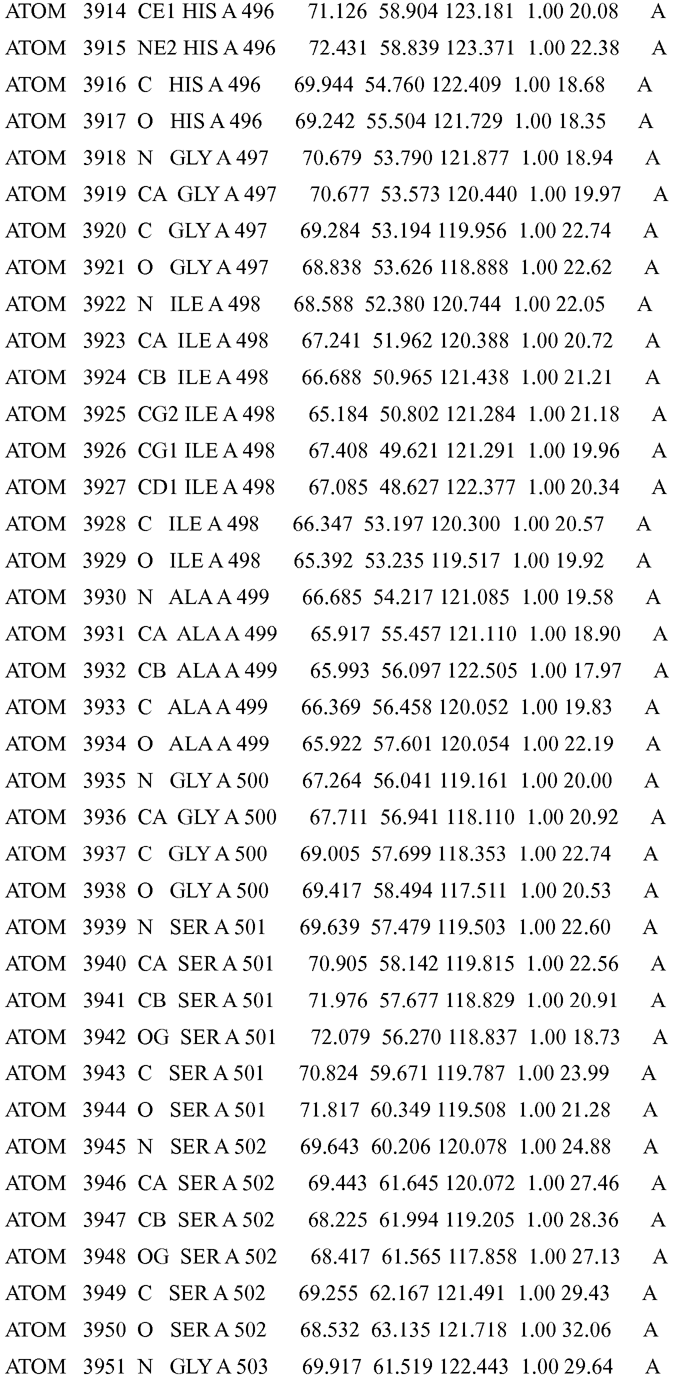

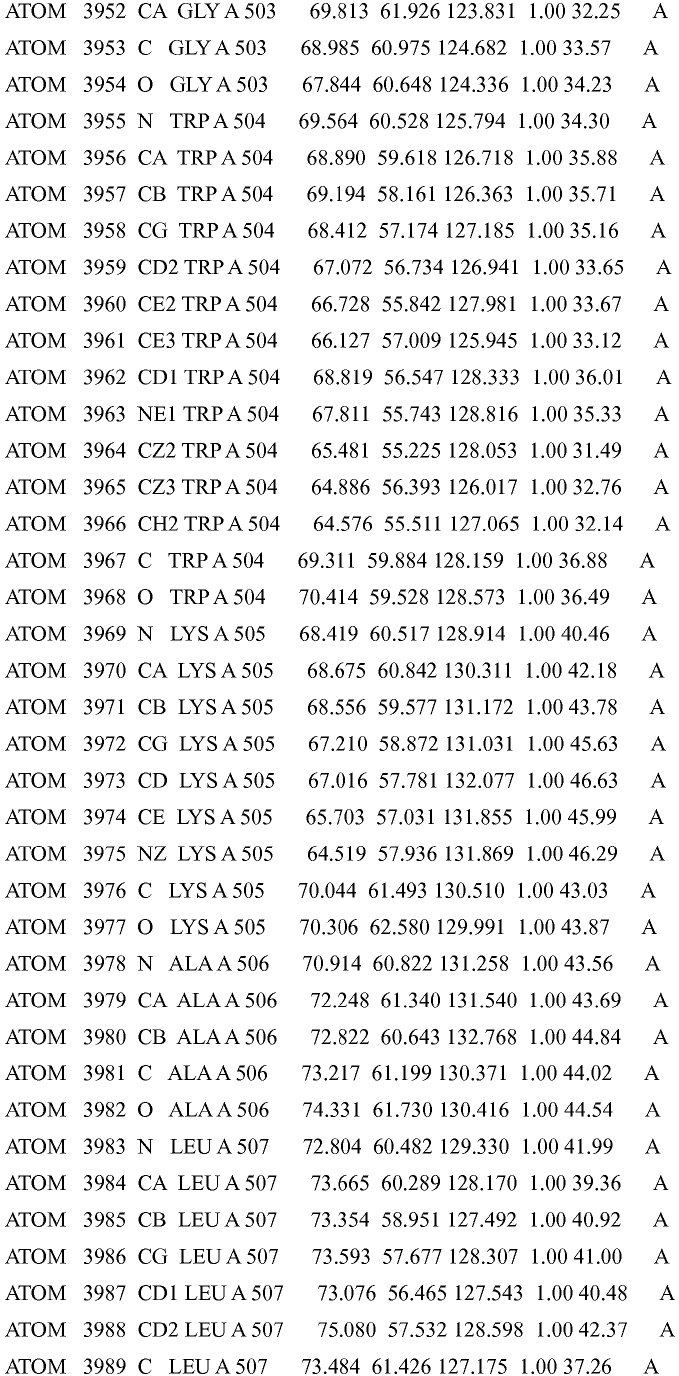

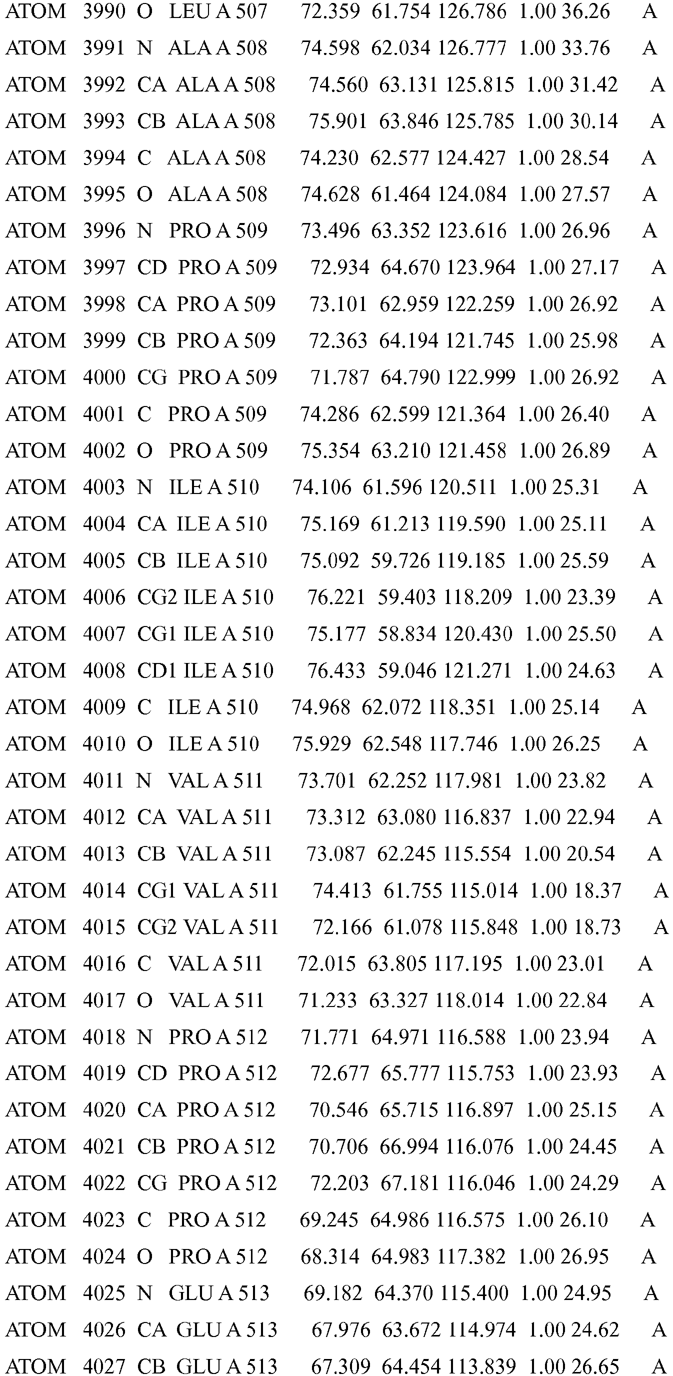

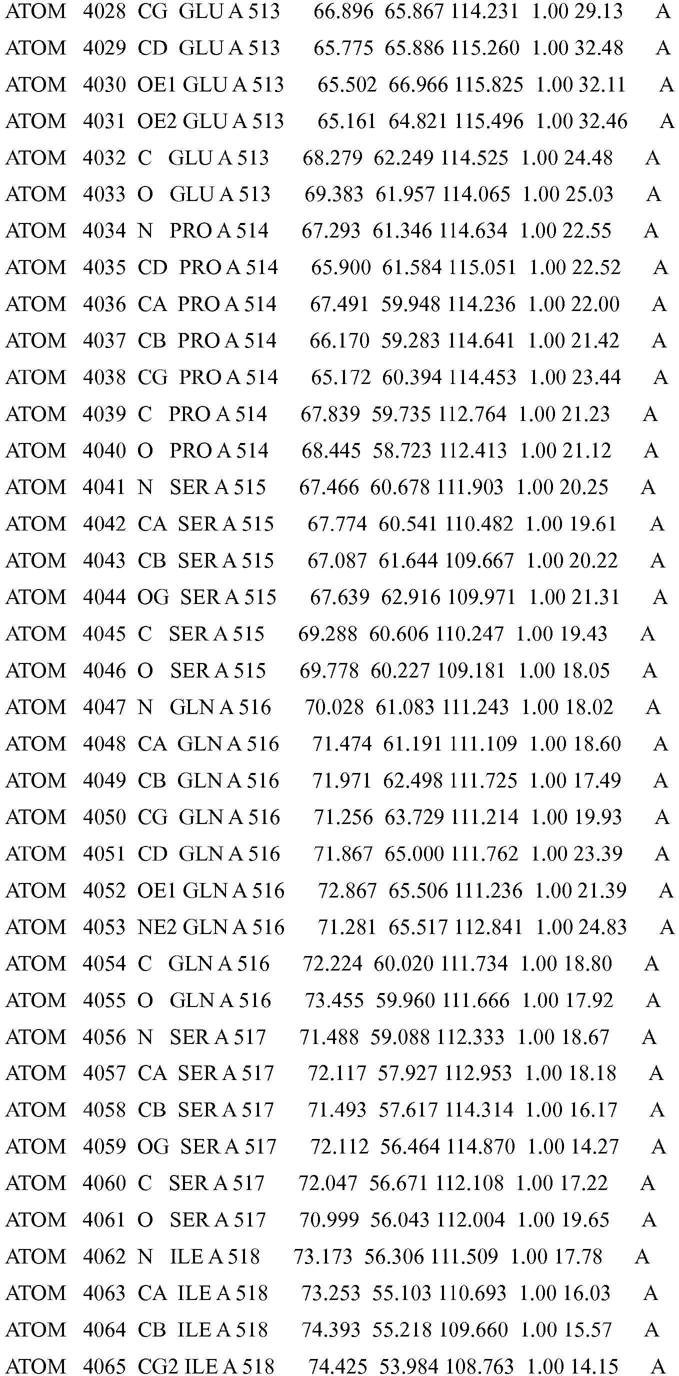

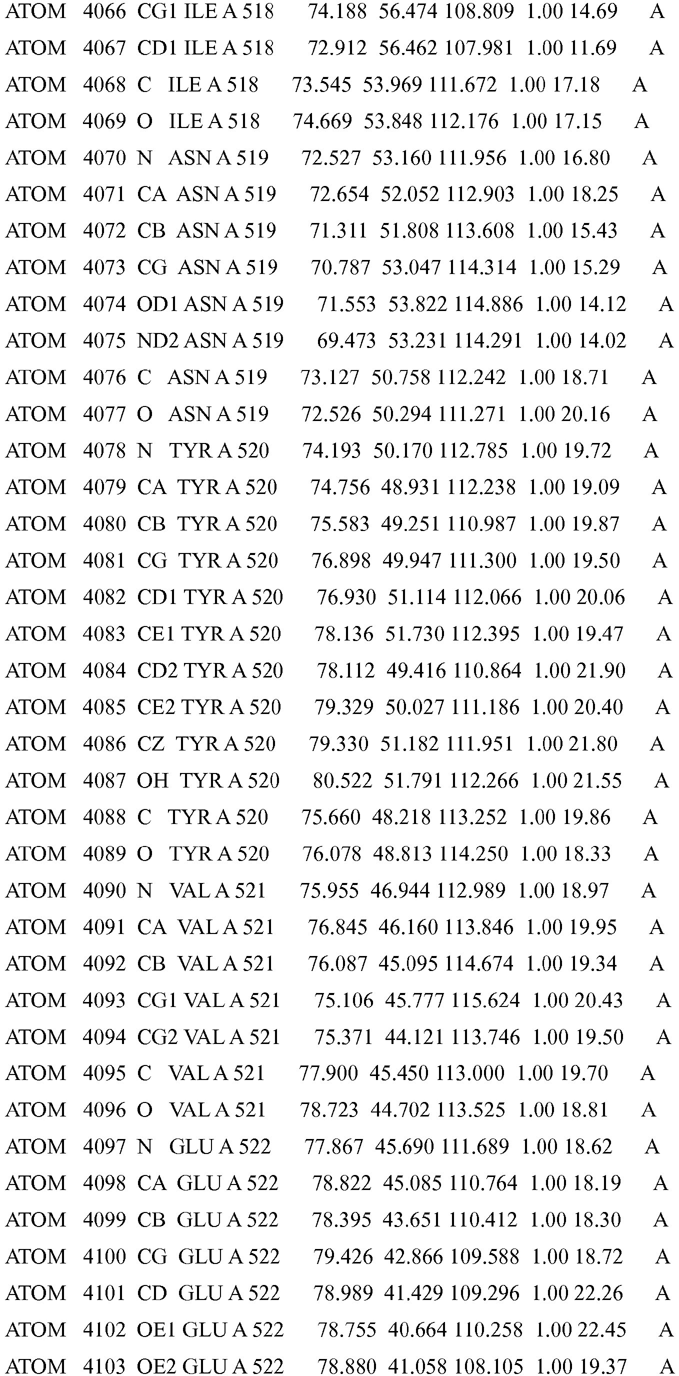

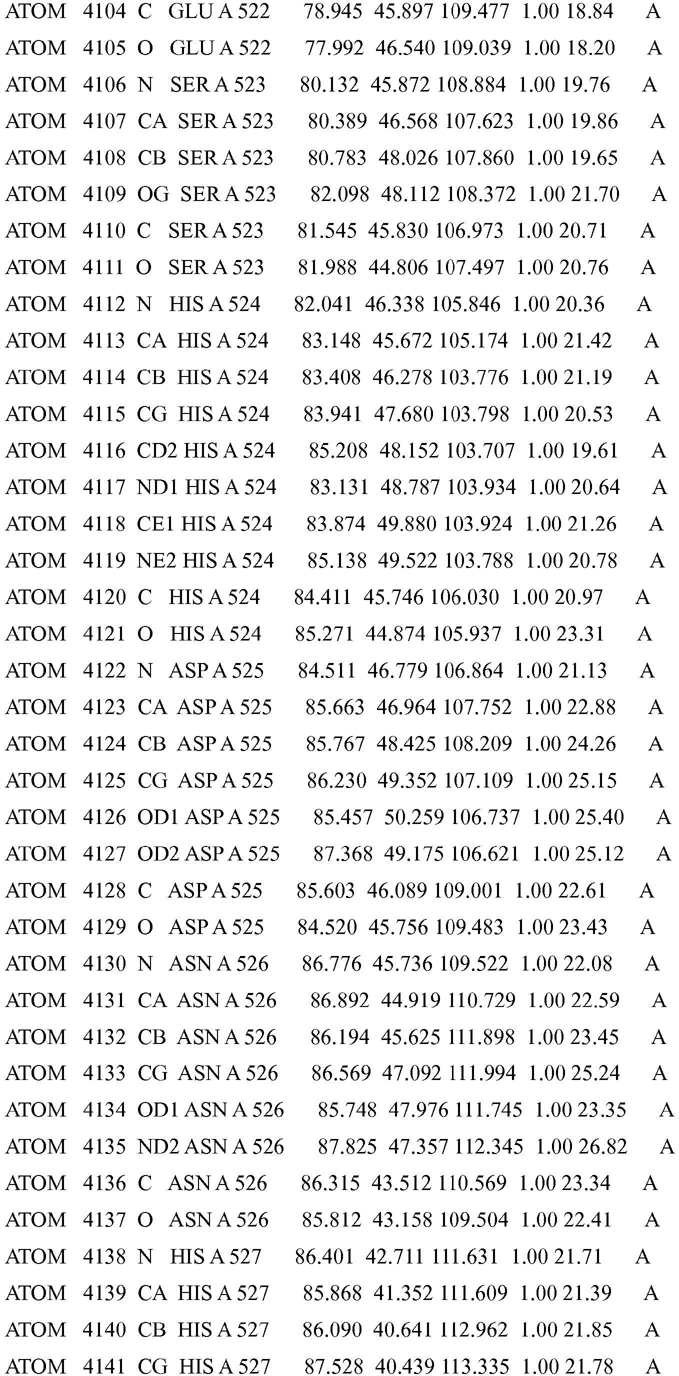

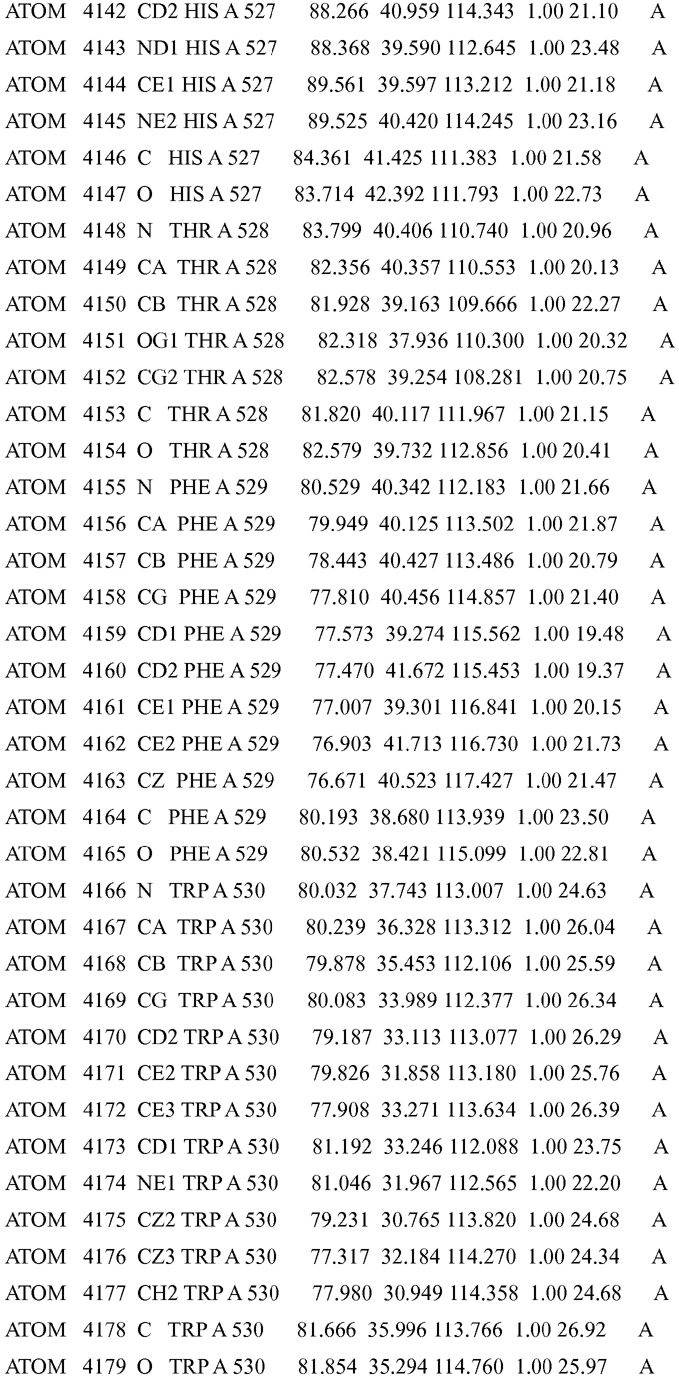

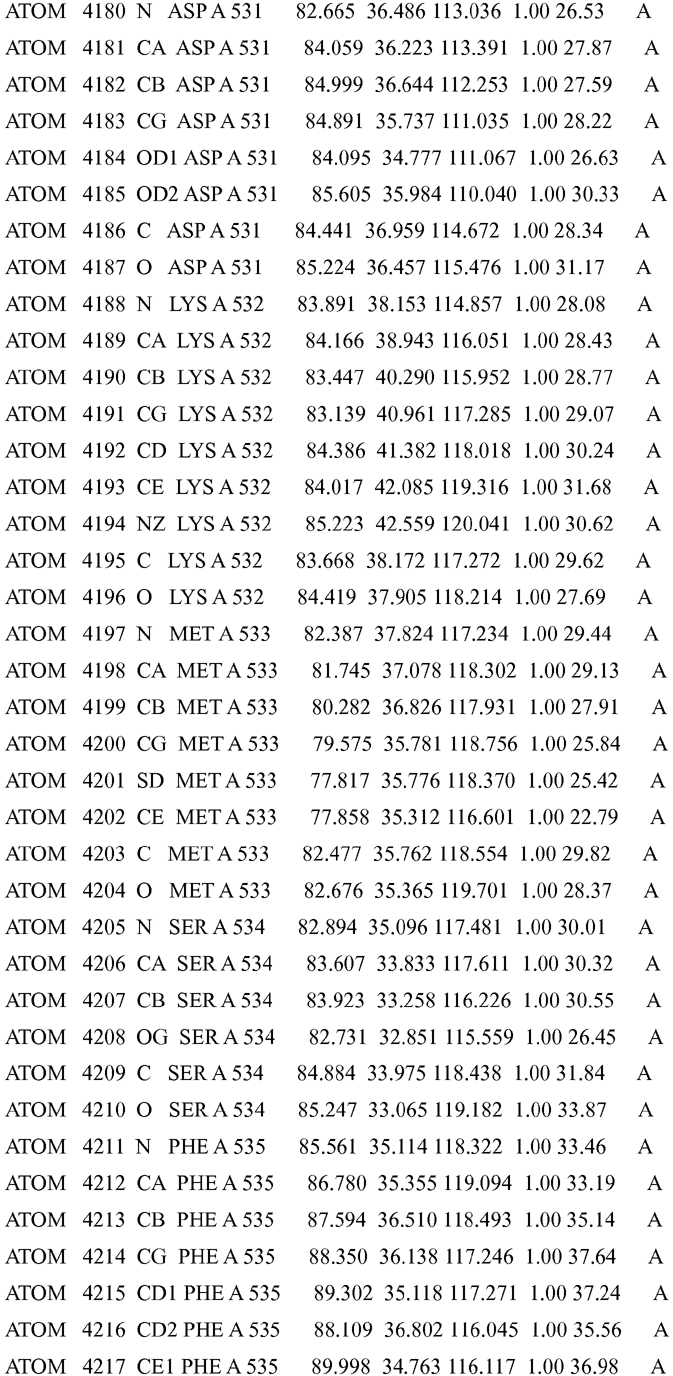

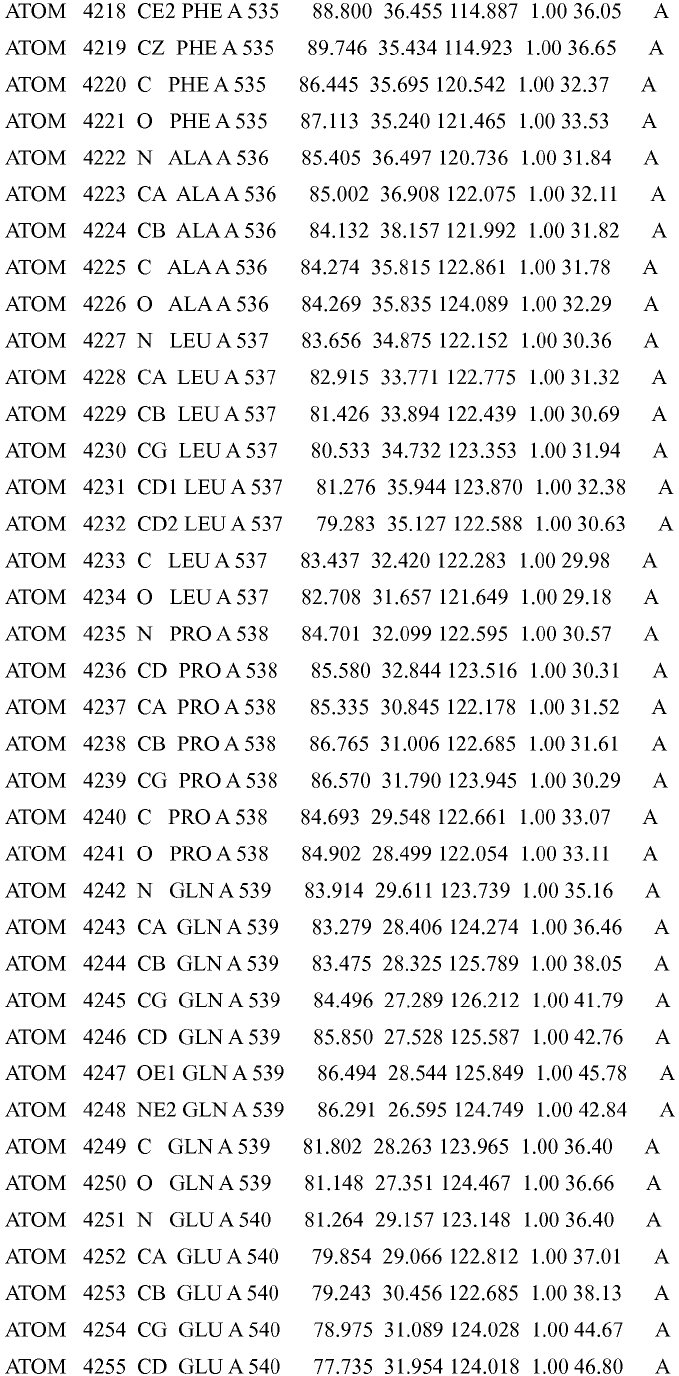

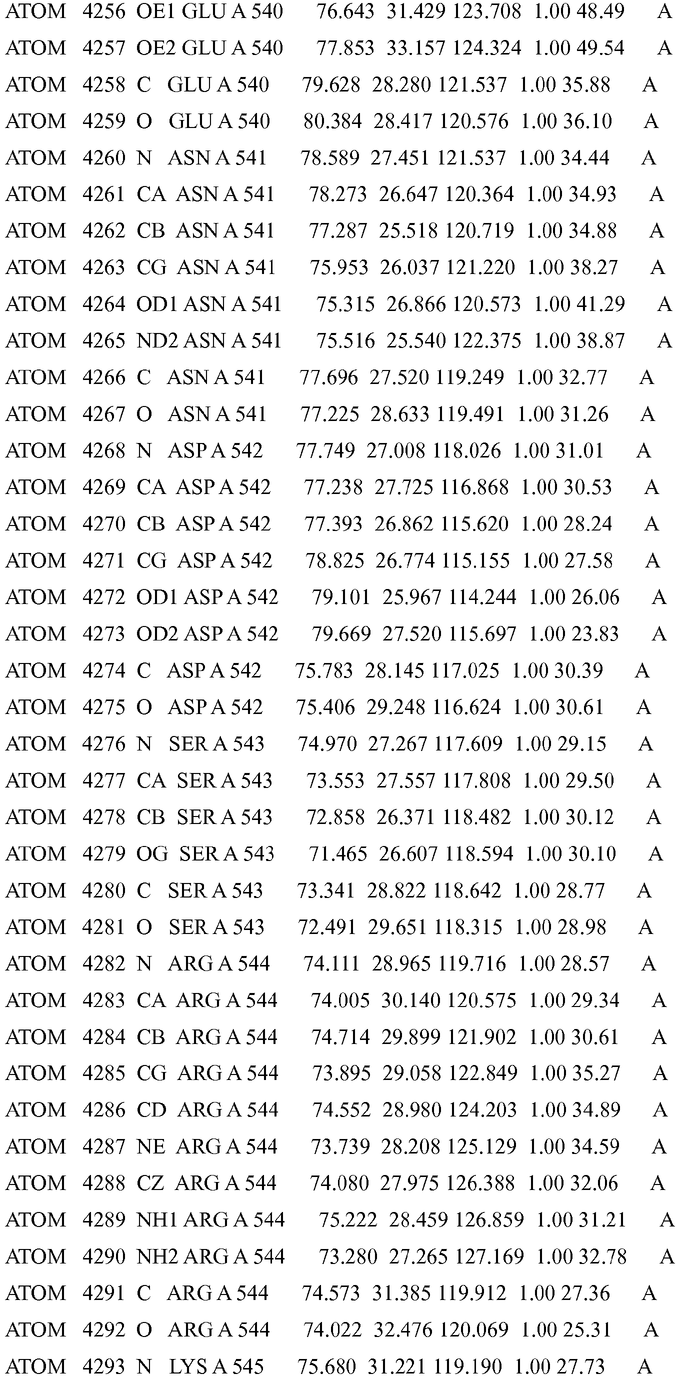

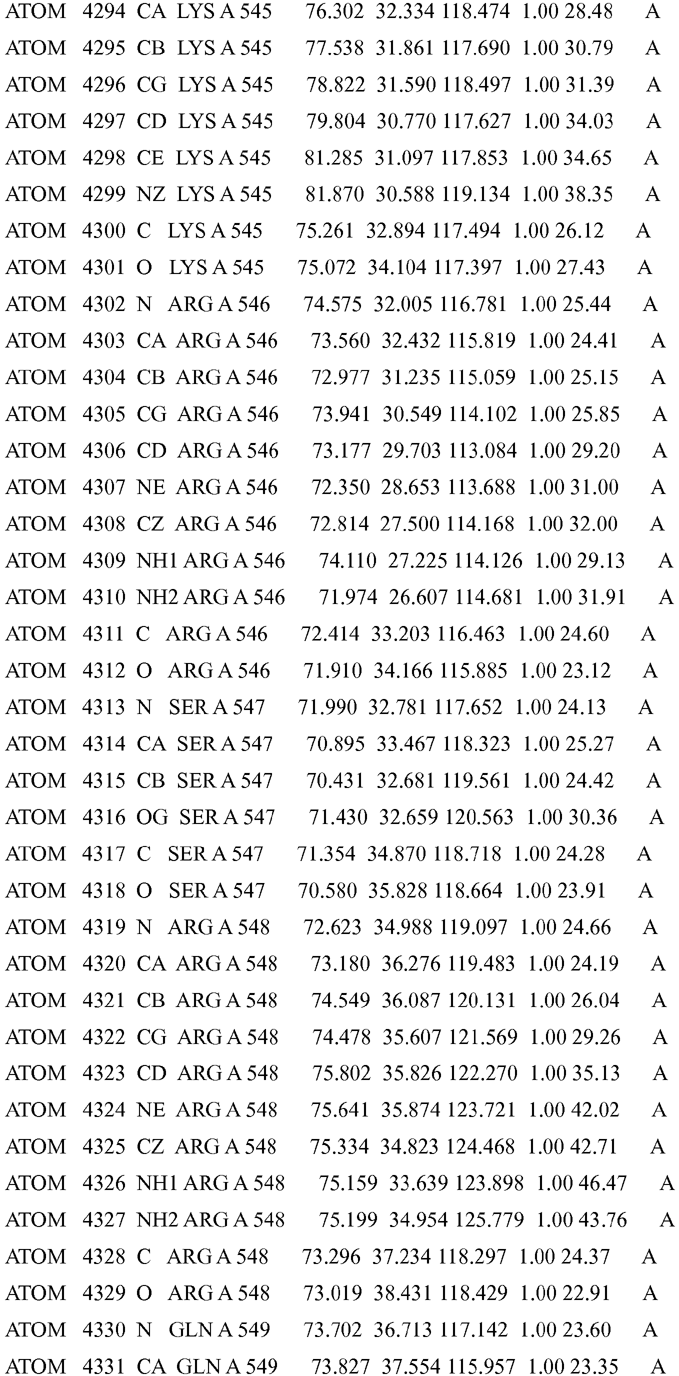

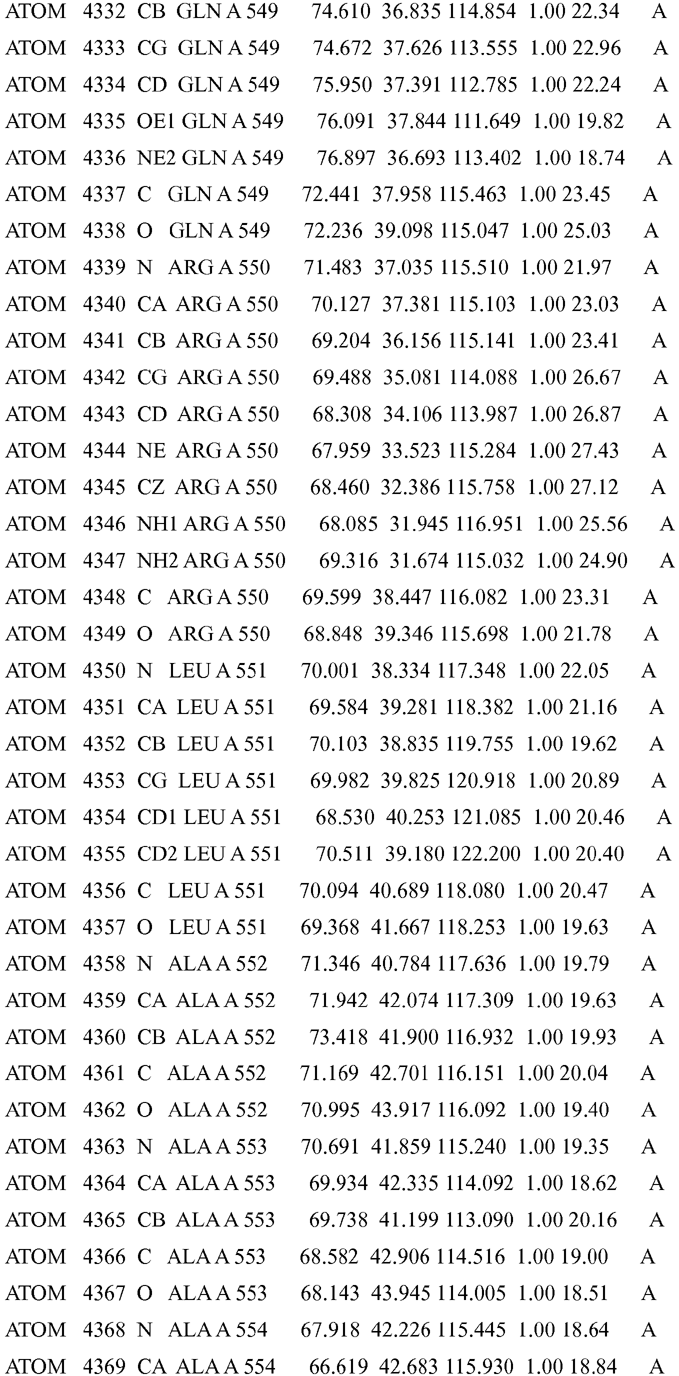

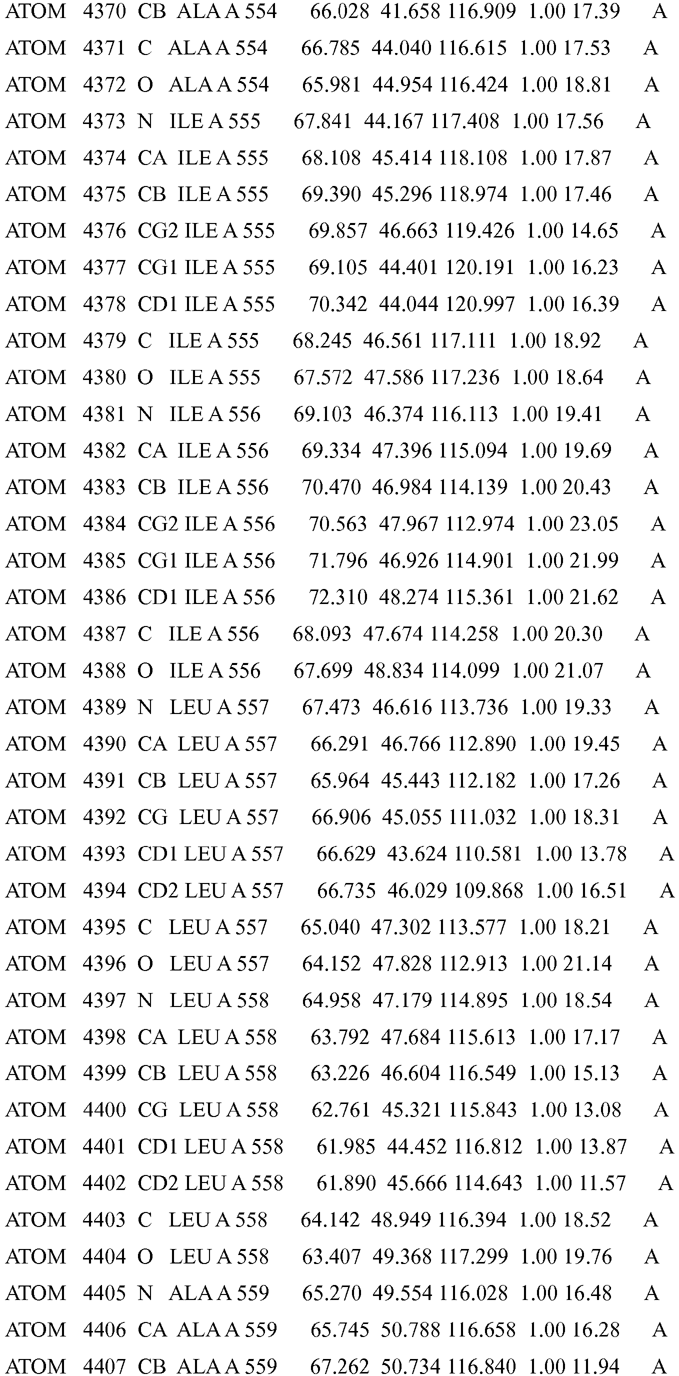

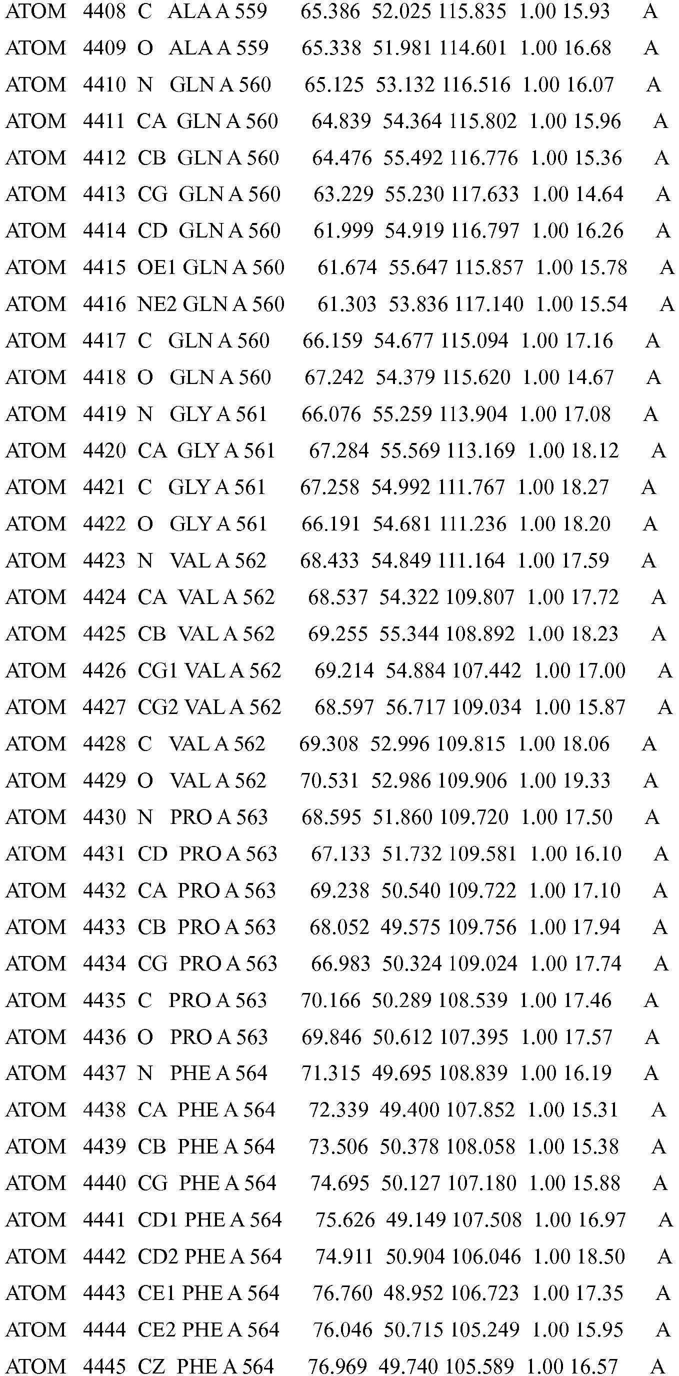

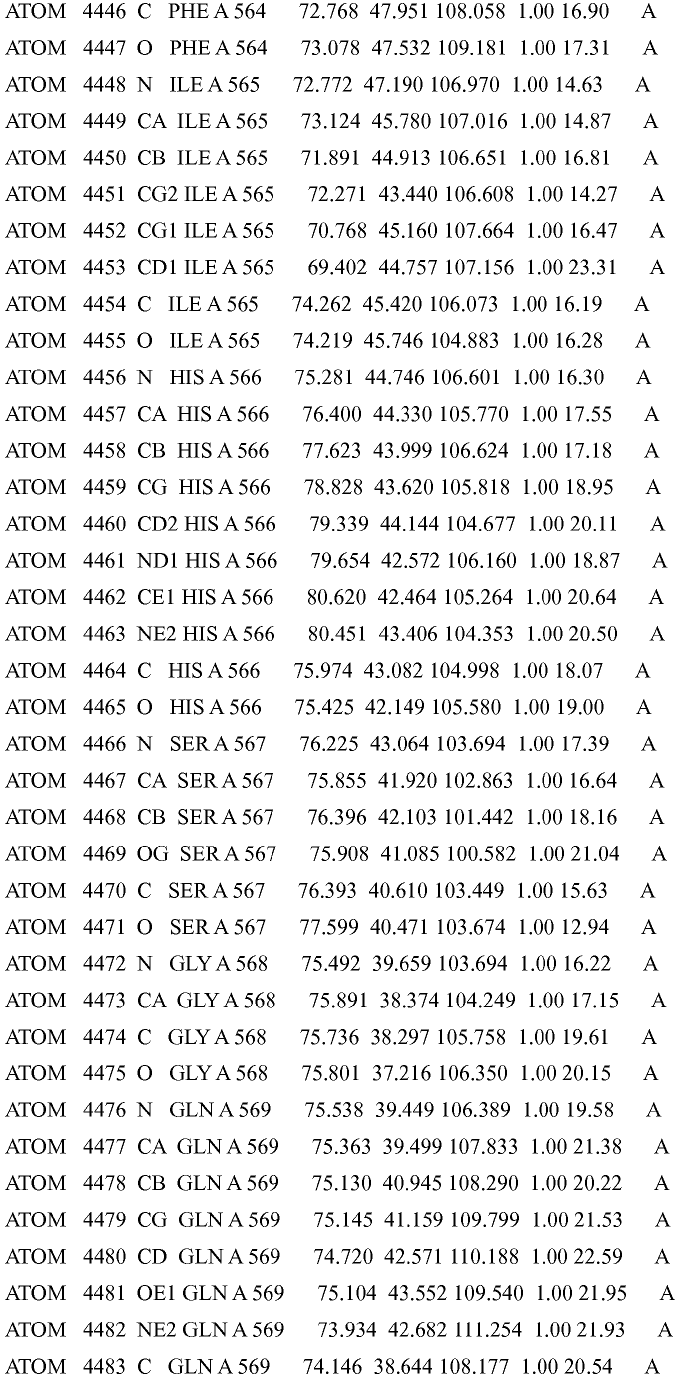

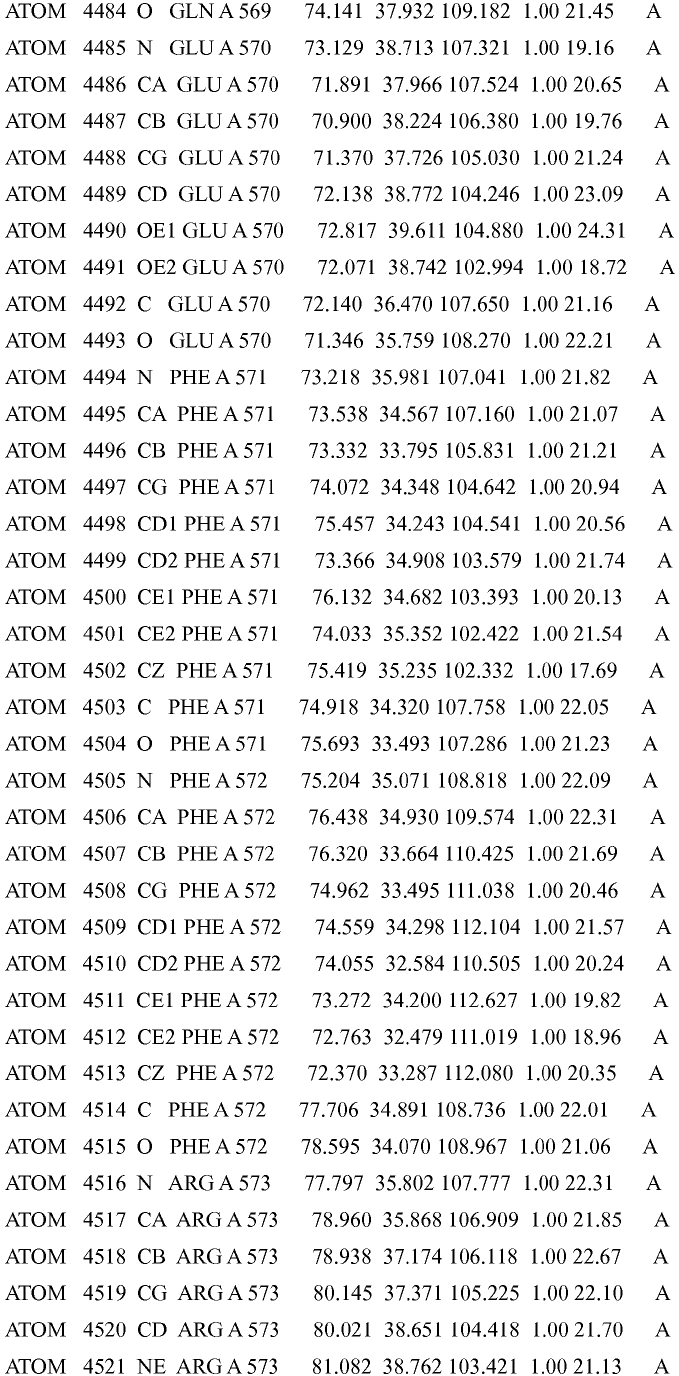

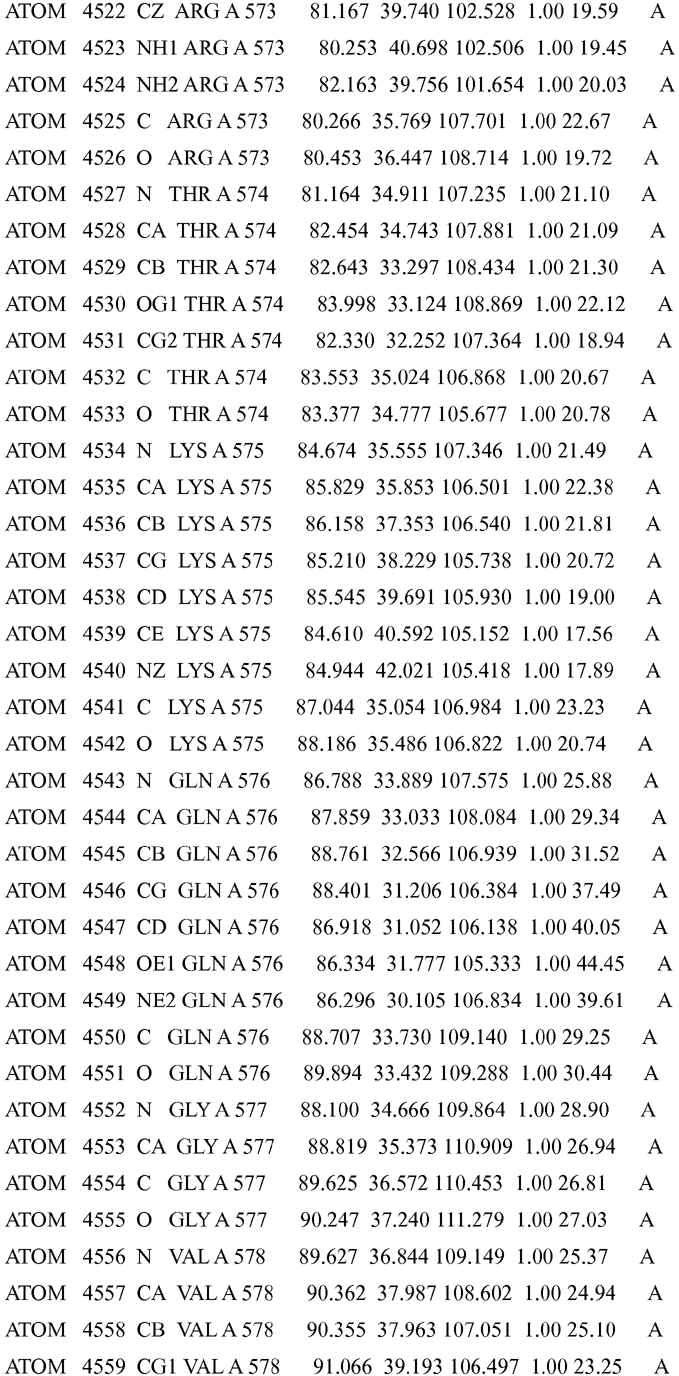

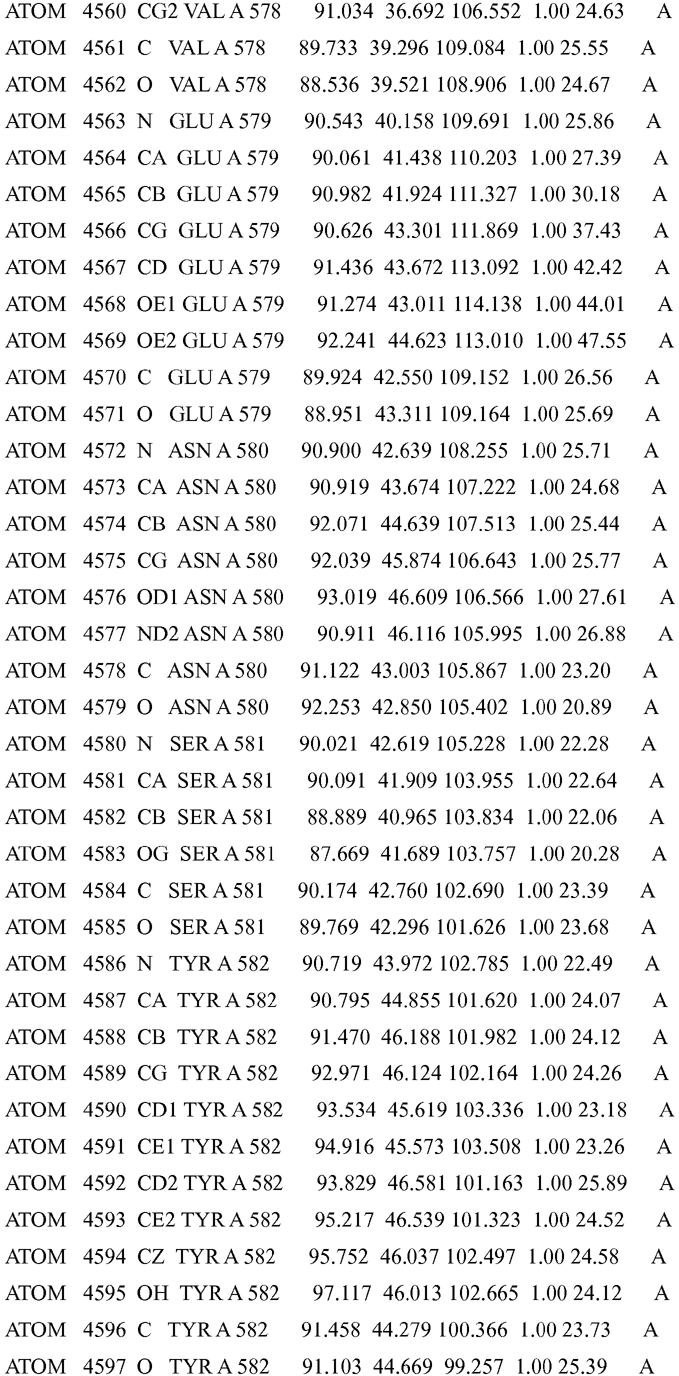

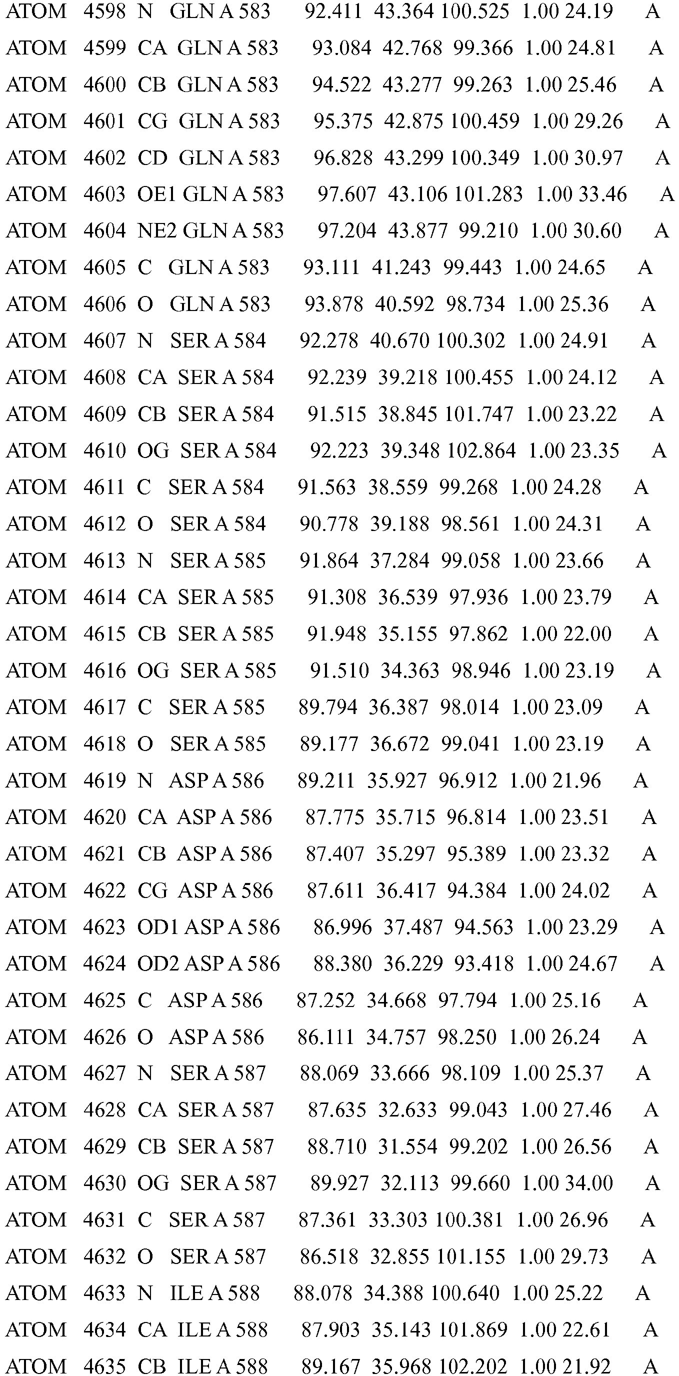

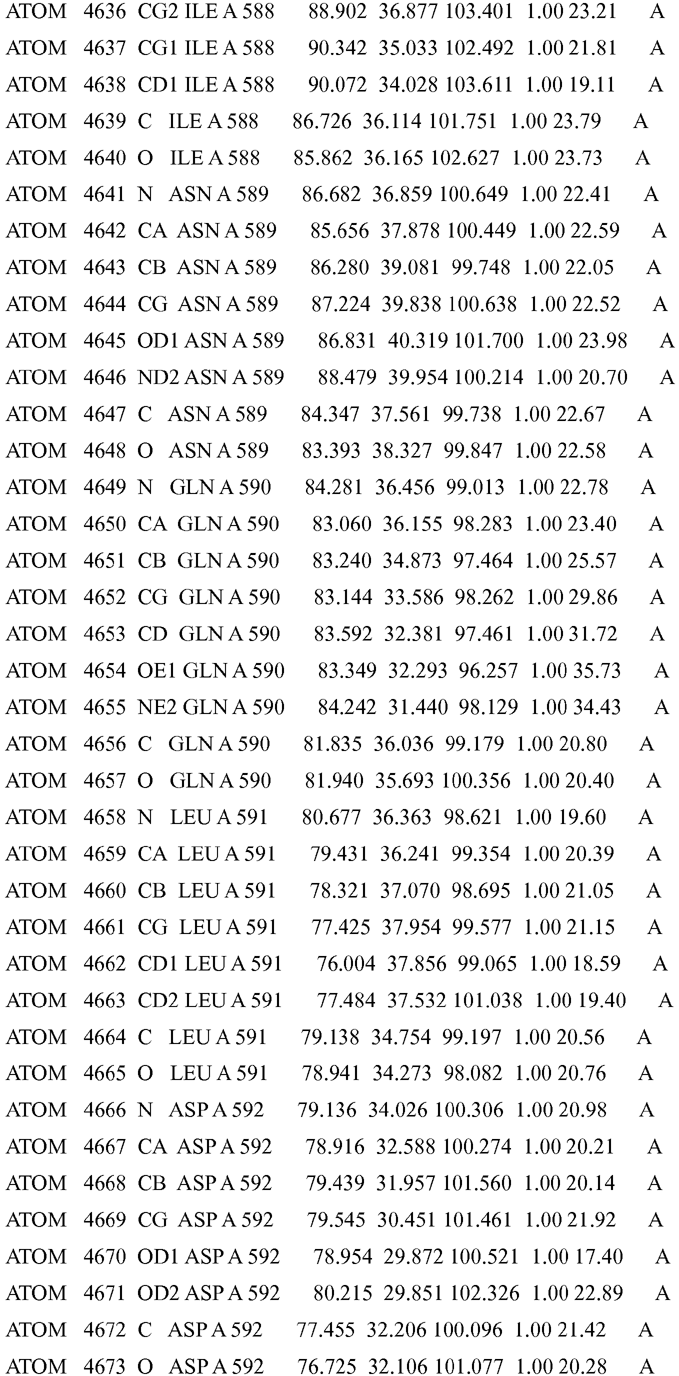

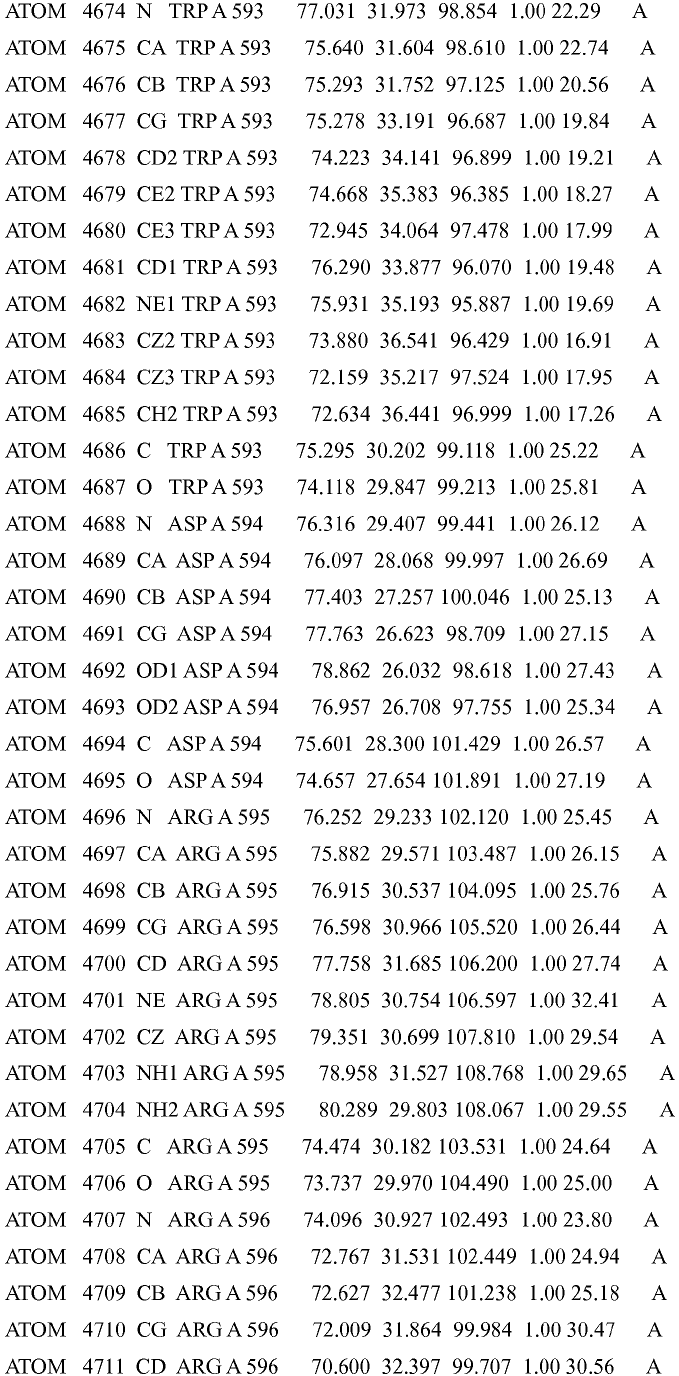

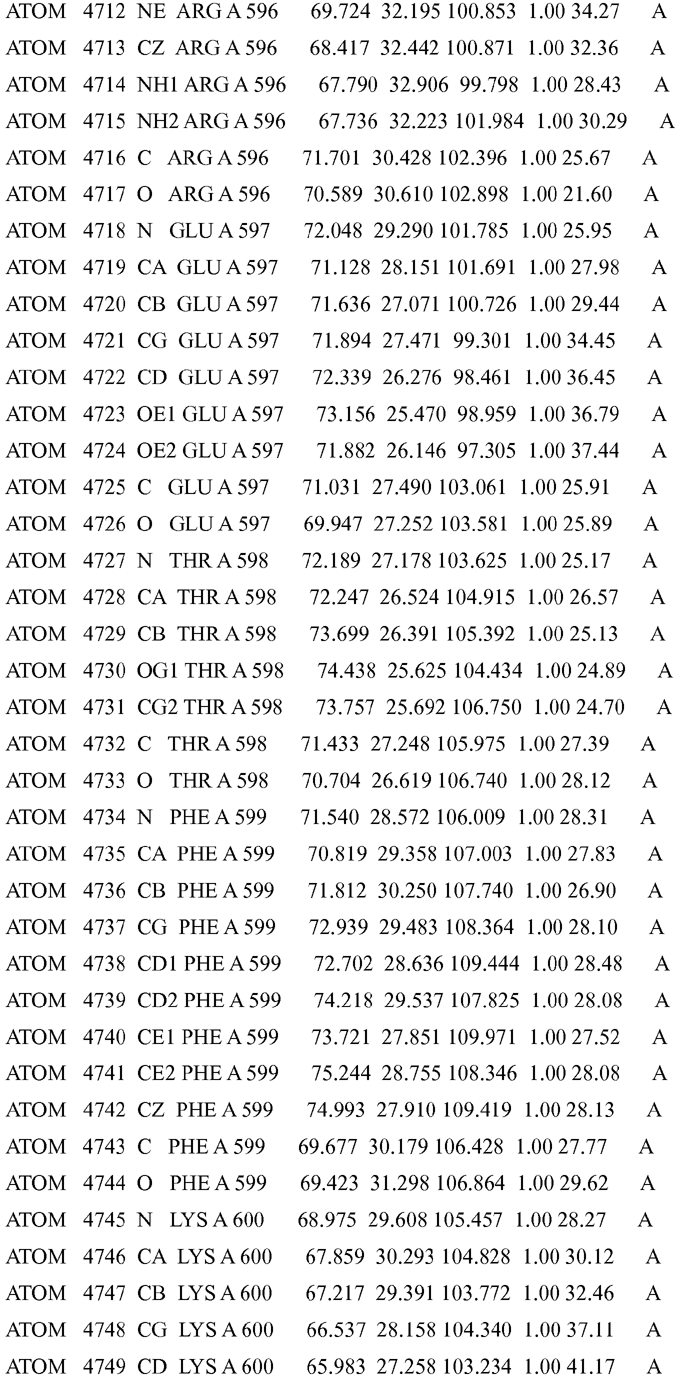

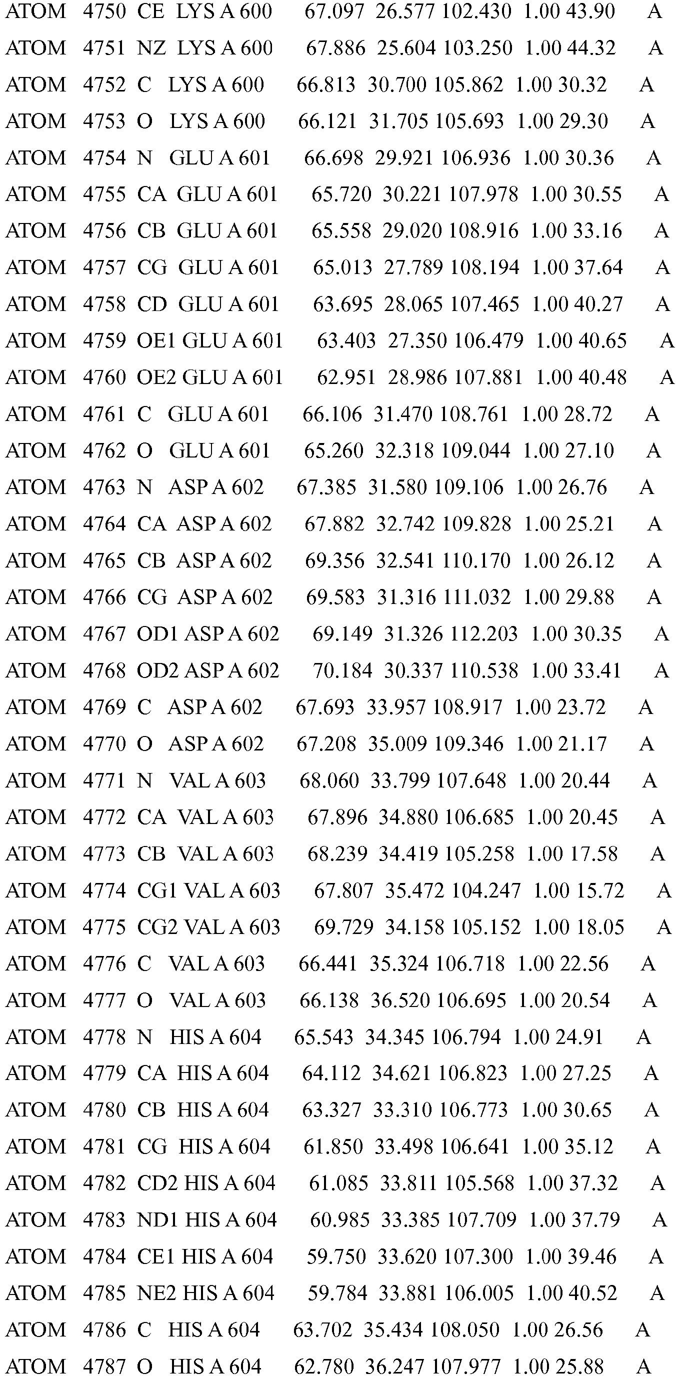

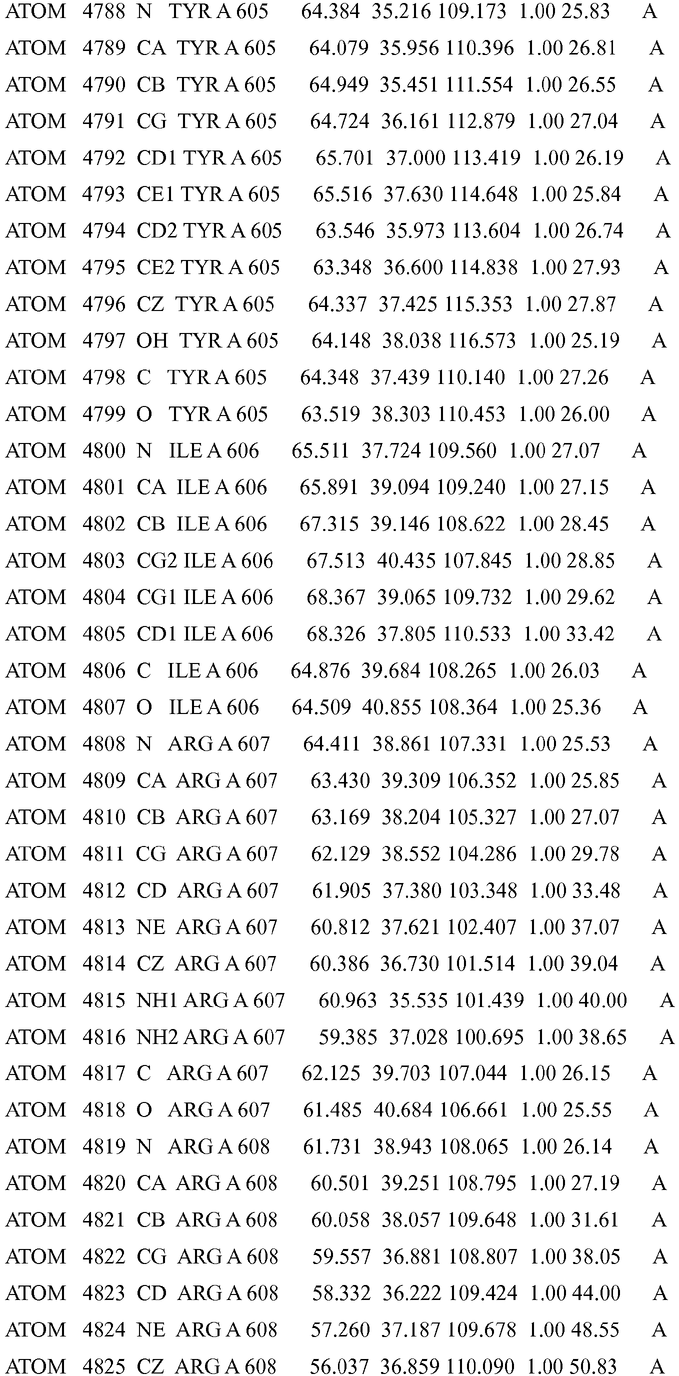

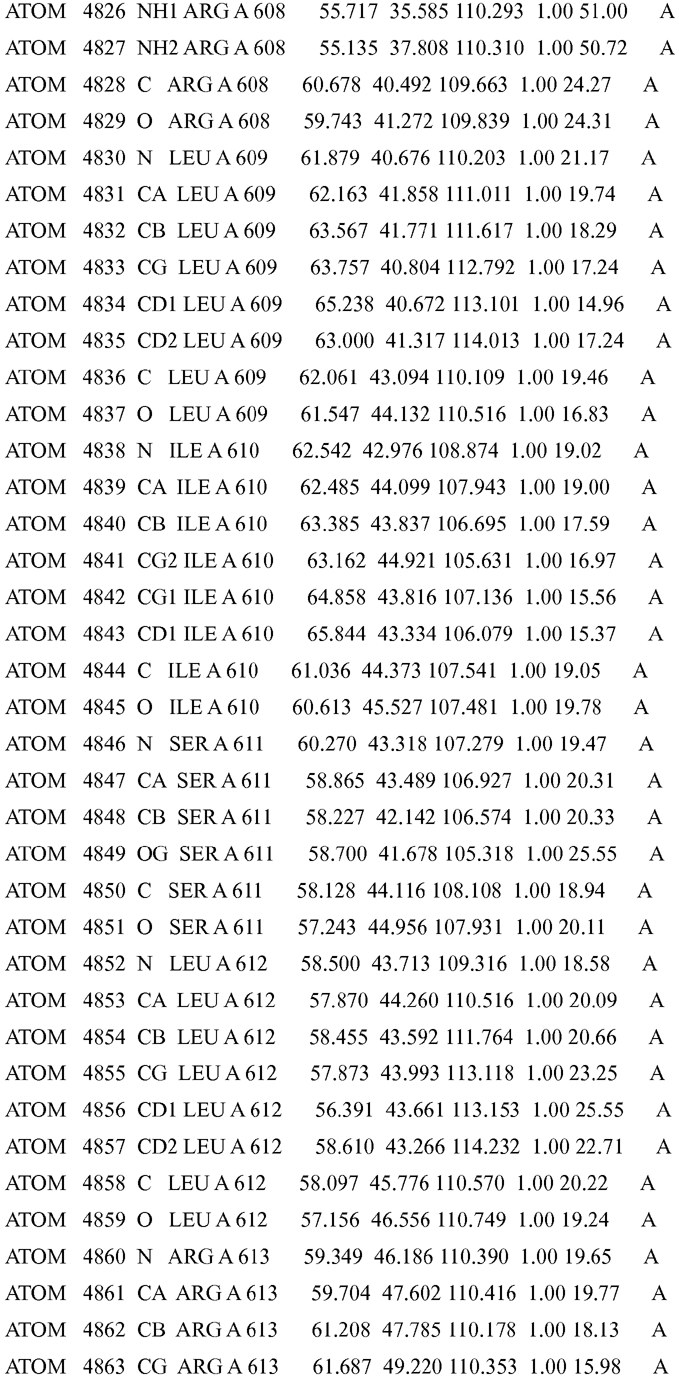

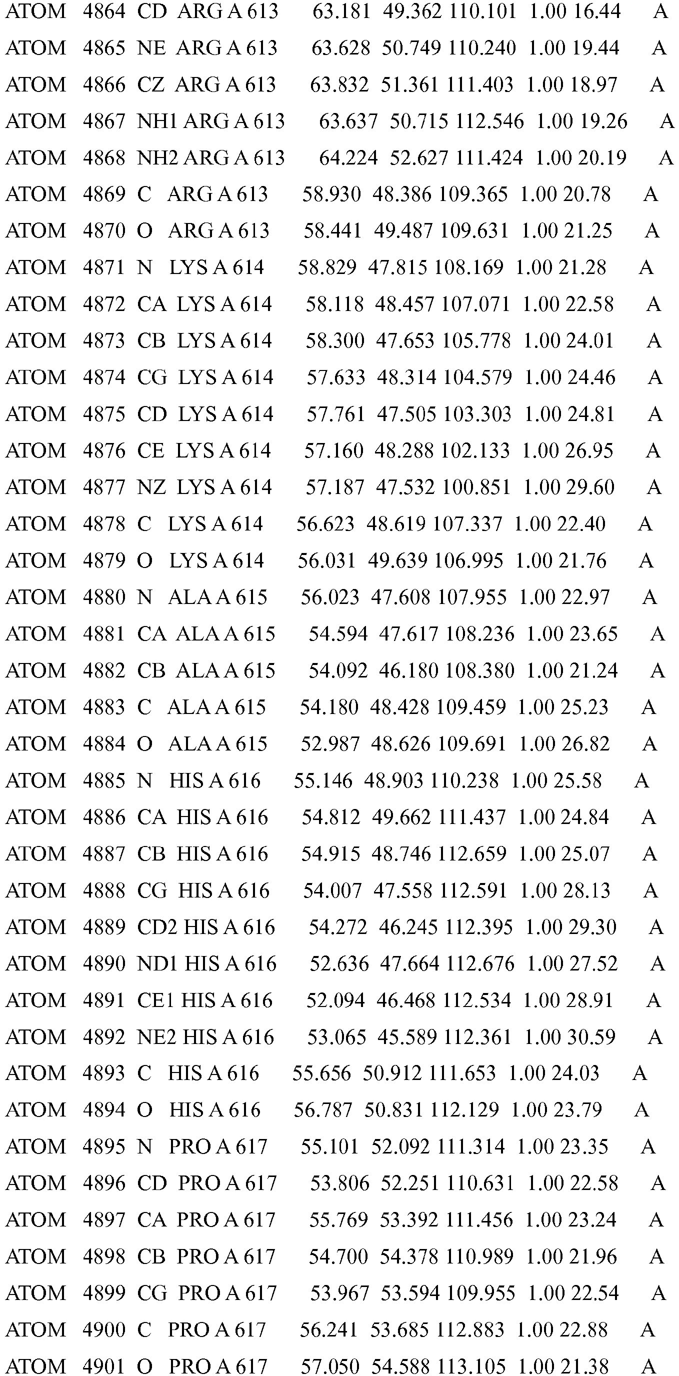

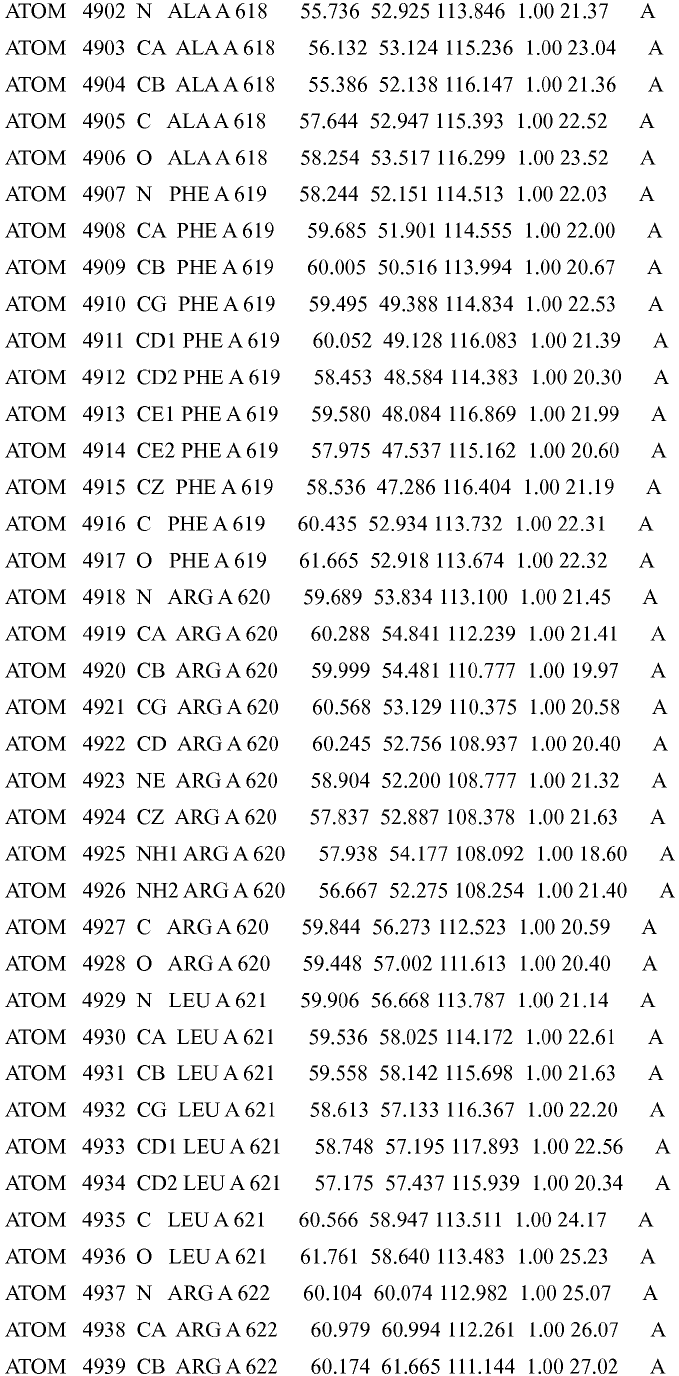

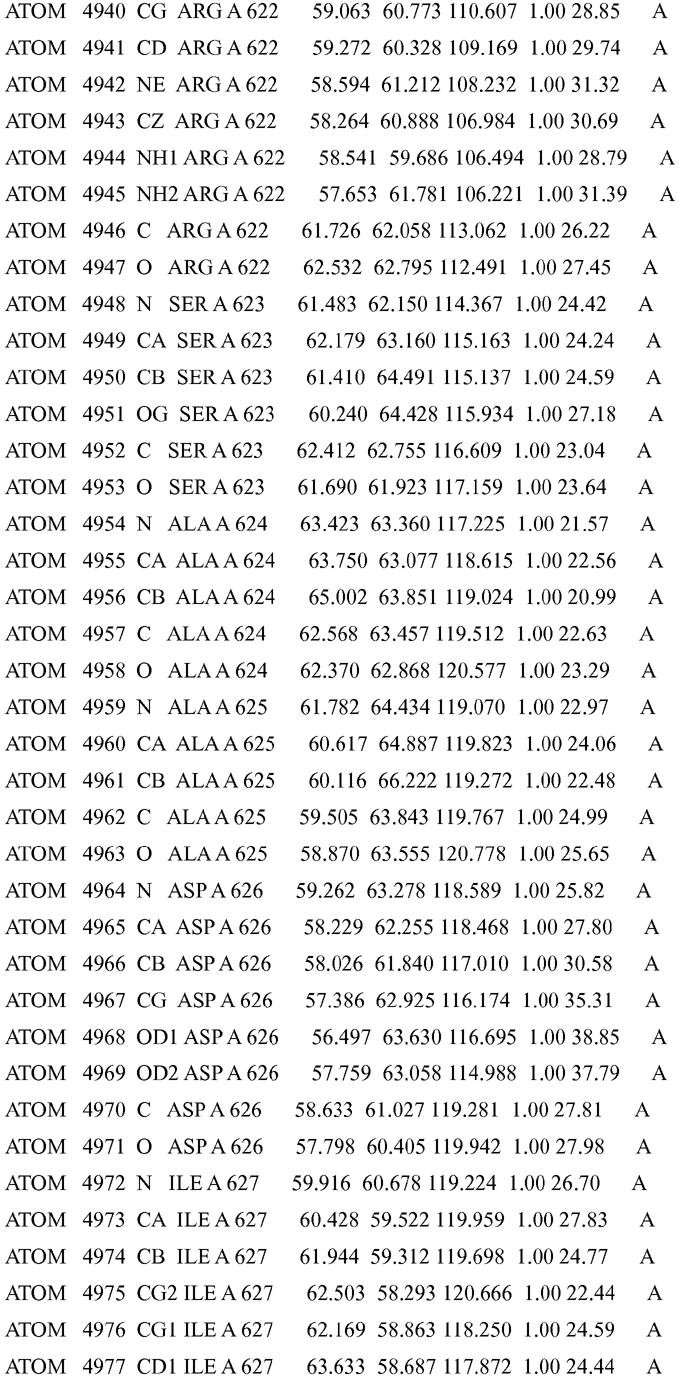

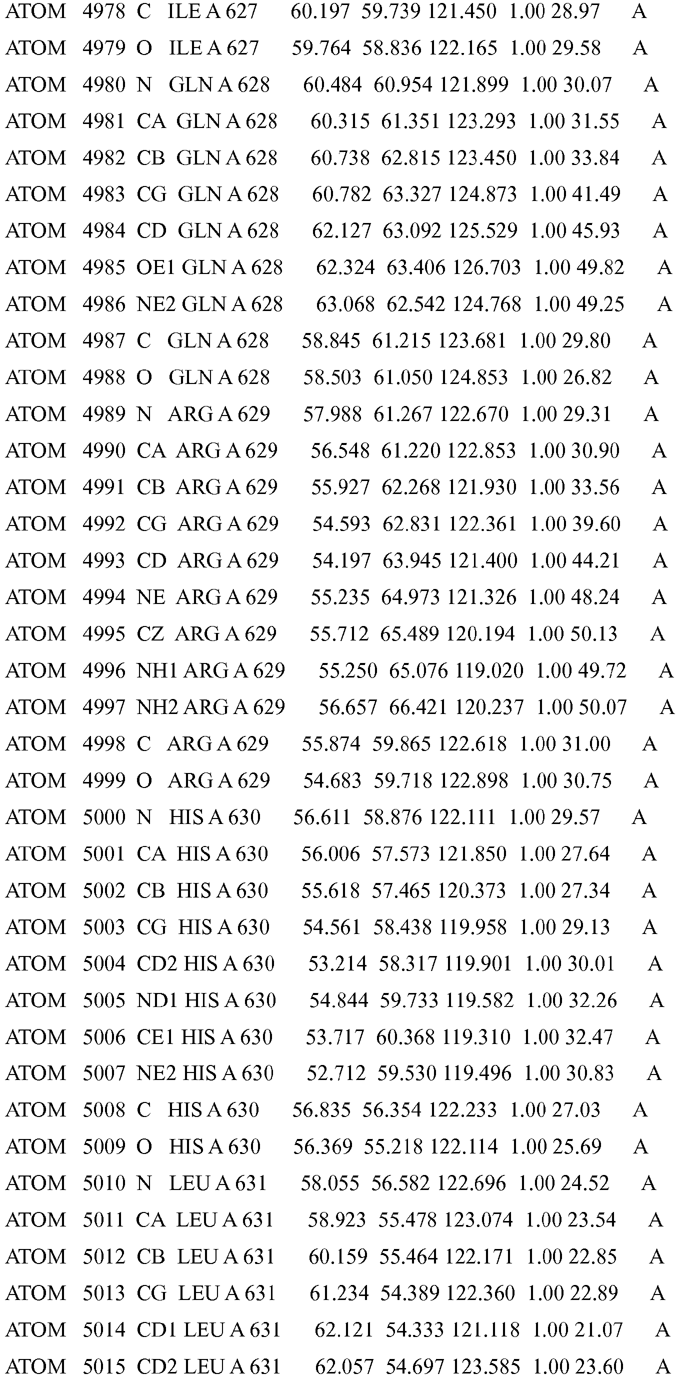

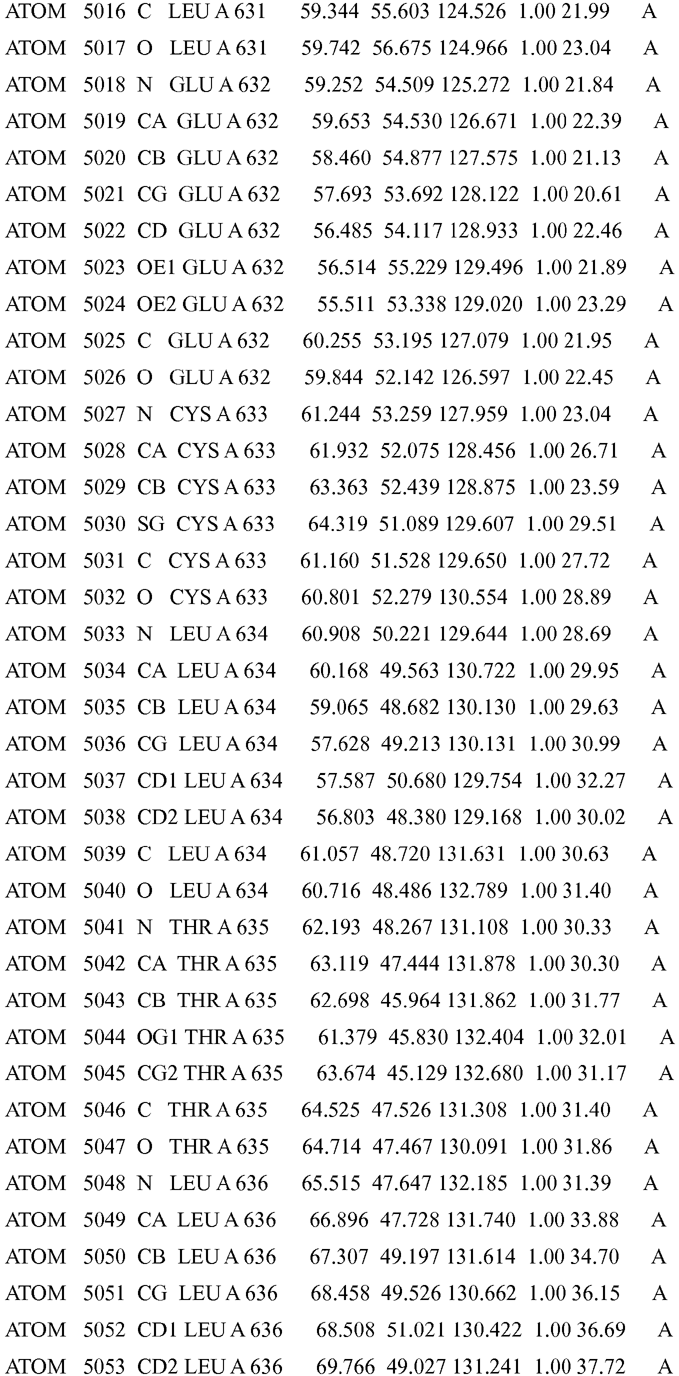

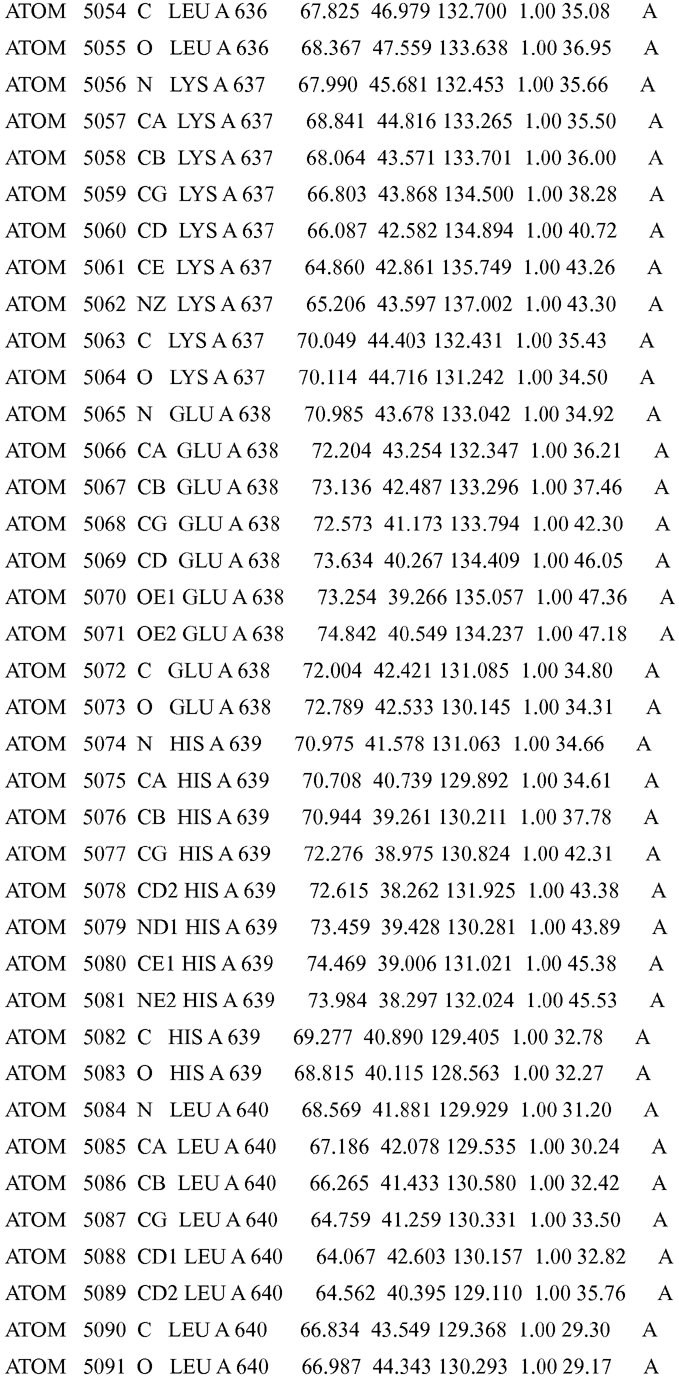

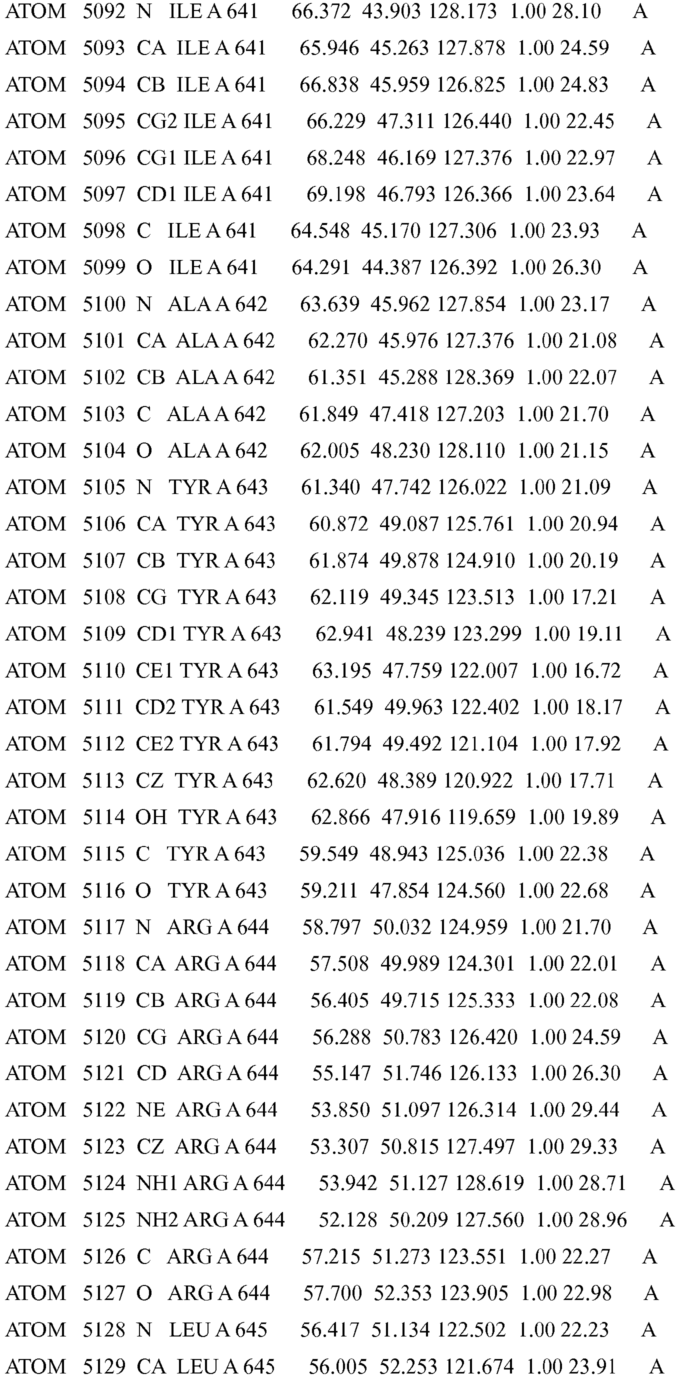

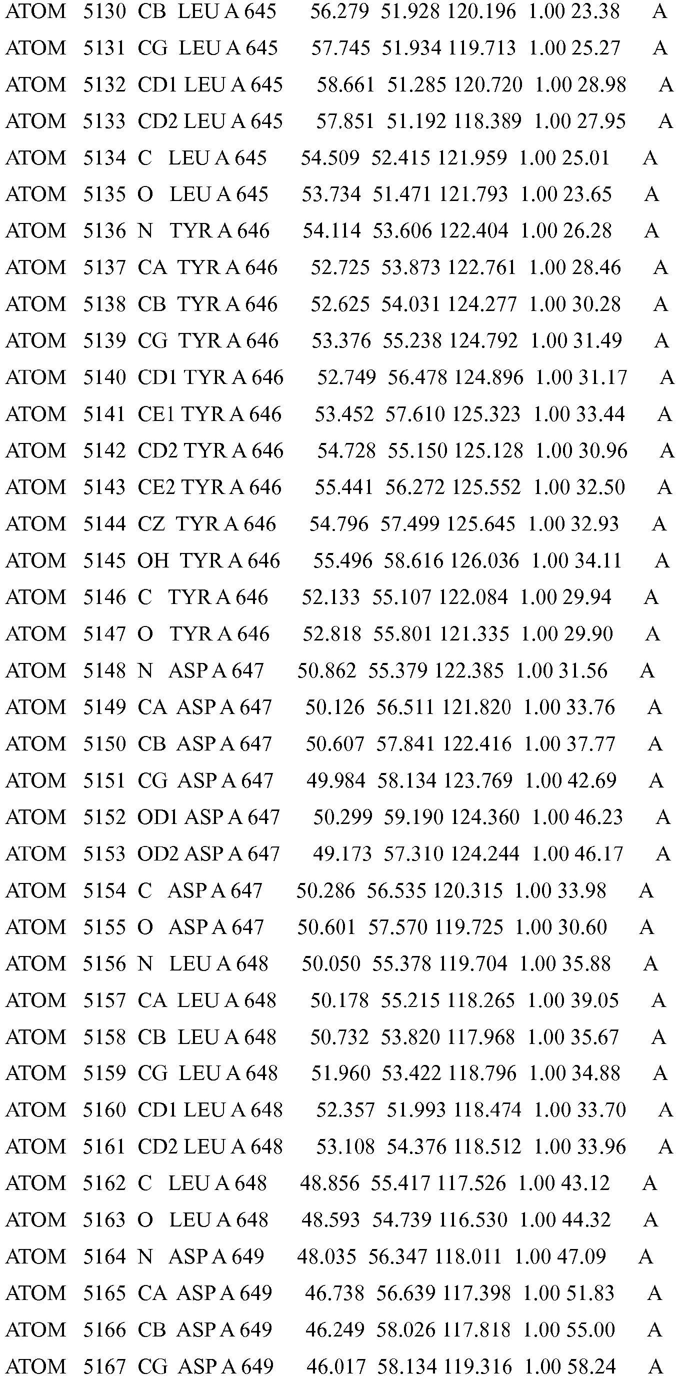

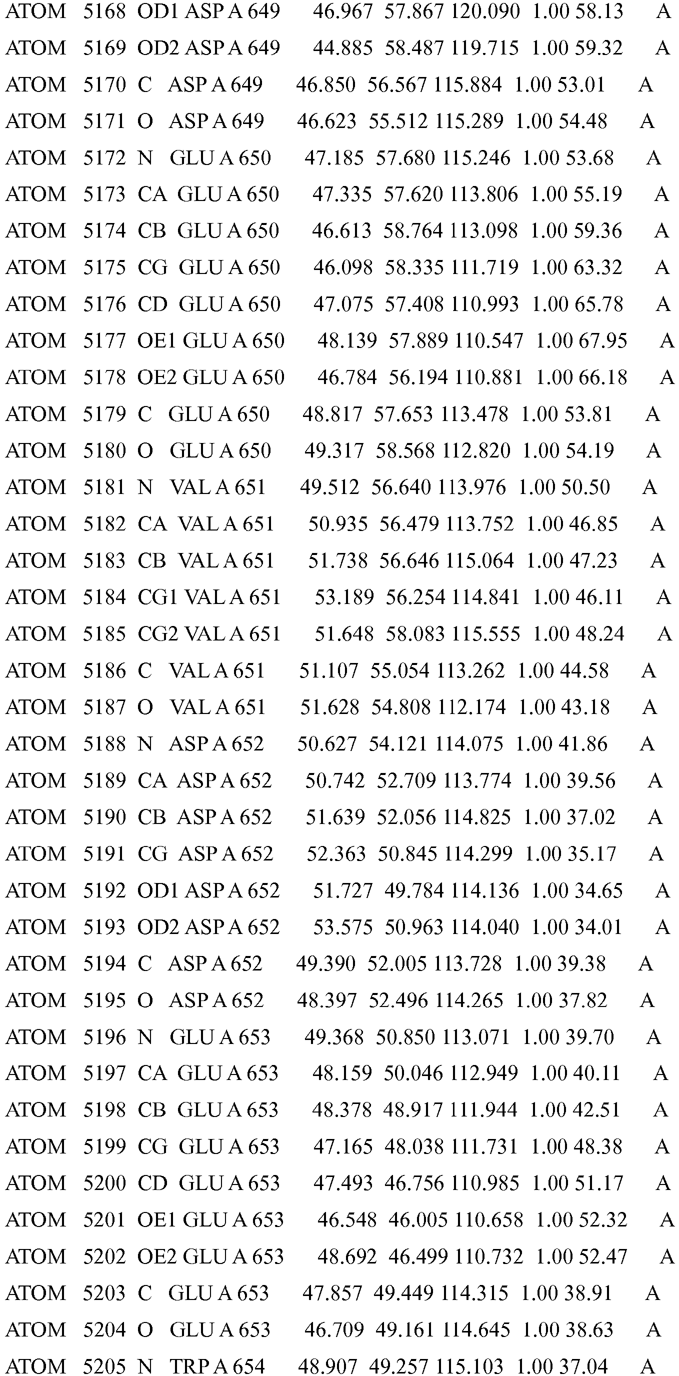

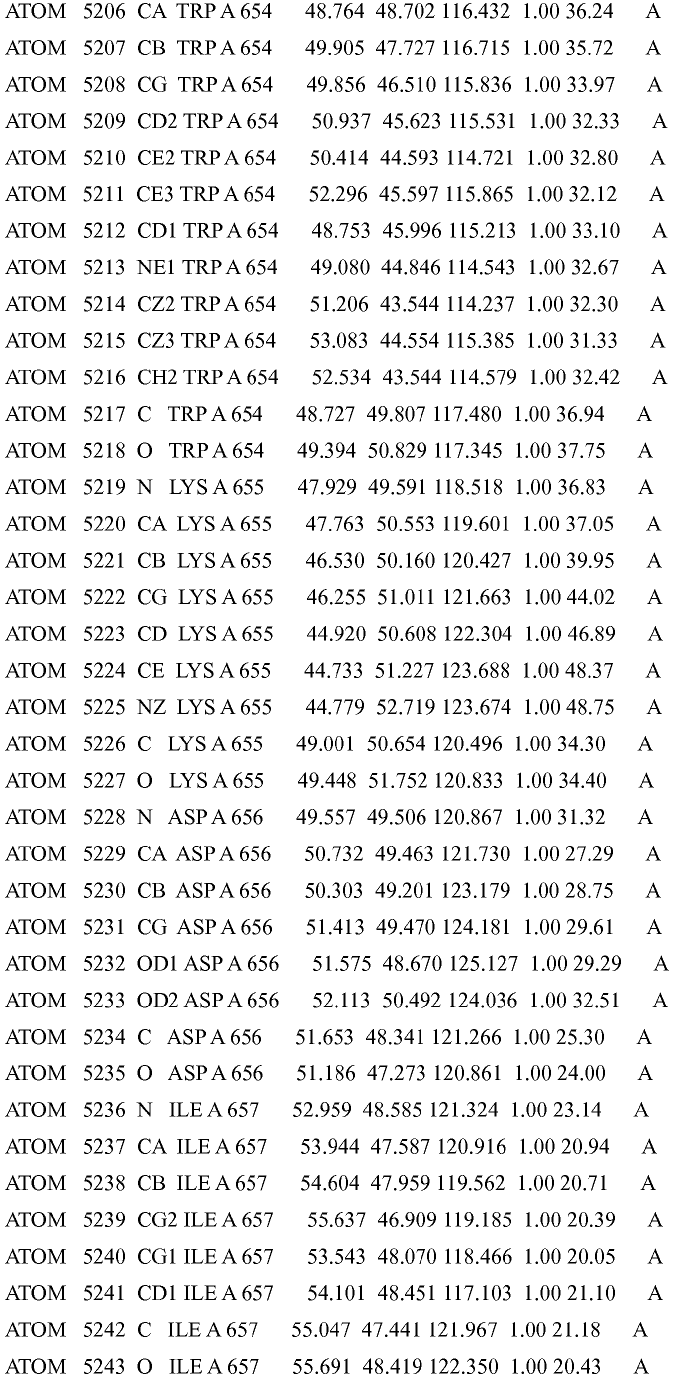

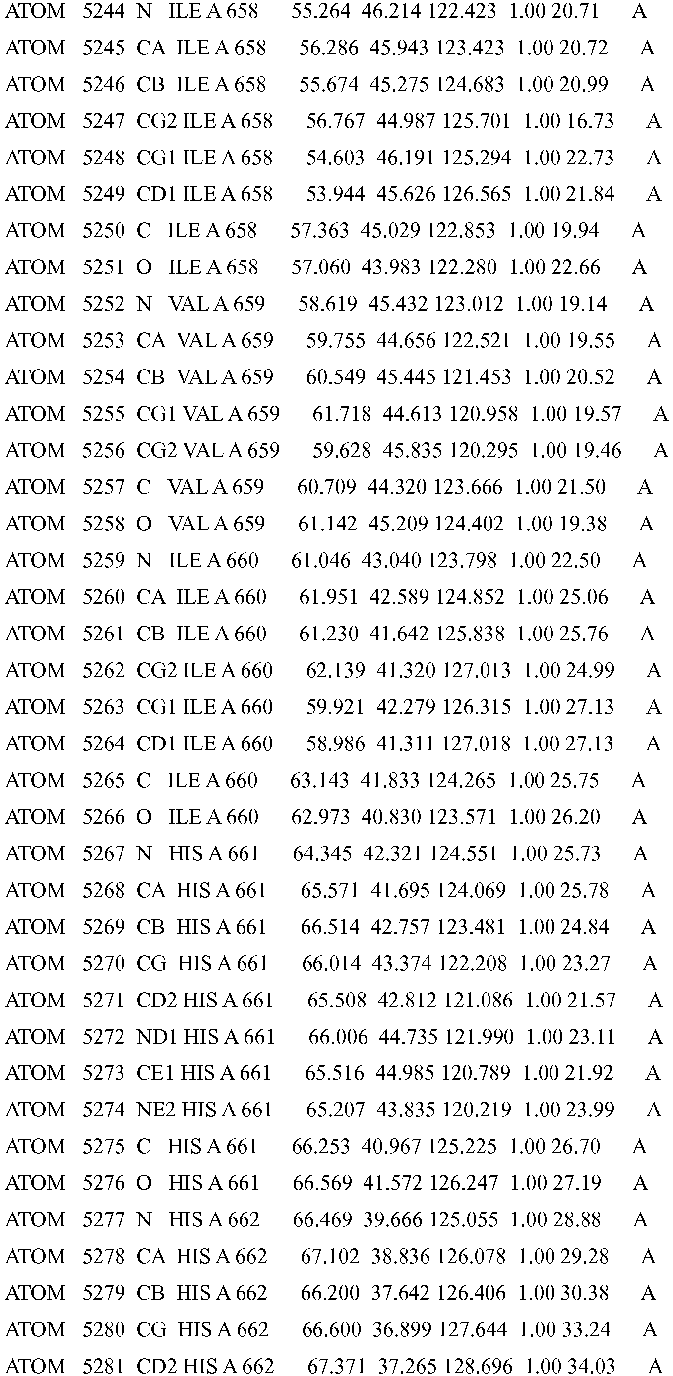

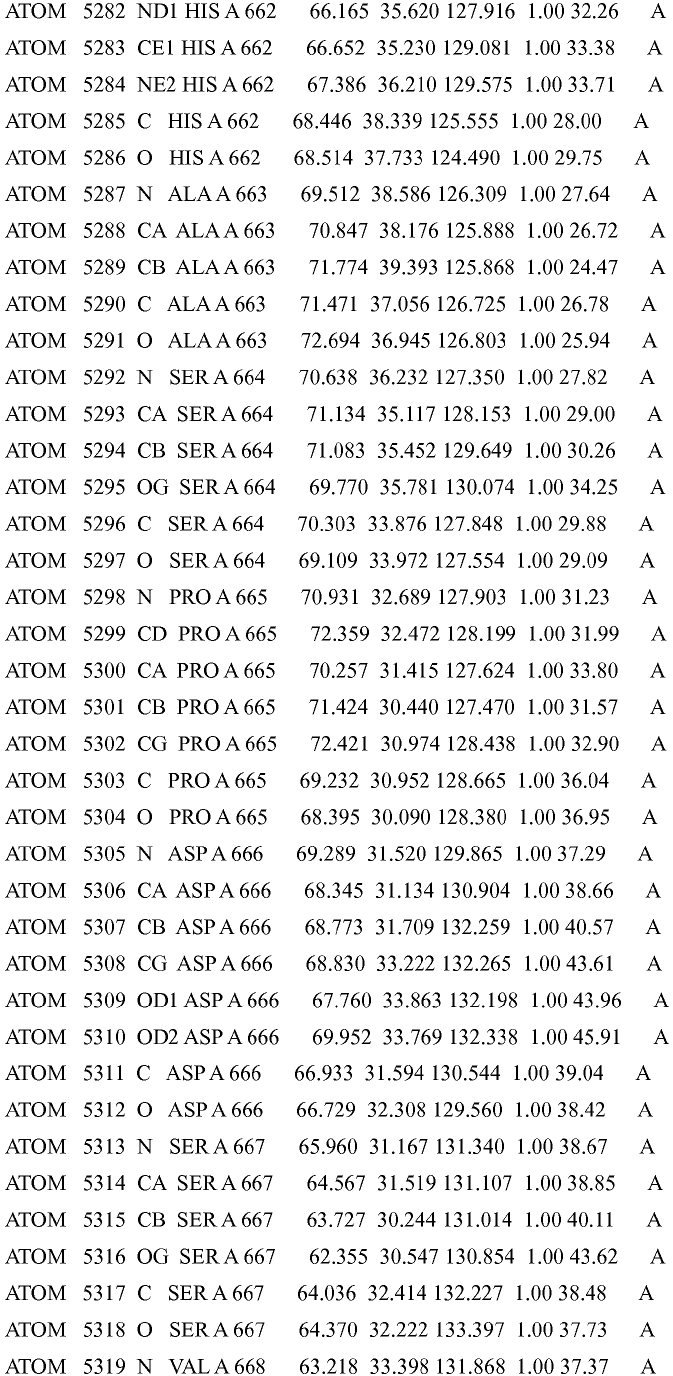

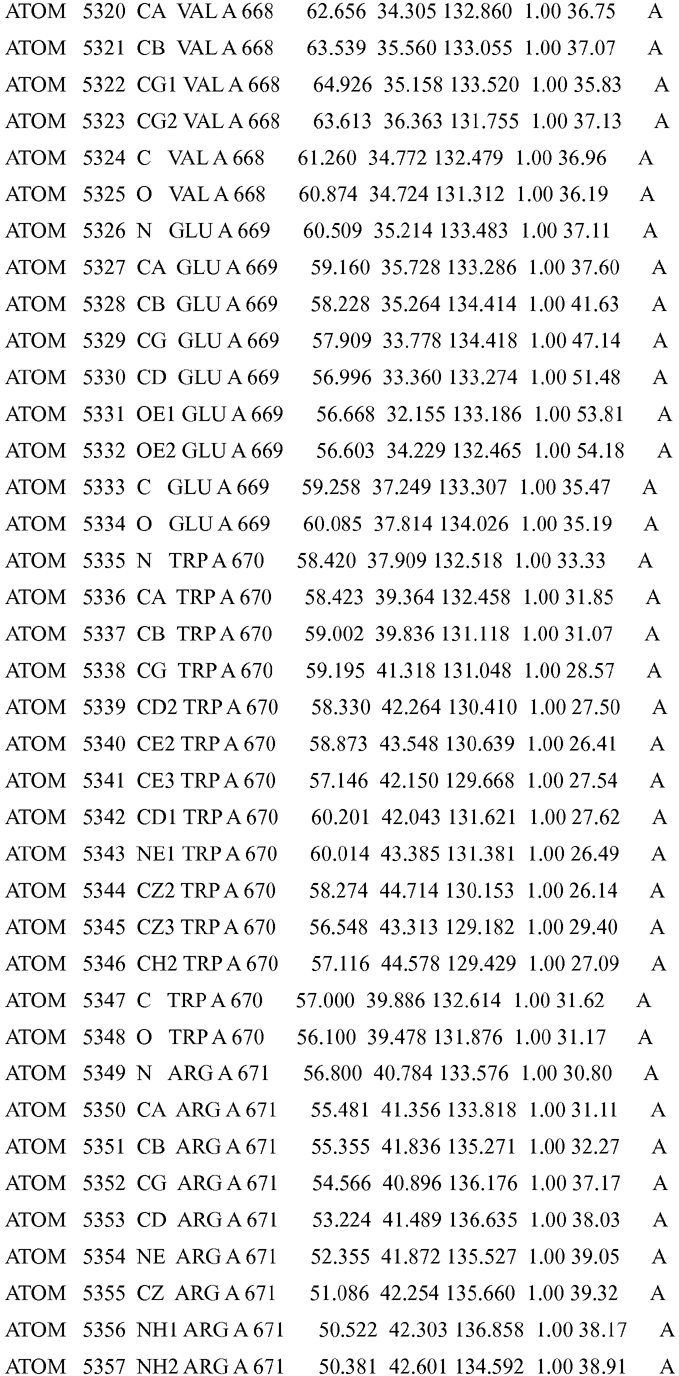

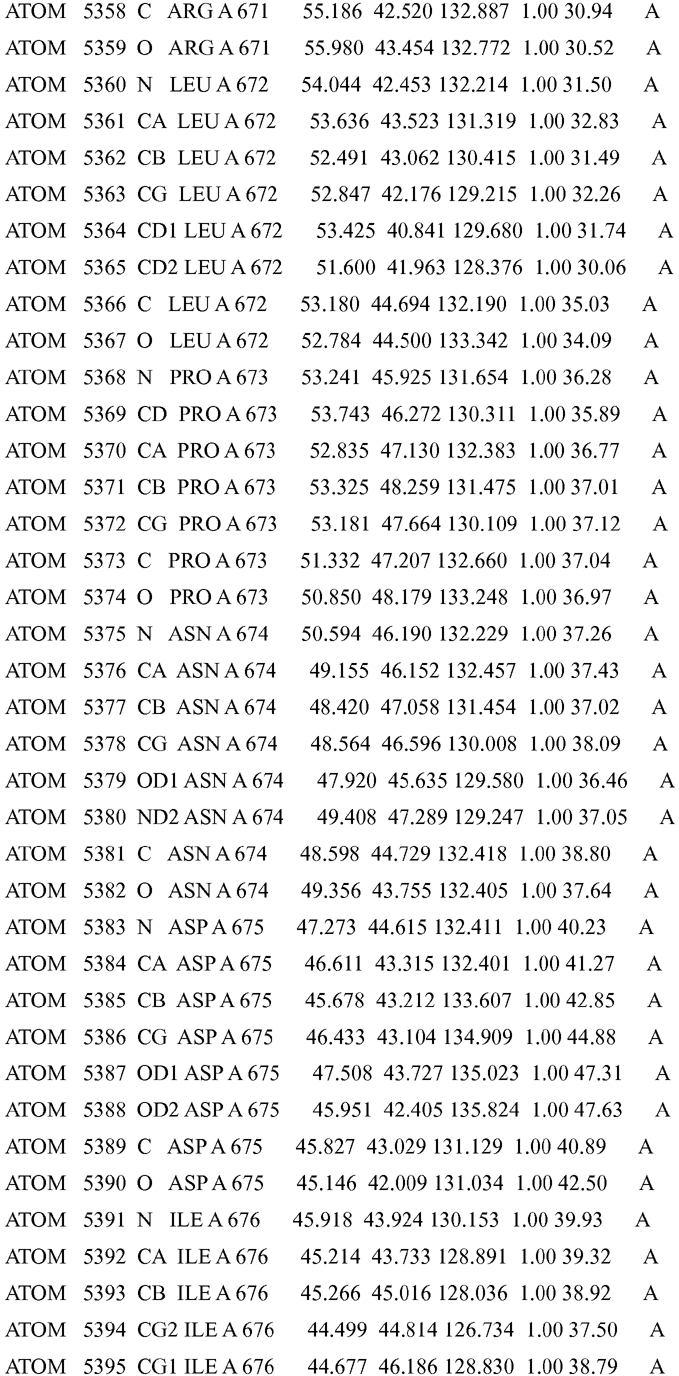

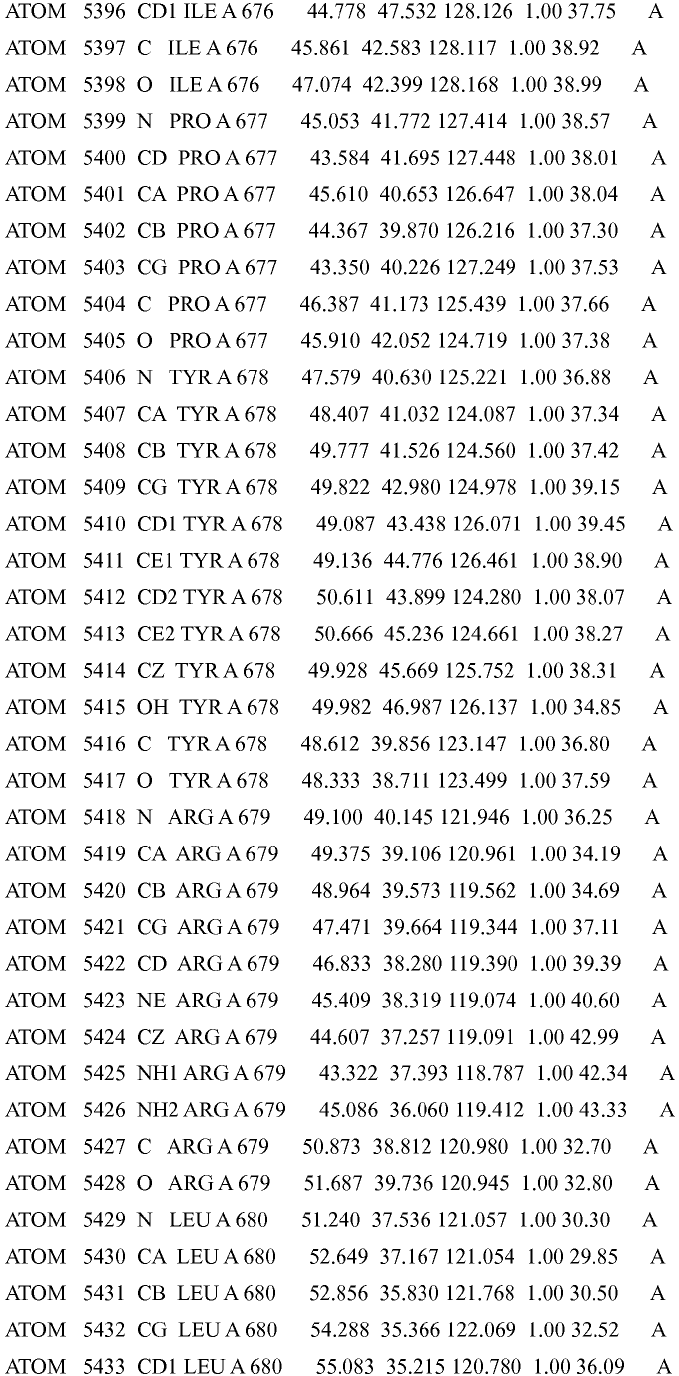

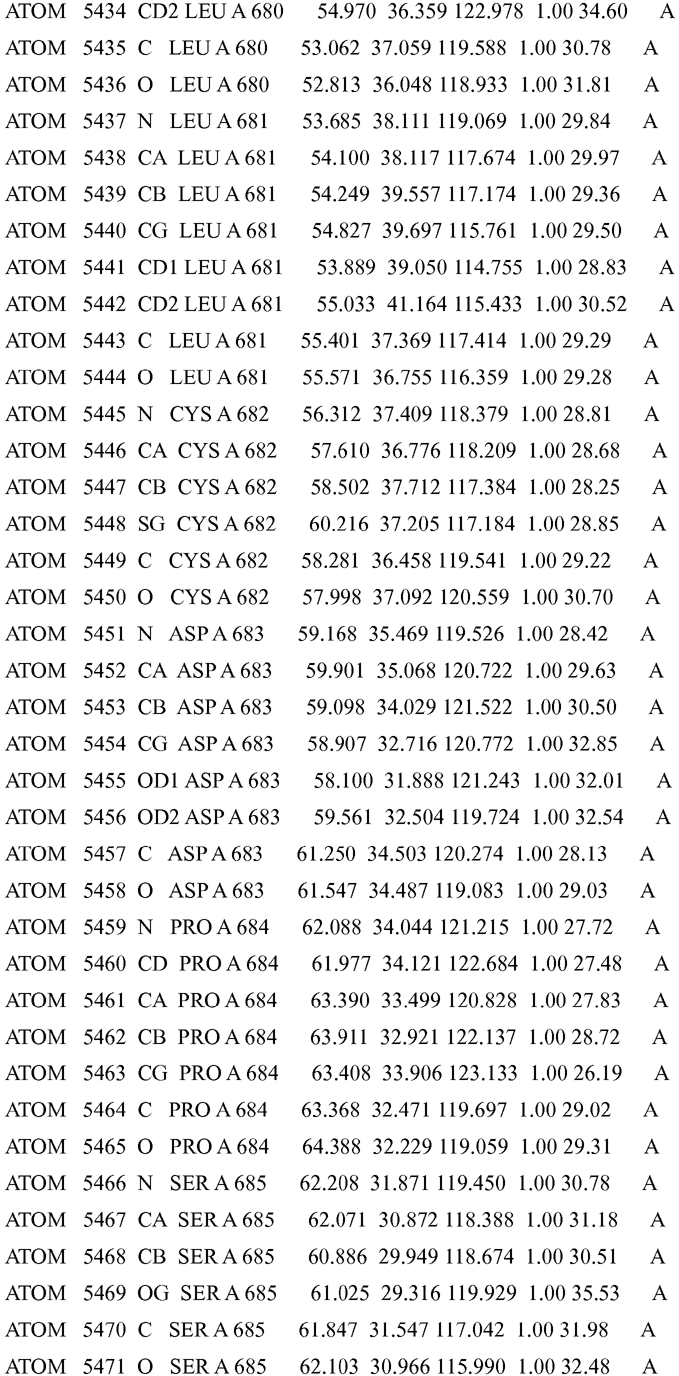

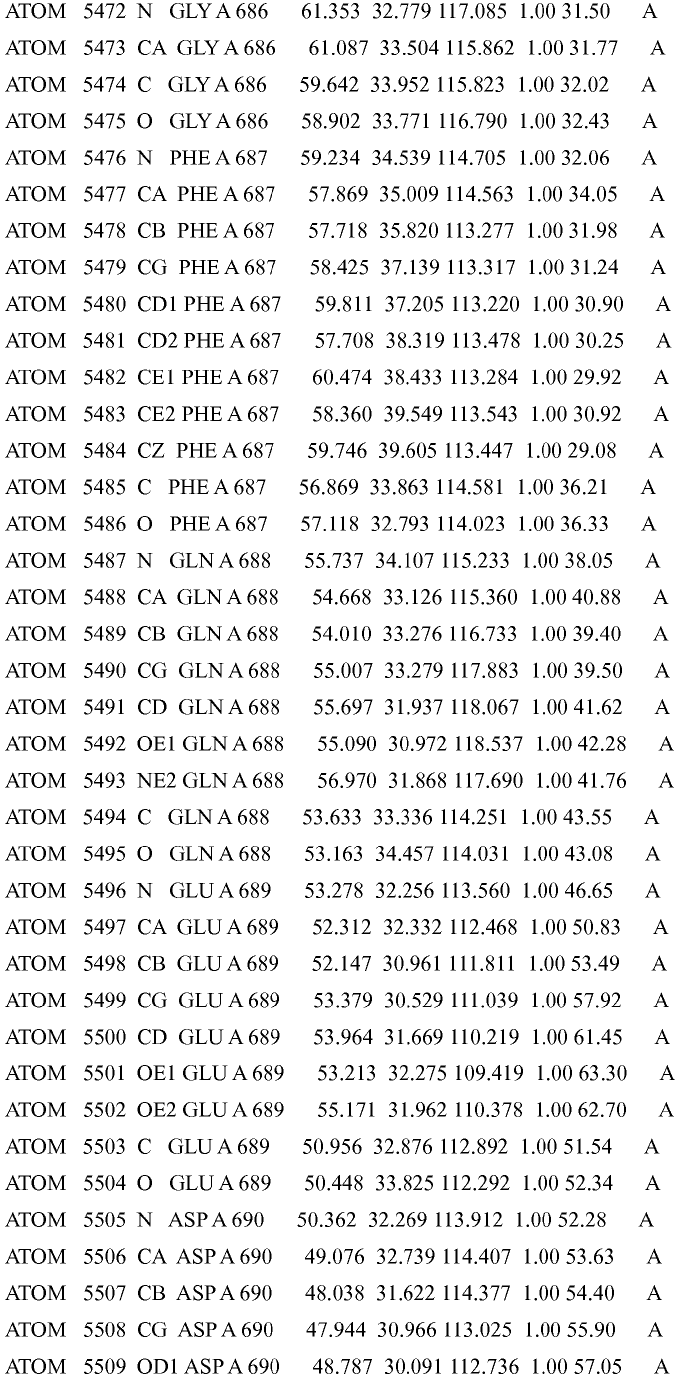

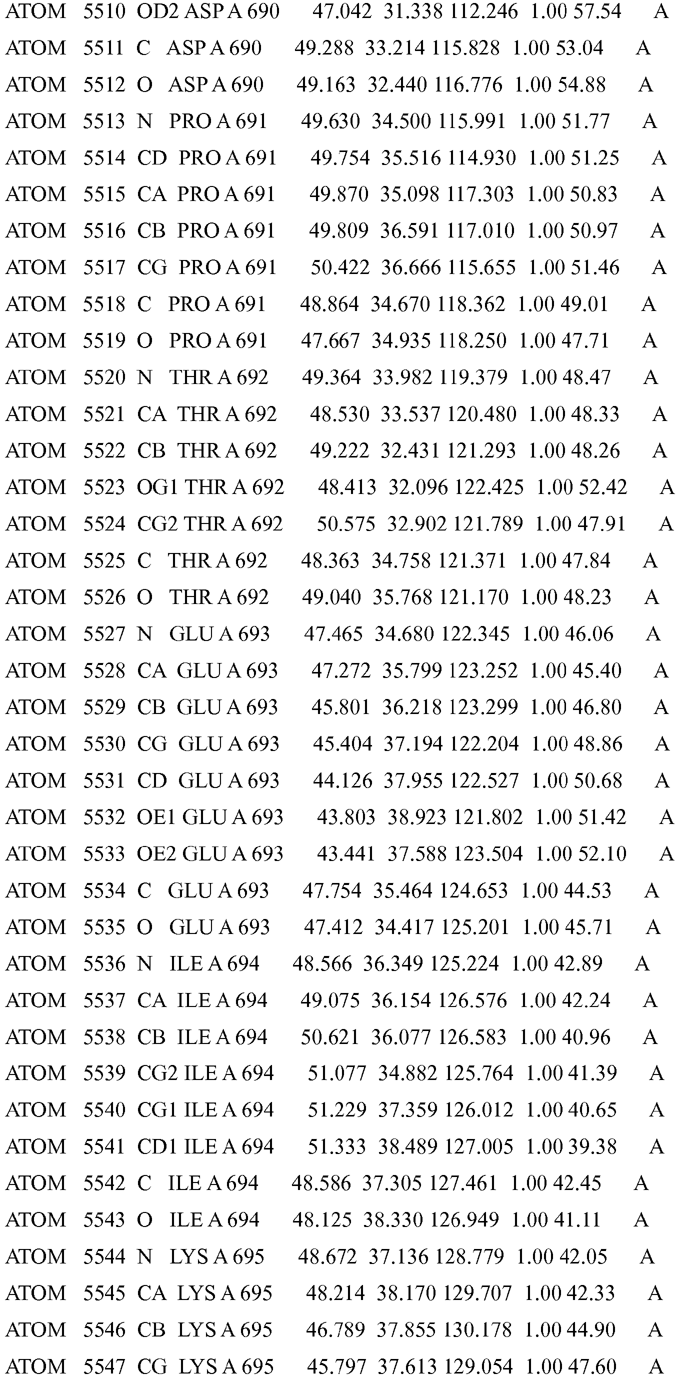

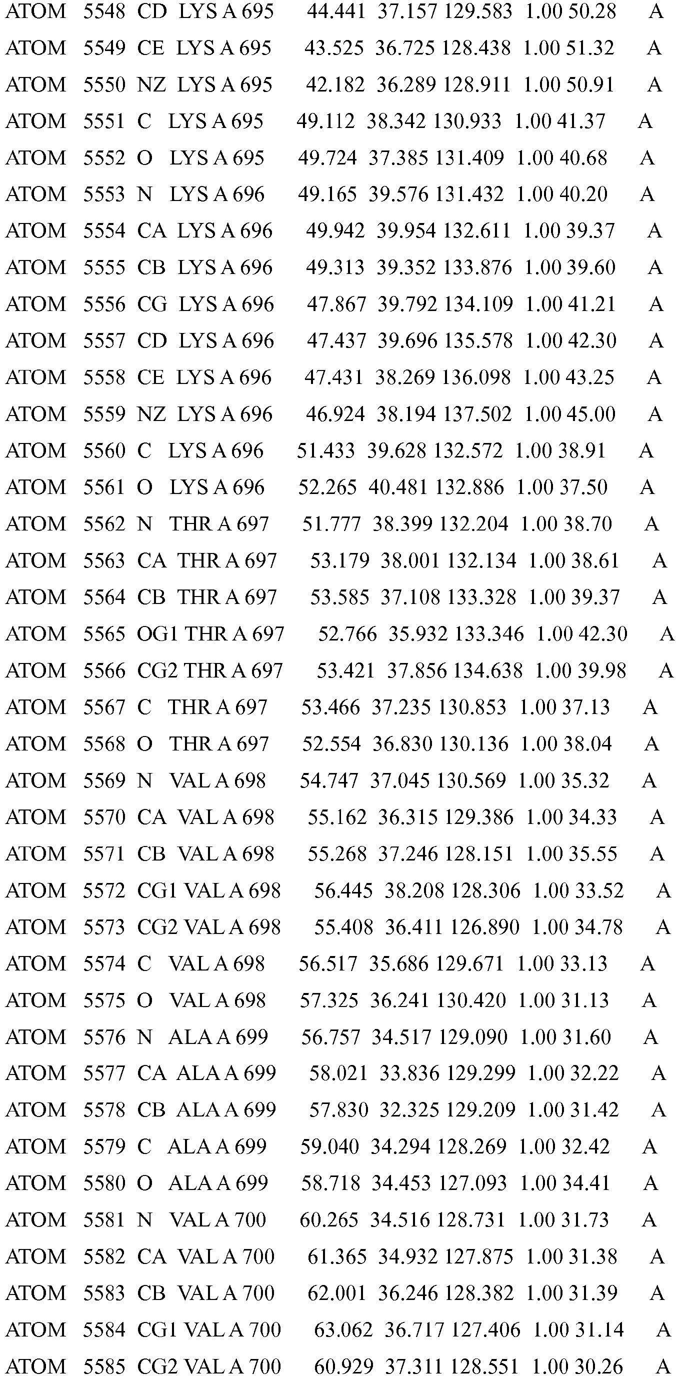

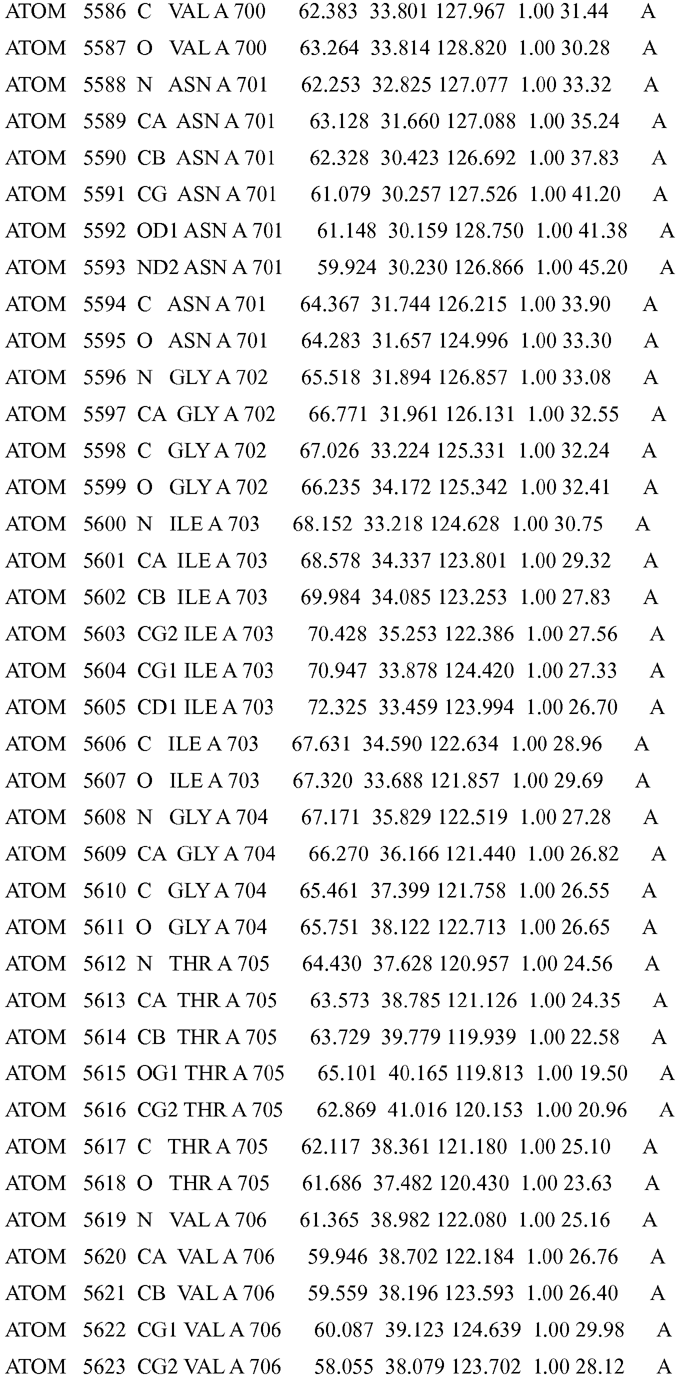

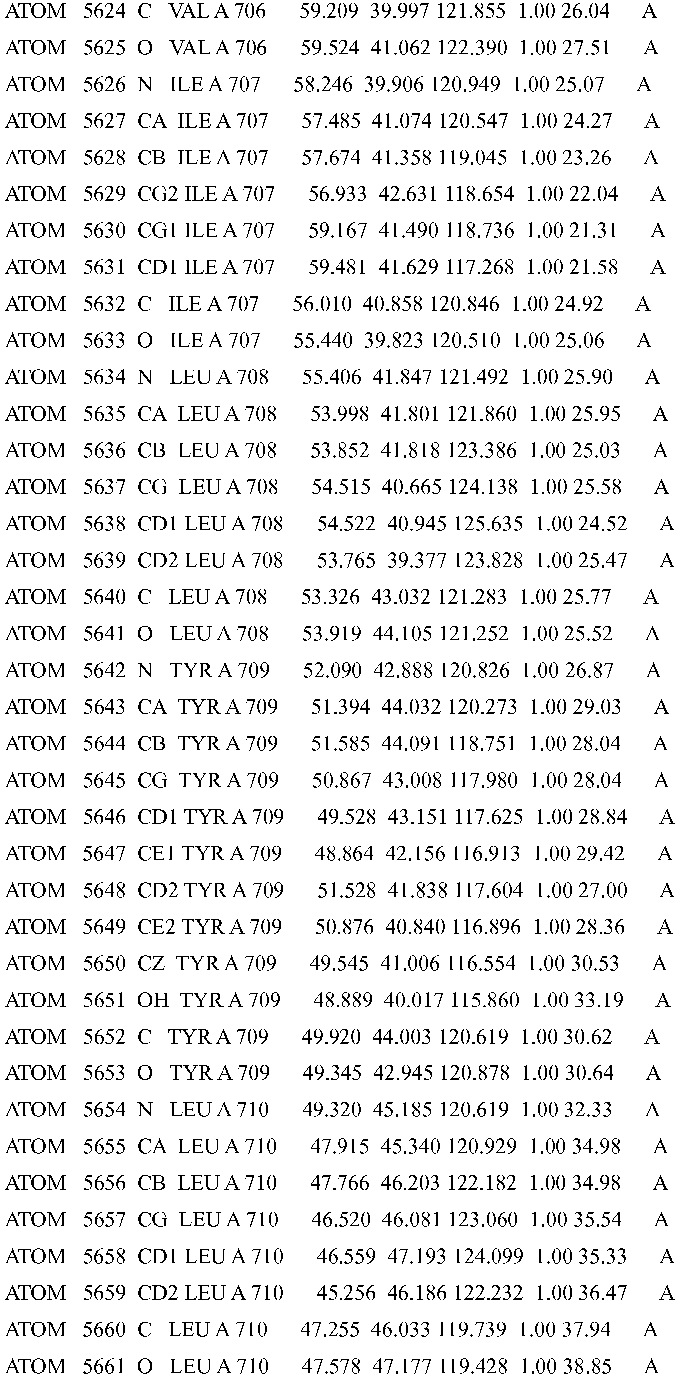

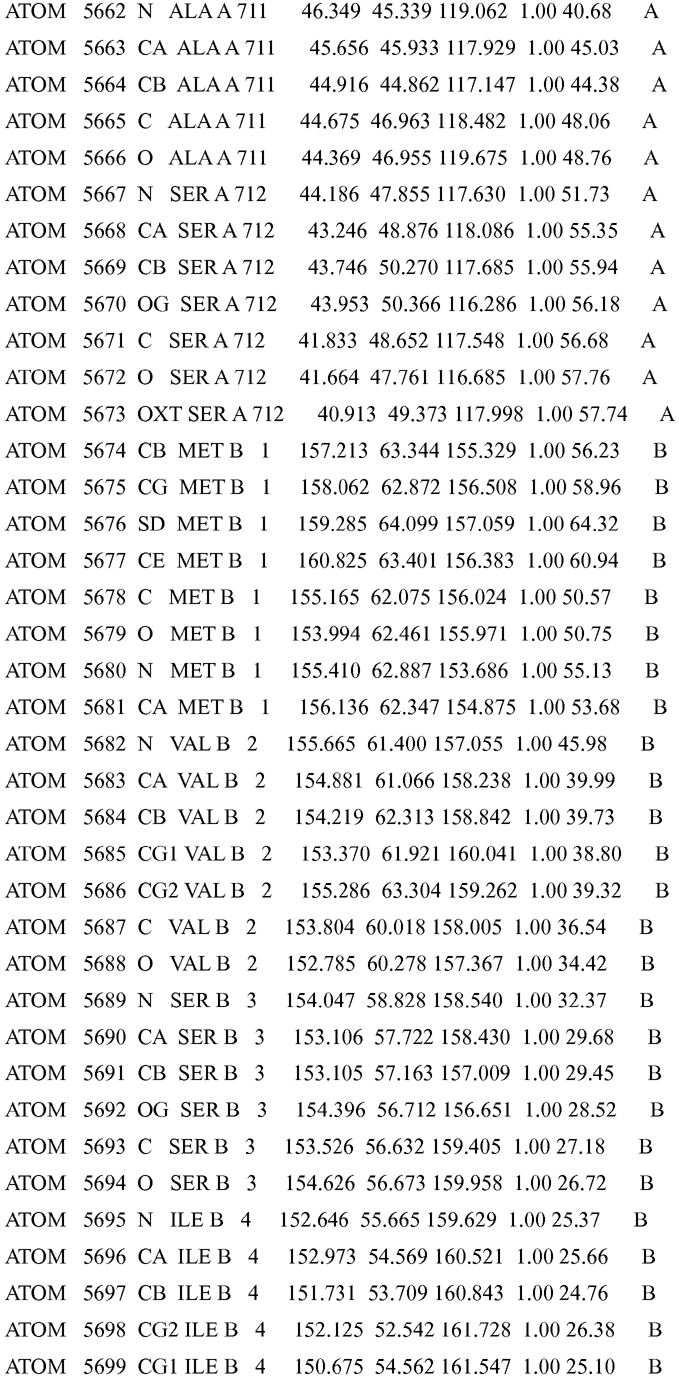

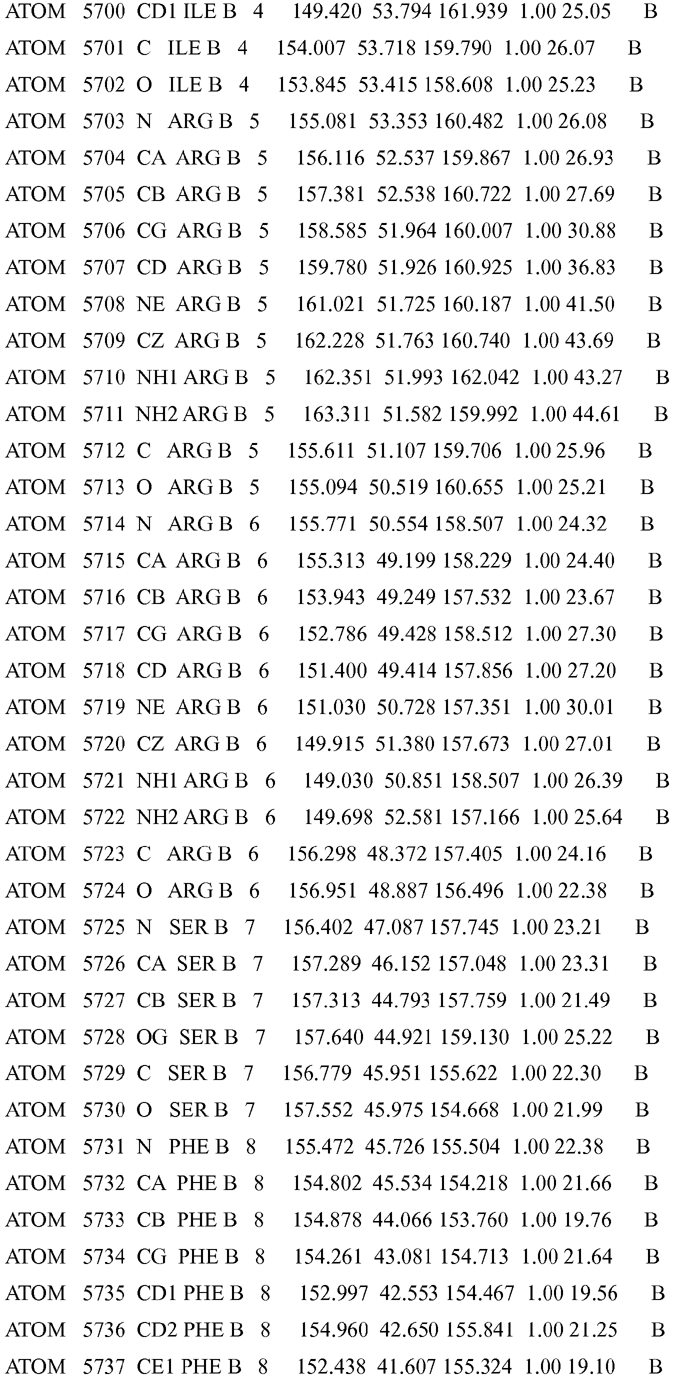

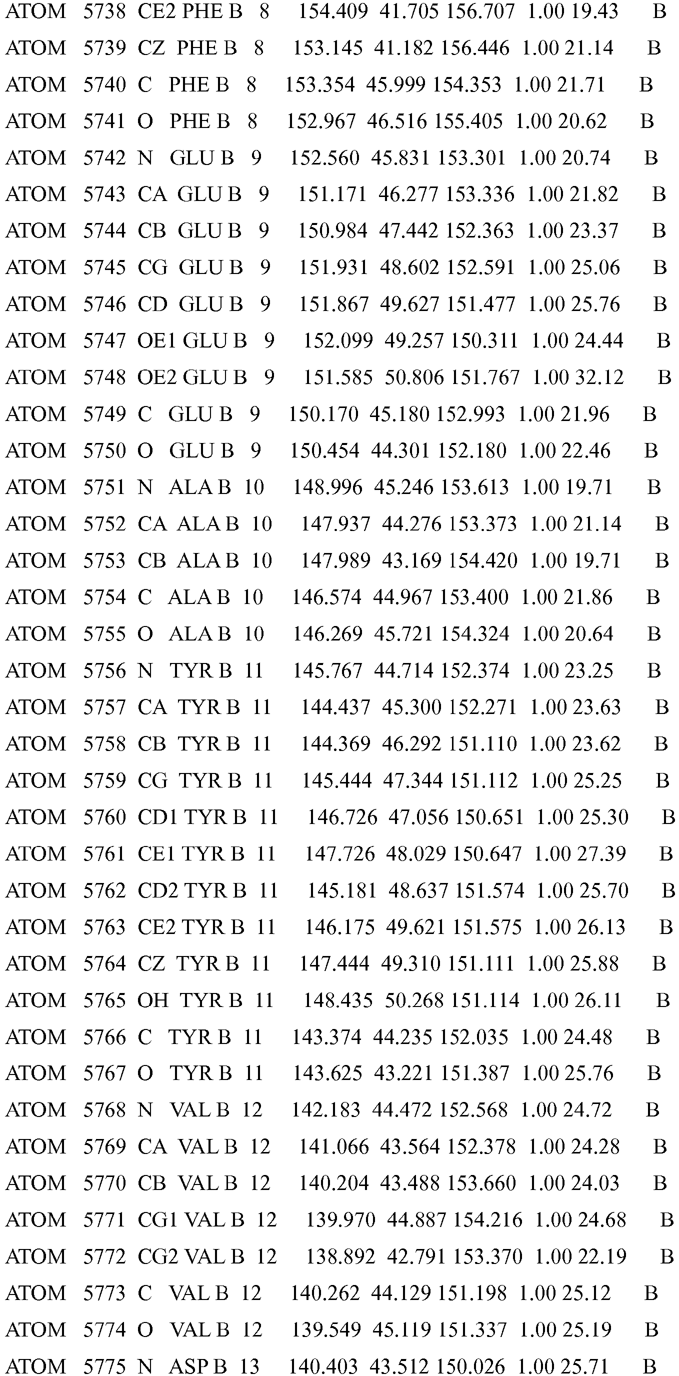

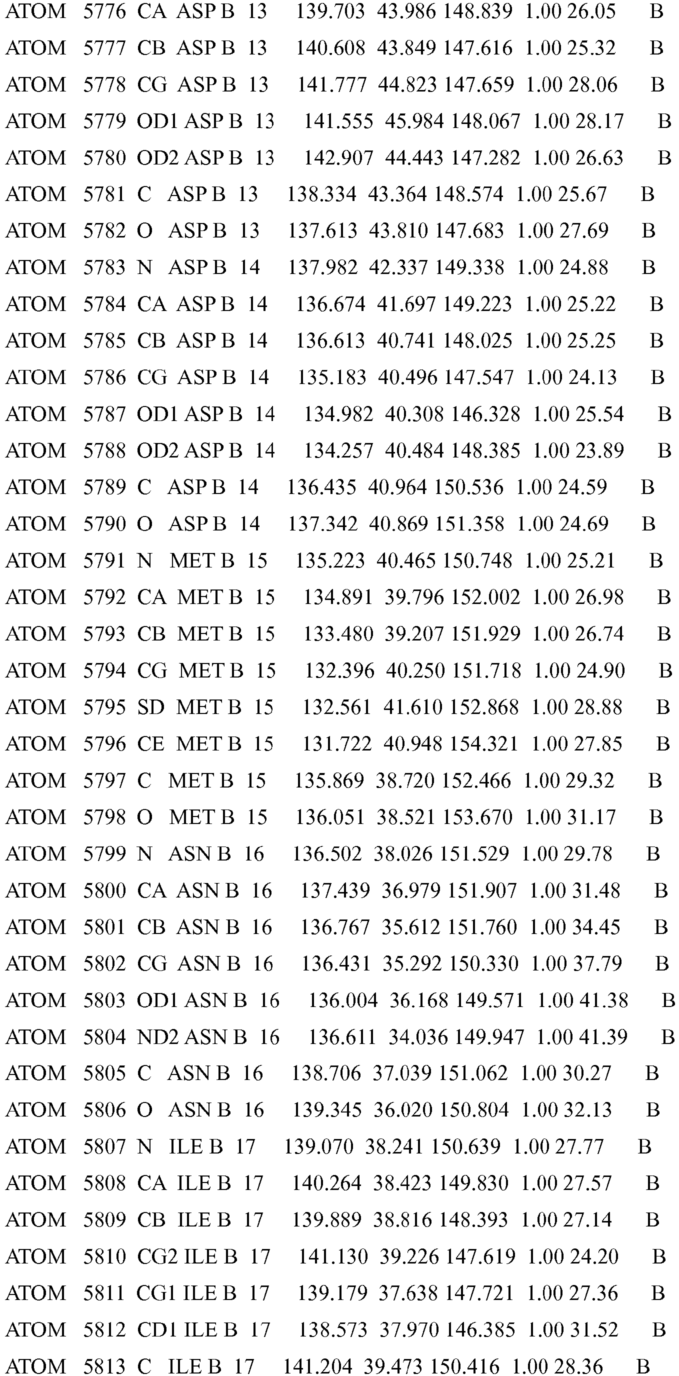

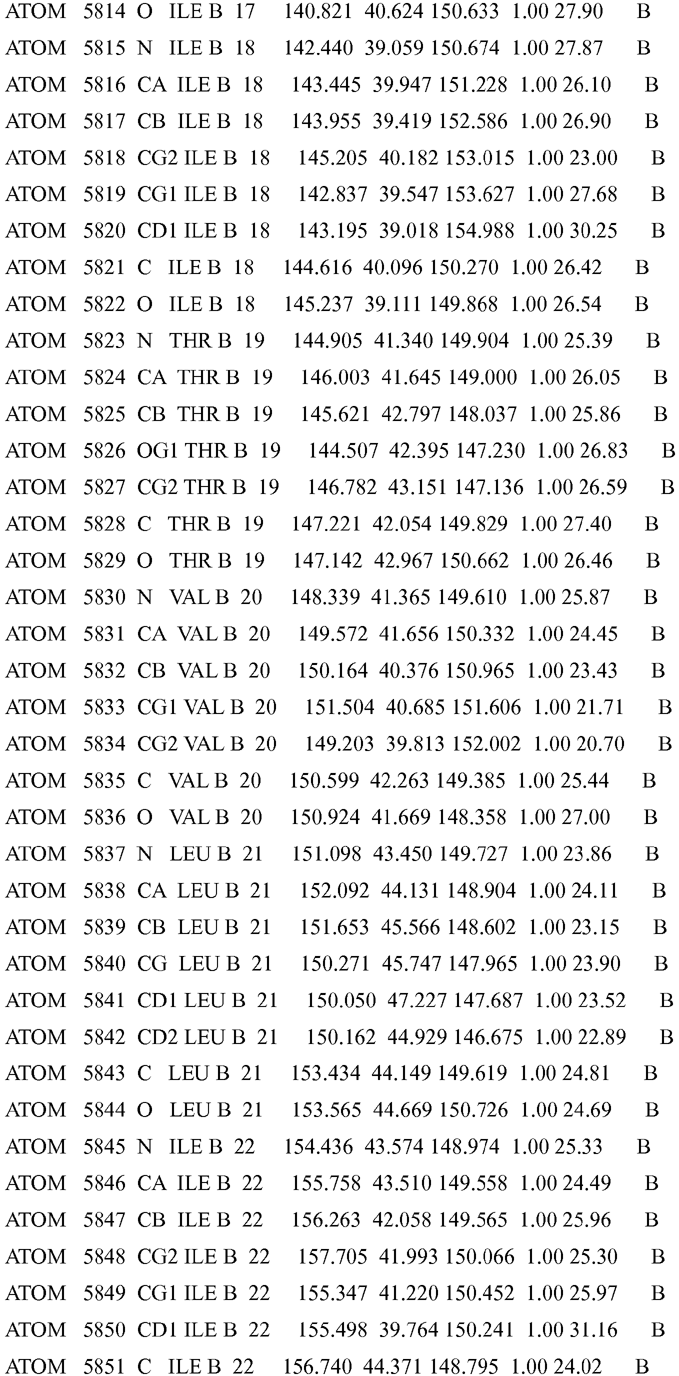

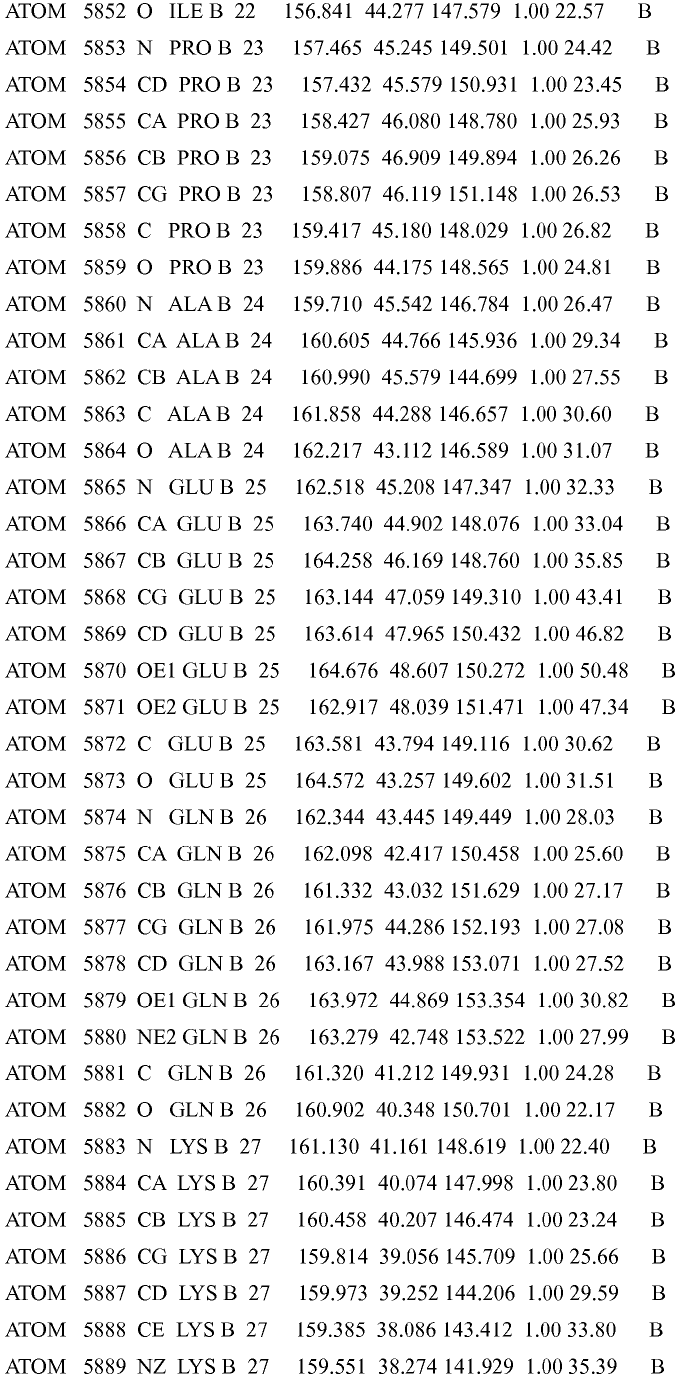

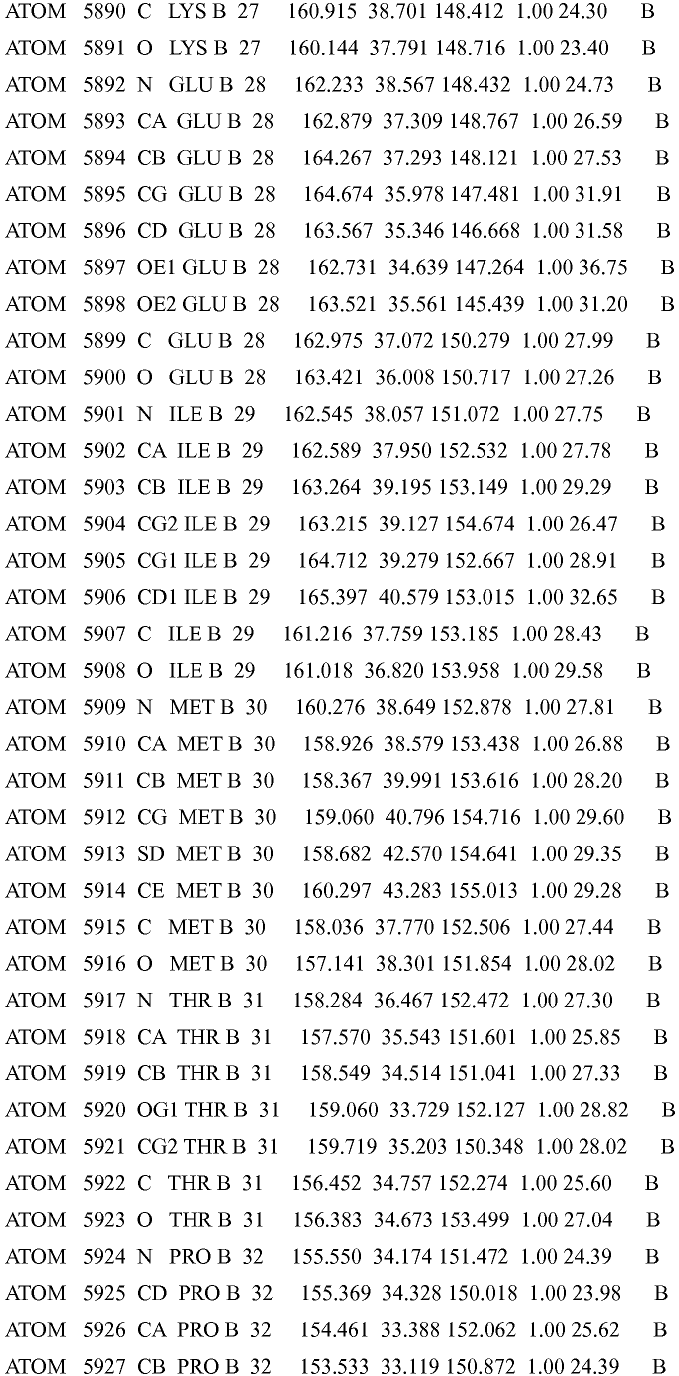

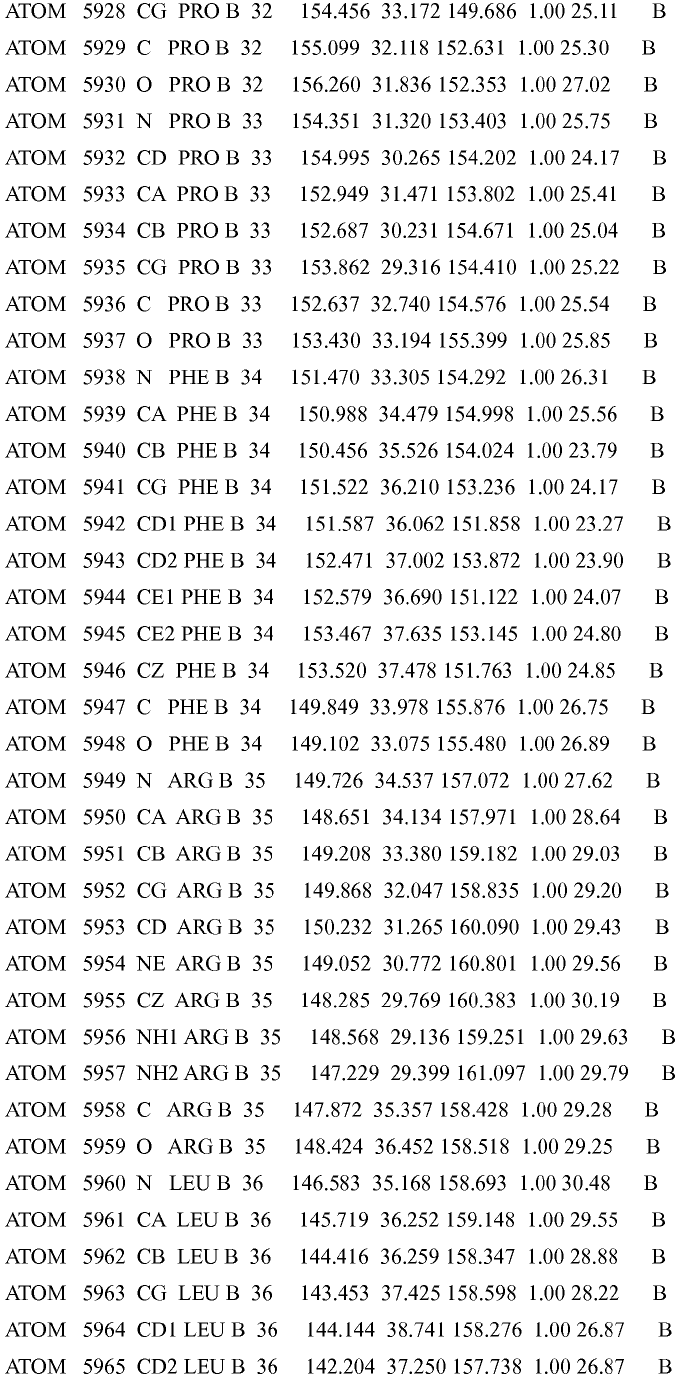

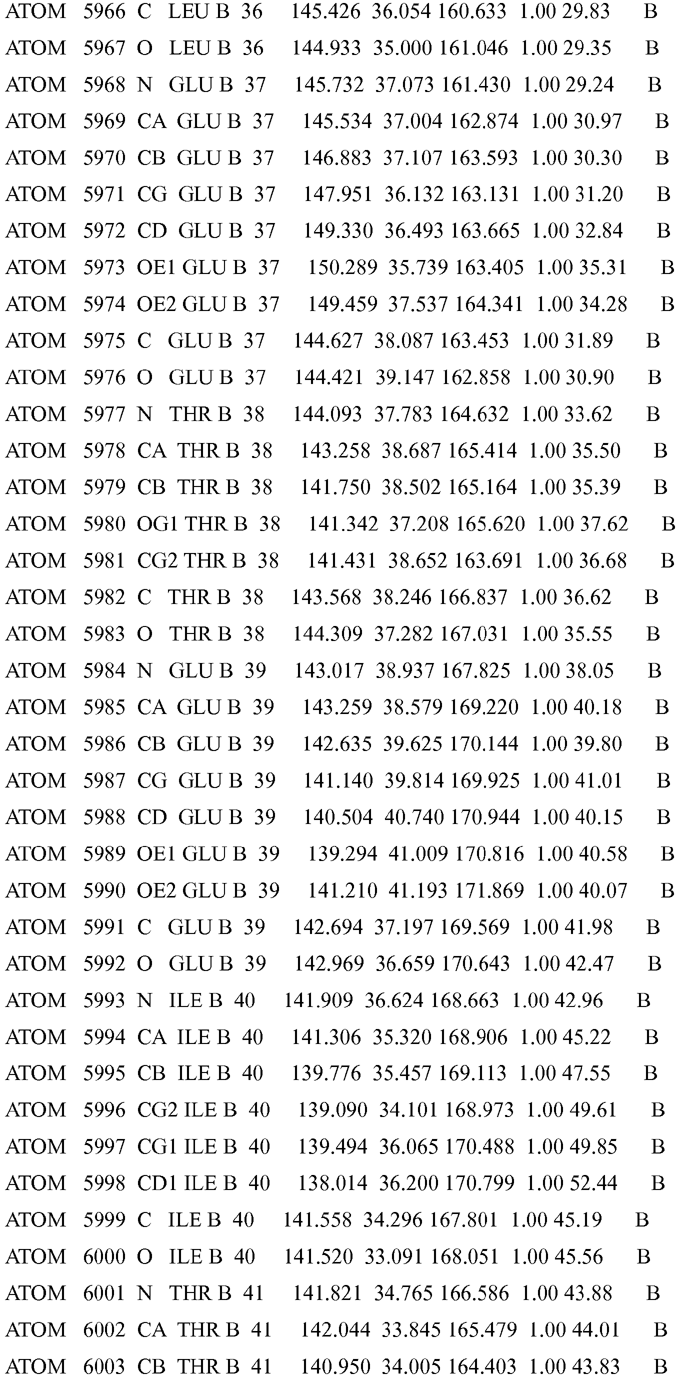

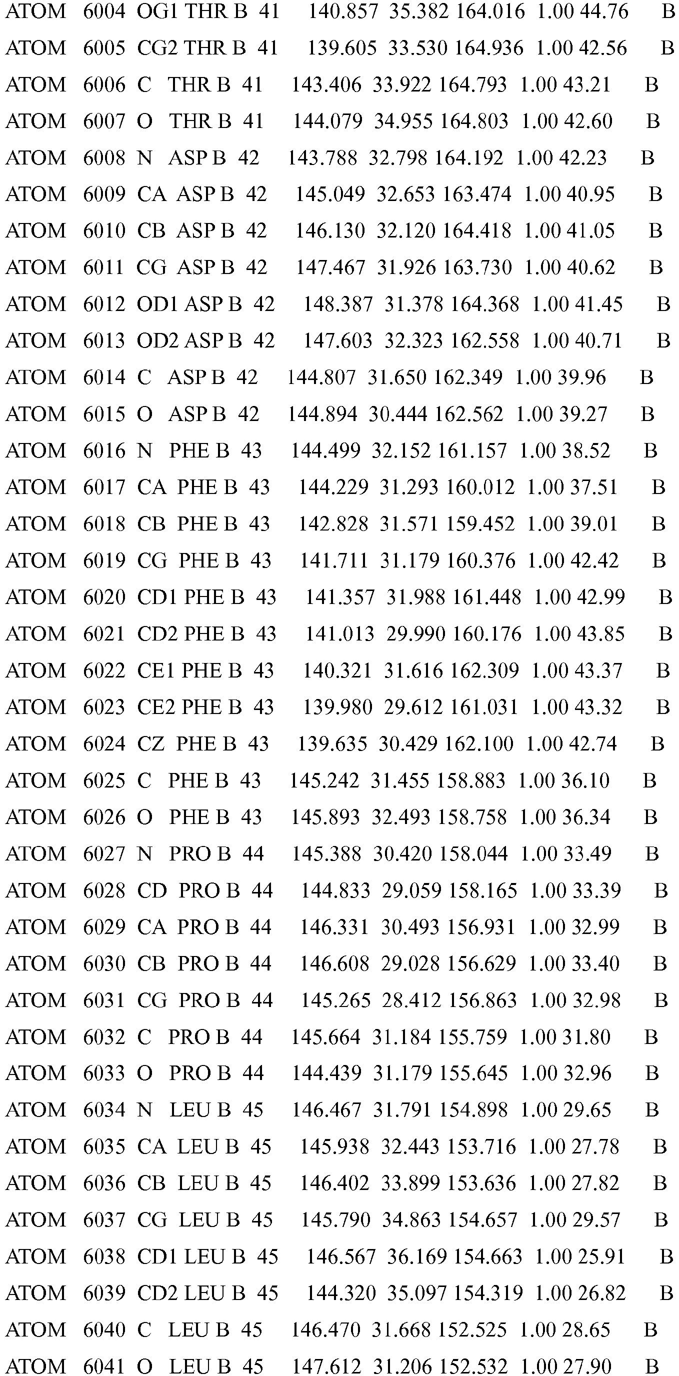

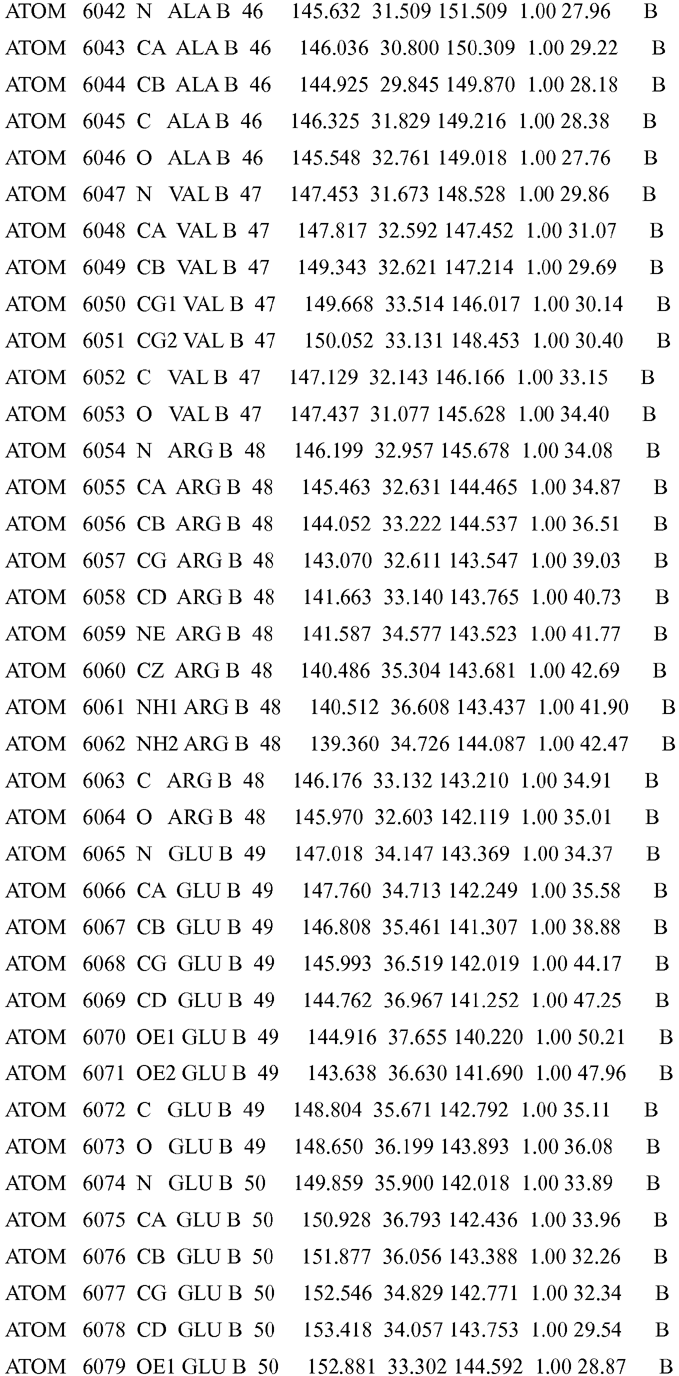

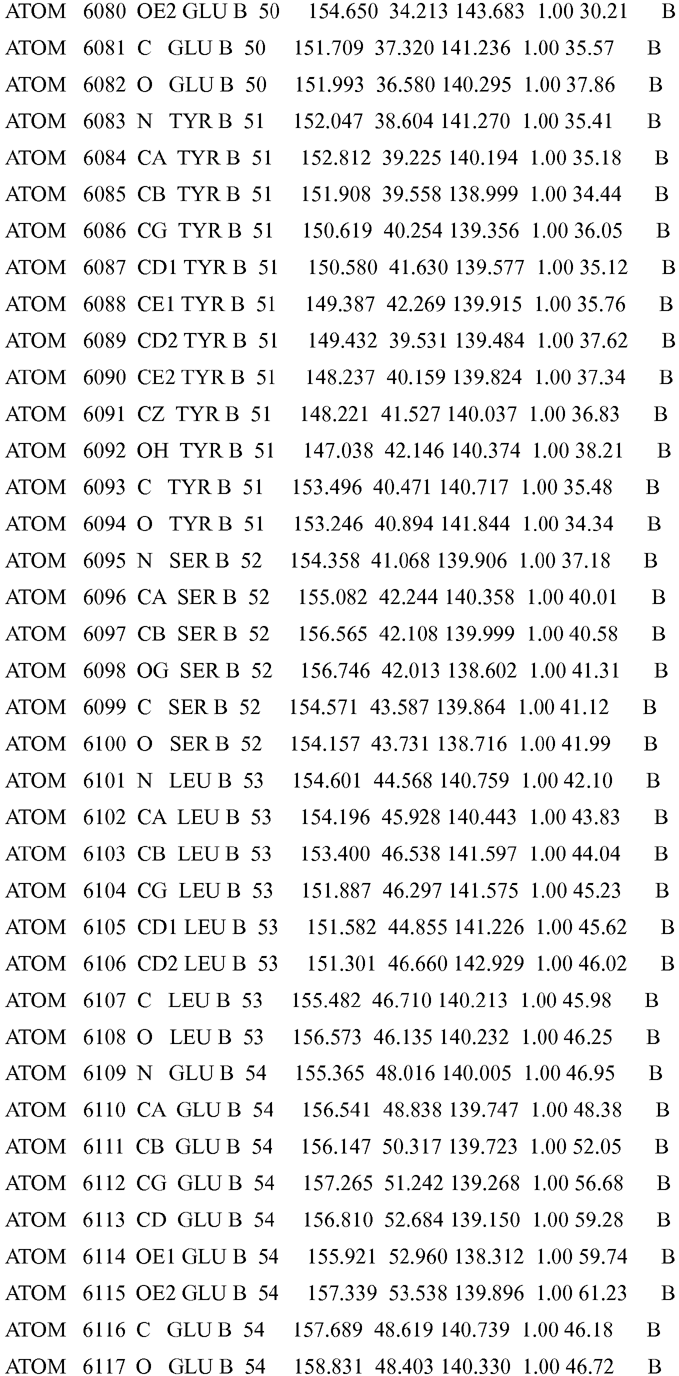

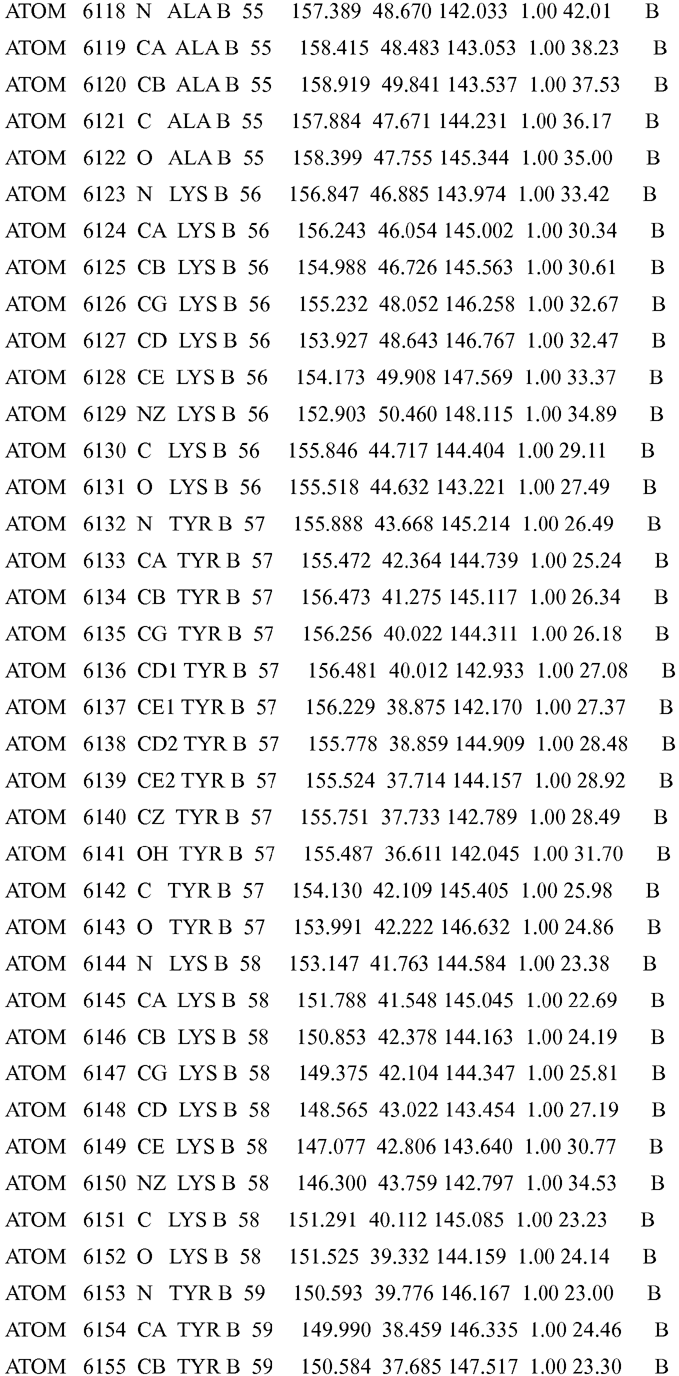

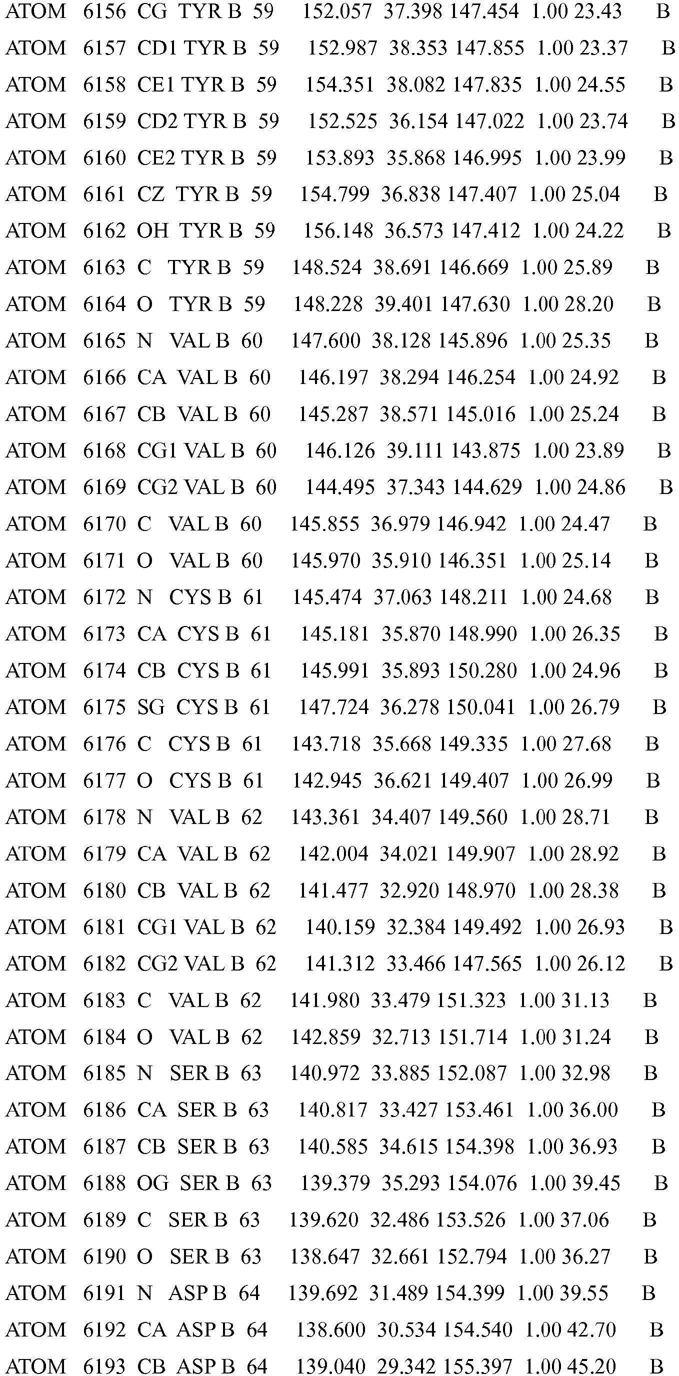

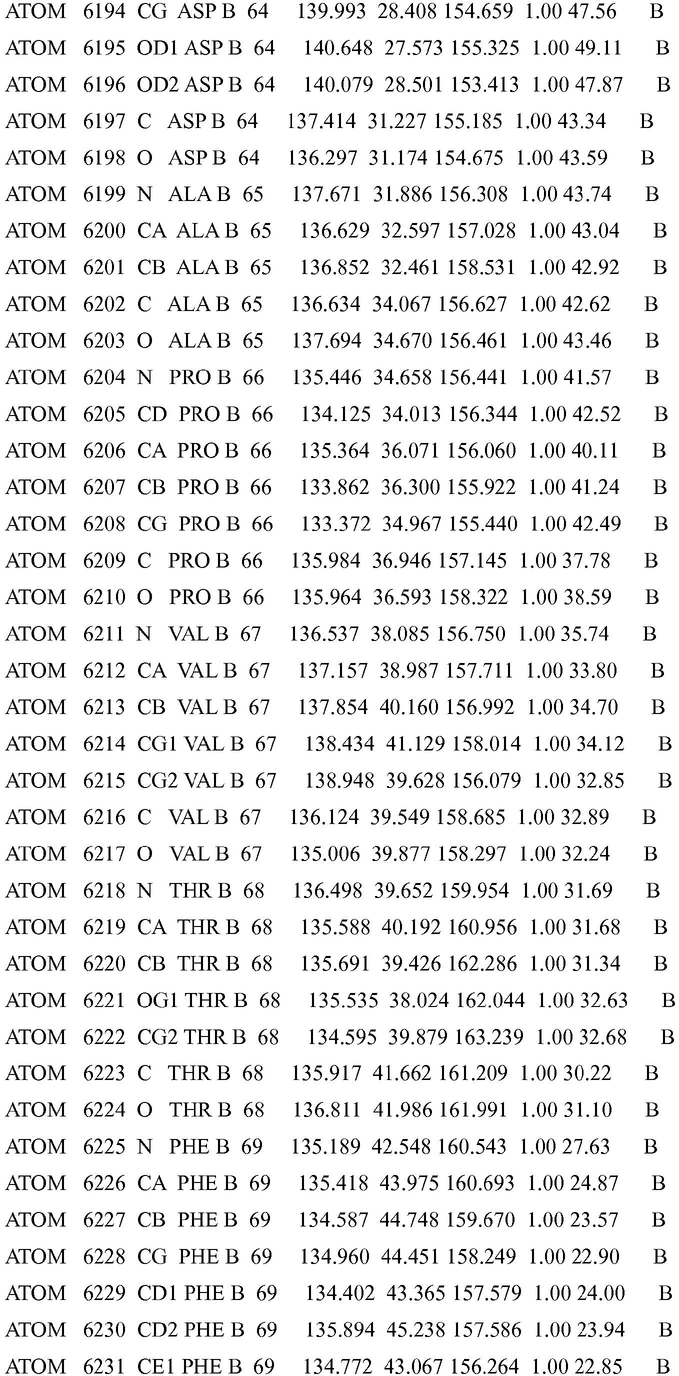

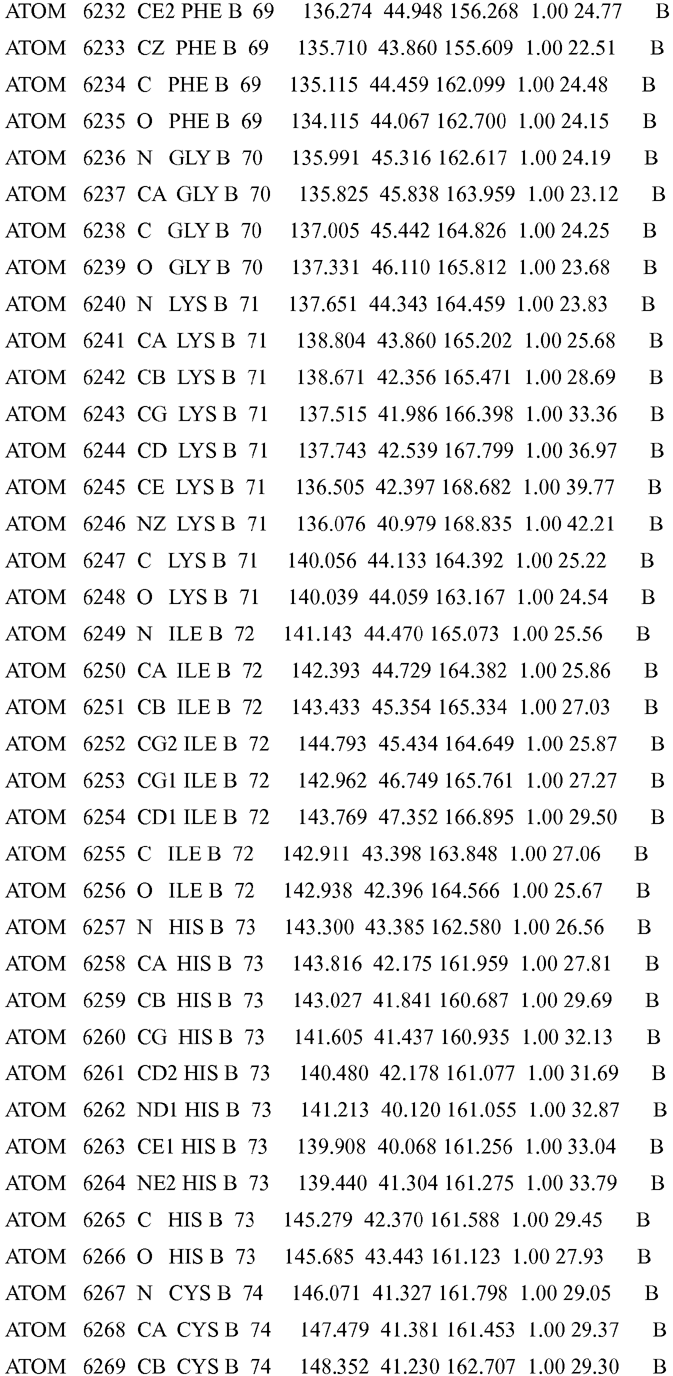

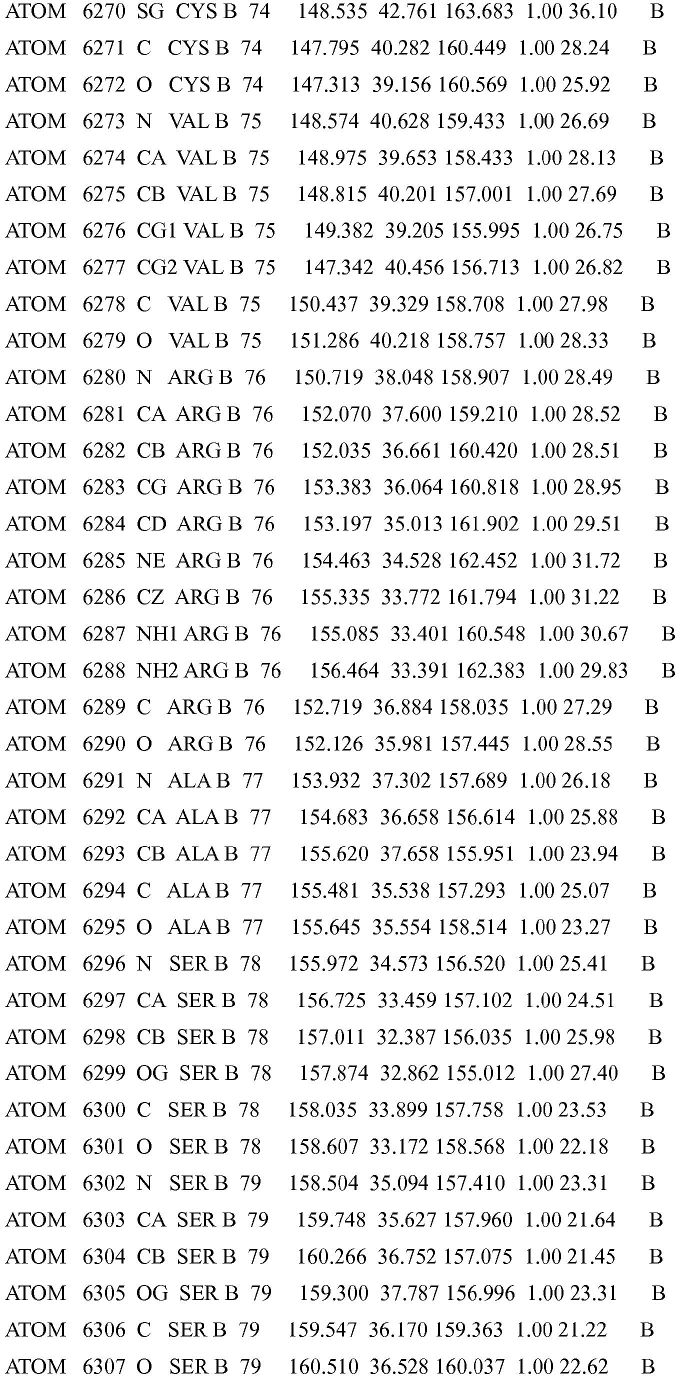

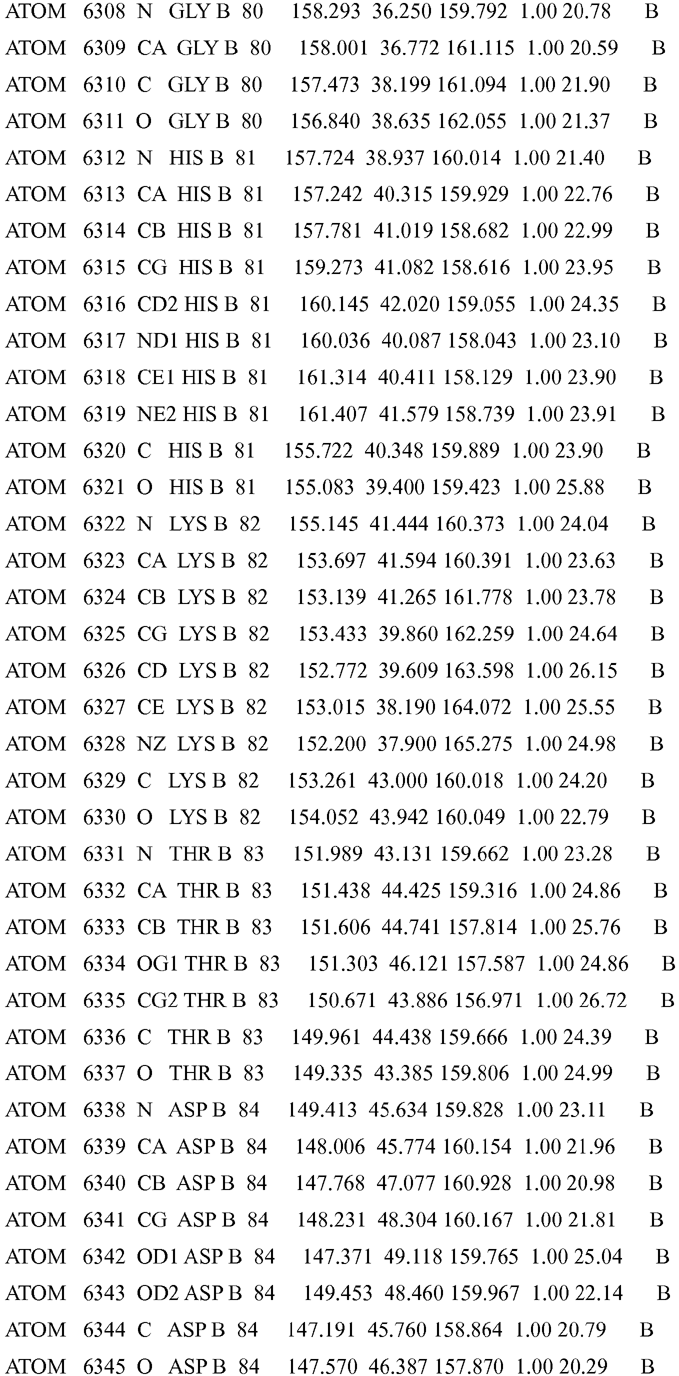

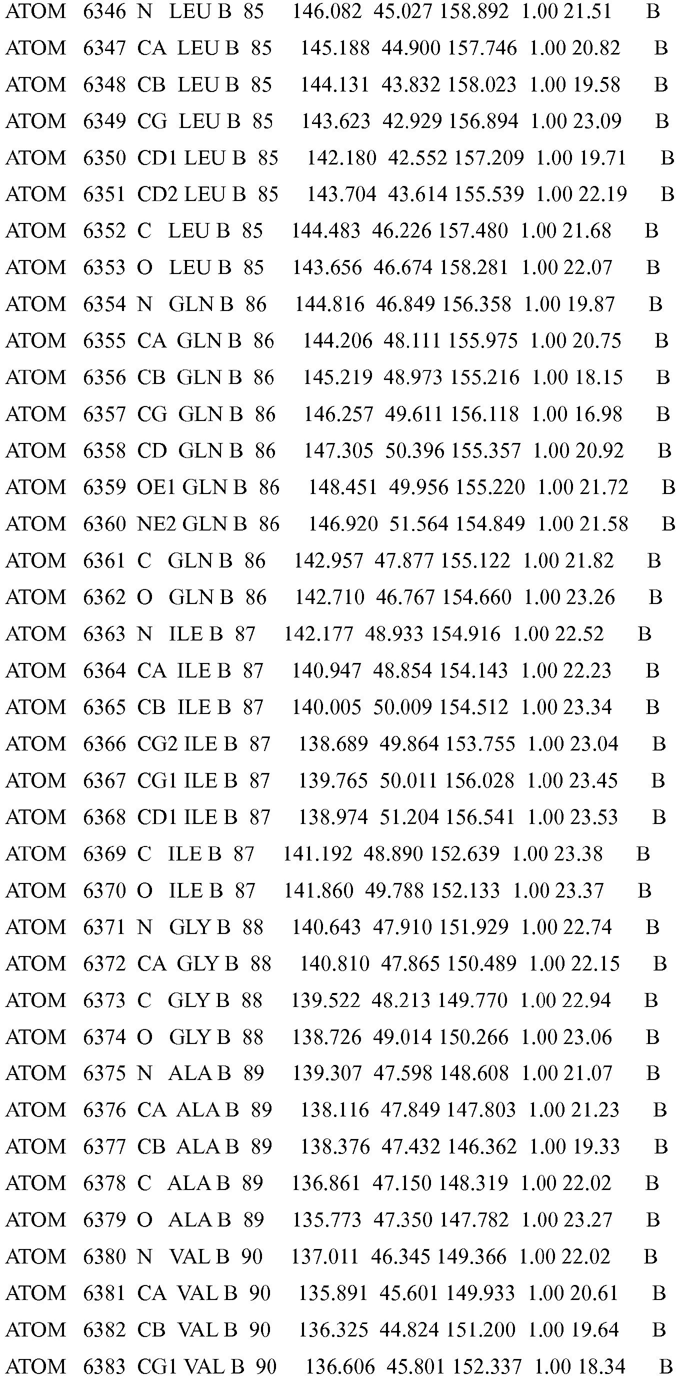

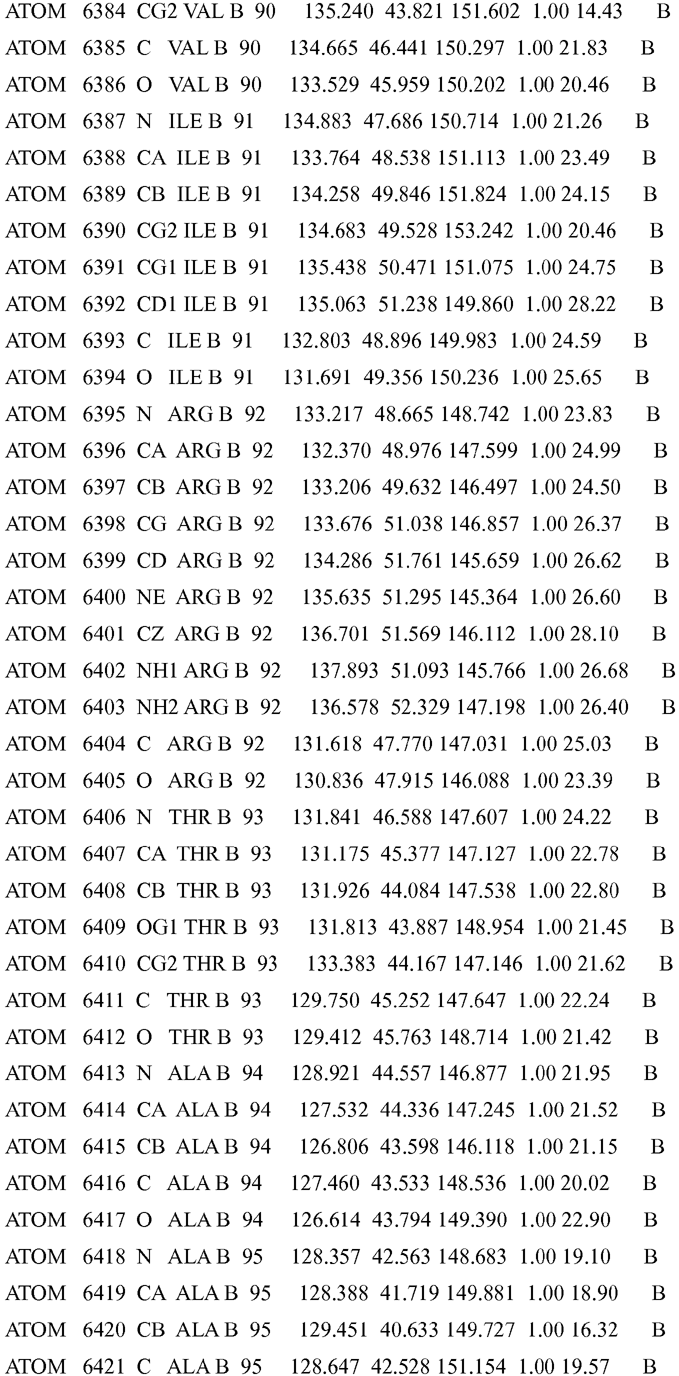

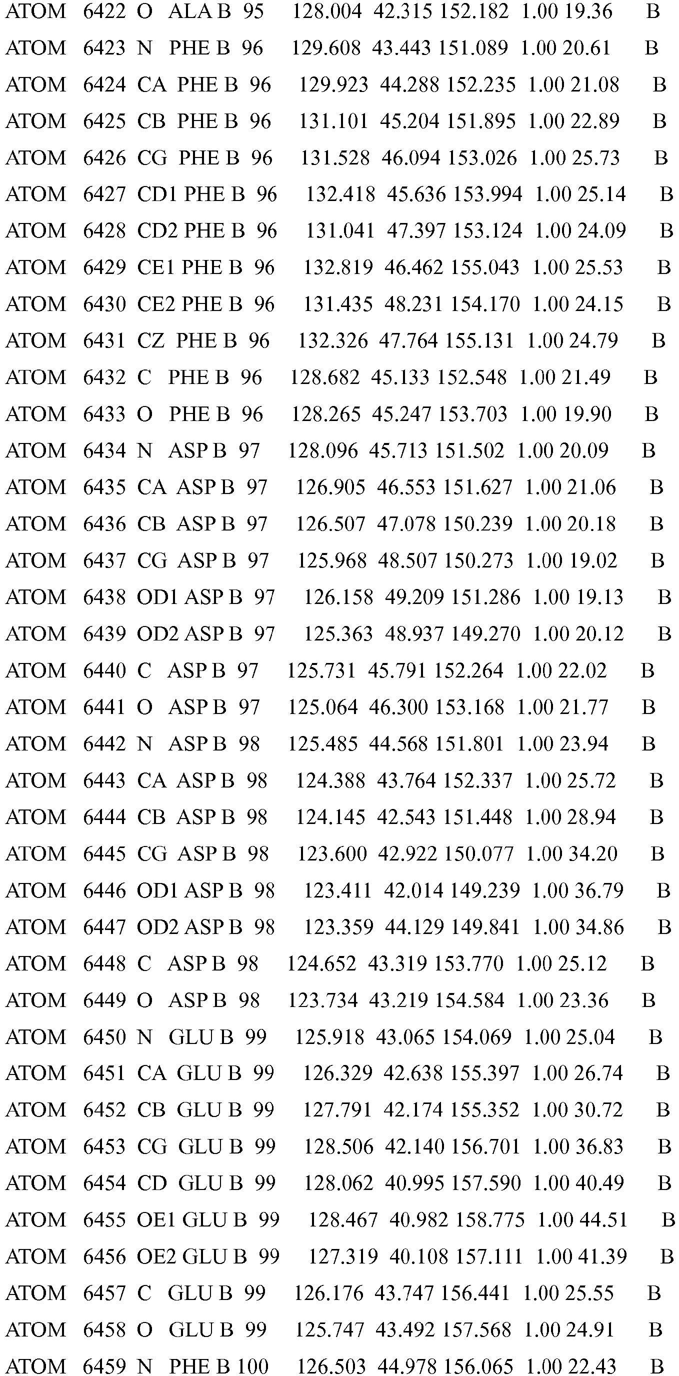

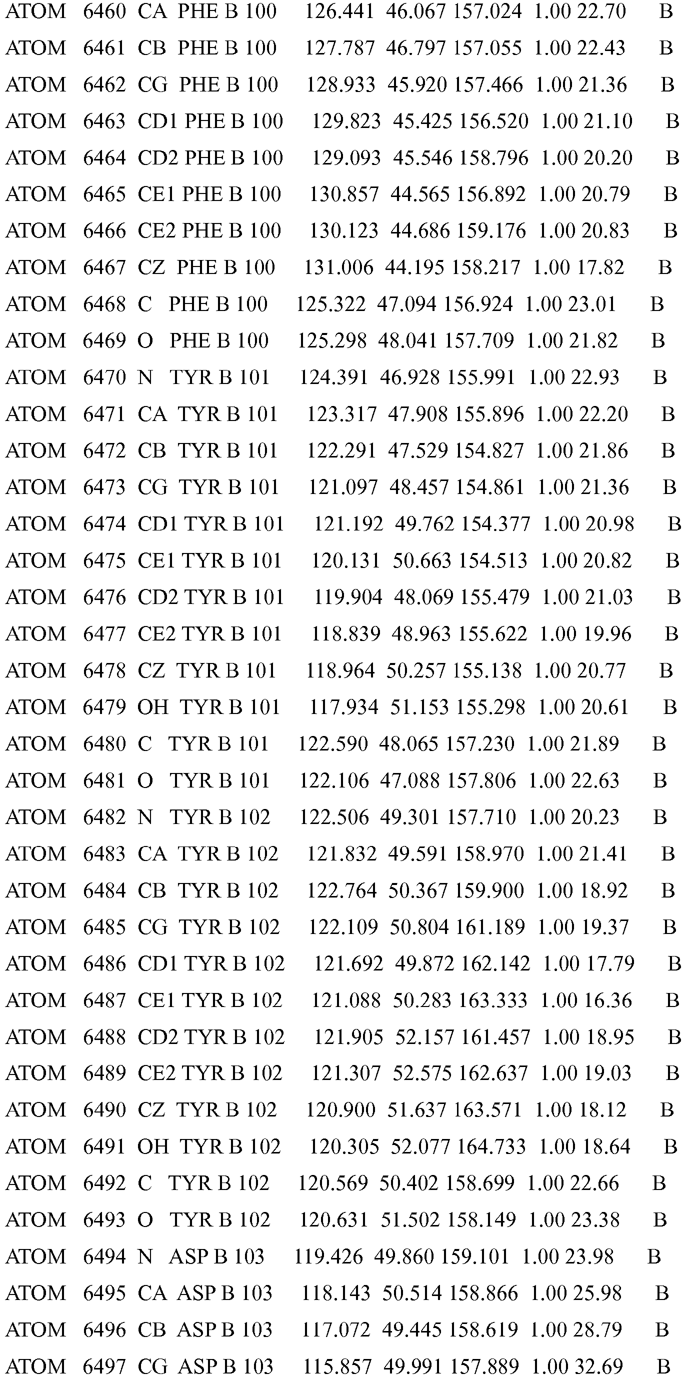

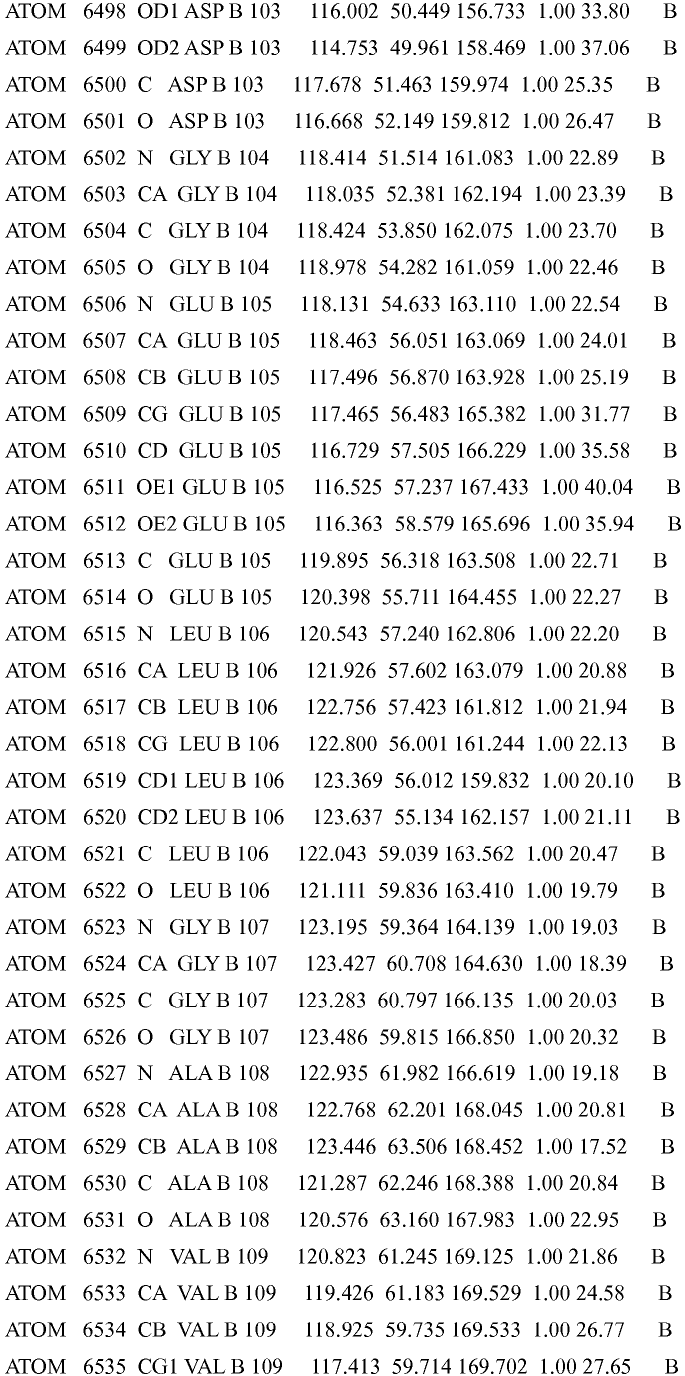

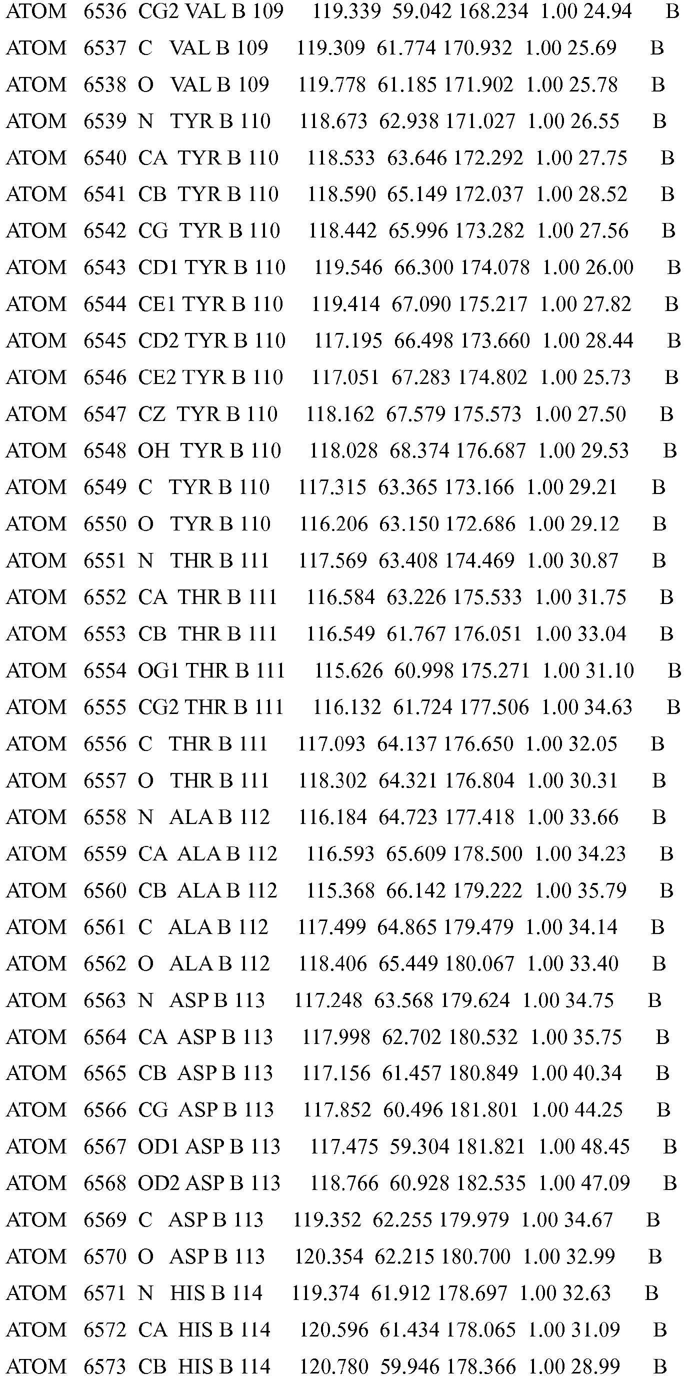

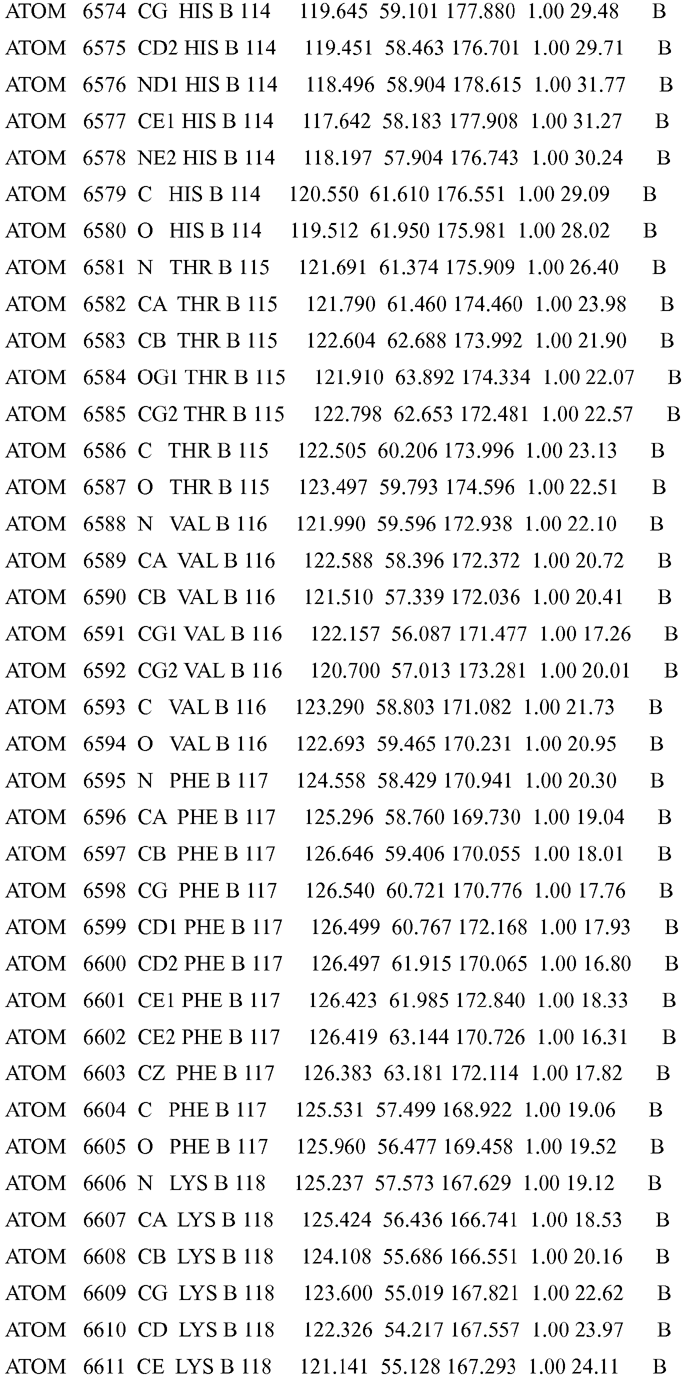

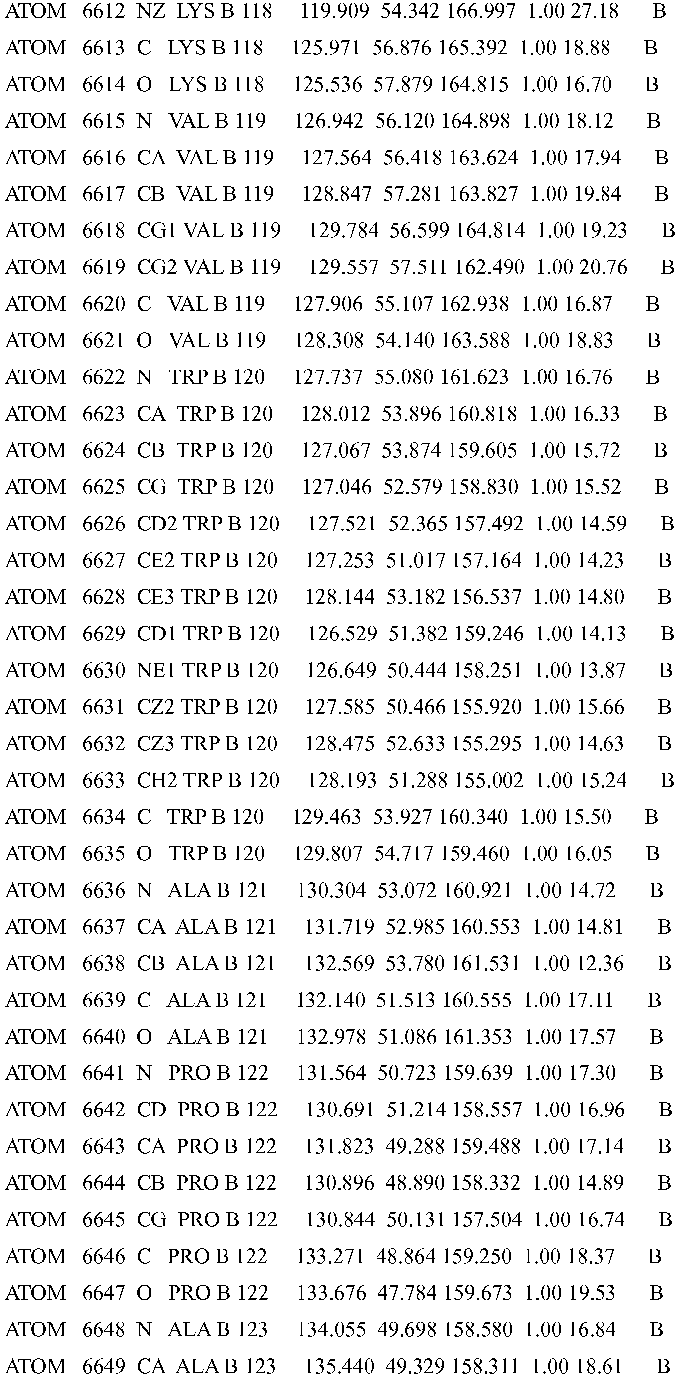

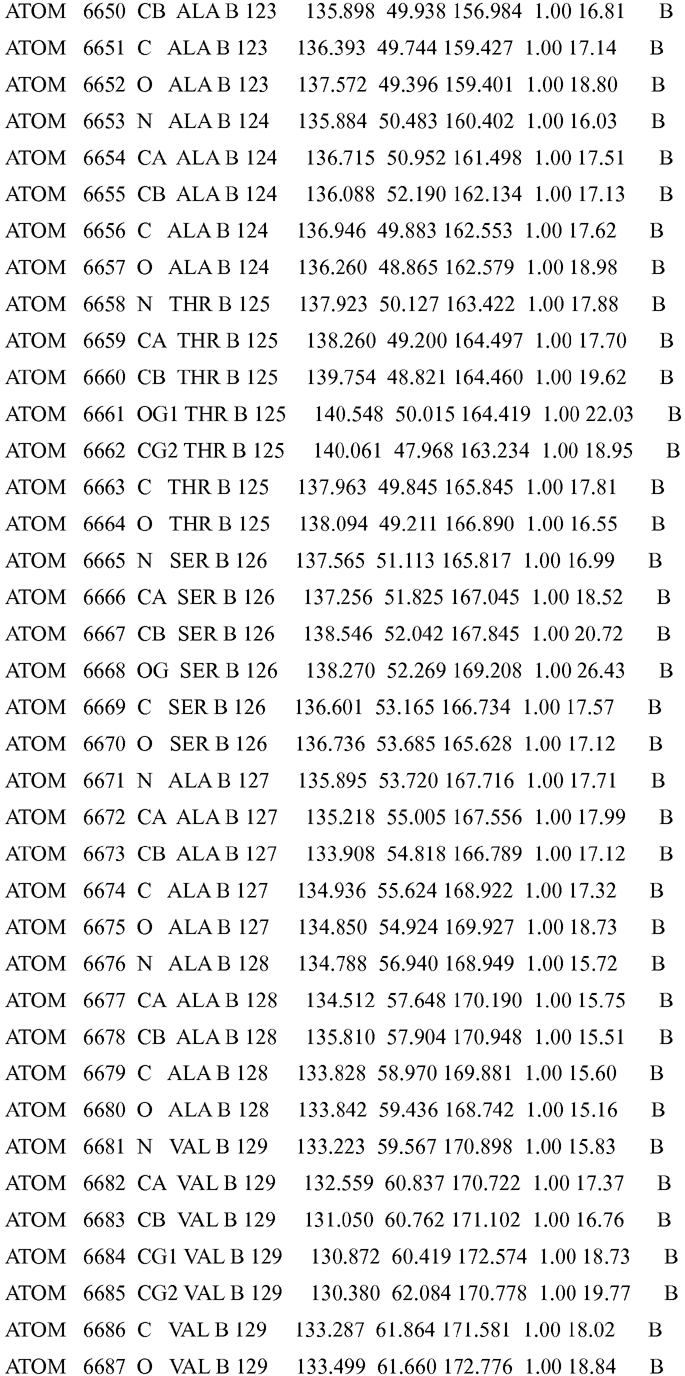

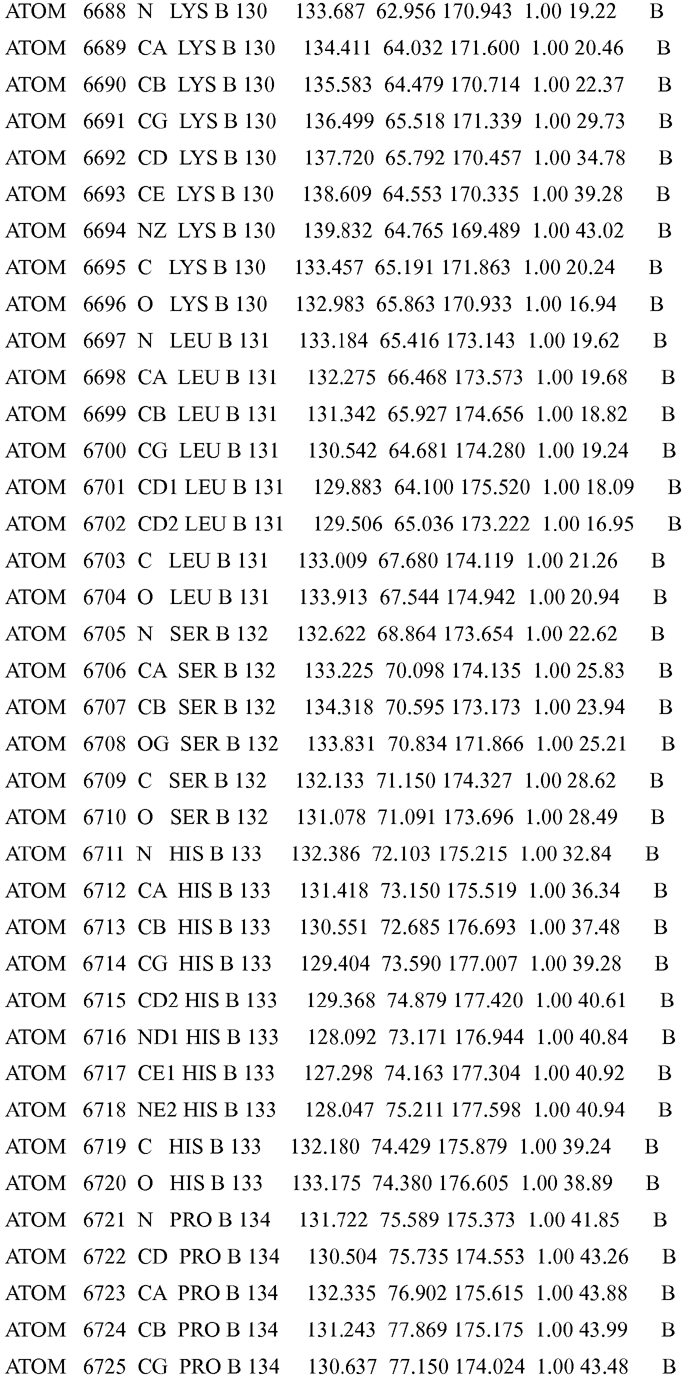

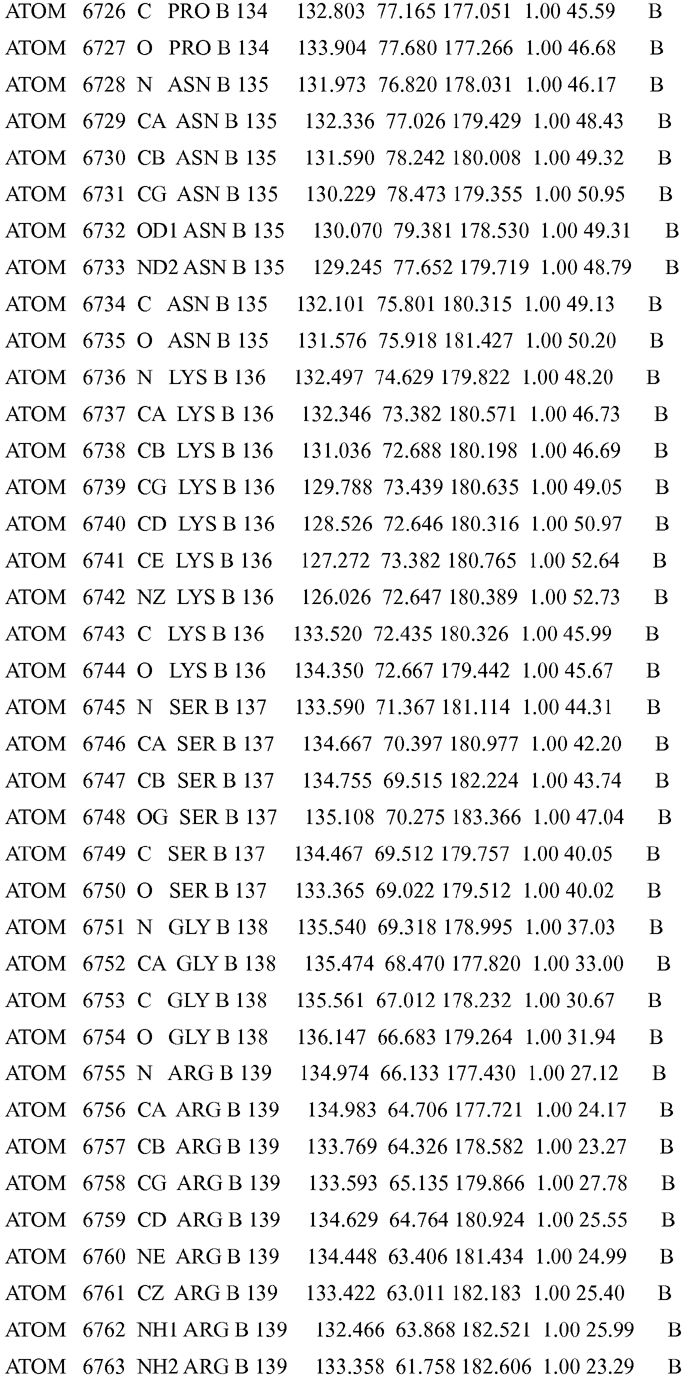

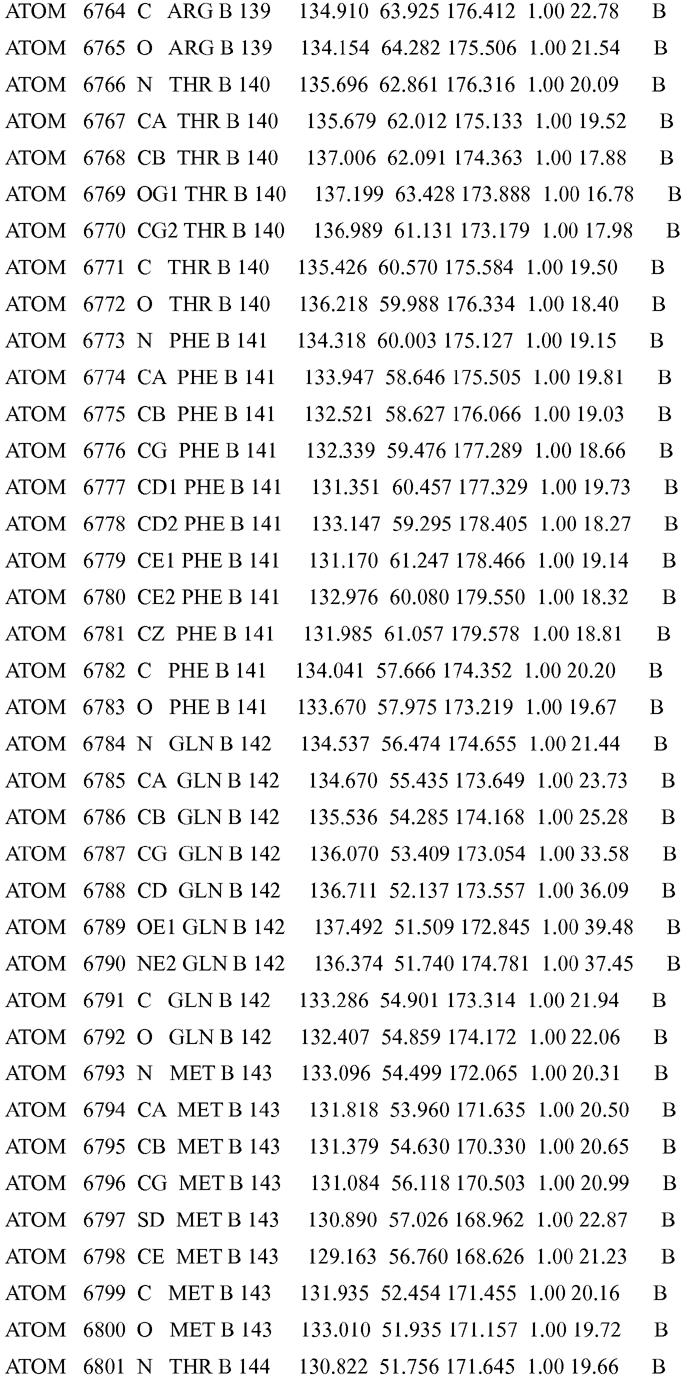

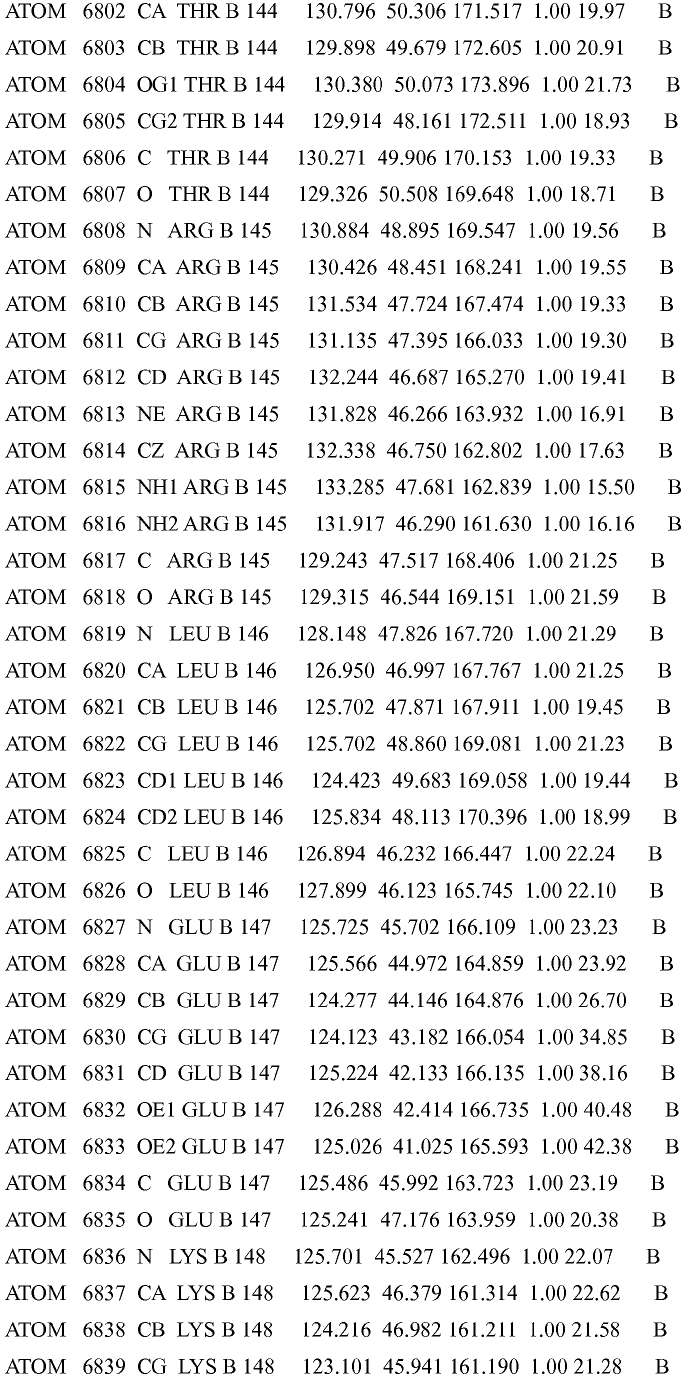

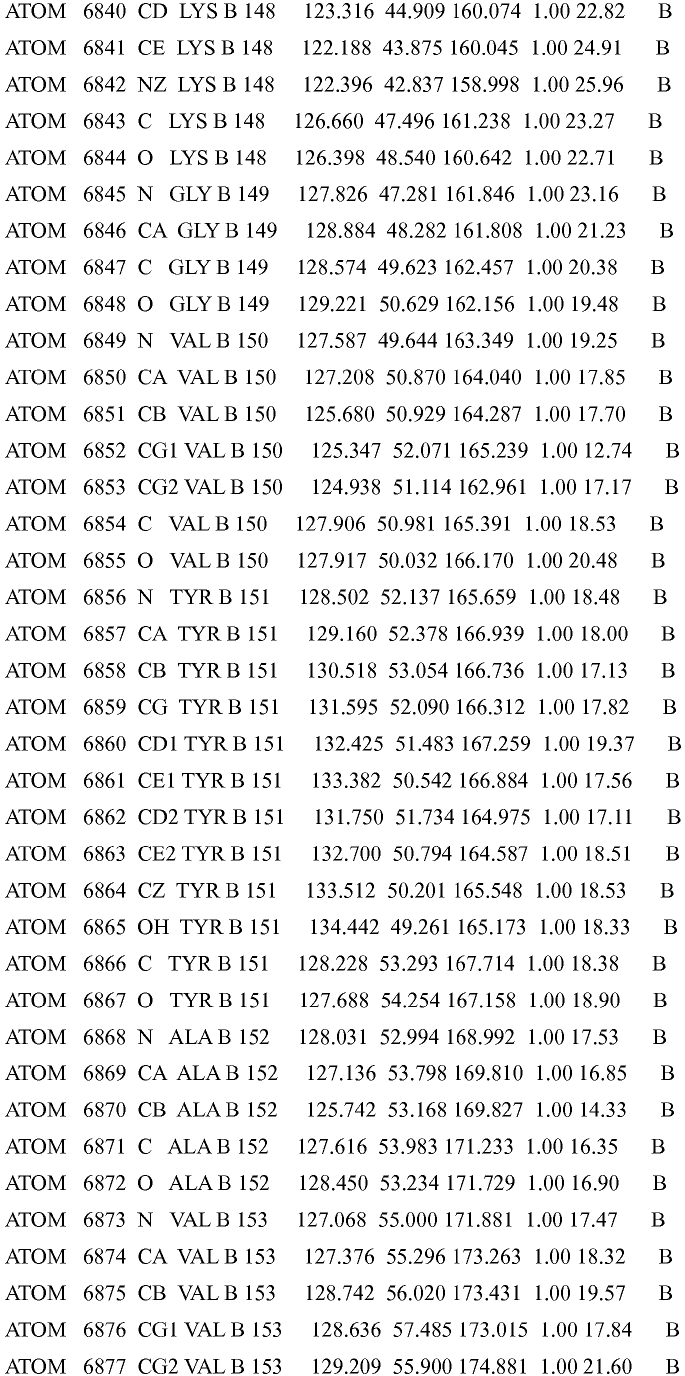

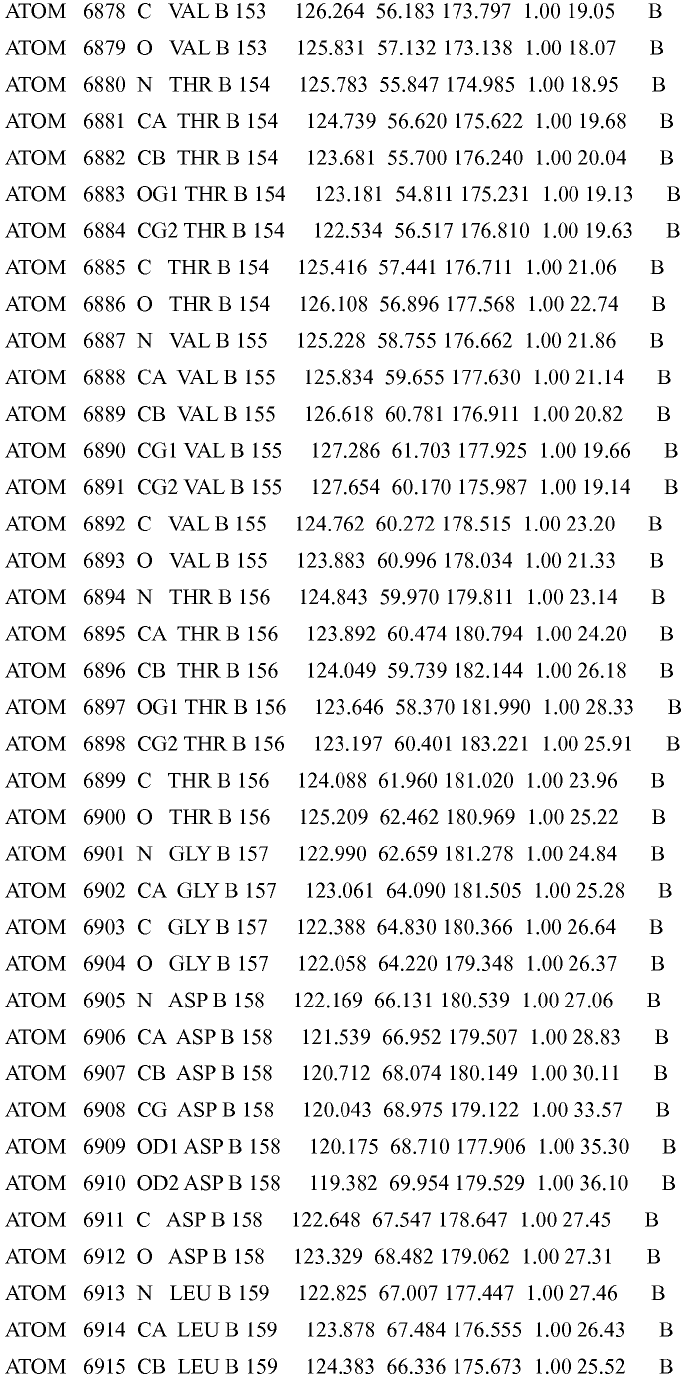

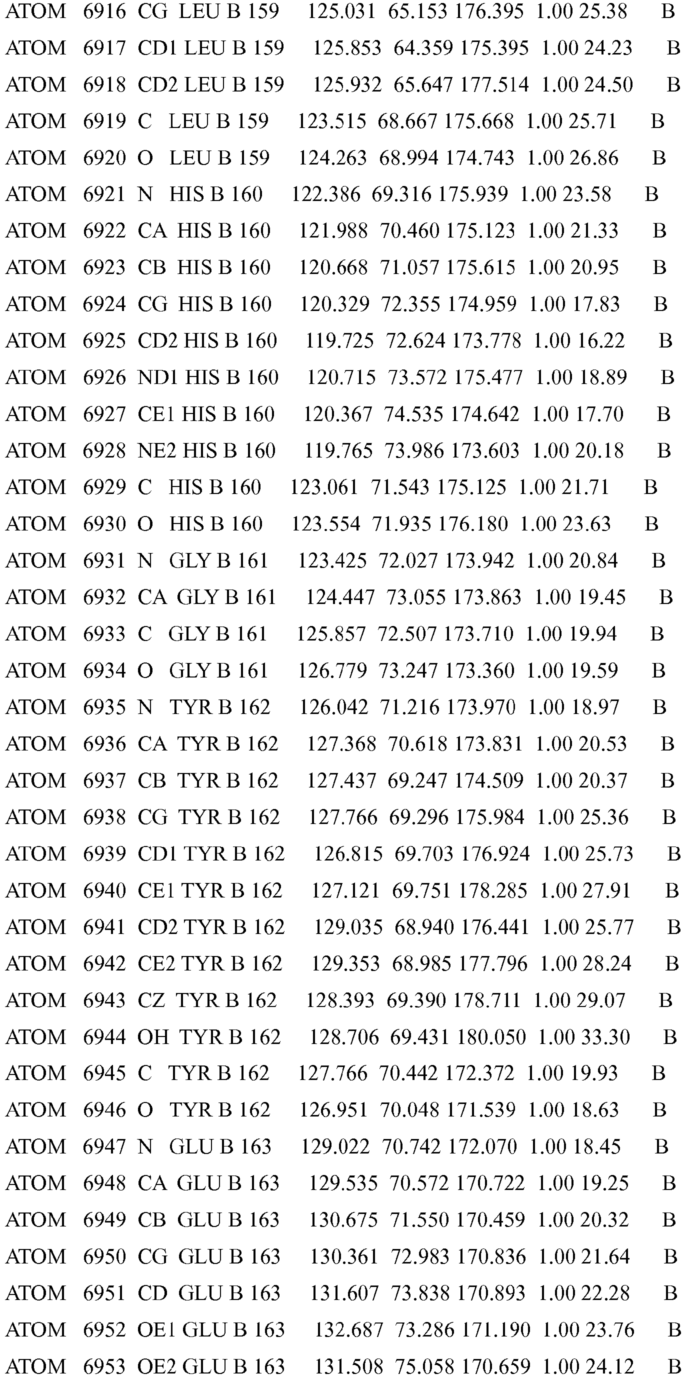

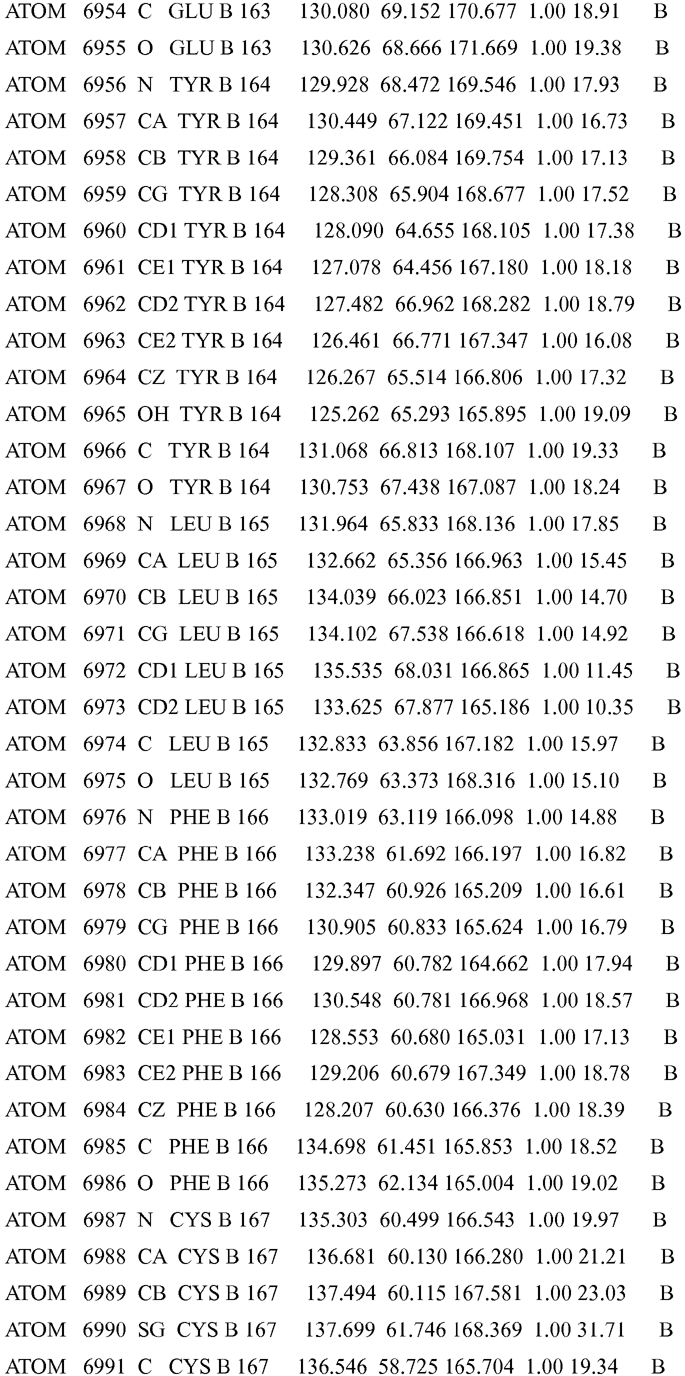

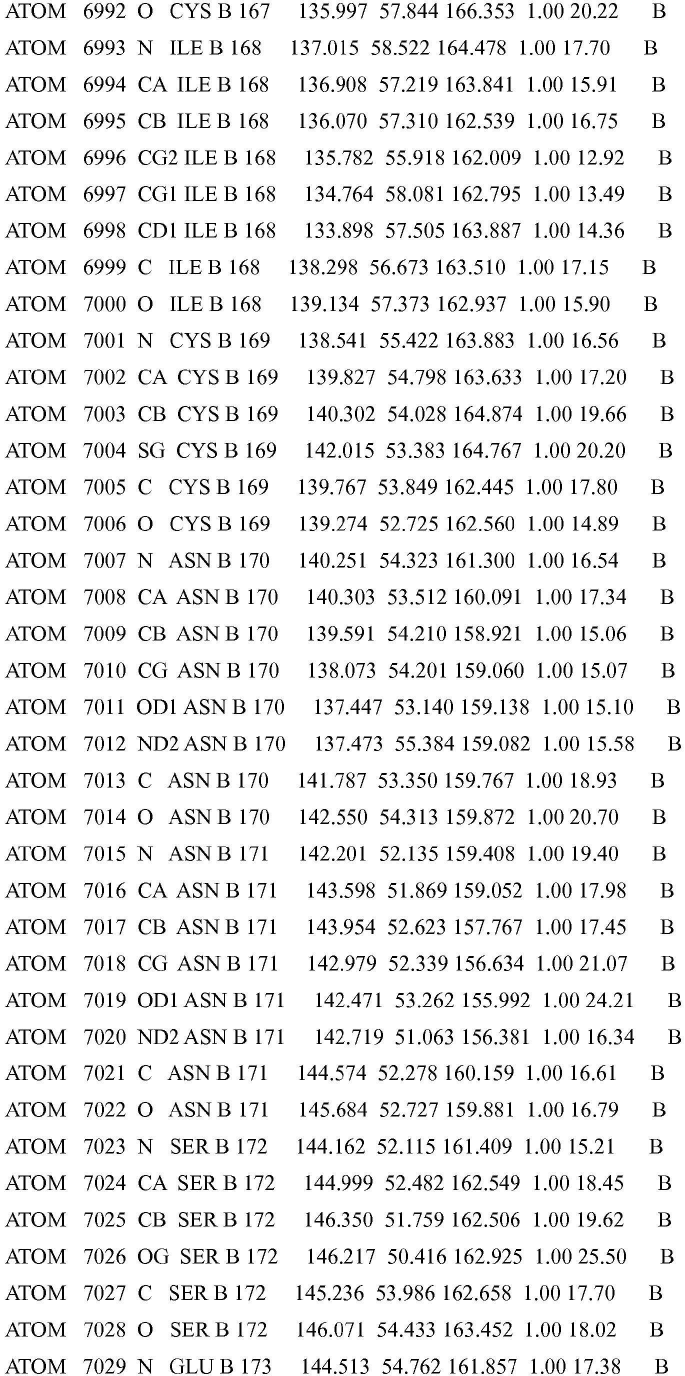

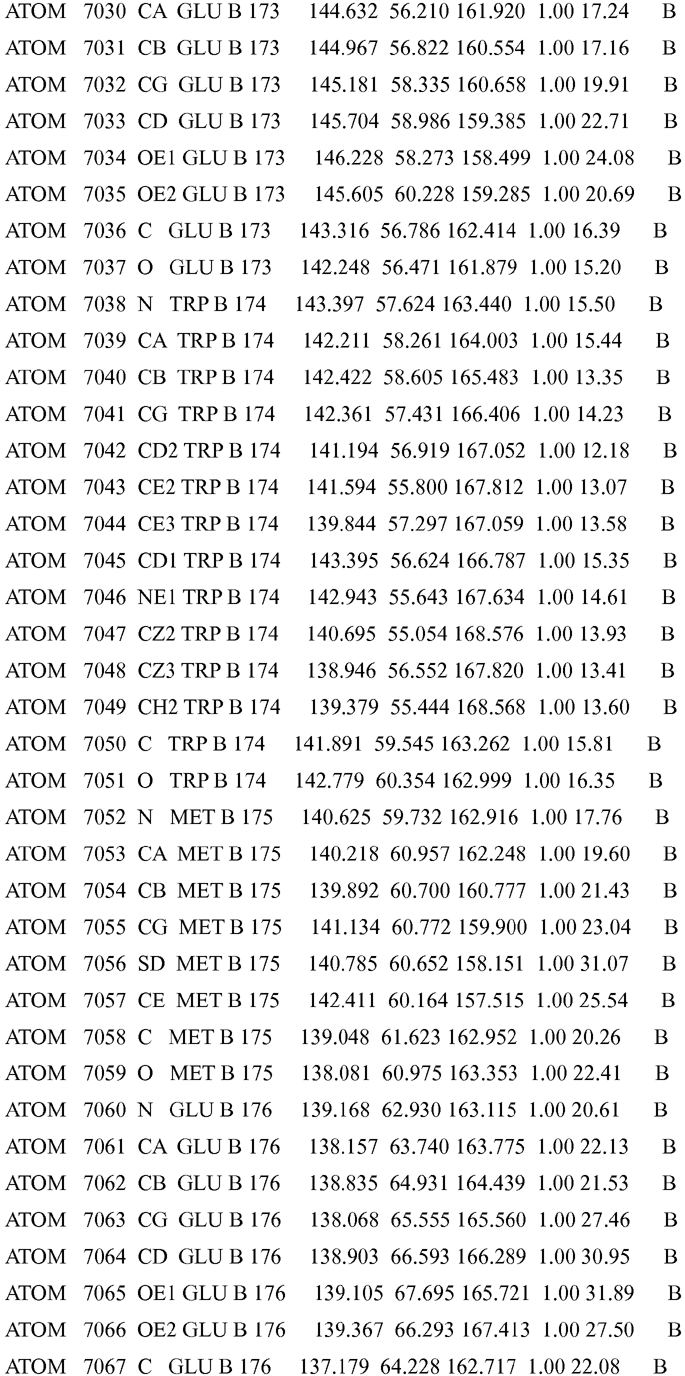

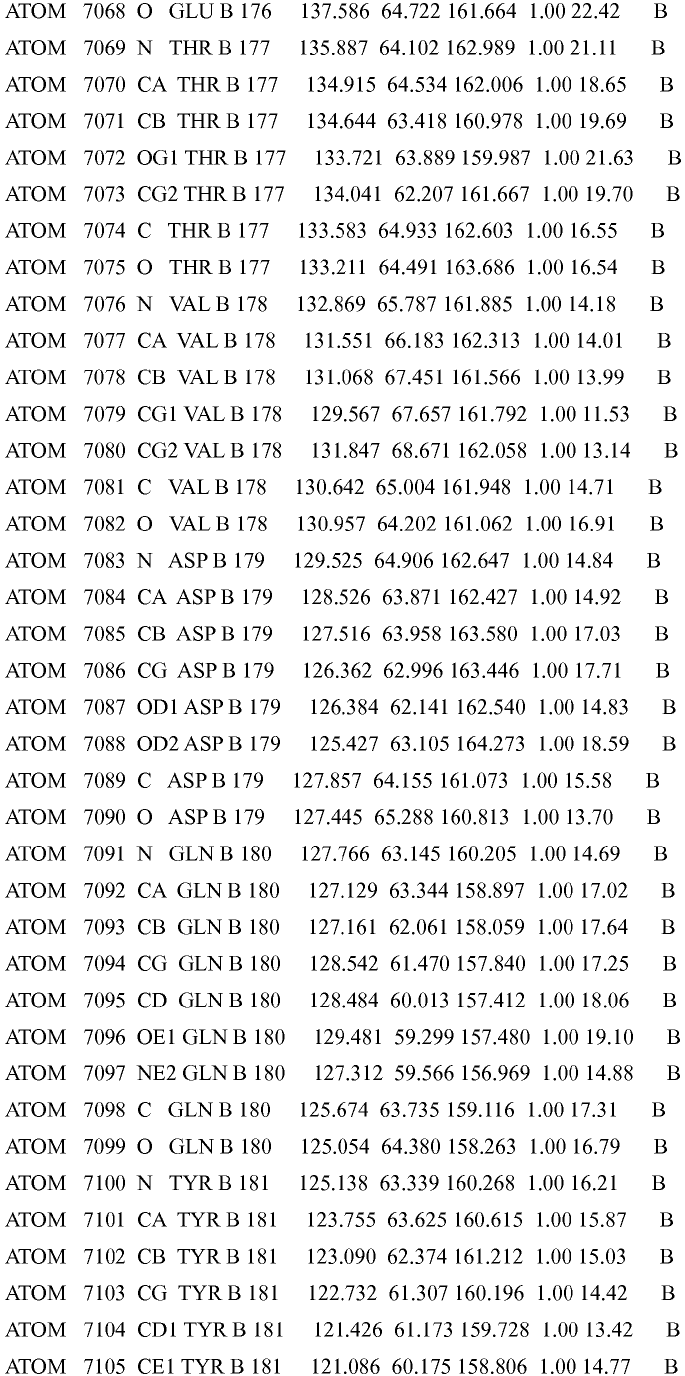

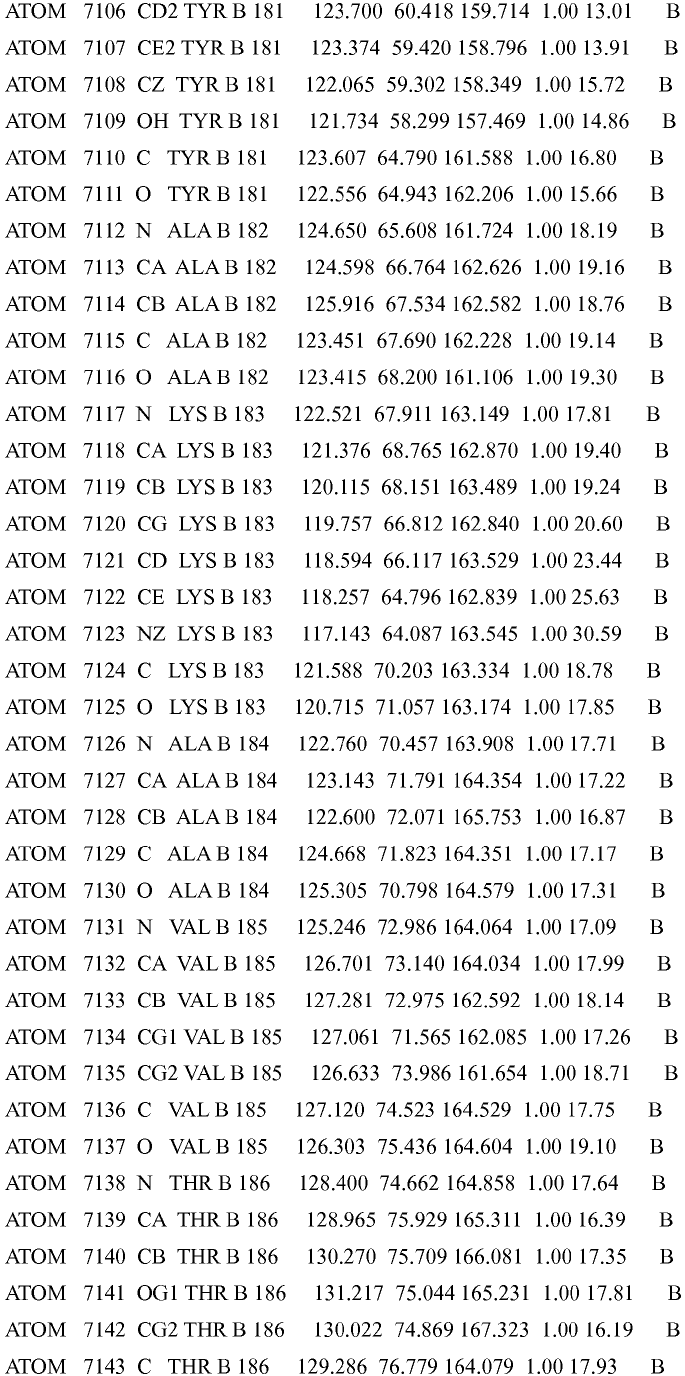

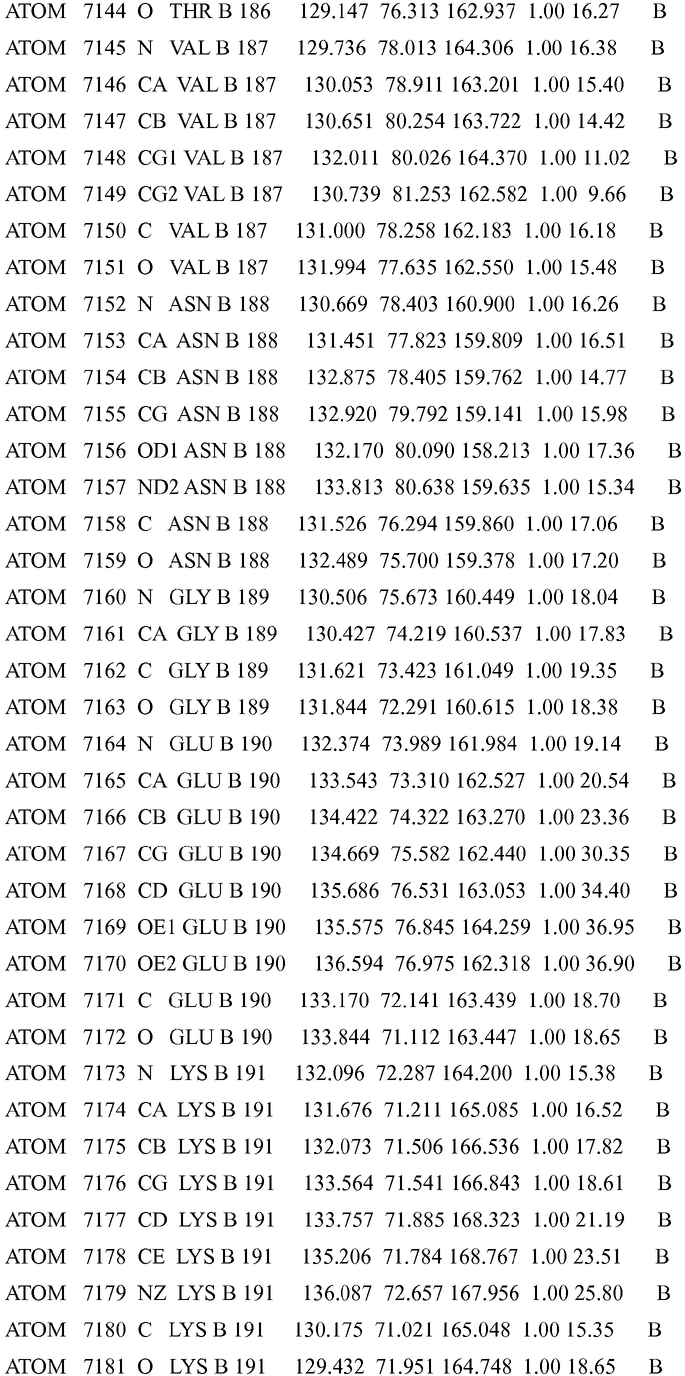

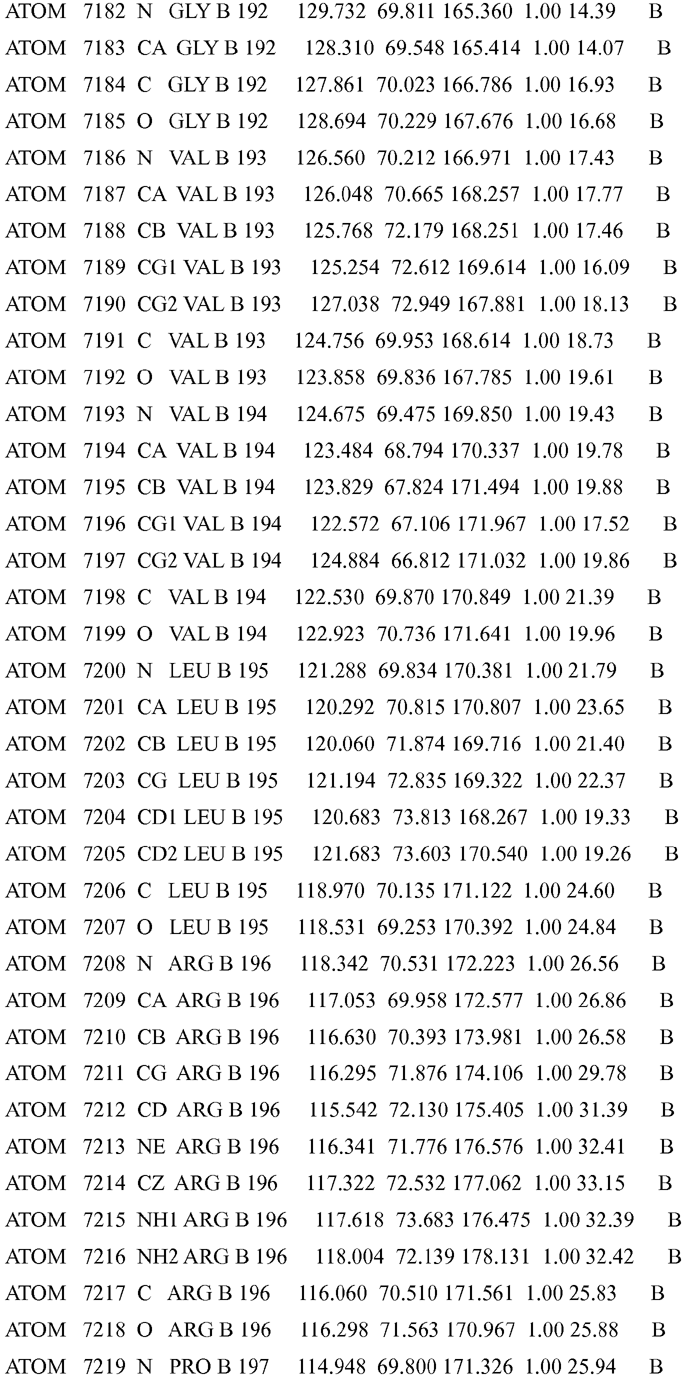

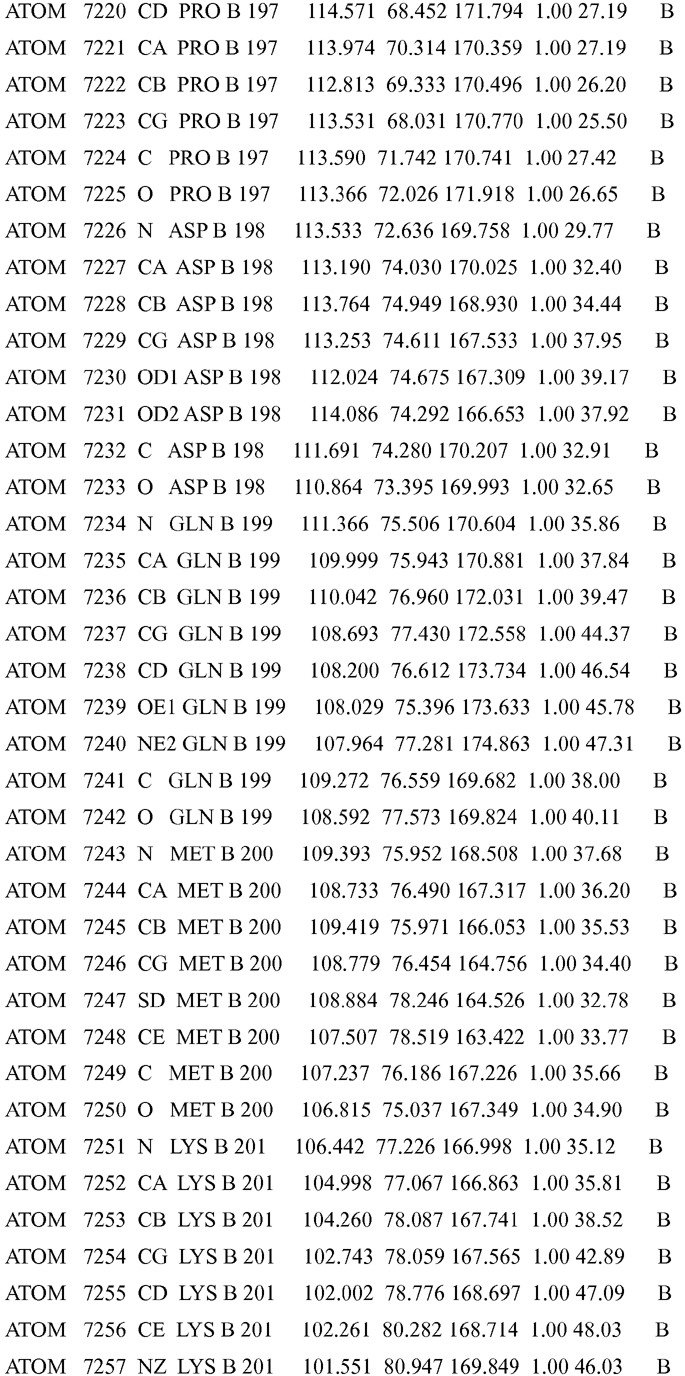

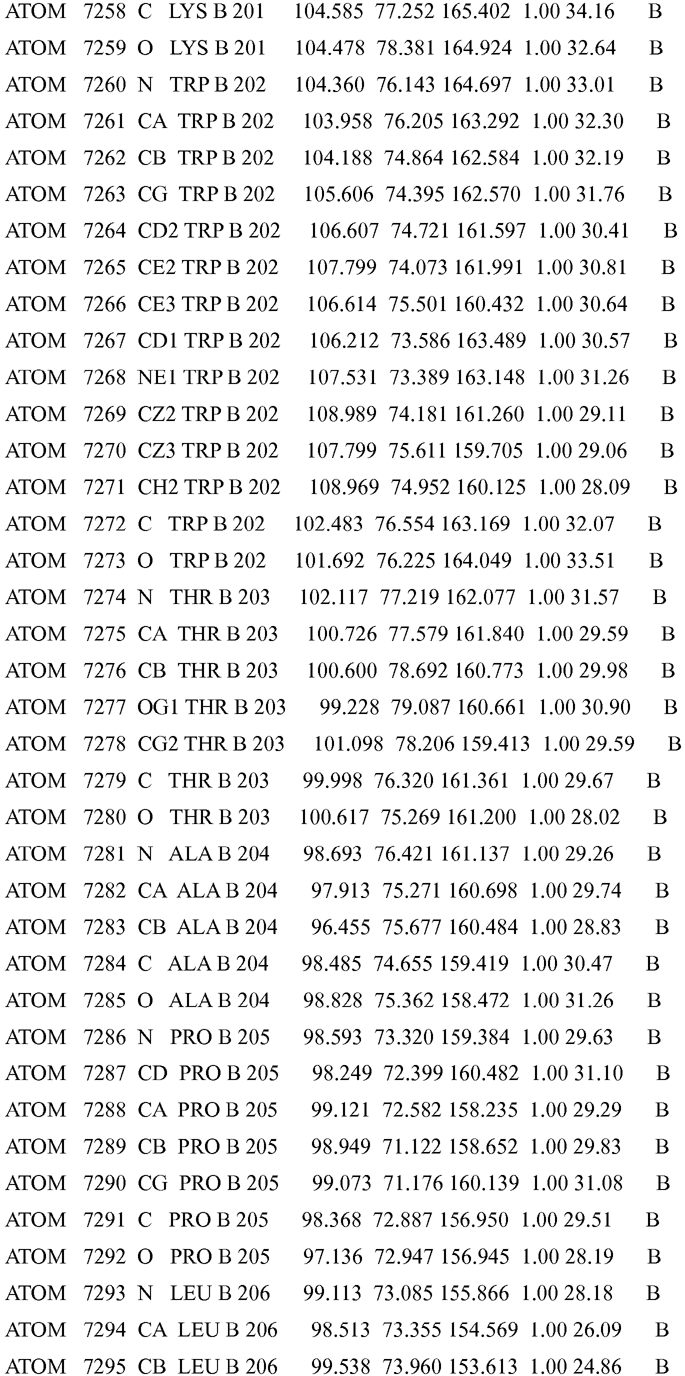

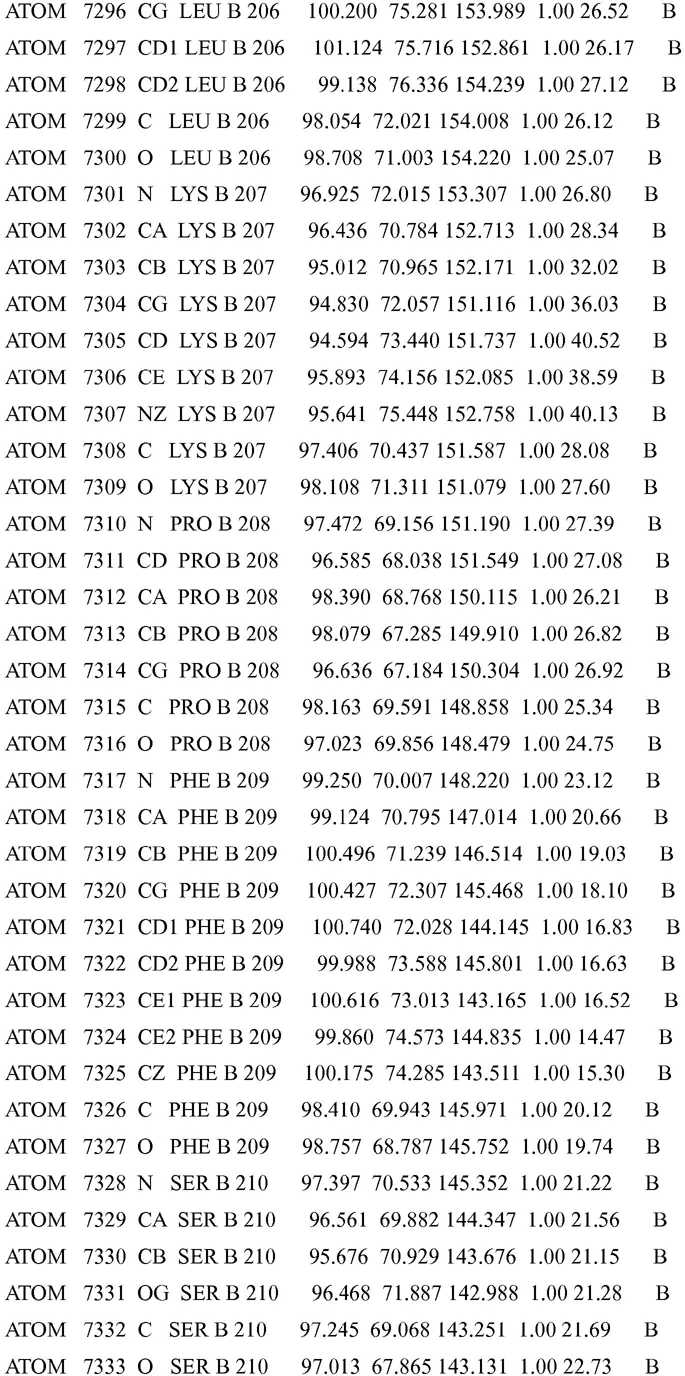

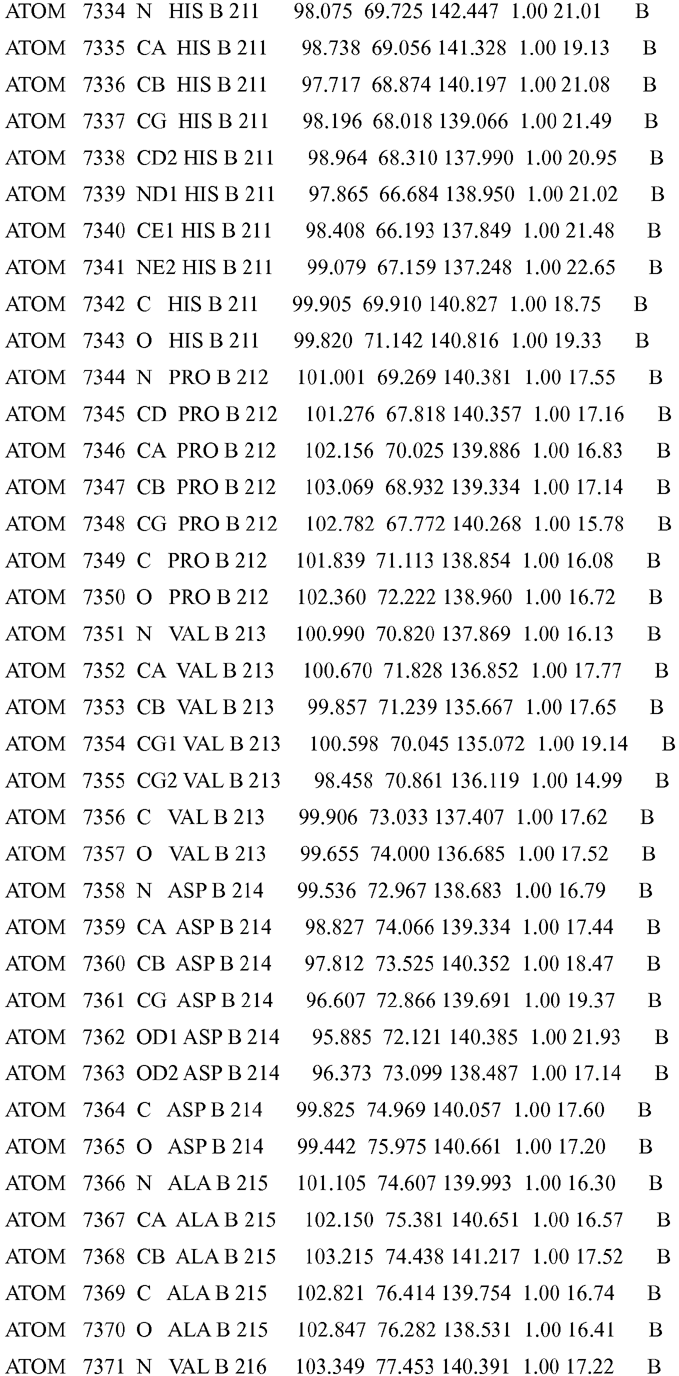

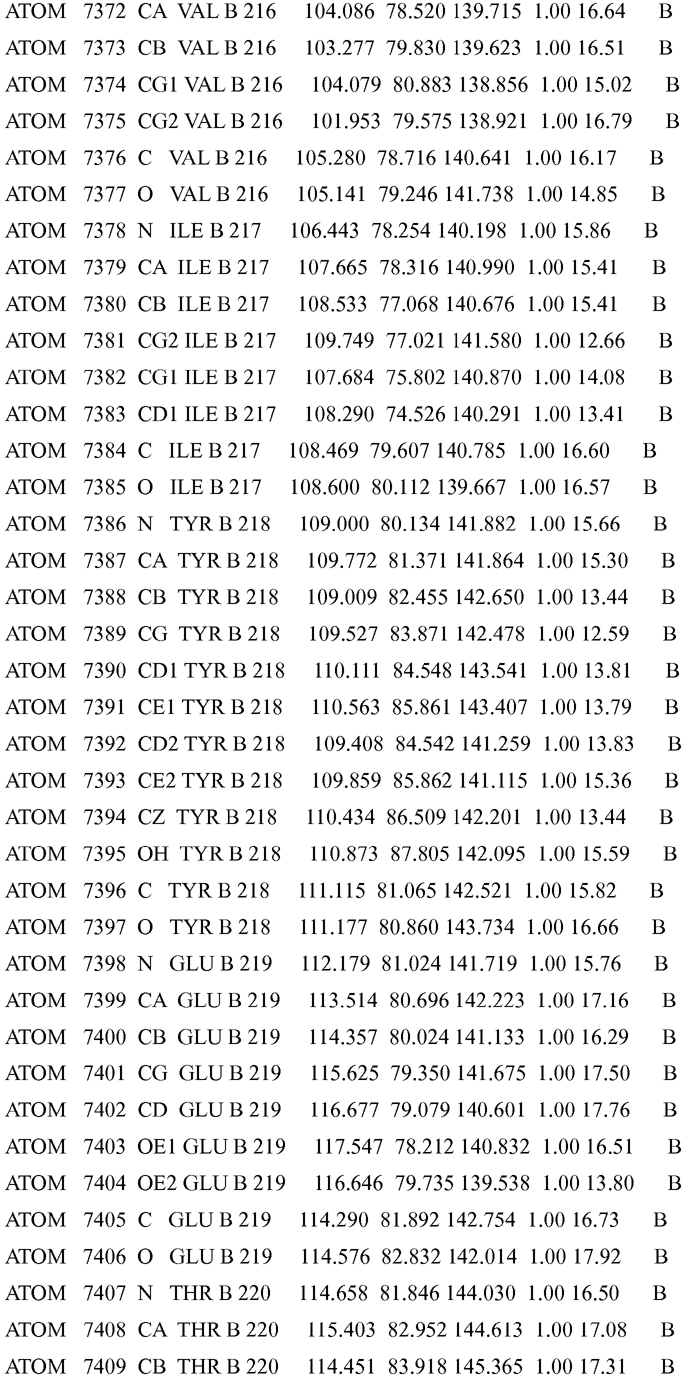

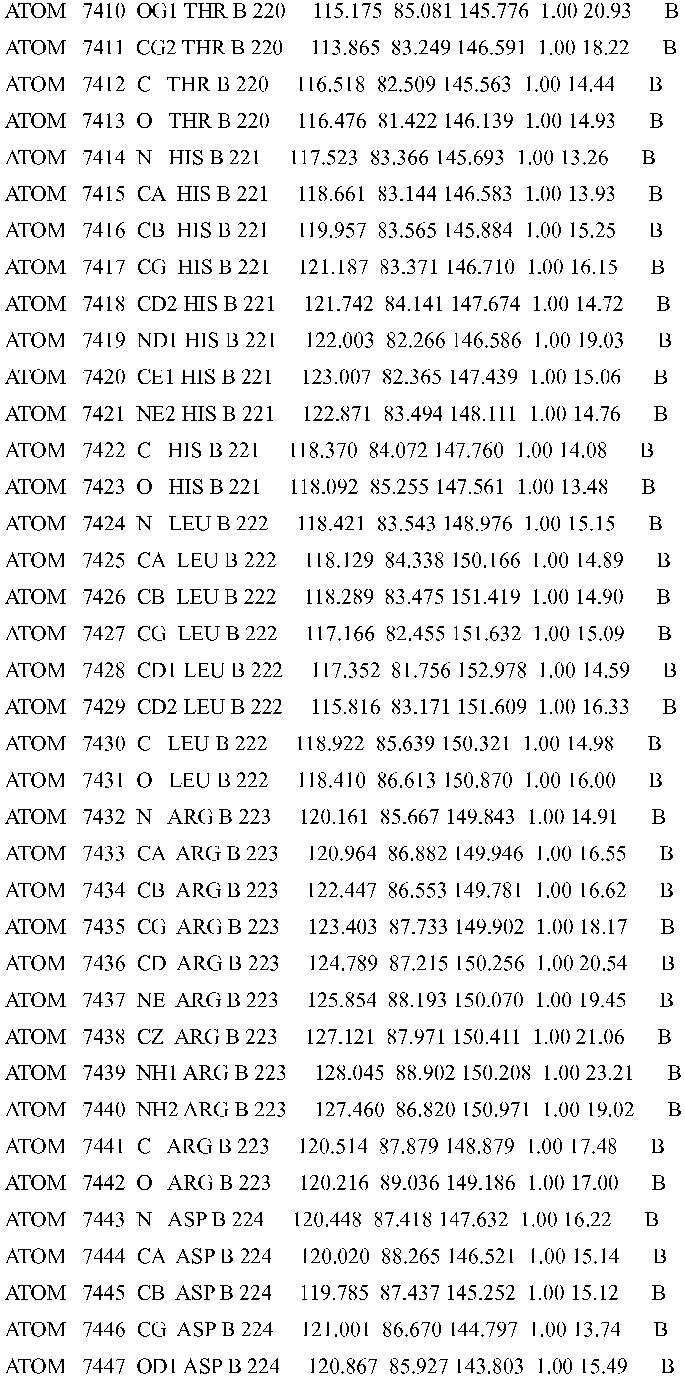

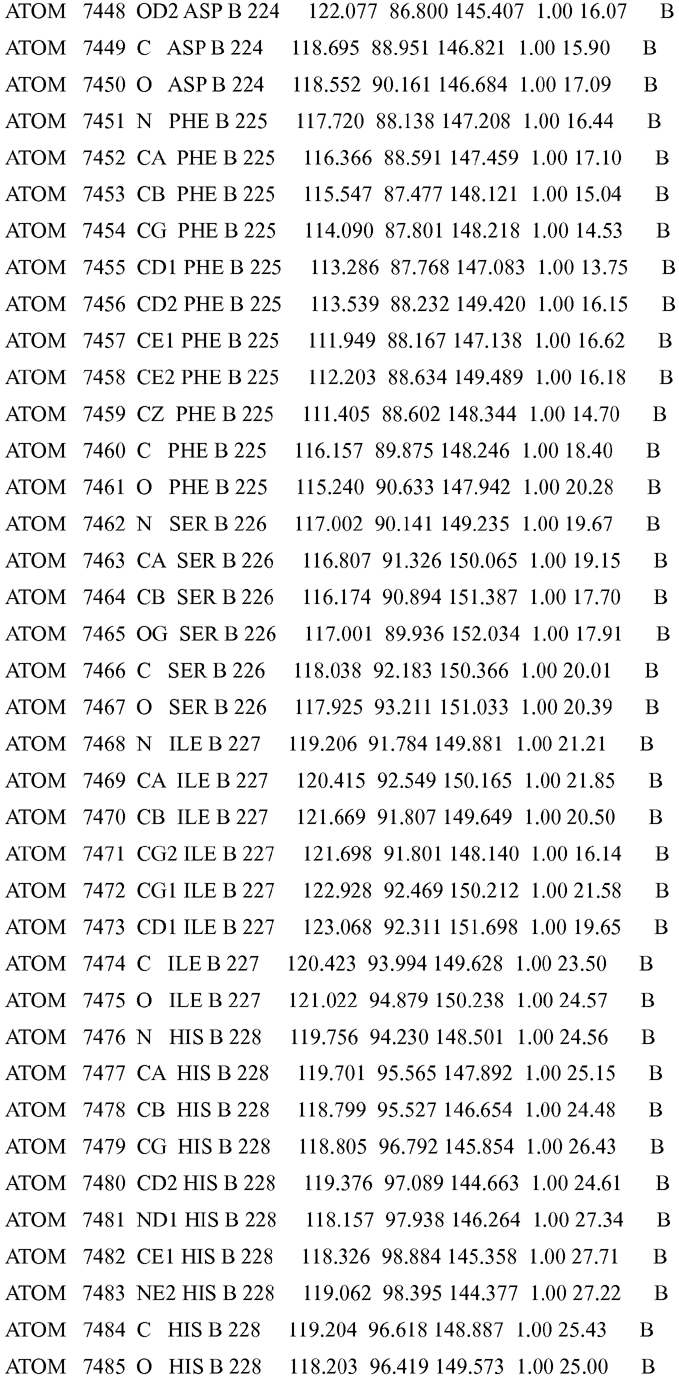

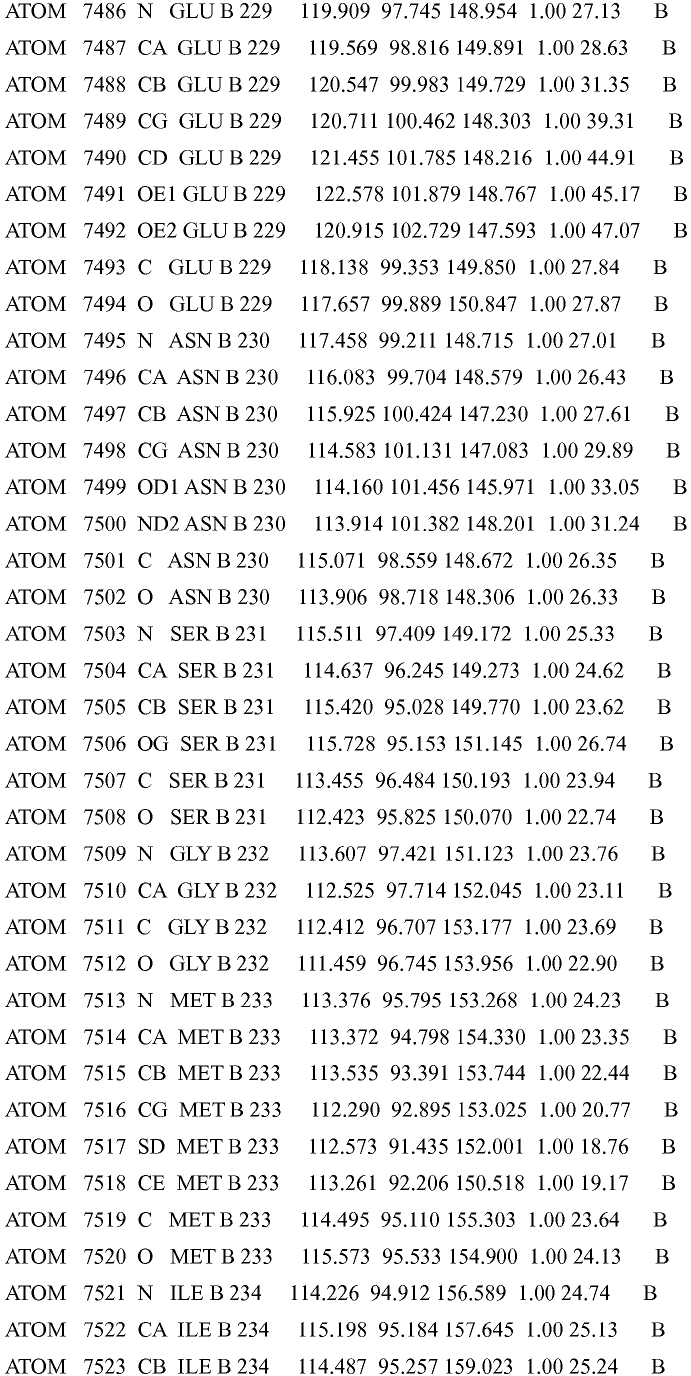

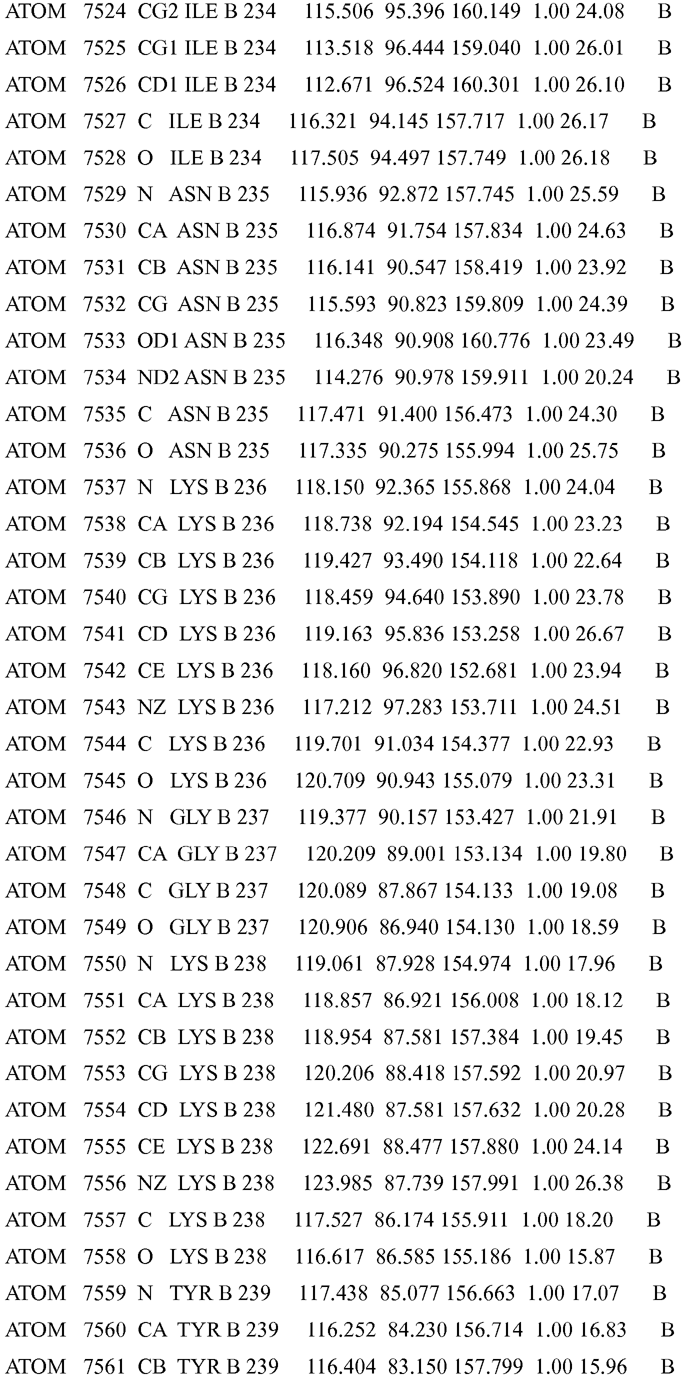

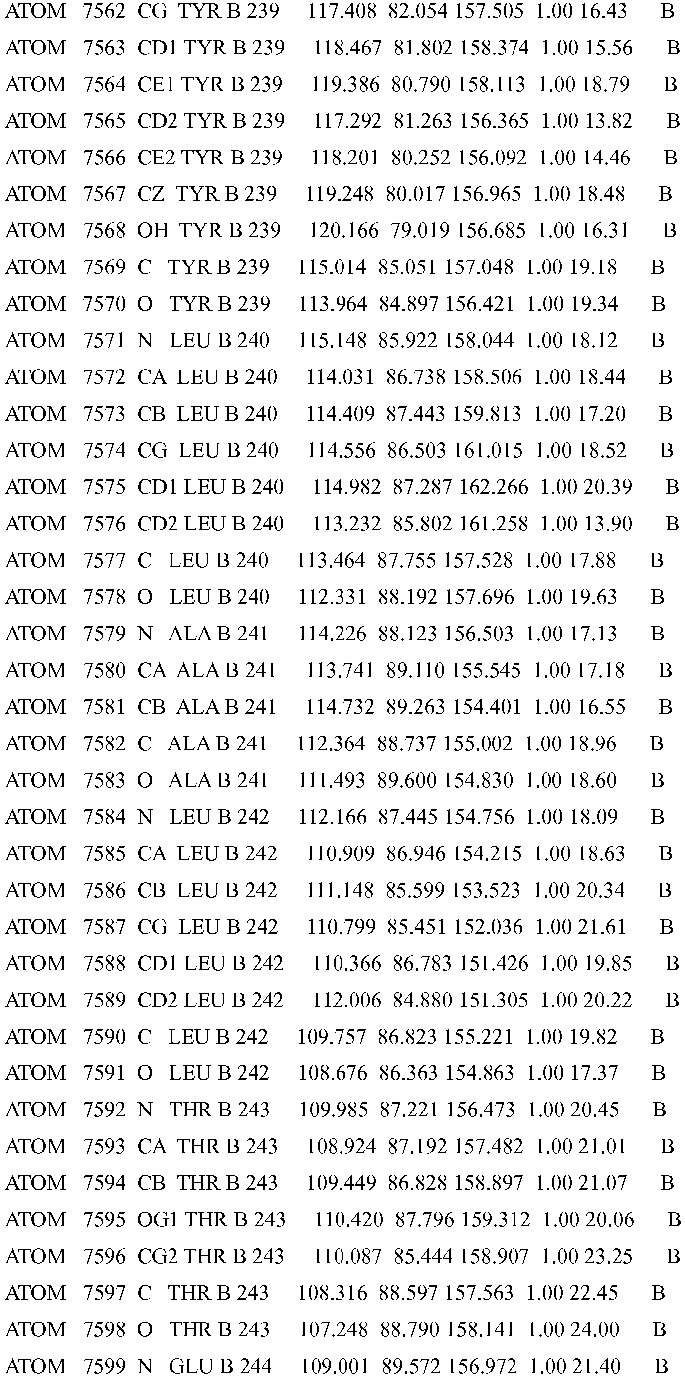

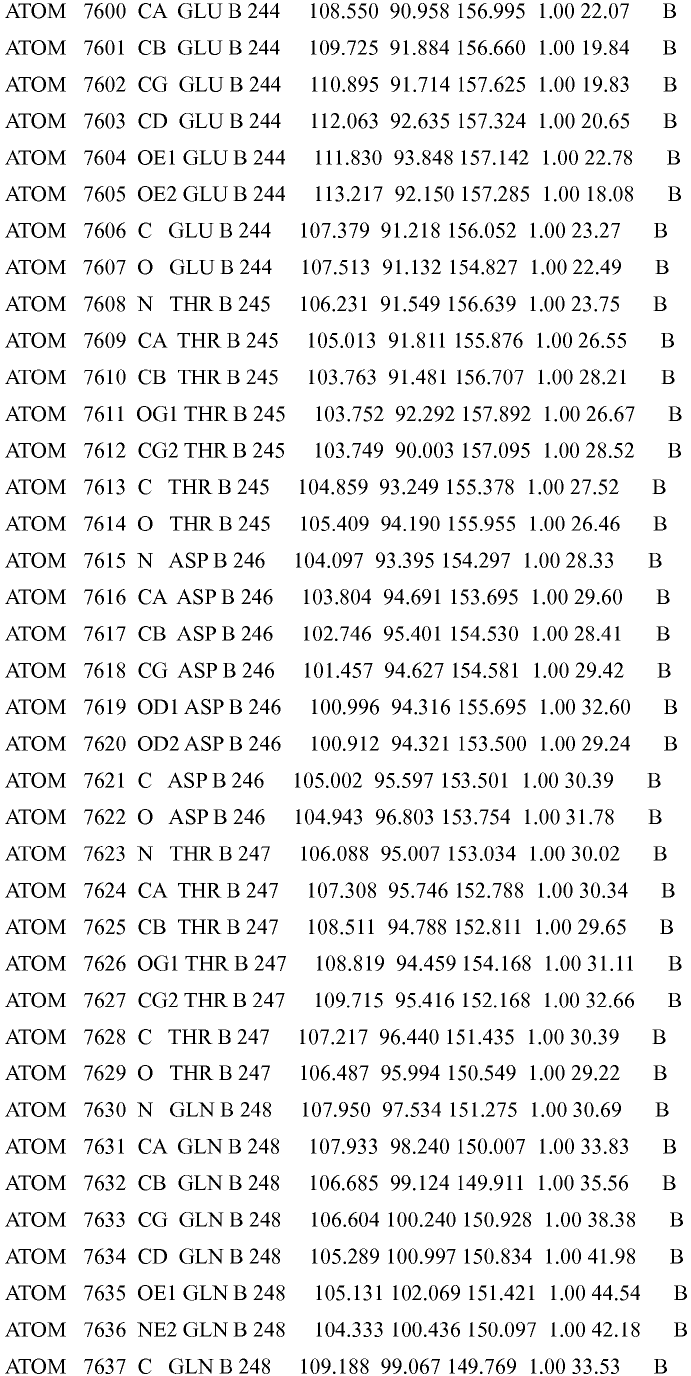

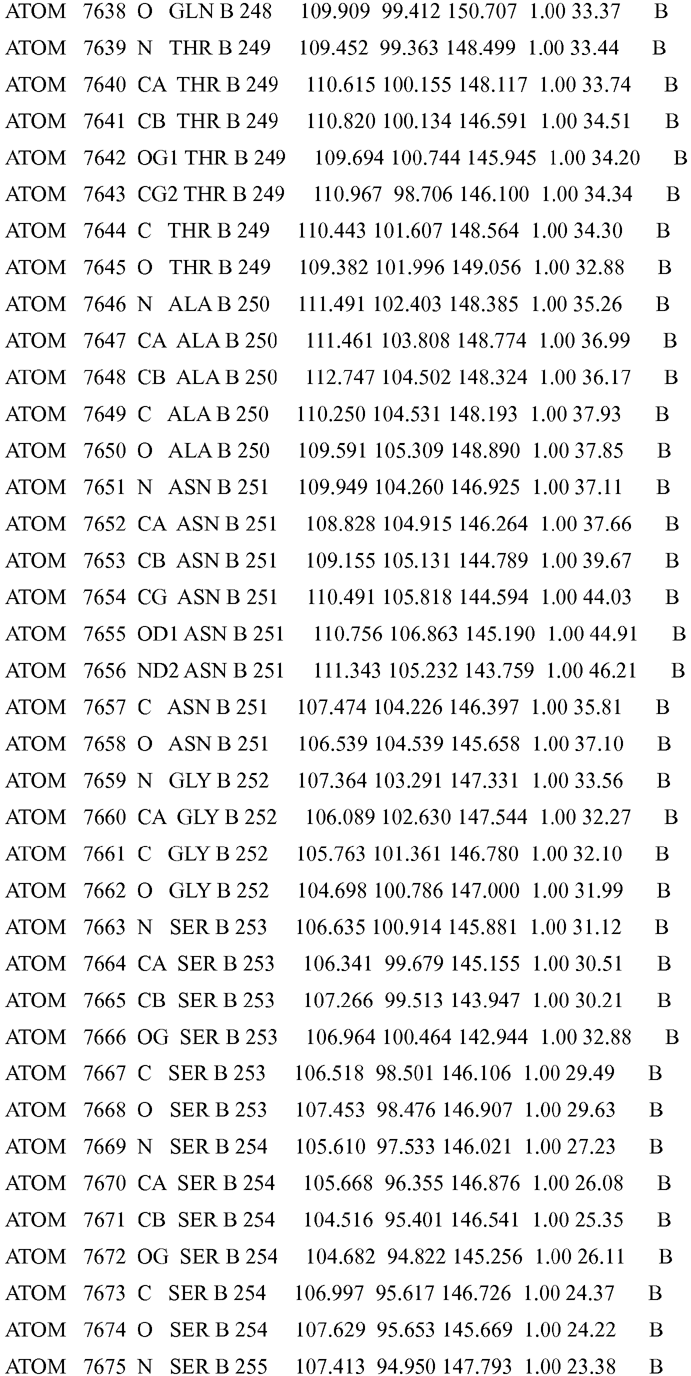

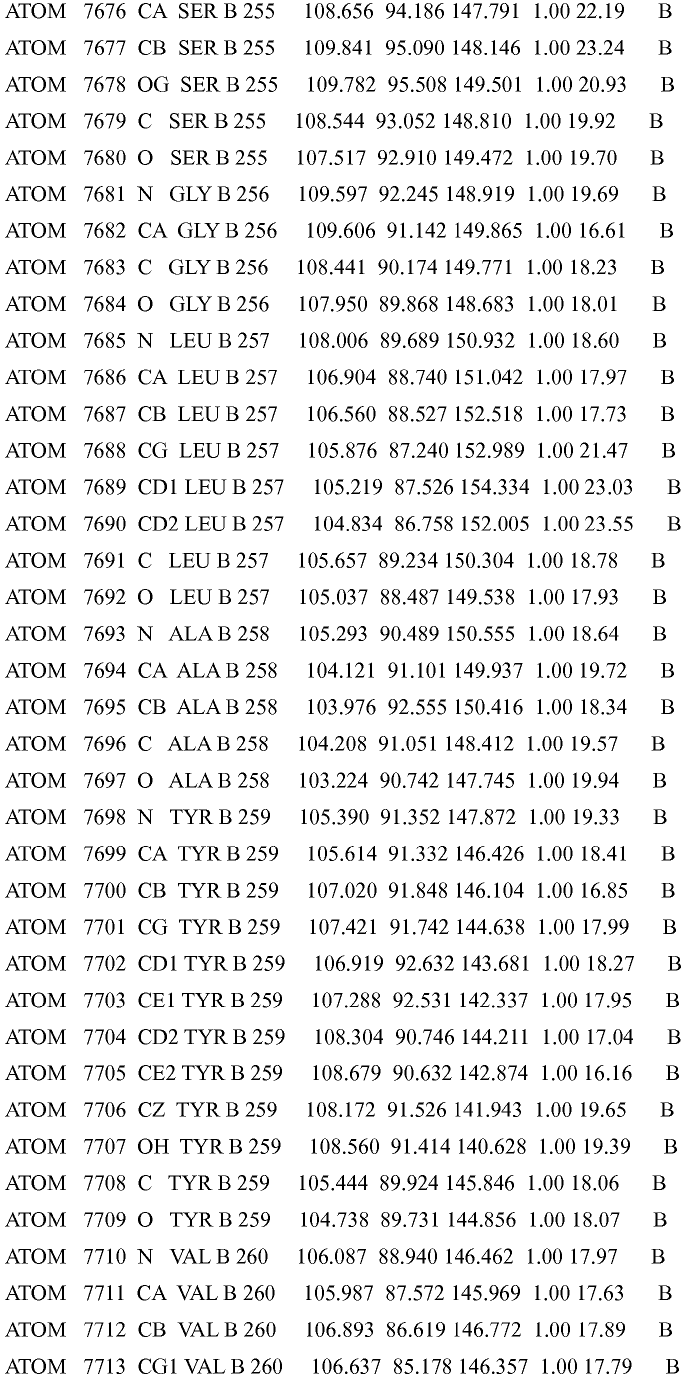

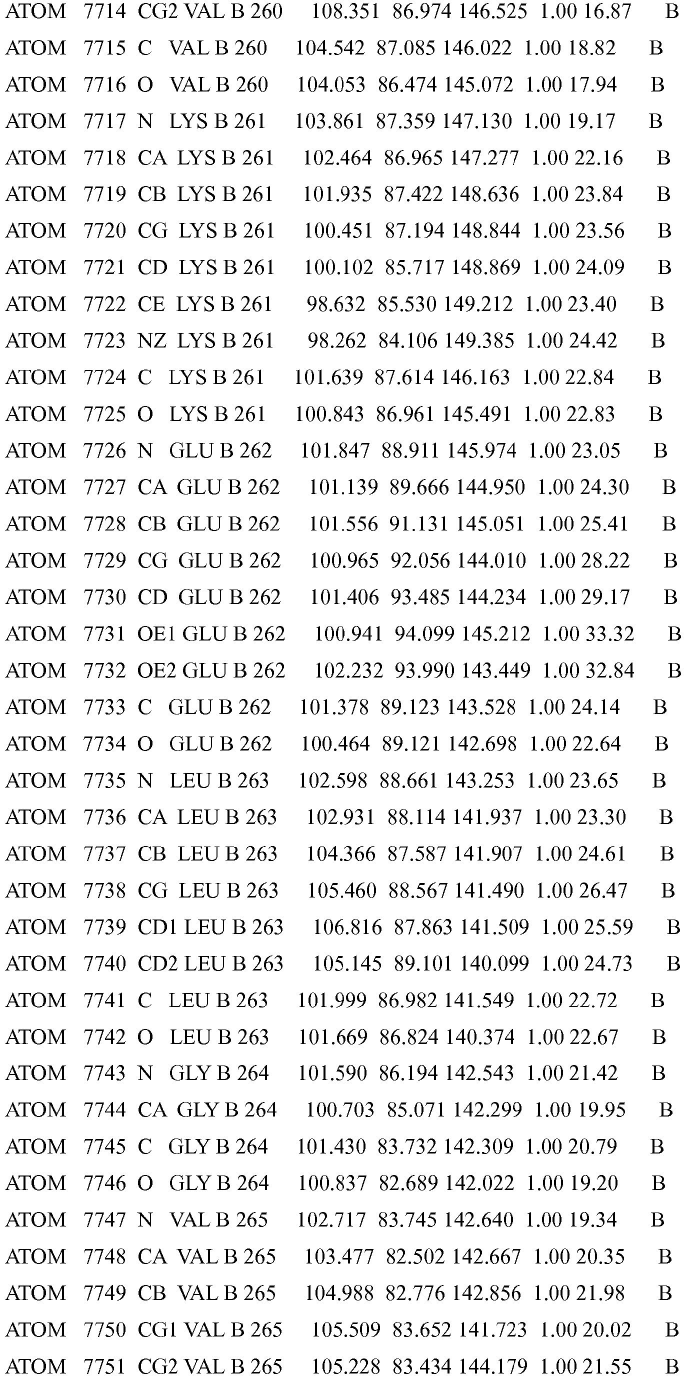

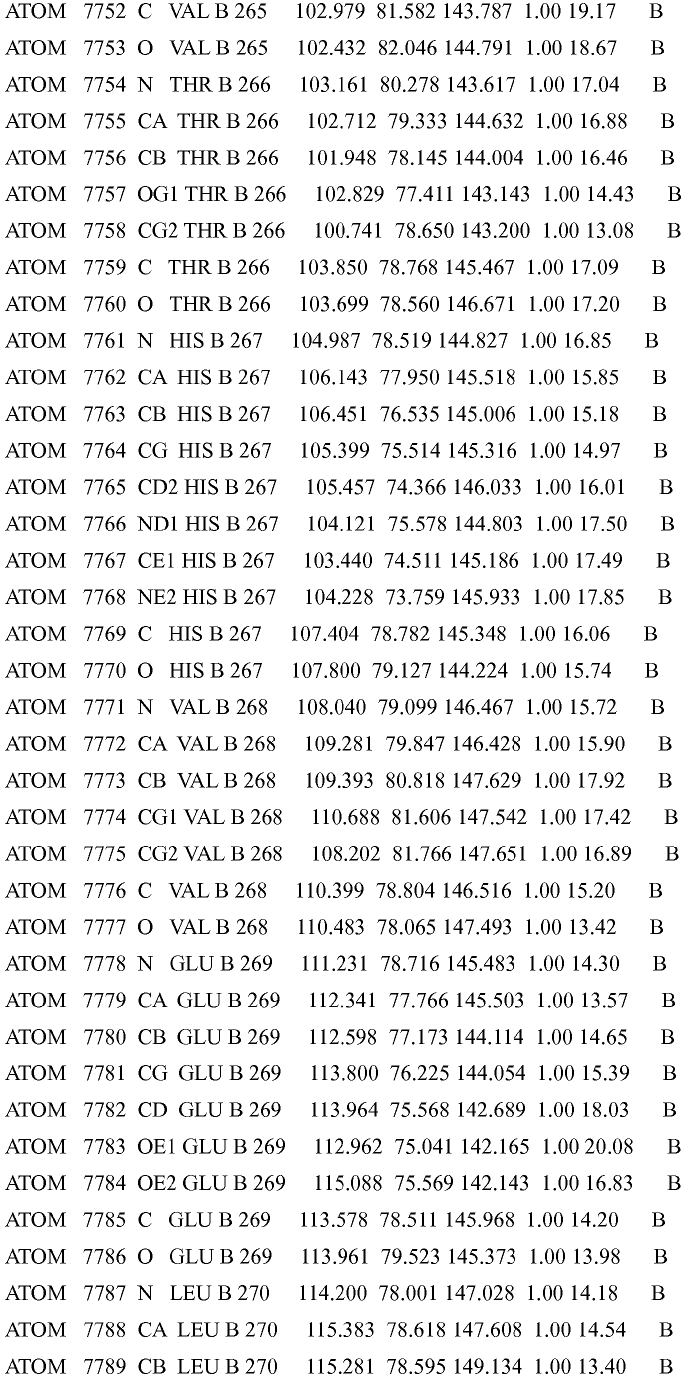

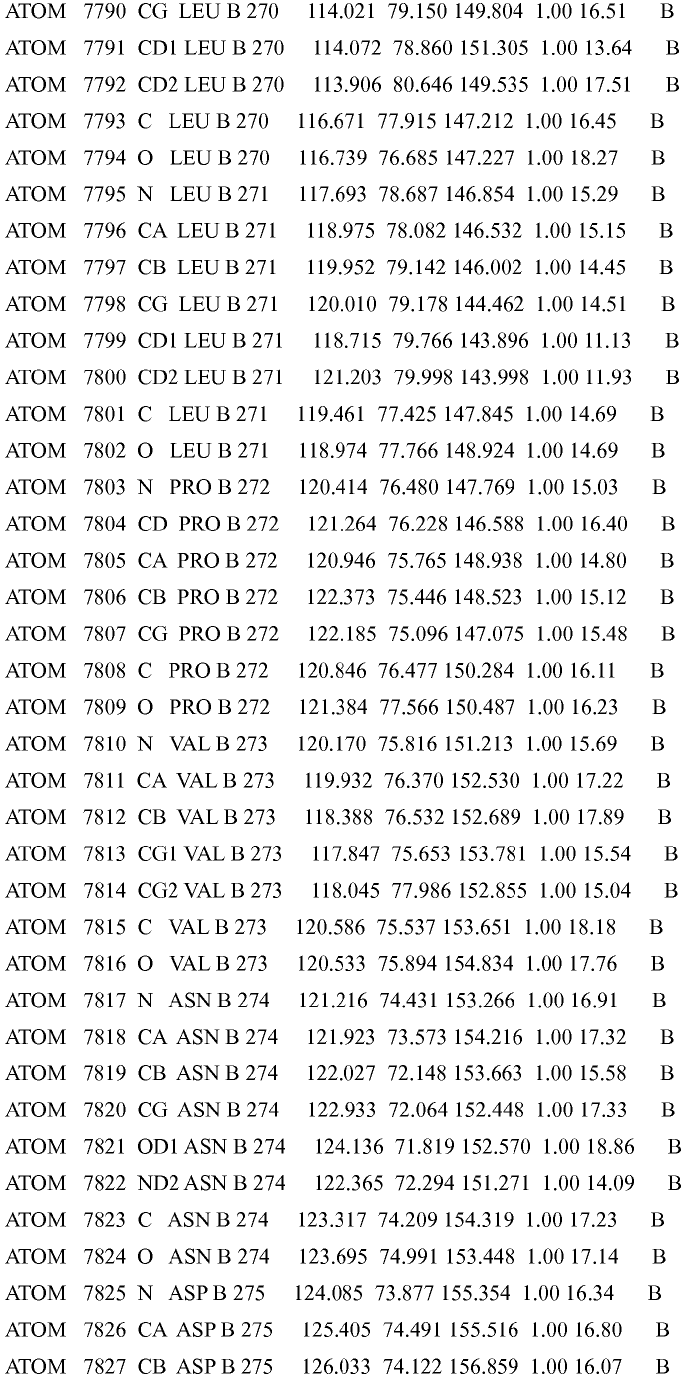

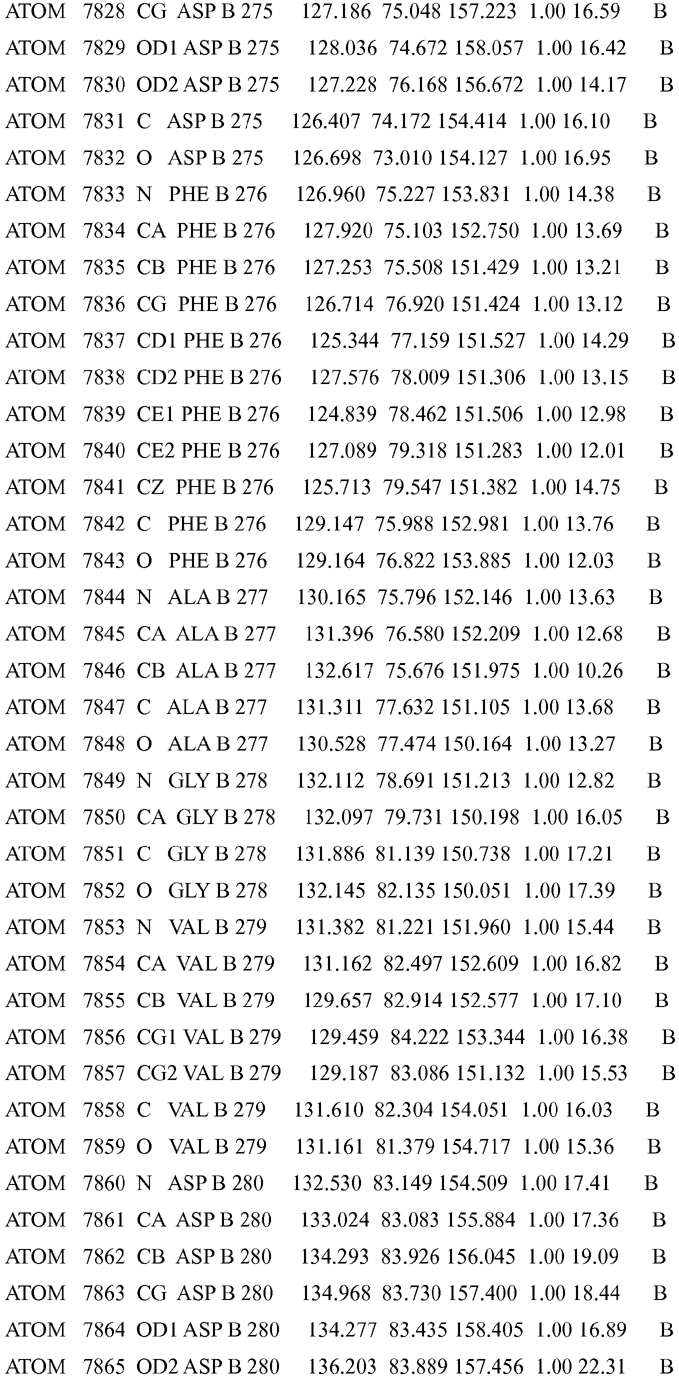

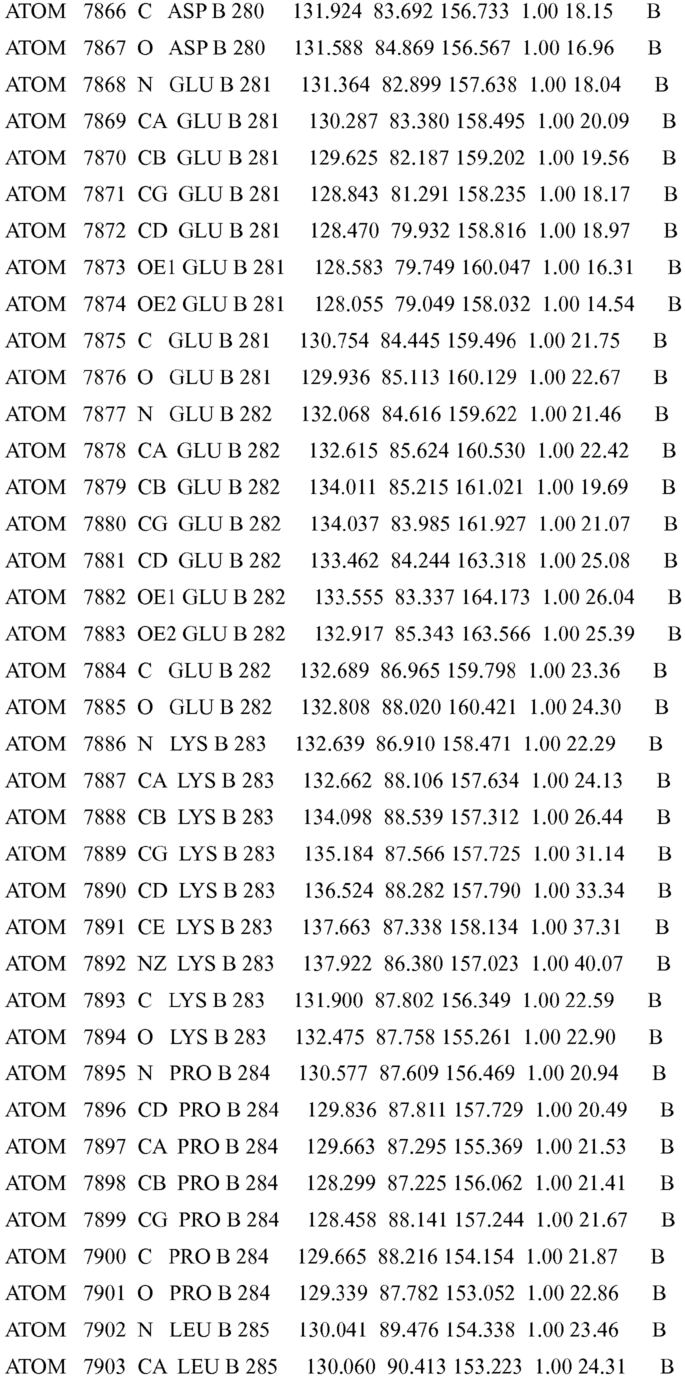

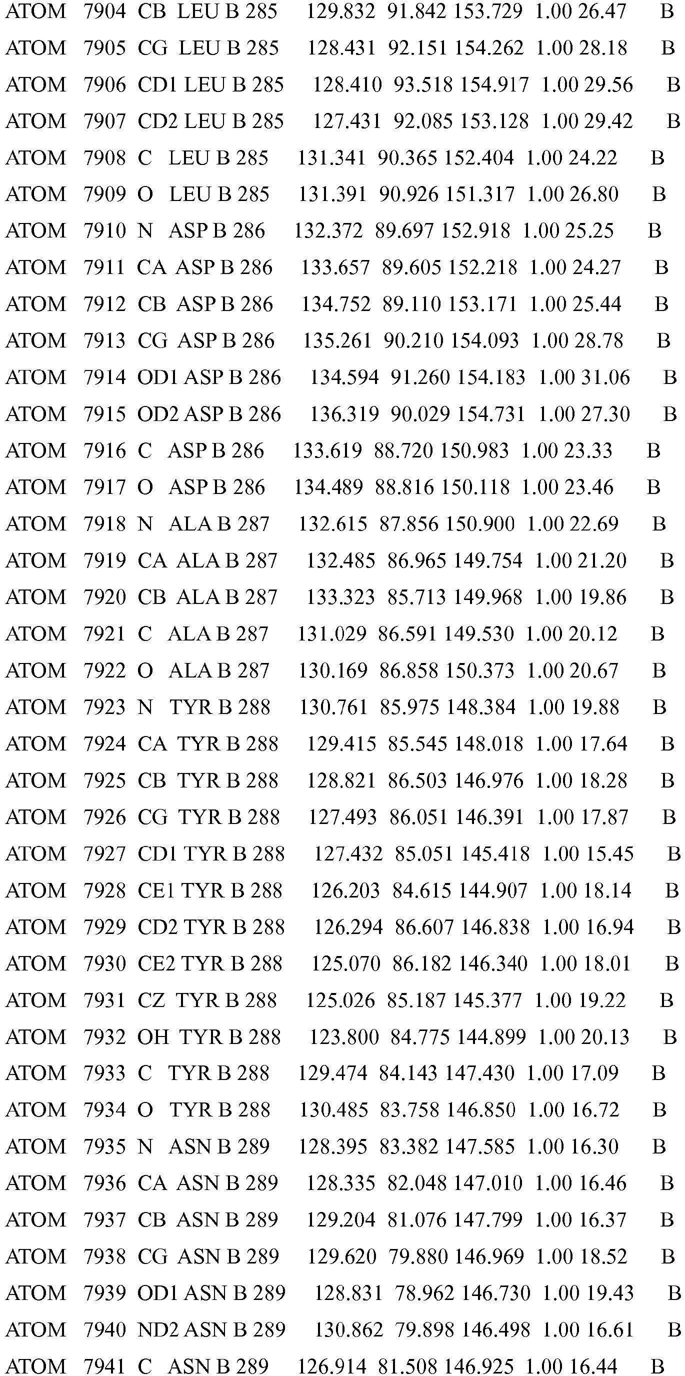

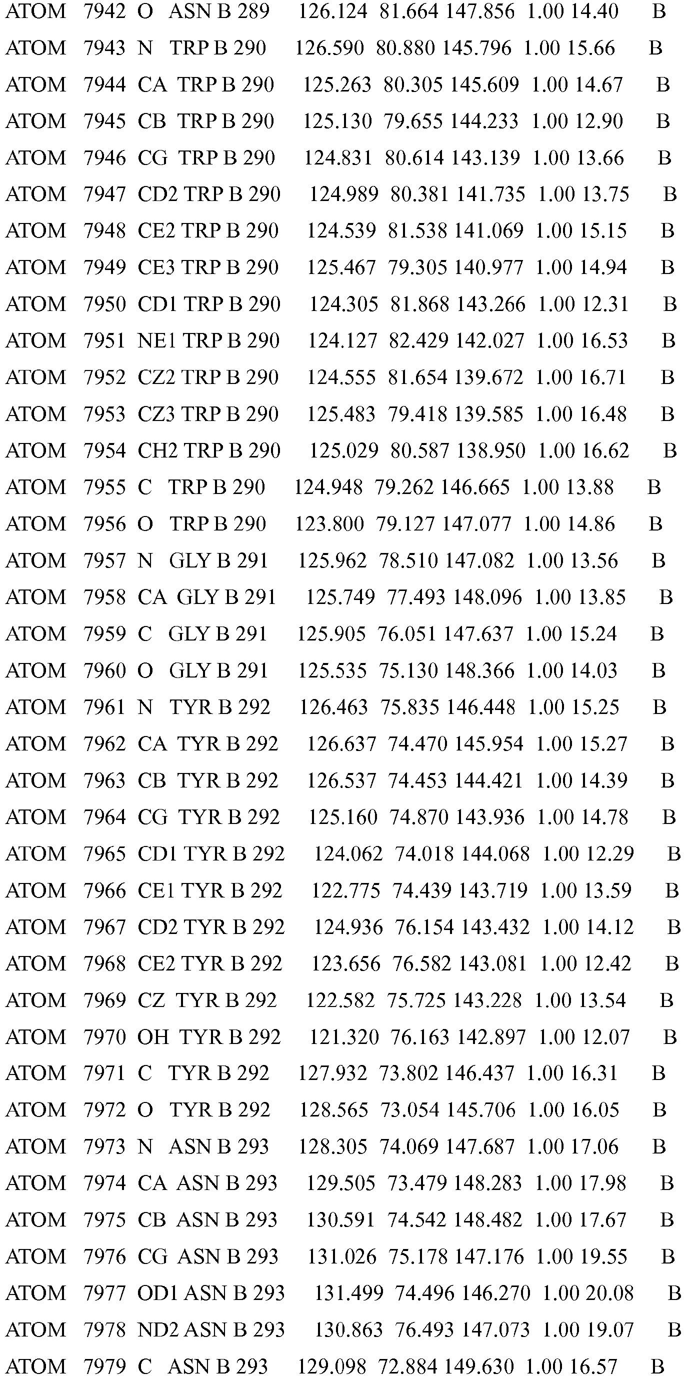

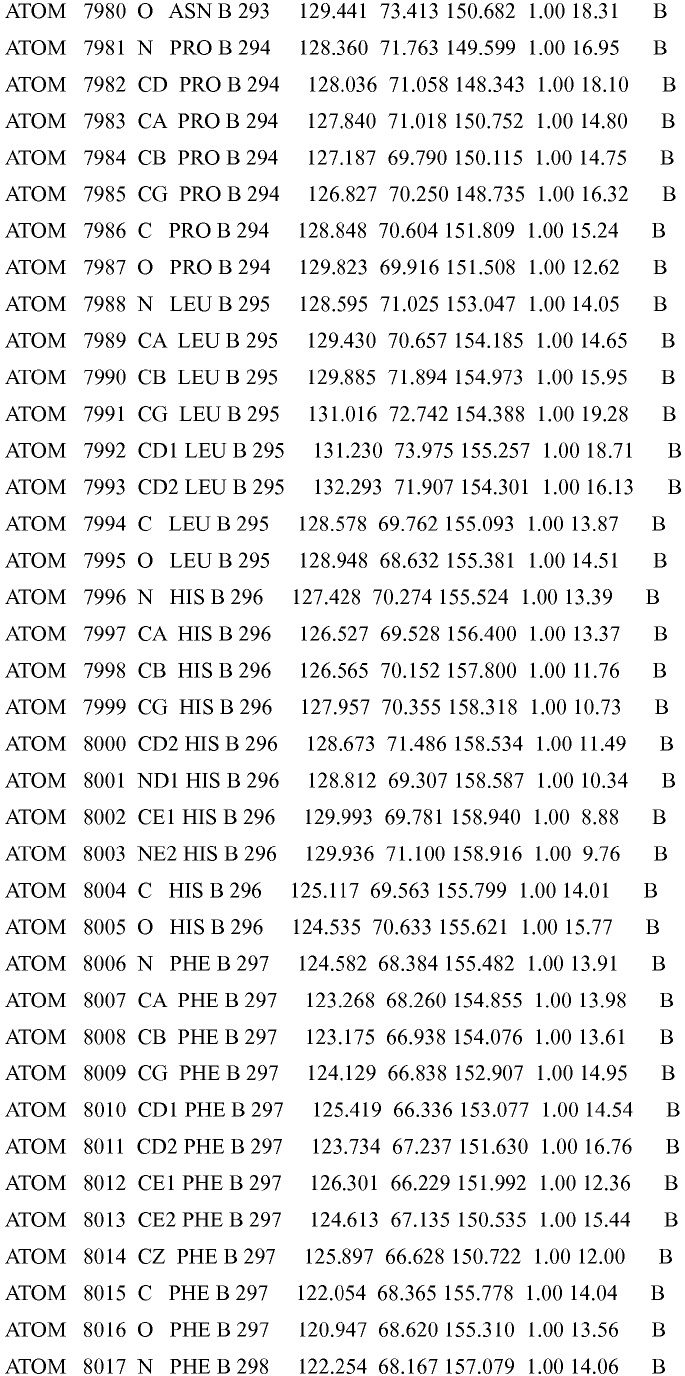

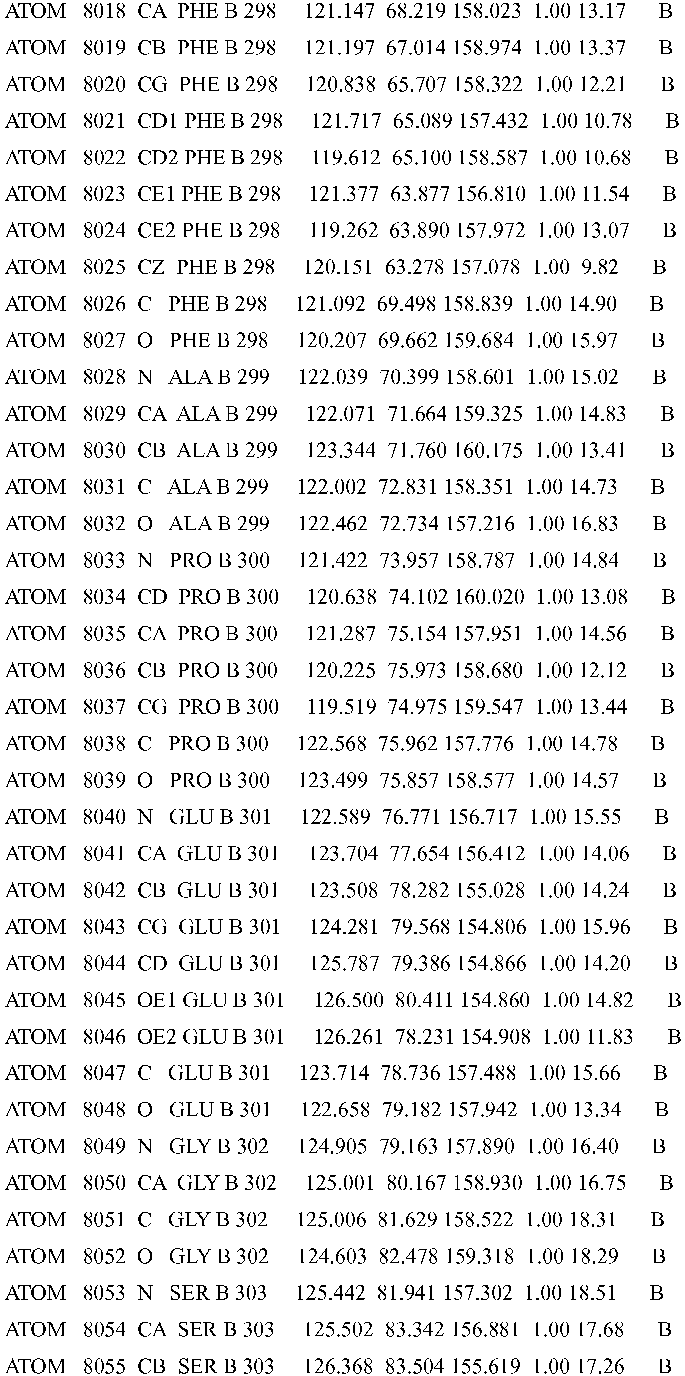

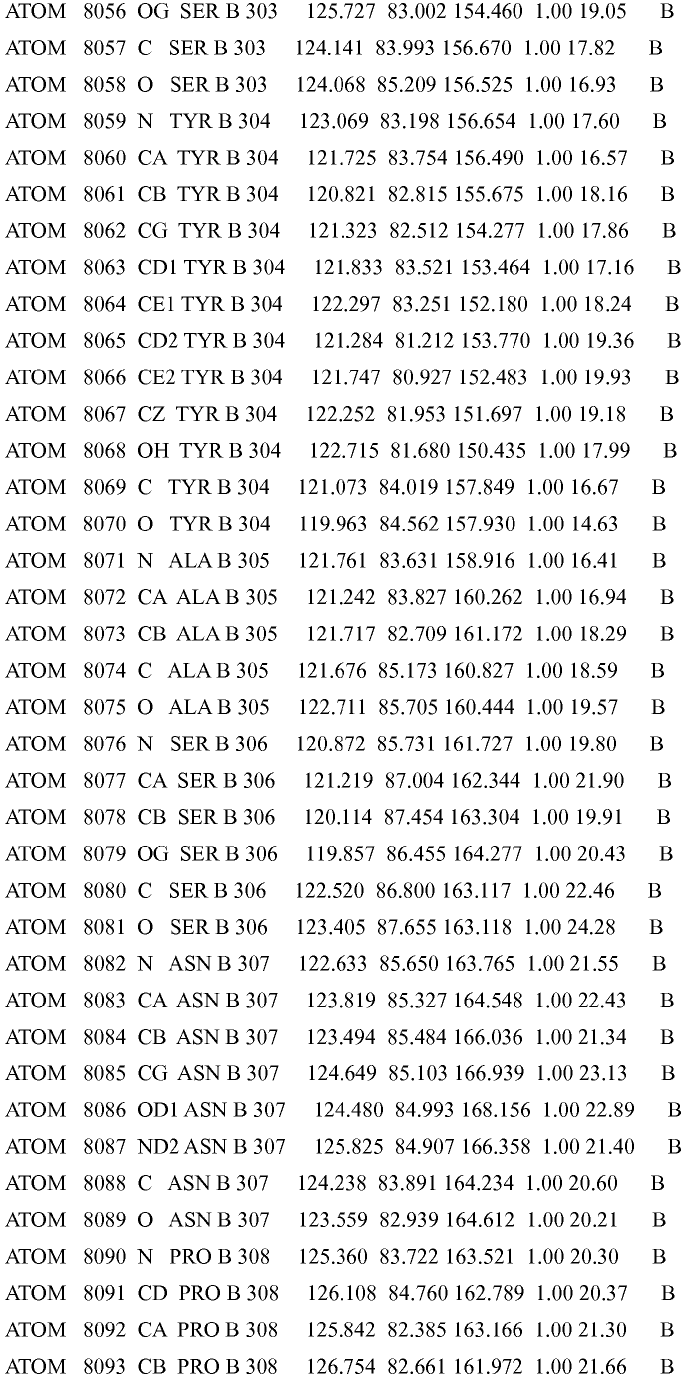

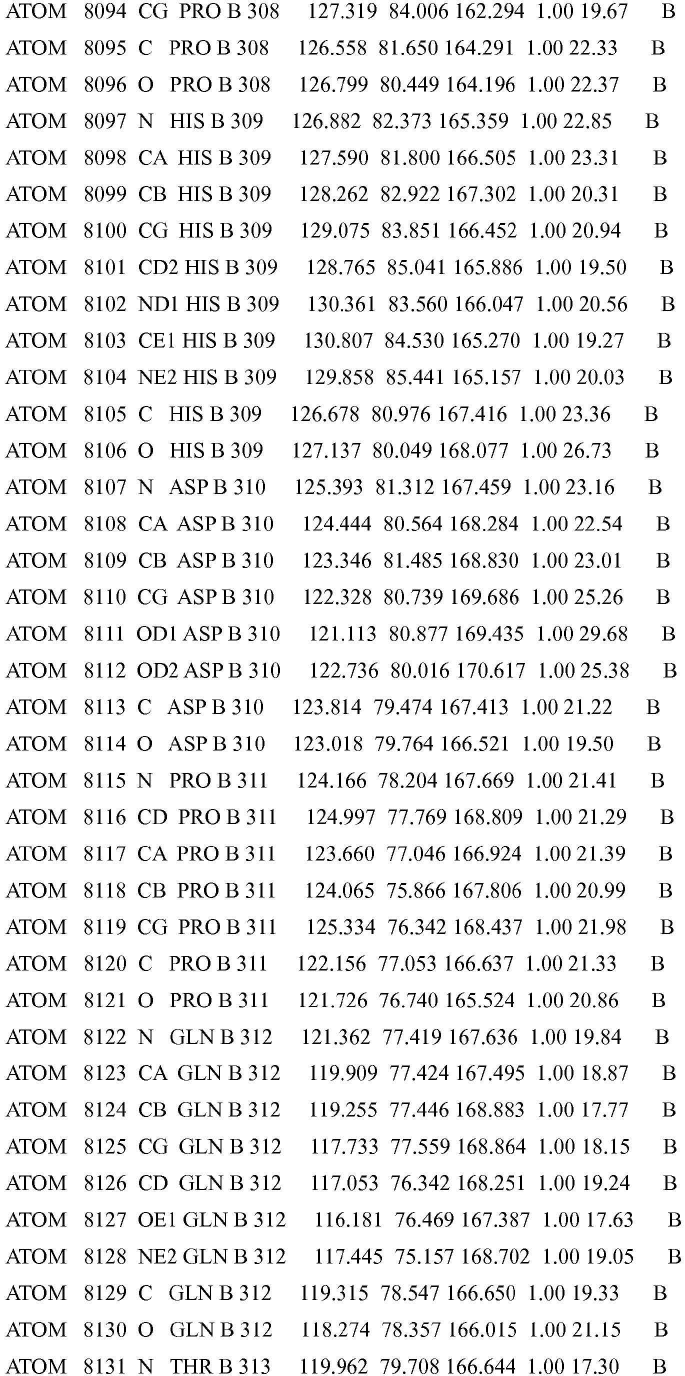

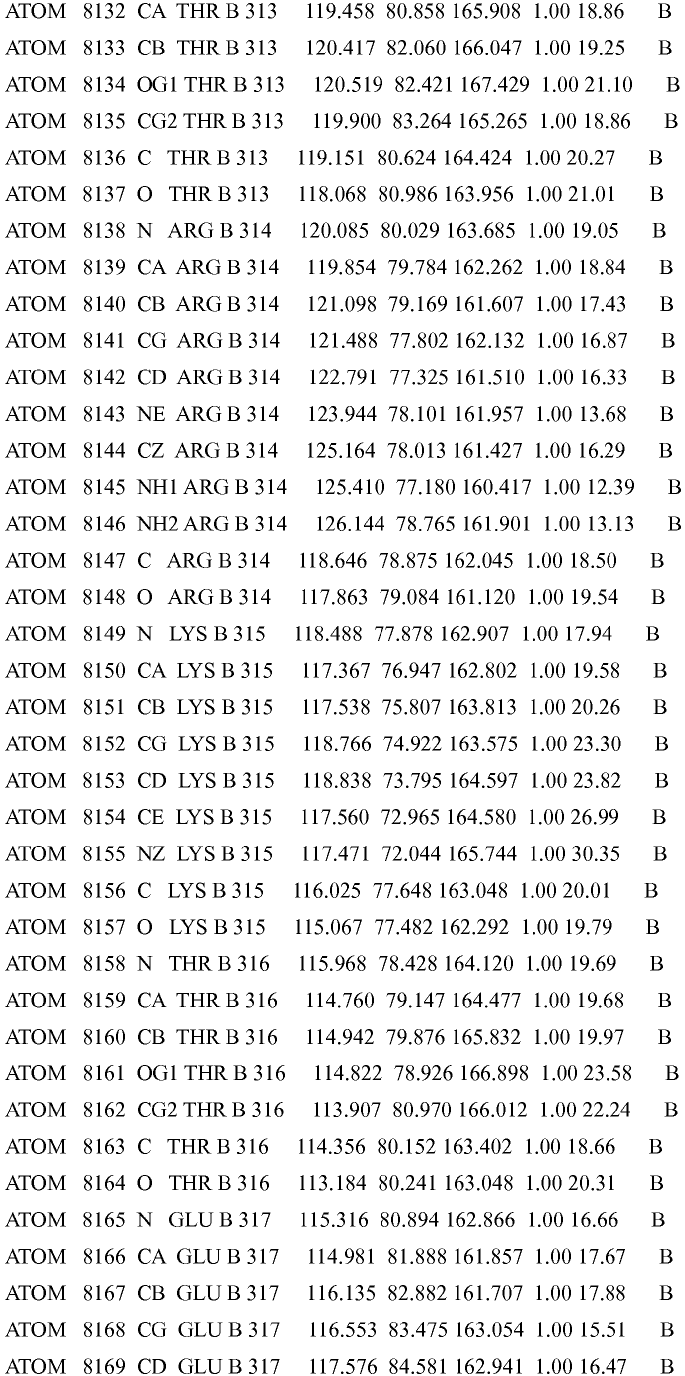

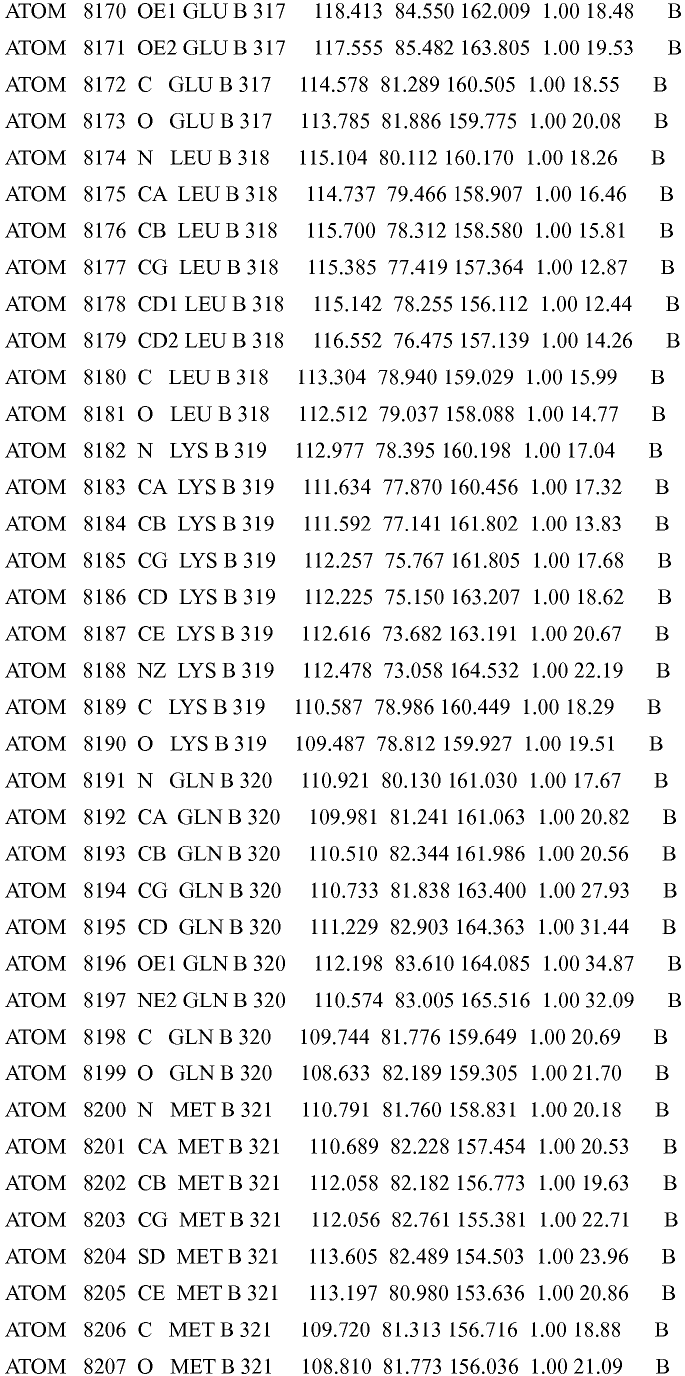

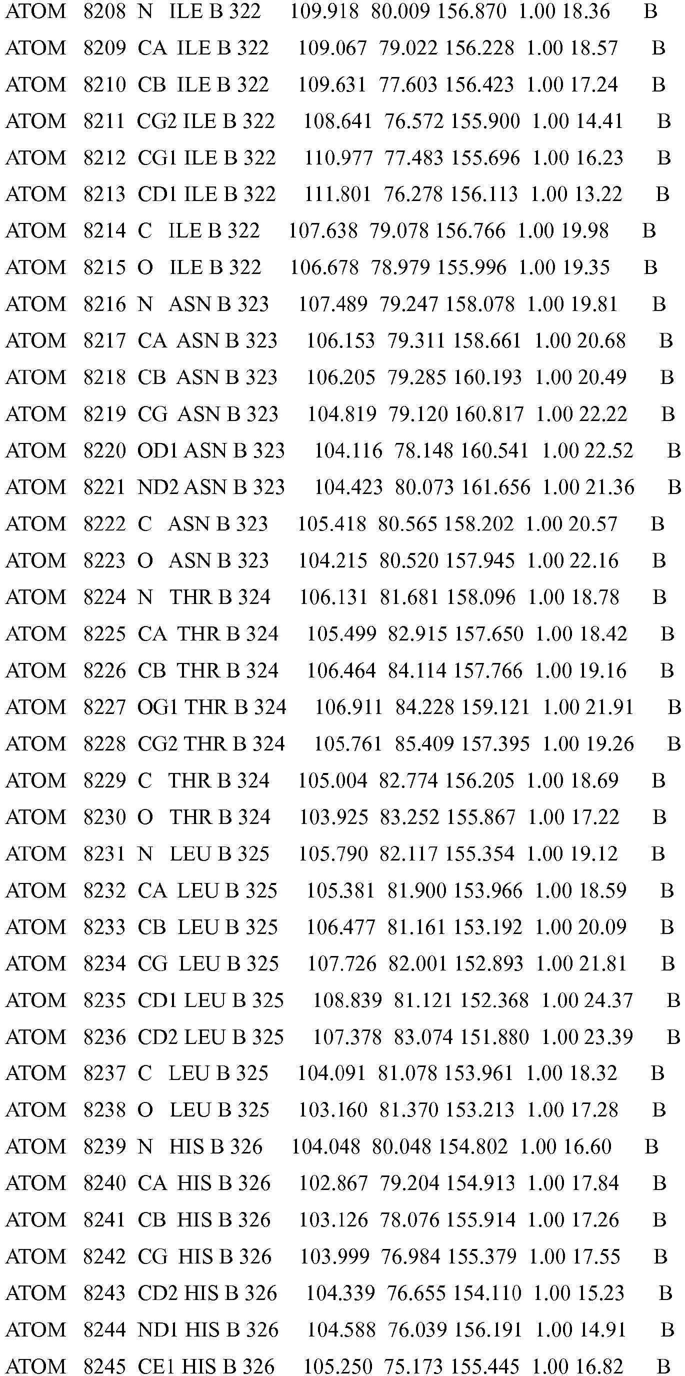

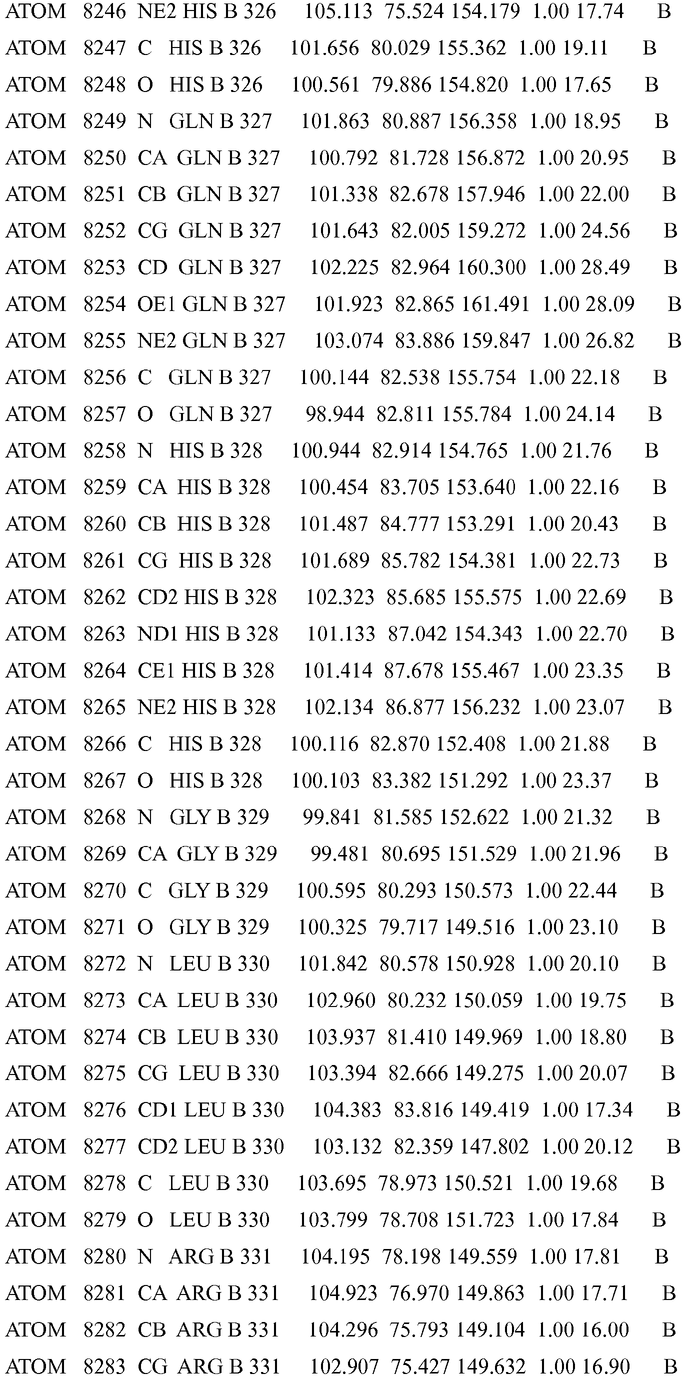

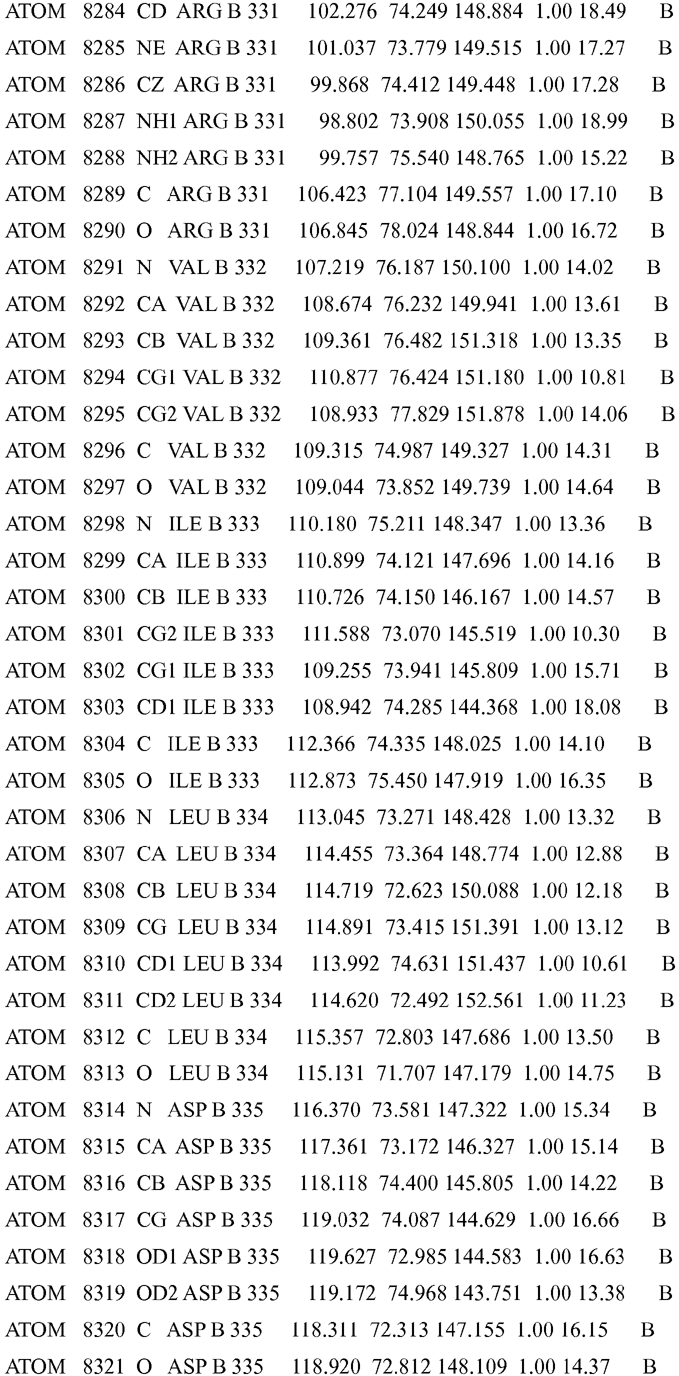

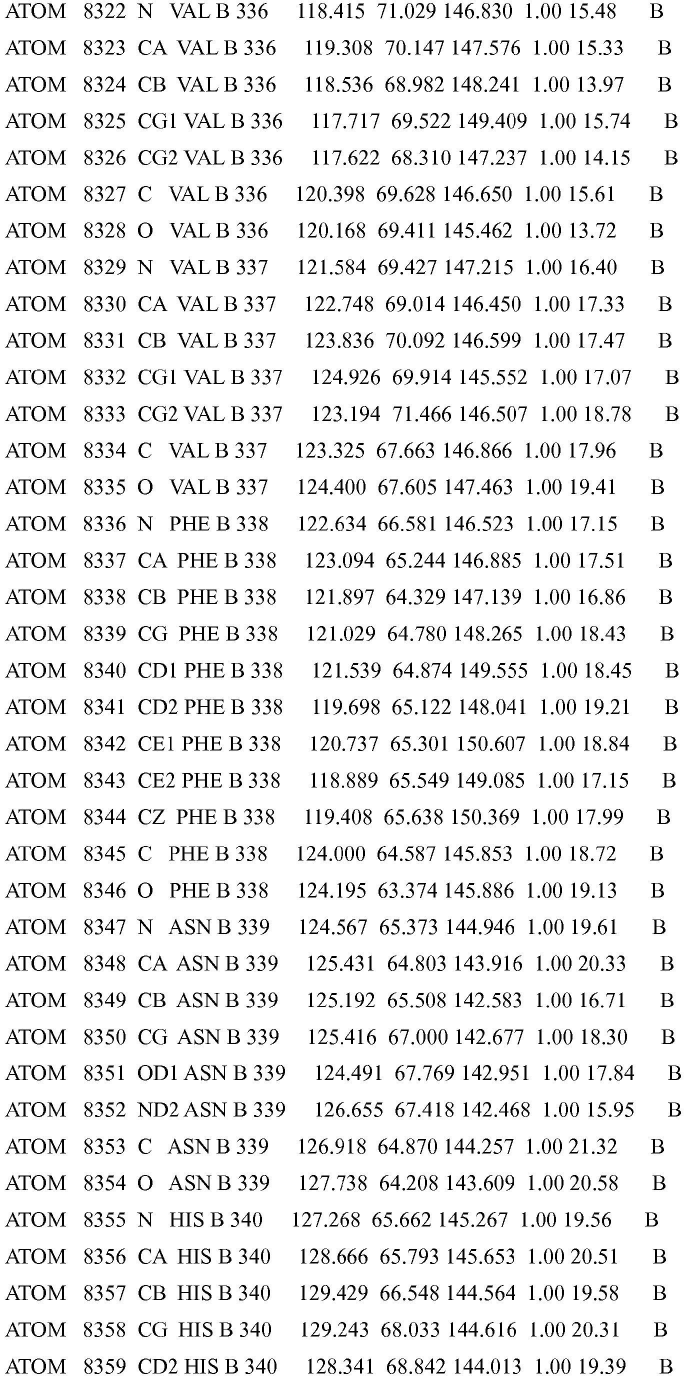

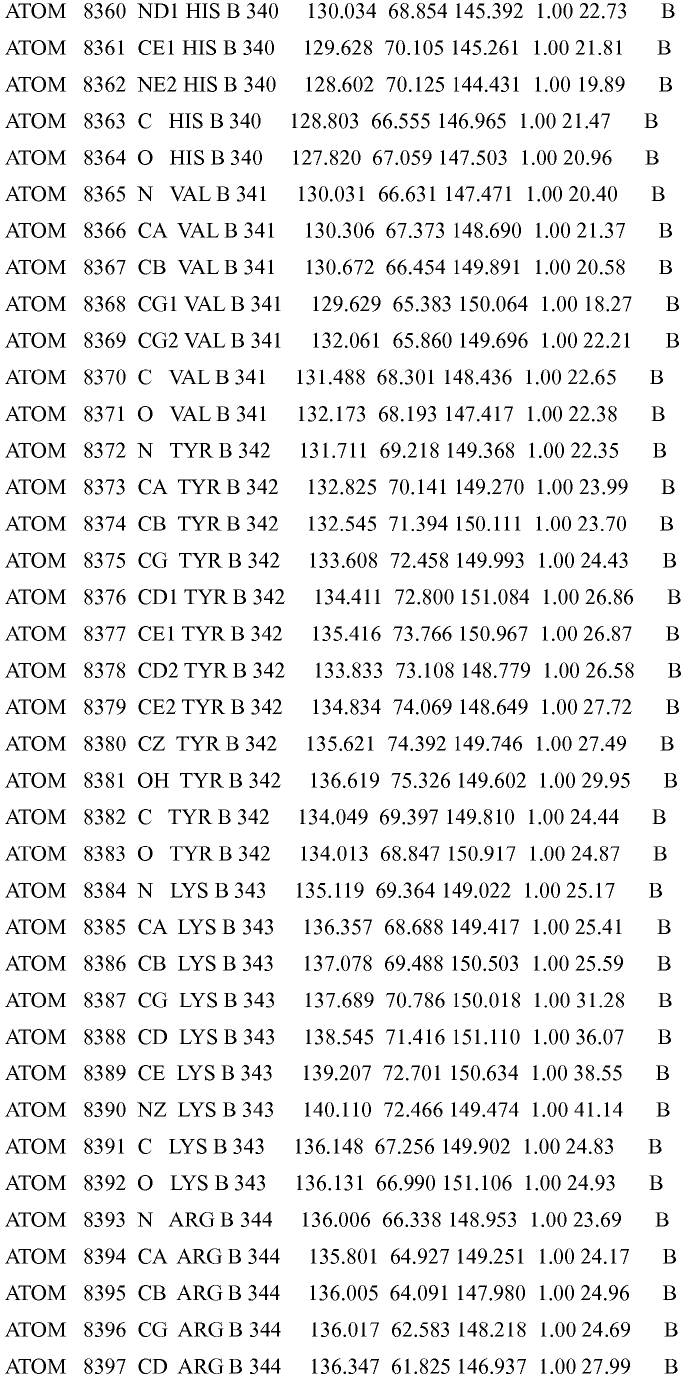

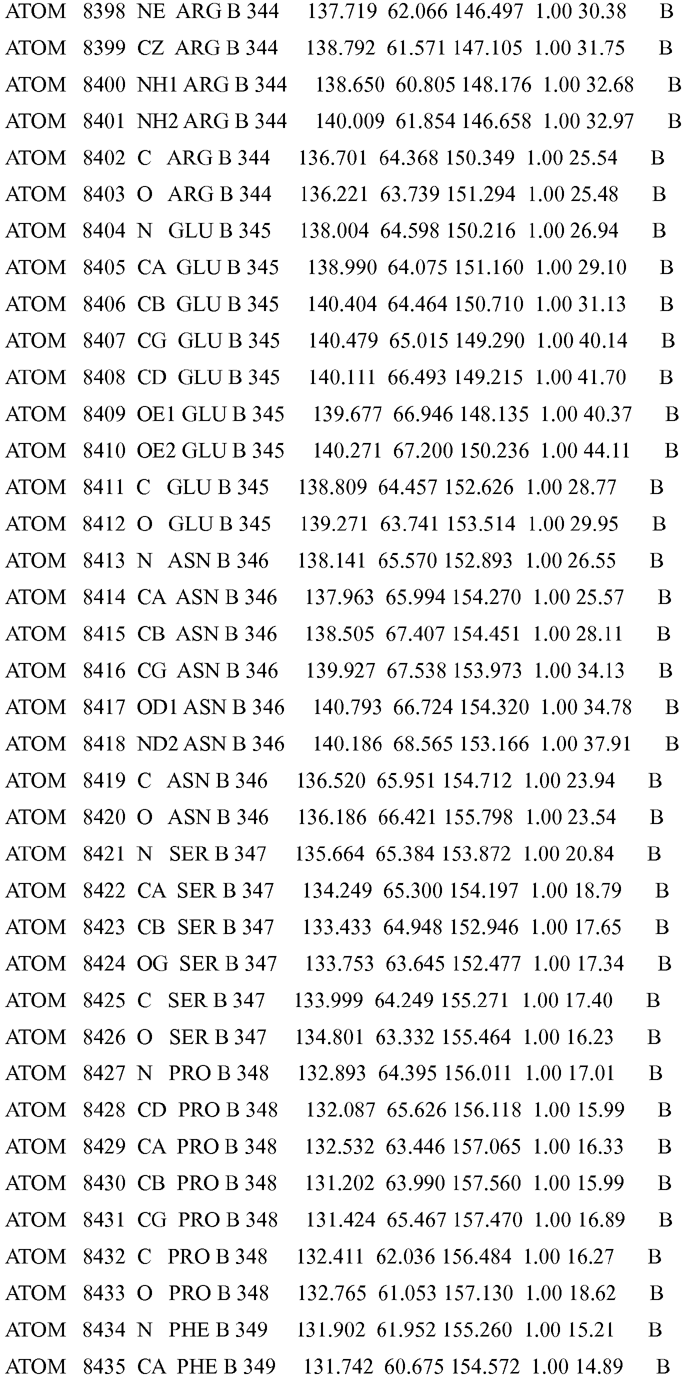

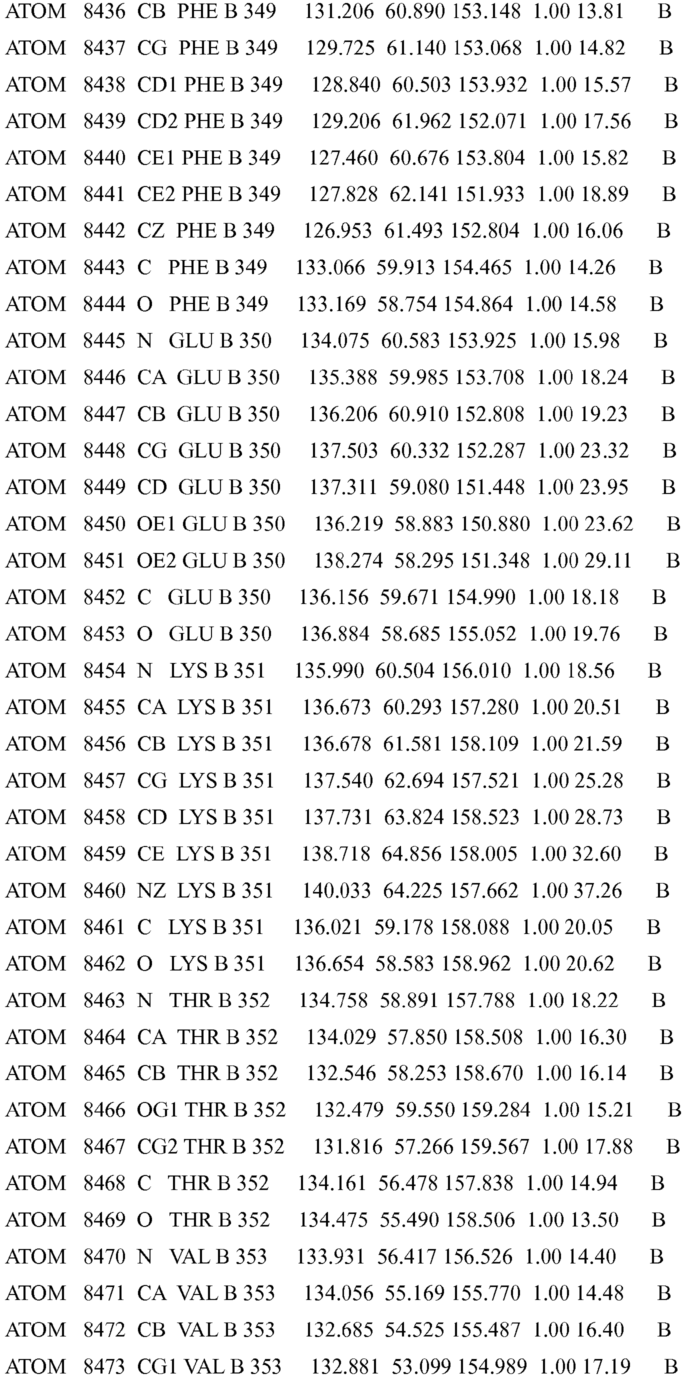

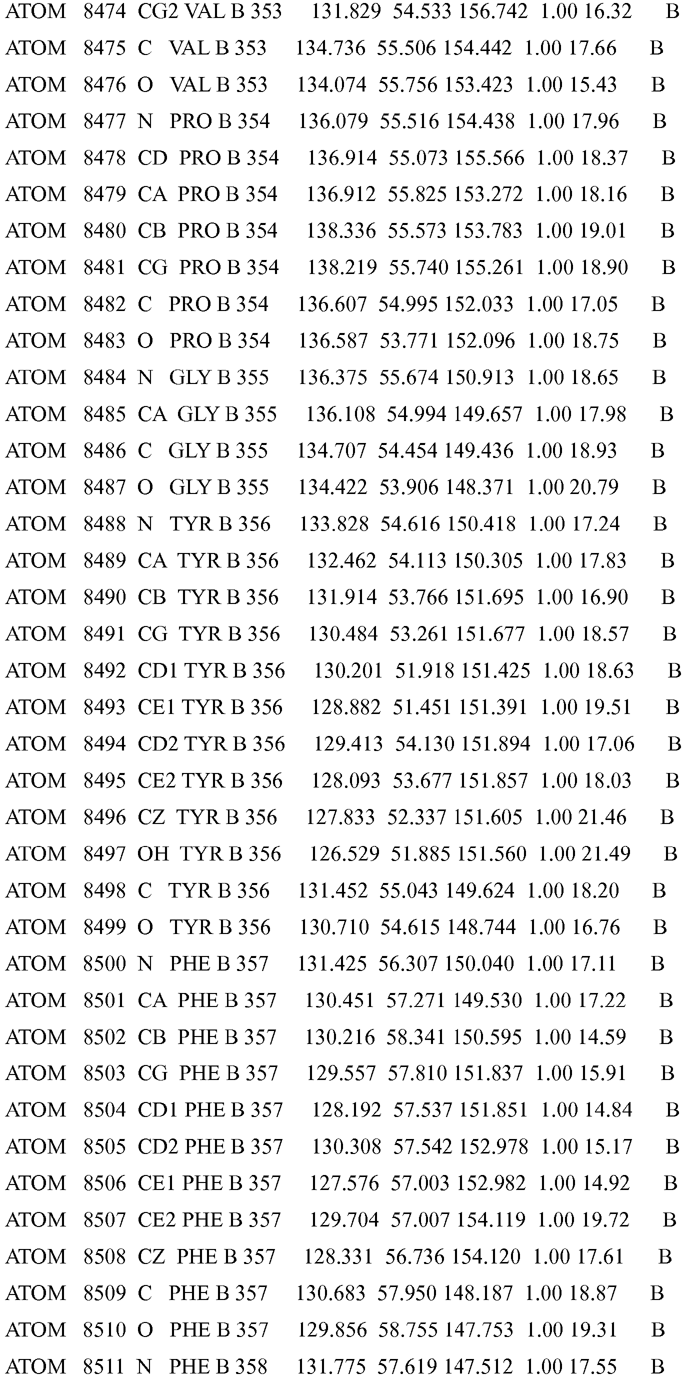

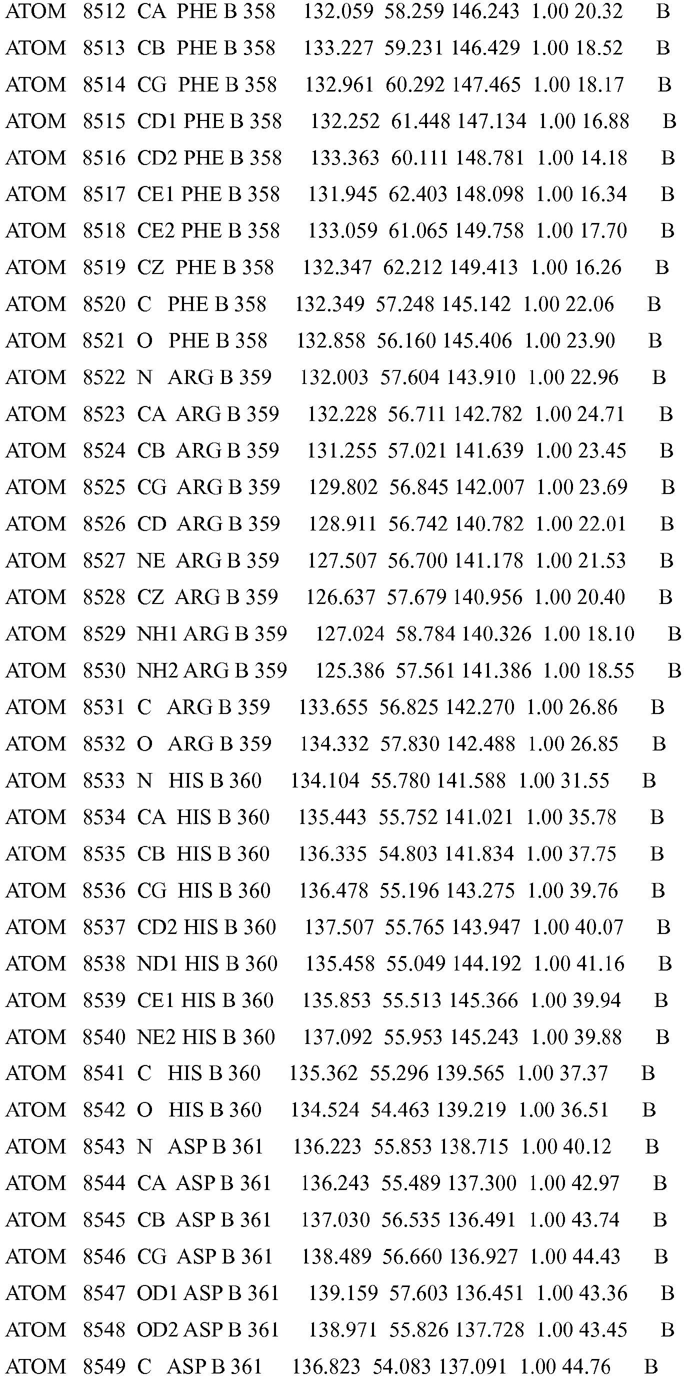

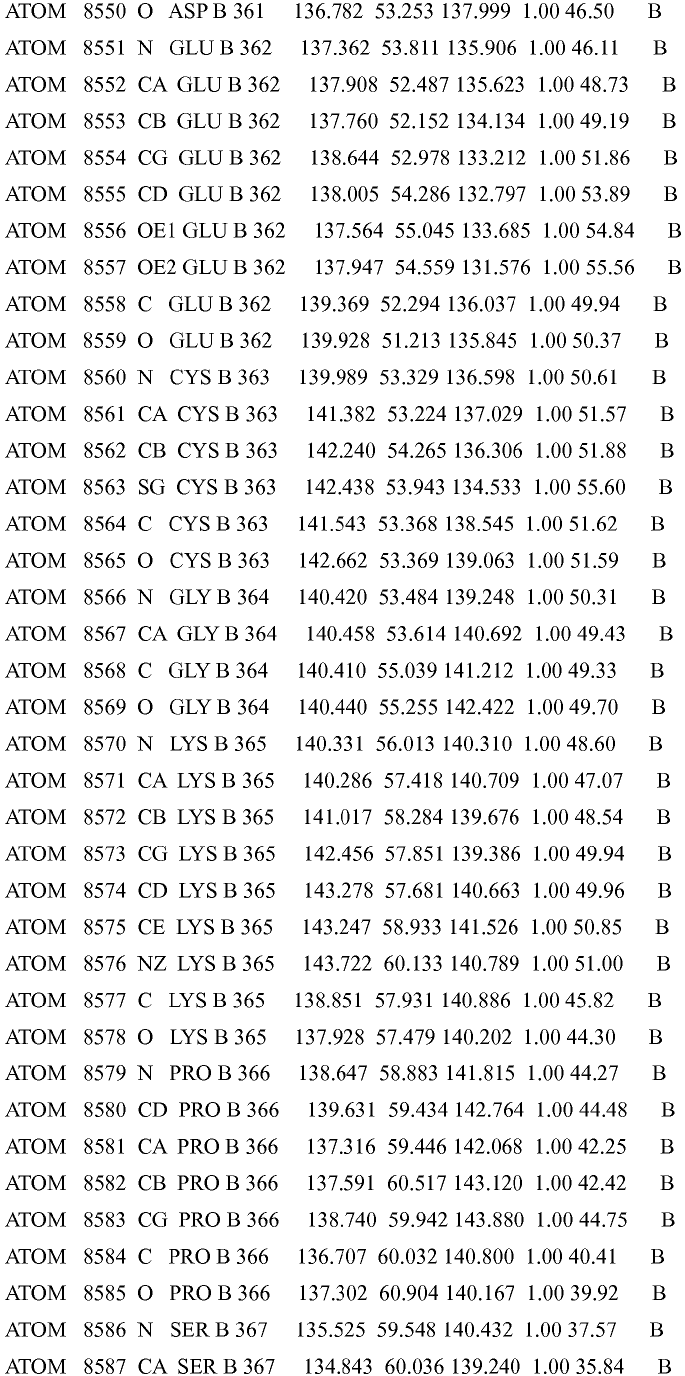

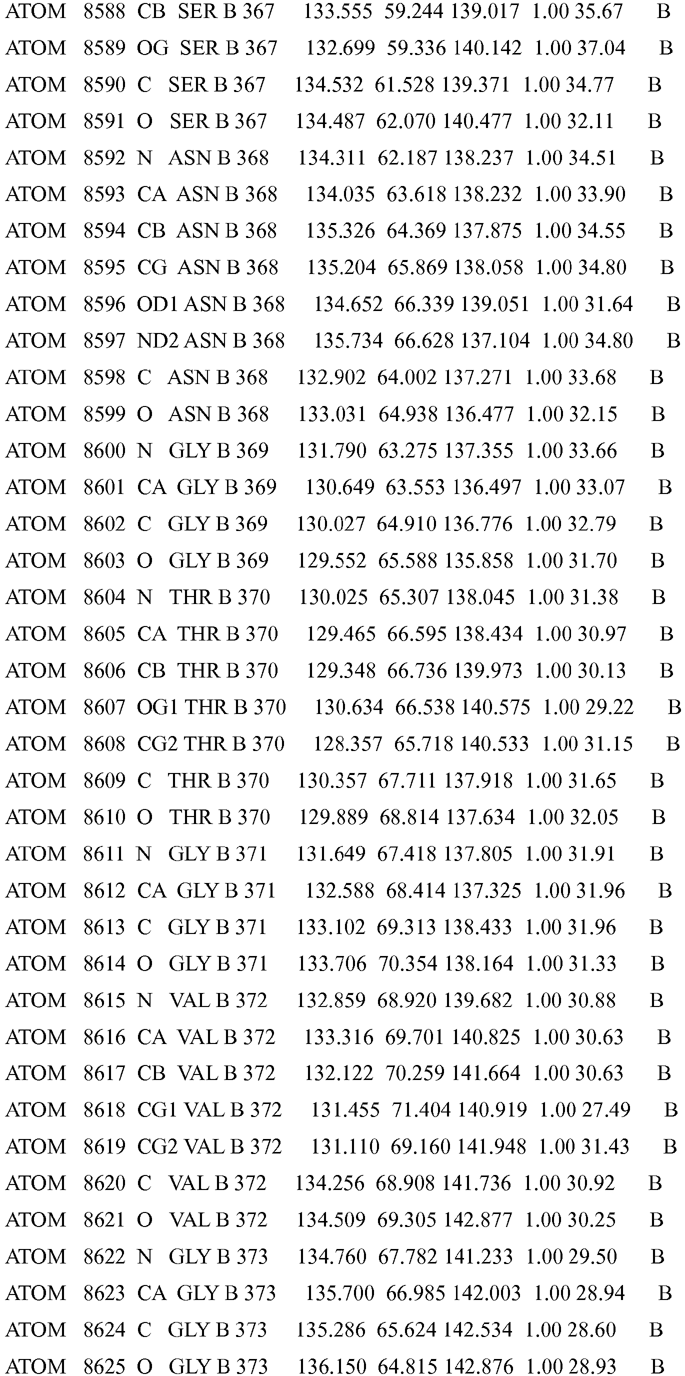

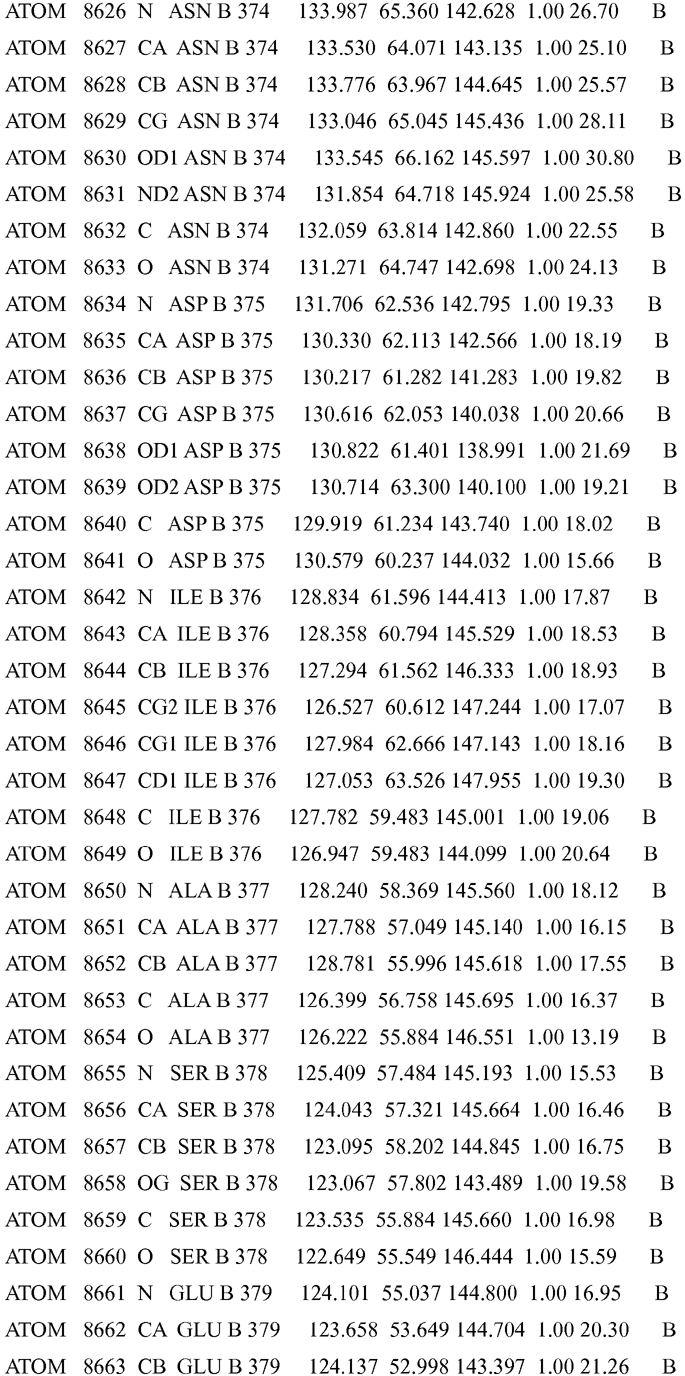

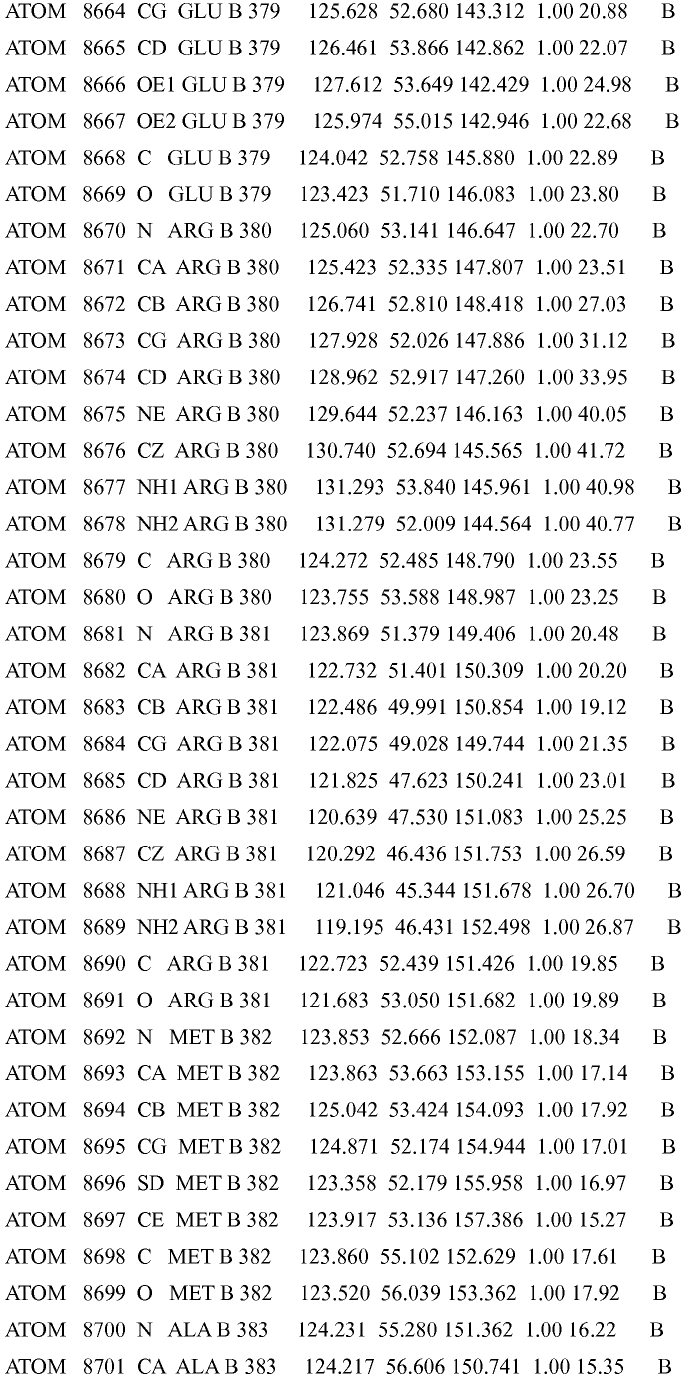

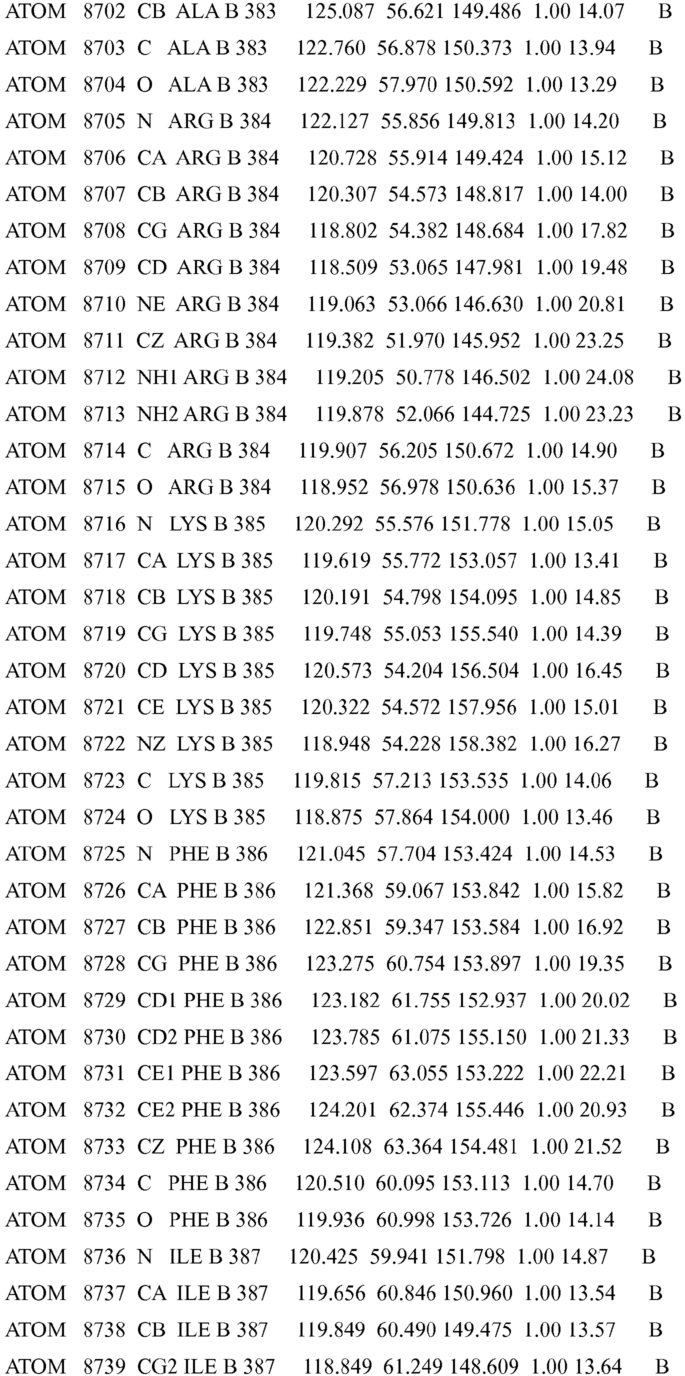

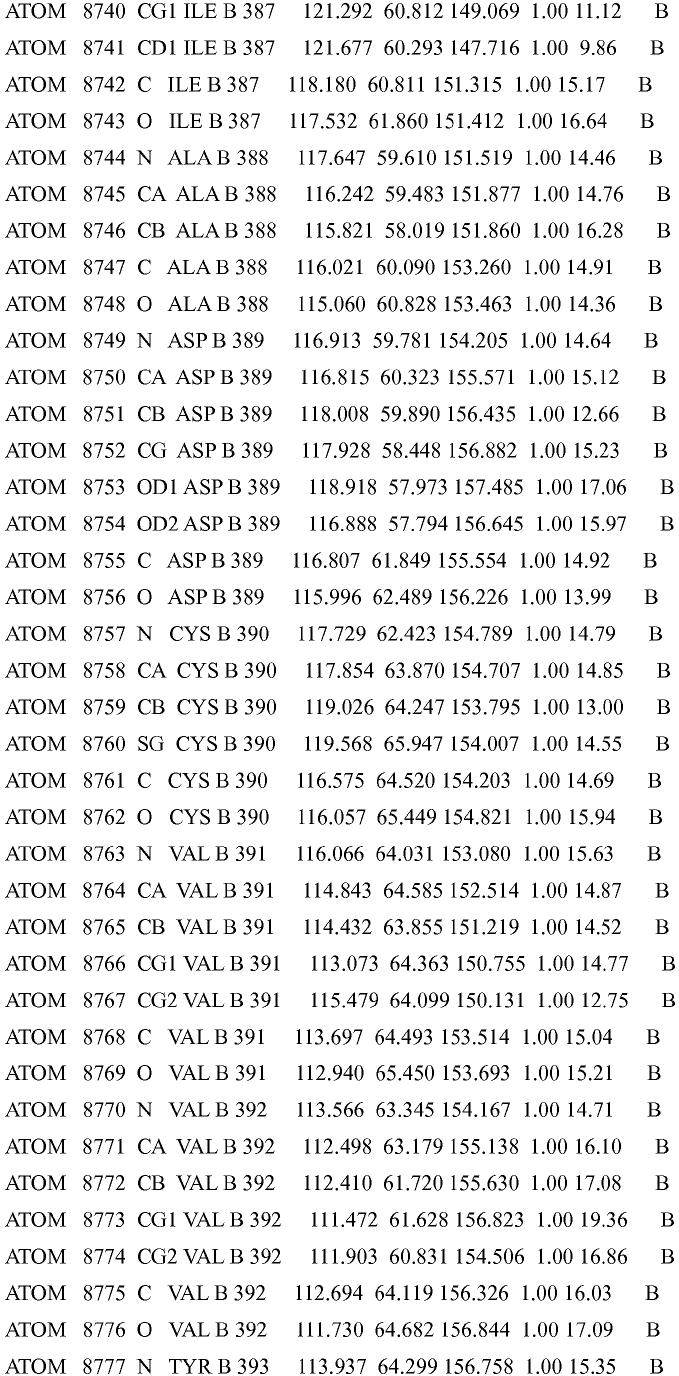

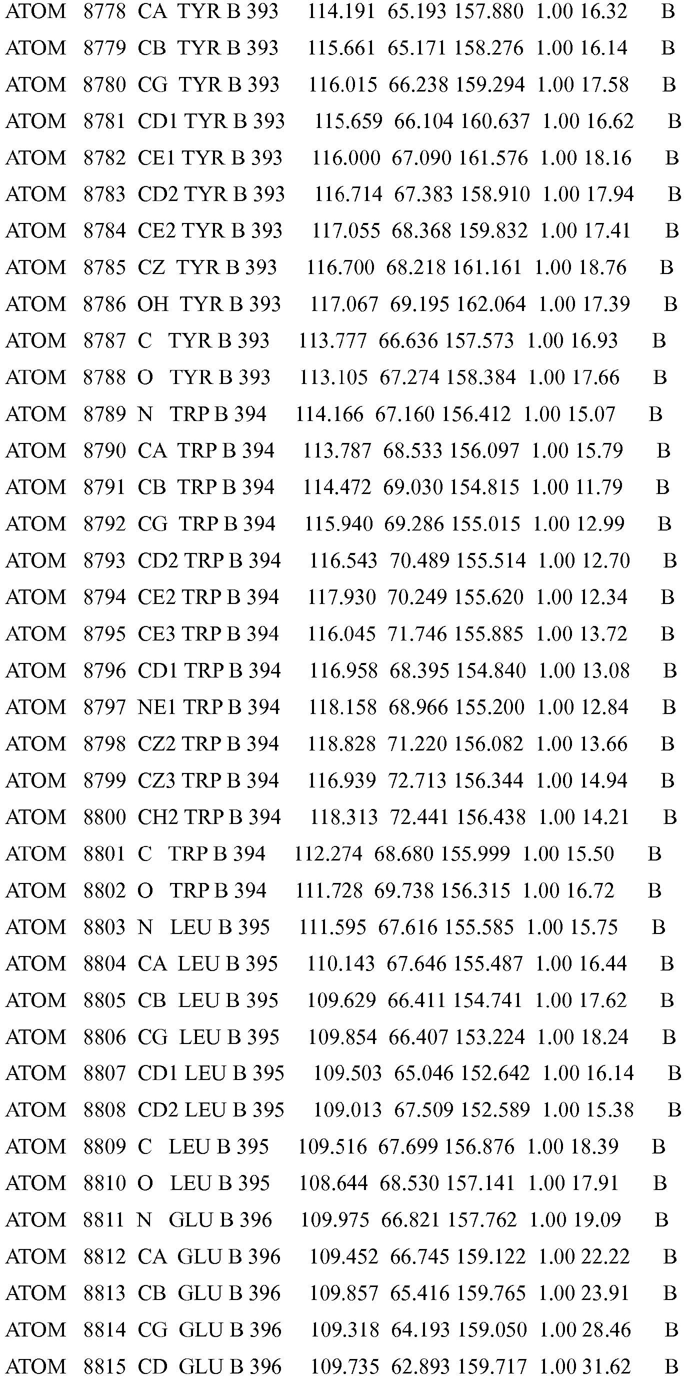

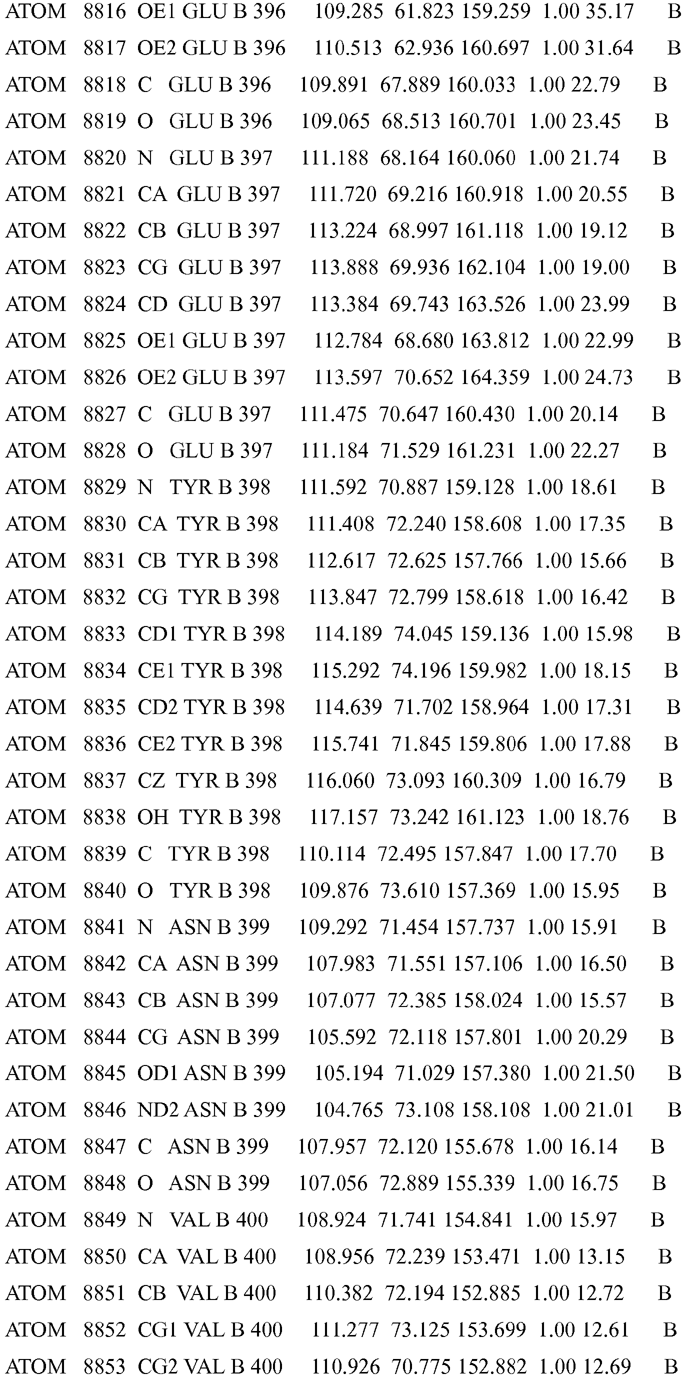

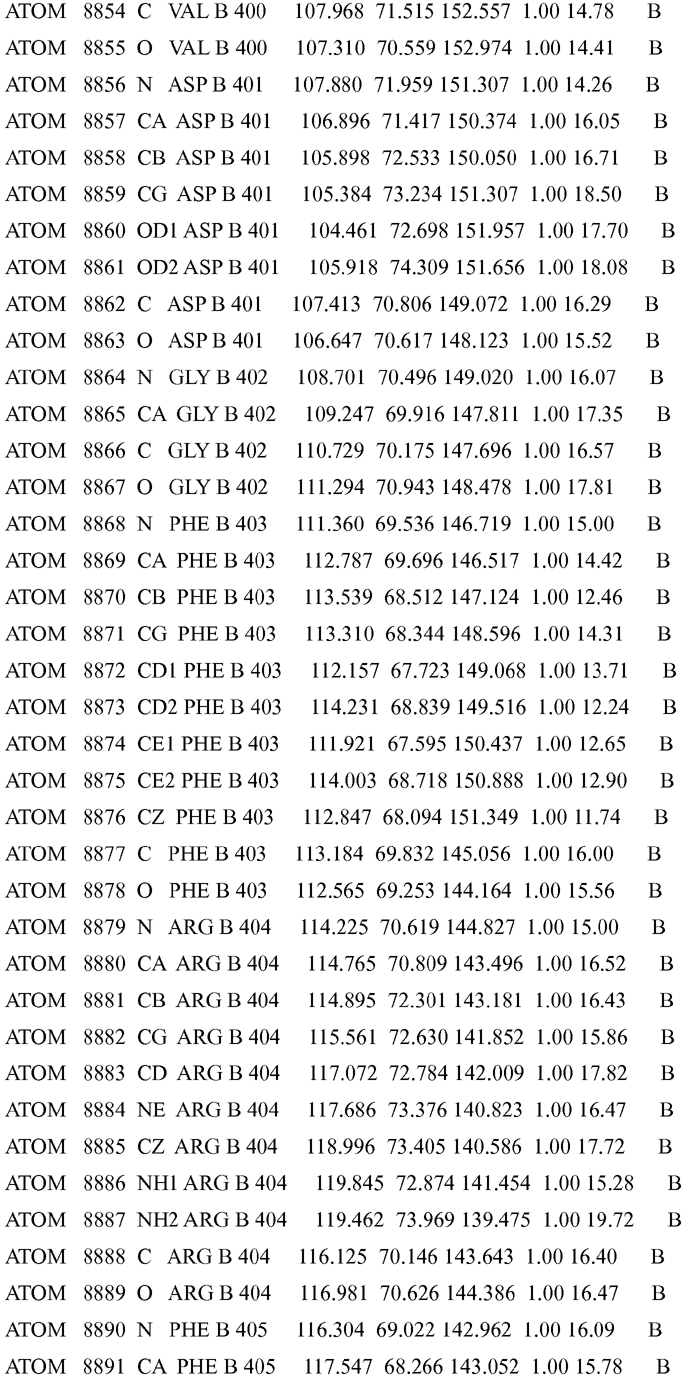

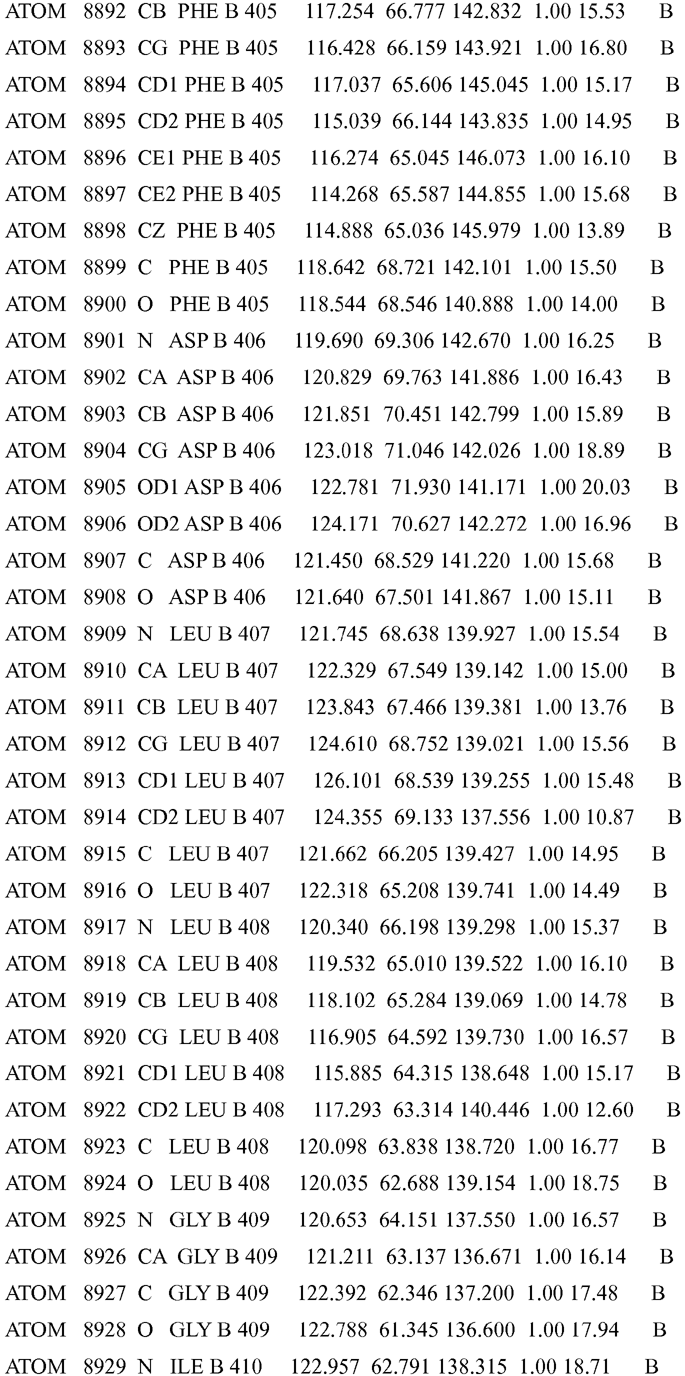

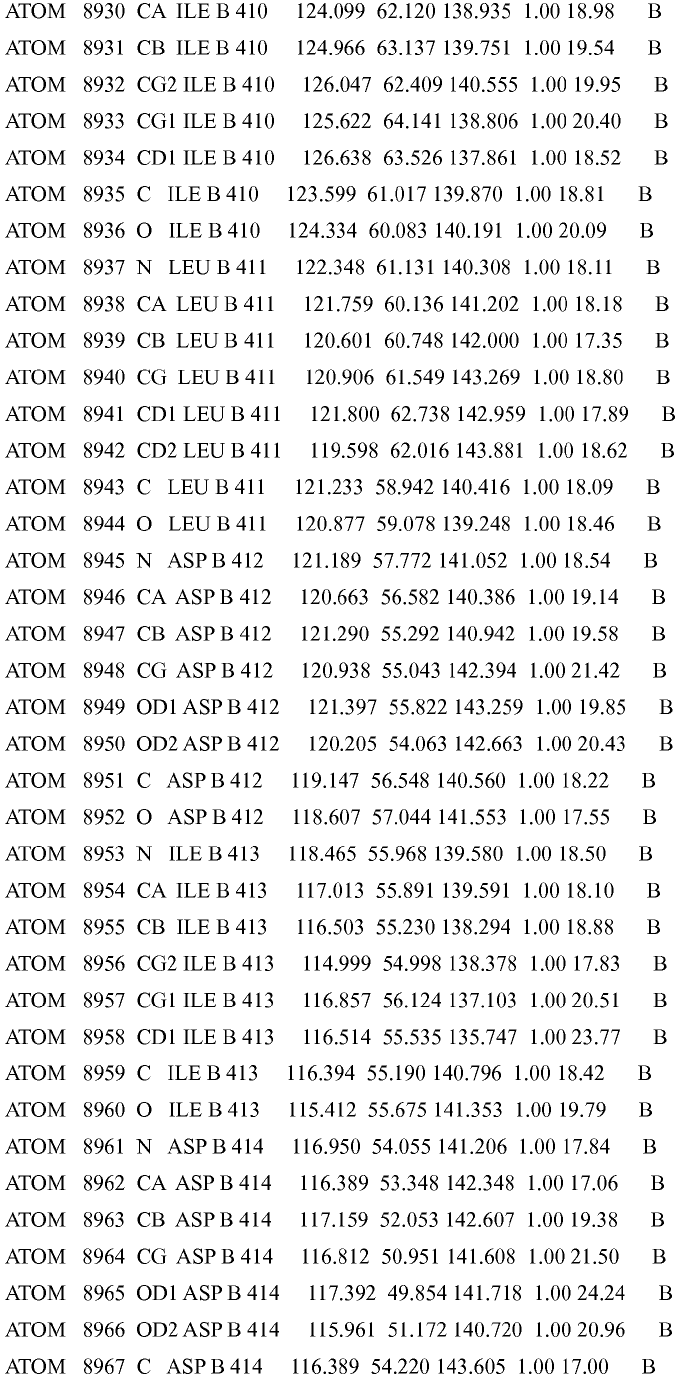

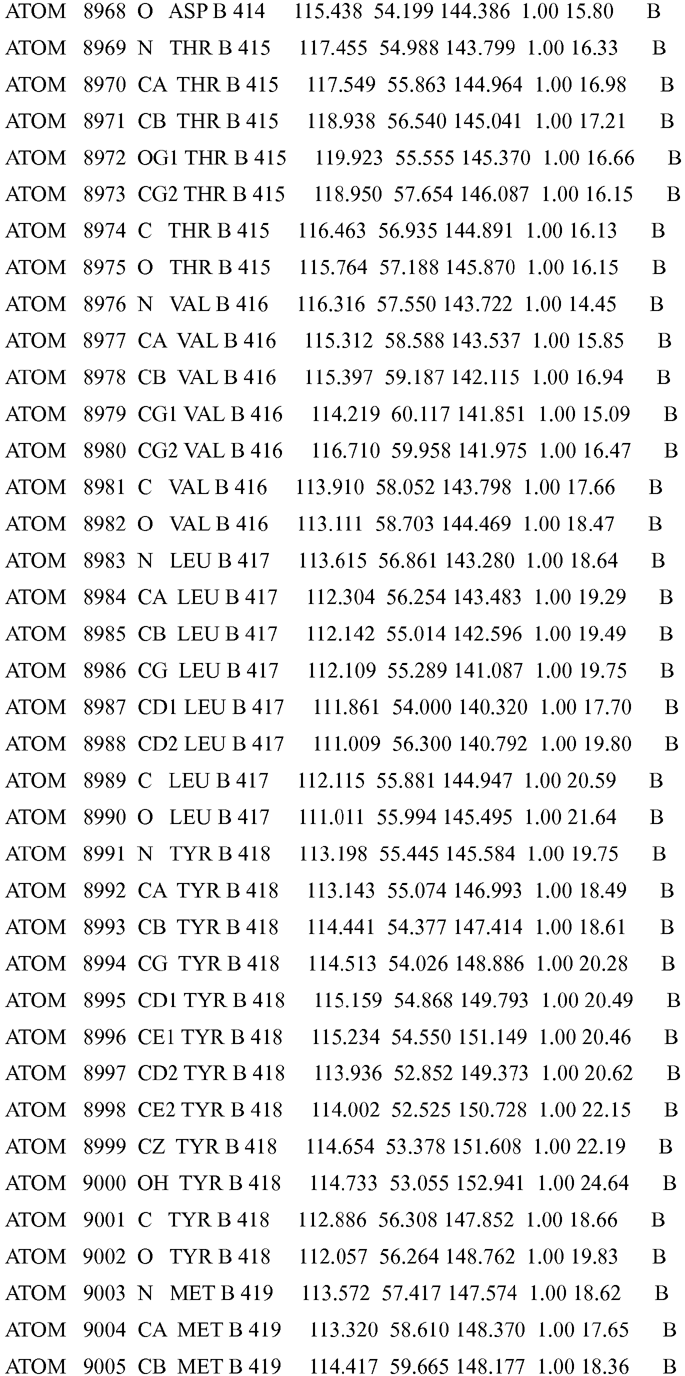

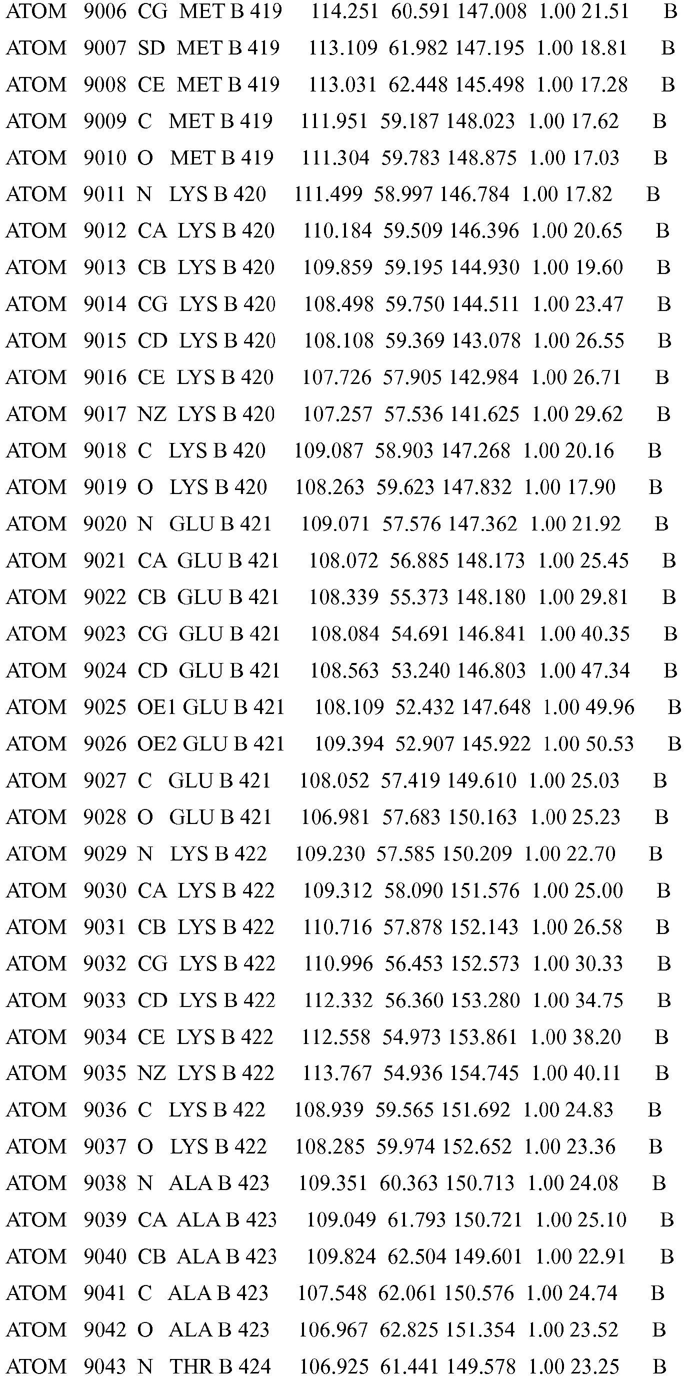

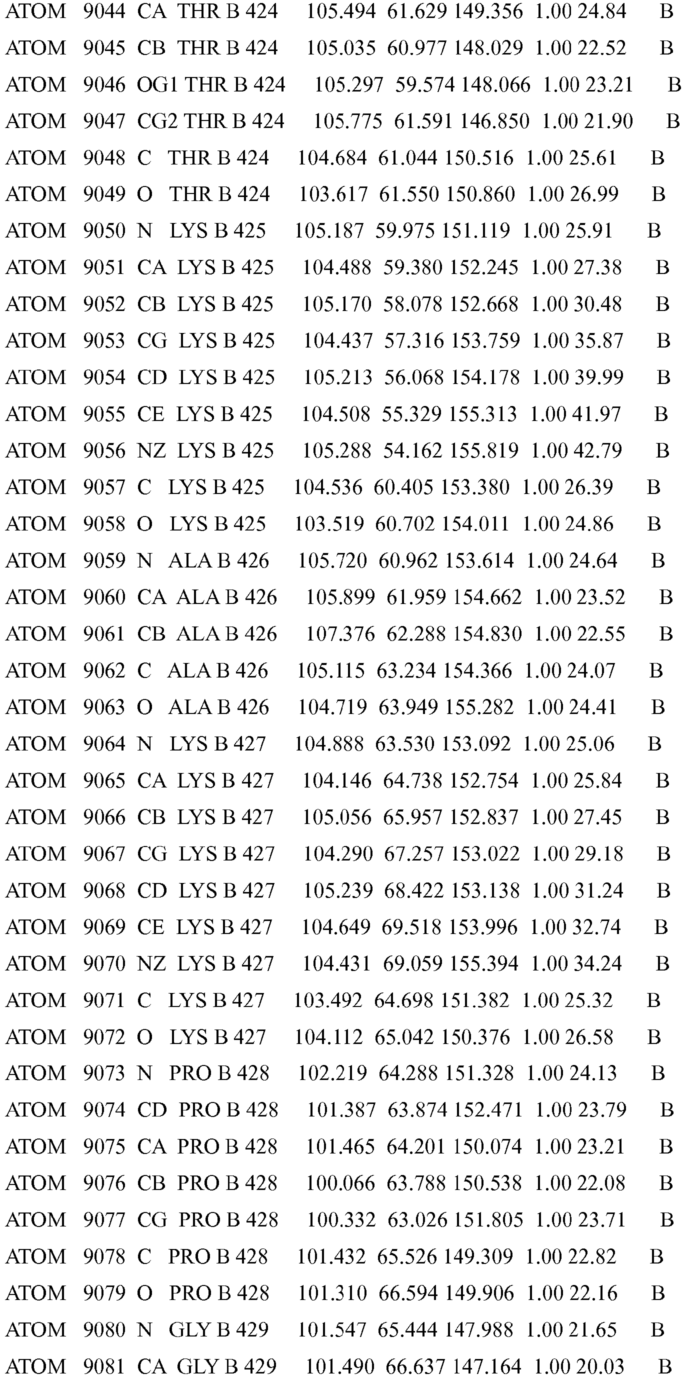

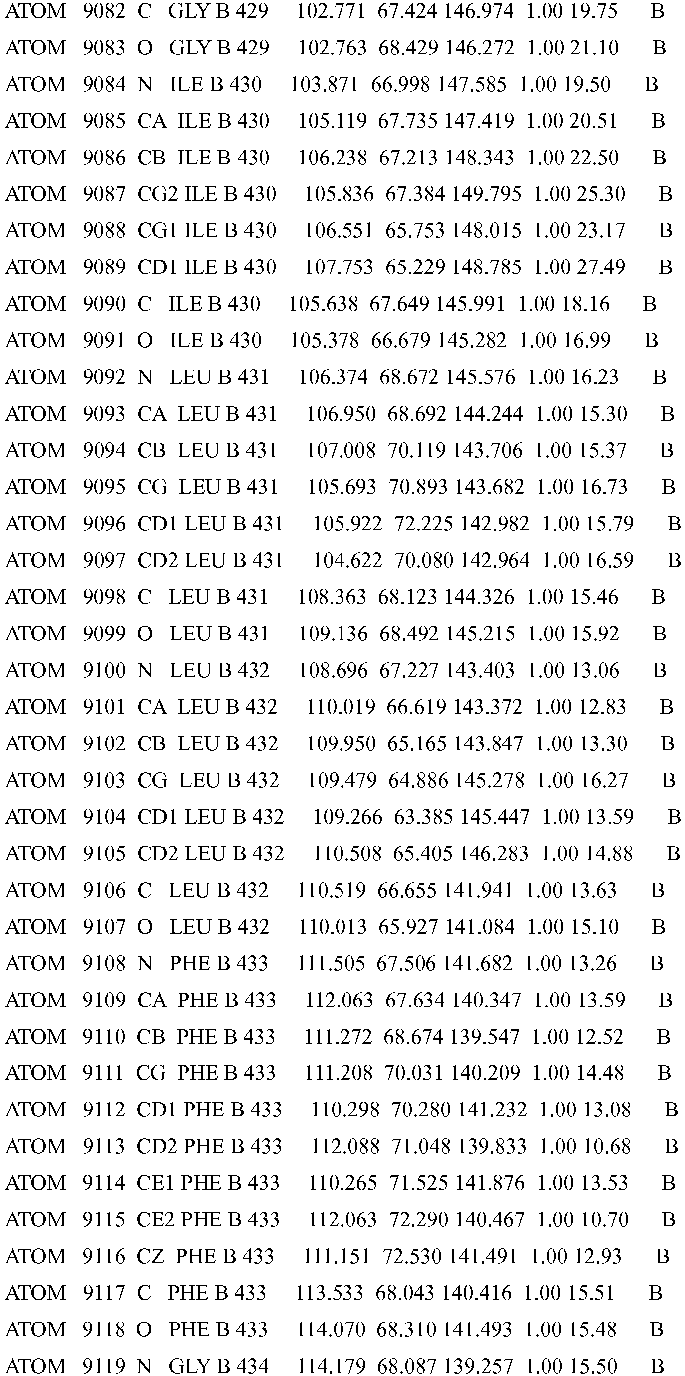

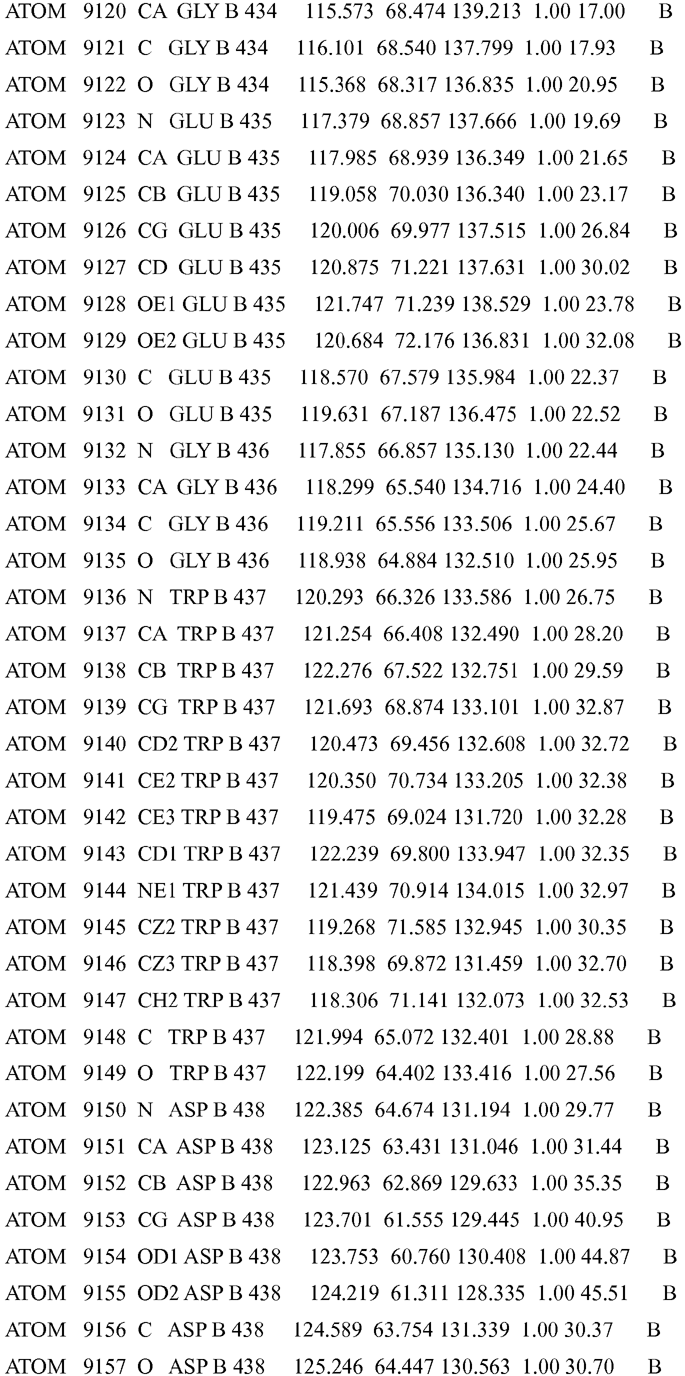

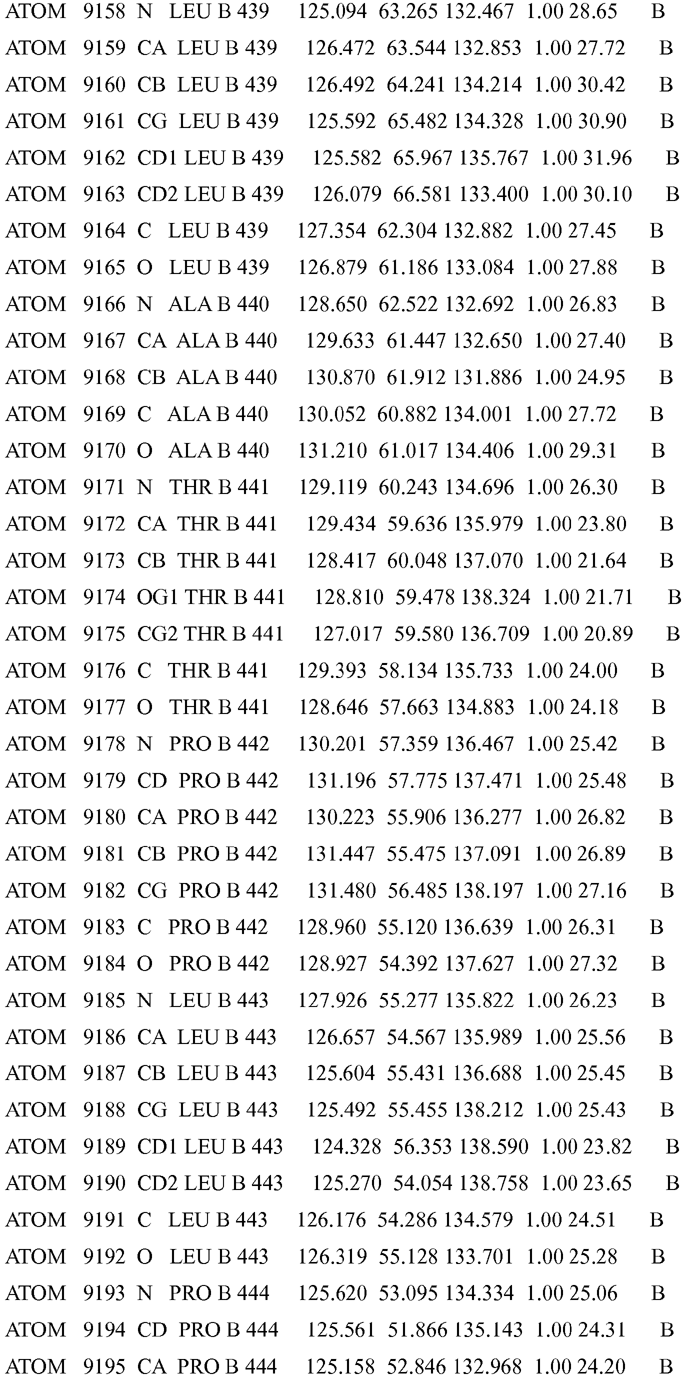

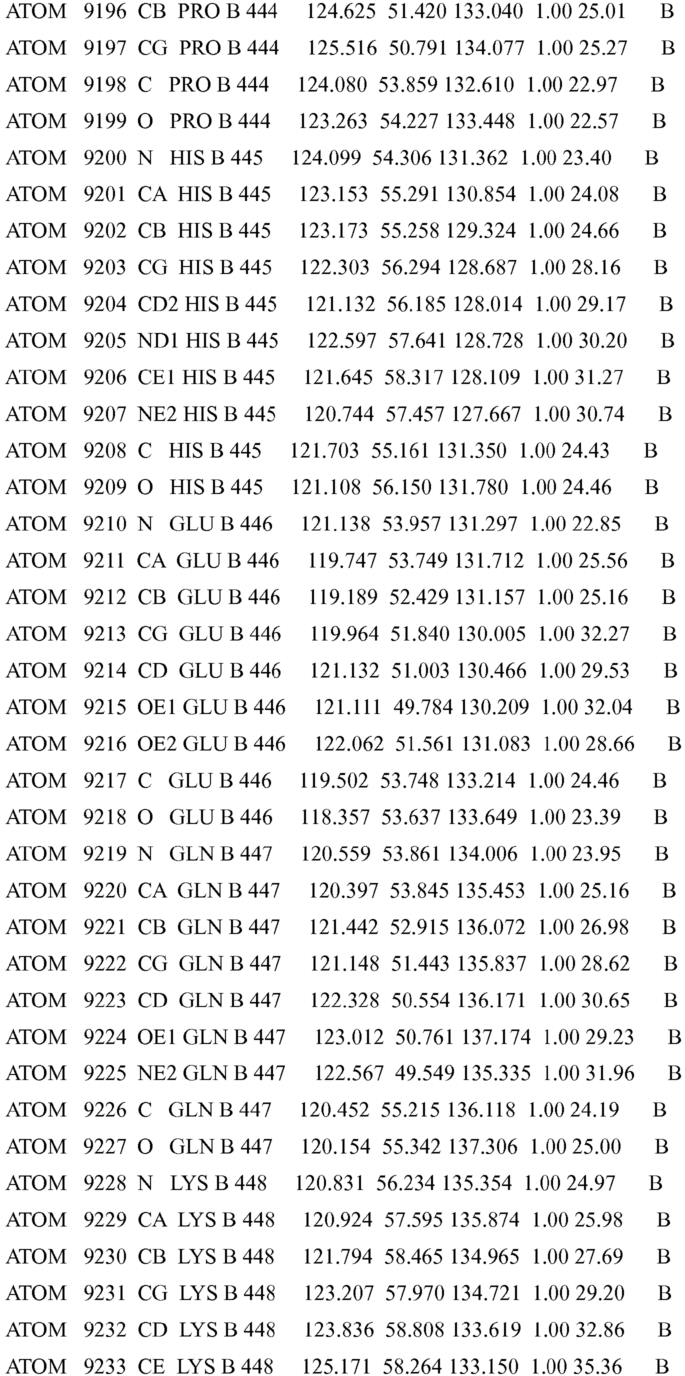

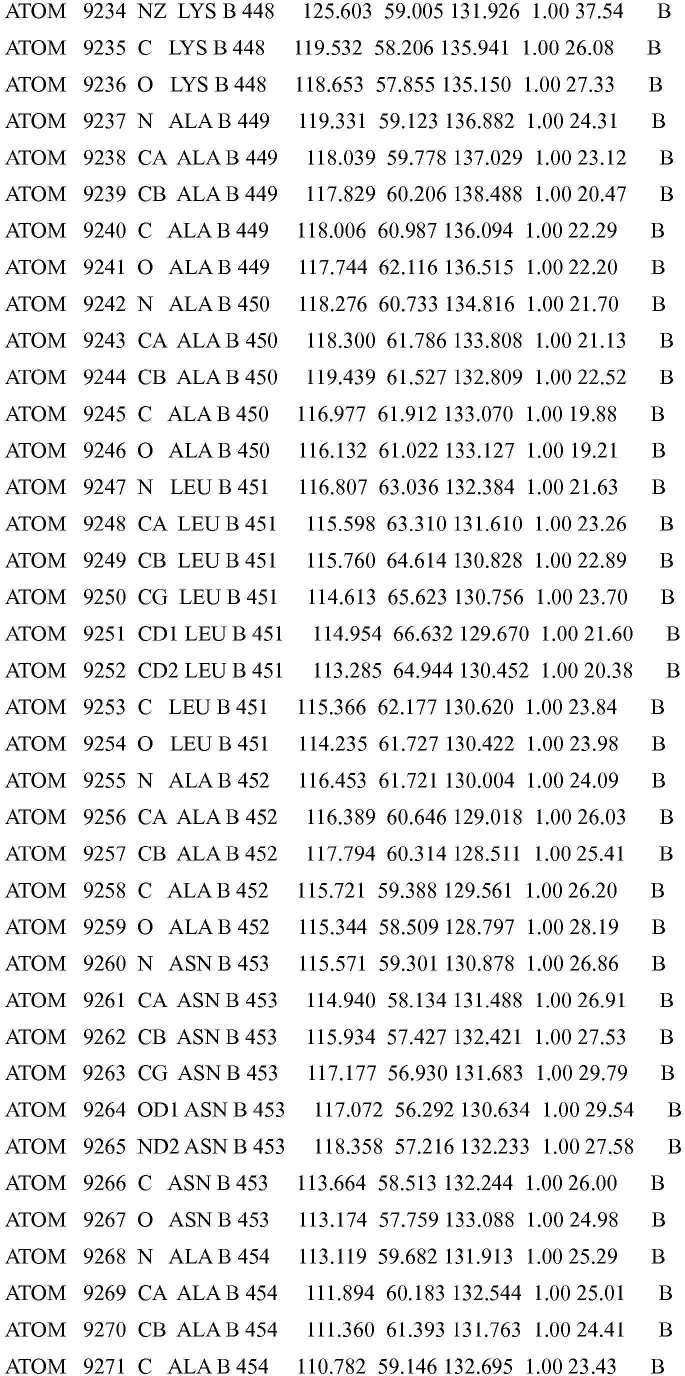

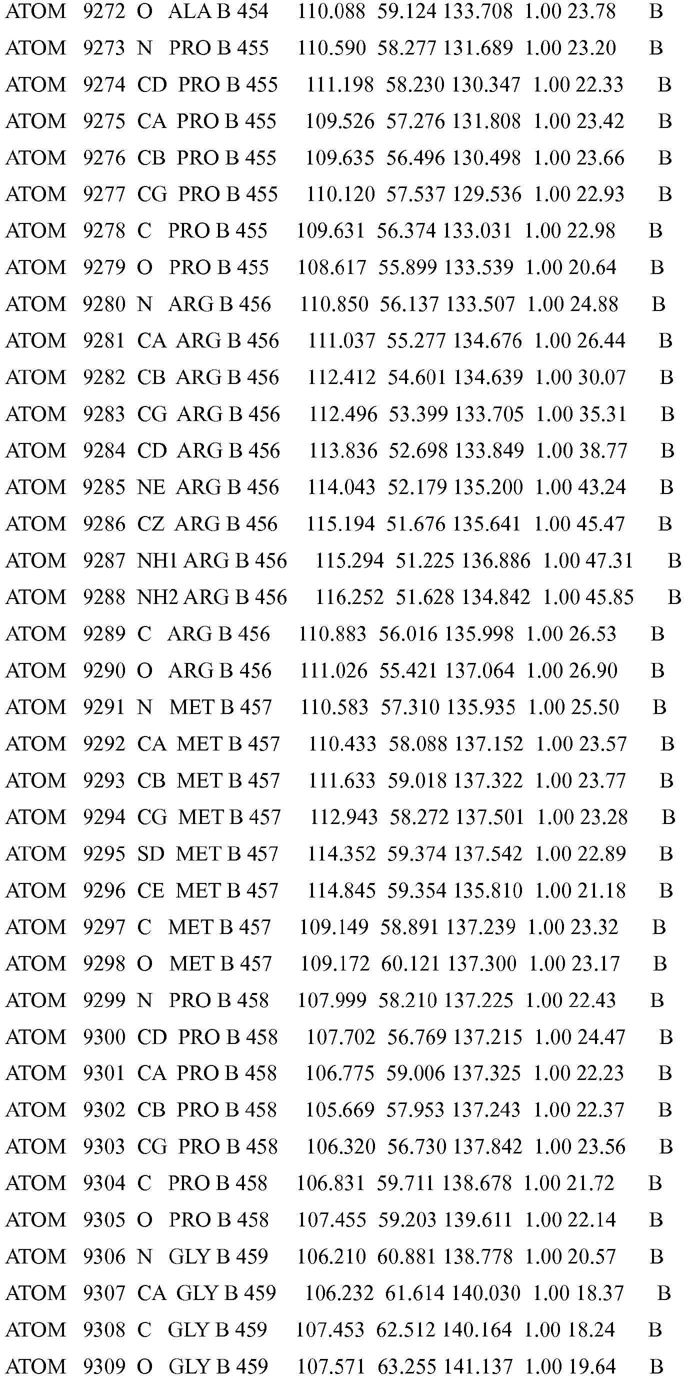

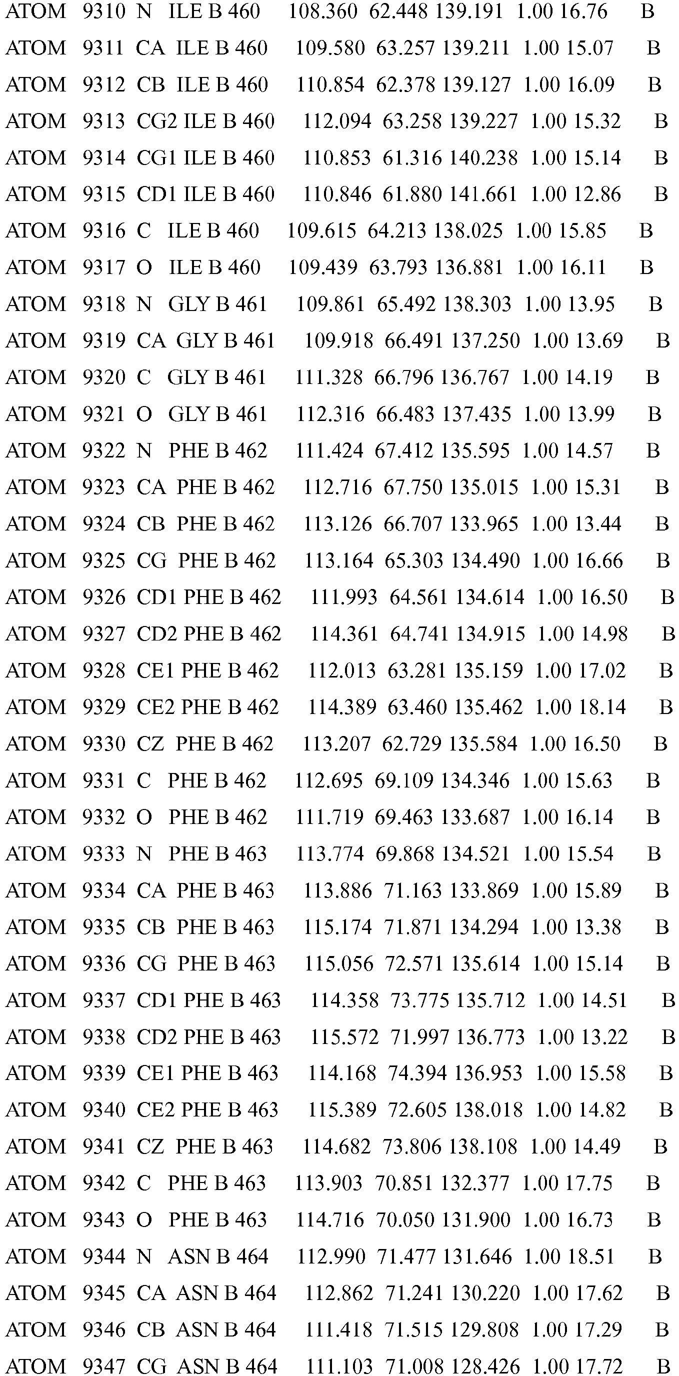

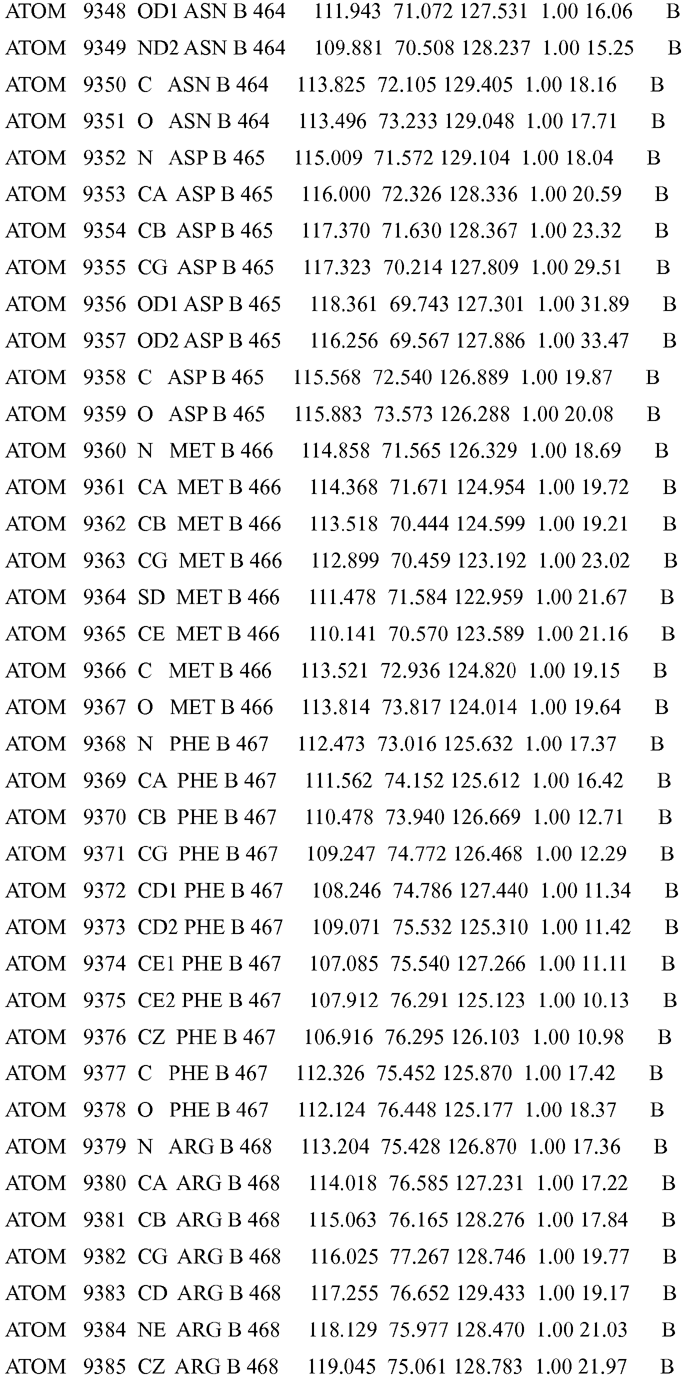

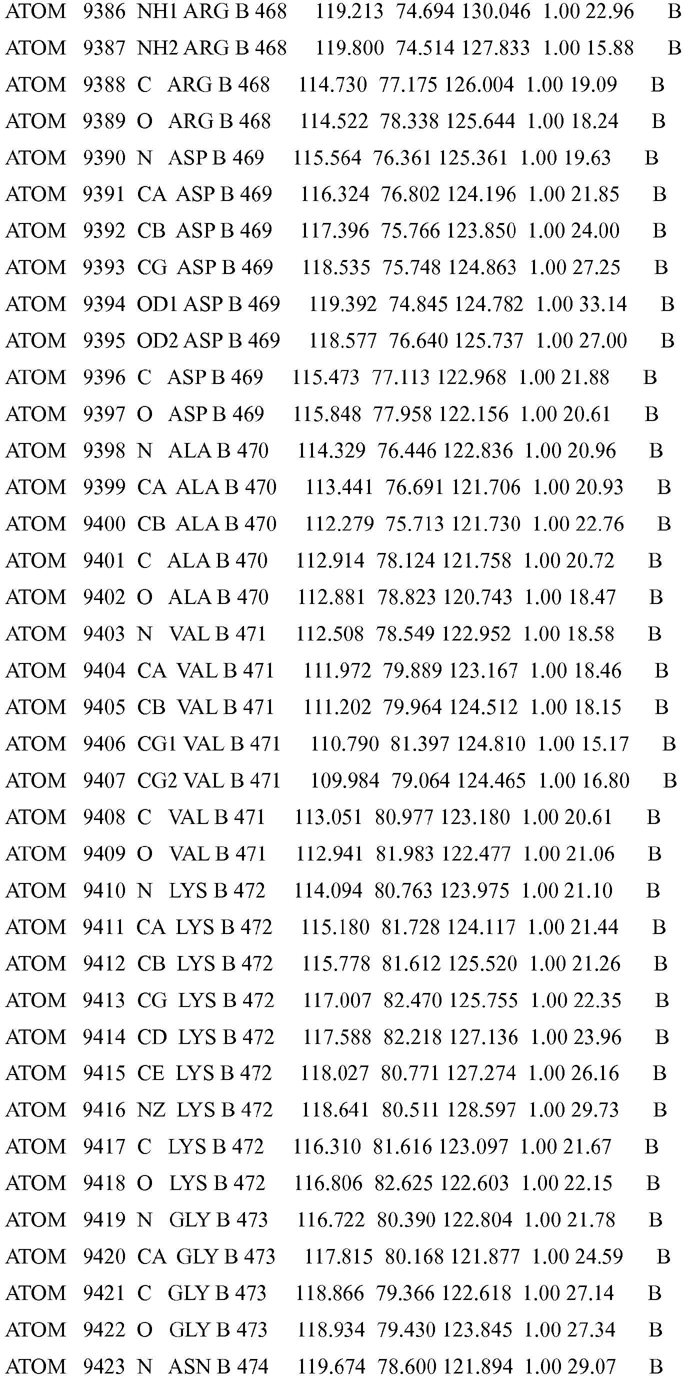

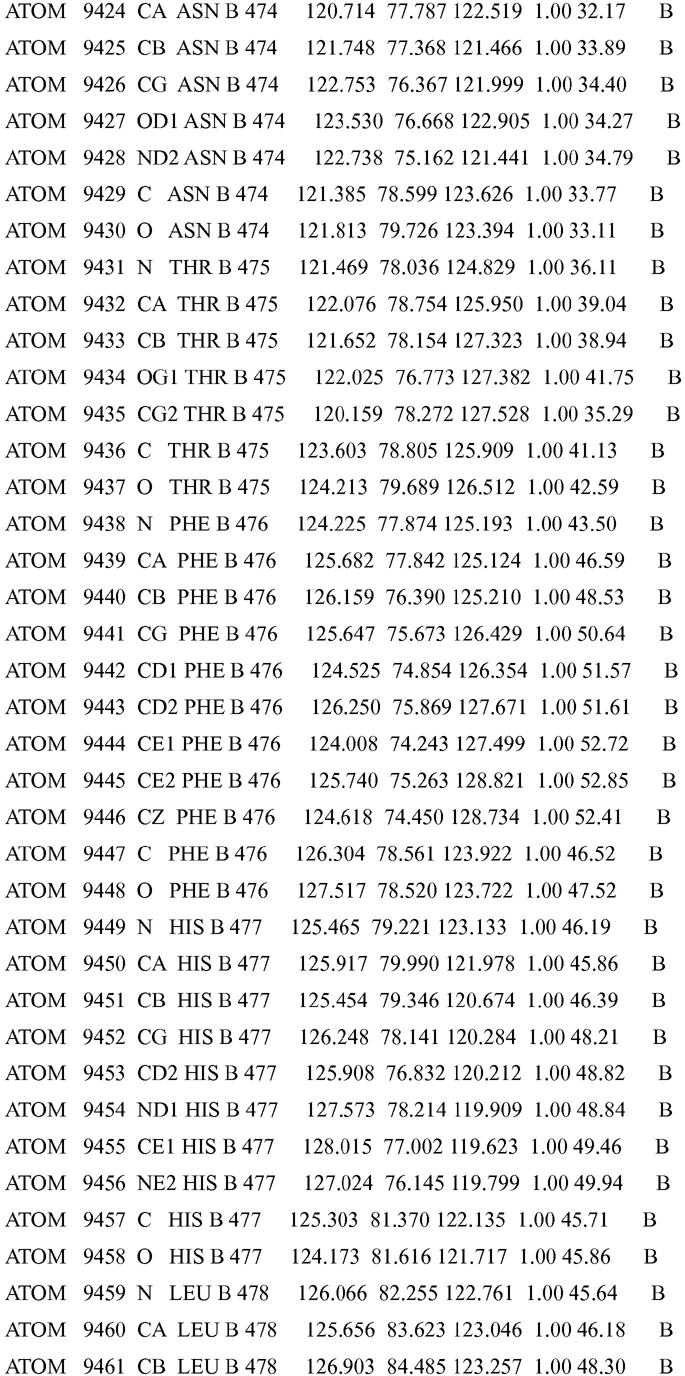

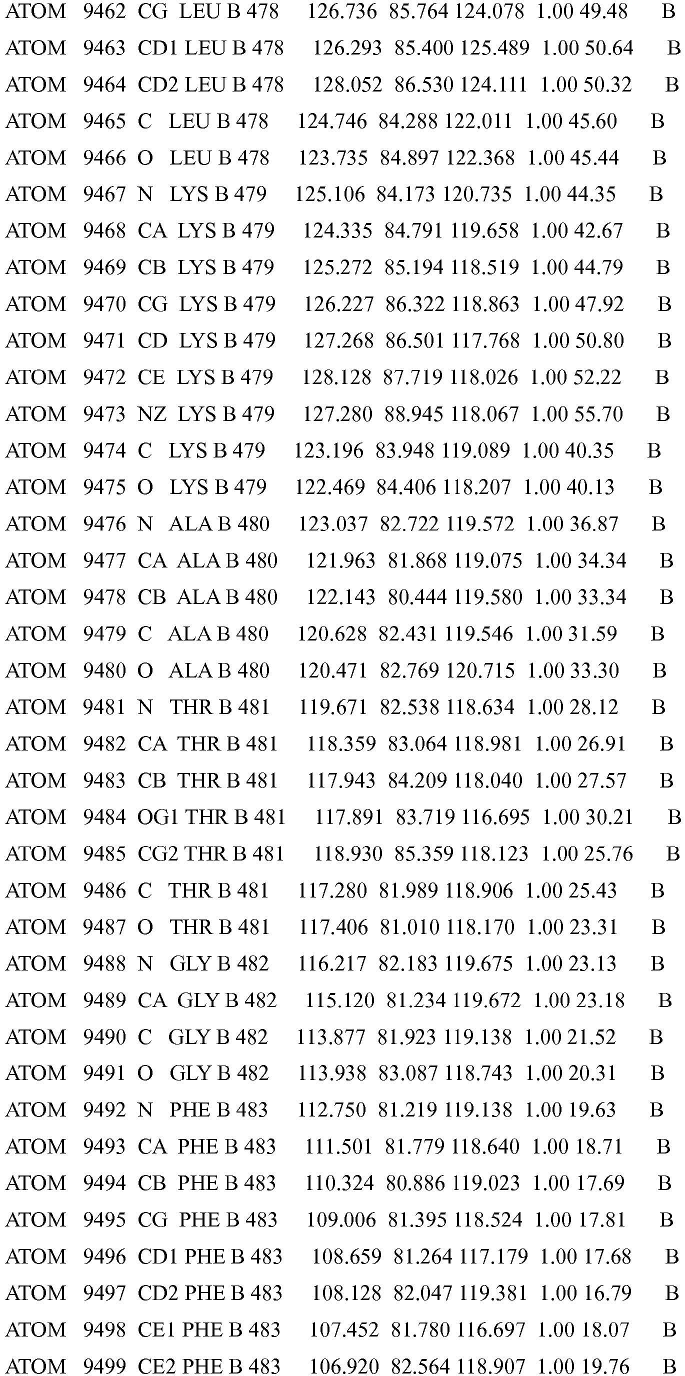

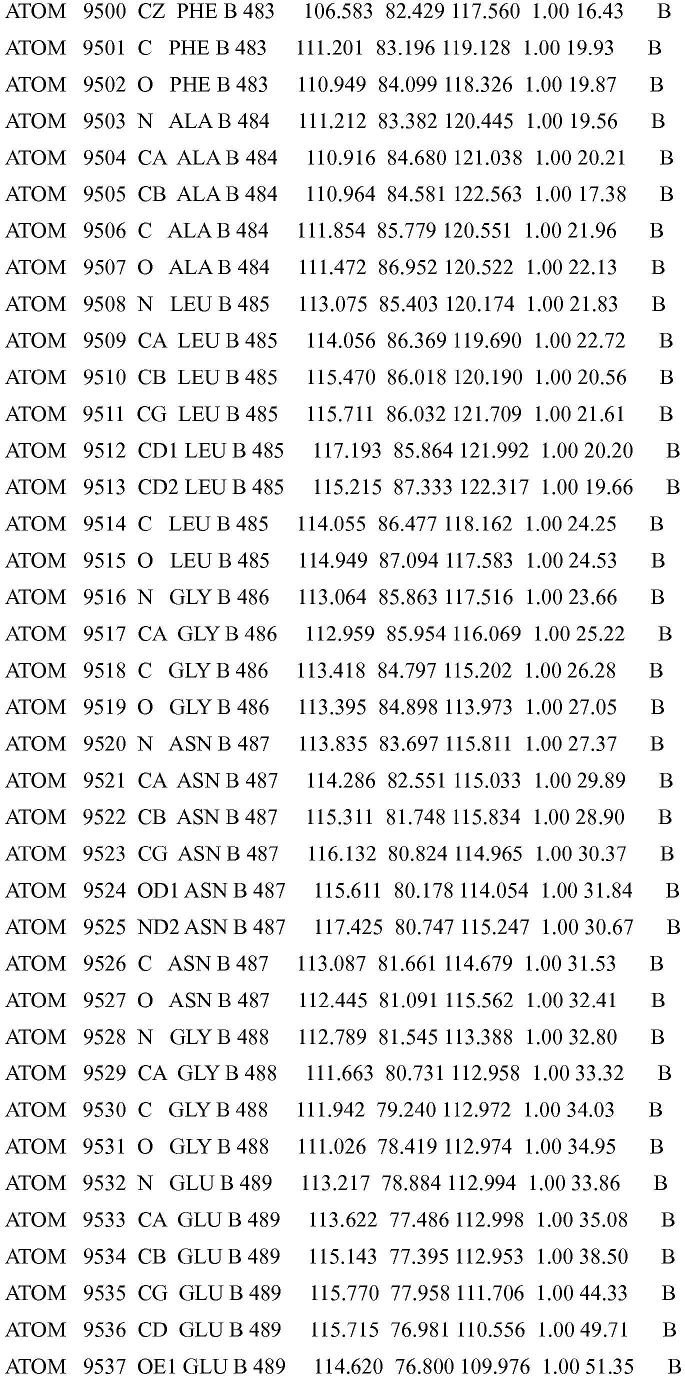

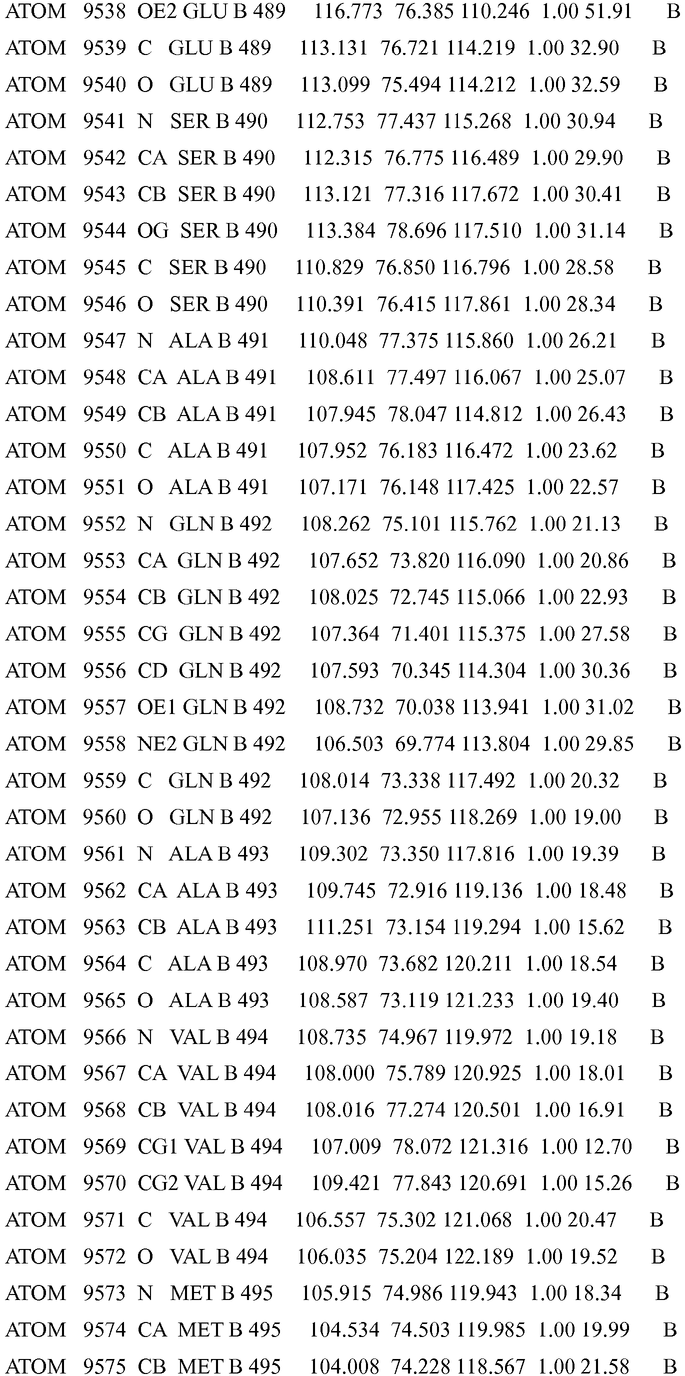

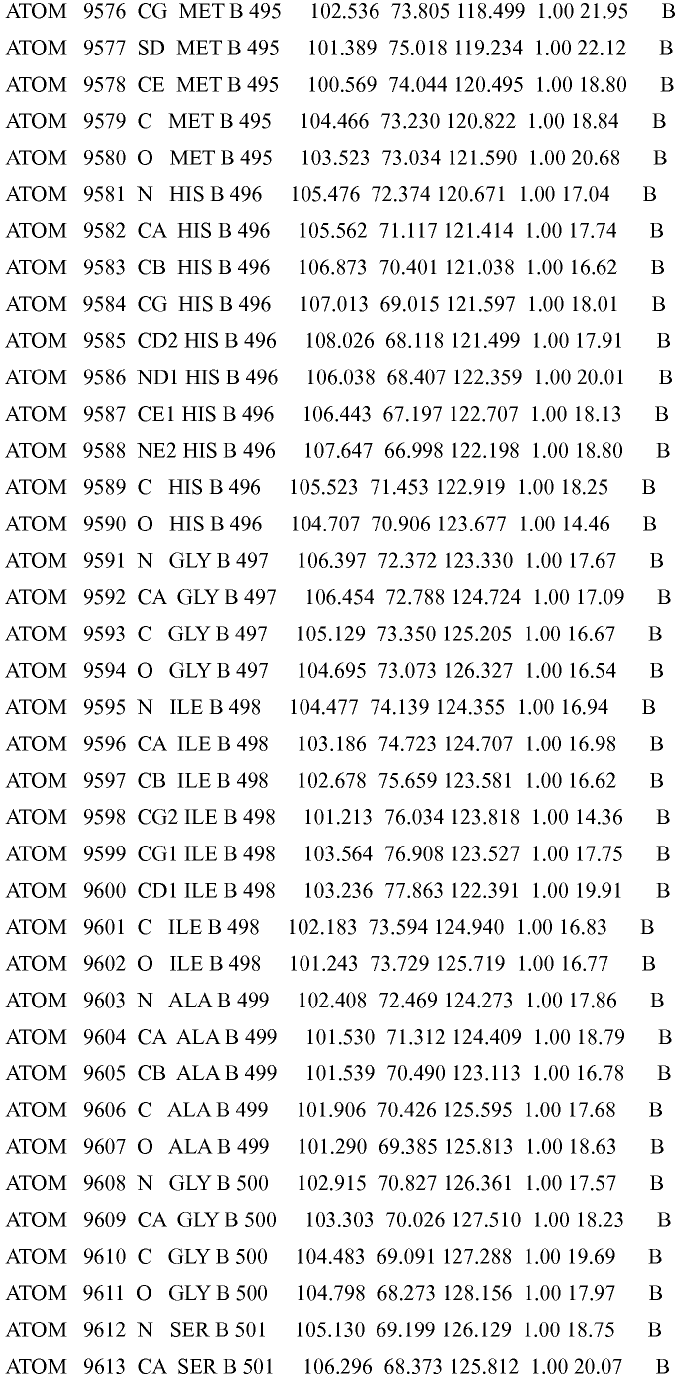

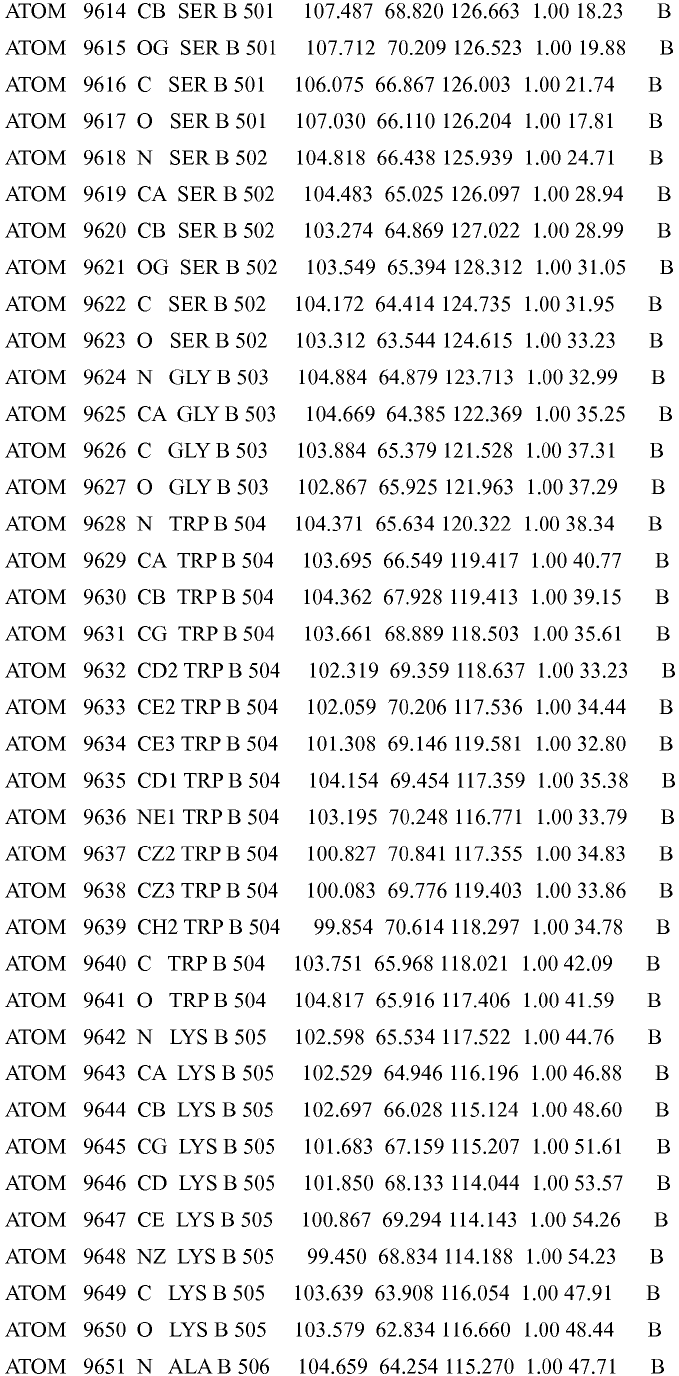

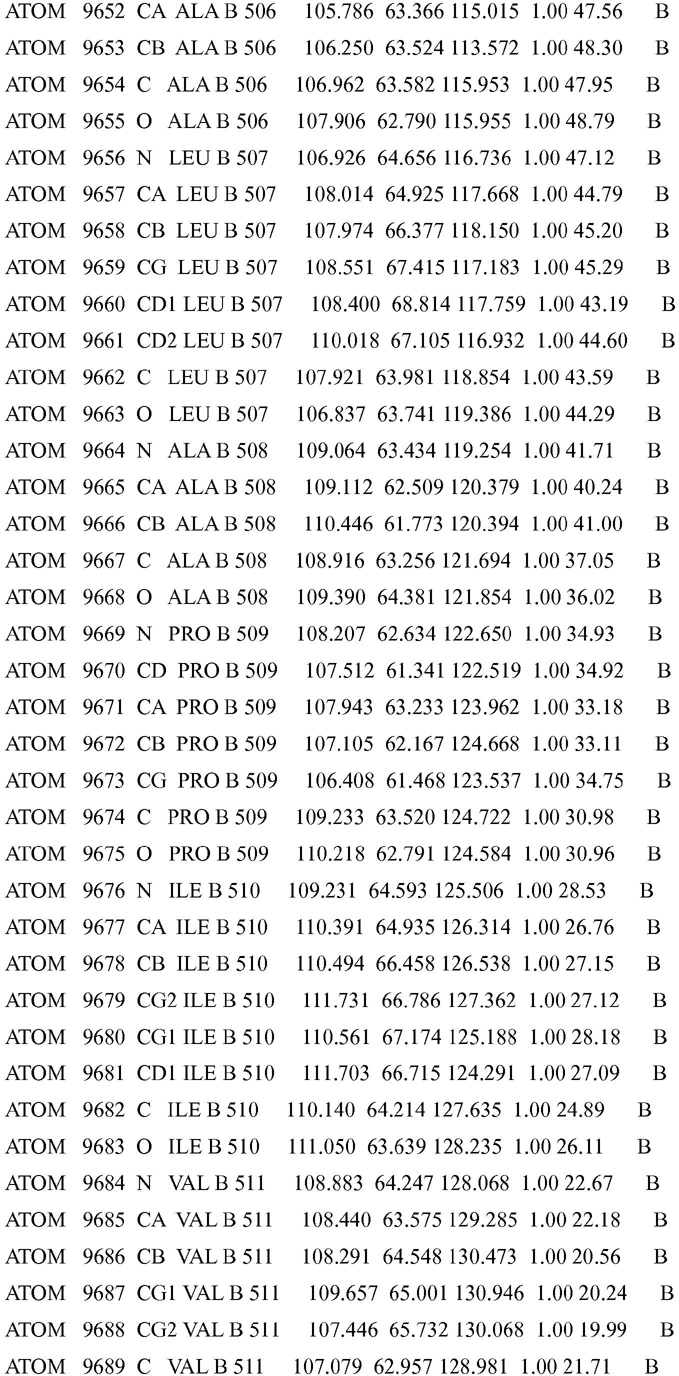

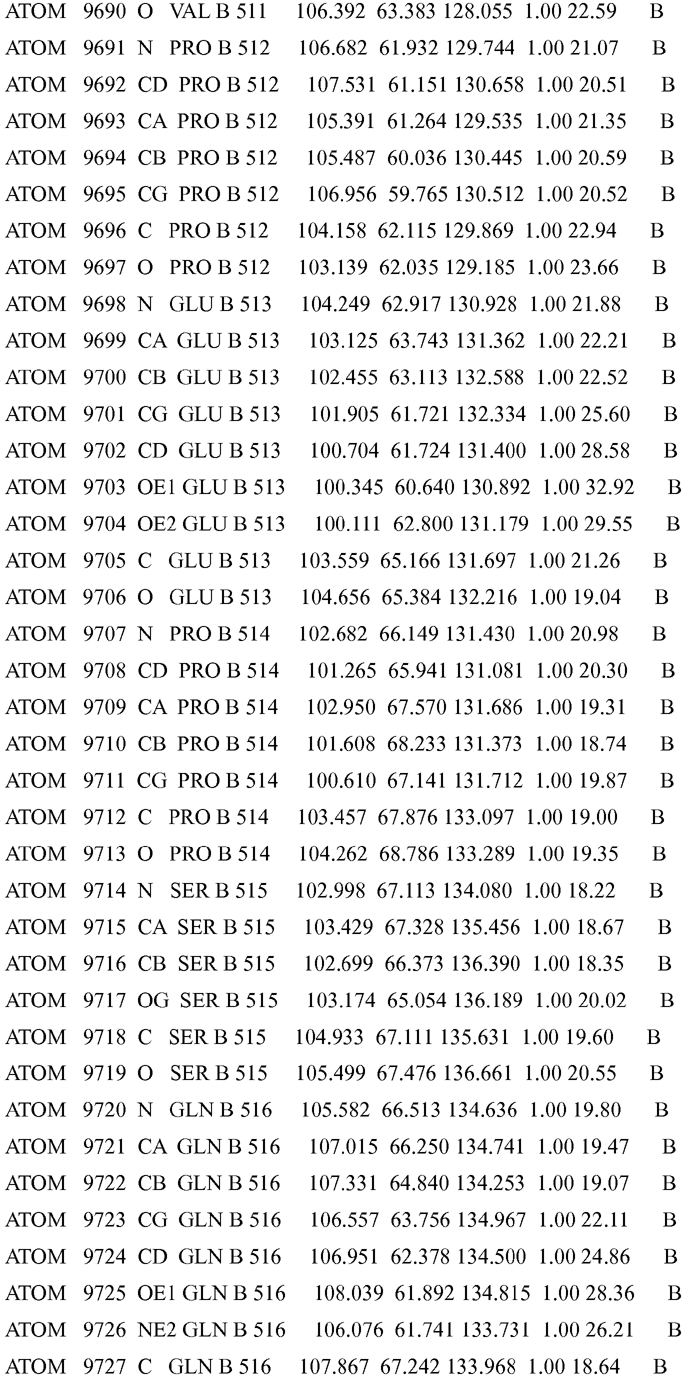

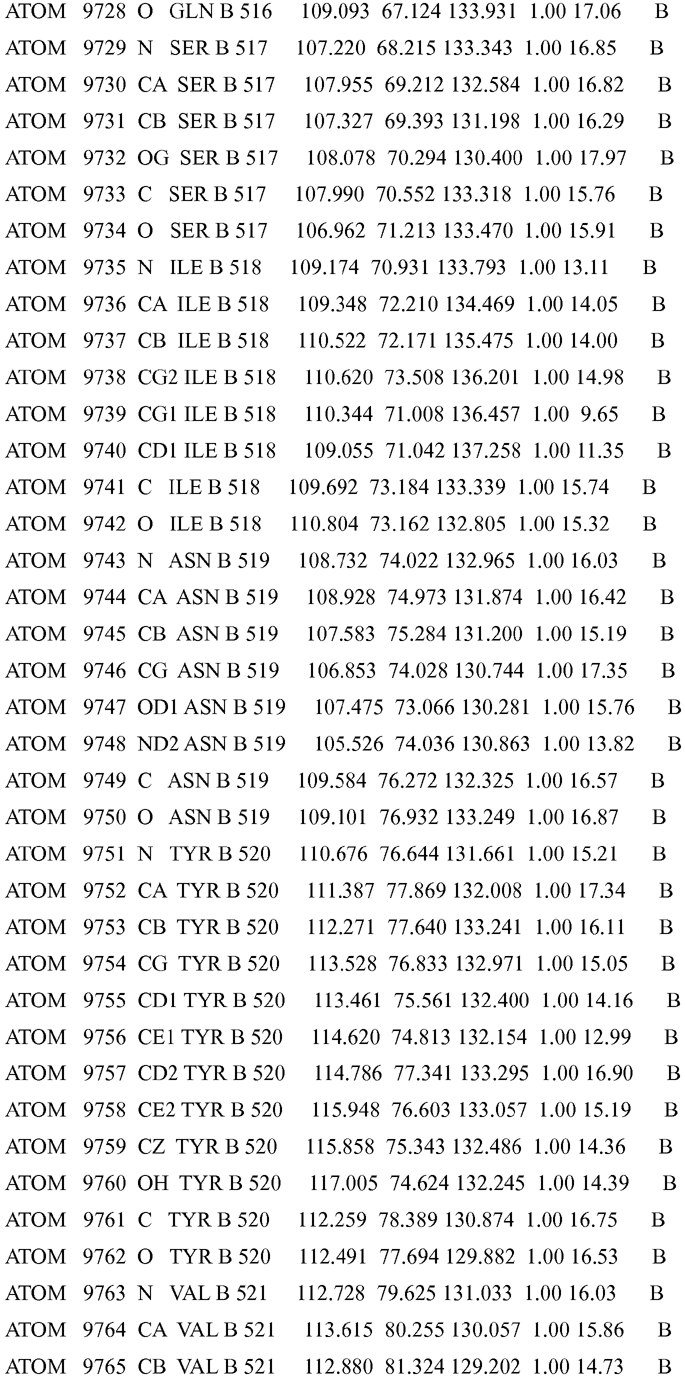

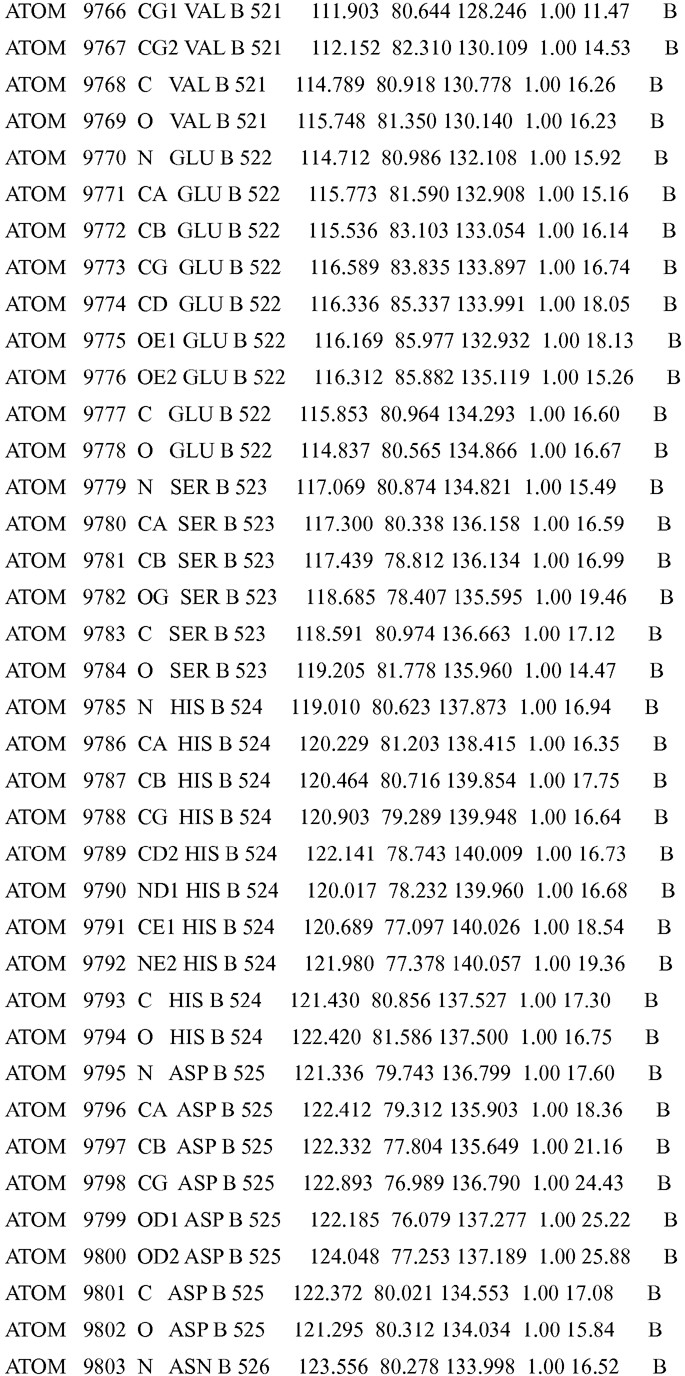

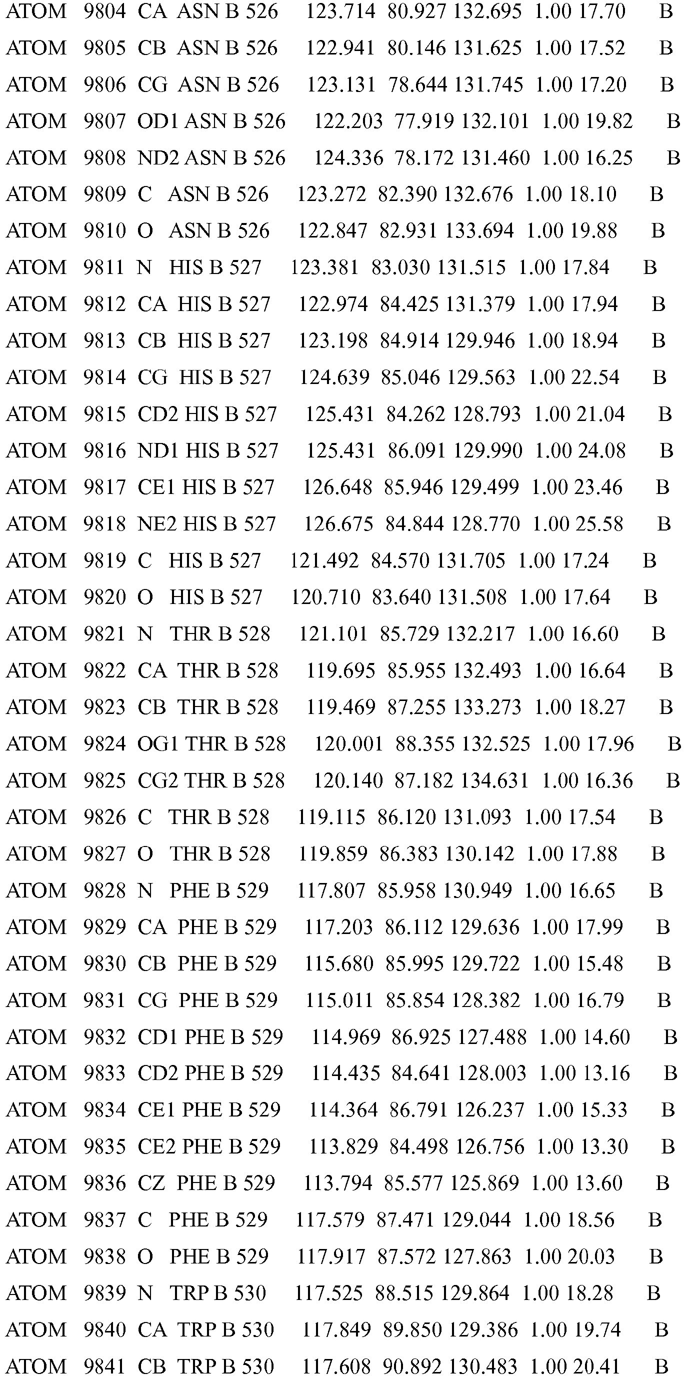

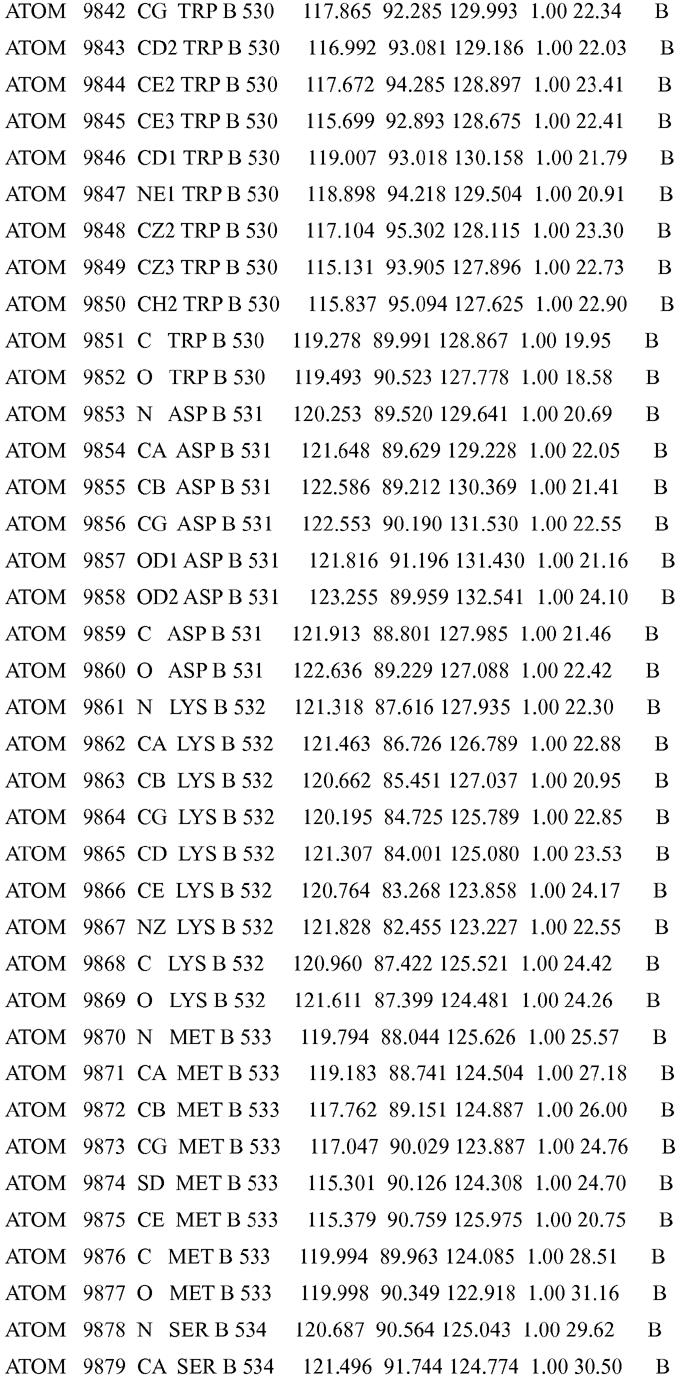

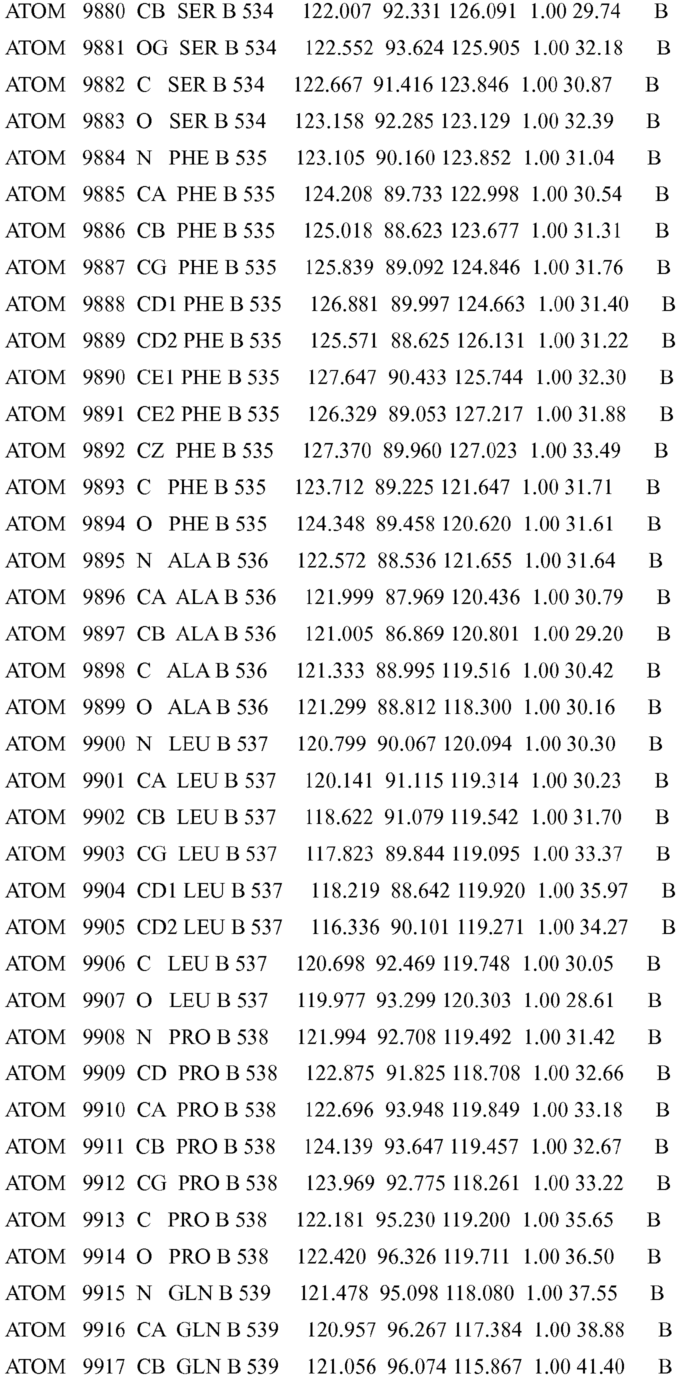

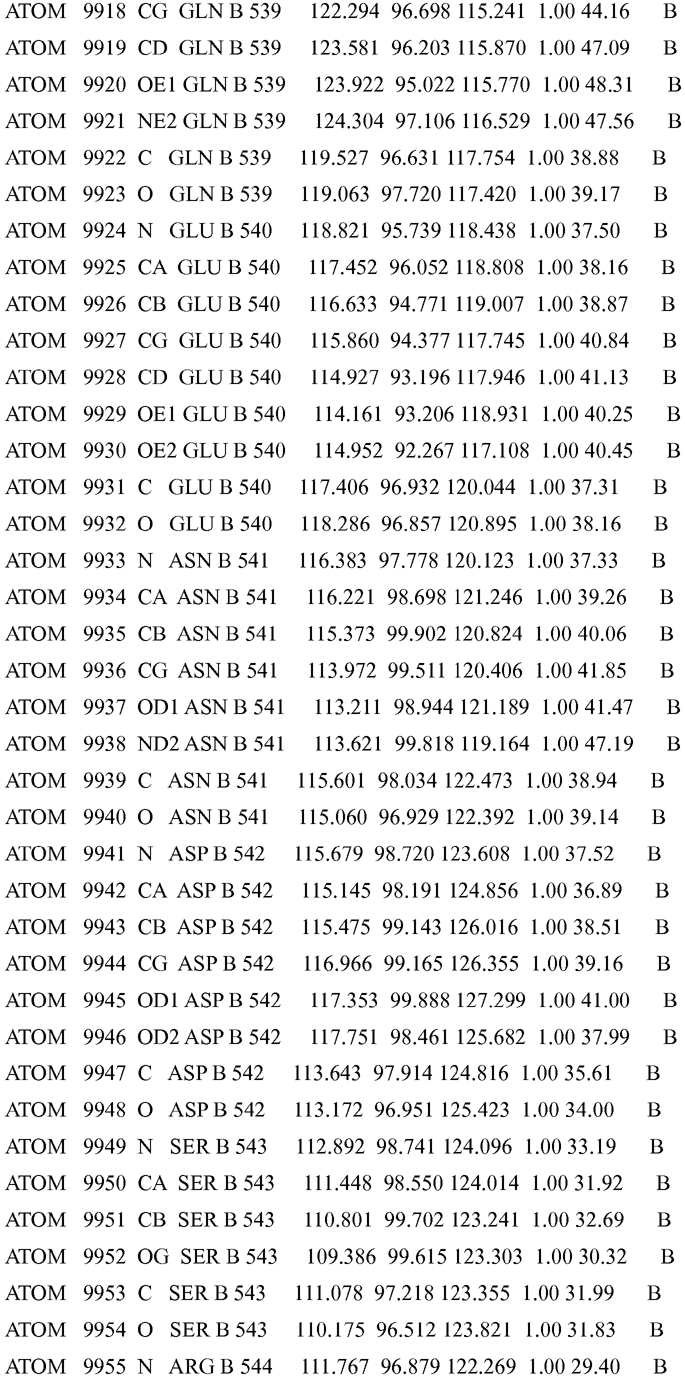

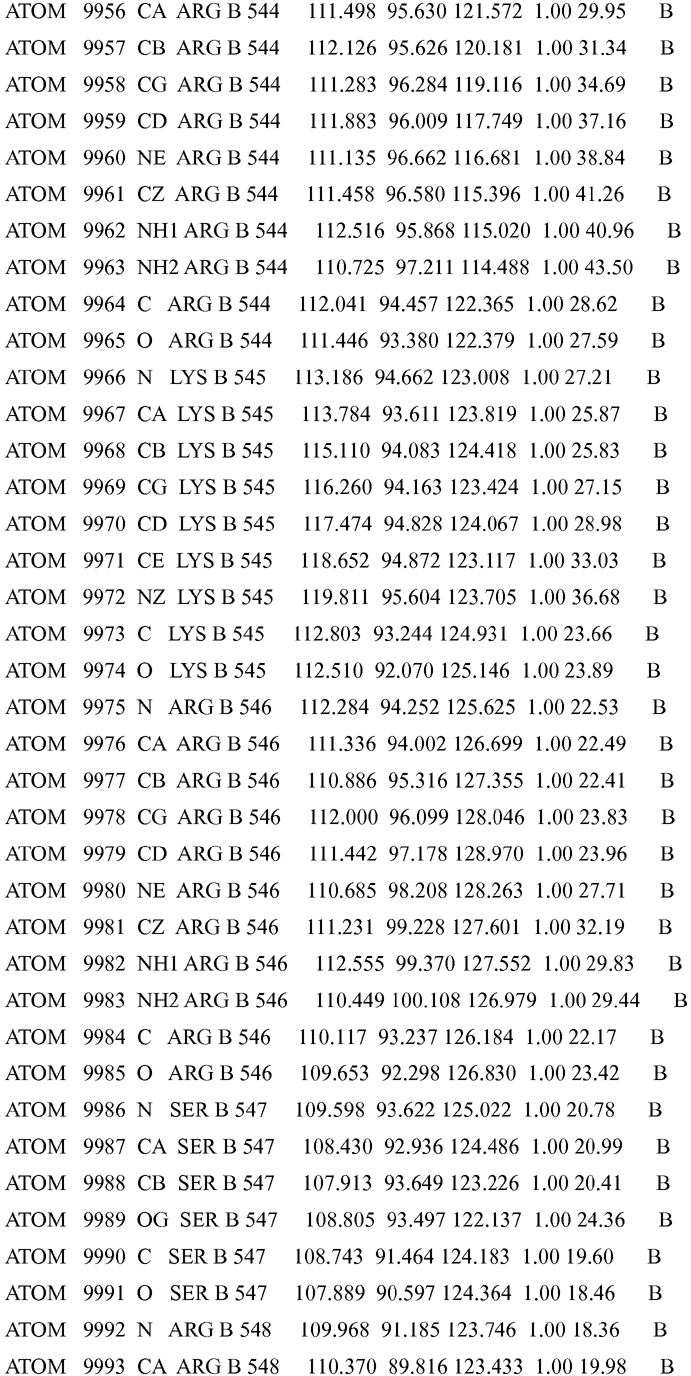

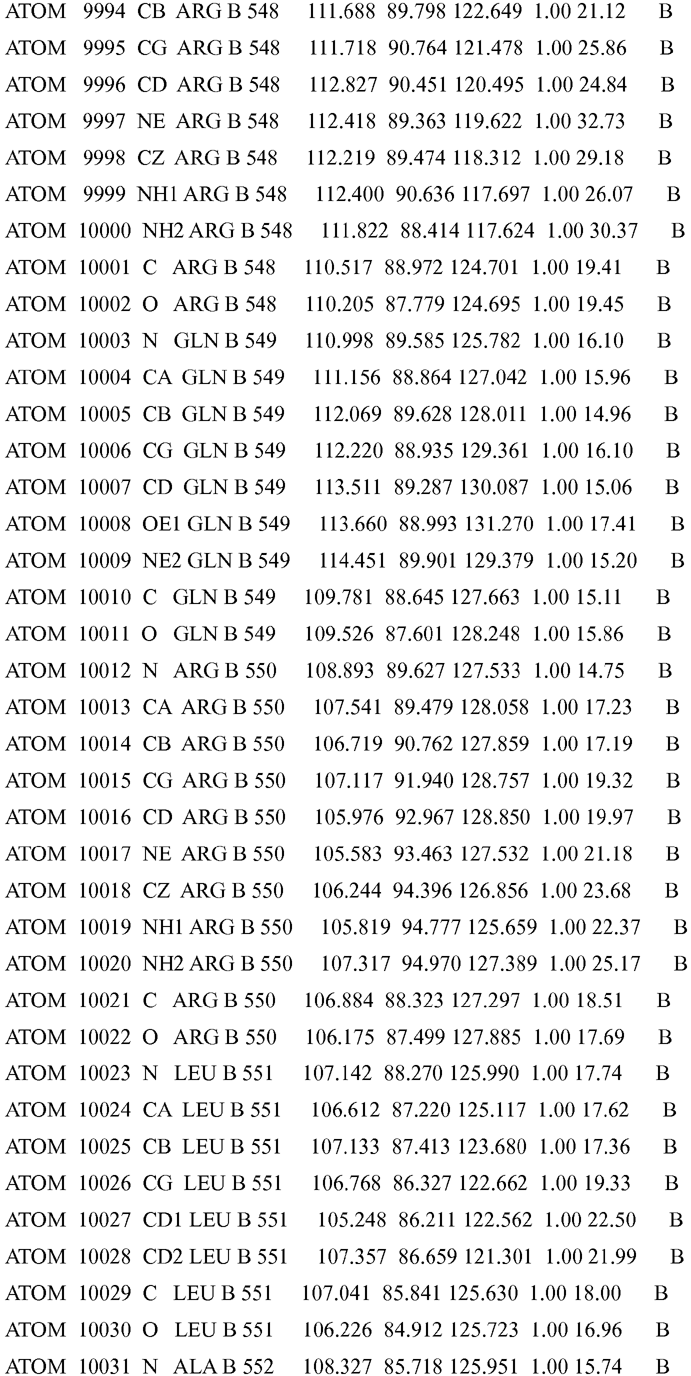

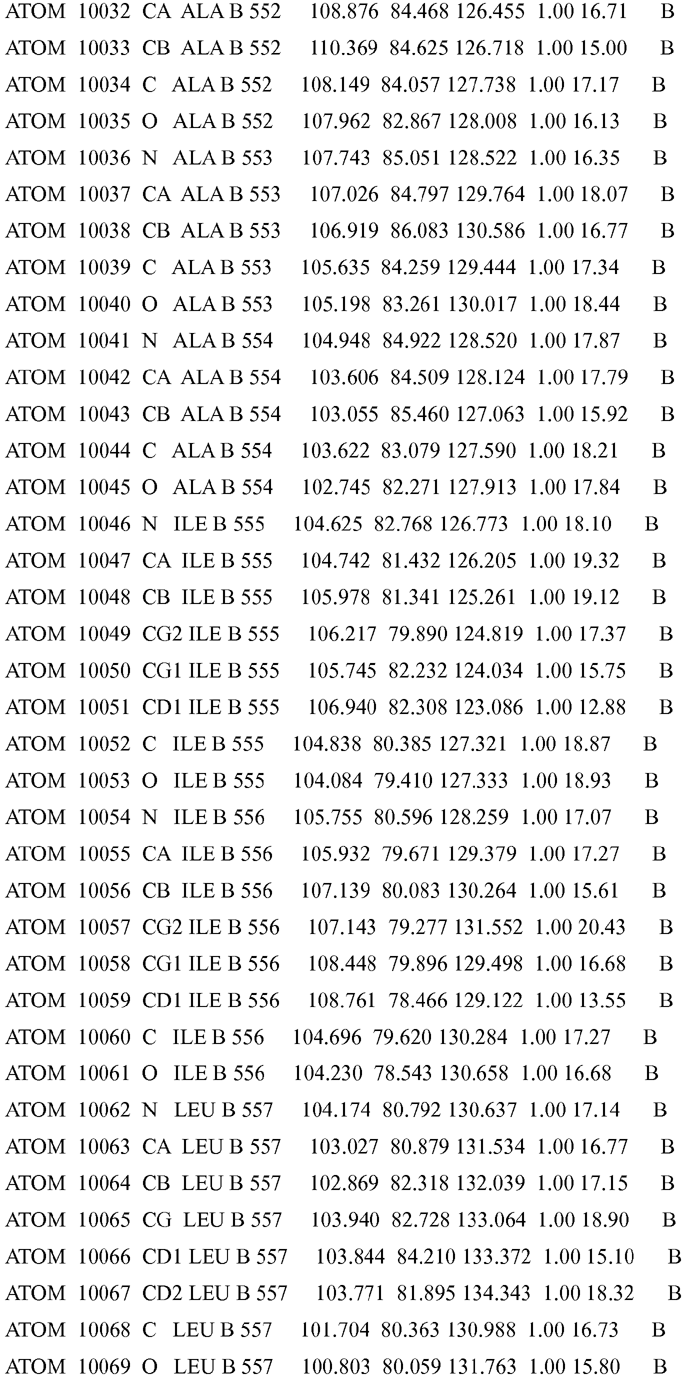

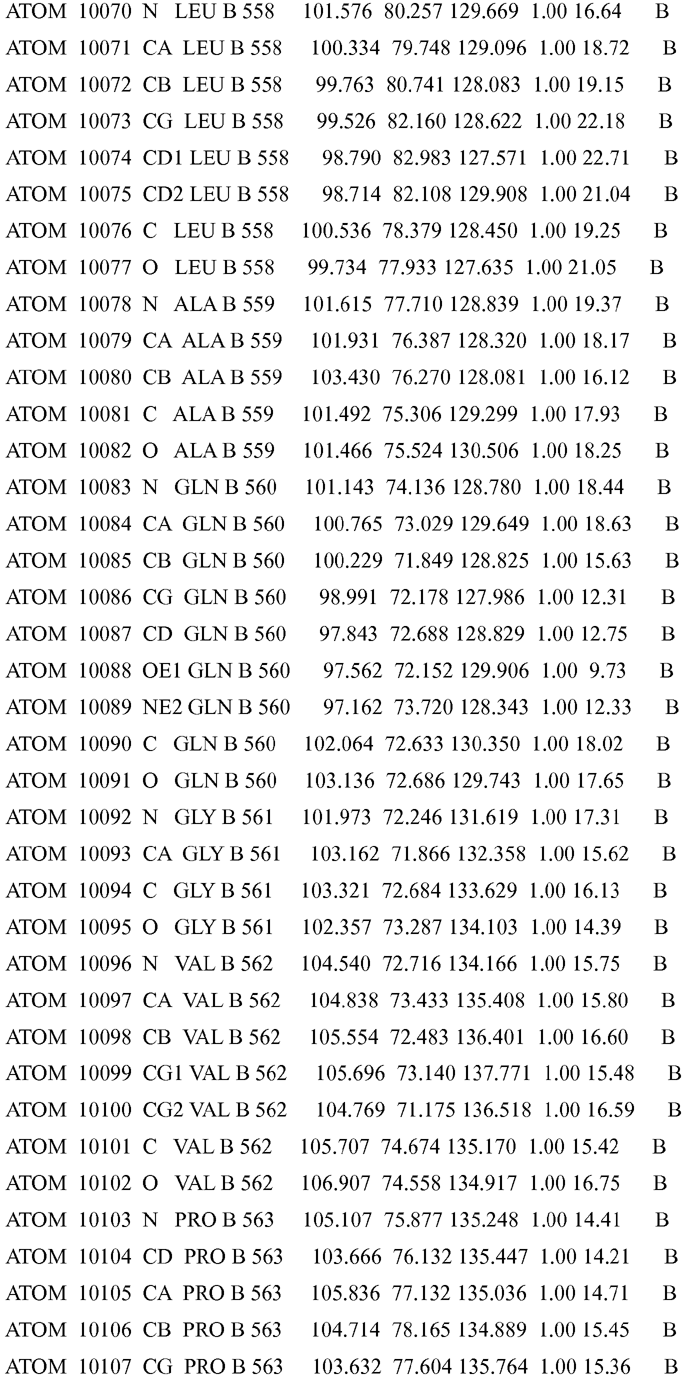

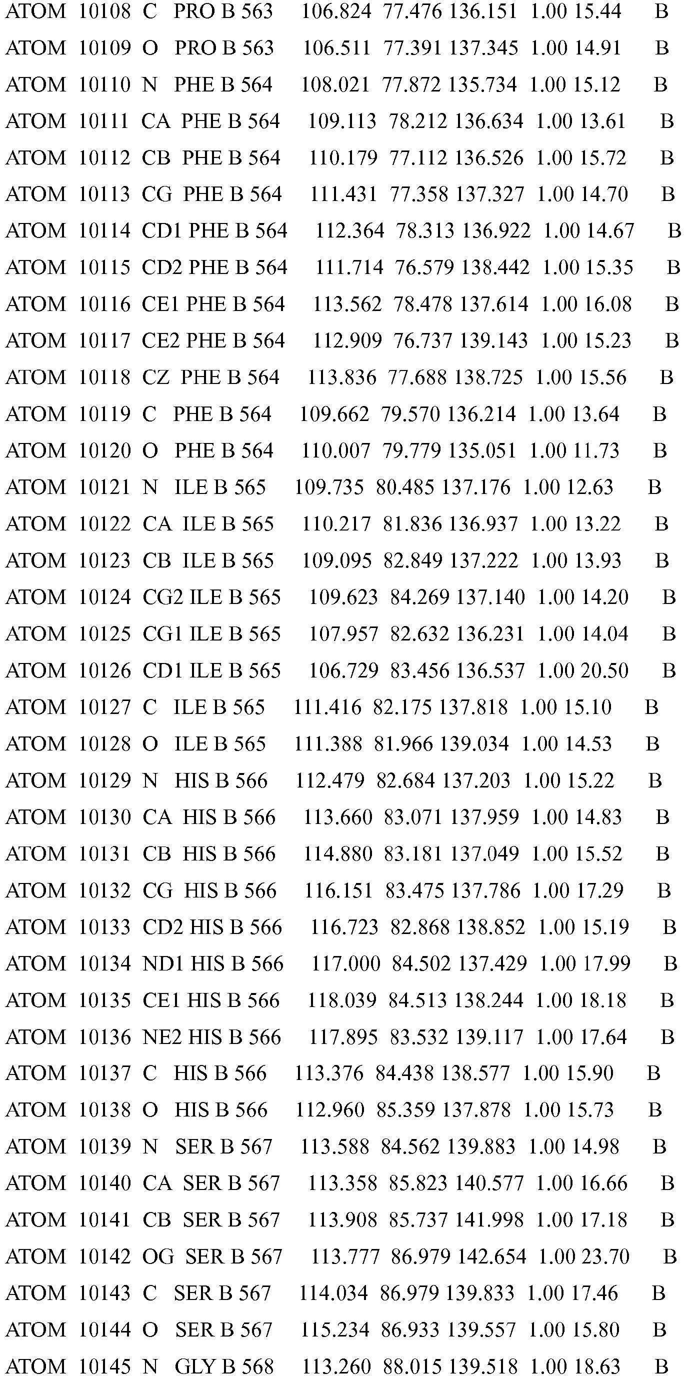

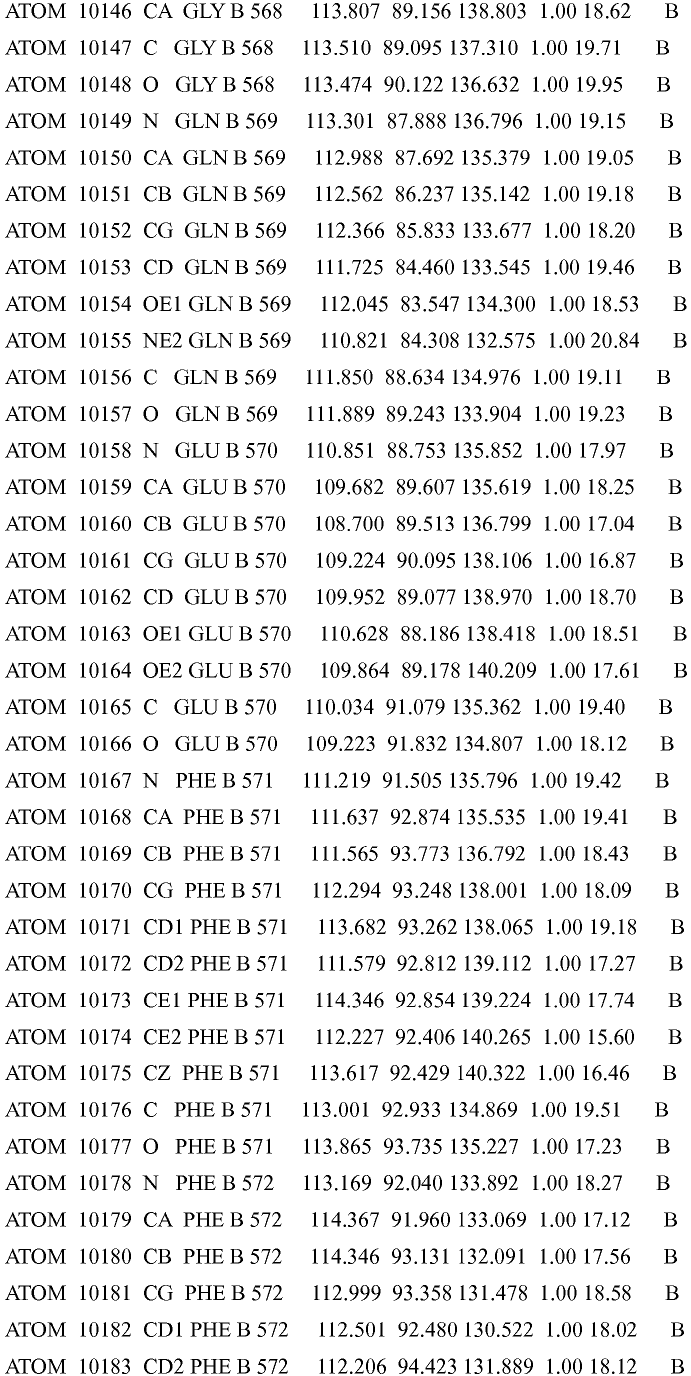

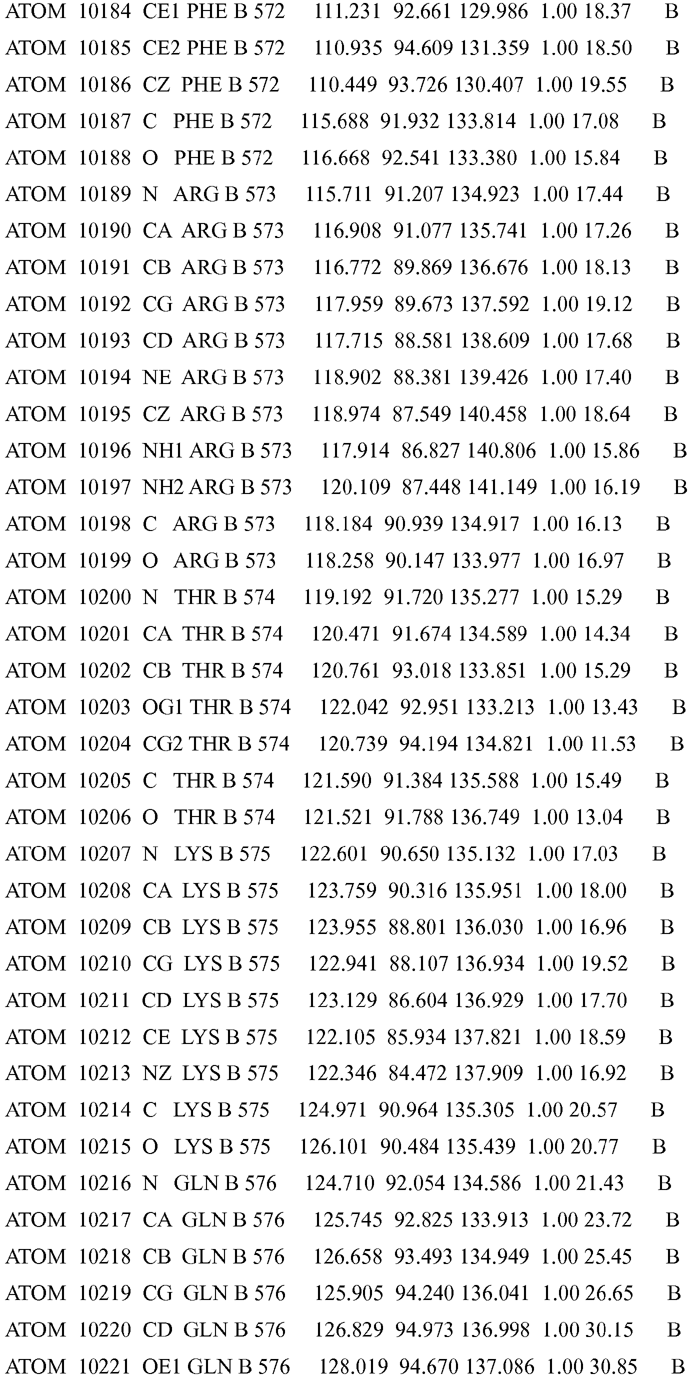

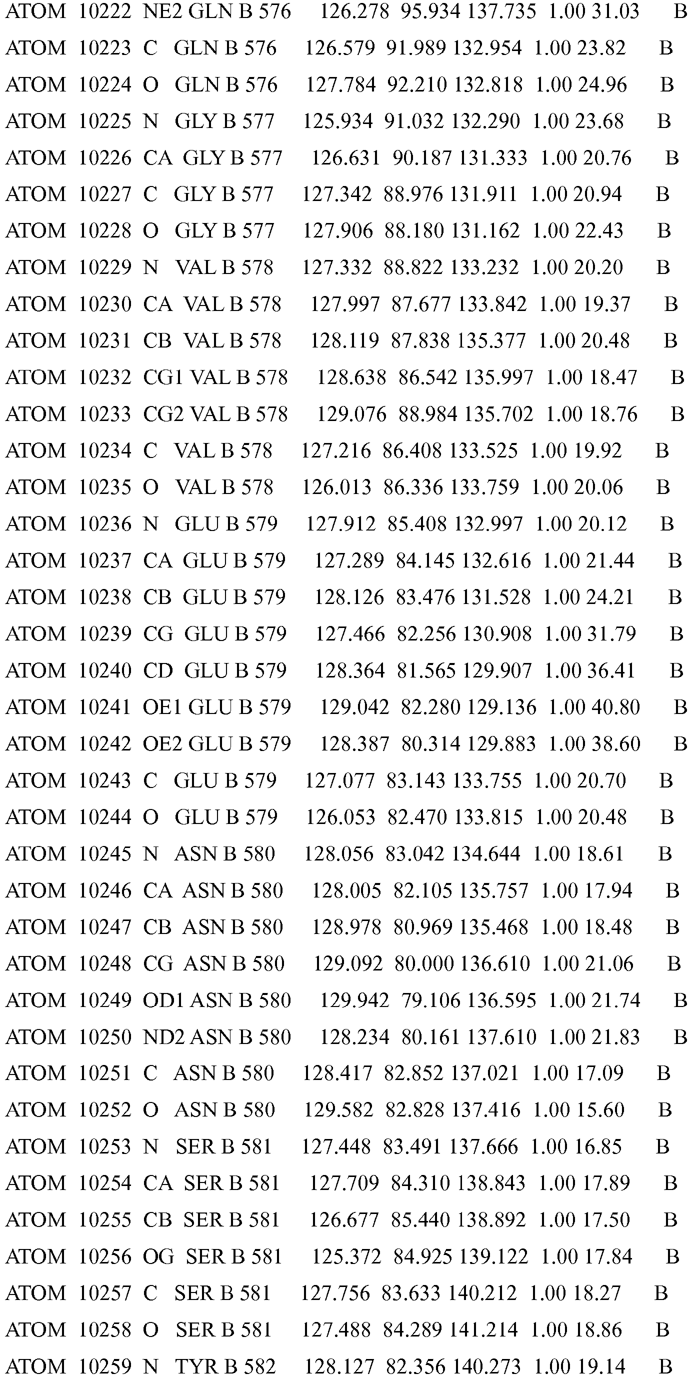

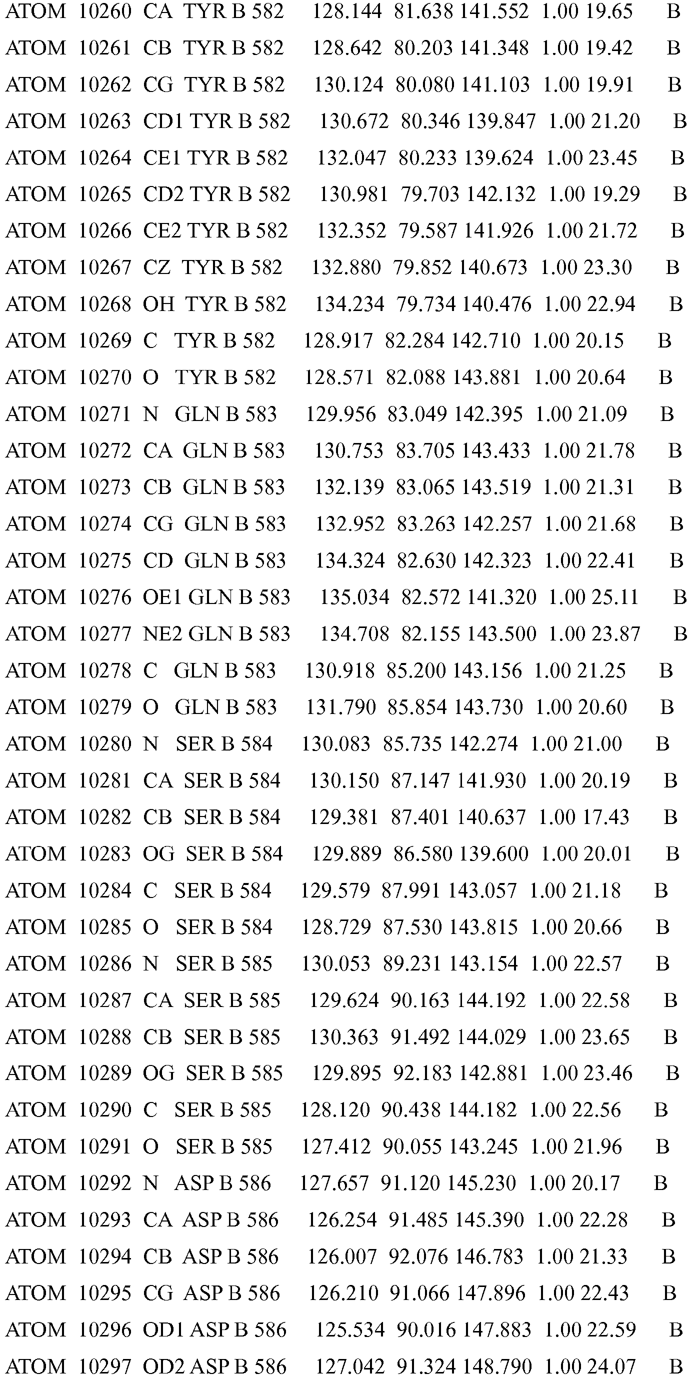

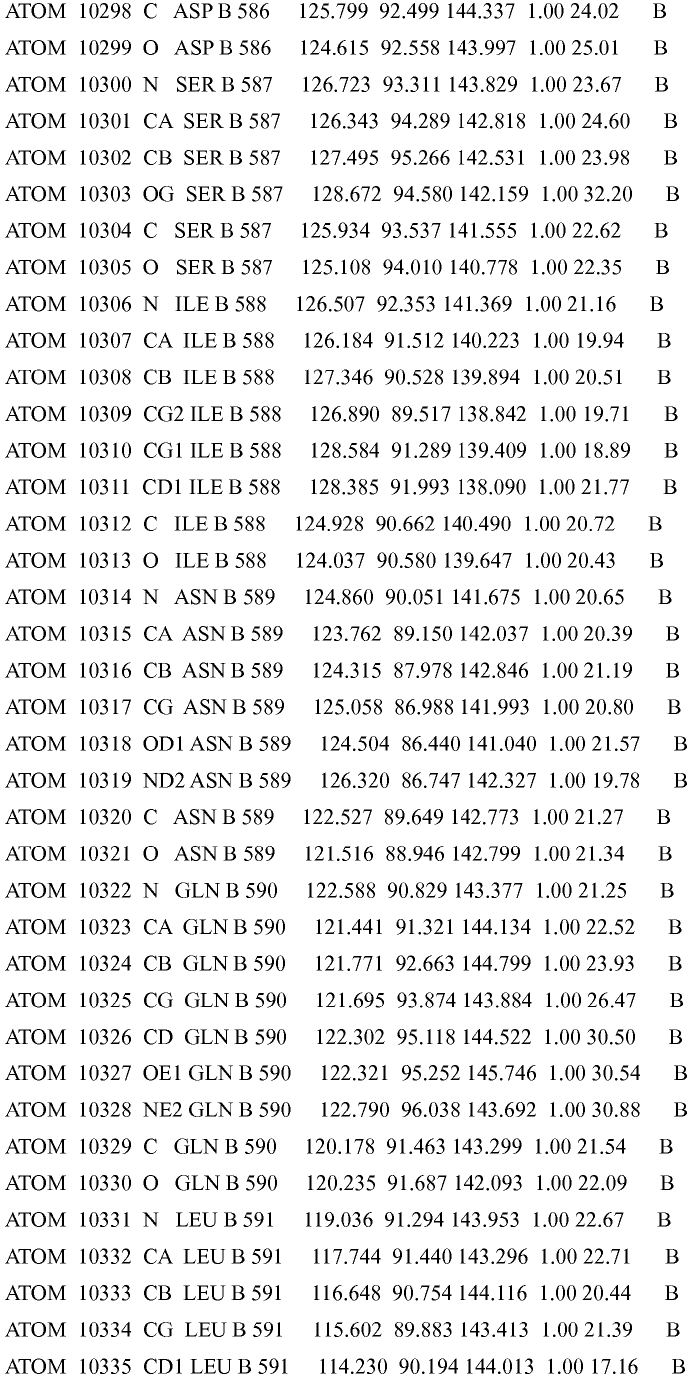

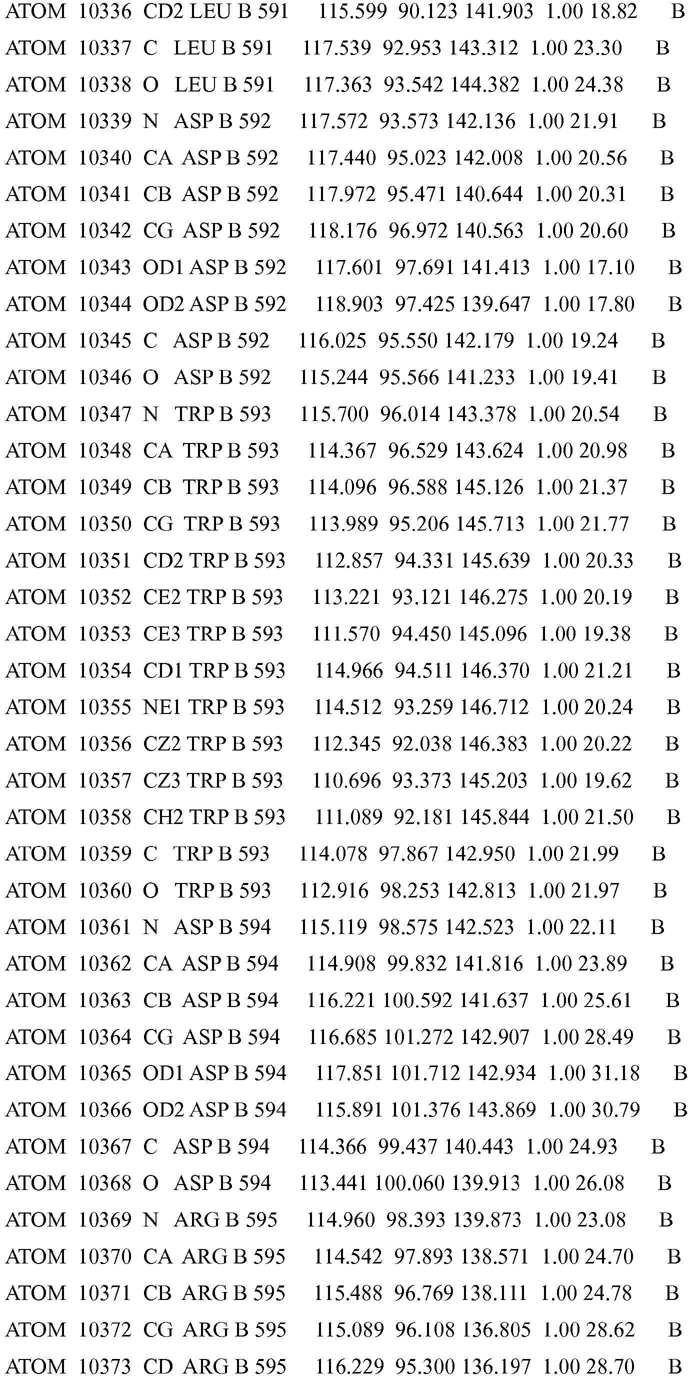

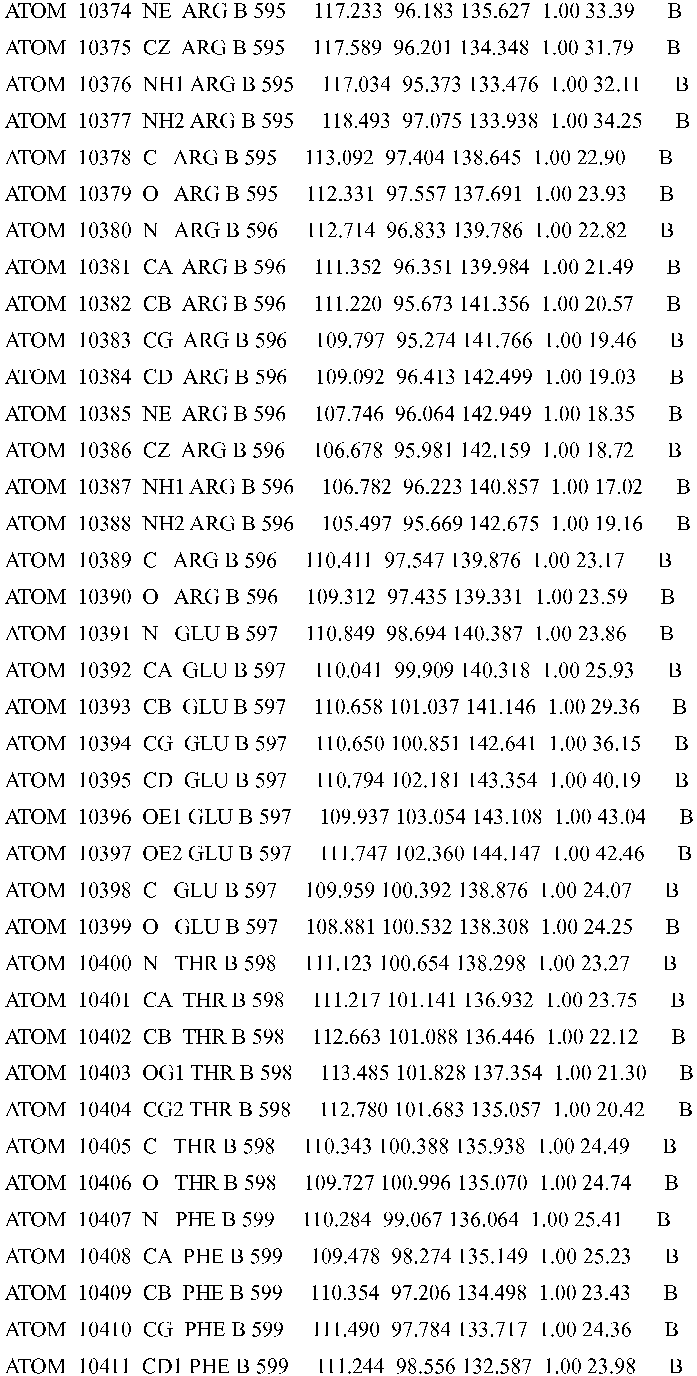

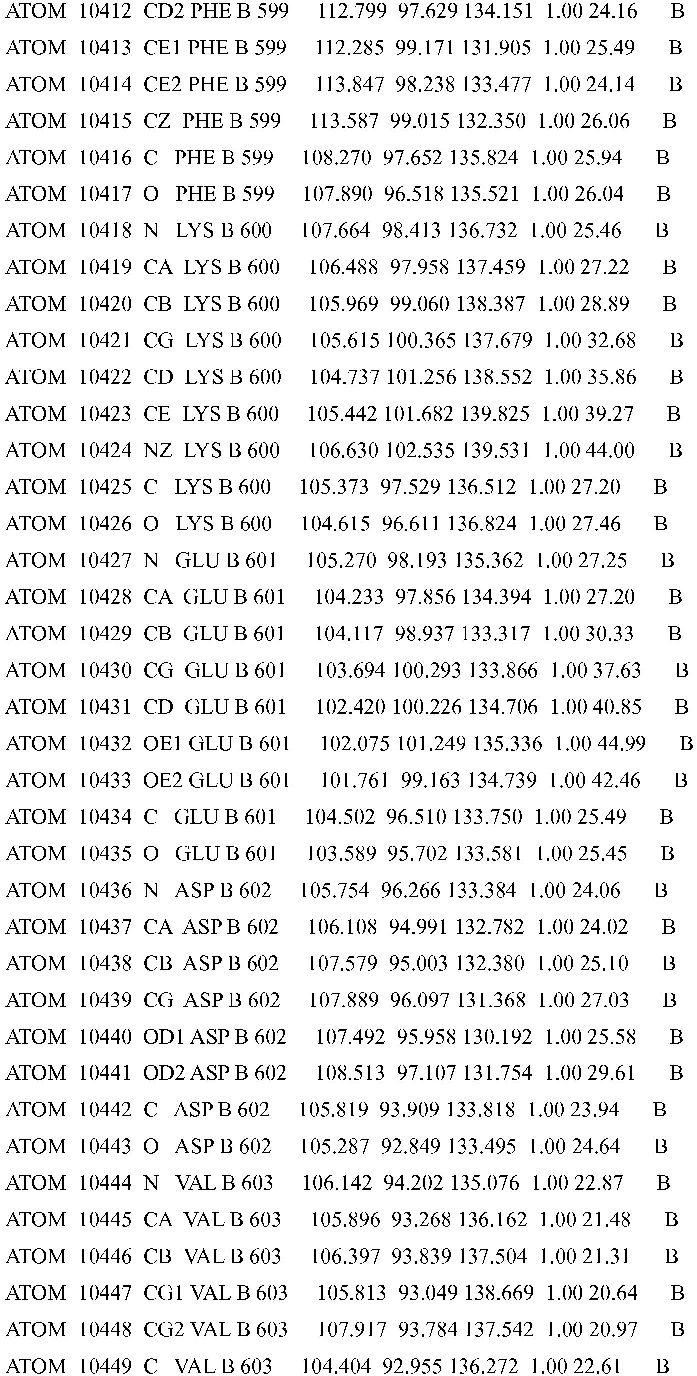

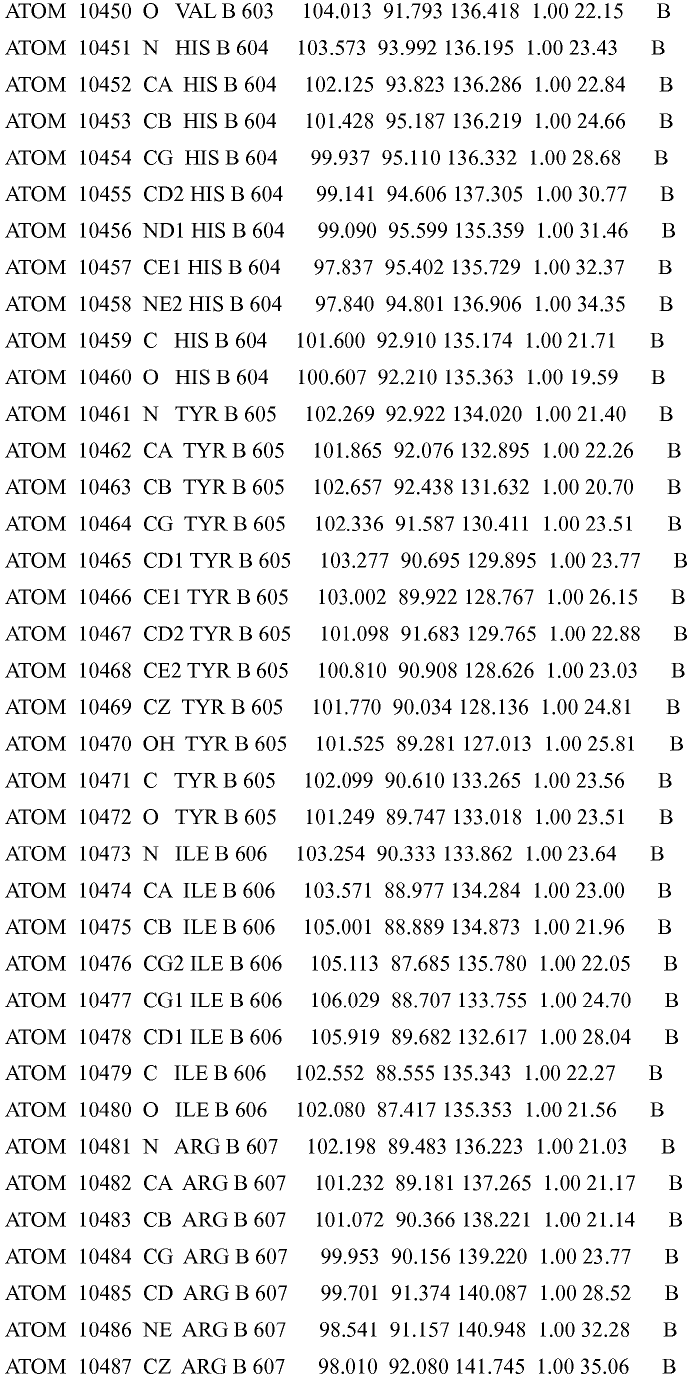

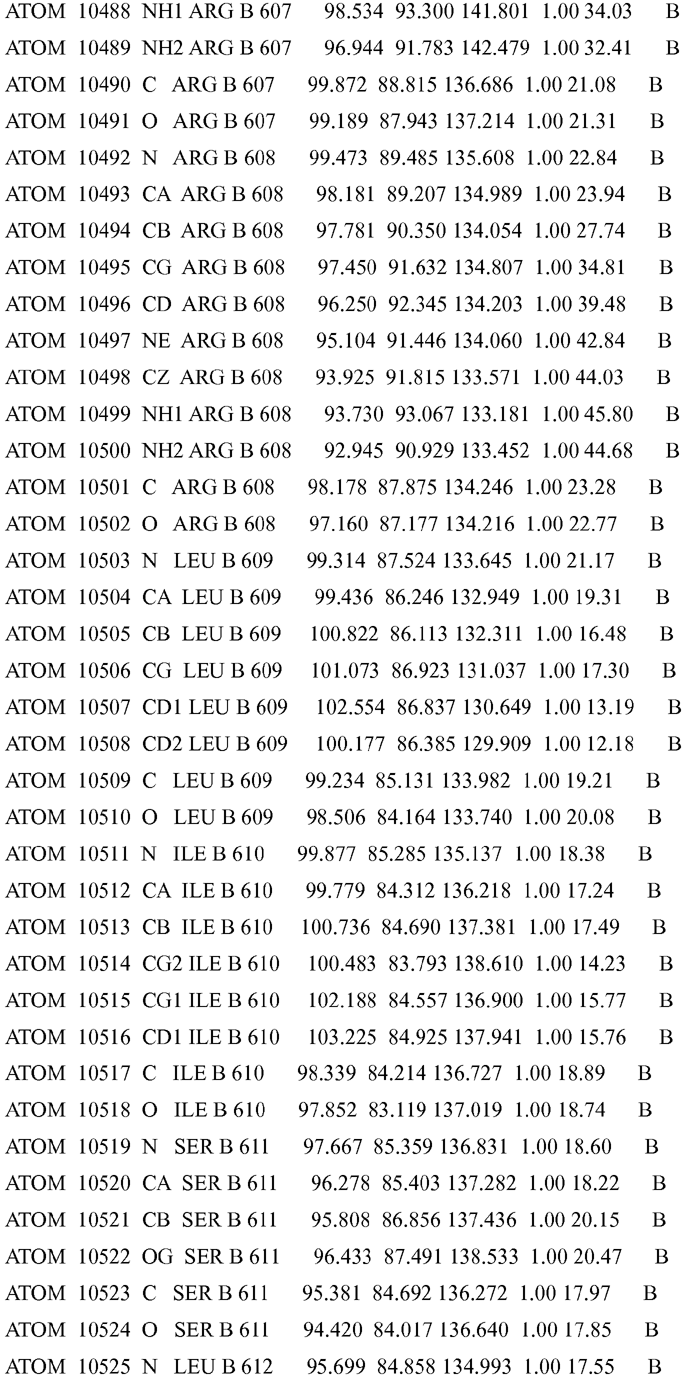

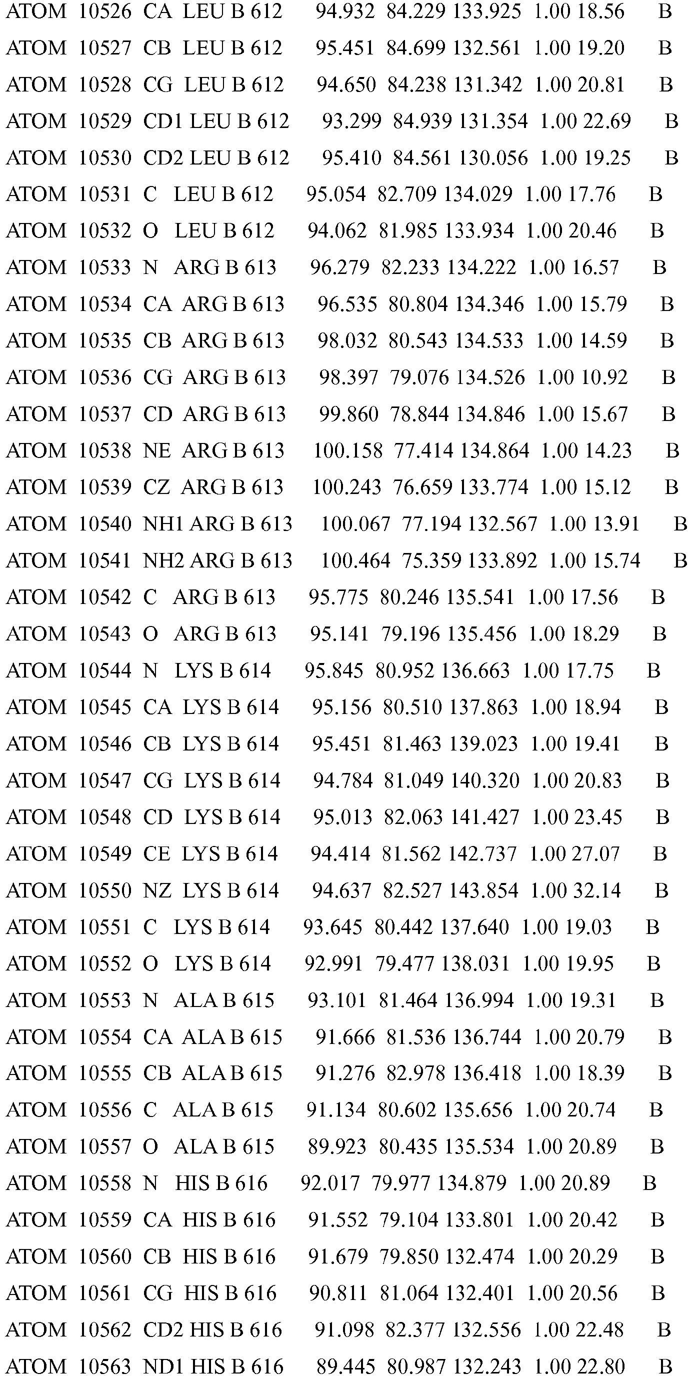

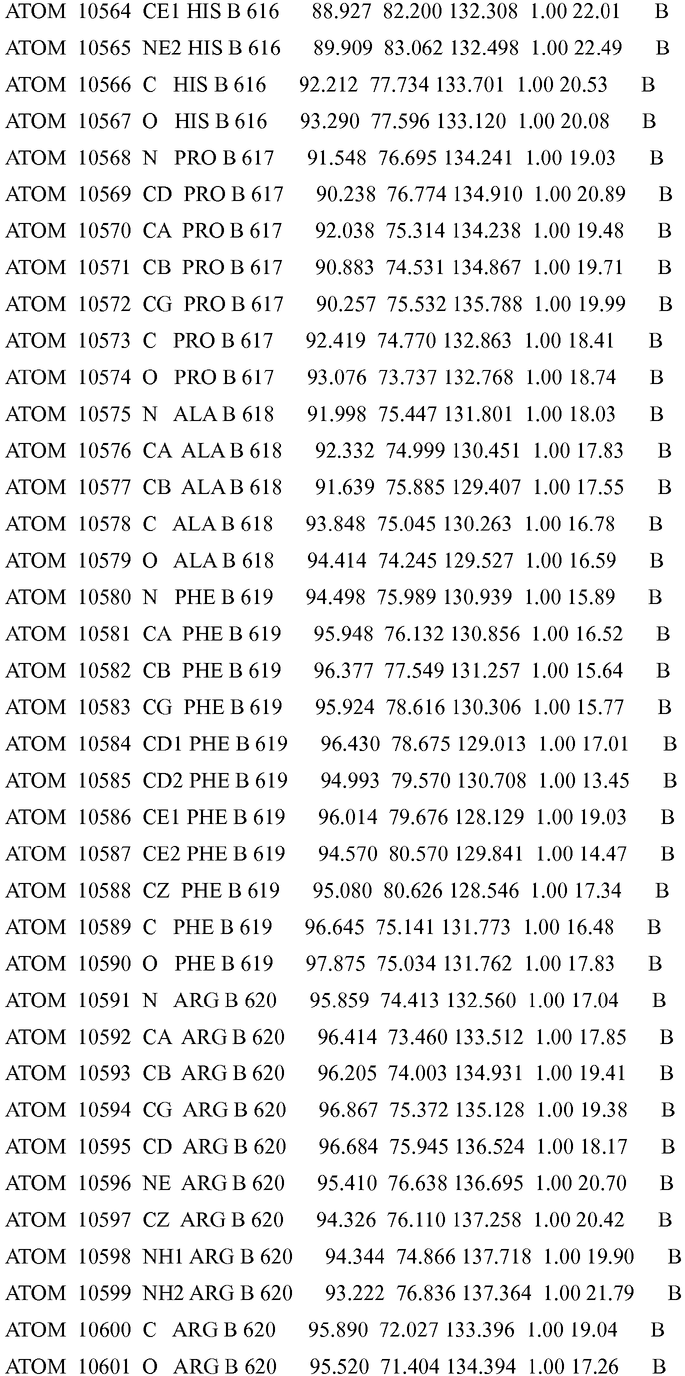

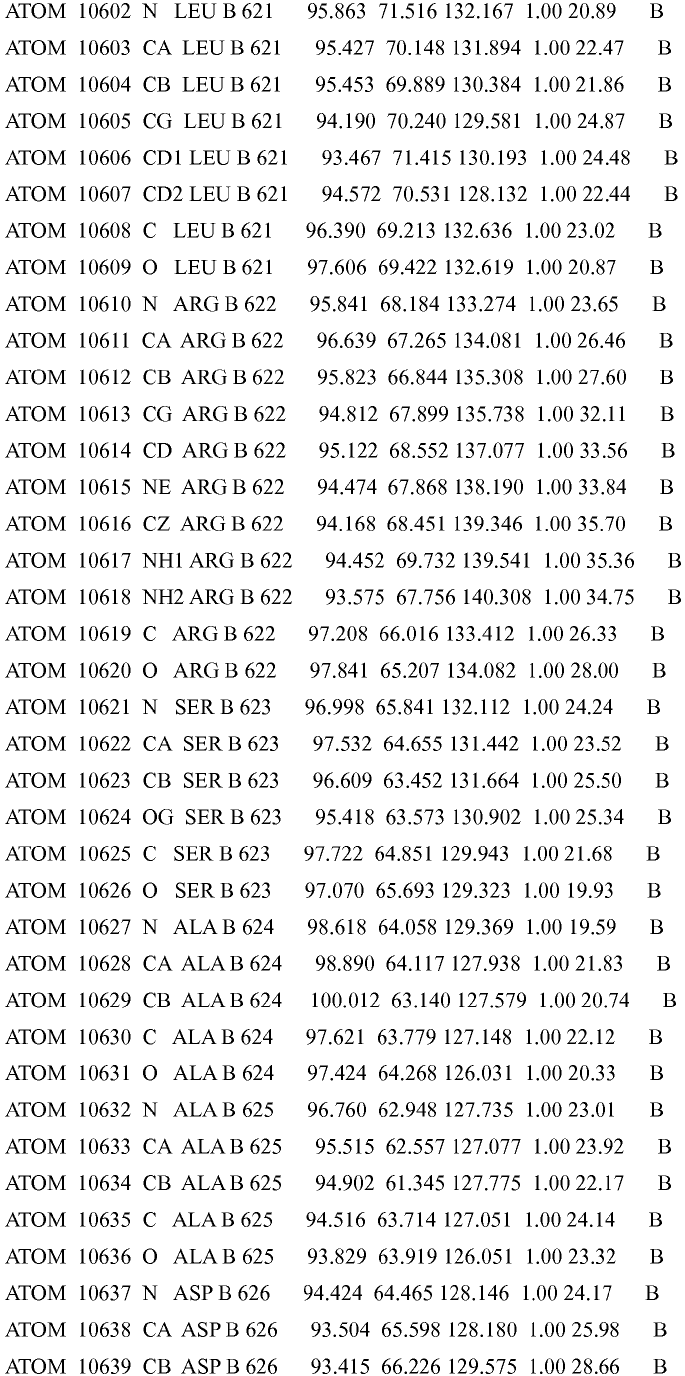

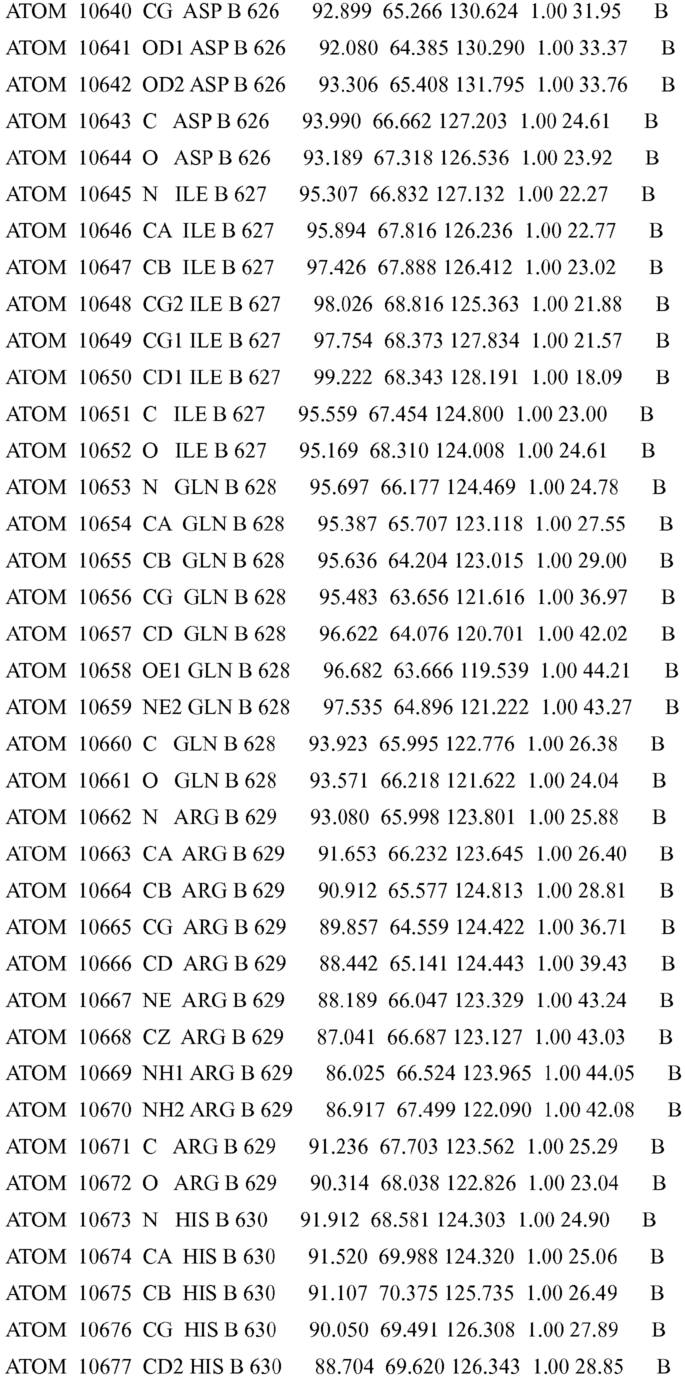

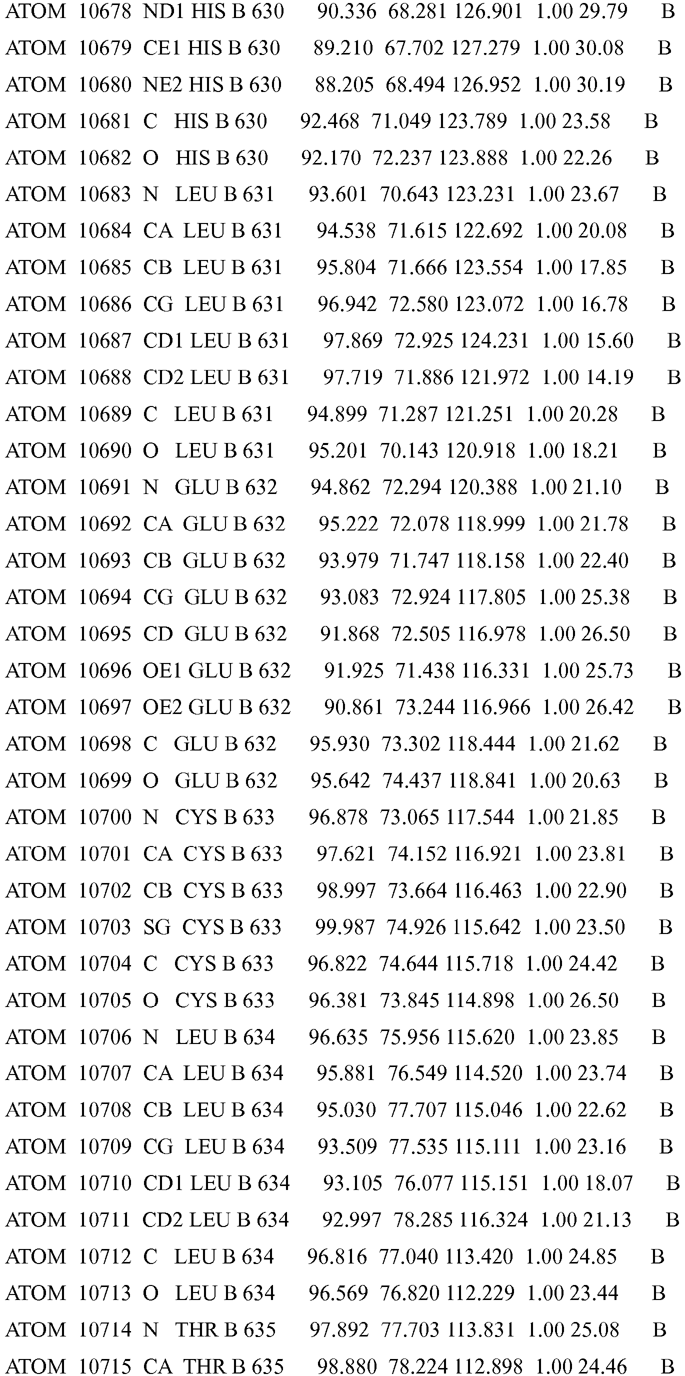

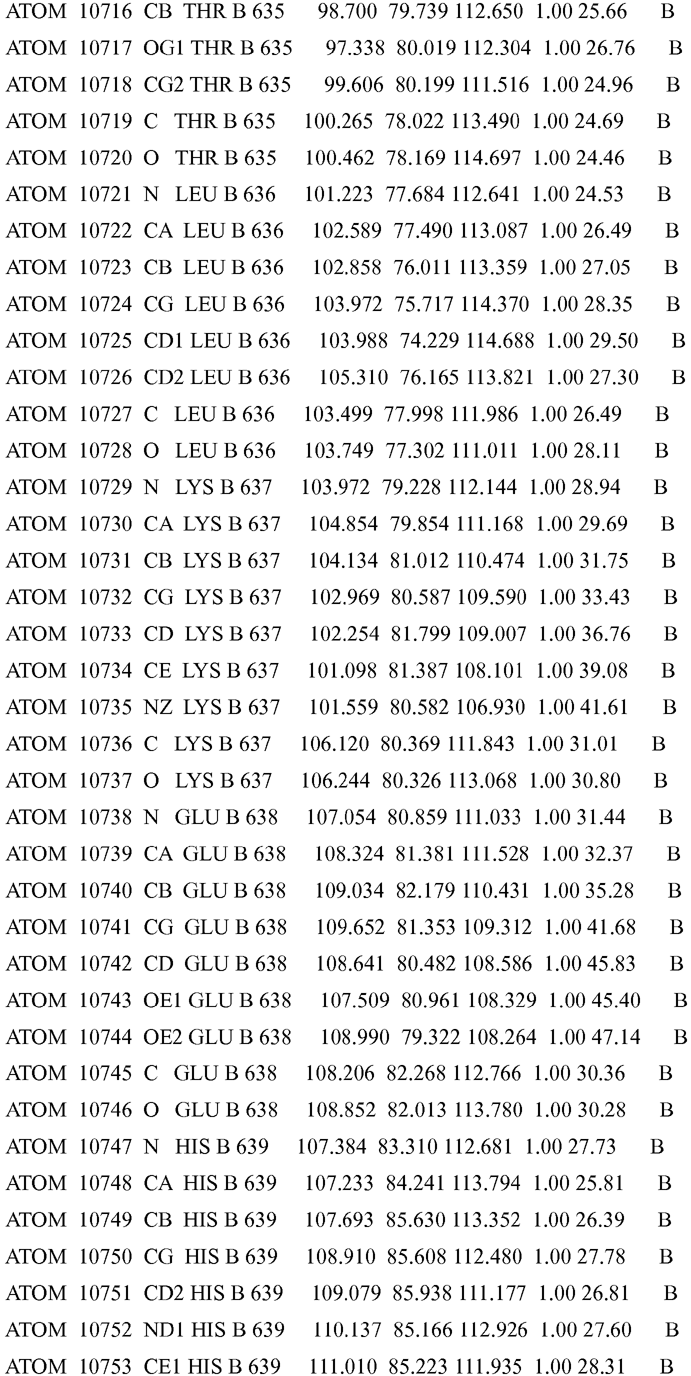

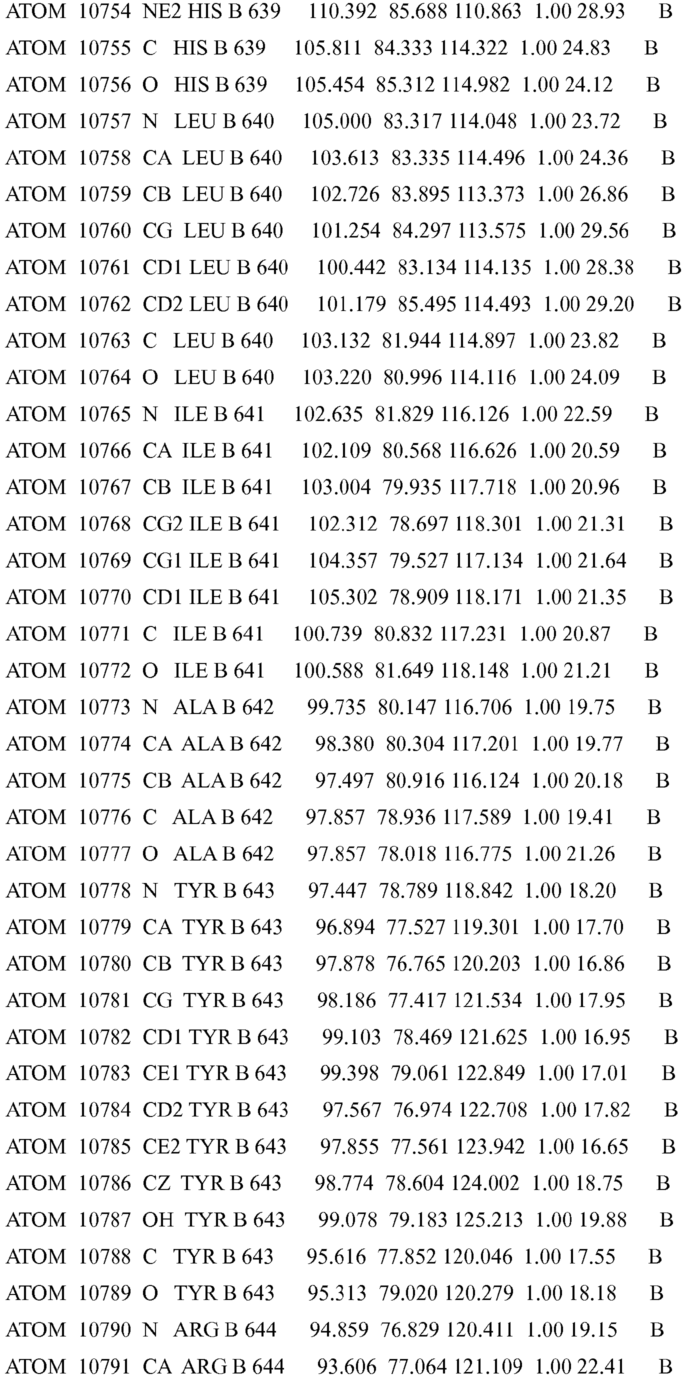

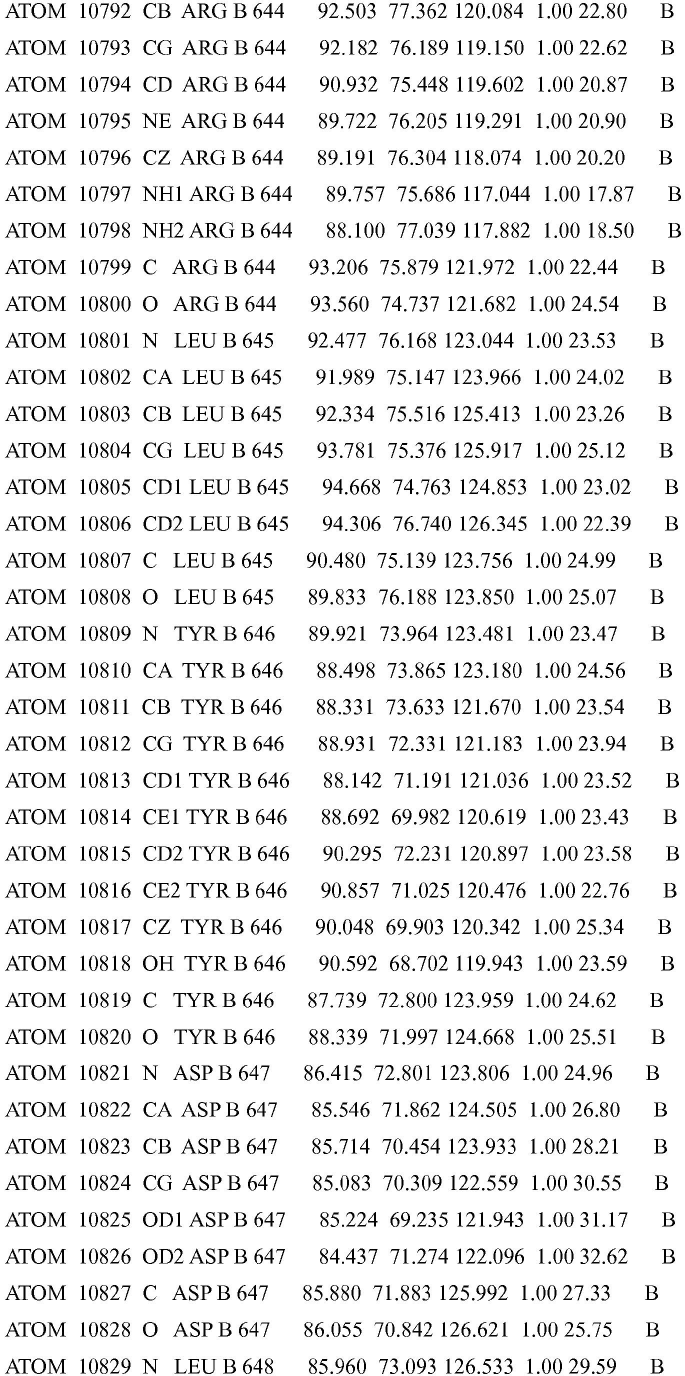

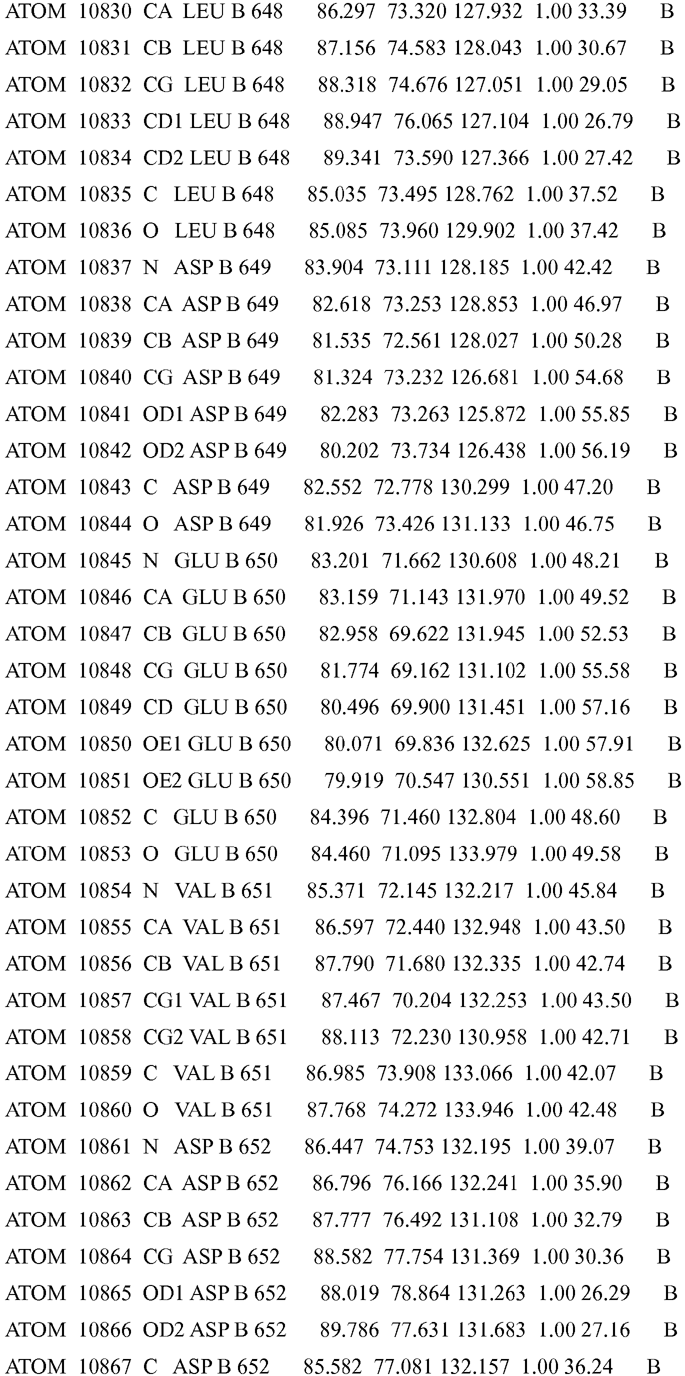

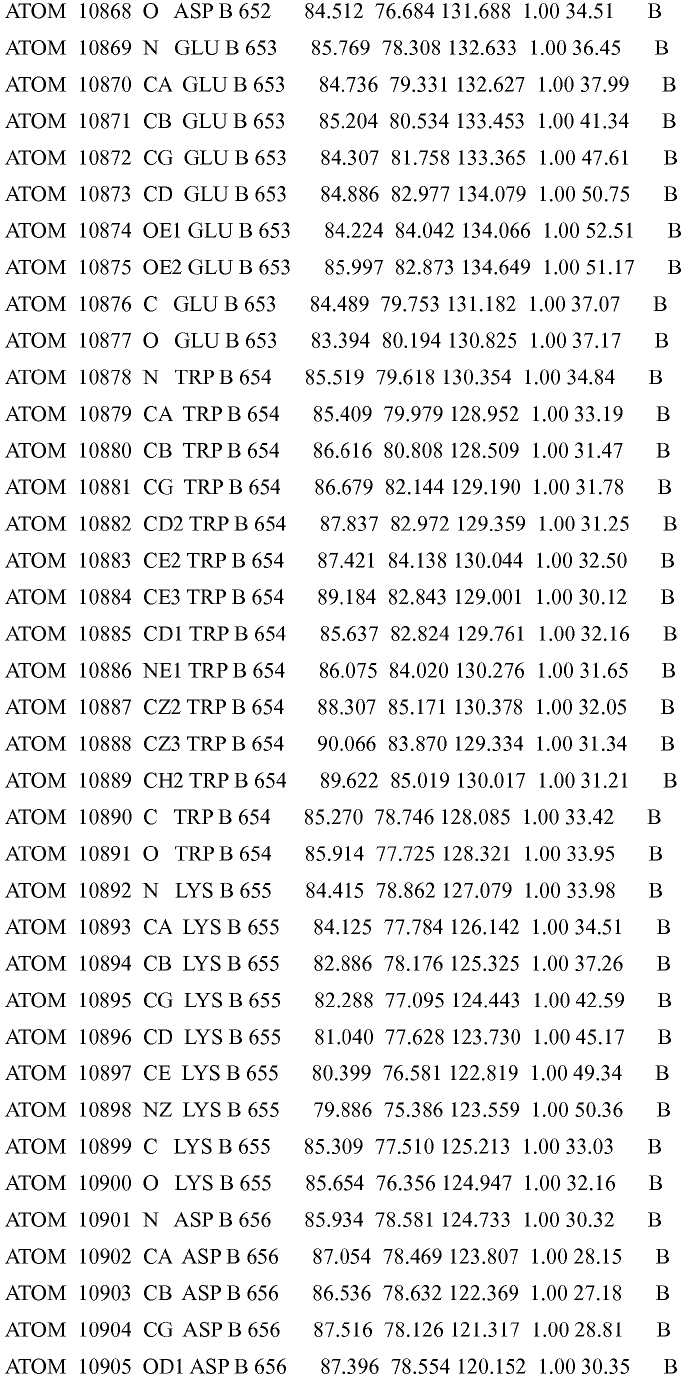

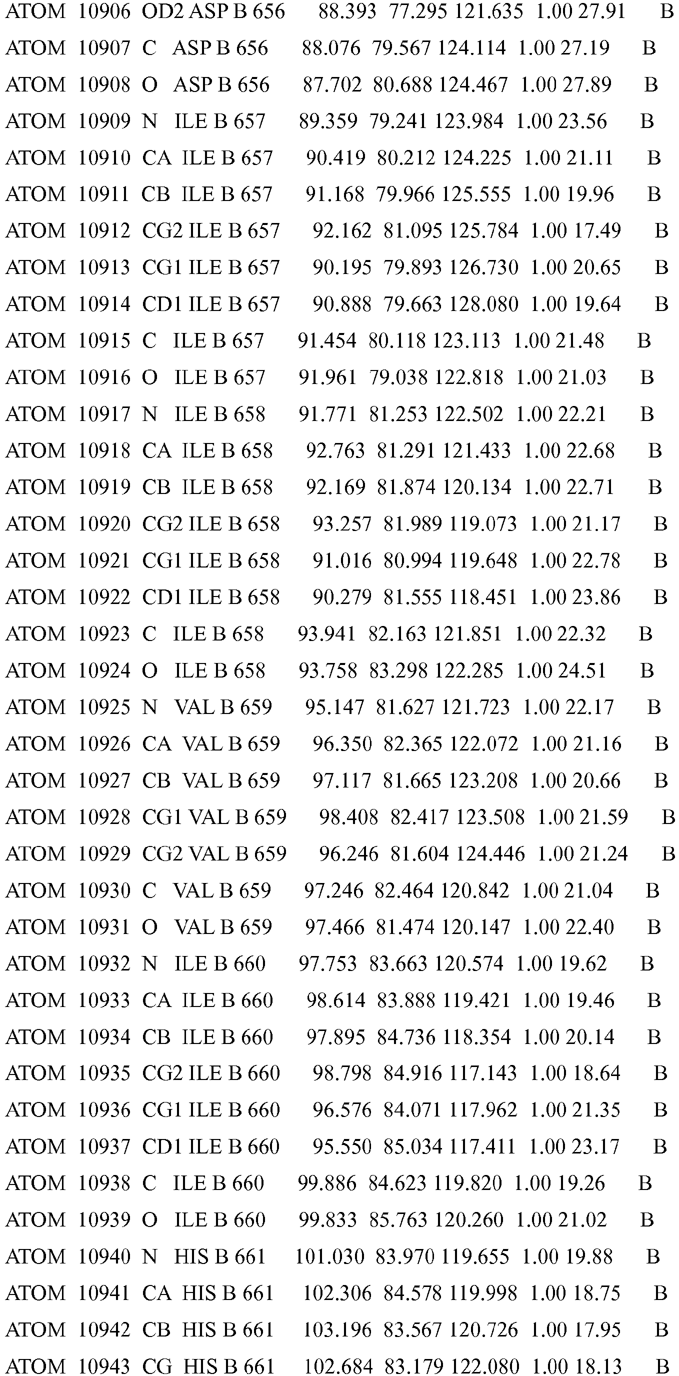

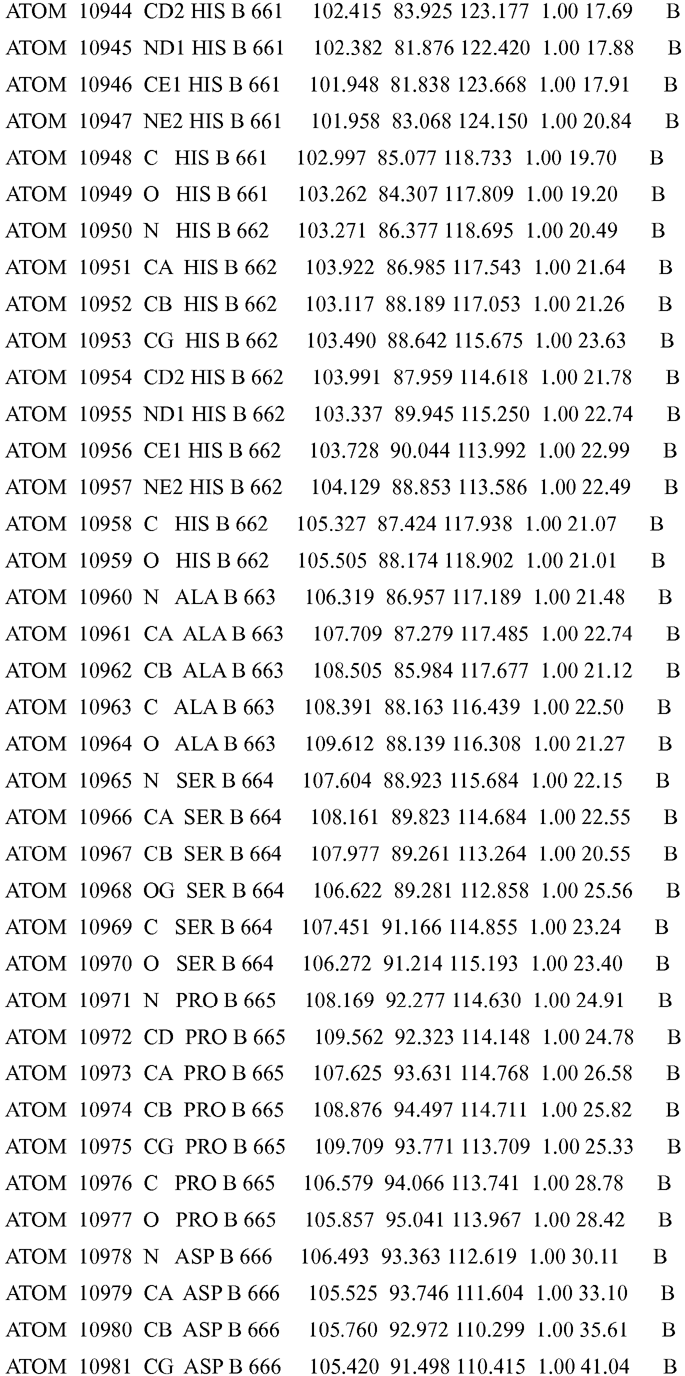

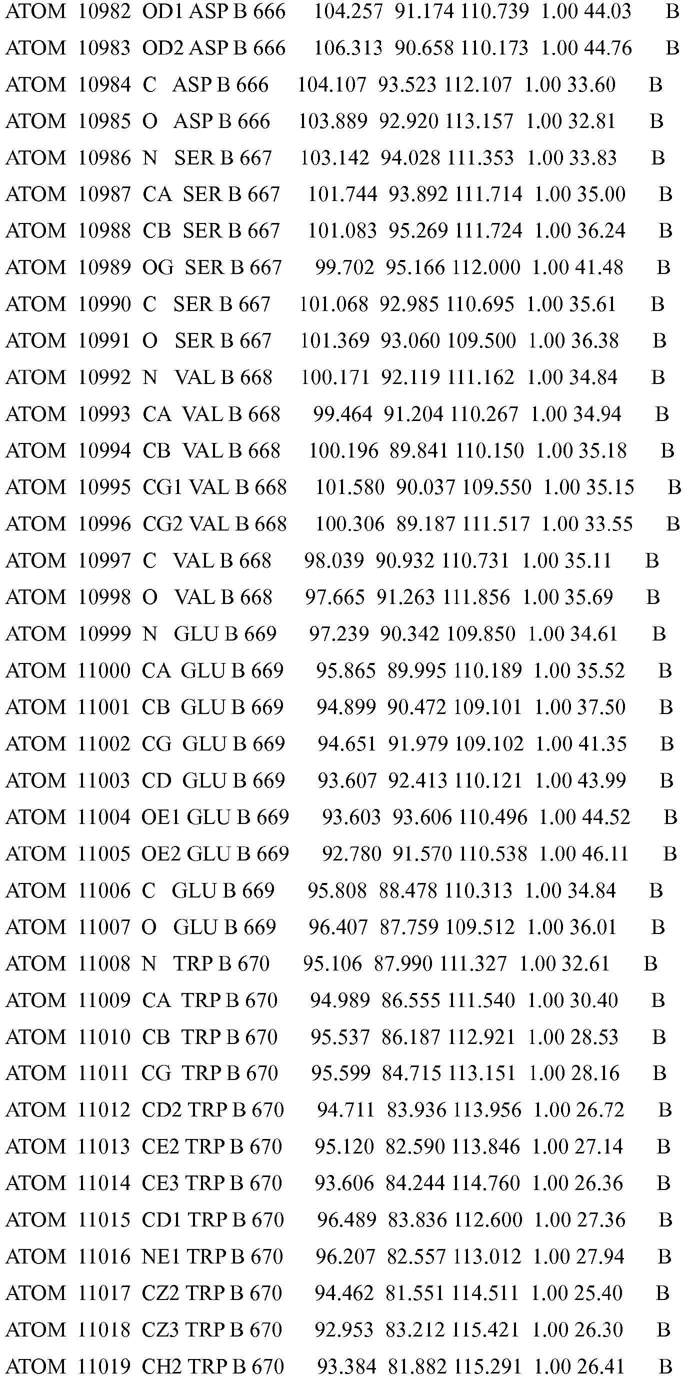

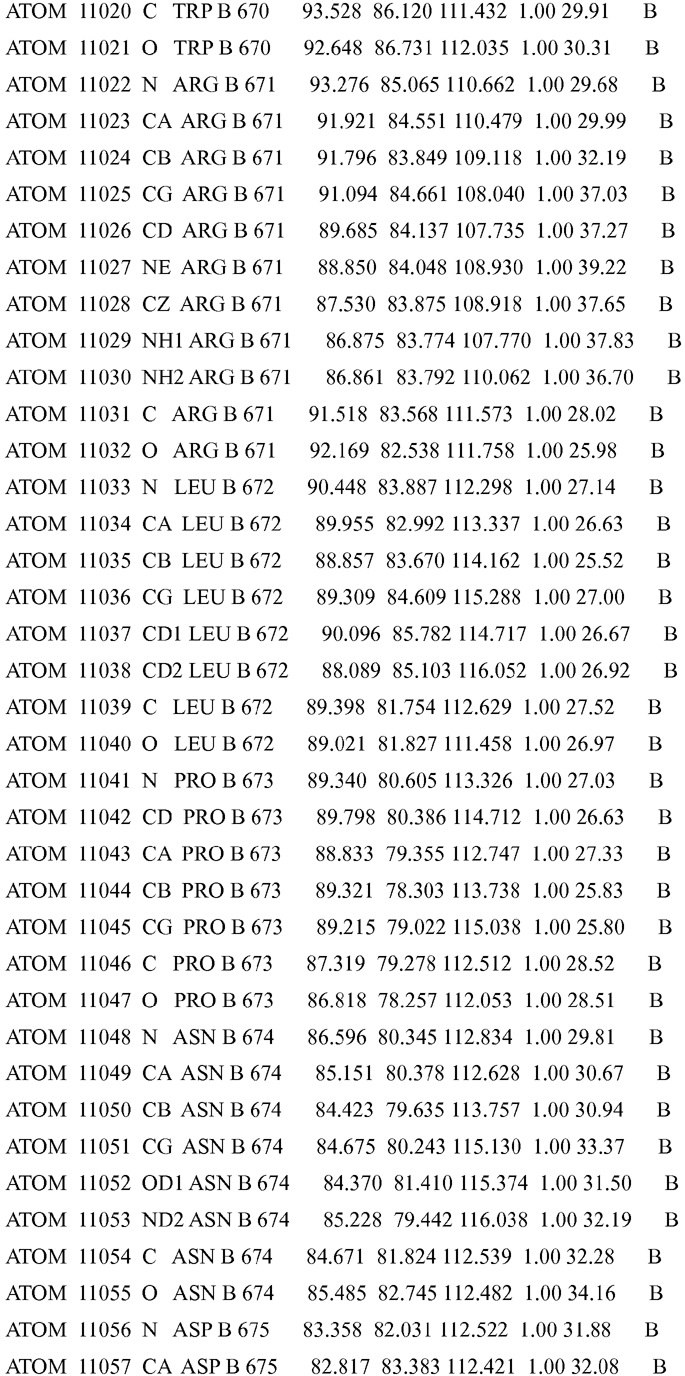

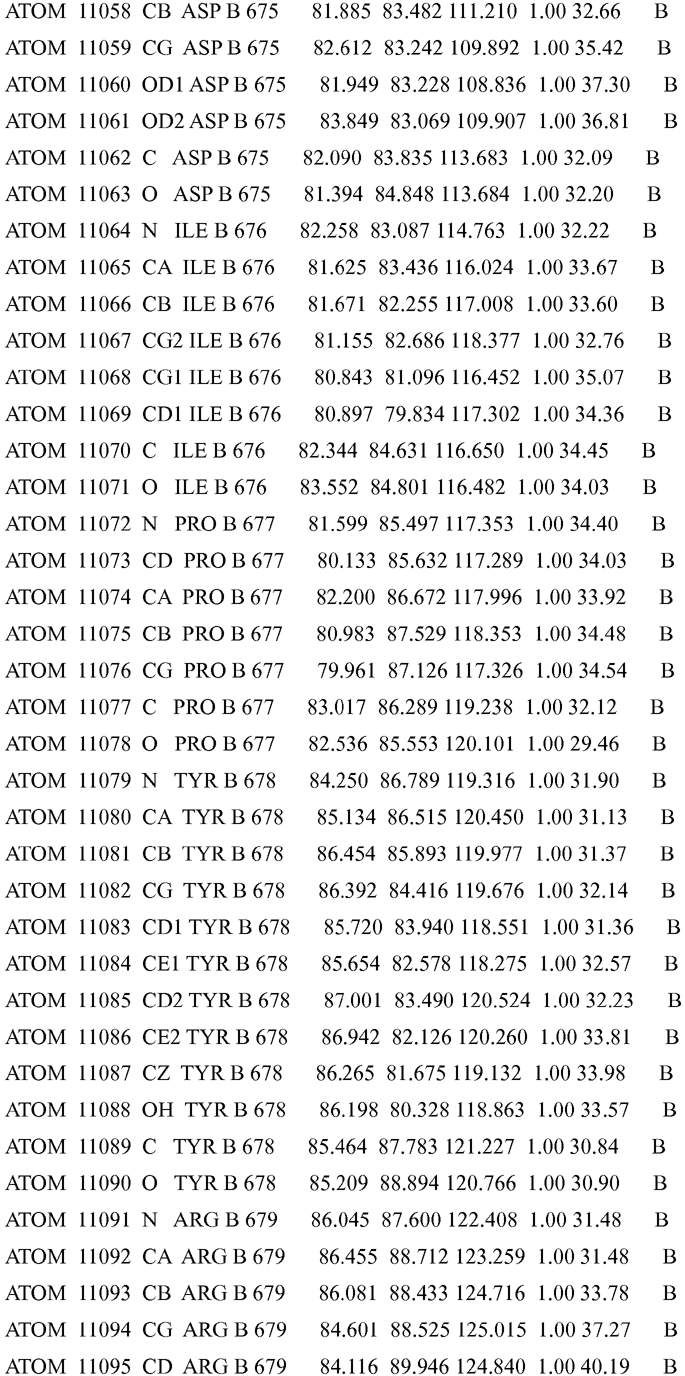

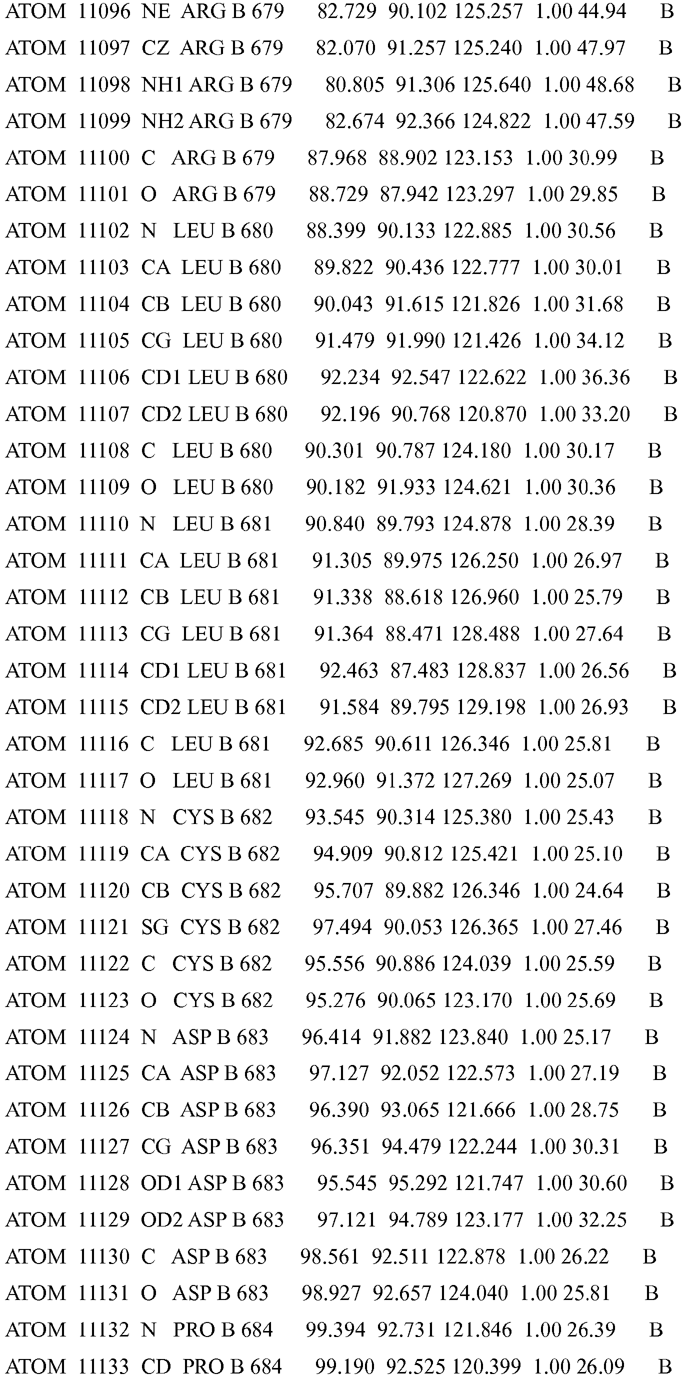

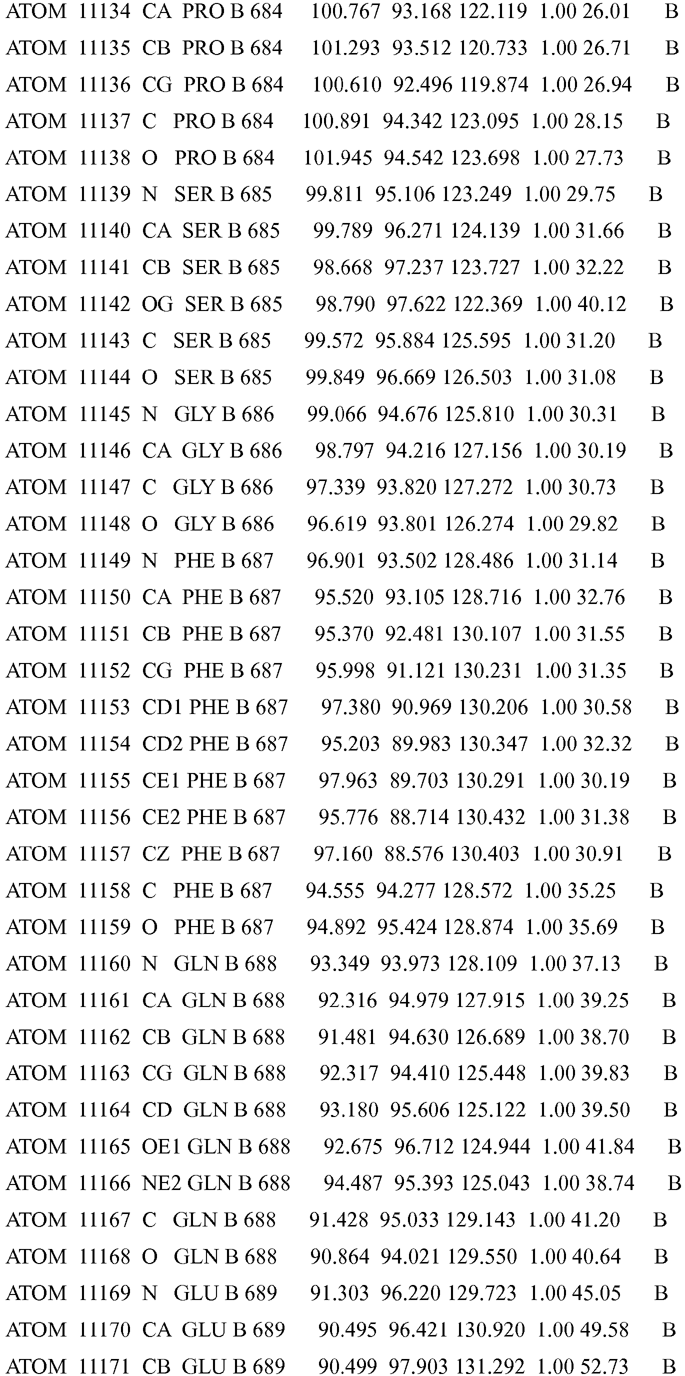

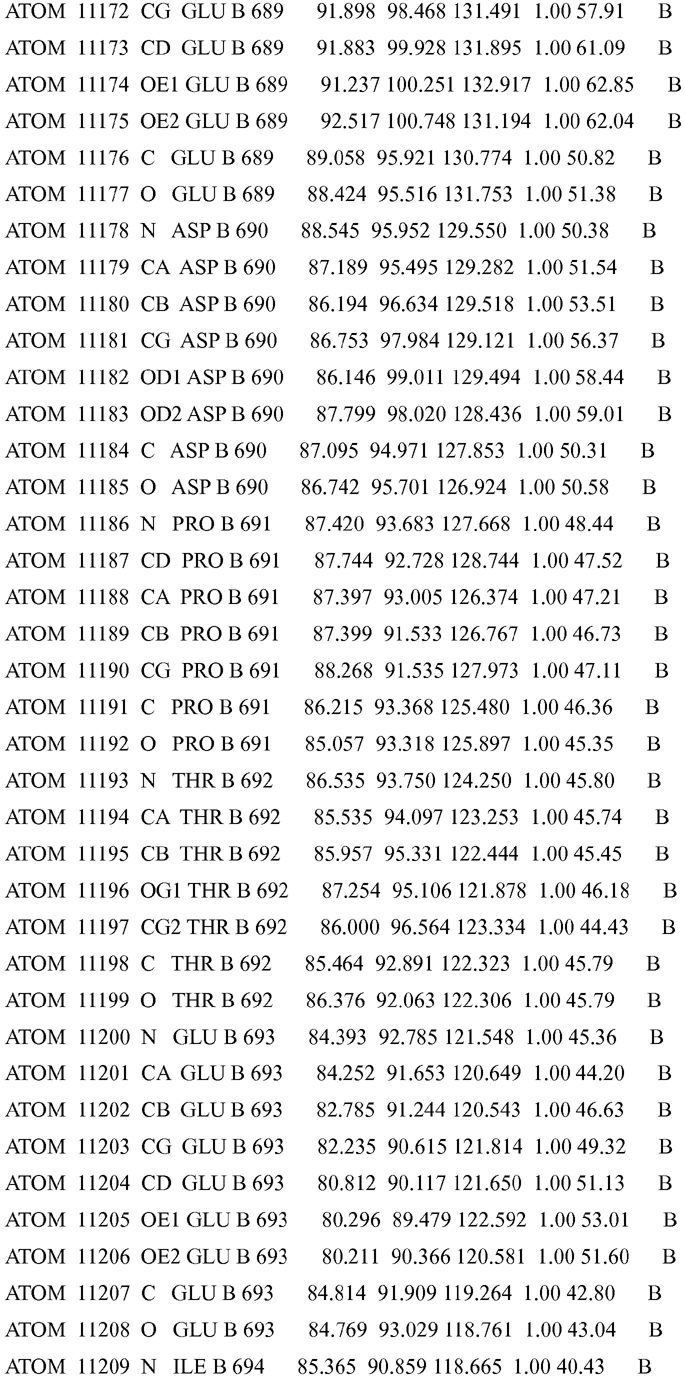

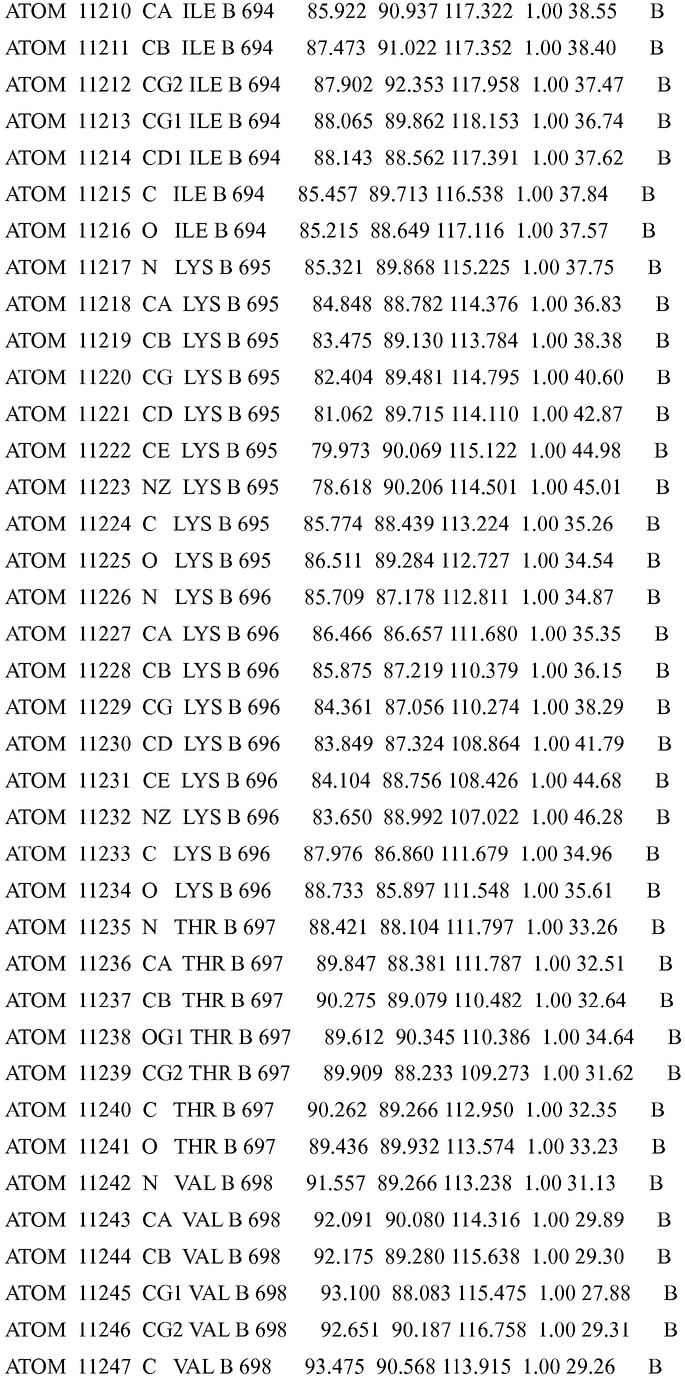

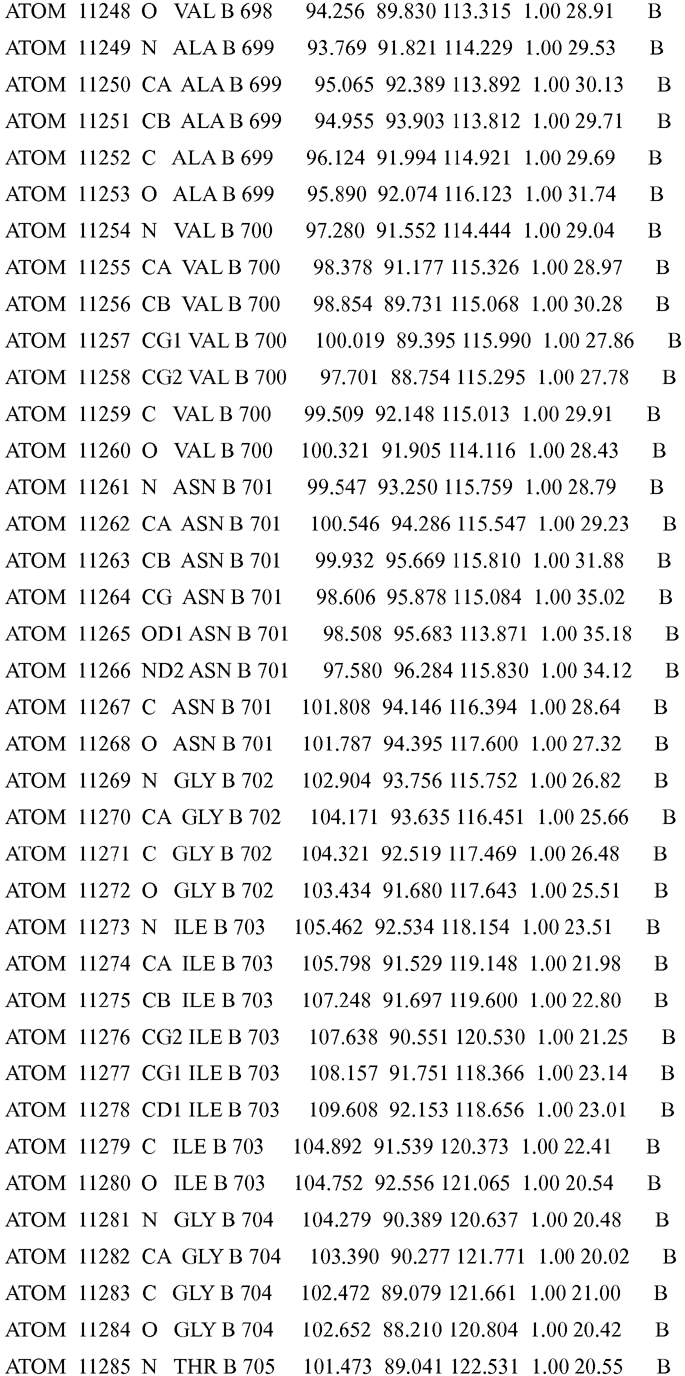

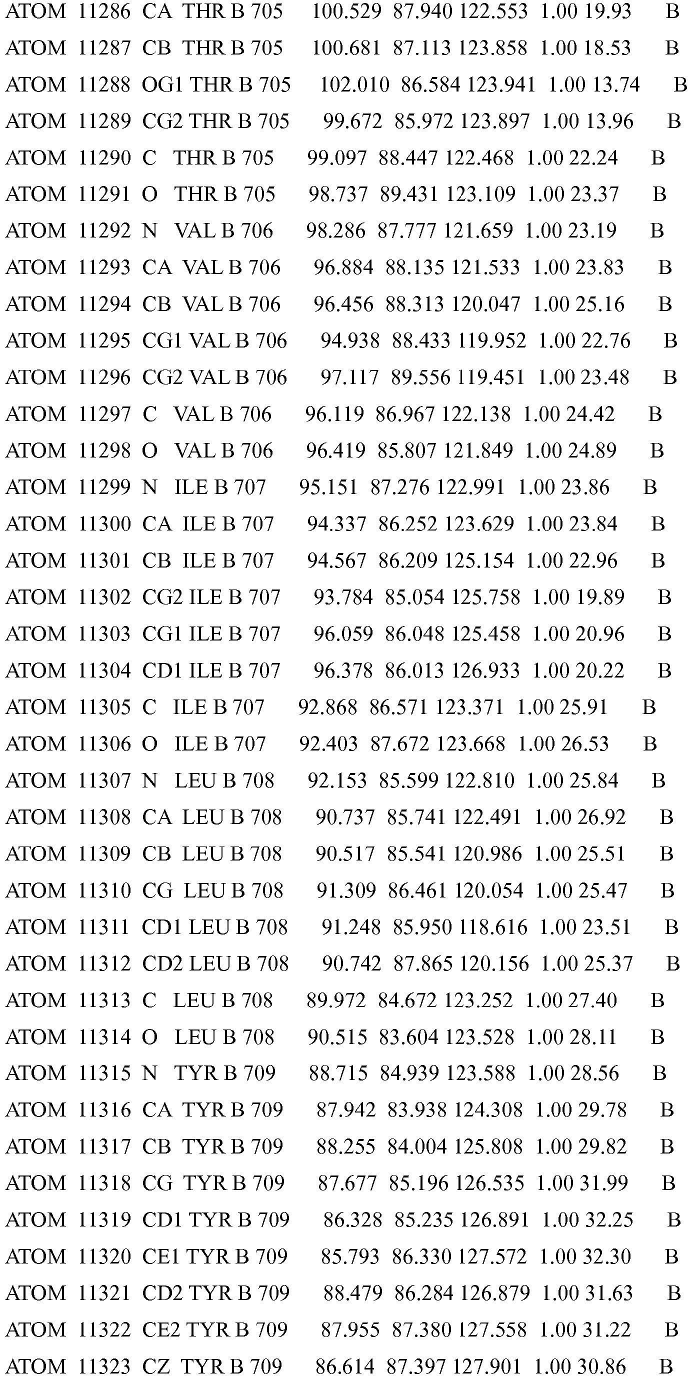

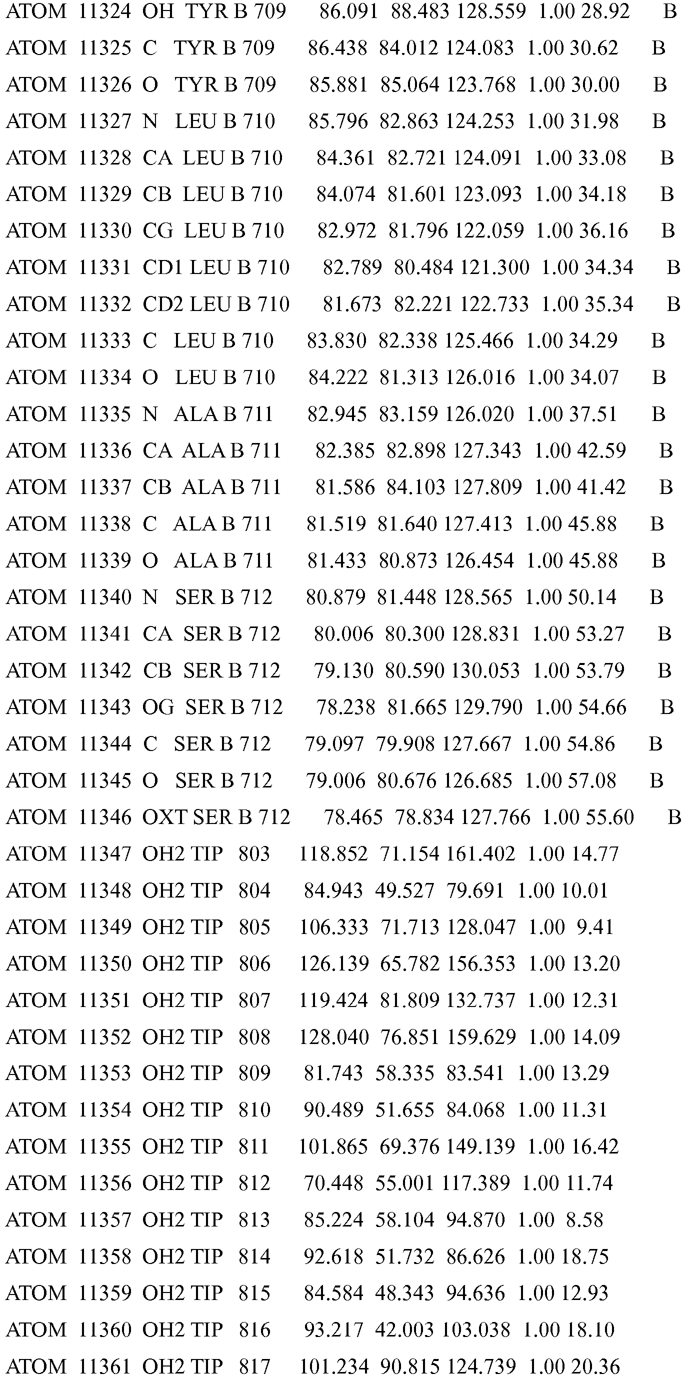

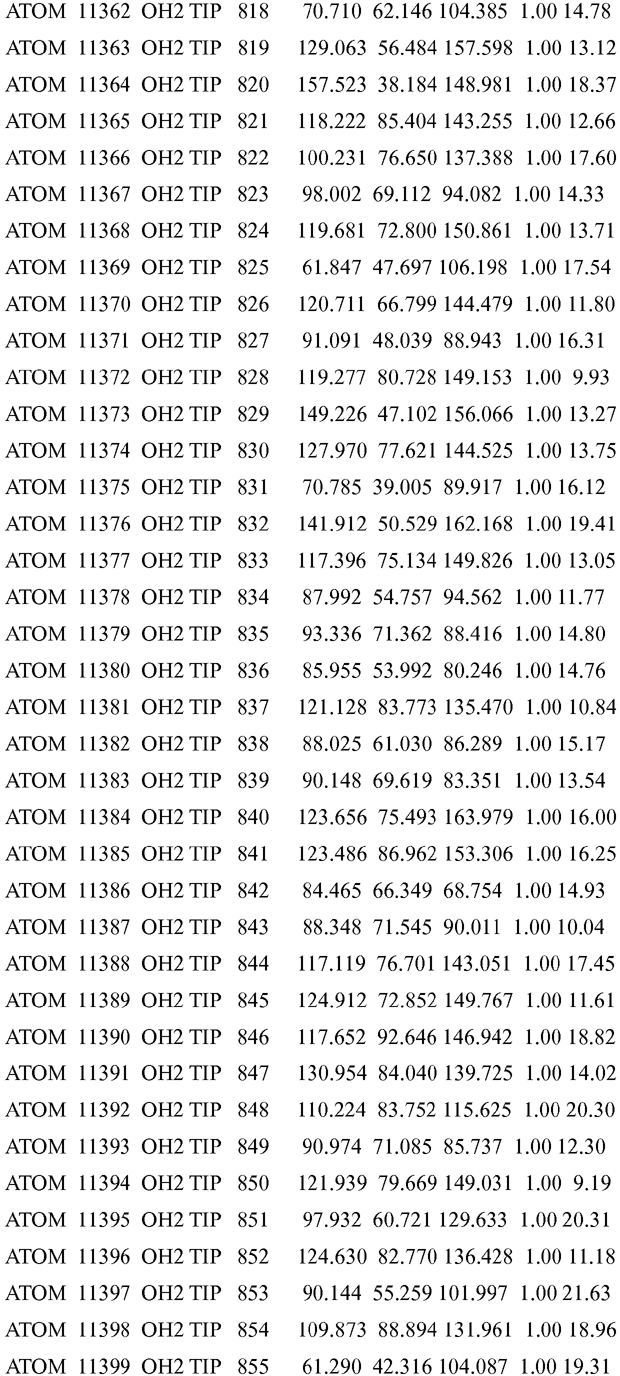

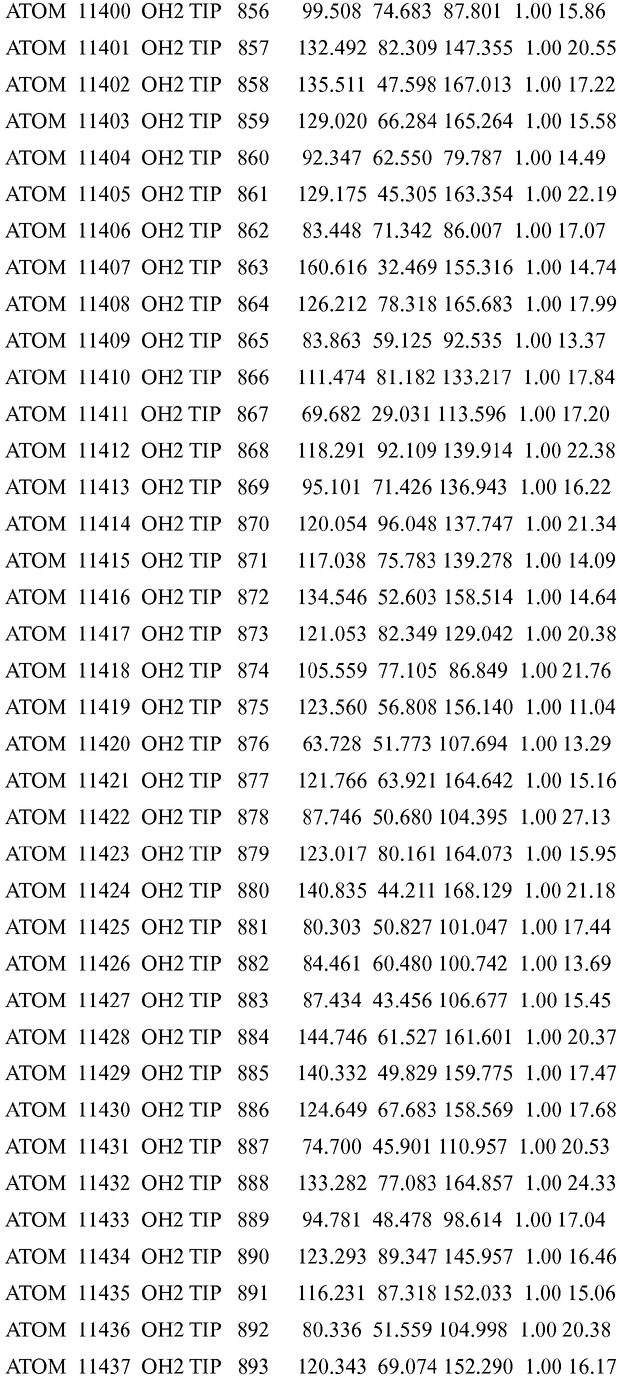

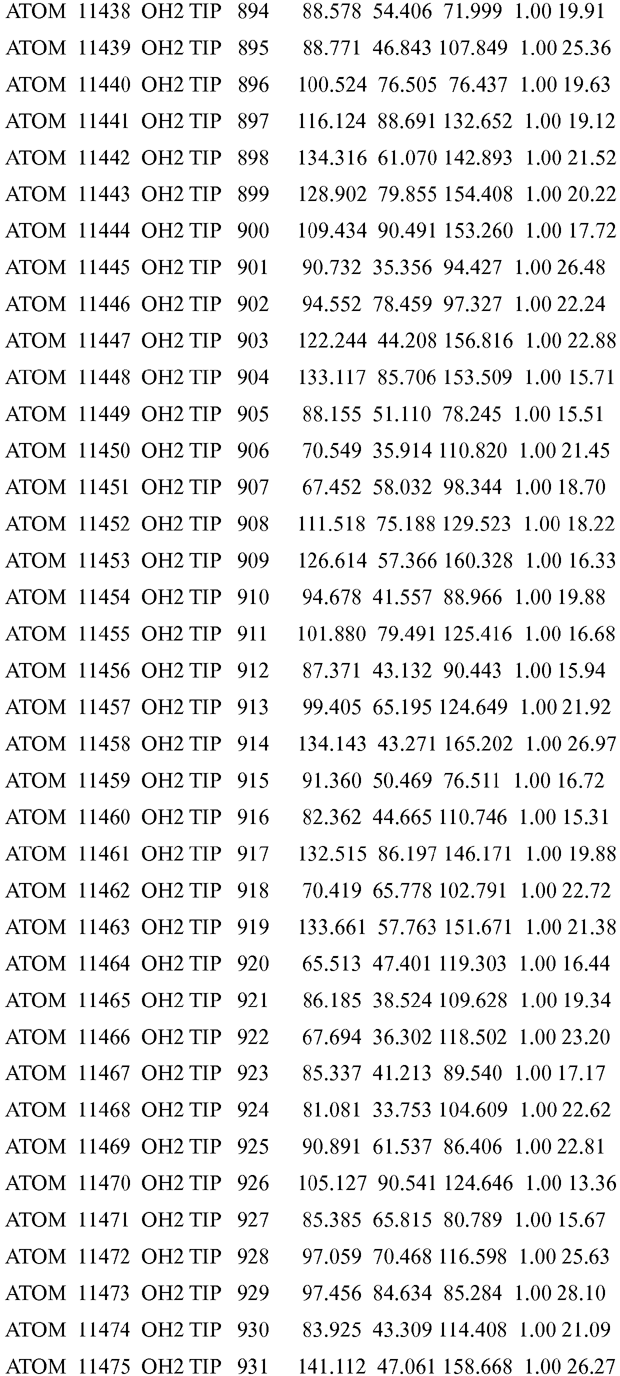

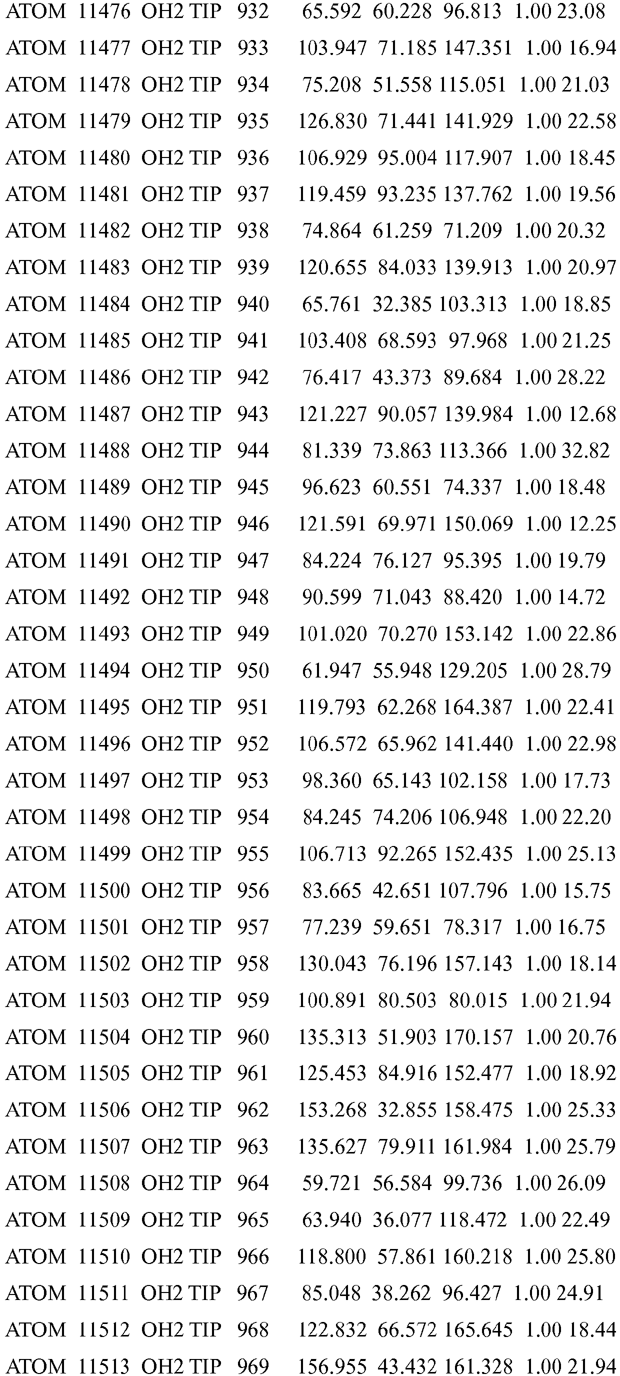

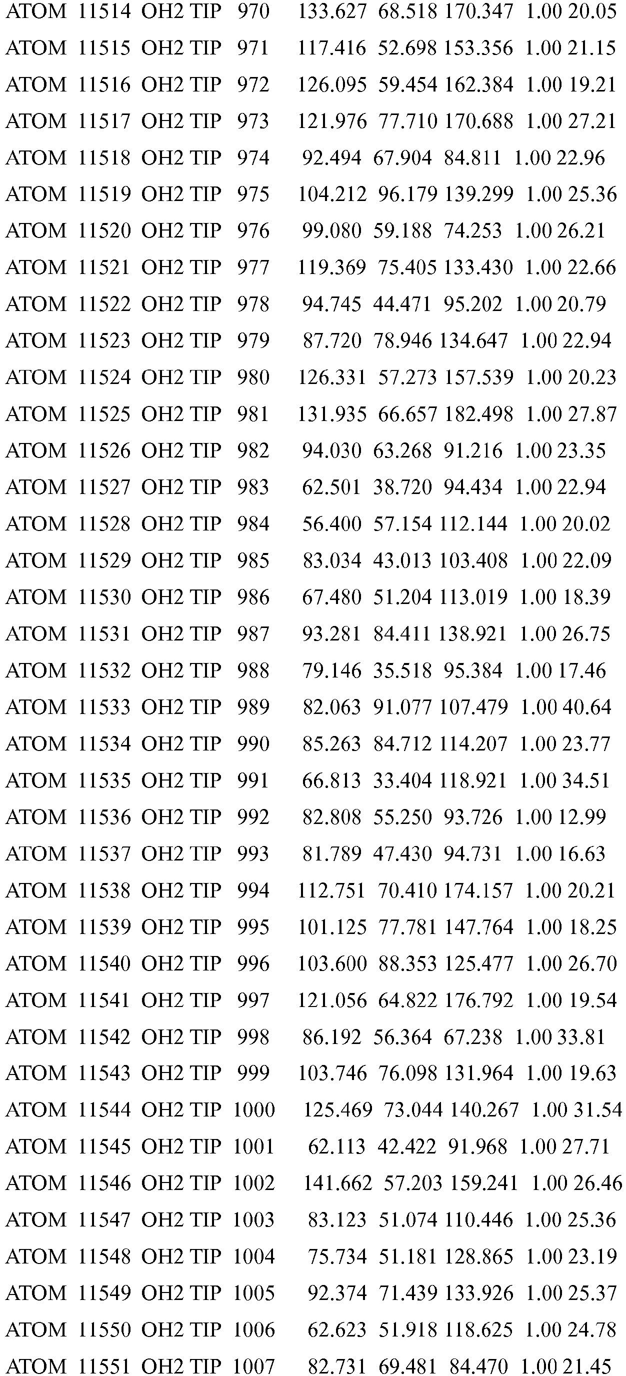

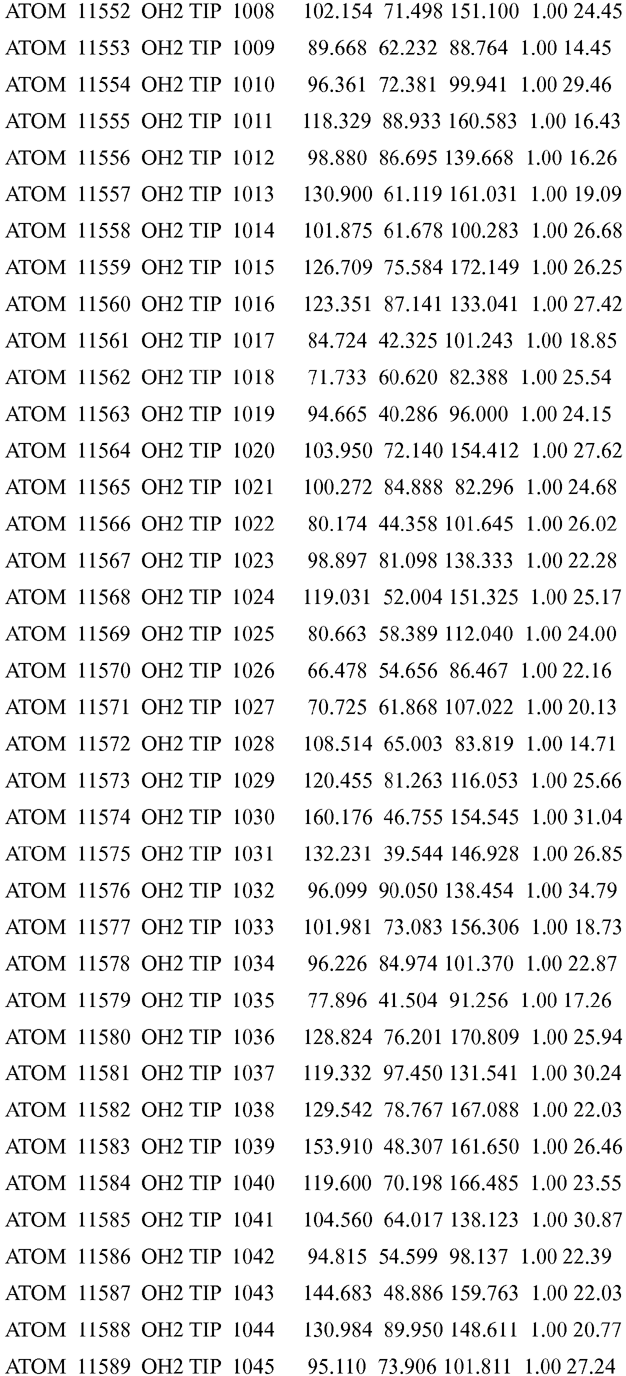

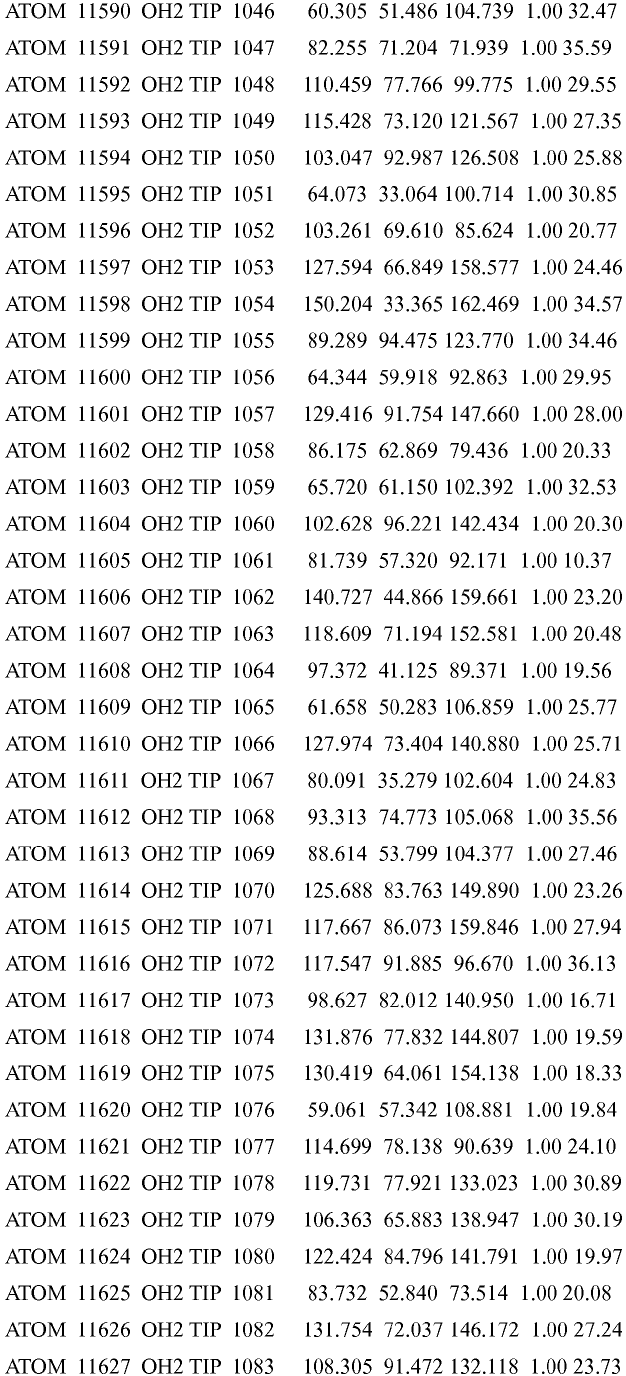

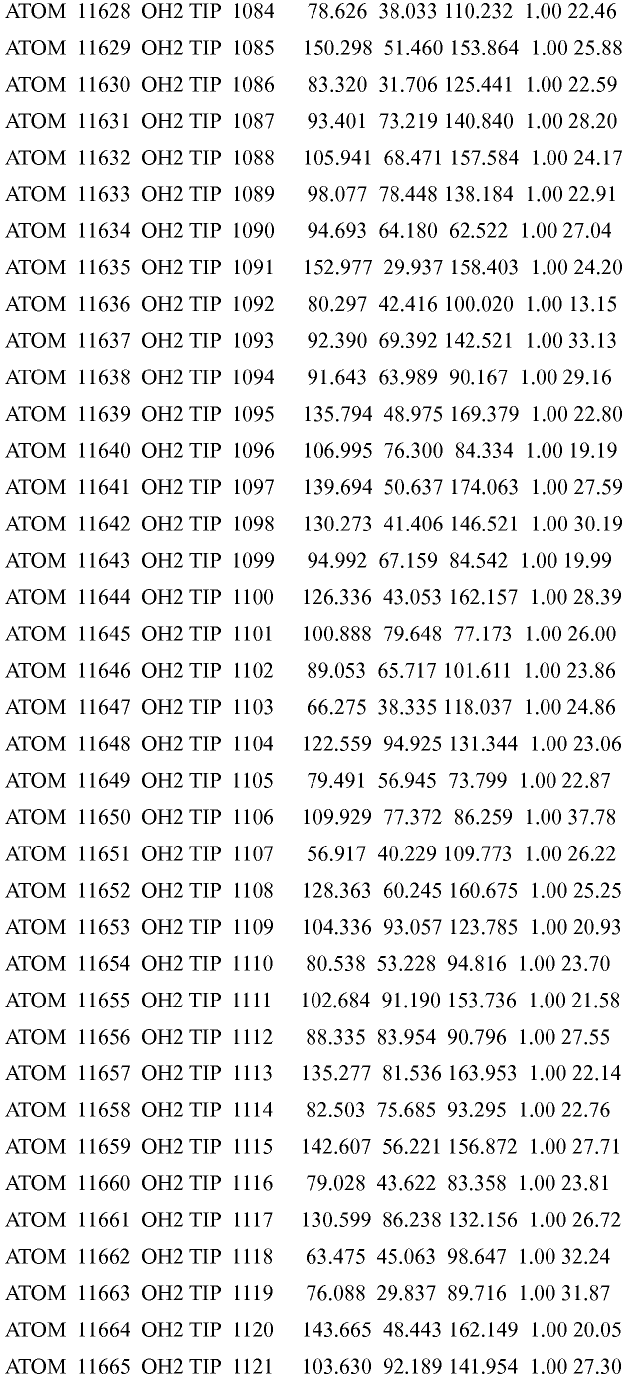

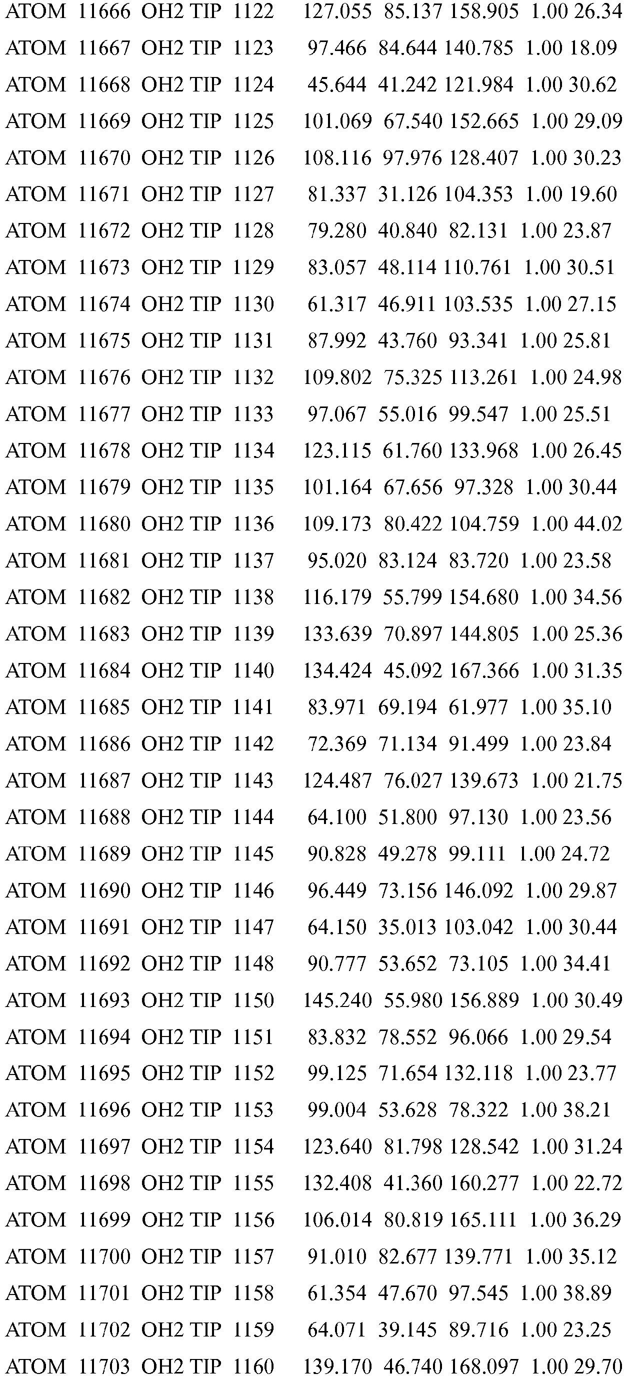

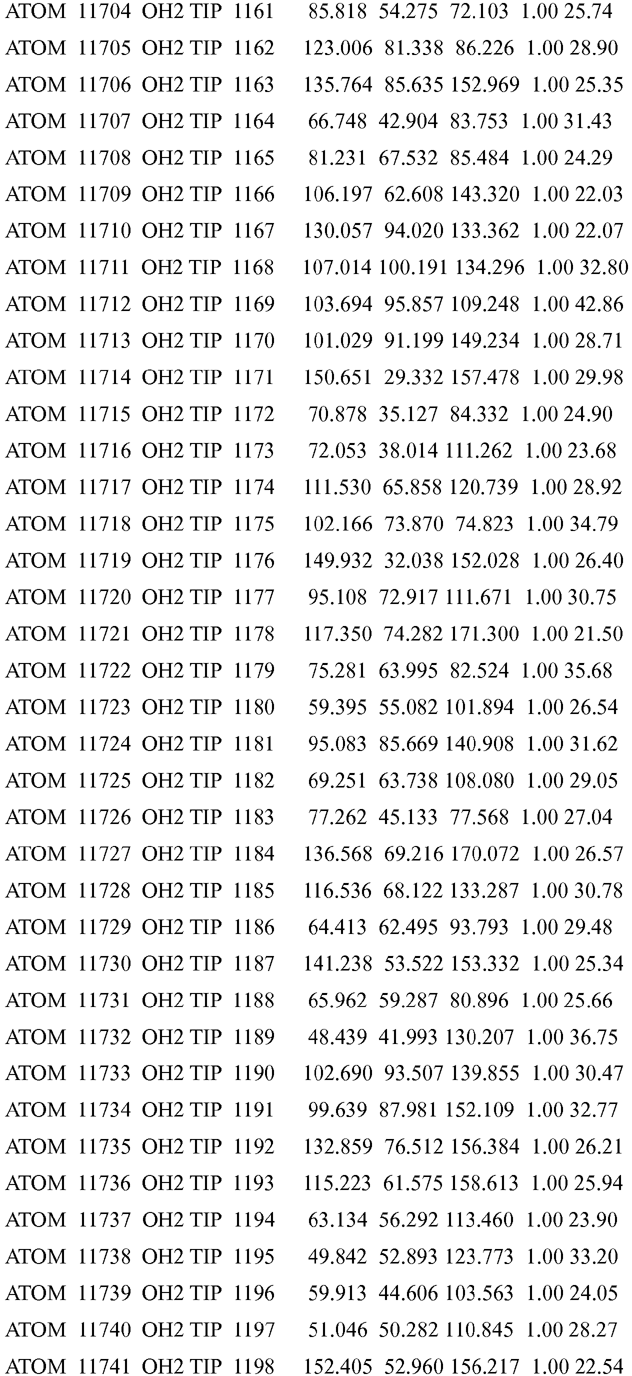

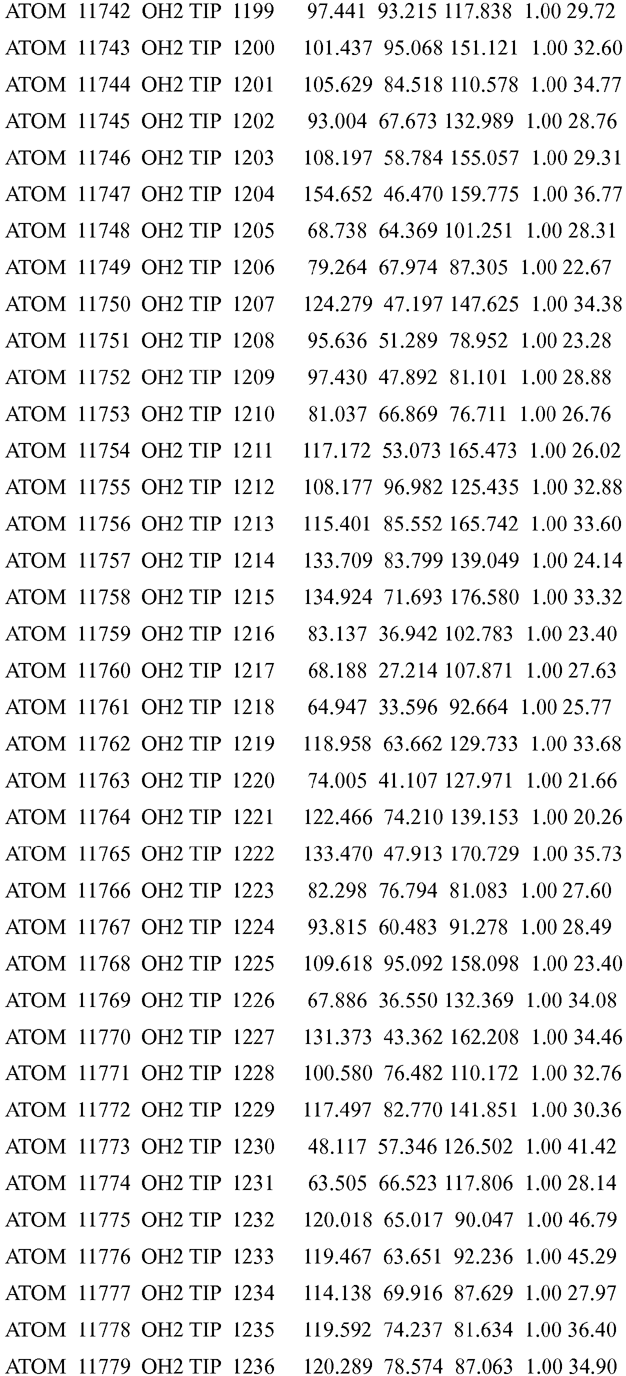

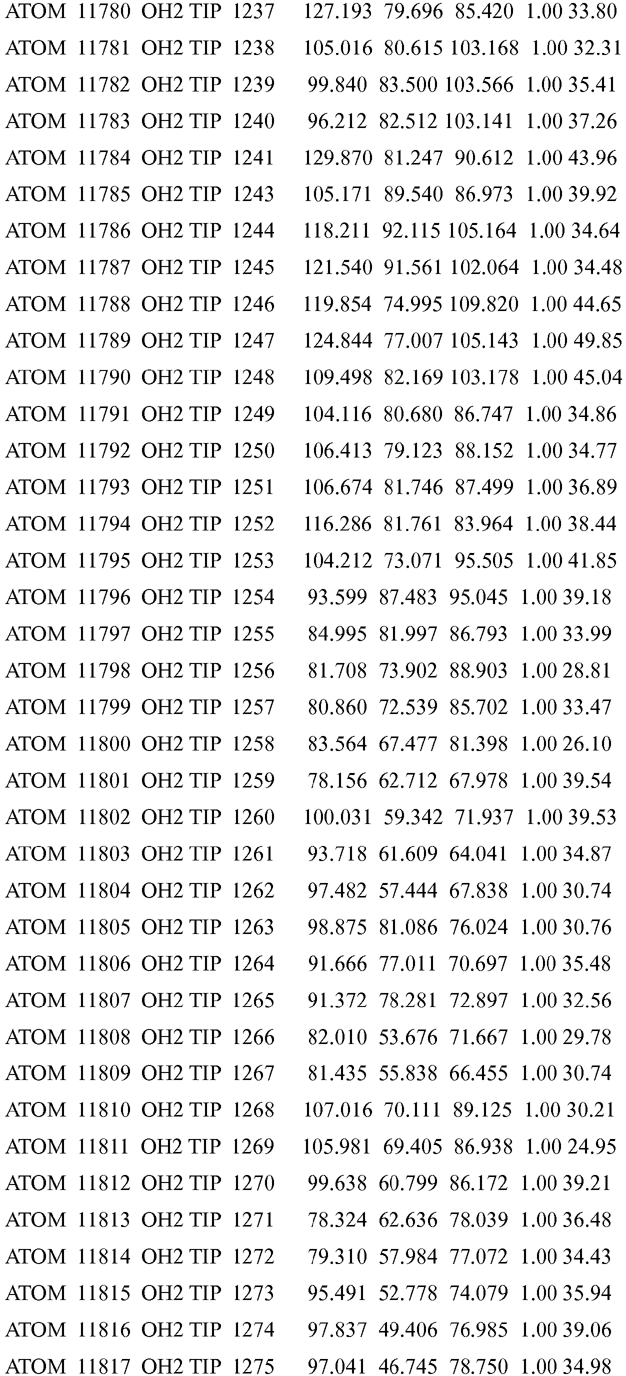

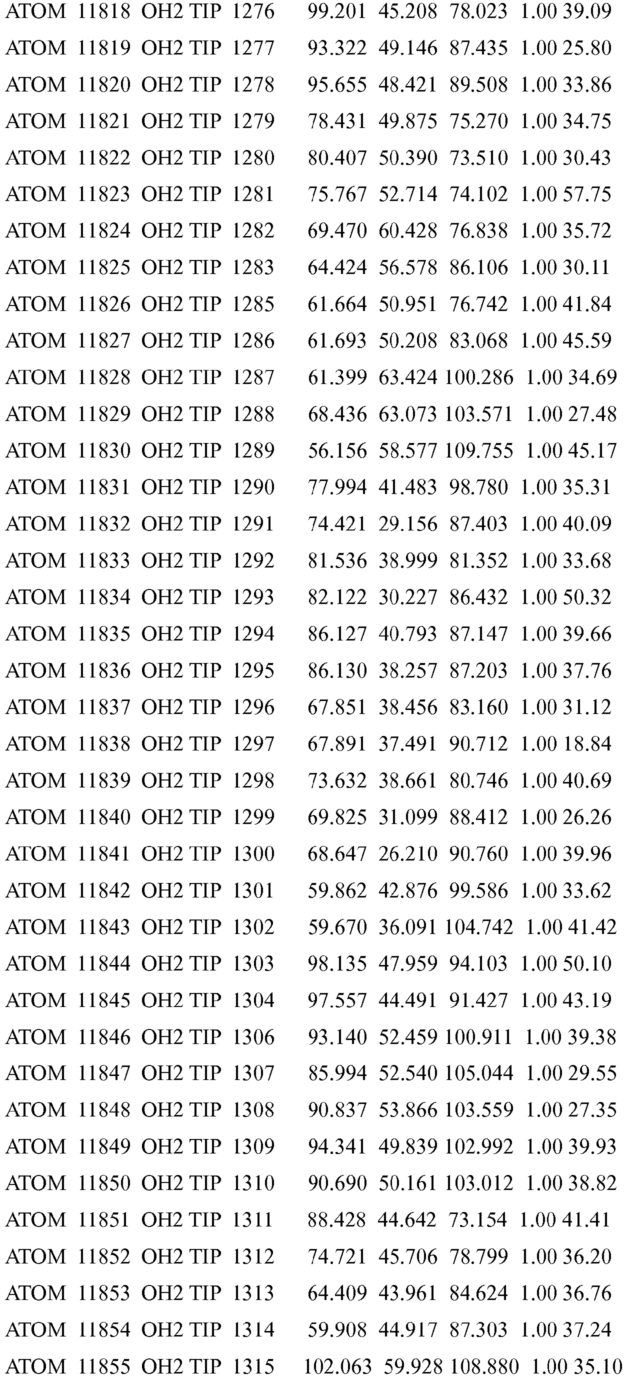

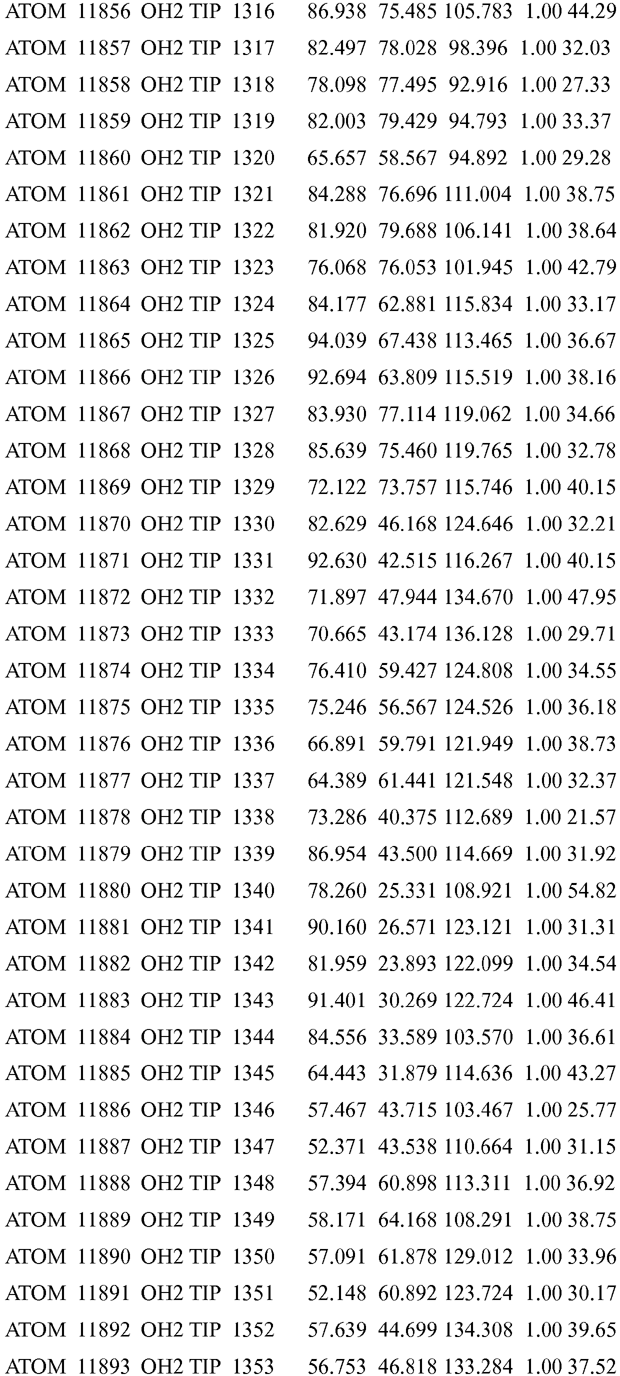

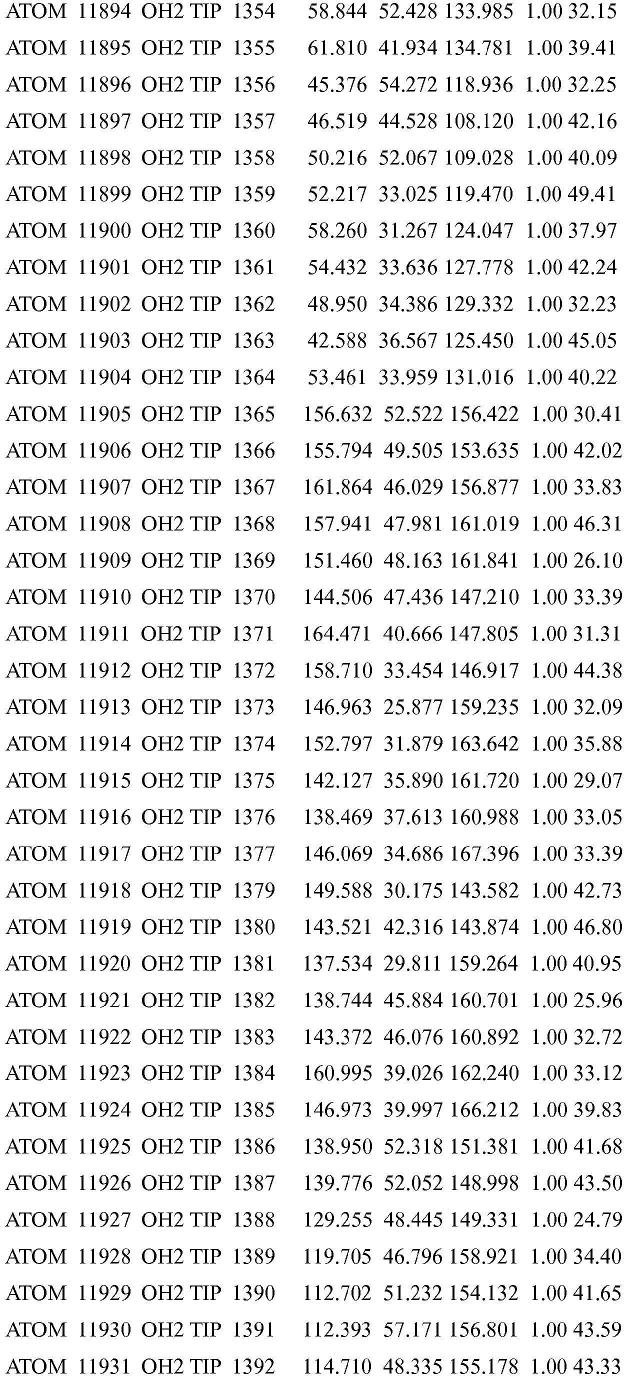

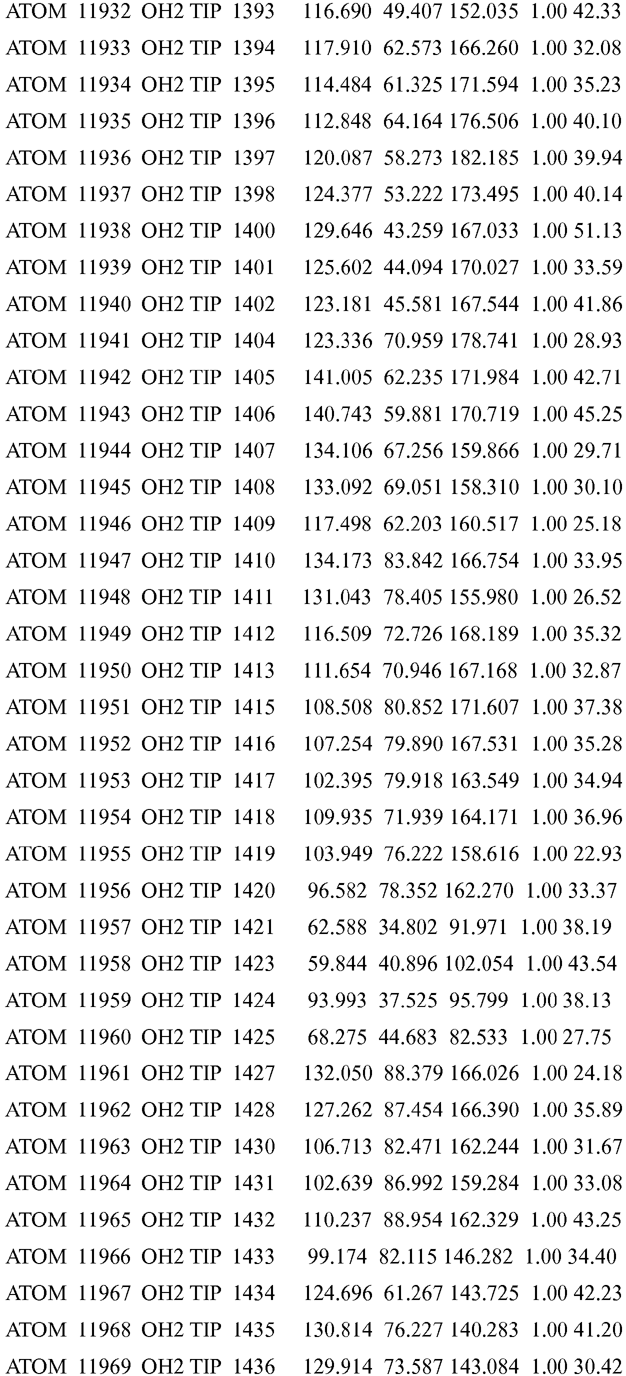

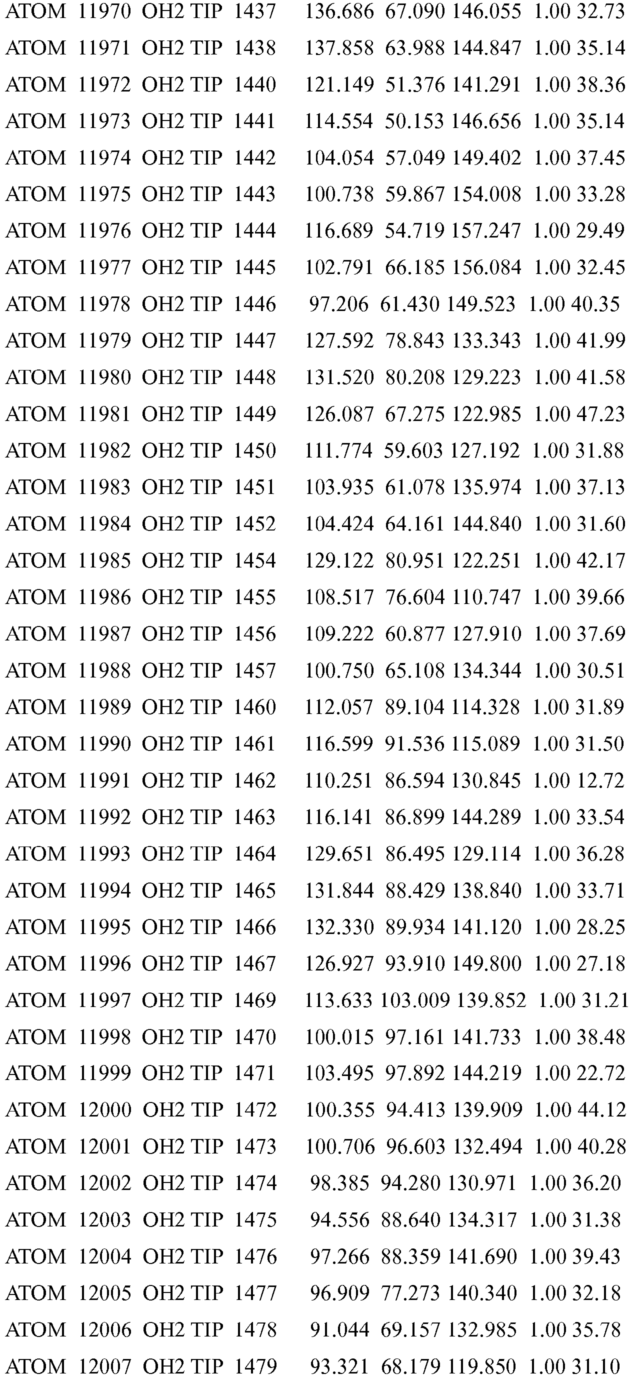

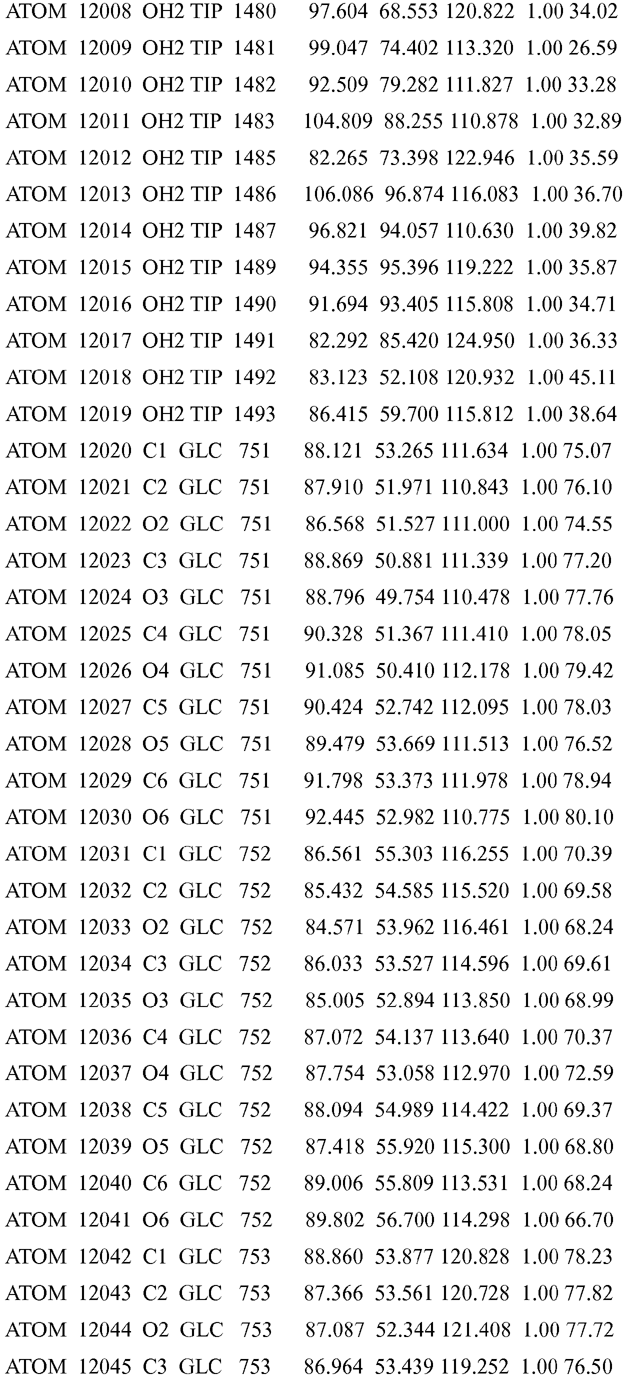

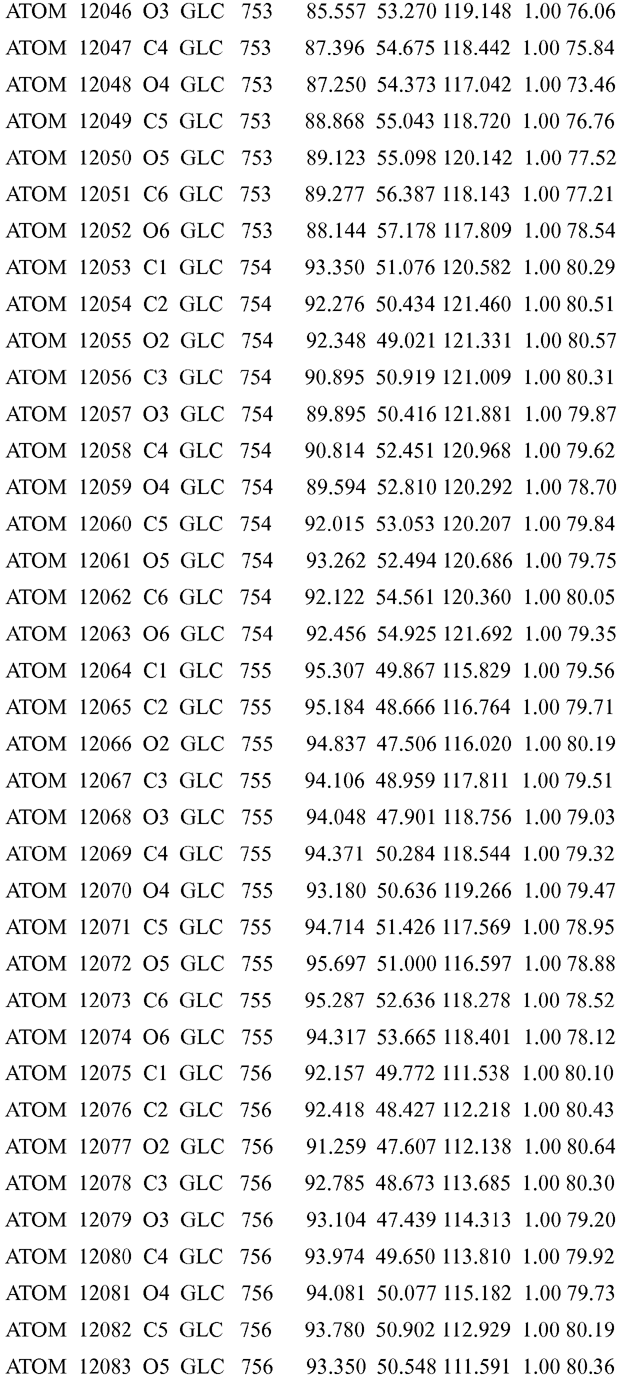

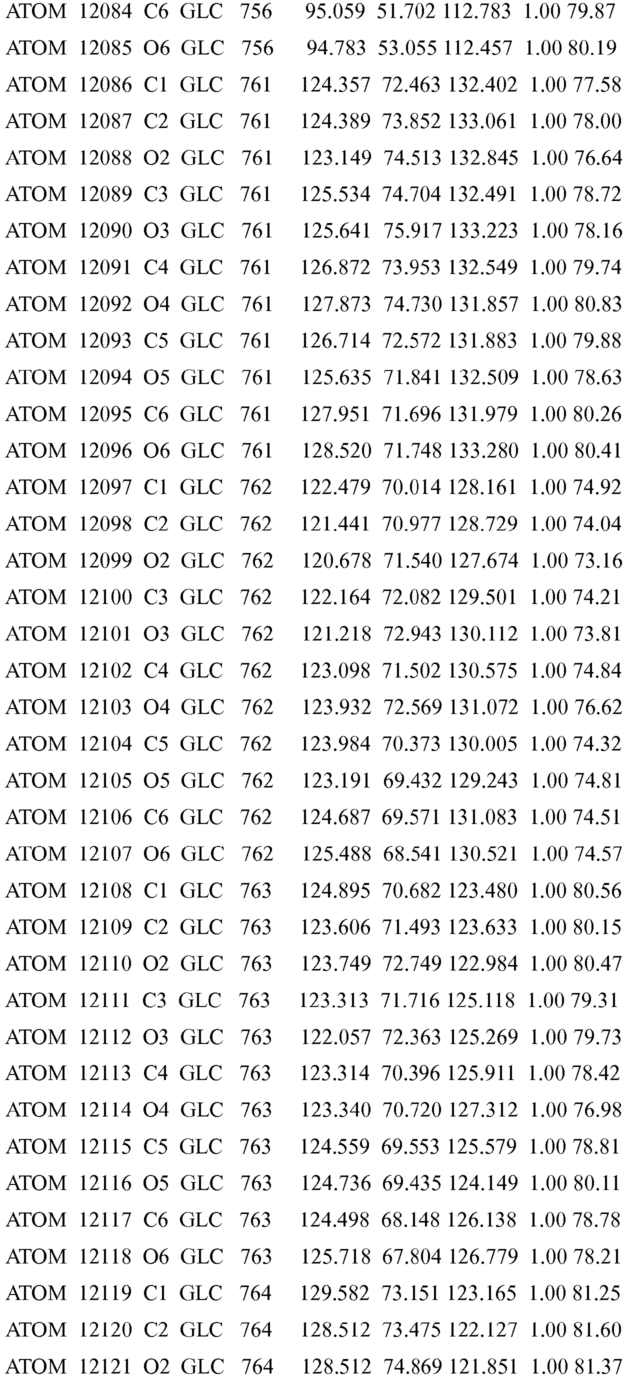

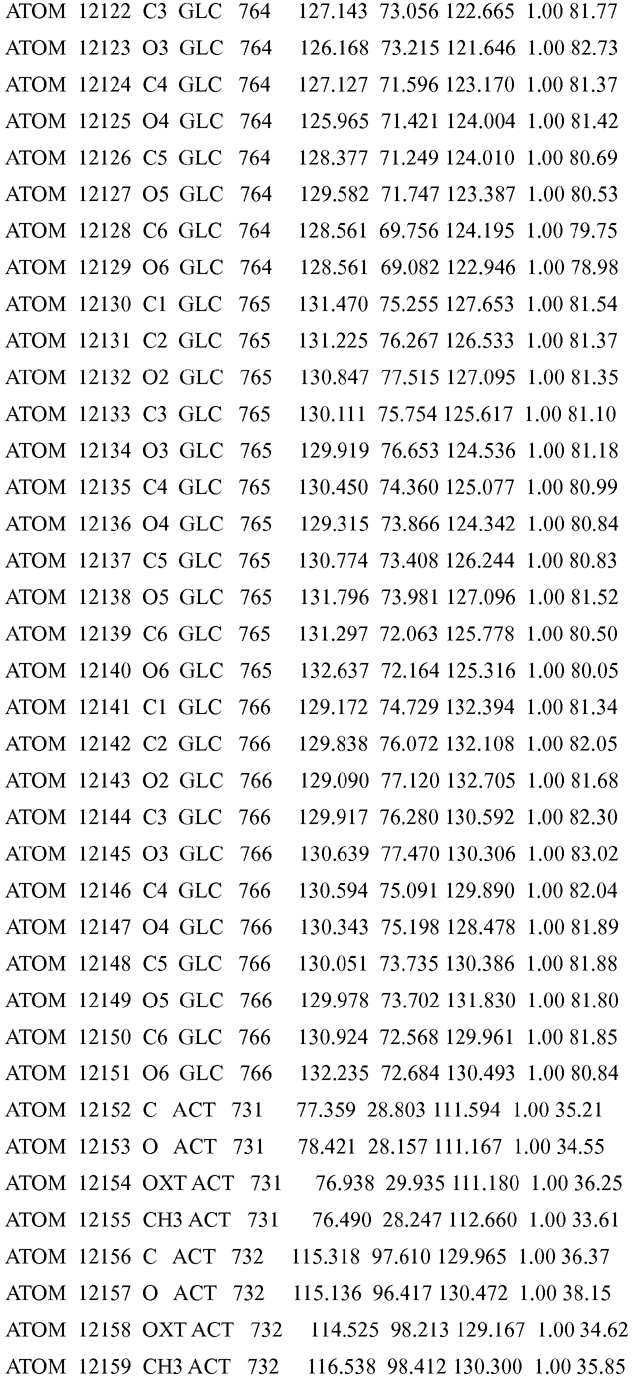

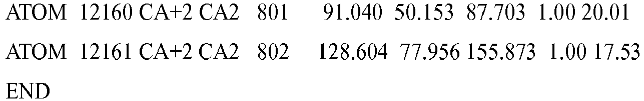

## Claims

1. A mutated enzyme, wherein the activity and/or substrate specificity in terms of its Km value and/or Kcat value for hydrolyzing an α-1,6-glycosidic linkage with respect to pullulan or amylopectin in 50 mM acetate buffer at pH 5.6 at 25 °C is improved as compared with the enzyme to be mutated, comprising an amino acid sequence in which one or two or more amino acids selected from the group shown below in an amino acid sequence of an enzyme (enzyme to be mutated) that hydrolyzes an α-1,6-glycosidic linkage, the group consisting of an amino acid in the substrate binding site of the enzyme and involved in binding of the substrate, which corresponds to an amino acid at the 476 position, an amino acid corresponding to an amino acid at the 292 position, an amino acid corresponding to an amino acid at the 371 position, an amino acid corresponding to an amino acid at the 406 position, an amino acid corresponding to an amino acid at the 407 position, an amino acid corresponding to an amino acid at the 437 position, an amino acid corresponding to an amino acid at the 465 position, an amino acid corresponding to an amino acid at the 475 position, an amino acid corresponding to an amino acid at the 525 position, an amino acid corresponding to an amino acid at the 526 position, an amino acid corresponding to an amino acid at the 580 position and an amino acid corresponding to an amino acid at the 582 position of the amino acid sequence set forth in SEQ ID NO: 2 is substituted with another amino acid or deleted, wherein the amino acid sequence of the enzyme to be mutated is the amino acid sequence set forth in SEQ ID NO: 13 and wherein the mutated enzyme differs from the parent enzyme in less than 10% with respect to the entire amino acid sequence.

2. The mutated enzyme according to claim 1, wherein the substituted or deleted amino acid is one or two or more amino acids selected from the group consisting of an amino acid corresponding to an amino acid at the 476 position, an amino acid corresponding to an amino acid at the 292 position, an amino acid corresponding to an amino acid at the 371 position, an amino acid corresponding to an amino acid at the 407 position, an amino acid corresponding to an amino acid at the 475 position, and an amino acid corresponding to an amino acid at the 582 position of the amino acid sequence set forth in SEQ ID NO: 2.

3. A gene encoding the mutated enzyme according to claim 1 or 2.

4. A recombinant DNA including the gene according to claim 3.

5. A microorganism carrying the recombinant DNA according to claim 4.

## Patentansprüche

1. Ein mutiertes Enzym, wobei die Aktivität und/oder Substratspezifizität in Bezug auf dessen Km-Wert und/oder Kcat-Wert zur Hydrolisierung einer α-1,6-glycosidischen Bindung in Bezug auf Pullulan oder Amylopektin in 50 mM Acetat-Puffer bei pH 5,6 bei 25 °C verbessert ist verglichen mit dem zu mutierenden Enzym, umfassend eine Aminosäuresequenz in welcher ein oder zwei oder mehrere Aminosäuren ausgewählt aus der Gruppe, wie gezeigt unten in einer Aminosäuresequenz eines Enzyms (zu mutierendes Enzym), das eine α-1,6-glycosidische Verbindung hydrolisiert, die Gruppe bestehend aus einer Aminosäure in der Substratbindungsstelle des Enzyms und involviert in Bindung des Substrats, welches einer Aminosäure an der Position 476 entspricht, eine Aminosäure entsprechend einer Aminosäure an der Position 292, eine Aminosäure entsprechend einer Aminosäure an der Position 371, eine Aminosäure entsprechend einer Aminosäure an der Position 406, eine Aminosäure entsprechend einer Aminosäure an der Position 407, eine Aminosäure entsprechend einer Aminosäure an der Position 437, eine Aminosäure entsprechend einer Aminosäure an der Position 465, eine Aminosäure entsprechend einer Aminosäure an der Position 475, eine Aminosäure entsprechend einer Aminosäure an der Position 525, eine Aminosäure entsprechend einer Aminosäure an der Position 526, eine Aminosäure entsprechend einer Aminosäure an der Position 580 und eine Aminosäure entsprechend einer Aminosäure an der Position 582 wie dargestellt in der Aminosäuresequenz SEQ ID NO: 2, substituiert mit einer anderen Aminosäure oder deletiert ist, wobei die Aminosäuresequenz des zu mutierenden Enzyms eine Aminosäuresequenz wie in SEQ ID NO: 13 dargestellt ist und wobei das mutierte Enzym sich unterscheidet von dem Vorläuferenzym in weniger als 10% in Bezug auf die ganze Aminosäuresequenz.

2. Mutiertes Enzym gemäß Anspruch 1, wobei die substituierte oder deletierte Aminosäure ist ein oder zwei oder mehrere Aminosäuren ausgewählt aus der Gruppe bestehend aus einer Aminosäure entsprechend einer Aminosäure an der Position 476, einer Aminosäure entsprechend einer Aminosäure an der Position 292, eine Aminosäure entsprechend einer Aminosäure an der Position 371, einer Aminosäure entsprechend einer Aminosäure an der Position 407, einer Aminosäure entsprechend einer Aminosäure an der Position 475 und einer Aminosäure entsprechend einer Aminosäure an der Position 582 der Aminosäuresequenz wie dargestellt in SEQ ID NO: 2.

3. Ein Gen kodierend das mutierte Enzym gemäß Anspruch 1 oder 2.

4. Eine rekombinante DNA enthaltend das Gen gemäß Anspruch 3.

5. Ein Mikroorganismus enthaltend die rekombinante DNA gemäß Anspruch 4.

## Revendications

1. Enzyme mutée, où l'activité et/ou la spécificité de substrat en termes de sa valeur Km et/ou de sa valeur Kcat pour l'hydrolyse d'une liaison α-1,6-glycosidique concernant le pullulane ou l'amylopectine dans un tampon acétate 50 mM à pH 5,6 à 25°C est améliorée comparée à l'enzyme destinée à être mutée, comprenant une séquence d'acides aminés dans laquelle un ou deux ou plusieurs acides aminés choisis dans le groupe montré ci-dessous dans une séquence d'acides aminés d'une enzyme (enzyme destinée à être mutée) qui hydrolyse une liaison α-1,6-glycosidique, le groupe consistant en un acide aminé dans le site de liaison de substrat de l'enzyme et impliqué dans la liaison du substrat, qui correspond à un acide aminé à la position 476, un acide aminé correspondant à un acide aminé à la position 292, un acide aminé correspondant à un acide aminé à la position 371, un acide aminé correspondant à un acide aminé à la position 406, un acide aminé correspondant à un acide aminé à la position 407, un acide aminé correspondant à un acide aminé à la position 437, un acide aminé correspondant à un acide aminé à la position 465, un acide aminé correspondant à un acide aminé à la position 475, un acide aminé correspondant à un acide aminé à la position 525, un acide aminé correspondant à un acide aminé à la position 526, un acide aminé correspondant à un acide aminé à la position 580 et un acide aminé correspondant à un acide aminé à la position 582 de la séquence d'acides aminés présentée dans SEQ ID NO: 2 est substitué avec un autre acide aminé ou délété, où la séquence d'acides aminés de l'enzyme destinée à être mutée est la séquence d'acides aminés présentée dans SEQ ID NO: 13 et où l'enzyme mutée diffère de l'enzyme mère de moins de 10 % concernant la séquence d'acides aminés entière.

2. Enzyme mutée selon la revendication 1, où l'acide aminé substitué ou délété est un ou deux ou plusieurs acides aminés choisis dans le groupe consistant en un acide aminé correspondant à un acide aminé à la position 476, un acide aminé correspondant à un acide aminé à la position 292, un acide aminé correspondant à un acide aminé à la position 371, un acide aminé correspondant à un acide aminé à la position 407, un acide aminé correspondant à un acide aminé à la position 475, et un acide aminé correspondant à un acide aminé à la position 582 de la séquence d'acides aminés présentée dans SEQ ID NO: 2.

3. Gène codant l'enzyme mutée selon la revendication 1 ou 2.

4. ADN recombinant incluant le gène selon la revendication 3.

5. Micro-organisme portant l'ADN recombinant selon la revendication 4.


### SEQUENCE LISTING

<110> AMANO ENZYME INC.
   MIKAMI, Bunzo
   IWAMOTO, Hiroyuki
   YAMAGUCHI, Shotaro
<120> METHODS OF DESIGNING MUTANT ENZYMES, METHODS OF PREPARING THE SAME, AND MUTANT ENZYMES
<130> P06081PE
<140> 07 767 790.4
   <141> 2007-06-28
<150> JP P2006-213490
   <151> 2006-08-04
<160> 16
<170> PatentIn version 3.5
<210> 1
   <211> 2233
   <212> DNA
   <213> Bacillus subtilis
<400> 1
   atggtcagca tccgccgcag cttcgaagcg tatgtcgatg acatgaatat cattactgtt      60
   ctgattcctg ctgaacaaaa ggaaatcatg acaccgccgt ttcggcttga gacagaaata     120
   acagattttc ctctggctgt cagggaggaa tactcccttg aagcaaaata caagtacgtc     180
   tgcgtatccg accatcctgt gacatttgga aaaatccatt gcgtcagagc atccagcggc     240
   cacaaaacgg atctccaaat tggcgcggtc atccggacgg cagcgtttga tgacgaattt     300
   tattatgacg gagagctggg cgccgtttat accgcggatc ataccgtatt taaagtatgg     360
   gcgcctgctg caacctcagc tgctgtcaag ctttcacacc ccaataaaag cgggcgcaca     420
   ttccaaatga ctcgcttgga aaaaggcgtc tatgccgtta cggtcacagg tgaccttcac     480
   ggatatgagt atttgttttg catctgcaac aattcagaat ggatggaaac agttgaccag     540
   tatgccaagg ctgtgactgt aaatggagag aagggcgtcg tcttgcgccc ggatcaaatg     600
   aaatggactg ctcctcttaa accattctca caccctgtgg atgccgtcat ctatgagacg     660
   catcttcgcg acttctccat ccatgaaaac agcggcatga taaacaaggg aaaatactta     720
   gcgctgacgg aaactgatac acaaaccgca aatggcagtt cttcgggatt agcgtatgta     780
   aaagagcttg gtgtgacaca tgtggagctt ctgccggtga atgattttgc cggagttgat     840
   gaagagaagc cgcttgatgc atacaattgg ggatataacc cgcttcattt ctttgccccg     900
   gagggaagct atgcctcaaa tcctcatgat cctcaaacga gaaaaacaga gctgaaacaa     960
   atgatcaata ccctgcatca gcacggtctg cgagtcattc tggatgttgt ttttaaccat    1020
   gtgtataaga gggagaattc cccctttgaa aagacagtgc ccggttattt tttccggcac    1080
   gacgaatgtg ggatgccatc aaacggcacc ggcgttggca atgatattgc atcagaaaga 1140
   aggatggcaa gaaaattcat tgcggattgc gtggtctatt ggcttgaaga atacaatgtt 1200
   gacggcttcc gctttgatct cctcgggatt ttagatattg acaccgtgct ttatatgaaa 1260
   gagaaagcaa ctaaggcaaa gcccggaatc ctgctttttg gagaagggtg ggacctggct 1320
   acaccgctgc cgcatgaaca gaaagctgct ttggcgaacg cgccaagaat gccgggcatc 1380
   ggctttttta atgatatgtt tcgtgacgct gtaaaaggga acacctttca ccttaaggca 1440
   acagggtttg cgctcggcaa cggtgaatca gcacaagctg tgatgcatgg aattgccggg 1500
   tcttccggat ggaaggcatt agcaccgatt gttccggaac caagccagtc catcaattat 1560
   gtcgaatcac acgacaatca caccttttgg gataaaatga gctttgcgct tcctcaagaa 1620
   aatgacagcc gaaagcgaag caggcaaagg cttgcagtcg cgattatttt gcttgcccaa 1680
   ggggtgccgt ttattcacag cggccaggaa tttttccgga cgaagcaggg agtggaaaac 1740
   agctatcaat ccagtgacag catcaaccag ctcgactggg atcgccgtga aacattcaaa 1800
   gaagatgttc actatatccg caggctgatc tcgctgagaa aagcgcatcc tgcattccgt 1860
   cttaggtccg ctgcagacat ccagcgccat cttgaatgct tgacgctaaa agaacacctt 1920
   atcgcataca ggctttatga tcttgacgag gttgacgaat ggaaagatat cattgttatc 1980
   catcacgcga gtccagactc cgtcgagtgg aggctgccaa acgacatacc ttatcggctt 2040
   ttatgtgatc catcaggatt tcaggaagac ccaacagaaa tcaagaaaac ggttgcagta 2100
   aacggcatcg gaacggttat cttatattta gcatcagatc ttaagagttt tgcttgacga 2160
   agtttgtgct atagcgctaa aatttaaagg acggctattg attagcatat gagactctca 2220
   atagctgtct ttt 2233
<210> 2
   <211> 718
   <212> PRT
   <213> Bacillus subtilis
<400> 2
   Met Val Ser Ile Arg Arg Ser Phe Glu Ala Tyr Val Asp Asp Met Asn
   1 5 10 15
   Ile Ile Thr Val Leu Ile Pro Ala Glu Gln Lys Glu Ile Met Thr Pro
   20 25 30
   Pro Phe Arg Leu Glu Thr Glu Ile Thr Asp Phe Pro Leu Ala Val Arg
   35 40 45
   Glu Glu Tyr Ser Leu Glu Ala Lys Tyr Lys Tyr Val Cys Val Ser Asp
   50 55 60
   His Pro Val Thr Phe Gly Lys Ile His Cys Val Arg Ala Ser Ser Gly
   65                  70                  75                  80
   His Lys Thr Asp Leu Gln Ile Gly Ala Val Ile Arg Thr Ala Ala Phe
                   85                  90                  95
   Asp Asp Glu Phe Tyr Tyr Asp Gly Glu Leu Gly Ala Val Tyr Thr Ala
               100                 105                 110
   Asp His Thr Val Phe Lys Val Trp Ala Pro Ala Ala Thr Ser Ala Ala
           115                 120                 125
   Val Lys Leu Ser His Pro Asn Lys Ser Gly Arg Thr Phe Gln Met Thr
       130                 135                 140
   Arg Leu Glu Lys Gly Val Tyr Ala Val Thr Val Thr Gly Asp Leu His
   145                 150                 155                 160
   Gly Tyr Glu Tyr Leu Phe Cys Ile Cys Asn Asn Ser Glu Trp Met Glu
                   165                 170                 175
   Thr Val Asp Gln Tyr Ala Lys Ala Val Thr Val Asn Gly Glu Lys Gly
               180                 185                 190
   Val Val Leu Arg Pro Asp Gln Met Lys Trp Thr Ala Pro Leu Lys Pro
           195                 200                 205
   Phe Ser His Pro Val Asp Ala Val Ile Tyr Glu Thr His Leu Arg Asp
       210                 215                 220
   Phe Ser Ile His Glu Asn Ser Gly Met Ile Asn Lys Gly Lys Tyr Leu
   225                 230                 235                 240
   Ala Leu Thr Glu Thr Asp Thr Gln Thr Ala Asn Gly Ser Ser Ser Gly
                   245                 250                 255
   Leu Ala Tyr Val Lys Glu Leu Gly Val Thr His Val Glu Leu Leu Pro
               260                 265                 270
   Val Asn Asp Phe Ala Gly Val Asp Glu Glu Lys Pro Leu Asp Ala Tyr
           275                 280                 285
   Asn Trp Gly Tyr Asn Pro Leu His Phe Phe Ala Pro Glu Gly Ser Tyr
       290                 295                 300
   Ala Ser Asn Pro His Asp Pro Gln Thr Arg Lys Thr Glu Leu Lys Gln
   305 310 315 320
   Met Ile Asn Thr Leu His Gln His Gly Leu Arg Val Ile Leu Asp Val
   325 330 335
   Val Phe Asn His Val Tyr Lys Arg Glu Asn Ser Pro Phe Glu Lys Thr
   340 345 350
   Val Pro Gly Tyr Phe Phe Arg His Asp Glu Cys Gly Met Pro Ser Asn
   355 360 365
   Gly Thr Gly Val Gly Asn Asp Ile Ala Ser Glu Arg Arg Met Ala Arg
   370 375 380
   Lys Phe Ile Ala Asp Cys Val Val Tyr Trp Leu Glu Glu Tyr Asn Val
   385 390 395 400
   Asp Gly Phe Arg Phe Asp Leu Leu Gly Ile Leu Asp Ile Asp Thr Val
   405 410 415
   Leu Tyr Met Lys Glu Lys Ala Thr Lys Ala Lys Pro Gly Ile Leu Leu
   420 425 430
   Phe Gly Glu Gly Trp Asp Leu Ala Thr Pro Leu Pro His Glu Gln Lys
   435 440 445
   Ala Ala Leu Ala Asn Ala Pro Arg Met Pro Gly Ile Gly Phe Phe Asn
   450 455 460
   Asp Met Phe Arg Asp Ala Val Lys Gly Asn Thr Phe His Leu Lys Ala
   465 470 475 480
   Thr Gly Phe Ala Leu Gly Asn Gly Glu Ser Ala Gln Ala Val Met His
   485 490 495
   Gly Ile Ala Gly Ser Ser Gly Trp Lys Ala Leu Ala Pro Ile Val Pro
   500 505 510
   Glu Pro Ser Gln Ser Ile Asn Tyr Val Glu Ser His Asp Asn His Thr
   515 520 525
   Phe Trp Asp Lys Met Ser Phe Ala Leu Pro Gln Glu Asn Asp Ser Arg
   530 535 540
   Lys Arg Ser Arg Gln Arg Leu Ala Val Ala Ile Ile Leu Leu Ala Gln
   545 550 555 560
   Gly Val Pro Phe Ile His Ser Gly Gln Glu Phe Phe Arg Thr Lys Gln
                   565                 570                 575
   Gly Val Glu Asn Ser Tyr Gln Ser Ser Asp Ser Ile Asn Gln Leu Asp
               580                 585                 590
   Trp Asp Arg Arg Glu Thr Phe Lys Glu Asp Val His Tyr Ile Arg Arg
           595                 600                 605
   Leu Ile Ser Leu Arg Lys Ala His Pro Ala Phe Arg Leu Arg Ser Ala
       610                 615                 620
   Ala Asp Ile Gln Arg His Leu Glu Cys Leu Thr Leu Lys Glu His Leu
   625                 630                 635                 640
   Ile Ala Tyr Arg Leu Tyr Asp Leu Asp Glu Val Asp Glu Trp Lys Asp
                   645                 650                 655
   Ile Ile Val Ile His His Ala Ser Pro Asp Ser Val Glu Trp Arg Leu
               660                 665                 670
   Pro Asn Asp Ile Pro Tyr Arg Leu Leu Cys Asp Pro Ser Gly Phe Gln
           675                 680                 685
   Glu Asp Pro Thr Glu Ile Lys Lys Thr Val Ala Val Asn Gly Ile Gly
       690                 695                 700
   Thr Val Ile Leu Tyr Leu Ala Ser Asp Leu Lys Ser Phe Ala
   705                 710                 715
<210> 3
   <211> 30
   <212> DNA
   <213> Artificial
<220>
   <223> PCR primer
<400> 3
   ggccatggtc agcatccgcc gcagcttcga 30
<210> 4
   <211> 30
   <212> DNA
   <213> Artificial
<220>
   <223> PCR primer
<400> 4
   ggctcgagtc aagcaaaact cttaagatct 30
<210> 5
   <211> 33
   <212> DNA
   <213> Artificial
<220>
   <223> PCR primer
<400> 5
   gtaaaaggga acaccggtca ccttaaggca ata 33
<210> 6
   <211> 33
   <212> DNA
   <213> Artificial
<220>
   <223> PCR primer
<400> 6
   gtaaaaggga acacctctca ccttaaggca ata 33
<210> 7
   <211> 20
   <212> DNA
   <213> Artificial
<220>
   <223> PCR primer
<400> 7
   ttattgccgg agagtggcga 20
<210> 8
   <211> 20
   <212> DNA
   <213> Artificial
<220>
   <223> PCR primer
<400> 8
   ccagactgct gacaaagtgc 20
<210> 9
   <211> 34
   <212> DNA
   <213> Artificial
<220>
   <223> PCR primer
<400> 9
   ggaattccat atggatgtcg tcgtccgctt accg 34
<210> 10
   <211> 29
   <212> DNA
   <213> Artificial
<220>
   <223> PCR primer
<400> 10
   gctctagatt atttactgct caccggcag 29
<210> 11
   <211> 19
   <212> DNA
   <213> Artificial
<220>
   <223> PCR primer
<400> 11
   gccggccgac tccggtgac 19
<210> 12
   <211> 3664
   <212> DNA
   <213> Klebsiella pneumoniae
<400> 12
   tggcgataag cgtggcgaat cataatcagt ttgcggtttc gggtcgtcat tccctttatt      60
   gatttactcc cccggcttcc tacgcccccc gctcatttgt gggggatgat tgcgcctggg     120
   aaagcaaaaa tatctaatta gcgcgctgta aagatattca tcctttctta ccttttataa     180
   tttaataagc atattaggga ctatcgaatg ctcagatata cctgtcatgc cctatttctt     240
   ggatcgttag tattattgag tggctgtgat aacagctctt cctcttctac ctctggctca     300
   ccgggttcac caggcaatcc tggcaaccca ggcactcccg gcacgcccga cccgcaggat     360
   gtcgtcgtcc gcttaccgga cgttgccgtc ccaggcgaag cggtgcaggc ttccgccagg     420
   caggctgtca ttcatctcgt cgatatcgcc ggcatcacca gcagcacgcc ggccgactat     480
   gcgacgaaaa acctctattt atggaacaac gaaacctgtg acgcgctgag cgcgccggtg     540
   gcggactgga atgatgtcag caccaccccg accggcagcg acaaatatgg cccttattgg     600
   gtgatcccgc tgactaaaga gagcggatgc atcaacgtta tcgtccgcga tggcaccaat     660
   aagcttatcg acagcgacct gcgcgtctct ttcagtgatt tcaccgatcg gacggtatcg     720
   gtcatcgccg gcaacagcgc ggtctatgac tcccgcgccg acgccttccg cgccgccttt     780
   ggcgtggcgc tggccgatgc gcactgggtc gataagacta ctctgctgtg gccgggtggg     840
   gaaaataaac ccattgtgcg cctctattac agccacagca gtaaggtggc cgccgacagt     900
   aacggcgaat ttagcgataa atatgtcaag ctgaccccca ccaccgtcag ccagcaggta     960
   agcatgcgct tcccgcatct cgccagctat ccagccttta aactgccgga tgatgttaac    1020
   gtcgatgaat tgctgcaggg cgagacggtg gcgatagccg cggaaagcga tgggatcctg    1080
   agctcagcca cccaggtgca gaccgccggc gtgctggacg atacctatgc cgccgccgcc 1140
   gaggcgctga gctatggcgc ccagctaacc gatagcggcg tgaccttccg cgtctgggcg 1200
   cccacggcgc agcaggtcga gctggtgatc tatagcgcgg acaagaaagt gatagccagt 1260
   cacccgatga cccgcgatag cgcctccggc gcctggtcct ggcagggggg aagcgacctg 1320
   aagggcgcct tctaccgcta cgcgatgacg gtctaccacc cgcagtcgcg taaagtcgag 1380
   cagtacgaag tgaccgatcc ctacgcccac agtttgtcga ccaactcgga gtacagccag 1440
   gtggtcgatc tcaacgacag cgcgctgaag ccggaaggct gggacgggct gacgatgccg 1500
   cacgcgcaga aaaccaaagc cgatctggcg aaaatgacga ttcacgaatc gcatattcgc 1560
   gatctctccg cctgggatca aaccgtgcct gcggagctgc gcggtaagta tctggcgctc 1620
   accgcccagg aaagcaatat ggtccagcat ctgaaacagc tgtcggcctc gggcgtgacc 1680
   catattgagc tgctgccggt cttcgatctg gcgacggtca atgagttcag cgacaaggtc 1740
   gccgatattc agcagccgtt cagccgcctg tgcgaggtca atagcgcggt gaagagcagc 1800
   gagttcgcgg gctattgcga cagcggttcg acggtcgaag aggtgctgac ccagctgaag 1860
   cagaacgaca gcaaggataa cccgcaggtg caggcgttga atacgctggt ggcgcagacc 1920
   gactcctata actggggcta cgatccgttc cactacacgg taccggaagg atcctacgcc 1980
   accgatccgg aaggcacggc gcgtattaaa gagttccgca ccatgattca ggcgatcaag 2040
   caggatctgg gaatgaacgt cattatggac gtggtgtaca accacaccaa cgccgccggc 2100
   ccgaccgatc gcacctcggt actggataag atcgtcccct ggtactacca gcgtctgaac 2160
   gaaaccaccg gcagcgtgga atcggctacc tgttgctccg actcggcgcc agagcaccgg 2220
   atgttcgcca agcttatcgc cgattcactg gcggtatgga ccaccgatta taagatcgat 2280
   ggcttccgct tcgacctgat gggctaccac ccgaaagcgc agatcctctc ggcctgggaa 2340
   cgcattaaag cgctgaaccc ggatatctac ttctttggcg aaggctggga ttccaaccag 2400
   agcgatcgct ttgaaattgc ctcgcaaatc aatctcaaag gcaccgggat cggcacgttc 2460
   tccgatcgtc tgcgcgacgc cgtgcgcggc ggcgggccgt tcgactccgg tgacgcatta 2520
   cgccagaacc agggcgtggg cagcggggct ggcgttctgc cgaatgagct gaccaccctg 2580
   agcgacgatc aggcgcgtca cctcgccgat ctgacccgtc tcggcatggc cggtaacctt 2640
   gcggactttg tgctgatcga caaagacggc gcggtgaaga gaggcagcga gattgattat 2700
   aacggcgcgc caggcggcta tgcggctgat ccgacggaag tcgtgaacta tgtgtcaaaa 2760
   cacgataacc agacgctgtg ggacatgatc agctataaag ccgctcagga ggcggatctc 2820
   gatacccgcg tccggatgca ggcggtgtcg ctggcgacgg tgatgctcgg ccaagggatc 2880
   gcctttgacc agcagggctc ggagctgctg cgctctaaat cctttacccg cgattcgtat 2940
   gattccggcg actggtttaa ccgcgtggac tactccctgc aggacaacaa ctacaacgtc 3000
   ggtatgccgc gcagcagcga tgatggcagc aattatgaca ttatcgcccg ggtgaaagac 3060
   gcggtggcta ctccgggtga aacggagctc aagcagatga ccgcgtttta tcaggagctg 3120
   accgcgctgc gtaaatcgtc tccgctgttt accctcggcg acggcgcgac ggtgatgaag 3180
   cgcgtggact tccgcaatac tggcgccgat cagcagacgg gtctgctggt gatgaccatc 3240
   gatgacggga tgcaggctgg cgccagtctg gacagccgtg tcgacggcat cgtggtggcg 3300
   atcaacgccg cgccggaaag ccggacgctg caggacttcg ccggcacatc gctccagctg 3360
   agcgctattc agcaggcggc gggcgaccgg tcgctggcga gcggcgtgca ggttgccgct 3420
   gacggttcgg tcacgctgcc ggcctggtcg gttgccgttc tcgagttgcc gcagggcgag 3480
   tcgcagggcg ctggcctgcc ggtgagcagt aaataacata acgtagggcg cccgtaaggg 3540
   cgcctctggc tgatgacaaa cctgtttcgt tattgtgctc gcctttctgt cgggtggcgg 3600
   ctttcgctta cccggcctac gaaagattgc aatttcaaac agttatgcag ttatctgtag 3660
   gccc 3664
<210> 13
   <211> 1083
   <212> PRT
   <213> Klebsiella pneumoniae
<400> 13
   Cys Asp Asn Ser Ser Ser Ser Ser Thr Ser Gly Ser Pro Gly Ser Pro
   1               5                   10                  15
   Gly Asn Pro Gly Asn Pro Gly Thr Pro Gly Thr Pro Asp Pro Gln Asp
               20                  25                  30
   Val Val Val Arg Leu Pro Asp Val Ala Val Pro Gly Glu Ala Val Gln
           35                  40                  45
   Ala Ser Ala Arg Gln Ala Val Ile His Leu Val Asp Ile Ala Gly Ile
       50                  55                  60
   Thr Ser Ser Thr Pro Ala Asp Tyr Ala Thr Lys Asn Leu Tyr Leu Trp
   65                  70                  75                  80
   Asn Asn Glu Thr Cys Asp Ala Leu Ser Ala Pro Val Ala Asp Trp Asn
                   85                  90                  95
   Asp Val Ser Thr Thr Pro Thr Gly Ser Asp Lys Tyr Gly Pro Tyr Trp
               100                 105                 110
   Val Ile Pro Leu Thr Lys Glu Ser Gly Cys Ile Asn Val Ile Val Arg
   115 120 125
   Asp Gly Thr Asn Lys Leu Ile Asp Ser Asp Leu Arg Val Ser Phe Ser
   130 135 140
   Asp Phe Thr Asp Arg Thr Val Ser Val Ile Ala Gly Asn Ser Ala Val
   145 150 155 160
   Tyr Asp Ser Arg Ala Asp Ala Phe Arg Ala Ala Phe Gly Val Ala Leu
   165 170 175
   Ala Asp Ala His Trp Val Asp Lys Thr Thr Leu Leu Trp Pro Gly Gly
   180 185 190
   Glu Asn Lys Pro Ile Val Arg Leu Tyr Tyr Ser His Ser Ser Lys Val
   195 200 205
   Ala Ala Asp Ser Asn Gly Glu Phe Ser Asp Lys Tyr Val Lys Leu Thr
   210 215 220
   Pro Thr Thr Val Ser Gln Gln Val Ser Met Arg Phe Pro His Leu Ala
   225 230 235 240
   Ser Tyr Pro Ala Phe Lys Leu Pro Asp Asp Val Asn Val Asp Glu Leu
   245 250 255
   Leu Gln Gly Glu Thr Val Ala Ile Ala Ala Glu Ser Asp Gly Ile Leu
   260 265 270
   Ser Ser Ala Thr Gln Val Gln Thr Ala Gly Val Leu Asp Asp Thr Tyr
   275 280 285
   Ala Ala Ala Ala Glu Ala Leu Ser Tyr Gly Ala Gln Leu Thr Asp Ser
   290 295 300
   Gly Val Thr Phe Arg Val Trp Ala Pro Thr Ala Gln Gln Val Glu Leu
   305 310 315 320
   Val Ile Tyr Ser Ala Asp Lys Lys Val Ile Ala Ser His Pro Met Thr
   325 330 335
   Arg Asp Ser Ala Ser Gly Ala Trp Ser Trp Gln Gly Gly Ser Asp Leu
   340 345 350
   Lys Gly Ala Phe Tyr Arg Tyr Ala Met Thr Val Tyr His Pro Gln Ser
   355 360 365
   Arg Lys Val Glu Gln Tyr Glu Val Thr Asp Pro Tyr Ala His Ser Leu
   370 375 380
   Ser Thr Asn Ser Glu Tyr Ser Gln Val Val Asp Leu Asn Asp Ser Ala
   385 390 395 400
   Leu Lys Pro Glu Gly Trp Asp Gly Leu Thr Met Pro His Ala Gln Lys
   405 410 415
   Thr Lys Ala Asp Leu Ala Lys Met Thr Ile His Glu Ser His Ile Arg
   420 425 430
   Asp Leu Ser Ala Trp Asp Gln Thr Val Pro Ala Glu Leu Arg Gly Lys
   435 440 445
   Tyr Leu Ala Leu Thr Ala Gln Glu Ser Asn Met Val Gln His Leu Lys
   450 455 460
   Gln Leu Ser Ala Ser Gly Val Thr His Ile Glu Leu Leu Pro Val Phe
   465 470 475 480
   Asp Leu Ala Thr Val Asn Glu Phe Ser Asp Lys Val Ala Asp Ile Gln
   485 490 495
   Gln Pro Phe Ser Arg Leu Cys Glu Val Asn Ser Ala Val Lys Ser Ser
   500 505 510
   Glu Phe Ala Gly Tyr Cys Asp Ser Gly Ser Thr Val Glu Glu Val Leu
   515 520 525
   Thr Gln Leu Lys Gln Asn Asp Ser Lys Asp Asn Pro Gln Val Gln Ala
   530 535 540
   Leu Asn Thr Leu Val Ala Gln Thr Asp Ser Tyr Asn Trp Gly Tyr Asp
   545 550 555 560
   Pro Phe His Tyr Thr Val Pro Glu Gly Ser Tyr Ala Thr Asp Pro Glu
   565 570 575
   Gly Thr Ala Arg Ile Lys Glu Phe Arg Thr Met Ile Gln Ala Ile Lys
   580 585 590
   Gln Asp Leu Gly Met Asn Val Ile Met Asp Val Val Tyr Asn His Thr
   595 600 605
   Asn Ala Ala Gly Pro Thr Asp Arg Thr Ser Val Leu Asp Lys Ile Val
   610 615 620
   Pro Trp Tyr Tyr Gln Arg Leu Asn Glu Thr Thr Gly Ser Val Glu Ser
   625 630 635 640
   Ala Thr Cys Cys Ser Asp Ser Ala Pro Glu His Arg Met Phe Ala Lys
   645 650 655
   Leu Ile Ala Asp Ser Leu Ala Val Trp Thr Thr Asp Tyr Lys Ile Asp
   660 665 670
   Gly Phe Arg Phe Asp Leu Met Gly Tyr His Pro Lys Ala Gln Ile Leu
   675 680 685
   Ser Ala Trp Glu Arg Ile Lys Ala Leu Asn Pro Asp Ile Tyr Phe Phe
   690 695 700
   Gly Glu Gly Trp Asp Ser Asn Gln Ser Asp Arg Phe Glu Ile Ala Ser
   705 710 715 720
   Gln Ile Asn Leu Lys Gly Thr Gly Ile Gly Thr Phe Ser Asp Arg Leu
   725 730 735
   Arg Asp Ala Val Arg Gly Gly Gly Pro Phe Asp Ser Gly Asp Ala Leu
   740 745 750
   Arg Gln Asn Gln Gly Val Gly Ser Gly Ala Gly Val Leu Pro Asn Glu
   755 760 765
   Leu Thr Thr Leu Ser Asp Asp Gln Ala Arg His Leu Ala Asp Leu Thr
   770 775 780
   Arg Leu Gly Met Ala Gly Asn Leu Ala Asp Phe Val Leu Ile Asp Lys
   785 790 795 800
   Asp Gly Ala Val Lys Arg Gly Ser Glu Ile Asp Tyr Asn Gly Ala Pro
   805 810 815
   Gly Gly Tyr Ala Ala Asp Pro Thr Glu Val Val Asn Tyr Val Ser Lys
   820 825 830
   His Asp Asn Gln Thr Leu Trp Asp Met Ile Ser Tyr Lys Ala Ala Gln
   835 840 845
   Glu Ala Asp Leu Asp Thr Arg Val Arg Met Gln Ala Val Ser Leu Ala
   850 855 860
   Thr Val Met Leu Gly Gln Gly Ile Ala Phe Asp Gln Gln Gly Ser Glu
   865 870 875 880
   Leu Leu Arg Ser Lys Ser Phe Thr Arg Asp Ser Tyr Asp Ser Gly Asp
   885 890 895
   Trp Phe Asn Arg Val Asp Tyr Ser Leu Gln Asp Asn Asn Tyr Asn Val
   900 905 910
   Gly Met Pro Arg Ser Ser Asp Asp Gly Ser Asn Tyr Asp Ile Ile Ala
   915 920 925
   Arg Val Lys Asp Ala Val Ala Thr Pro Gly Glu Thr Glu Leu Lys Gln
   930 935 940
   Met Thr Ala Phe Tyr Gln Glu Leu Thr Ala Leu Arg Lys Ser Ser Pro
   945 950 955 960
   Leu Phe Thr Leu Gly Asp Gly Ala Thr Val Met Lys Arg Val Asp Phe
   965 970 975
   Arg Asn Thr Gly Ala Asp Gln Gln Thr Gly Leu Leu Val Met Thr Ile
   980 985 990
   Asp Asp Gly Met Gln Ala Gly Ala Ser Leu Asp Ser Arg Val Asp Gly
   995 1000 1005
   Ile Val Val Ala Ile Asn Ala Ala Pro Glu Ser Arg Thr Leu Gln
   1010 1015 1020
   Asp Phe Ala Gly Thr Ser Leu Gln Leu Ser Ala Ile Gln Gln Ala
   1025 1030 1035
   Ala Gly Asp Arg Ser Leu Ala Ser Gly Val Gln Val Ala Ala Asp
   1040 1045 1050
   Gly Ser Val Thr Leu Pro Ala Trp Ser Val Ala Val Leu Glu Leu
   1055 1060 1065
   Pro Gln Gly Glu Ser Gln Gly Ala Gly Leu Pro Val Ser Ser Lys
   1070 1075 1080
<210> 14
   <211> 922
   <212> PRT
   <213> Bacillus sp.
<400> 14
   Asp Gly Thr Thr Thr Asn Val Ile Val His Tyr Phe Arg Pro Gly Gly
   1 5 10 15
   Asp Tyr Gln Ser Trp Ser Leu Trp Met Trp Pro Glu Gly Gly Asp Gly
   20 25 30
   Asn Asn Tyr Asn Phe Asn Gly Thr Asp Ser Tyr Gly Glu Ile Ala Asn
   35 40 45
   Val Ser Ile Pro Gly Ser Pro Ser Lys Val Gly Ile Ile Val Arg Thr
   50 55 60
   Gln Asp Trp Ala Lys Asp Val Ser Gln Asp Arg Tyr Ile Asp Leu Ser
   65 70 75 80
   Lys Gly His Glu Val Trp Leu Val Gln Gly Asn Ser Gln Ile Phe Tyr
   85 90 95
   Asn Glu Lys Asp Ala Glu Asp Ala Ala Glu Pro Ala Val Ser Asn Ala
   100 105 110
   Tyr Leu Asp Ala Pro Asn Lys Val Leu Val Lys Leu Ser Gln Pro Phe
   115 120 125
   Thr Leu Gly Glu Gly Ala Ser Gly Phe Thr Val His Asp Asp Thr Ala
   130 135 140
   Asn Gly Asp Ile Pro Val Thr Lys Val Lys Asn Ala Asn Lys Val Glu
   145 150 155 160
   Asp Val Thr Ala Ile Leu Ala Gly Thr Phe Gln His Ile Phe Gly Gly
   165 170 175
   Ser Asp Trp Ala Pro Asp Asn His Ser Thr Gln Leu Lys Lys Val Asn
   180 185 190
   Asp Asn Leu Tyr Gln Phe Ser Gly Glu Leu Pro Gly Gly Ser Tyr Gln
   195 200 205
   Tyr Lys Val Ala Leu Asn Asp Ser Trp Asn Ser Ser Tyr Pro Ser Asp
   210 215 220
   Asn Ile Asn Leu Thr Val Pro Asp Gly Gly Ala His Val Thr Phe Ser
   225 230 235 240
   Tyr Val Pro Ser Thr His Ala Val Tyr Asp Thr Ile Asn Asn Pro Gly
   245 250 255
   Ala Asn Leu Pro Leu Asp Gly Ser Gly Ile Lys Thr Asp Leu Val Thr
   260 265 270
   Val Thr Leu Gly Glu Asn Pro Asp Val Ser His Thr Leu Ser Ile Gln
   275 280 285
   Thr Asp Gly Phe Lys Thr Gly Arg Val Ile Pro Arg Asn Val Leu Asp
   290 295 300
   Phe Ser Gln Tyr Tyr Tyr Ser Gly Glu Asp Leu Gly Asn Thr Tyr Thr
   305 310 315 320
   Lys Lys Ala Thr Thr Phe Lys Val Trp Ala Pro Thr Ser Thr Lys Val
   325 330 335
   Asn Val Leu Leu Tyr Asn Lys Ala Ala Gly Ala Leu Thr Lys Thr Val
   340 345 350
   Pro Met Lys Ala Ser Gly His Gly Val Trp Ser Val Thr Val Pro Gln
   355 360 365
   Asn Leu Glu Asn Trp Tyr Tyr Leu Tyr Glu Val Thr Gly Gln Gly Ser
   370 375 380
   Thr Arg Thr Ala Val Asp Pro Tyr Ala Thr Ala Ile Ala Pro Asn Gly
   385 390 395 400
   Thr Arg Gly Met Val Val Asp Leu Ala Lys Thr Asn Pro Thr Gly Trp
   405 410 415
   Lys Ser Asp Lys His Met Thr Pro Lys Asn Ile Glu Asp Glu Val Ile
   420 425 430
   Tyr Glu Met His Val Arg Asp Phe Ser Ile Asp Ser Asn Ser Gly Met
   435 440 445
   Thr Asn Lys Gly Lys Tyr Leu Ala Leu Thr Glu Lys Gly Thr Lys Gly
   450 455 460
   Pro Glu Asn Val Lys Thr Gly Val Asp Ser Leu Lys Gln Leu Gly Ile
   465 470 475 480
   Thr His Val Gln Leu Gln Pro Val Phe Ala Phe Asn Ser Val Asp Glu
   485 490 495
   Thr Asp Pro Thr Gln Tyr Asn Trp Gly Tyr Asp Pro Arg Asn Tyr Asn
   500 505 510
   Val Pro Glu Gly Gln Tyr Ala Thr Asp Ala Asn Gly Thr Thr Arg Ile
   515 520 525
   Lys Glu Phe Lys Glu Met Val Leu Ser Leu His Arg Asn His Ile Gly
   530 535 540
   Val Asn Met Asp Val Val Tyr Asn His Thr Phe Ala Thr Gln Ile Ser
   545 550 555 560
   Asp Phe Asp Lys Ile Val Pro Gln Tyr Tyr Tyr Arg Thr Asp Asp Ala
   565 570 575
   Gly Asn Tyr Thr Asn Gly Ser Gly Thr Gly Asn Glu Val Ala Ala Glu
   580 585 590
   Arg Pro Met Val Gln Lys Phe Ile Ile Asp Ser Leu Lys Tyr Trp Val
   595 600 605
   Asn Glu Tyr His Ile Asp Gly Phe Arg Phe Asp Leu Met Ala Leu Leu
   610 615 620
   Gly Lys Asp Thr Met Ala Lys Ala Ala Gln Glu Leu His Ala Ile Asp
   625 630 635 640
   Pro Gly Ile Ala Leu Tyr Gly Glu Pro Trp Thr Gly Gly Thr Ser Ala
   645 650 655
   Leu Pro Thr Asp Gln Leu Leu Thr Lys Gly Val Gln Lys Gly Met Gly
   660 665 670
   Val Ala Val Phe Asn Asp Asn Leu Arg Asn Gly Leu Asp Gly Asn Val
   675 680 685
   Phe Asp Ala Ser Ser Gln Gly Phe Ala Thr Gly Ala Thr Gly Leu Thr
   690 695 700
   Asp Val Ile Lys Lys Gly Val Glu Gly Ser Ile Asn Asp Phe Thr Ser
   705 710 715 720
   Ser Pro Gly Glu Thr Ile Asn Tyr Val Thr Ser His Asp Asn Tyr Thr
   725 730 735
   Leu Trp Asp Lys Ile Ala Gln Ser Asn Pro Asn Asp Ser Glu Ala Asp
   740 745 750
   Arg Ile Lys Met Asp Glu Leu Ala Gln Ala Val Val Val Thr Ser Gln
   755 760 765
   Gly Val Pro Phe Met Gln Gly Gly Glu Glu Met Leu Arg Thr Lys Gly
       770                 775                 780
   Gly Asn Ser Asn Ser Tyr Asn Ala Gly Asp Ala Val Asn Glu Phe Asp
   785                 790                 795                 800
   Trp Ser Arg Lys Ala Gln Tyr Ser Asp Val Phe Asn Tyr Tyr Ser Gly
                   805                 810                 815
   Leu Ile His Leu Arg Leu Ala His Pro Ala Phe Arg Met Thr Thr Ala
               820                 825                 830
   Asn Gln Ile Lys Glu His Leu Gln Phe Ile Asp Ser Pro Asp Asn Thr
           835                 840                 845
   Val Ala Tyr Glu Leu Thr Asn His Ala Asn Lys Asp Lys Trp Gly Asn
       850                 855                 860
   Ile Val Val Ile Tyr Asn Pro Asn Lys Thr Ala Glu Thr Val Asn Leu
   865                 870                 875                 880
   Pro Ser Gly Lys Trp Ala Ile Asn Ala Thr Asn Gly Lys Ile Gly Glu
                   885                 890                 895
   Ser Thr Leu Ser His Ala Glu Gly His Val Gln Val Pro Gly Ile Ser
               900                 905                 910
   Met Met Ile Leu His Gln Glu Thr Asn Lys
           915                 920
<210> 15
   <211> 928
   <212> PRT
   <213> Bacillus deramificans
<400> 15
   Asp Gly Asn Thr Thr Thr Ile Ile Val His Tyr Phe Arg Pro Ala Gly
   1               5                   10                  15
   Asp Tyr Gln Pro Trp Ser Leu Trp Met Trp Pro Lys Asp Gly Gly Gly
               20                  25                  30
   Ala Glu Tyr Asp Phe Asn Gln Pro Ala Asp Ser Phe Gly Ala Val Ala
           35                  40                  45
   Ser Ala Asp Ile Pro Gly Asn Pro Ser Gln Val Gly Ile Ile Val Arg
       50                  55                  60
   Thr Gln Asp Trp Thr Lys Asp Val Ser Ala Asp Arg Tyr Ile Asp Leu
   65 70 75 80
   Ser Lys Gly Asn Glu Val Trp Leu Val Glu Gly Asn Ser Gln Ile Phe
   85 90 95
   Tyr Asn Glu Lys Asp Ala Glu Asp Ala Ala Lys Pro Ala Val Ser Asn
   100 105 110
   Ala Tyr Leu Asp Ala Ser Asn Gln Val Leu Val Lys Leu Ser Gln Pro
   115 120 125
   Leu Thr Leu Gly Glu Gly Ala Ser Gly Phe Thr Val His Asp Asp Thr
   130 135 140
   Ala Asn Lys Asp Ile Pro Val Thr Ser Val Lys Asp Ala Ser Leu Gly
   145 150 155 160
   Gln Asp Val Thr Ala Val Leu Ala Gly Thr Phe Gln His Ile Phe Gly
   165 170 175
   Gly Ser Asp Trp Ala Pro Asp Asn His Ser Thr Leu Leu Lys Lys Val
   180 185 190
   Thr Asn Asn Leu Tyr Gln Phe Ser Gly Asp Leu Pro Glu Gly Asn Tyr
   195 200 205
   Gln Tyr Lys Val Ala Leu Asn Asp Ser Trp Asn Asn Pro Ser Tyr Pro
   210 215 220
   Ser Asp Asn Ile Asn Leu Thr Val Pro Ala Gly Gly Ala His Val Thr
   225 230 235 240
   Phe Ser Tyr Ile Pro Ser Thr His Ala Val Tyr Asp Thr Ile Asn Asn
   245 250 255
   Pro Asn Ala Asp Leu Gln Val Glu Ser Gly Val Lys Thr Asp Leu Val
   260 265 270
   Thr Val Thr Leu Gly Glu Asp Pro Asp Val Ser His Thr Leu Ser Ile
   275 280 285
   Gln Thr Asp Gly Tyr Gln Ala Lys Gln Val Ile Pro Arg Asn Val Leu
   290 295 300
   Asn Ser Ser Gln Tyr Tyr Tyr Ser Gly Asp Asp Leu Gly Asn Thr Tyr
   305 310 315 320
   Thr Gln Lys Ala Thr Thr Phe Lys Val Trp Ala Pro Thr Ser Thr Gln
                   325                 330                 335
   Val Asn Val Leu Leu Tyr Asp Ser Ala Thr Gly Ser Val Thr Lys Ile
               340                 345                 350
   Val Pro Met Thr Ala Ser Gly His Gly Val Trp Glu Ala Thr Val Asn
           355                 360                 365
   Gln Asn Leu Glu Asn Trp Tyr Tyr Met Tyr Glu Val Thr Gly Gln Gly
       370                 375                 380
   Ser Thr Arg Thr Ala Val Asp Pro Tyr Ala Thr Ala Ile Ala Pro Asn
   385                 390                 395                 400
   Gly Thr Arg Gly Met Ile Val Asp Leu Ala Lys Thr Asp Pro Ala Gly
                   405                 410                 415
   Trp Asn Ser Asp Lys His Ile Thr Pro Lys Asn Ile Glu Asp Glu Val
               420                 425                 430
   Ile Tyr Glu Met Asp Val Arg Asp Phe Ser Ile Asp Pro Asn Ser Gly
           435                 440                 445
   Met Lys Asn Lys Gly Lys Tyr Leu Ala Leu Thr Glu Lys Gly Thr Lys
       450                 455                 460
   Gly Pro Asp Asn Val Lys Thr Gly Ile Asp Ser Leu Lys Gln Leu Gly
   465                 470                 475                 480
   Ile Thr His Val Gln Leu Met Pro Val Phe Ala Ser Asn Ser Val Asp
                   485                 490                 495
   Glu Thr Asp Pro Thr Gln Asp Asn Trp Gly Tyr Asp Pro Arg Asn Tyr
               500                 505                 510
   Asp Val Pro Glu Gly Gln Tyr Ala Thr Asn Ala Asn Gly Asn Ala Arg
           515                 520                 525
   Ile Lys Glu Phe Lys Glu Met Val Leu Ser Leu His Arg Glu His Ile
       530                 535                 540
   Gly Val Asn Met Asp Val Val Tyr Asn His Thr Phe Ala Thr Gln Ile
   545                 550                 555                 560
   Ser Asp Phe Asp Lys Ile Val Pro Glu Tyr Tyr Tyr Arg Thr Asp Asp
   565 570 575
   Ala Gly Asn Tyr Thr Asn Gly Ser Gly Thr Gly Asn Glu Ile Ala Ala
   580 585 590
   Glu Arg Pro Met Val Gln Lys Phe Ile Ile Asp Ser Leu Lys Tyr Trp
   595 600 605
   Val Asn Glu Tyr His Ile Asp Gly Phe Arg Phe Asp Leu Met Ala Leu
   610 615 620
   Leu Gly Lys Asp Thr Met Ser Lys Ala Ala Ser Glu Leu His Ala Ile
   625 630 635 640
   Asn Pro Gly Ile Ala Leu Tyr Gly Glu Pro Trp Thr Gly Gly Thr Ser
   645 650 655
   Ala Leu Pro Asp Asp Gln Leu Leu Thr Lys Gly Ala Gln Lys Gly Met
   660 665 670
   Gly Val Ala Val Phe Asn Asp Asn Leu Arg Asn Ala Leu Asp Gly Asn
   675 680 685
   Val Phe Asp Ser Ser Ala Gln Gly Phe Ala Thr Gly Ala Thr Gly Leu
   690 695 700
   Thr Asp Ala Ile Lys Asn Gly Val Glu Gly Ser Ile Asn Asp Phe Thr
   705 710 715 720
   Ser Ser Pro Gly Glu Thr Ile Asn Tyr Val Thr Ser His Asp Asn Tyr
   725 730 735
   Thr Leu Trp Asp Lys Ile Ala Leu Ser Asn Pro Asn Asp Ser Glu Ala
   740 745 750
   Asp Arg Ile Lys Met Asp Glu Leu Ala Gln Ala Val Val Met Thr Ser
   755 760 765
   Gln Gly Val Pro Phe Met Gln Gly Gly Glu Glu Met Leu Arg Thr Lys
   770 775 780
   Gly Gly Asn Asp Asn Ser Tyr Asn Ala Gly Asp Ala Val Asn Glu Phe
   785 790 795 800
   Asp Trp Ser Arg Lys Ala Gln Tyr Pro Asp Val Phe Asn Tyr Tyr Ser
   805 810 815
   Gly Leu Ile His Leu Arg Leu Asp His Pro Ala Phe Arg Met Thr Thr
   820 825 830
   Ala Asn Glu Ile Asn Ser His Leu Gln Phe Leu Asn Ser Pro Glu Asn
   835 840 845
   Thr Val Ala Tyr Glu Leu Thr Asp His Val Asn Lys Asp Lys Trp Gly
   850 855 860
   Asn Ile Ile Val Val Tyr Asn Pro Asn Lys Thr Val Ala Thr Ile Asn
   865 870 875 880
   Leu Pro Ser Gly Lys Trp Ala Ile Asn Ala Thr Ser Gly Lys Val Gly
   885 890 895
   Glu Ser Thr Leu Gly Gln Ala Glu Gly Ser Val Gln Val Pro Gly Ile
   900 905 910
   Ser Met Met Ile Leu His Gln Glu Val Ser Pro Asp His Gly Lys Lys
   915 920 925
<210> 16
   <211> 750
   <212> PRT
   <213> Pseudomonas amyloderamosa
<400> 16
   Ala Ile Asn Ser Met Ser Leu Gly Ala Ser Tyr Asp Ala Gln Gln Ala
   1 5 10 15
   Asn Ile Thr Phe Arg Val Tyr Ser Ser Gln Ala Thr Arg Ile Val Leu
   20 25 30
   Tyr Leu Tyr Ser Ala Gly Tyr Gly Val Gln Glu Ser Ala Thr Tyr Thr
   35 40 45
   Leu Ser Pro Ala Gly Ser Gly Val Trp Ala Val Thr Val Pro Val Ser
   50 55 60
   Ser Ile Lys Ala Ala Gly Ile Thr Gly Ala Val Tyr Tyr Gly Tyr Arg
   65 70 75 80
   Ala Trp Gly Pro Asn Trp Pro Tyr Ala Ser Asn Trp Gly Lys Gly Ser
   85 90 95
   Gln Ala Gly Phe Val Ser Asp Val Asp Ala Asn Gly Asp Arg Phe Asn
   100 105 110
   Pro Asn Lys Leu Leu Leu Asp Pro Tyr Ala Gln Glu Val Ser Gln Asp
           115                 120                 125
   Pro Leu Asn Pro Ser Asn Gln Asn Gly Asn Val Phe Ala Ser Gly Ala
       130                 135                 140
   Ser Tyr Arg Thr Thr Asp Ser Gly Ile Tyr Ala Pro Lys Gly Val Val
   145                 150                 155                 160
   Leu Val Pro Ser Thr Gln Ser Thr Gly Thr Lys Pro Thr Arg Ala Gln
                   165                 170                 175
   Lys Asp Asp Val Ile Tyr Glu Val His Val Arg Gly Phe Thr Glu Gln
               180                 185                 190
   Asp Thr Ser Ile Pro Ala Gln Tyr Arg Gly Thr Tyr Tyr Gly Ala Gly
           195                 200                 205
   Leu Lys Ala Ser Tyr Leu Ala Ser Leu Gly Val Thr Ala Val Glu Phe
       210                 215                 220
   Leu Pro Val Gln Glu Thr Gln Asn Asp Ala Asn Asp Val Val Pro Asn
   225                 230                 235                 240
   Ser Asp Ala Asn Gln Asn Tyr Trp Gly Tyr Met Thr Glu Asn Tyr Phe
                   245                 250                 255
   Ser Pro Asp Arg Arg Tyr Ala Tyr Asn Lys Ala Ala Gly Gly Pro Thr
               260                 265                 270
   Ala Glu Phe Gln Ala Met Val Gln Ala Phe His Asn Ala Gly Ile Lys
           275                 280                 285
   Val Tyr Met Asp Val Val Tyr Asn His Thr Ala Glu Gly Gly Thr Trp
       290                 295                 300
   Thr Ser Ser Asp Pro Thr Thr Ala Thr Ile Tyr Ser Trp Arg Gly Leu
   305                 310                 315                 320
   Asp Asn Ala Thr Tyr Tyr Glu Leu Thr Ser Gly Asn Gln Tyr Phe Tyr
                   325                 330                 335
   Asp Asn Thr Gly Ile Gly Ala Asn Phe Asn Thr Tyr Asn Thr Val Ala
               340                 345                 350
   Gln Asn Leu Ile Val Asp Ser Leu Ala Tyr Trp Ala Asn Thr Met Gly
           355                 360                 365
   Val Asp Gly Phe Arg Phe Asp Leu Ala Ser Val Leu Gly Asn Ser Cys
   370 375 380
   Leu Asn Gly Ala Tyr Thr Ala Ser Ala Pro Asn Cys Pro Asn Gly Gly
   385 390 395 400
   Tyr Asn Phe Asp Ala Ala Asp Ser Asn Val Ala Ile Asn Arg Ile Leu
   405 410 415
   Arg Glu Phe Thr Val Arg Pro Ala Ala Gly Gly Ser Gly Leu Asp Leu
   420 425 430
   Phe Ala Glu Pro Trp Ala Ile Gly Gly Asn Ser Tyr Gln Leu Gly Gly
   435 440 445
   Phe Pro Gln Gly Trp Ser Glu Trp Asn Gly Leu Phe Arg Asp Ser Leu
   450 455 460
   Arg Gln Ala Gln Asn Glu Leu Gly Ser Met Thr Ile Tyr Val Thr Gln
   465 470 475 480
   Asp Ala Asn Asp Phe Ser Gly Ser Ser Asn Leu Phe Gln Ser Ser Gly
   485 490 495
   Arg Ser Pro Trp Asn Ser Ile Asn Phe Ile Asp Val His Asp Gly Met
   500 505 510
   Thr Leu Lys Asp Val Tyr Ser Cys Asn Gly Ala Asn Asn Ser Gln Ala
   515 520 525
   Trp Pro Tyr Gly Pro Ser Asp Gly Gly Thr Ser Thr Asn Tyr Ser Trp
   530 535 540
   Asp Gln Gly Met Ser Ala Gly Thr Gly Ala Ala Val Asp Gln Arg Arg
   545 550 555 560
   Ala Ala Arg Thr Gly Met Ala Phe Glu Met Leu Ser Ala Gly Thr Pro
   565 570 575
   Leu Met Gln Gly Gly Asp Glu Tyr Leu Arg Thr Leu Gln Cys Asn Asn
   580 585 590
   Asn Ala Tyr Asn Leu Asp Ser Ser Ala Asn Trp Leu Thr Tyr Ser Trp
   595 600 605
   Thr Thr Asp Gln Ser Asn Phe Tyr Thr Phe Ala Gln Arg Leu Ile Ala
   610 615 620
   Phe Arg Lys Ala His Pro Ala Leu Arg Pro Ser Ser Trp Tyr Ser Gly
   625                 630                 635                 640
   Ser Gln Leu Thr Trp Tyr Gln Pro Ser Gly Ala Val Ala Asp Ser Asn
                   645                 650                 655
   Tyr Trp Asn Asn Thr Ser Asn Tyr Ala Ile Ala Tyr Ala Ile Asn Gly
               660                 665                 670
   Pro Ser Leu Gly Asp Ser Asn Ser Ile Tyr Val Ala Tyr Asn Gly Trp
           675                 680                 685
   Ser Ser Ser Val Thr Phe Thr Leu Pro Ala Pro Pro Ser Gly Thr Gln
       690                 695                 700
   Trp Tyr Arg Val Thr Asp Thr Cys Asp Trp Asn Asp Gly Ala Ser Thr
   705                 710                 715                 720
   Phe Val Ala Pro Gly Ser Glu Thr Leu Ile Gly Gly Ala Gly Thr Thr
                   725                 730                 735
   Tyr Gly Gln Cys Gly Gln Ser Leu Leu Leu Leu Ile Ser Lys
               740                 745                 750